# EUROPEAN PATENT SPECIFICATION

(11) **EP 4 161 931 B1**
(45) Date of publication and mention of the grant of the patent: **18.02.2026**
(21) Application number: 21732822.8
(22) Date of filing: 07.06.2021
(51) Int. Cl.: C07D 487/04, A61P 31/04, A61K 31/4985

(54) **NOVEL IMIDAZOPYRAZINE DERIVATIVES**
IMIDAZOPYRAZINDERIVATE
NOUVEAUX DÉRIVÉS D'IMIDAZOPYRAZINE

(30) Priority: 09.06.2020 WO PCT/CN2020/095147
(43) Date of publication of application: 12.04.2023
(73) Proprietor: F. Hoffmann-La Roche AG, 4070 Basel (CH)
(72) Inventor: CHENG, Zhanling, Shanghai, 201203 (CN); KRAMER, Christian, 4070 Basel (CH); LERNER, Christian, 4070 Basel (CH); LIU, Yongqiang, Shanghai, 201203 (CN); NETTEKOVEN, Matthias, 4070 Basel (CH); PFLIEGER, Philippe, 4070 Basel (CH); SCHMITT, Sébastien, 4070 Basel (CH); STOLL, Theodor, 4070 Basel (CH); WANG, Jianhua, Shanghai, 201203 (CN); WANG, Yongguang, Shanghai, 201203 (CN); YANG, Song, Shanghai, 201203 (CN); ZHOU, Chengang, Shanghai, 201203 (CN)
(74) Representative: Neuhaus, Christian Michel
(86) International application number: PCT/EP2021/065088
(87) International publication number: WO 2021/249896

(56) References cited:
- WO-A1-2012/168733
- WO-A1-2020/126953
- WO-A1-2020/127075

## Description

### Field of the Invention

The present invention relates to novel imidazopyrazine derivatives which exhibit antibacterial properties. The invention also relates to methods of using the compounds for the treatment or prevention of bacterial infections and resulting diseases, in particular for the treatment or prevention of infections with *Acinetobacter baumannii* and resulting diseases.

### Background of the Invention

*Acinetobacter baumannii* is a Gram-negative, aerobic, nonfermenting bacterium recognized over the last decades as an emergining pathogen with very limited treatment options.

*A. baumannii* is considered to be a serious threat by the US Centers for Disease Control and Prevention and belongs to the so called 'ESKAPE' pathogens (***E**nterococcus faecium, **S**taphylococcus aureus, **K**lebsiella pneumoniae, **A**cinetobacter baumannii, **P**seudomonas aeruginosa* and ***E**nterobacter species & E. coli*) that currently cause the majority of nosocomial infections and effectively "escape" the activity of antimicrobial agents.

*A. baumannii* is most often encountered in intensive care units and surgical wards, where extensive antibiotic use has enabled selection for resistance against all known antimicrobials and where it causes infections that include bacteremia, pneumonia, meningitis, urinary tract infection, and wound infection.

*A. baumannii* has an exceptional ability to upregulate and acquire resistance determinants and shows an environmental persistance that allows its survival and spread in the nosocomial setting, making this organism a frequent cause of outbreaks of infection and an endemic, health care-associated pathogen.

Due to increasing antibiotic resistance to most if not all available therapeutic options, Muti-Drug Resistant (MDR) *A. baumanniii* infections, especially those caused by Carbapenem resistant *A. baumannii,* are extremely difficult or even impossible to treat with high mortality rate as well as increased morbidity and length of stay in intensive care unit.

*Acinetobacter baumannii* has been defined and still remains "a prime example of a mismatch between unmet medical needs and the current antimicrobial research and development pipeline" according to the Antimicrobial Availability Task Force (AATF) of the Infectious Diseases Society of America (IDSA). Thus, there is a high demand and need to identify compounds suitable for the treatment of diseases and infections caused by *Acinetobacter baumannii.*

The present invention provides novel compounds which exhibit activity against drug-susceptible as well as drug-resistant strains of *Acinetobacter baumannii.*

### Summary of the Invention

In a first aspect, the present invention provides compounds of formula (II) or (III) 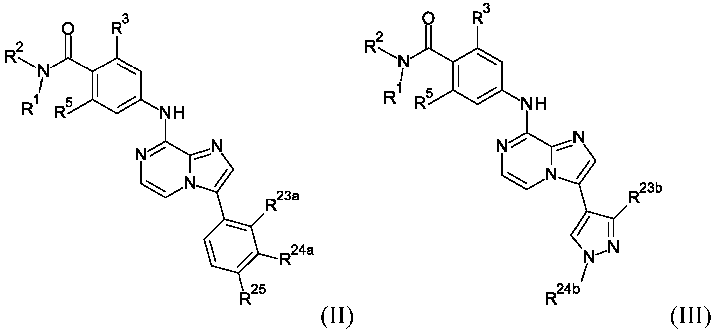 or pharmaceutically acceptable salts thereof, wherein R^{x} and R³ to R⁵ are as defined herein.

In one aspect, the present invention provides a process of manufacturing the compounds of formula (II) or (III) described herein, comprising:
(i) Suzuki coupling of a compound (A), wherein X is a halogen or a triflate, preferably wherein X is iodo, and wherein the double cross indicates the point of attachment of (A) to the remainder of formula (II) or (III) , or a synthetic precursor thereof, 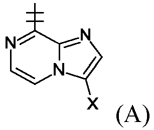 with a boronic acid (R=H) or a boronic acid ester (R=alkyl or both R, taken together with the atoms to which they are attached, form a heterocycle) (B), wherein R⁴ is are as defined herein, 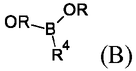 to form said compound of formula (II) or (III) or a synthetic precursor thereof; or
(ii) Buchwald coupling of a compound (C), wherein the double cross indicates the point of attachment of (C) to R⁴ as defined herein, or a synthetic precursor thereof, 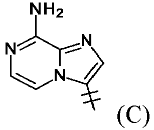 with an aryl halide (D), wherein R³ and R⁵ are as defined herein, X is a halogen and the double cross indicates the point of attachment of (D) to the remainder of formula (II) or (III) , or a synthetic precursor thereof, 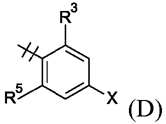 to form said compound of formula (II) or (III) , or a synthetic precursor thereof; or
(iii) nucleophilic aromatic substitution (SNAr) comprising reacting a compound (C1), wherein X is a halogen and the double cross indicates the point of attachment of (C1) to R⁴ as defined herein, or a synthetic precursor thereof, 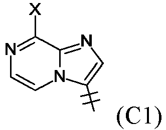 with an aniline derivative (D1), wherein wherein R³ and R⁵ are as defined herein and the double cross indicates the point of attachment of (D1) to the remainder of formula (II) or (III) , or a synthetic precursor thereof, 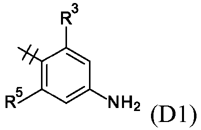 to form said compound of formula (II) or (III) , or a synthetic precursor thereof; or
(iv) amide coupling comprising reacting a carboxylic acid (E), wherein the double cross indicates the point of attachment of (E) to the remainder of formula (II) or (III) , or a synthetic precursor thereof, 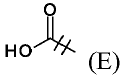 with a primary or secondary amine R^{y}, to form a compound of formula (G), wherein R^{y'} equals R^{x} as defined herein, or is a synthetic precursor thereof

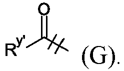

In a further aspect, the present invention provides a compound of formula (II) or (III) as described herein, when manufactured according to the processes described herein.

In a further aspect, the present invention provides a compound of formula (II) or (III) as described herein, or a pharmaceutically acceptable salt thereof, for use as therapeutically active substance.

In a further aspect, the present invention provides a pharmaceutical composition comprising a compound of formula (II) or (III) as described herein, or a pharmaceutically acceptable salt thereof, and a therapeutically inert carrier.

In a further aspect, the present invention provides a compound of formula (II) or (III) as described herein, or a pharmaceutically acceptable salt thereof, for use as antibiotic.

In a further aspect, the present invention provides a compound of formula (II) or (III) as described herein, or a pharmaceutically acceptable salt thereof, for use in the treatment or prevention of nosocomial infections and resulting diseases.

In a further aspect, the present invention provides a compound of formula (II) or (III) as described herein, or a pharmaceutically acceptable salt thereof, for use in the treatment or prevention of infections and resulting diseases caused by Gram-negative bacteria.

In a further aspect, the present invention provides a compound of formula (II) or (III) as described herein, or a pharmaceutically acceptable salt thereof, for use in the treatment or prevention of infections and resulting diseases caused by ***E**nterococcus faecium, **S**taphylococcus aureus, **K**lebsiella pneumoniae, **A**cinetobacter baumannii, **P**seudomonas aeruginosa, **E**nterobacter species* or *E. coli,* or a combination therof.

### Detailed Description of the Invention

### Definitions

The invention is defined by the claims. Any subject-matter falling outside the scope of the claims is provided for information purposes only. In addition, any reference to methods of treatment in the subsequent paragraphs of this description is to be interpreted as references to the compounds, pharmaceutical compositions and medicaments of the present invention for use in a method for treatment of the human or animal body by therapy.

Features, integers, characteristics, compounds, chemical moieties or groups described in conjunction with a particular aspect, embodiment or example of the invention are to be understood to be applicable to any other aspect, embodiment or example described herein, unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive.

The term "alkyl" refers to a mono- or multivalent, e.g., a mono- or bivalent, linear or branched saturated hydrocarbon group of 1 to 6 carbon atoms ("C₁-C₆-alkyl"), e.g., 1, 2, 3, 4, 5, or 6 carbon atoms. In some embodiments, the alkyl group contains 1 to 3 carbon atoms, e.g., 1, 2 or 3 carbon atoms. Some non-limiting examples of alkyl include methyl, ethyl, propyl, 2-propyl (isopropyl), n-butyl, iso-butyl, sec-butyl, tert-butyl, and 2,2-dimethylpropyl. A particularly preferred, yet non-limiting example of alkyl is methyl.

The term "alkynyl" denotes a monovalent linear or branched hydrocarbon group of 2 to 6 carbon atoms with at least one triple bond ("C₂-C₆-alkynyl"). In particular embodiments, alkynyl has 2 to 4 carbon atoms with at least one triple bond. Examples of alkynyl include ethynyl, propynyl, n-butynyl or isobutynyl. Preferred alkynyl is propynyl.

The term "alkynyloxy" refers to an alkynyl group, as previously defined, attached to the parent molecular moiety via an oxygen atom. Unless otherwise specified, the alkynyloxy group contains 2 to 6 carbon atoms ("C₂-C₆-alkynyloxy"). In some preferred embodiments, the alkynyloxy group contains 2 to 4 carbon atoms. In still other embodiments, the alkynyloxy group contains 2 to 3 carbon atoms. Some non-limiting examples of alkynyloxy groups include ethynoxy, propynoxy, and butynoxy.

The term "alkoxy" refers to an alkyl group, as previously defined, attached to the parent molecular moiety via an oxygen atom. Unless otherwise specified, the alkoxy group contains 1 to 6 carbon atoms ("C₁-C₆-alkoxy"). In some preferred embodiments, the alkoxy group contains 1 to 4 carbon atoms. In still other embodiments, the alkoxy group contains 1 to 3 carbon atoms. Some non-limiting examples of alkoxy groups include methoxy, ethoxy, n-propoxy, isopropoxy, n-butoxy, isobutoxy and tert-butoxy. A particularly preferred, yet non-limiting example of alkoxy is methoxy.

The term "halogen" or "halo" refers to fluoro (F), chloro (Cl), bromo (Br), or iodo (I). Preferably, the term "halogen" or "halo" refers to fluoro (F), chloro (Cl) or bromo (Br). Particularly preferred, yet non-limiting examples of "halogen" or "halo" are fluoro (F) and chloro (Cl).

The term "cycloalkyl" as used herein refers to a saturated or partly unsaturated monocyclic or bicyclic hydrocarbon group of 3 to 10 ring carbon atoms ("C₃-C₁₀-cycloalkyl"). In some preferred embodiments, the cycloalkyl group is a saturated monocyclic hydrocarbon group of 3 to 8 ring carbon atoms, in particular 3 to 7 ring carbon atoms. "Bicyclic cycloalkyl" refers to cycloalkyl moieties consisting of two saturated carbocycles having two carbon atoms in common, i.e., the bridge separating the two rings is either a single bond or a chain of one or two ring atoms, and to spirocyclic moieties, i.e., the two rings are connected via one common ring atom. Preferably, the cycloalkyl group is a saturated monocyclic hydrocarbon group of 3 to 6 ring carbon atoms, e.g., of 3, 4, 5 or 6 carbon atoms. Some non-limiting examples of cycloalkyl include cyclopropyl, cyclobutyl, cyclopentyl, cyclohexyl and cycloheptyl.

The term "aminoalkynyloxy" refers to an alkynyloxy group, wherein at least one of the hydrogen atoms of the alkynyloxy group has been replaced by an amino group. Preferably, "aminoalkynyloxy" refers to an alkoxy group wherein 1, 2 or 3 hydrogen atoms of the alkynyloxy group have been replaced by an amino group. A preferred, yet non-limiting example of 4-aminobut-2-ynoxy.

The term "aminoalkyl" refers to an alkyl group, wherein at least one of the hydrogen atoms of the alkyl group has been replaced by an amino group. Preferably, "aminoalkyl" refers to an alkyl group wherein 1, 2 or 3 hydrogen atoms of the alkyl group have been replaced by an amino group. A preferred, yet non-limiting example of aminoalkyl is aminomethyl.

The terms "heterocycloalkyl" and "heterocyclyl" are used interchangeably and refer to a saturated or partly unsaturated mono- or bicyclic, preferably monocyclic ring system of 3 to 20 ring atoms, preferably 3 to 15 ring atoms, more preferably 3 to 10 ring atoms, most preferably 3 to 6 ring atoms, wherein 1, 2, or 3 of said ring atoms are heteroatoms selected from N, O and S, the remaining ring atoms being carbon ("C₁-C₁₉-heterocyclyl"). Preferably, 1 to 2 of said ring atoms are selected from N and O, the remaining ring atoms being carbon. "Bicyclic heterocyclyl" refers to heterocyclic moieties consisting of two cycles having two ring atoms in common, i.e., the bridge separating the two rings is either a single bond or a chain of one or two ring atoms, and to spirocyclic moieties, i.e., the two rings are connected via one common ring atom. Some non-limiting examples of monocyclic heterocyclyl groups include azetidin-3-yl, azetidin-2-yl, oxetan-3-yl, oxetan-2-yl, 2-oxopyrrolidin-1-yl, 2-oxopyrrolidin-3-yl, 5-oxopyrrolidin-2-yl, 5-oxopyrrolidin-3-yl, 2-oxo-1-piperidyl, 2-oxo-3-piperidyl, 2-oxo-4-piperidyl, 6-oxo-2-piperidyl, 6-oxo-3-piperidyl, 1-piperidinyl, 2-piperidinyl, 3-piperidinyl, 4-piperidinyl, morpholino, morpholin-2-yl, morpholin-3-yl, piperazinyl, and dihydroyridyl.

The term "aryl" refers to a monocyclic, bicyclic, or tricyclic carbocyclic ring system having a total of 6 to 14 ring members ("C₆-C₁₄-aryl"), preferably, 6 to 12 ring members, and more preferably 6 to 10 ring members, and wherein at least one ring in the system is aromatic. Some non-limiting examples of aryl include phenyl and 9H-fluorenyl (e.g. 9H-fluoren-9-yl). A particularly preferred, yet non-limiting example of aryl is phenyl.

The term "aryloxy" refers to an aryl group, as previously defined, attached to the parent molecular moiety via an oxygen atom. A preferred, yet non-limiting example of aryloxy is phenoxy.

The term "heteroaryl" refers to a mono- or multivalent, monocyclic or bicyclic, preferably bicyclic ring system having a total of 5 to 14 ring members, preferably, 5 to 12 ring members, and more preferably 5 to 10 ring members, wherein at least one ring in the system is aromatic, and at least one ring in the system contains one or more heteroatoms. Preferably, "heteroaryl" refers to a 5-10 membered heteroaryl comprising 1, 2, 3 or 4 heteroatoms independently selected from O, S and N. Most preferably, "heteroaryl" refers to a 5-10 membered heteroaryl comprising 1 to 2 heteroatoms independently selected from O and N. Some non-limiting examples of heteroaryl include 2-pyridyl, 3-pyridyl, 4-pyridyl, indol-1-yl, 1H-indol-2-yl, 1H-indol-3-yl, 1H-indol-4-yl, 1H-indol-5-yl, 1H-indol-6-yl, 1H-indol-7-yl, 1,2-benzoxazol-3-yl, 1,2-benzoxazol-4-yl, 1,2-benzoxazol-5-yl, 1,2-benzoxazol-6-yl, 1,2-benzoxazol-7-yl, 1H-indazol-3-yl, 1H-indazol-4-yl, 1H-indazol-5-yl, 1H-indazol-6-yl, 1H-indazol-7-yl, pyrazol-1-yl, 1H-pyrazol-3-yl, 1H-pyrazol-4-yl, 1H-pyrazol-5-yl, imidazol-1-yl, 1H-imidazol-2-yl, 1H-imidazol-4-yl, 1H-imidazol-5-yl, oxazol-2-yl, oxazol-4-yl, oxazol-5-yl, thiazolyl, pyrazolyl, pyridazinyl, pyrimidinyl, isoxazolyl, and oxadiazolyl. Preferred, yet non-limiting examples of heteroaryl include pyrazolyl and pyridyl.

The term "heteroaryloxy" refers to a heteroaryl group, as previously defined, attached to the parent molecular moiety via an oxygen atom. A preferred, yet non-limiting example of heteroaryloxy is pyridoxy.

The term "hydroxy" refers to an -OH group.

The term "amino" refers to an -NH₂ group.

The term "cyano" refers to a -CN (nitrile) group.

The term "carboxy" refers to a -COOH group.

The term "carbamoyl" refers to a -C(O)NH₂ group.

The term "carbonyl" refers to a -C(O)- group.

The term "alkoxycarbonyl" refers to a -C(O)-O-alkyl group (i.e., an alkyl ester).

The term "haloalkyl" refers to an alkyl group, wherein at least one of the hydrogen atoms of the alkyl group has been replaced by a halogen atom, preferably fluoro. Preferably, "haloalkyl" refers to an alkyl group wherein 1, 2 or 3 hydrogen atoms of the alkyl group have been replaced by a halogen atom, most preferably fluoro. Particularly preferred, yet non-limiting examples of haloalkyl are trifluoromethyl and trifluoroethyl.

The term "haloalkoxy" refers to an alkoxy group, wherein at least one of the hydrogen atoms of the alkoxy group has been replaced by a halogen atom, preferably fluoro. Preferably, "haloalkoxy" refers to an alkoxy group wherein 1, 2 or 3 hydrogen atoms of the alkoxy group have been replaced by a halogen atom, most preferably fluoro. A particularly preferred, yet non-limiting example of haloalkoxy is trifluoromethoxy (-OCF₃).

The term "cyanoalkoxy" refers to an alkoxy group, wherein at least one of the hydrogen atoms of the alkoxy group has been replaced by a cyano group. Preferably, "cyanoalkoxy" refers to an alkoxy group wherein 1, 2 or 3 hydrogen atoms of the alkoxy group have been replaced by a cyano group. A particularly preferred, yet non-limiting example of cyanoalkoxy is cyanomethoxy.

The term "cyanoalkyl" refers to an alkyl group, wherein at least one of the hydrogen atoms of the alkyl group has been replaced by a cyano group. Preferably, "cyanoalkyl" refers to an alkyl group wherein 1, 2 or 3 hydrogen atoms of the alkyl group have been replaced by a cyano group. A particularly preferred, yet non-limiting example of cyanoalkyl is cyanomethyl.

The term "carboxyalkyl" refers to an alkyl group, wherein at least one of the hydrogen atoms of the alkyl group has been replaced by a carboxy group. Preferably, "carboxyalkyl" refers to an alkyl group wherein 1, 2 or 3 hydrogen atoms of the alkyl group have been replaced by a carboxy group. A particularly preferred, yet non-limiting example of carboxyalkyl is carboxymethyl.

The term "carbamoylalkyl" refers to an alkyl group, wherein at least one of the hydrogen atoms of the alkyl group has been replaced by a carbamoyl group. Preferably, "carbamoylalkyl" refers to an alkyl group wherein 1, 2 or 3 hydrogen atoms of the alkyl group have been replaced by a carbamoyl group. A particularly preferred, yet non-limiting example of carbamoylalkyl is 2-amino-2-oxo-ethyl.

The term "hydroxyalkyl" refers to an alkyl group, wherein at least one of the hydrogen atoms of the alkyl group has been replaced by a hydroxy group. Preferably, "hydroxyalkyl" refers to an alkyl group wherein 1, 2 or 3 hydrogen atoms, most preferably 1 hydrogen atom of the alkyl group have been replaced by a hydroxy group. Preferred, yet non-limiting examples of hydroxyalkyl are hydroxymethyl and hydroxyethyl (e.g. 2-hydroxyethyl). A particularly preferred, yet non-limiting example of hydroxyalkyl is hydroxymethyl.

The term "pharmaceutically acceptable salt" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid and the like, in particular hydrochloric acid, and organic acids such as acetic acid, trifluoroacetic acid, propionic acid, glycolic acid, pyruvic acid, oxalic acid, maleic acid, malonic acid, succinic acid, fumaric acid, tartaric acid, lactic acid, citric acid, benzoic acid, cinnamic acid, mandelic acid, methanesulfonic acid, ethanesulfonic acid, p-toluenesulfonic acid, salicylic acid, N-acetylcystein and the like. In addition these salts may be prepared by addition of an inorganic base or an organic base to the free acid. Salts derived from an inorganic base include, but are not limited to, the sodium, potassium, lithium, ammonium, calcium, magnesium salts and the like. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyimine resins and the like. Particular pharmaceutically acceptable salts of compounds of formula (II) or (III) are hydrochlorides, fumarates, lactates (in particular derived from L-(+)-lactic acid), tartrates (in particular derived from L-(+)-tartaric acid) and trifluoroacetates.

The term "protective group" (PG) denotes the group which selectively blocks a reactive site in a multifunctional compound such that a chemical reaction can be carried out selectively at another unprotected reactive site in the meaning conventionally associated with it in synthetic chemistry. Protective groups can be removed at the appropriate point. Exemplary protective groups are amino-protective groups, carboxy-protective groups or hydroxy-protective groups. Particular protective groups are the tert-butoxycarbonyl (Boc), benzyloxycarbonyl (Cbz), fluorenylmethoxycarbonyl (Fmoc) and benzyl (Bn). Further particular protective groups are the tert-butoxycarbonyl (Boc) and the fluorenylmethoxycarbonyl (Fmoc). More particular protective group is the tert-butoxycarbonyl (Boc). Exemplary protective groups and their application in organic synthesis are described, for example, in "Protective Groups in Organic Chemistry" by T. W. Greene and P. G. M. Wutts, 5th Ed., 2014, John Wiley & Sons, N.Y.

The compounds of formula (II) or (III) can contain several asymmetric centers and can be present in the form of optically pure enantiomers, mixtures of enantiomers such as, for example, racemates, optically pure diastereioisomers, mixtures of diastereoisomers, diastereoisomeric racemates or mixtures of diastereoisomeric racemates.

According to the Cahn-Ingold-Prelog Convention, the asymmetric carbon atom can be of the "R" or "S" configuration.

The term "treatment" as used herein includes: (1) inhibiting the state, disorder or condition (e.g. arresting, reducing or delaying the development of the disease, or a relapse thereof in case of maintenance treatment, of at least one clinical or subclinical symptom thereof); and/or (2) relieving the condition (i.e., causing regression of the state, disorder or condition or at least one of its clinical or subclinical symptoms). The benefit to a patient to be treated is either statistically significant or at least perceptible to the patient or to the physician. However, it will be appreciated that when a medicament is administered to a patient to treat a disease, the outcome may not always be effective treatment.

The term "prevention" as used herein includes: preventing or delaying the appearance of clinical symptoms of the state, disorder or condition developing in a mammal and especially a human that may be afflicted with or predisposed to the state, disorder or condition but does not yet experience or display clinical or subclinical symptoms of the state, disorder or condition.

The term "mammal" as used herein includes both humans and non-humans and includes but is not limited to humans, non-human primates, canines, felines, murines, bovines, equines, and porcines. In a particularly preferred embodiment, the term "mammal" refers to humans.

The term "nosocomial infection" refers to a hospital-acquired infection (HAI), which is an infection that is acquired in a hospital or other health care facility. To emphasize both hospital and nonhospital settings, it is sometimes instead called a health care-associated infection (HAI or HCAI). Such an infection can be acquired in hospitals, nursing homes, rehabilitation facilities, outpatient clinics, or other clinical settings.

### Compounds of the Invention

In a first aspect, the present invention provides compounds of formula (II) or (III) or a pharmaceutically acceptable salt thereof 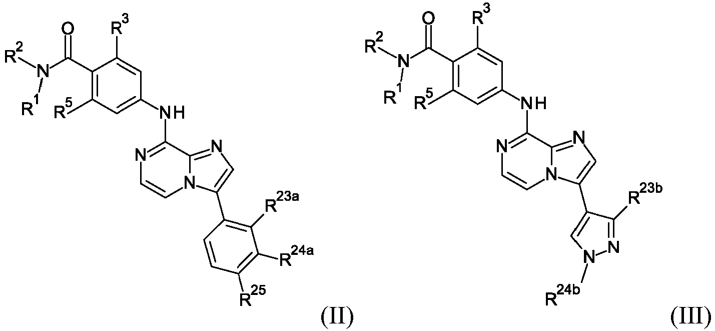 wherein:
R¹ is selected from R⁶-C₁-C₆-alkyl-, R⁶-C₁-C₆-alkyl-CH(C₁-C₆-alkoxycarbonyl)-, R⁷-C₁-C₆-alkyl-X-C₁-C₆-alkyl-, a group 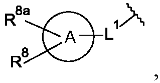 and a group 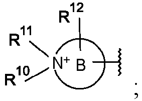 and
R² is selected from hydrogen and C₁-C₆-alkyl;
or R¹ and R², taken together with the nitrogen atom to which they are attached, form a 3- to 14-membered heterocycle that is substituted with R⁹ and R^{9a};
R³ is selected from hydrogen, halogen, and C₁-C₆-alkyl; or
R³ and R², taken together with the atoms to which they are attached, form a 3- to 14-membered heterocycle;
R⁵ is selected from hydrogen, C₁-C₆-alkyl, and halogen;
R⁶ is a group R¹⁶-L²-;
R⁷ is a group R¹⁶-L³-;
R⁸ is a group R¹⁶-L⁴-;
R^{8a}, R^{9a}, R^{11a}, R¹², R¹⁸, R¹⁹, R²⁰, R²¹, R²², R²³, R²⁴, and R²⁵ are independently selected from hydrogen, halogen, hydroxy, cyano, amino, carbamoyl, carboxy, C₁-C₆-alkyl, C₁-C₆-alkoxy, halo-C₁-C₆-alkyl, cyano-C₁-C₆-alkyl, carboxy-C₁-C₆-alkyl, hydroxy-C₁-C₆-alkyl, halo-C₁-C₆-alkoxy, cyano-C₁-C₆-alkoxy, C₂-C₆-alkynyloxy, amino-C₂-C₆-alkynyloxy, aryloxy, (5- to 14-membered heteroaryl)oxy, C₁-C₆-alkyl-(5- to 14-membered heteroaryl)oxy, and C₁-C₆-alkoxycarbonyl;
R⁹ is a group R¹⁶-L⁵- or a group 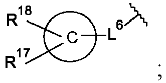
R¹⁰ is selected from C₁-C₆-alkyl, C₁-C₆-alkoxycarbonyl-C₁-C₆-alkyl, C₁-C₆-alkyl-NH-C₁-C₆-alkyl, amino-C₁-C₆-alkyl, carbamoyl, carbamoyl-C₁-C₆-alkyl, carboxy-C₁-C₆-alkyl, cyano-C₁-C₆-alkyl, halo-C₁-C₆-alkyl, hydroxy-C₁-C₆-alkyl, and a group 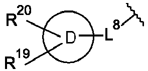 ;
R¹¹ is selected from C₁-C₆-alkyl, carbamoyl, carboxy, carboxy-C₁-C₆-alkyl, and hydroxy-C₁-C₆-alkyl;
R¹³ and R¹⁴ are independently selected from C₁-C₆-alkyl, amino-C₁-C₆-alkyl, carbamoyl-C₁-C₆-alkyl, carboxy-C₁-C₆-alkyl, cyano-C₁-C₆-alkyl, hydroxy-C₁-C₆-alkyl, halo-C₁-C₆-alkyl, C₁-C₆-alkoxycarbonyl-C₁-C₆-alkyl, and a group 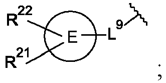
or R¹³ and R¹⁴, taken together with the nitrogen atom to which they are attached, form a 3-to 14-membered heterocycle which is optionally substituted with 1-3 substituents selected from halogen, amino, hydroxy, cyano, C₁-C₆-alkyl, halo-C₁-C₆-alkyl, C₁-C₆-alkoxy, and halo-C₁-C₆-alkoxy;
R¹⁵ is C₁-C₆-alkyl;
R¹⁶ is selected from a group 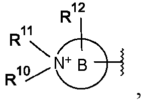 a group 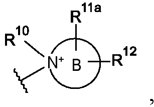 and a group 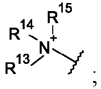
R¹⁷ is a group R¹⁶-L⁷-;
R^{23a} is hydrogen or halogen;
R^{23b} is halo-C₁-C₆-alkyl;
R^{24a} is hydrogen or halogen;
R^{24b} is selected from hydrogen, C₁-C₆-alkyl, cyano-C₁-C₆-alkyl, and halo-C₁-C₆-alkyl;
R²⁵ selected from halogen, C₁-C₆-alkoxy, cyano-C₁-C₆-alkoxy, halo-C₁-C₆-alkoxy, C₂-C₆-alkynyloxy, amino-C₂-C₆-alkynyloxy, aryloxy, (5- to 14-membered heteroaryl)oxy, and C₁-C₆-alkyl-(5- to 14-membered heteroaryl)oxy;
L¹ is selected from a covalent bond, carbonyl, and -C₁-C₆-alkyl-;
L² and L³ are independently selected from a covalent bond, carbonyl, -C₁-C₆-alkyl-C(O)-NH-, -C(O)-NH-, -NH-C(O)-, -C(O)-NH-CH(C₁-C₆-alkoxycarbonyl)-, -C₁-C₆-alkyl-CH(OH)-C₁-C₆-alkyl-C(O)-, and -C₁-C₆-alkyl-NH-;
L⁴, L⁵, L⁶, L⁷, and L⁸ are independently selected from a covalent bond, carbonyl, -C₁-C₆-alkyl-, -C₁-C₆-alkyl-C(O)-, -C₁-C₆-alkyl-NH-C(O)-, -C₁-C₆-alkyl-C(O)-NH-, -C₁-C₆-alkyl-NH-C(O)-C₁-C₆-alkyl-, -C₁-C₆-alkyl-C(O)-NH-C₁-C₆-alkyl-, -C(O)-NH-, -NH-C(O)-, -C(O)-NH-CH(C₁-C₆-alkoxycarbonyl)-, -C₁-C₆-alkyl-CH(OH)-C₁-C₆-alkyl-C(O)-, and -C₁-C₆-alkyl-NH-;
L⁹ is -C₁-C₆-alkyl- or -C(O)-C₁-C₆-alkyl-;
A, C, D, and E are each independently selected from C₆-C₁₀-aryl, C₃-C₁₀-cycloalkyl, 3- to 14-membered heterocyclyl, and 5- to 14-membered heteroaryl;
B is a 3- to 14-membered heterocycle;
F is C₆-C₁₀-aryl or 5- to 14-membered heteroaryl; and
X is selected from O, S, SO, SO₂, NH, and N(C₁-C₆-alkyl).

Also disclosed herein are compounds of formula (I) 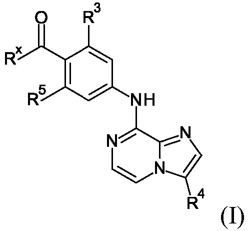 wherein:
R^{x} is a group 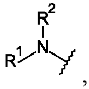 a group 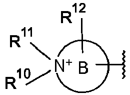 or a group 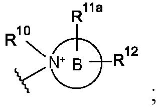
R¹ is selected from R⁶-C₁-C₆-alkyl-, R⁶-C₁-C₆-alkyl-CH(C₁-C₆-alkoxycarbonyl)-, R⁷-C₁-C₆-alkyl-X-C₁-C₆-alkyl-, a group 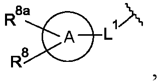 and a group 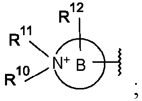 and
R² is selected from hydrogen and C₁-C₆-alkyl;
or R¹ and R², taken together with the nitrogen atom to which they are attached, form a 3- to 14-membered heterocycle that is substituted with R⁹ and R^{9a};
R³ is selected from hydrogen, halogen, and C₁-C₆-alkyl; or
R³ and R², taken together with the atoms to which they are attached, form a 3- to 14-membered heterocycle;
R⁴ is a group 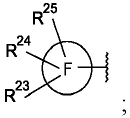
R⁵ is selected from hydrogen, C₁-C₆-alkyl, and halogen;
R⁶ is a group R¹⁶-L²-;
R⁷ is a group R¹⁶-L³-;
R⁸ is a group R¹⁶-L⁴-;
R^{8a}, R^{9a}, R^{11a}, R¹², R¹⁸, R¹⁹, R²⁰, R²¹, R²², R²³, R²⁴, and R²⁵ are independently selected from hydrogen, halogen, hydroxy, cyano, amino, carbamoyl, carboxy, C₁-C₆-alkyl, C₁-C₆-alkoxy, halo-C₁-C₆-alkyl, cyano-C₁-C₆-alkyl, carboxy-C₁-C₆-alkyl, hydroxy-C₁-C₆-alkyl, halo-C₁-C₆-alkoxy, cyano-C₁-C₆-alkoxy, C₂-C₆-alkynyloxy, amino-C₂-C₆-alkynyloxy, aryloxy, (5- to 14-membered heteroaryl)oxy, C₁-C₆-alkyl-(5- to 14-membered heteroaryl)oxy, and C₁-C₆-alkoxycarbonyl;
R⁹ is a group R¹⁶-L⁵- or a group 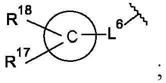
R¹⁰ is selected from C₁-C₆-alkyl, C₁-C₆-alkoxycarbonyl-C₁-C₆-alkyl, C₁-C₆-alkyl-NH-C₁-C₆-alkyl, amino-C₁-C₆-alkyl, carbamoyl, carbamoyl-C₁-C₆-alkyl, carboxy-C₁-C₆-alkyl, cyano-C₁-C₆-alkyl, halo-C₁-C₆-alkyl, hydroxy-C₁-C₆-alkyl, and a group 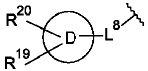 ;
R¹¹ is selected from C₁-C₆-alkyl, carbamoyl, carboxy, carboxy-C₁-C₆-alkyl, and hydroxy-C₁-C₆-alkyl;
R¹³ and R¹⁴ are independently selected from C₁-C₆-alkyl, amino-C₁-C₆-alkyl, carbamoyl-C₁-C₆-alkyl, carboxy-C₁-C₆-alkyl, cyano-C₁-C₆-alkyl, hydroxy-C₁-C₆-alkyl, halo-C₁-C₆-alkyl, C₁-C₆-alkoxycarbonyl-C₁-C₆-alkyl, and a group 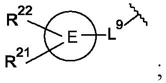
or R¹³ and R¹⁴, taken together with the nitrogen atom to which they are attached, form a 3-to 14-membered heterocycle which is optionally substituted with 1-3 substituents selected from halogen, amino, hydroxy, cyano, C₁-C₆-alkyl, halo-C₁-C₆-alkyl, C₁-C₆-alkoxy, and halo-C₁-C₆-alkoxy;
R¹⁵ is C₁-C₆-alkyl;
R¹⁶ is selected from a group 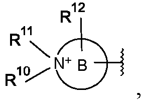 a group 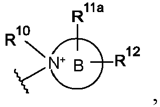 and a group 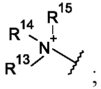
R¹⁷ is a group R¹⁶-L⁷-;
L¹ is selected from a covalent bond, carbonyl, and -C₁-C₆-alkyl-;
L² and L³ are independently selected from a covalent bond, carbonyl, -C₁-C₆-alkyl-C(O)-NH-, -C(O)-NH-, -NH-C(O)-, -C(O)-NH-CH(C₁-C₆-alkoxycarbonyl)-, -C₁-C₆-alkyl-CH(OH)-C₁-C₆-alkyl-C(O)-, and -C₁-C₆-alkyl-NH-;
L⁴, L⁵, L⁶, L⁷, and L⁸ are independently selected from a covalent bond, carbonyl, -C₁-C₆-alkyl-, -C₁-C₆-alkyl-C(O)-, -C₁-C₆-alkyl-NH-C(O)-, -C₁-C₆-alkyl-C(O)-NH-, -C₁-C₆-alkyl-NH-C(O)-C₁-C₆-alkyl-, -C₁-C₆-alkyl-C(O)-NH-C₁-C₆-alkyl-, -C(O)-NH-, -NH-C(O)-, -C(O)-NH-CH(C₁-C₆-alkoxycarbonyl)-, -C₁-C₆-alkyl-CH(OH)-C₁-C₆-alkyl-C(O)-, and -C₁-C₆-alkyl-NH-;
L⁹ is -C₁-C₆-alkyl- or -C(O)-C₁-C₆-alkyl-;
A, C, D, and E are each independently selected from C₆-C₁₀-aryl, C₃-C₁₀-cycloalkyl, 3- to 14-membered heterocyclyl, and 5- to 14-membered heteroaryl;
B is a 3- to 14-membered heterocycle;
F is C₆-C₁₀-aryl or 5- to 14-membered heteroaryl; and
X is selected from O, S, SO, SO₂, NH, and N(C₁-C₆-alkyl).

In one embodiment, the present invention provides a compound of formula (II) or (III) as defined herein, or a pharmaceutically acceptable salt thereof, wherein the compound of formula (II) or (III) is a compound of formula (II) 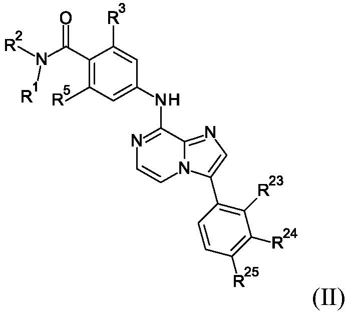 wherein R¹, R², R³, and R⁵ are as defined herein;
R²³ is hydrogen or halogen;
R²⁴ is hydrogen or halogen; and
R²⁵ selected from halogen, C₁-C₆-alkoxy, cyano-C₁-C₆-alkoxy, halo-C₁-C₆-alkoxy, C₂-C₆-alkynyloxy, amino-C₂-C₆-alkynyloxy, aryloxy, (5- to 14-membered heteroaryl)oxy, and C₁-C₆-alkyl-(5- to 14-membered heteroaryl)oxy.

In one embodiment, the present invention provides a compound of formula (II) or (III) as defined herein, or a pharmaceutically acceptable salt thereof, wherein the compound of formula (II) or (III) is a compound of formula (III) 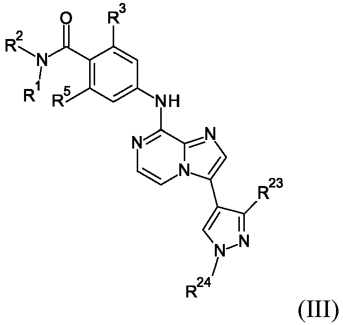 wherein R¹, R², R³, and R⁵ are as defined herein;
R²³ is halo-C₁-C₆-alkyl; and
R²⁴ is selected from hydrogen, C₁-C₆-alkyl, cyano-C₁-C₆-alkyl, and halo-C₁-C₆-alkyl.

Also disclosed herein is a compound of formula (I) as defined herein, or a pharmaceutically acceptable salt thereof, wherein:
R^{x} is a group 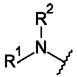 or a group

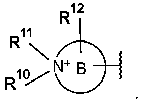

In a preferred embodiment, the present invention provides a compound of formula (I) as defined herein, or a pharmaceutically acceptable salt thereof, wherein R^{x} is a group 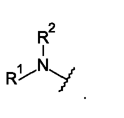 .

In one embodiment, the present invention provides a compound of formula (II) or (III) as defined herein, or a pharmaceutically acceptable salt thereof, wherein:
R¹ is selected from R⁶-C₁-C₆-alkyl-, R⁶-C₁-C₆-alkyl-CH(C₁-C₆-alkoxycarbonyl)-, R⁷-C₁-C₆-alkyl-O-C₁-C₆-alkyl-, a group 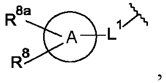 and a group 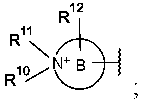
R² is selected from hydrogen and C₁-C₆-alkyl;
R⁶ is a group R¹⁶-L²-;
R⁷ is a group R¹⁶-L³-;
R⁸ is a group R¹⁶-L⁴-;
R^{8a} is hydrogen or C₁-C₆-alkoxycarbonyl;
R¹⁰ is selected from C₁-C₆-alkyl, C₁-C₆-alkoxycarbonyl-C₁-C₆-alkyl, C₁-C₆-alkyl-NH-C₁-C₆-alkyl, amino-C₁-C₆-alkyl, carbamoyl-C₁-C₆-alkyl, carboxy-C₁-C₆-alkyl, cyano-C₁-C₆-alkyl, and a group 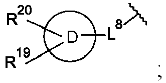
R¹¹ is selected from C₁-C₆-alkyl, carbamoyl, carboxy-C₁-C₆-alkyl, and hydroxy-C₁-C₆-alkyl;
R^{11a} is hydrogen or carboxy;
R¹² is hydrogen or hydroxy;
R¹³ is selected from C₁-C₆-alkyl, amino-C₁-C₆-alkyl, carbamoyl-C₁-C₆-alkyl, carboxy-C₁-C₆-alkyl, cyano-C₁-C₆-alkyl, hydroxy-C₁-C₆-alkyl, C₁-C₆-alkoxycarbonyl-C₁-C₆-alkyl, and a group 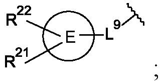 and
R¹⁴ is selected from C₁-C₆-alkyl, carboxy-C₁-C₆-alkyl, a group 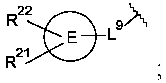 or R¹³ and R¹⁴, taken together with the nitrogen atom to which they are attached, form a 3-to 14-membered heterocycle;
R¹⁵ is C₁-C₆-alkyl;
R¹⁶ is selected from a group 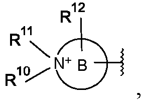 a group 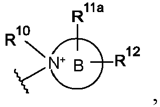 and a group 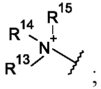
R¹⁷ is a group R¹⁶-L⁷-;
R¹⁸ is hydrogen;
R¹⁹ is selected from hydrogen, amino, and C₁-C₆-alkyl;
R²⁰ is hydrogen or C₁-C₆-alkyl;
R²¹ and R²² are both hydrogen;
L¹ is a covalent bond or -C₁-C₆-alkyl-;
L² is selected from a covalent bond, carbonyl, -C₁-C₆-alkyl-C(O)-NH-, -C(O)-NH-, - C(O)-NH-CH(C₁-C₆-alkoxycarbonyl)-, -C₁-C₆-alkyl-CH(OH)-C₁-C₆-alkyl-C(O)-, and -C₁-C₆-alkyl-NH-;
L³ is a covalent bond;
L⁴ is selected from carbonyl, -C₁-C₆-alkyl-C(O)-, -C₁-C₆-alkyl-C(O)-NH-, -C(O)-NH-, - C₁-C₆-alkyl-CH(OH)-C₁-C₆-alkyl-C(O)-, and -C₁-C₆-alkyl-NH-;
L⁷ is -C₁-C₆-alkyl-;
L⁸ is selected from a covalent bond, -C₁-C₆-alkyl-, and -C₁-C₆-alkyl-NH-C(O)-C₁-C₆-alkyl-;
L⁹ is -C₁-C₆-alkyl- or -C(O)-C₁-C₆-alkyl-;
A is C₃-C₁₀-cycloalkyl or a 3- to 14-membered heterocycle;
B is a 3- to 14-membered heterocycle;
D is selected from C₃-C₁₀-cycloalkyl, 3- to 14-membered heterocyclyl, and 5- to 14-membered heteroaryl; and
E is 3- to 14-membered heterocyclyl.

In a preferred embodiment, the present invention provides a compound of formula (II) or (III) as defined herein, or a pharmaceutically acceptable salt thereof, wherein:
R¹ is selected from R⁶-C₁-C₆-alkyl-, R⁷-C₁-C₆-alkyl-O-C₁-C₆-alkyl-, and a group 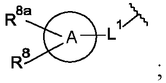
R² is selected from hydrogen and C₁-C₆-alkyl;
R⁶ is a group R¹⁶-L²-;
R⁷ is a group R¹⁶-L³-;
R⁸ is a group R¹⁶-L⁴-;
R^{8a} is hydrogen;
R¹⁰ is selected from carbamoyl-C₁-C₆-alkyl, cyano-C₁-C₆-alkyl, C₁-C₆-alkyl, carboxy-C₁-C₆-alkyl, and a group 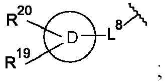
R¹¹ is C₁-C₆-alkyl or carboxy-C₁-C₆-alkyl;
R^{11a} and R¹² are both hydrogen;
R¹³ is selected from C₁-C₆-alkyl, amino-C₁-C₆-alkyl, carbamoyl-C₁-C₆-alkyl, and a group 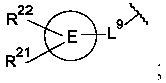
R¹⁴ is C₁-C₆-alkyl or carboxy-C₁-C₆-alkyl;
R¹⁵ is C₁-C₆-alkyl;
R¹⁶ is selected from a group 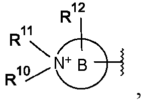 a group 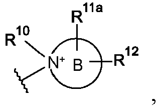 and a group 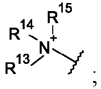
R¹⁹ and R²⁰ are both hydrogen;
R²¹ and R²² are both hydrogen;
L¹, L², and L³ are a covalent bond;
L⁴ is -C₁-C₆-alkyl-C(O)- or -C₁-C₆-alkyl-C(O)-NH-;
L⁸ and L⁹ are -C₁-C₆-alkyl-;
A is C₃-C₁₀-cycloalkyl or a 3- to 14-membered heterocycle;
B is a 3- to 14-membered heterocycle;
D is 3- to 14-membered heterocyclyl; and
E is 3- to 14-membered heterocyclyl.

In a particularly preferred embodiment, the present invention provides a compound of formula (II) or (III) as defined herein, or a pharmaceutically acceptable salt thereof, wherein:
R¹ is selected from R⁶-(CH₂)ₙ-, R⁷-(CH₂)₂-O-(CH₂)₂-, and a group 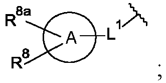
R² is selected from hydrogen and methyl;
R⁶ is a group R¹⁶-L²-;
R⁷ is a group R¹⁶-L³-;
R⁸ is a group R¹⁶-L⁴-;
R^{8a} is hydrogen;
R¹⁰ is selected from carbamoyl-CH₂-, methyl, carboxy-CH₂-, cyano-CH₂-, and a group 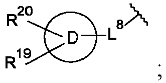
R¹¹ is methyl or carboxy-CH₂-;
R^{11a} and R¹² are both hydrogen;
R¹³ is selected from methyl, aminopropyl, carbamoyl-CH₂-, and a group 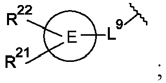
R¹⁴ is methyl or carboxymethyl;
R¹⁵ is methyl;
R¹⁶ is selected from a group 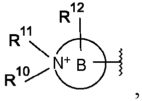 a group 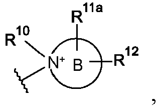 and a group 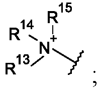
R¹⁹ and R²⁰ are both hydrogen;
R²¹ and R²² are both hydrogen;
L¹, L², and L³ are a covalent bond;
L⁴ is -CH₂-C(O)- or -CH₂-C(O)-NH-;
L⁸ and L⁹ are both -CH₂-;
A is cyclohexyl or pyrrolidinyl;
B is pyrrolidinyl, piperidyl, 3-azabicyclo[3.1.0]hexan-6-yl;
D is pyrrolidin or azetidin;
E is azetidine; and
n is 1, 2 or 5.

In one embodiment, the present invention provides a compound of formula (II) or (III) as defined herein, or a pharmaceutically acceptable salt thereof, wherein:
R¹ and R², taken together with the nitrogen atom to which they are attached, form a 3- to 14-membered heterocycle that is substituted with R⁹ and R^{9a};
R⁹ is a group R¹⁶-L⁵- or a group 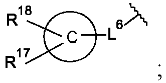
R^{9a} is selected from hydrogen, hydroxy, and C₁-C₆-alkoxycarbonyl;
R¹⁰ is selected from C₁-C₆-alkyl, C₁-C₆-alkyl-NH-C₁-C₆-alkyl, amino-C₁-C₆-alkyl, carbamoyl-C₁-C₆-alkyl, and a group 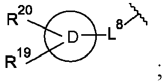
R¹¹ is C₁-C₆-alkyl;
R¹² is hydrogen or hydroxy;
R¹³ is C₁-C₆-alkyl or a group 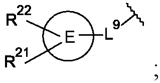 and
R¹⁴ and R¹⁵ are both C₁-C₆-alkyl;
R¹⁶ is a group 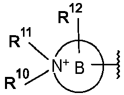 or a group 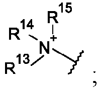
R¹⁷ is a group R¹⁶-L⁷-;
R¹⁸ is hydrogen;
R¹⁹ is hydrogen or amino;
R²⁰, R²¹, R²² are hydrogen;
B is a 3- to 14-membered heterocycle;
C is a 3- to 14-membered heterocycle;
D is C₃-C₁₀-cycloalkyl or 3- to 14-membered heterocyclyl;
E is a 3- to 14-membered heterocycle;
L⁵ is selected from a covalent bond, carbonyl, -C₁-C₆-alkyl-, -C₁-C₆-alkyl-C(O)-, and - C₁-C₆-alkyl-NH-C(O)-;
L⁶ is carbonyl;
L⁷ is -C₁-C₆-alkyl-; and
L⁸ and L⁹ are both -C₁-C₆-alkyl-.

In a preferred embodiment, the present invention provides a compound of formula (II) or (III) as defined herein, or a pharmaceutically acceptable salt thereof, wherein:
R¹ and R², taken together with the nitrogen atom to which they are attached, form a 3- to 14-membered heterocycle that is substituted with R⁹ and R^{9a};
R⁹ is a group R¹⁶-L⁵-;
R^{9a} is hydrogen;
R¹⁰ is carbamoyl-C₁-C₆-alkyl or a group 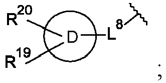 R¹¹ is C₁-C₆-alkyl;
R¹³, R¹⁴, and R¹⁵ are C₁-C₆-alkyl;
R¹⁶ is a group 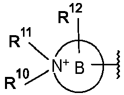 or a group 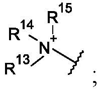
R¹², R¹⁹, and R²⁰ are hydrogen;
B is a 3- to 14-membered heterocycle;
D is a 3- to 14-membered heterocycle;
L⁵ is selected from carbonyl, -C₁-C₆-alkyl-, and -C₁-C₆-alkyl-C(O)-; and
L⁸ is -C₁-C₆-alkyl-.

In a particularly preferred embodiment, the present invention provides a compound of formula (II) or (III) as defined herein, or a pharmaceutically acceptable salt thereof, wherein:
R¹ and R², taken together with the nitrogen atom to which they are attached, form a piperazinyl ring that is substituted with R⁹ and R^{9a};
R⁹ is a group R¹⁶-L⁵-;
R^{9a} is hydrogen;
R¹⁰ is carbamoylmethyl or a group 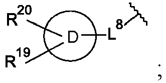
R¹¹ is methyl;
R¹³, R¹⁴, and R¹⁵ are methyl;
R¹⁶ is a group 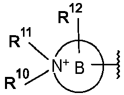 or a group 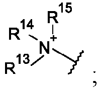
R¹², R¹⁹, and R²⁰ are hydrogen;
B is piperidyl;
D is pyrrolidinyl;
L⁵ is selected from carbonyl, -(CH₂)₂-, and -CH₂-C(O)-; and
L⁸ is -CH₂-.

In one embodiment, the present invention provides a compound of formula (II) or (III) as defined herein, or a pharmaceutically acceptable salt thereof, wherein:
R³ is selected from fluoro, chloro, methyl and ethyl;
or R³ and R², taken together with the atoms to which they are attached, form a 6-oxo-2,3-dihydropyridinyl ring.

In one embodiment, the present invention provides a compound of formula (I) as defined herein, or a pharmaceutically acceptable salt thereof, wherein:
R⁴ is a group 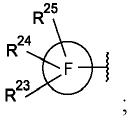 wherein
R²³ is selected from halogen, C₁-C₆-alkyl, C₁-C₆-alkoxy, halo-C₁-C₆-alkyl, cyano-C₁-C₆-alkyl, halo-C₁-C₆-alkoxy, cyano-C₁-C₆-alkoxy, C₂-C₆-alkynyloxy, amino-C₂-C₆-alkynyloxy, aryloxy, (5- to 14-membered heteroaryl)oxy, and C₁-C₆-alkyl-(5- to 14-membered heteroaryl)oxy;
R²⁴ is selected from hydrogen, halogen and halo-C₁-C₆-alkyl;
R²⁵ is hydrogen or halogen; and
F is C₆-C₁₀-aryl or 5- to 14-membered heteroaryl.

Also disclosed herein is a compound of formula (I) as defined herein, or a pharmaceutically acceptable salt thereof, wherein:
R⁴ is a group 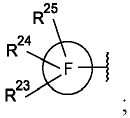 wherein
R²³ is selected from C₁-C₆-alkoxy, halo-C₁-C₆-alkyl, cyano-C₁-C₆-alkyl, halo-C₁-C₆-alkoxy, cyano-C₁-C₆-alkoxy, and amino-C₂-C₆-alkynyloxy;
R²⁴ is selected from hydrogen, halogen and halo-C₁-C₆-alkyl;
R²⁵ is hydrogen or halogen; and
F is C₆-C₁₀-aryl or 5- to 14-membered heteroaryl.

In a particularly preferred embodiment, the present invention provides a compound of formula (I) as defined herein, or a pharmaceutically acceptable salt thereof, wherein:
R⁴ is a group 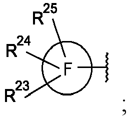 wherein
R²³ is selected from methoxy, 2,2-difluoroethyl, cyanomethyl, difluoromethoxy, cyanomethoxy, and 4-aminobut-2-ynoxy;
R²⁴ is selected from hydrogen, fluoro and CF₃;
R²⁵ is hydrogen or fluoro; and
F is phenyl or pyrazolyl.

In a preferred embodiment, the present invention provides a compound of formula (II) or (III) as defined herein, or a pharmaceutically acceptable salt thereof, wherein R⁵ is hydrogen or C₁-C₆-alkyl.

In a particularly preferred embodiment, the present invention provides a compound of formula (II) or (III) as defined herein, or a pharmaceutically acceptable salt thereof, wherein R⁵ is hydrogen or methyl.

Also disclosed is a compound of formula (I) as defined herein, or a pharmaceutically acceptable salt thereof, wherein:
R^{x} is a group 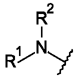 or a group 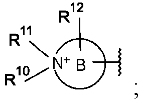
R¹ is selected from R⁶-C₁-C₆-alkyl-, R⁶-C₁-C₆-alkyl-CH(C₁-C₆-alkoxycarbonyl)-, R⁷-C₁-C₆-alkyl-O-C₁-C₆-alkyl-, a group 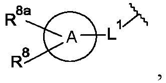 and a group 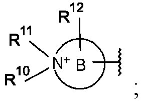 and
R² is selected from hydrogen and C₁-C₆-alkyl; or
R¹ and R², taken together with the nitrogen atom to which they are attached, form a 3- to 14-membered heterocycle that is substituted with R⁹ and R^{9a};
R³ is selected from hydrogen, halogen, and C₁-C₆-alkyl; or
R³ and R², taken together with the atoms to which they are attached, form a 3- to 14-membered heterocycle;
R⁴ is a group 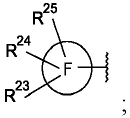
R⁵ is hydrogen or C₁-C₆-alkyl;
R⁶ is a group R¹⁶-L²-;
R⁷ is a group R¹⁶-L³-;
R⁸ is a group R¹⁶-L⁴-;
R^{8a} is hydrogen or C₁-C₆-alkoxycarbonyl;
R⁹ is a group R¹⁶-L⁵- or a group 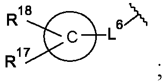
R^{9a} is selected from hydrogen, hydroxy, and C₁-C₆-alkoxycarbonyl;
R¹⁰ is selected from C₁-C₆-alkyl, C₁-C₆-alkoxycarbonyl-C₁-C₆-alkyl, C₁-C₆-alkyl-NH-C₁-C₆-alkyl, amino-C₁-C₆-alkyl, carbamoyl-C₁-C₆-alkyl, carboxy-C₁-C₆-alkyl, cyano-C₁-C₆-alkyl, and a group 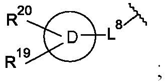
R¹¹ is selected from C₁-C₆-alkyl, carbamoyl, carboxy-C₁-C₆-alkyl, and hydroxy-C₁-C₆-alkyl;
R^{11a} is hydrogen or carboxy;
R¹² is hydrogen or hydroxy;
R¹³ is selected from C₁-C₆-alkyl, amino-C₁-C₆-alkyl, carbamoyl-C₁-C₆-alkyl, carboxy-C₁-C₆-alkyl, cyano-C₁-C₆-alkyl, hydroxy-C₁-C₆-alkyl, C₁-C₆-alkoxycarbonyl-C₁-C₆-alkyl, and a group 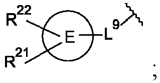 and
R¹⁴ is selected from C₁-C₆-alkyl, carboxy-C₁-C₆-alkyl, a group 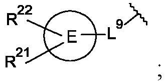 or R¹³ and R¹⁴, taken together with the nitrogen atom to which they are attached, form a 3-to 14-membered heterocycle;
R¹⁵ is C₁-C₆-alkyl;
R¹⁶ is selected from a group 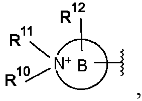 a group 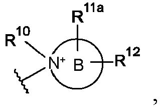 and a group 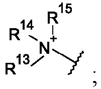
R¹⁷ is a group R¹⁶-L⁷-;
R¹⁸ is hydrogen;
R¹⁹ is selected from hydrogen, amino, and C₁-C₆-alkyl;
R²⁰ is hydrogen or C₁-C₆-alkyl;
R²¹ and R²² are both hydrogen;
R²³ is selected from halogen, C₁-C₆-alkyl, C₁-C₆-alkoxy, halo-C₁-C₆-alkyl, cyano-C₁-C₆-alkyl, halo-C₁-C₆-alkoxy, cyano-C₁-C₆-alkoxy, C₂-C₆-alkynyloxy, amino-C₂-C₆-alkynyloxy, aryloxy, (5- to 14-membered heteroaryl)oxy, and C₁-C₆-alkyl-(5- to 14-membered heteroaryl)oxy;
R²⁴ is selected from hydrogen, halogen and halo-C₁-C₆-alkyl;
R²⁵ is hydrogen or halogen;
L¹ is a covalent bond or -C₁-C₆-alkyl-;
L² is selected from a covalent bond, carbonyl, -C₁-C₆-alkyl-C(O)-NH-, -C(O)-NH-, - C(O)-NH-CH(C₁-C₆-alkoxycarbonyl)-, -C₁-C₆-alkyl-CH(OH)-C₁-C₆-alkyl-C(O)-, and -C₁-C₆-alkyl-NH-;
L³ is a covalent bond;
L⁴ is selected from carbonyl, -C₁-C₆-alkyl-C(O)-, -C₁-C₆-alkyl-C(O)-NH-, -C(O)-NH-, - C₁-C₆-alkyl-CH(OH)-C₁-C₆-alkyl-C(O)-, and -C₁-C₆-alkyl-NH-;
L⁵ is selected from a covalent bond, carbonyl, -C₁-C₆-alkyl-, -C₁-C₆-alkyl-C(O)-, and - C₁-C₆-alkyl-NH-C(O)-;
L⁶ is carbonyl;
L⁷ is -C₁-C₆-alkyl-;
L⁸ is selected from a covalent bond, -C₁-C₆-alkyl-, and -C₁-C₆-alkyl-NH-C(O)-C₁-C₆-alkyl-;
L⁹ is -C₁-C₆-alkyl- or -C(O)-C₁-C₆-alkyl-;
A is C₃-C₁₀-cycloalkyl or a 3- to 14-membered heterocycle;
B is a 3- to 14-membered heterocycle;
C is a 3- to 14-membered heterocycle;
D is selected from C₃-C₁₀-cycloalkyl, 3- to 14-membered heterocyclyl, and 5- to 14-membered heteroaryl;
E is 3- to 14-membered heterocyclyl; and
F is C₆-C₁₀-aryl or 5- to 14-membered heteroaryl.

Also disclosed herein is a compound of formula (I) as defined herein, or a pharmaceutically acceptable salt thereof, wherein:
R^{x} is a group 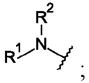
R¹ is selected from R⁶-C₁-C₆-alkyl-, R⁷-C₁-C₆-alkyl-O-C₁-C₆-alkyl-, and a group 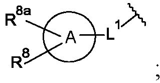 and
R² is selected from hydrogen and C₁-C₆-alkyl; or
R¹ and R², taken together with the nitrogen atom to which they are attached, form a 3- to 14-membered heterocycle that is substituted with R⁹ and R^{9a};
R³ is selected from hydrogen, halogen, and C₁-C₆-alkyl; or
R³ and R², taken together with the atoms to which they are attached, form a 3- to 14-membered heterocycle;
R⁴ is a group 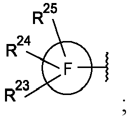
R⁵ is hydrogen or C₁-C₆-alkyl;
R⁶ is a group R¹⁶-L²-;
R⁷ is a group R¹⁶-L³-;
R⁸ is a group R¹⁶-L⁴-;
R^{8a} is hydrogen;
R⁹ is a group R¹⁶-L⁵-;
R^{9a} is hydrogen;
R¹⁰ is selected from carbamoyl-C₁-C₆-alkyl, cyano-C₁-C₆-alkyl, C₁-C₆-alkyl, carboxy-C₁-C₆-alkyl, and a group 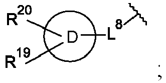
R¹¹ is C₁-C₆-alkyl or carboxy-C₁-C₆-alkyl;
R^{11a} and R¹² are both hydrogen;
R¹³ is selected from C₁-C₆-alkyl, amino-C₁-C₆-alkyl, carbamoyl-C₁-C₆-alkyl, and a group 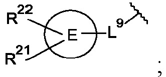
R¹⁴ is C₁-C₆-alkyl or carboxy-C₁-C₆-alkyl;
R¹⁵ is C₁-C₆-alkyl;
R¹⁶ is selected from a group 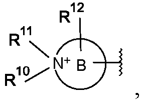 a group 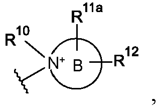 and a group 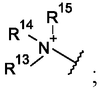
R¹⁹ and R²⁰ are both hydrogen;
R²¹ and R²² are both hydrogen;
R²³ is selected from C₁-C₆-alkoxy, halo-C₁-C₆-alkyl, cyano-C₁-C₆-alkyl, halo-C₁-C₆-alkoxy, cyano-C₁-C₆-alkoxy, and amino-C₂-C₆-alkynyloxy;
R²⁴ is selected from hydrogen, halogen and halo-C₁-C₆-alkyl;
R²⁵ is hydrogen or halogen;
L¹, L², and L³ are a covalent bond;
L⁴ is -C₁-C₆-alkyl-C(O)- or -C₁-C₆-alkyl-C(O)-NH-;
L⁵ is selected from carbonyl, -C₁-C₆-alkyl-, and -C₁-C₆-alkyl-C(O)-;
L⁸ and L⁹ are -C₁-C₆-alkyl-;
A is C₃-C₁₀-cycloalkyl or a 3- to 14-membered heterocycle;
B is a 3- to 14-membered heterocycle;
D is 3- to 14-membered heterocyclyl;
E is 3- to 14-membered heterocyclyl; and
F is C₆-C₁₀-aryl or 5- to 14-membered heteroaryl.

Also disclosed herein is a compound of formula (I) as defined herein, or a pharmaceutically acceptable salt thereof, wherein:
R^{x} is a group 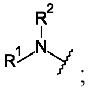
R¹ is selected from R⁶-(CH₂)ₙ-, R⁷-(CH₂)₂-O-(CH₂)₂-, and a group 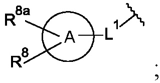 and
R² is selected from hydrogen and methyl; or
R¹ and R², taken together with the nitrogen atom to which they are attached, form a piperazinyl ring that is substituted with R⁹ and R^{9a};
R³ is selected from fluoro, chloro, methyl and ethyl; or
R³ and R², taken together with the atoms to which they are attached, form a 6-oxo-2,3-dihydropyridinyl ring;
R⁴ is a group 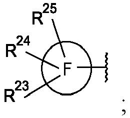
R⁵ is hydrogen or methyl;
R⁶ is a group R¹⁶-L²-;
R⁷ is a group R¹⁶-L³-;
R⁸ is a group R¹⁶-L⁴-;
R^{8a} is hydrogen;
R⁹ is a group R¹⁶-L⁵-;
R^{9a} is hydrogen;
R¹⁰ is selected from carbamoyl-CH₂-, methyl, carboxy-CH₂-, cyano-CH₂-, and a group 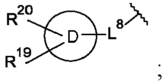
R¹¹ is methyl or carboxy-CH₂-;
R^{11a} and R¹² are both hydrogen;
R¹³ is selected from methyl, aminopropyl, carbamoyl-CH₂-, and a group 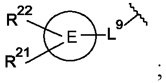
R¹⁴ is methyl or carboxymethyl;
R¹⁵ is methyl;
R¹⁶ is selected from a group 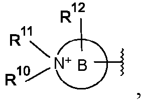 a group 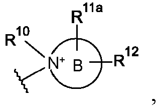 and a group 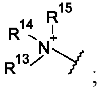
R¹⁹ and R²⁰ are both hydrogen;
R²¹ and R²² are both hydrogen;
R²³ is selected from methoxy, 2,2-difluoroethyl, cyanomethyl, difluoromethoxy, cyanomethoxy, and 4-aminobut-2-ynoxy;
R²⁴ is selected from hydrogen, fluoro and CF₃;
R²⁵ is hydrogen or fluoro;
L¹, L², and L³ are a covalent bond;
L⁴ is -CH₂-C(O)- or -CH₂-C(O)-NH-;
L⁵ is selected from carbonyl, -(CH₂)₂-, and -CH₂-C(O)-;
L⁸ and L⁹ are both -CH₂-;
A is cyclohexyl or pyrrolidinyl;
B is pyrrolidinyl, piperidyl, 3-azabicyclo[3.1.0]hexan-6-yl;
D is pyrrolidin or azetidin;
E is azetidine;
F is phenyl or pyrazolyl; and
n is 1, 2 or 5.

In one, the present invention provides a compound of formula (II) or (III) as defined herein, or a pharmaceutically acceptable salt thereof, wherein the compound of formula (II) or (III) is selected from the compounds disclosed in *Table 1.*

In one, the present invention provides a compound of formula (II) or (III) as defined herein, or a pharmaceutically acceptable salt thereof, wherein the compound of formula (II) or (III) is selected from any of the the examples disclosed herein.

In one embodiment, the present invention provides pharmaceutically acceptable salts of the compounds of formula (II) or (III) as described herein, especially pharmaceutically acceptable salts selected from hydrochlorides, fumarates, lactates (in particular derived from L-(+)-lactic acid), tartrates (in particular derived from L-(+)-tartaric acid) and trifluoroacetates. In yet a further particular embodiment, the present invention provides compounds according to formula (II) or (III) as described herein (i.e., as "free bases" or "free acids", respectively).

Also disclosed are compounds of formula (II) or (III) that are isotopically-labeled by having one or more atoms therein replaced by an atom having a different atomic mass or mass number. Examples of isotopes that can be incorporated into the compounds of formula (II) or (III) include isotopes of hydrogen, carbon, nitrogen, oxygen, phosphorous, sulfur, fluorine, chlorine, and iodine, such as, but not limited to, ²H, ³H , ¹¹C, ¹³C, ¹⁴C, ¹³ N, ¹⁵ N, ¹⁵O, ¹⁷O, ¹⁸O, ³¹P, ³²P, ³⁵S, ¹⁸F, ³⁶Cl, ¹²³I, and ¹²⁵I, respectively. Certain isotopically-labeled compounds of formula (II) or (III), for example, those incorporating a radioactive isotope, are useful in drug and/or substrate tissue distribution studies. The radioactive isotopes tritium, i.e. ³H, and carbon-14, i.e., ¹⁴C, are particularly useful for this purpose in view of their ease of incorporation and ready means of detection. For example, a compound of formula (II) or (III) can be enriched with 1, 2, 5, 10, 25, 50, 75, 90, 95, or 99 percent of a given isotope.

Substitution with heavier isotopes such as deuterium, i.e. ²H, may afford certain therapeutic advantages resulting from greater metabolic stability, for example, increased *in vivo* half-life or reduced dosage requirements.

Substitution with positron emitting isotopes, such as ¹¹C, ¹⁸F, ¹⁵O and ¹³N, can be useful in Positron Emission Topography (PET) studies for examining substrate receptor occupancy. Isotopically-labeled compounds of formula (II) or (III) can generally be prepared by conventional techniques known to those skilled in the art or by processes analogous to those described in the Examples as set out below using an appropriate isotopically-labeled reagent in place of the non-labeled reagent previously employed.

### Processes of Manufacturing

Also disclosed herein is the preparation of compounds of formula I of the present invention may be carried out in sequential or convergent synthetic routes. Syntheses of the compounds of the invention are shown in the following schemes. The skills required for carrying out the reactions and purifications of the resulting products are known to those skilled in the art. The substituents and indices used in the following description of the processes have the significance given herein before unless indicated to the contrary. In more detail, the compounds of formula I can be manufactured by the methods given below, by the methods given in the examples or by analogous methods. Appropriate reaction conditions for the individual reaction steps are known to a person skilled in the art. Also, for reaction conditions described in literature affecting the described reactions see for example: Comprehensive Organic Transformations: A Guide to Functional Group Preparations, 2nd Edition, Richard C. Larock. John Wiley & Sons, New York, NY. 1999). We find it convenient to carry out the reactions in the presence or absence of a solvent. There is no particular restriction on the nature of the solvent to be employed, provided that it has no adverse effect on the reaction or the reagents involved and that it can dissolve the reagents, at least to some extent. The described reactions can take place over a wide range of temperatures, and the precise reaction temperature is not critical to the invention. It is convenient to carry out the described reactions in a temperature range between -78 °C to reflux. The time required for the reaction may also vary widely, depending on many factors, notably the reaction temperature and the nature of the reagents. However, a period of from 0.5 h to several days will usually suffice to yield the described intermediates and compounds. The reaction sequence is not limited to the one displayed in the schemes, however, depending on the starting materials and their respective reactivity the sequence of reaction steps can be freely altered. Starting materials are either commercially available or can be prepared by methods analogous to the methods given below, by methods described in references cited in the description or in the examples, or by methods known in the art.

The following types of organic reactions - or variations thereof - were used throughout the invention. Reagent examples given below are options which work in this invention, but can also be substituted by alternatives well known in the field and described throughout the literature.

### Amide formation

There are myriad ways to form an amide bond. In this invention several ones were used which are generally described as follows.

The corresponding carboxylic acid and a suitable base were combined in a suitable solvent. Then a coupling reagent was added and the mixture stirred for a short period (usually 5 - 30 minutes). Then the corresponding amine was added and stirring at an appropriate temperature was continued until reaction monitoring revealed formation of sufficient amounts of product. Alternatively carboxylic acid, base, amine and coupling reagent can be combined from the start. Examples for a suitable base are DIPEA or Et₃N, an example for a coupling reagent is HAUT or 1-propanephosphonic anhydride and a suitable solvent would be DMF. These reaction usually proceed at room temperature, but depending on the reactivity the temperature may be eleveated.

An alternative is the formation of an activated ester form the corresponding acid using N-hydroxysuccinimide with the use of 1-ethyl-(3-(3-dimethylamino)propyl)-carbodiimide hydrochloride and subsequent reaction with the corresponding amine to form the amide.

### Urea formation

There are many ways known in the field how to form a urea which can be applied to synthesize the molecules in this invention. One option which can be used is stirring of the two respective amines in a suitable solvent, a base and a coupling reagent at an appropriate temperature. Alternatively one of the amines, the base and coupling reagent in a solvent are stirred first, then the resulting intermediate reacted with the second amine. Example for suitable solvents are DMF, NMP and DCM. DIPEA is an example for a suitable base and examples for a coupling reagent would be N,N'-carbonyldiimidazole or bis(trichloromethyl)carbonate. Depending on the reactivity, the reactions can proceed at various temperatures.

### SNAr

Electrophile and nucleophile were combined in a solvent and heated to an appropriate temperature until reaction monitoring showed formation of a suitable amount of product. In certain cases a catalytic additive can promote the reaction and heating in a sealed reaction vessel can be beneficial. An examples for a suitable solvent is acetonitrile, an example for a catalytic addivite is acetic acid. Depending on the reactivity the required temperature can vary widely, but often temperatures around the boiling point of the mixture were used. In case when working in sealed reaction vessels temperature slightly above could be used.

### Suzuki coupling

Aryl-halide and boronate were combined with a solvent. A base and catalyste were added and the reaction mixture was heated to an appropriate tempereature and stirred until reaction monitoring confirmed formation of sufficient amounts of product. It can be advantageous to perform the reaction under exclusion of oxigen and in a sealed reaction vessel. Halide and boronate can be used equimolar or either one in excess. Suitable solvents are for example dioxane or a mixture of dioxane and water. An example for a suitable base is sodium carbonate (solid or in aq solution) and suitable catalysts can be 1,1'-bis(diphenylphosphino)ferrocenedichloro palladium(II). Reaction temperatures are usually above room temperatures, preferred in the range of the boiling point of the solvent mixture - or in case a sealed reaction vessel was used slightly above.

### Reductive Amination

Reaction of an amine with a ketone or aldehyde in a suitable solvent and a suitable reducing agent, sometimes also by adding a suitable catalyst. Examples for a suitable solvent are THF, methanol or ethanol. Sometimes imine formaion and subsequent addition of the reducing agent may be needed. Examples for reducing agents are sodium triacetoxyborohydride or sodium cyanoborohydride. An example for a suitable catalyst is acetic acid. The reactions can proceed at room temperature, but may require heating.

### Alkylation

To a mixture of the corresponding nucleophile in a suitable solvent was added the alkylating agent (usually in excess) and a base. The mixture was then stirred at a suitable temperature until reaction monitoring showed formation of sufficient amounts of product. Examples for suitable solvents are dioxane and acetonitrile, the reactions usually proceed at room temperature, but depending on reactivity a higher temperature may be needed.

### Boc, tBu-Ester and Trt Deprotection

The protected starting material was combined with a suitable solvent. Then a suitable acid was added an the mixture stirred at a suitable temperature until reaction monitoring showed formation of a sufficient amount of product. Examples for suitable solvents are dioxane, methanol and dichloromethane. Examples for suitable acids are HCl or TFA. The concentrations of those acids can vary from very dilute to very concentrated. In case of HCl the preferred concentration range is between 0.5 - 4 M, for TFA in the range of 10 - 90%. Depending on the susceptibility of other functional groups like oxetanes or azetidines TFA may be more appropriate than HCl. Those reactions usually work well at room temperature, but depending on the stability or when tBu-Ester were present elevated temperatures may be needed.

An alternative method is to combine the protected starting materials in a suitable solvent with lithium chloride and a base and heat until reaction monitoring shows formation of a sufficient amount of product. Examples for suitable solvents are DMF and a suitable base triethylamine.

### Phtalimid deprotection

Phtalimid protection groups can be removed by stirring the compound with hydrazine hydrate in a suitable solvent like ethanol at slightly elevated temperature.

### Cbz Deprotection

Cbz deprotection can be done under standard hydrogenation conditions. A possible way is to disolve the starting material in a suitable solvent like methanol or ethanol, add a suitable catalyst like 10% Pd/C and hydrogenate under hydrogen atmosphere with or without pressure at a suitable temperature which is usually room temperature.

### Ester Hydrolysis

In the synthesis sequence the deprotection of an acid moiety in form of a hydrolysis from the corresponding ester may be needed. One way is a saponification procedure, where the corresponding ester is stirred with an appropriate strong base like aqueous sodium hydroxide in suitable solvents like THF, methanol, water or mixtures thereof. Those reaction usually proceed at room temperature, but may require heating.

### Synthesis pathways to prepare the compounds of the invention

To build the molecules of this invention the following bonds were formed in various order using standard methods known to people skilled in the art.

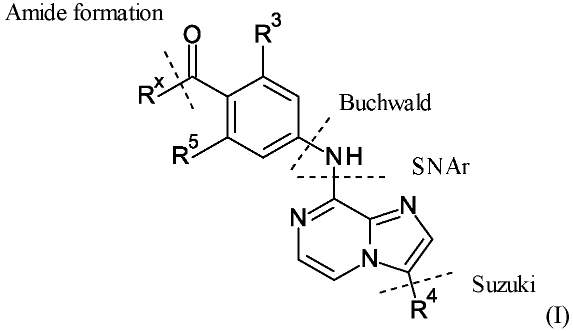

To form the bond between the central imidazo[1,2-a]pyrazine core and R⁴ a Suzuki approach can be used. Instead of R⁴, also a precursor (R^{z}) which can then be turned into R⁴ can be employed. 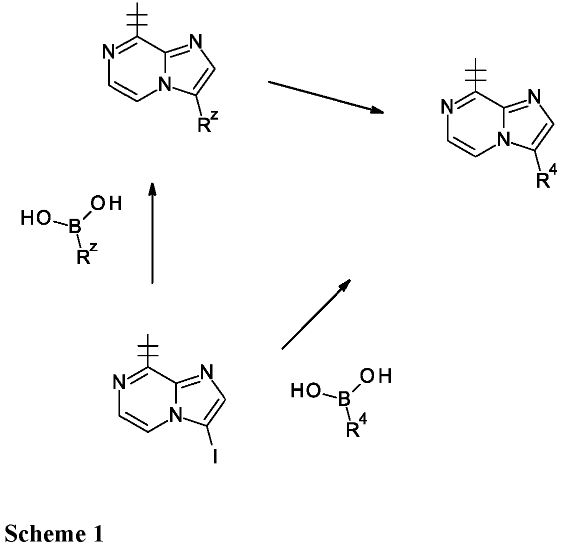 The formation can be generalized as in the *Scheme 1,* while the Suzuki could be turned around with the halogen on R⁴ / R^{z} while the boronate is present on the imidazo[1,2-a] pyrazine moiety. Alternatively to boronic acids also boronic acid esters, trifluoroborates or other suitable coupling partner can be used, other halogens than iodine can be used. The above described general procedure for a Suzuki coupling can be used. See Example 1 step 2 for a representative procedure.

*Intermediates Type 1*, which can be a boronic acid ester, but also a boronic acid, trifluoroborates or other suitable coupling partner, can be commercially available, but depending on the substitution they may have to be prepared.

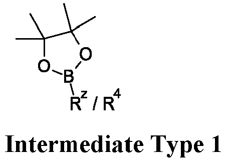

A representative sequence is depicted in *Scheme* 2 where first the substitution can be modified by alkylation of the phenol moiety (see general procedure for alkylation) using an alkyl bromide in acetonitrile in presence of a base. This is then followed by a borylation using bis(pinacolato)diboron in the presence of a base like potassium acetate and a palladium catalyst in a suitable solvent like dioxane at elevated temperature. See steps 1 and 2 of example 2 for an implementation.

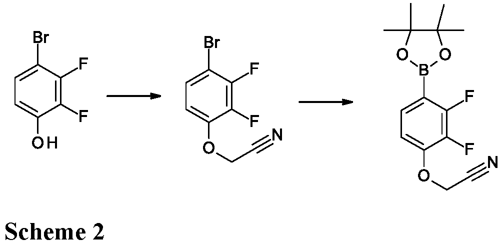

Modification of the substitution pattern can also be achieved via SNAr using an appropriate activated halo-heteroaromatic system (e.g. 2-chloropyrimidine) where this is stirred with the corresponding phenol in presence of a base (for example potassium carbonate) in a suitable solvent (for example DMSO) at elevated temperature. Example 3, step 5 is a representative example for such a transformation.

Another way to modify the substitution pattern is by alkylating the aromatic ring directly, for example when it consist of a pyrazole as shown in *Scheme 3.*

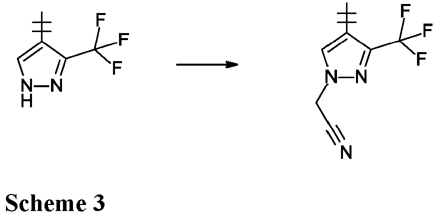

A representative procedure can be found in the preparation of Example 4.

It may be required to synthesize a suitable alkylating agent. One possibility is by building up a mesylate, like shown in *Scheme 4.* This is done by forming a Boc protected amine from a commercial diol-monomesylate by reacting it with ammonium hydroxide and Boc₂O. The remaining alcohol can then be transformed to the corresponding mesylate, for example by reacting it with mesyl chloride in the presence of a suitable base like triethylamine in a suitable solvent like DCM. This mesylate can then be used to alkylate the aromatic ring. Representative procedures can be found in the preparation of Example 5.

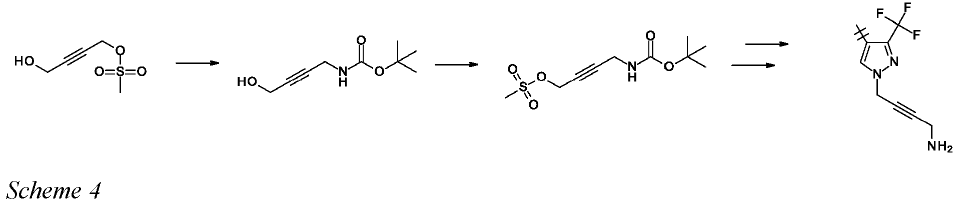

Another method to modify the substitution pattern is by doing an Ullman type coupling, where the phenol is reacted with an appropriate halo-aromatic system in the presence of a base, an appropriate ligand and a copper(I) source at elevated temperatures. An example for a base would be cesium carbonate, for the ligand / catalyst pair would be 2-oxocyclohexanecarboxylate / copper(I)bromide in a suitable solvent like acetonitrile. Also this halo-aromatic system may have to be built up first and later also further modified. The sequence can look like depicted in *Scheme* 5, where benzyl azide is reacted with an alkine in the presence of copper(I)iodide and NBS to form a protected triazole. This is then coupled with the respective phenole like described above. After attaching the imidazopyrazine moitey and subseqent modifications on this part the protecting group is hydrogenated under standard conditions followed by another alkylation of the triazole.

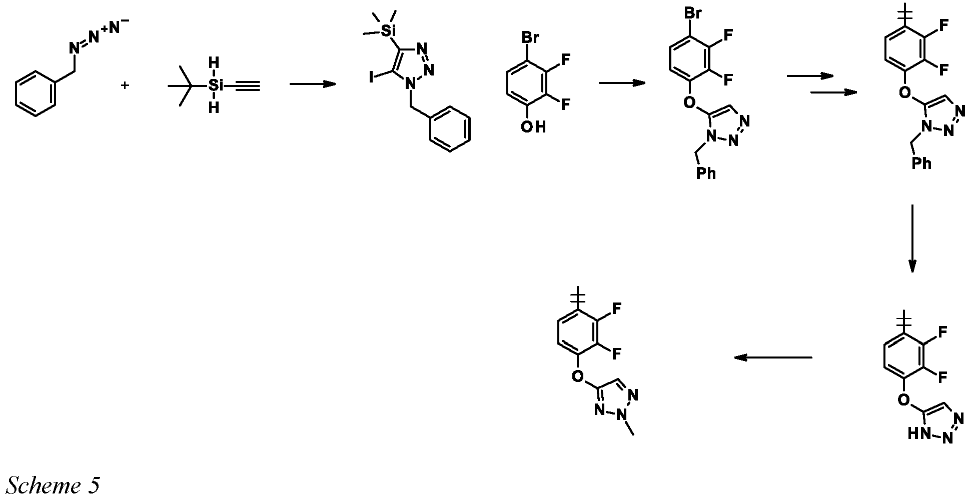

A representative procedure can be found in the preparation of example 6.

Alternatively, modifications like the alkylation can done after the boronate was coupled to the imidazo[1,2-a] pyrazine moiety. At times it may be important to use protective groups on certain functionalities. An example for this can be found in the synthesis of example 7.

In case where the aromatic ring is a pyrazole, it may be important to protect the pyrazole nitrogen for the synthesis to be successful. An example for such a sequence is shown in *Scheme* 6 where the aromatic ring is iodinated using a suitable reagent like n-iodosuccinimid. The pyrazol is then protected with a trityl-group by first deprotonating the pyrazole using a suitable base like sodium hydride and then a reagent like trityl-chloride. At last the haloaromatic system is then transformed into the corresponding boronic acid by halogen-lithium exchange using a suitable reagent like butyl lithium followed by the addition of a suitable reagent like boron isopropoxide.

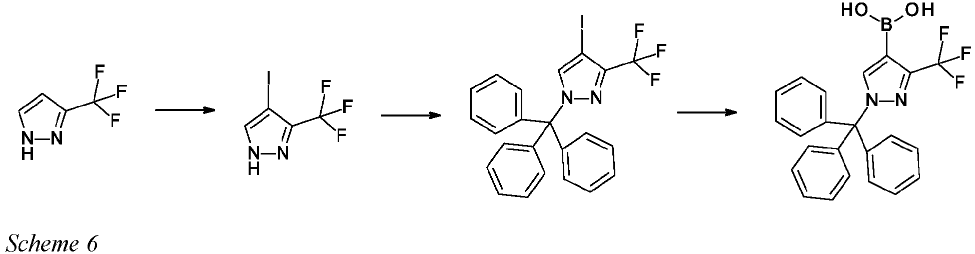

The trityl group can be cleaved at a later timepoint using a strong acid like HCl in dioxane. Representative procedures can be found in the preparation of Example 8.

The bond between the nitrogen and the imidazo[1,2-a] pyrazine moiety can be formed using an SNAr reaction starting from the 8-halo-imidazo[1,2-a] pyrazine and the corresponding aniline using the general procedure for a SNAr described above (*Scheme 7*).

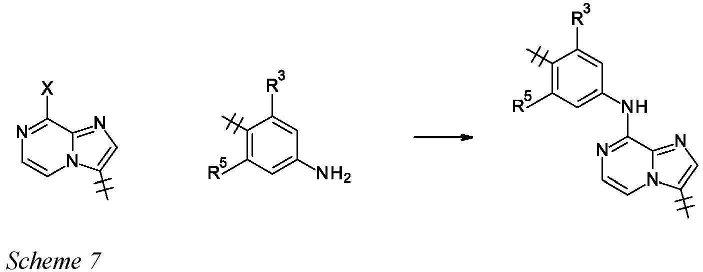

The bond between the 8-amino-imidazo[1,2-a] pyrazine and the neighbouring benzene ring can be established via Buchwald procedure (*Scheme 8*) where the 8-amino-imidazo[1,2-a] pyrazine is coupled with a halobenzene in the presence of a suitable base and a suitable palladium catalyst in a suitable solvent, usually under inert atmosphere at elevated temperature. An example for this is potassium carbonate or sodium tert.-butoxide as base, tert.-butanol or THF as solvent and Brettphos Pd G3 or 1,1'-bis(diphenylphosphino)ferrocene / tris(dibenzylideneacetone)dipalladium (0) as catalyst at elevated temperature. Depending on the substrates another catalyst or catalyst / ligand combination may be suitable.

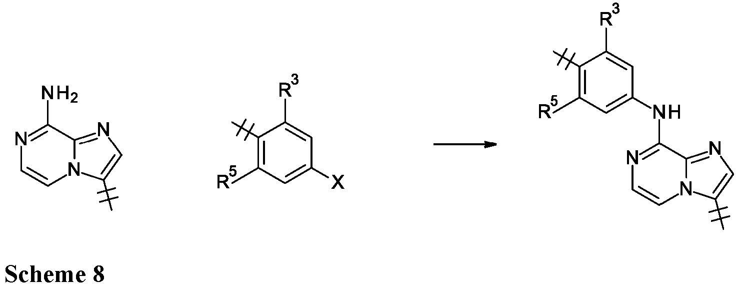

The amide bond present in formula (I) can be constructed with a variety of amide couplings known in the field (*Scheme 9*)*.* But also amide bonds present in R^{x} can be constructed in this manner and are described in the general procedure above. It may be possible to have an amine in hand which already consist of the full R^{x} part.

However, it may also exist as a synthetic precursor (R^{y'}) from which R^{x} has to be built up previously or after the fact, where the use of protecting groups like Boc, tBu or Cbz can be used to mask a reactive functionality in R^{x} like an amine or an acid. Subsequent deprotection as described in the general procedures followed by more amide couplings / urea formations / reductive aminations / alkylations / reductions can be employed (which may again require subsequent deprotections). A representative sequence can be found in example 9 steps 3 - 7 where R^{y} was coupled and the resulting R^{y'} was then further modified to form R^{x} (Boc-deprotection → amide coupling → Boc deprotection → reductive amination → alkylation to form the quarternary ammonium centre).

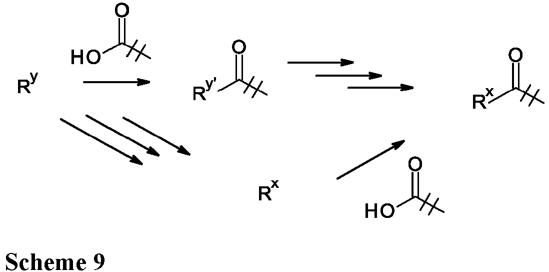

Depending on the structure of R^{x} a different sequence of synthesis steps may be required to get there from R^{y}. An alternative sequence from the one above can be found in example 10 (Boc deprotection → reductive amination → alkylation to form the quarternary ammonium centre → Boc deprotection).

An alcohol in Rx can be obtained from reducing a corresponding ester to this alcohol by using a reducing agent (for example sodium borohydride), if needed in presence of an agent like lithium chloride in suitable solvents like THF, ethanol or mixtures thereof.

There are cases where parts of R^{y} also haves to be built. In cases where R¹⁴ = aminoalkyl compounds can be built like shown in *Scheme 10.* R^{y} is attached by amide coupling and boc deprotection (a). In parallel an aldehyde is formed from an alcolohl which contains a phtalimid protected amine (b) with the use of tetrabutylammonium chloride, N-chloro succinimid and 2,2,6,6-tetramethyl-1-oxido-piperidine in a buffered solution (NaHCO₃ / K₂CO₃). The two products are then coupled by reductive amination and further modified by reductive amination and alkylation and subsequent deprotections. Specific procedures can be found in example 11. 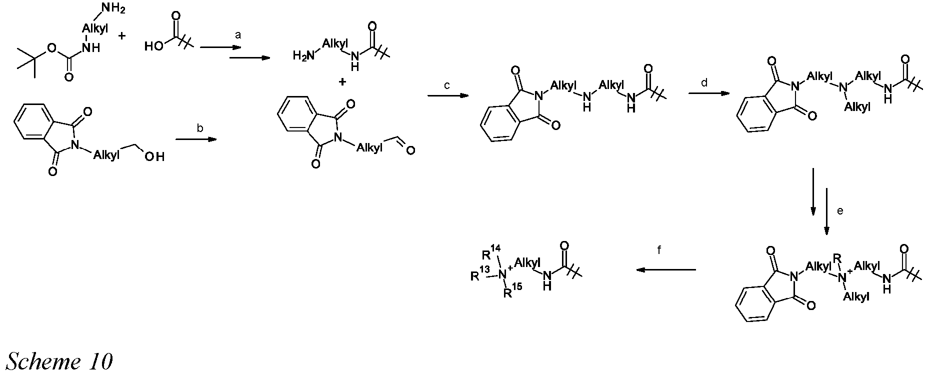 Another example of building up R^{y} before doing the amide coupling and subsequent further derivatization is shown in *Scheme 11.* Building R^{y}: Amide coupling of a Cbz bearing amine with a N-Boc bearing carboxylic acid, followed by Cbz-Deprotection. Amide coupling to attach R^{y} to the rest of the molecule (as shown in *Scheme 9*), then further derivatization to get to the desired final compound.

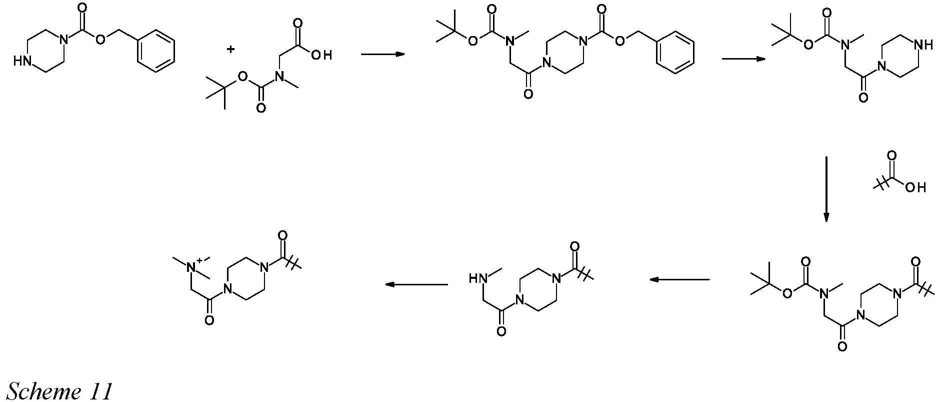

Representative procedures can be found in the preparation of Example 12.

The methods needed to create R^{x} are covered in the procedures given above and usable sequences are well represented in the specific examples.

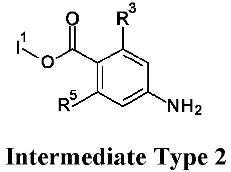

Intermediates of Type 2, where I¹ can be alkyl or H, may be commercially available, or may have to be synthesized. A possible use of those intermediates is in an SNAr reaction to attach them to the imidazopyrazine part (see above) There are multiple ways how to prepare them. In case where R3 = Alkyl and R5 = H, a possible sequence is depicted in *Scheme* 12 where a Suzuki type reaction using an alkenyl boronate followed by a subsequent hydrogenation. A representative is example 13 steps 1 and 2. Alternatively a direct approach can be used by using the corresponding alkylborate solution under Suzuki conditions with a base like potassium carbonate in DMF. A respresentative procedure can be found in step 1 of example 4.

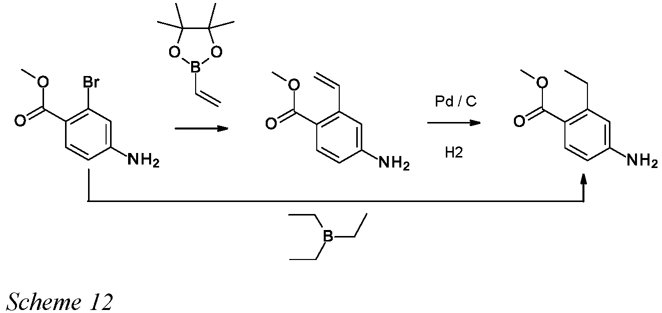

Alternatively they can be available from the corresponding nitro compounds as shown in *Scheme* 13 by reduction via Bechamp conditions. A representative procedure can be found in step 1 of example 14. A combination with the modifications in *Scheme* 12 is possible.

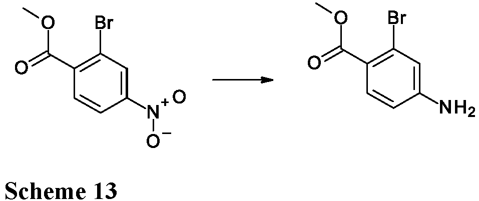

*Intermediates of Type 3* may be commercially available, or may have to be synthesized. They are typically used like *Intermediates of Type 2.*

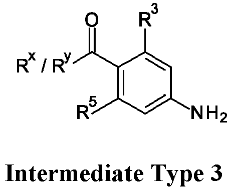

A synthetic sequence for the preparation of those compounds is to start from an intermediate accessible via the synthesis of an *Intermediate Type 2* (*Scheme 11 and 12*)*.* Amide formation as described above installs R^{X} or a precursor thereof (R^{y}). In the latter case the precursor may have to be further modified before finally the nitro group is reduced to the corresponding aniline, for example by hydrogenation in the presence of Pd/C in a suitable solvent like methanol. A representative procedure can be found in steps 4 - 7 of example 2 or steps 4-7 of example 15.

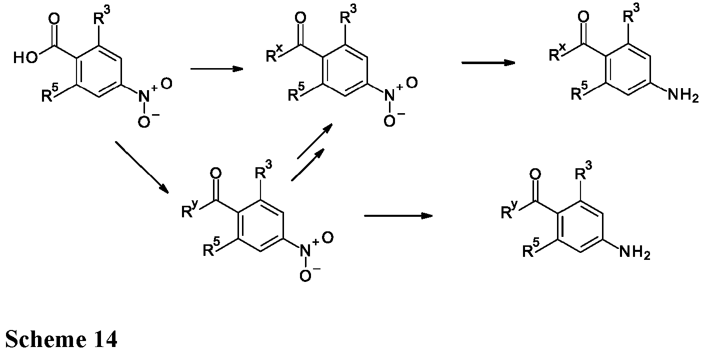

Intermedates of Type 4 are used to attach the moiety to the imidazo[1,2-a] pyrazine part, for example by Buchwald coupling like described above

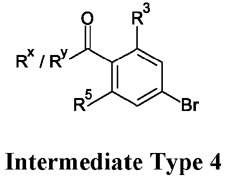

They can be synthesized by similar methods as described for *Intermediates of Type 2 and 3.* Another way to synthesize them is shown in *scheme 15* where an alcohol is transformed to the corresponding mesylate by reacting it with methanesulfonyl chloride in the presence of a base like triethylamine in a suitable solvent like DCM. In a next step the resulting material is used to alkylate an amide. For this the amide is first deprotonated with a suitable base like sodium hydride, followed by addition of the mesylate and heating. Representative procedures can be found in example 16.

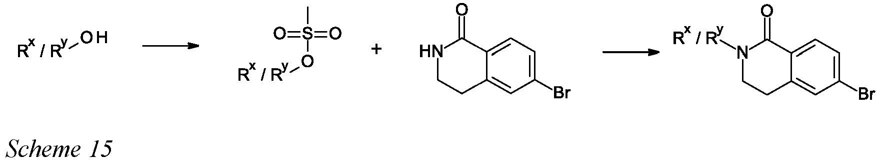

A similar approach can be used to form alkyl-amine bonds in R^{x}. Formation of the corresponding mesylate from an alcohol in Ry followed by treatment with a corresponding amine in presence of a suitable base which usually does not have to be as strong as above (for example DIPEA). A representative example can be found in the preparation of Example 17.

Another way to synthesize *Intermediates of Type* 4 is depicted in *Scheme 16.* Protection of an acid as a tert.-butyl ester, for example by stirring it with N,N-dimethylformamide di-tert-butyl acetal in a suitable solvent like dry toluene at elevated temperatures is followed by Buchwald coupling to the imidazopyrazine moiety followed by acidic deprotection (for example by stirring in HCl in dioxane at elevated temperatures) to form the acid again.

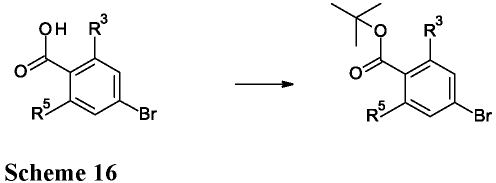

*Intermediates of Type 5* can be used as reagent as shown in *Scheme 8.*

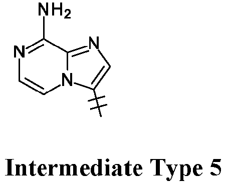

They can be formed by reacting the corresponding chloride with ammonia as shown in *Scheme* 17. A possibility is to use aqueous ammonia and perform the reaction in a suitable solvent like isopropanol in a sealed vessel at elevated temperature. A representative procedure can be found in the preparation of Example 18.

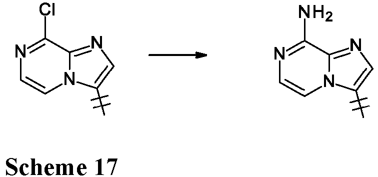

For practical reasons it may be advantageous to form the quarternary ammonium center late in the synthesis, which is in this invention usually done by an alkylation as described in the general procedures.

As described there are always a multitude of ways how to synthesize the molecules of this invention. *Scheme 18* gives a non-comprehensive overview of the multitudes of possible synthesis pathway. In this particular scheme each arrow can consist of multiple steps described above for the corresponding transformation.

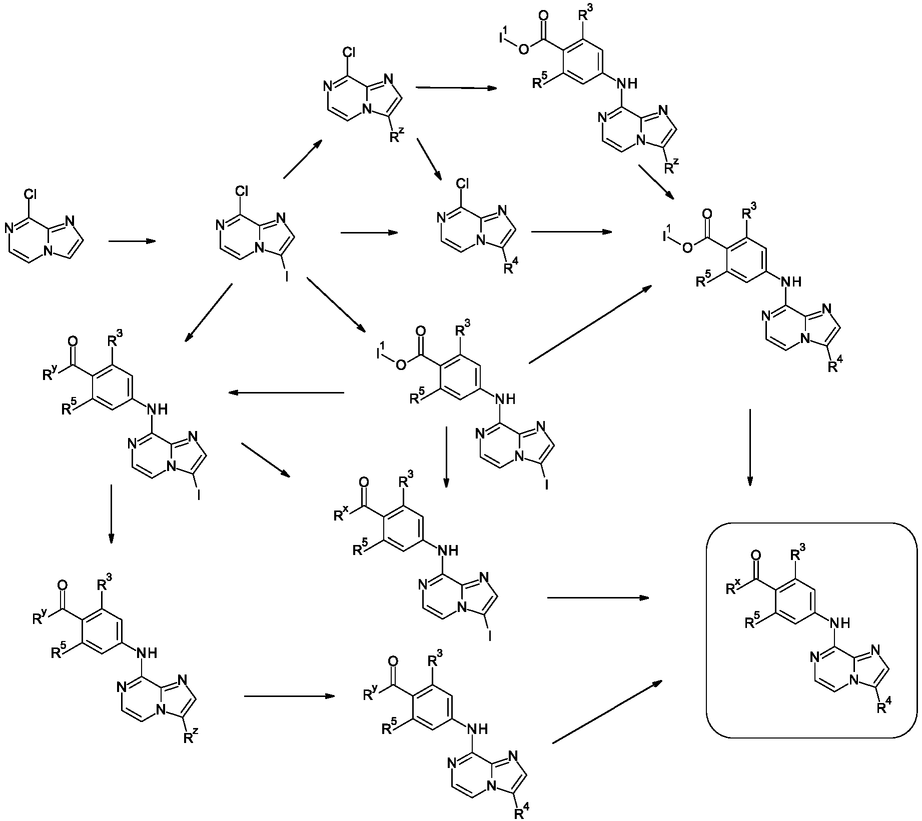

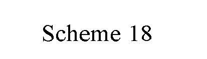

Also disclosed herein is a process of manufacturing the compounds of formula (I) described herein, comprising:
(i) Suzuki coupling of a compound (A), wherein X is a halogen or a triflate, preferably wherein X is iodo, and wherein the double cross indicates the point of attachment of (A) to the remainder of formula (I), or a synthetic precursor thereof, 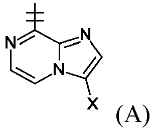 with a boronic acid (R=H) or a boronic acid ester (R=alkyl or both R, taken together with the atoms to which they are attached, form a heterocycle) (B), wherein R⁴ is are as defined herein, 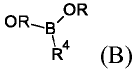 to form said compound of formula (I) or a synthetic precursor thereof; or
(ii) Buchwald coupling of a compound (C), wherein the double cross indicates the point of attachment of (C) to R⁴ as defined herein, or a synthetic precursor thereof, 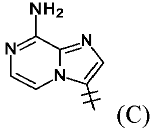 with an aryl halide (D), wherein R³ and R⁵ are as defined herein, X is a halogen and the double cross indicates the point of attachment of (D) to the remainder of formula (I), or a synthetic precursor thereof, 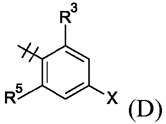 to form said compound of formula (I), or a synthetic precursor thereof; or
(iii) nucleophilic aromatic substitution (SNAr) comprising reacting a compound (C1), wherein X is a halogen and the double cross indicates the point of attachment of (C1) to R⁴ as defined herein, or a synthetic precursor thereof, 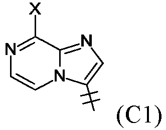 with an aniline derivative (D1), wherein wherein R³ and R⁵ are as defined herein and the double cross indicates the point of attachment of (D1) to the remainder of formula (I), or a synthetic precursor thereof, 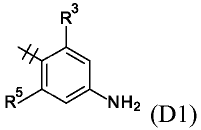 to form said compound of formula (I), or a synthetic precursor thereof; or
(iv) amide coupling comprising reacting a carboxylic acid (E), wherein the double cross indicates the point of attachment of (E) to the remainder of formula (I), or a synthetic precursor thereof, 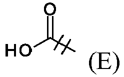 with a primary or secondary amine R^{y}, to form a compound of formula (G), wherein R^{y'} equals R^{x} as defined herein, or is a synthetic precursor thereof

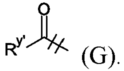

Also disclosed is a process of the present invention comprising all of the steps (i), (ii) and (iv) as outlined above.

Also disclosed is a process of the present invention comprising all of the steps (i), (iii) and (iv) as outlined above.

The sequence of steps (i), (ii), (iii) and (iv) as outlined above can be varied freely.

Also disclosed is a compound of formula (II) or (III) as described herein, or a pharmaceutically acceptable salt thereof, when manufactured according to the processes disclosed herein.

### Using the Compounds of the Invention

As illustrated in the experimental section, the compounds of formula (II) or (III) and their pharmaceutically acceptable salts possess valuable pharmacological properties for the treatment or prevention of infections and resulting diseases, particularly bacteremia, pneumonia, meningitis, urinary tract infection, and wound infection, caused by pathogens, particularly by bacteria, more particularly by *Acinetobacter* species, most particularly by *Acinetobacter baumannii.*

The compounds of formula (II) or (III) and their pharmaceutically acceptable salts exhibit activity as antibiotics, particularly as antibiotics against *Acinetobacter* species, more particularly as antibiotics against *Acinetobacter baumannii,* most particularly as pathogen-specific antibiotics against *Acinetobacter baumannii.*

The compounds of formula (II) and (III) and their pharmaceutically acceptable salts can be used as antibiotics, i.e. as antibacterial pharmaceutical ingredients suitable in the treatment and prevention of bacterial infections, particularly in the treatment and prevention of bacterial infections caused by *Acinetobacter* species, more particularly in the treatment and prevention of bacterial infections caused by *Acinetobacter baumannii.*

The compounds of the present invention can be used, either alone or in combination with other drugs, for the treatment or prevention of infections and resulting diseases, particularly bacteremia, pneumonia, meningitis, urinary tract infection, and wound infection, caused by pathogens, particularly by bacteria, more particularly caused by *Acinetobacter* species, most particularly by *Acinetobacter baumannii.*

In one aspect, the present invention provides compounds of formula (II) or (III) or their pharmaceutically acceptable salts as described herein for use as therapeutically active substances.

In a further aspect, the present invention provides a compound of formula (II) or (III) as described herein, or a pharmaceutically acceptable salt thereof, for use as antibiotic.

Also disclosed is a compound of formula (II) or (III) as described herein, or a pharmaceutically acceptable salt thereof, for use in the treatment or prevention of nosocomial infections and resulting diseases.

The nosocomial infections and resulting diseases are selected from bacteremia, pneumonia, meningitis, urinary tract infection and wound infection, or a combination thereof.

In a further aspect, the present invention provides a compound of formula (II) or (III) as described herein, or a pharmaceutically acceptable salt thereof, for use in the treatment or prevention of infections and resulting diseases caused by Gram-negative bacteria.

Such infections and resulting diseases caused by Gram-negative bacteria are selected from bacteremia, pneumonia, meningitis, urinary tract infection and wound infection, or a combination thereof.

In a further aspect, the present invention provides a compound of formula (II) or (III) as described herein, or a pharmaceutically acceptable salt thereof, for use in the treatment or prevention of infections and resulting diseases caused by ***E**nterococcus faecium, **S**taphylococcus aureus, **K**lebsiella pneumoniae, **A**cinetobacter baumannii, **P**seudomonas aeruginosa, **E**nterobacter species* or *E. coli,* or a combination therof.

Also disclosed herein is a method for the treatment or prevention of infections and resulting diseases caused by ***E**nterococcus faecium, **S**taphylococcus aureus, **K**lebsiella pneumoniae, **A**cinetobacter baumannii, **P**seudomonas aeruginosa, **E**nterobacter species* or *E. coli,* or a combination therof, which method comprises administering a compound of formula (I) as described herein, or a pharmaceutically acceptable salt thereof, to a mammal.

In a further aspect, the present invention provides the use of a compound of formula (II) or (III) as described herein, or a pharmaceutically acceptable salt thereof, as an antibiotic.

In a further aspect, the present invention provides the use of a compound of formula (II) or (III) as described herein, or a pharmaceutically acceptable salt thereof, for the treatment or prevention of infections and resulting diseases caused by ***E**nterococcus faecium, **S**taphylococcus aureus, **K**lebsiella pneumoniae, **A**cinetobacter baumannii, **P**seudomonas aeruginosa, **E**nterobacter species* or *E. coli,* or a combination therof.

In a further aspect, the present invention provides the use of a compound of formula (II) or (III) as described herein, or a pharmaceutically acceptable salt thereof, for the preparation of medicaments useful for the treatment or prevention of infections and resulting diseases caused by *Enterococcus faecium, **S**taphylococcus aureus, **K**lebsiella pneumoniae, **A**cinetobacter baumannii, **P**seudomonas aeruginosa, **E**nterobacter species* or *E. coli,* or a combination therof.

Such infections and resulting diseases caused by ***E**nterococcus faecium, **S**taphylococcus aureus, **K**lebsiella pneumoniae, **A**cinetobacter baumannii, **P**seudomonas aeruginosa, **E**nterobacter species* or *E. coli,* or a combination therof, are selected from bacteremia, pneumonia, meningitis, urinary tract infection and wound infection, or a combination thereof.

In a further aspect, the present invention provides compounds of formula (II) or (III) or their pharmaceutically acceptable salts as defined above for use in the treatment or prevention of infections and resulting diseases, particularly bacteremia, pneumonia, meningitis, urinary tract infection, and wound infection, caused by pathogens, particularly by bacteria, more particularly caused by *Acinetobacter* species, most particularly by *Acinetobacter baumannii.*

Also disclosed herein is a method for the treatment or prevention of infections and resulting diseases, particularly bacteremia, pneumonia, meningitis, urinary tract infection, and wound infection, caused by pathogens, particularly by bacteria, more particularly caused by *Acinetobacter* species, most particularly by *Acinetobacter baumannii,* which method comprises administering a compound of formula (I) or a pharmaceutically acceptable salt thereof as defined above to a mammal.

In a further aspect, the present invention provides the use of compounds of formula (II) or (III) or their pharmaceutically acceptable salts as defined above for the treatment or prevention of infections and resulting diseases, particularly bacteremia, pneumonia, meningitis, urinary tract infection, and wound infection, caused by pathogens, particularly by bacteria, more particularly caused by *Acinetobacter* species, most particularly by *Acinetobacter baumannii.*

In a further aspect, the present invention provides the use of compounds of formula (II) or (III) or their pharmaceutically acceptable salts as defined above for the preparation of medicaments for the treatment or prevention of infections and resulting diseases, particularly bacteremia, pneumonia, meningitis, urinary tract infection, and wound infection, caused by pathogens, particularly by bacteria, more particularly caused by *Acinetobacter* species, most particularly by *Acinetobacter baumannii.* Such medicaments comprise compounds of formula (II) or (III) or their pharmaceutically acceptable salts as defined above.

### Pharmaceutical Compositions and Administration

In one aspect, the present invention provides pharmaceutical compositions comprising compounds of formula (II) or (III) or their pharmaceutically acceptable salts as defined above and one or more pharmaceutically acceptable excipients. Exemplary pharmaceutical compositions are described in Examples 156 to 159.

In a further aspect, the present invention relates to pharmaceutical compositions comprising compounds of formula (II) or (III) or their pharmaceutically acceptable salts as defined above and one or more pharmaceutically acceptable excipients for the treatment or prevention of infections and resulting diseases, particularly bacteremia, pneumonia, meningitis, urinary tract infection, and wound infection, caused by pathogens, particularly by bacteria, more particularly caused by *Acinetobacter* species, most particularly by *Acinetobacter baumannii.*

The compounds of formula (II) or (III) and their pharmaceutically acceptable salts can be used as medicaments (e.g. in the form of pharmaceutical preparations). The pharmaceutical preparations can be administered internally, such as orally (e.g. in the form of tablets, coated tablets, dragées, hard and soft gelatin capsules, solutions, emulsions or suspensions), nasally (e.g. in the form of nasal sprays) or rectally (e.g. in the form of suppositories). However, the administration can also be effected parentally, such as intramuscularly or intravenously (e.g. in the form of injection solutions or infusion solutions).

The compounds of formula (II) or (III) and their pharmaceutically acceptable salts can be processed with pharmaceutically inert, inorganic or organic excipients for the production of tablets, coated tablets, dragées and hard gelatin capsules. Lactose, corn starch or derivatives thereof, talc, stearic acid or its salts etc. can be used, for example, as such excipients for tablets, dragées and hard gelatin capsules.

Suitable excipients for soft gelatin capsules are, for example, vegetable oils, waxes, fats, semisolid substances and liquid polyols, etc.

Suitable excipients for the production of solutions and syrups are, for example, water, polyols, saccharose, invert sugar, glucose, etc.

Suitable excipients for injection solutions are, for example, water, alcohols, polyols, glycerol, vegetable oils, etc.

Suitable excipients for suppositories are, for example, natural or hardened oils, waxes, fats, semisolid or liquid polyols, etc.

Moreover, the pharmaceutical preparations can contain preservatives, solubilizers, viscosity-increasing substances, stabilizers, wetting agents, emulsifiers, sweeteners, colorants, flavorants, salts for varying the osmotic pressure, buffers, masking agents or antioxidants. They can also contain still other therapeutically valuable substances.

The dosage can vary in wide limits and will, of course, be fitted to the individual requirements in each particular case. In general, in the case of oral administration a daily dosage of about 0.1 mg to 20 mg per kg body weight, preferably about 0.5 mg to 4 mg per kg body weight (e.g. about 300 mg per person), divided into preferably 1-3 individual doses, which can consist, for example, of the same amounts, should be appropriate. It will, however, be clear that the upper limit given herein can be exceeded when this is shown to be indicated.

### Co-Administration of Compounds of Formula (I) and Other Agents

Also disclosed herein are compounds of formula (I) or salts thereof or a compound disclosed herein or a pharmaceutically acceptable salt thereof may be employed alone or in combination with other agents for treatment. For example, the second agent of the pharmaceutical combination formulation or dosing regimen may have complementary activities to the compound of formula (I) such that they do not adversely affect each other. The compounds may be administered together in a unitary pharmaceutical composition or separately. In one embodiment a compound or a pharmaceutically acceptable salt can be co-administered with an antibiotic, in particular with an antibiotic for the treatment or prevention of infections and resulting diseases caused by ***E**nterococcus faecium, **S**taphylococcus aureus, **K**lebsiella pneumoniae, **A**cinetobacter baumannii, **P**seudomonas aeruginosa, **E**nterobacter species* or *E. coli,* or a combination therof.

The term "co-administering" refers to either simultaneous administration, or any manner of separate sequential administration, of a compound of formula (I) or a salt thereof or a compound disclosed herein or a pharmaceutically acceptable salt thereof and a further active pharmaceutical ingredient or ingredients, including antibiotic agents. If the administration is not simultaneous, the compounds are administered in a close time proximity to each other. Furthermore, it does not matter if the compounds are administered in the same dosage form, e.g. one compound may be administered intravenously and another compound may be administered orally.

Typically, any agent that has antimicrobial activity may be co-administered. Particular examples of such agents are Carbapenems (meropenem), Fluoroquinolone (Ciprofloxacin), Aminoglycoside (amikacin), Tetracyclines (tigecycline), Colistin, Sulbactam, Sulbactam+Durlobactam, Cefiderocol (Fetroja), macrocyclic peptides as exemplified e.g. in WO 2017072062 A1, WO 2019185572 A1 and WO 2019206853 A1, and Macrolides (erythromycin).

Also disclosed is a pharmaceutical composition described herein, further comprising an additional therapeutic agent.

Also disclosed is a pharmaceutical combination comprising a compound of formula (I) described herein and an additional therapeutic agent.

Such additional therapeutic agent is an antibiotic agent.

Also disclosed is an pharmaceutical combination where the additional therapeutic agent is an antibiotic agent that is useful for the treatment or prevention of infections and resulting diseases caused by ***E**nterococcus faecium, **S**taphylococcus aureus, **K**lebsiella pneumoniae, **A**cinetobacter baumannii, **P**seudomonas aeruginosa, **E**nterobacter species* or *E. coli,* or a combination therof.

Such additional therapeutic agent is an antibiotic agent selected from Carbapenems (meropenem), Fluoroquinolone (Ciprofloxacin), Aminoglycoside (amikacin), Tetracyclines (tigecycline), Colistin, Sulbactam, Sulbactam+Durlobactam, Cefiderocol (Fetroja), macrocyclic peptides as exemplified in WO 2017072062 A1, WO 2019185572 A1 and WO 2019206853 A1, and Macrolides (erythromycin).

### Examples

The invention will be more fully understood by reference to the following examples.

In case the preparative examples are obtained as a mixture of enantiomers, the pure enantiomers can be separated by methods described herein or by methods known to the man skilled in the art, such as e.g., chiral chromatography (e.g., chiral SFC) or crystallization.

All reaction examples and intermediates were prepared under an argon atmosphere if not specified otherwise.

The following abbreviations are used throughout the present patent specification:
ACN = acetonitrile
AcOEt = ethyl acetate
AcOH = acetic acid
aq = aqueous
Brettphos Pd G3 = [(2-Di-cyclohexylphosphino-3,6-dimethoxy-2' ,4' ,6' - triisopropyl-1,1' - biphenyl)-2-(2' -amino-1,1' -biphenyl)]palladium(II) methanesulfonate methanesulfonate (CAS Number 1470372-59-8)
CAN = cerammonium nitrate
DCM = dichloromethane
DIEA = diisopropylethylamine
DIPEA = diisopropylethylamine
DMA = dimethylacetamide
DMF = dimethylformamide
ESI = electron spray ionization
EtOAc = ethyl acetate
FA = formic acid
h = hour(s)
HATU = 2-(3H-[1,2,3]triazolo[4,5-b]pyridin-3-yl)-1,1,3,3-tetramethylisouronium hexafluorophosphate(V)
Hunig's base = diisopropylethylamine
HV = high vacuum
LiOH = lithium hydroxide
MeCN = acetonitrile
MeOH = methanol
MgSO₄ = magnesium sulfate
MS = mass spectrum
NaBH₃CN = sodium cyanoborohydride
NaBH(OAc)₃ = sodium triacetoxyborohydride
NaOH = sodium hydroxide
Na₂SO₄= sodium sulfate
Na₂CO₃ = sodium carbonate
NIS = N-iodosuccinimide
NMP = N-methylpyrrolidinone
Pd(dppf)Cl2 = [1,1' -Bis(diphenylphosphino)ferrocene]dichloropalladium(II)
Pd-118 = [1,1' -Bis(di-tert-butylphosphino)ferrocene]dichloropalladium(II) [95408-45-0]
PdCl₂(dtbpf) [1,1' -Bis(di-tert-butylphosphino)ferrocene]dichloropalladium(II) [95408-45-0]
PE = petrol ether
prep HPLC = preparative HPLC, an additive in the eluent can be given in a bracket
RT = room temperature
TBTU = 2-(1H-Benzotriazole-1-yl)-1,1,3,3-tetramethylaminium tetrafluoroborate
TEA = triethylaminea
THF = tetrahydrofurane
TFA = trifluoroacetic acid

### Example 1

### 2-(2-(4-((3-(4-(difluoromethoxy)phenyl)imidazo[1,2-apyrazin-8-yl)amino)-2-methylbenzamido)ethoxy)-N,N,N-trimethylethanaminium iodide

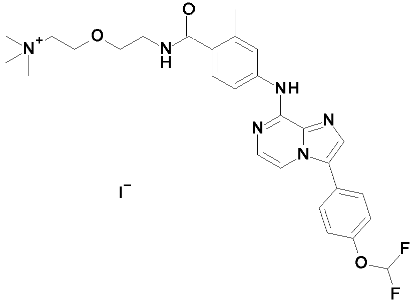

### Step 1) Intermediate 1

### 8-chloro-3-iodoimidazo[1,2-a]pyrazine

8-chloroimidazo[1,2-a]pyrazine (5.2 g, 33.9 mmol, Eq: 1) and NIS (8 g, 35.6 mmol, Eq: 1.05) were combined with DMF (34 ml). The reaction mixture was stirred at RT for 48 h. Water was added to the suspension and the solid was filtered, washed with water and MeOH and dried under HV to afford the title compound (7.17 g, 25.6 mmol, 75.8% yield) as an off-white solid. MS (ESI, m/z): 280.0 [M+H]⁺.

### Step 2)

### 8-chloro-3-(4-(difluoromethoxy)phenyl)imidazo[1,2-a]pyrazine

**8-chloro-3-iodoimidazo[1,2-a]pyrazine** (2 g, 7.16 mmol, Eq: 1) and 2-(4-(difluoromethoxy)phenyl)-4,4,5,5-tetramethyl-1,3,2-dioxaborolane (2.32 g, 8.59 mmol, Eq: 1.2) were combined with dioxane (12 ml) and water (6 ml). Na₂CO₃ (1.52 g, 14.3 mmol, Eq: 2) and 1,1'-bis(diphenylphosphino)ferrocenedichloro palladium(II) dichloromethane complex (262 mg, 358 µmol, Eq: 0.05) were added. The reaction mixture was heated to 80 °C and stirred for 20 h. The reaction mixture was poured into 50 mL of H₂O and extracted with EtOAc (3 x 50 mL). The organic layers were dried over MgSO₄ and concentrated in vacuo.

The crude material was purified by flash chromatography (silica gel, 80 g, 0% to 40% EtOAc in heptane) to afford the title compound (480 mg, 1.62 mmol, 22.7% yield) as a white solid. MS (ESI, m/z): 296.1 [M+H]⁺.

### Step 3) Intermediate 2

### 4-((3-(4-(difluoromethoxy)phenyl)imidazo[1,2-a]pyrazin-8-yl)amino)-2-methylbenzoic acid

8-chloro-3-(4-(difluoromethoxy)phenyl)imidazo[1,2-a]pyrazine (1.14 g, 3.86 mmol, Eq: 1), 4-amino-2-methylbenzoic acid (699 mg, 4.63 mmol, Eq: 1.2) were combined with acetonitrile (15 ml). The reaction mixture was heated to 80 °C and stirred for 2 days. The reaction mixture was filtered through sintered glass and washed with acetonitrile and methanol. The solid was dried under high vacuum for 30 min. Recrystallization from methanol (4 ml) and acetonitrile (8 ml) afforded the title compound (650 mg, 1.58 mmol, 41.1% yield) as a white solid. MS (ESI, m/z): 411.3 [M+H]⁺.

### Step 4)

### Tert-butyl (2-(2-(4-((3-(4-(difluoromethoxy)phenyl)imidazo[1,2-a]pyrazin-8-yl)amino)-2-methylbenzamido)ethoxy)ethyl)carbamate

4-((3-(4-(difluoromethoxy)phenyl)imidazo[1,2-a]pyrazin-8-yl)amino)-2-methylbenzoic acid (650 mg, 1.58 mmol, Eq: 1) and tert-butyl (2-(2-aminoethoxy)ethyl)carbamate (485 mg, 2.38 mmol, Eq: 1.50) were combined with DMF (7 ml). HATU (1.2 g, 3.17 mmol, Eq: 2.0) and DIPEA (614 mg, 830 µl, 4.75 mmol, Eq: 3.0) were added and the reaction mixture was stirred at RT for 24 h. Tert-butyl (2-(2-aminoethoxy)ethyl)carbamate (162 mg, 792 µmol, Eq: 0.5) and DIPEA (205 mg, 277 µl, 1.58 mmol, Eq: 1) were added again and the reaction mixture was stirred for 48 h. The product was concentrated in high vacuum and the crude material was purified by flash chromatography (silica gel, 40 g, 0% to 10% MeOH in DCM). The product was purified further by another flash chromatography (silica gel, 40 g, 50% to 100% EtOAc in heptane) to afford the title compound (810 mg, 1.35 mmol, 85.7% yield) as a light brown solid. MS (ESI, m/z): 597.4 [M+H]⁺.

### Step 5)

### N-(2-(2-aminoethoxy)ethyl)-4-((3-(4-(difluoromethoxy)phenyl)imidazo[1,2-a]pyrazin-8-yl)amino)-2-methylbenzamide hydrochloride

Tert-butyl (2-(2-(4-((3-(4-(difluoromethoxy)phenyl)imidazo[1,2-a]pyrazin-8-yl)amino)-2-methylbenzamido)ethoxy)ethyl)carbamate (650 mg, 1.09 mmol, Eq: 1) and HCl 3M in MeOH (8.4 g, 7 ml, 21 mmol, Eq: 19.3) were combined and stirred at room temperature for 7 h. The product was concentrated under HV to afford the title compound (643 mg, 1.2 mmol, 111% yield) as a yellow solid. MS (ESI, m/z): 497.34 [M+H]⁺.

### Step 6)

### 2-(2-(4-((3-(4-(Difluoromethoxy)phenyl)imidazo[1,2-a]pyrazin-8-yl)amino)-2-methylbenzamido)ethoxy)-N,N,N-trimethylethanaminium iodide

N-(2-(2-aminoethoxy)ethyl)-4-((3-(4-(difluoromethoxy)phenyl)imidazo[1,2-a]pyrazin-8-yl)amino)-2-methylbenzamide hydrochloride (10 mg, 18.8 µmol, Eq: 1) was dissolved in 1,4-dioxane (1 ml) and iodomethane (22.8 mg, 10 µl, 161 µmol, Eq: 8.56) was added. The reaction mixture was stirred at room temperature. No reaction observed so iodomethane (45.6 mg, 20 µl, 321 µmol, Eq: 17.1) and DIPEA (14.8 mg, 20 µl, 115 µmol, Eq: 6.1) were added and the reaction mixture was stirred for 20 h at room temperature. The crude material was purified by preparative HPLC to afford the title compound (6.6 mg, 9.9 µmol, 52.8% yield) as a colorless oil. MS (ESI, m/z): 539.35 [M]⁺.

### Example 2

### 2-[1-[4-[[3-[4-(Cyanomethoxy)-2,3-difluoro-phenyl]imidazo[1,2-a]pyrazin-8-yl]amino]-2-methyl-benzoyl]-4-piperidyllethyl-trimethyl-ammonium formate

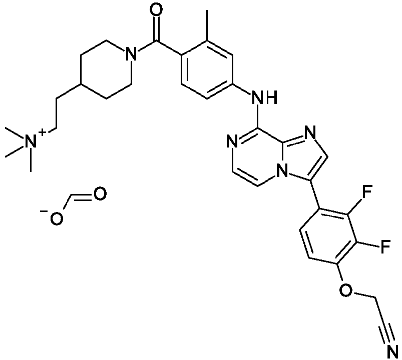

### Step 1)

### 2-(4-Bromo-2,3-difluoro-phenoxy)acetonitrile

To a mixture of 2-bromoacetonitrile (6.89 g, 57.42 mmol, 1.2 eq) in acetonitrile (250 mL) was added potassium carbonate (9.92 g, 71.77 mmol, 1.5 eq) and 4-bromo-2,3-difluoro-phenol (10 g, 47.85 mmol, 1 eq) at 25 °C. The mixture was stirred at 50 °C for 16 h. The reaction mixture was filtered and concentrated. The residue was purified via chromatography (PE/EA=20/1-5/1) to afford the title compound (11.3 g, 45.56 mmol, 95.22% yield) as a white solid. 1H NMR (400 MHz, CHLOROFORM-d) Shift 7.26-7.37 (m, 1H), 6.84 (ddd, J=2.14, 7.34, 9.23 Hz, 1H), 4.86 (s, 2H).

### Step 2) Intermediate 15

### 2-[2,3-Difluoro-4-(4,4,5,5-tetramethyl-1,3,2-dioxaborolan-2-yl)phenoxy]acetonitrile

To a mixture of 2-(4-bromo-2,3-difluoro-phenoxy)acetonitrile (11.3 g, 45.56 mmol, 1 eq),bis(pinacolato)diboron (13.88 g, 54.67 mmol, 1.2 eq) and potassium acetate (13.41 g, 136.68 mmol, 3 eq) in 1,4-dioxane (250 mL) was added [1,1'-bis(diphenylphosphino)ferrocene]dichloropalladium(II) (1.67 g, 2.28 mmol, 0.05 eq) at 25°C under N₂. The mixture was stirred at 70°C for 4 h. The reaction mixture was cooled down and filtered. The filtrate was concentrated. The residue was purified via chromatography (PE/EA=100/1-10/1) and trituration (PE, 50 mL) to afford the title compound (7 g, 23.72 mmol, 52.07% yield) as a yellow solid. MS (ESI, m/z): 296.0 [M+H]⁺.

### Step 3) Intermediate 11

### 2-[4-(8-Chloroimidazo[1,2-a]pyrazin-3-yl)-2,3-difluoro-phenoxy]acetonitrile

A mixture of 8-chloro-3-iodo-imidazo[1,2-a]pyrazine (Intermediate 1, 3.03 g, 10.84 mmol, 0.8 eq), 2-[2,3-difluoro-4-(4,4,5,5-tetramethyl-1,3,2-dioxaborolan-2-yl)phenoxy]acetonitrile (4 g, 13.56 mmol, 1 eq), sodium carbonate (4.31 g, 40.67 mmol, 3 eq) and [1,1'-bis(diphenylphosphino)ferrocene]dichloropalladium(II) (0.99 g, 1.36 mmol, 0.1 eq) in 1,4-dioxane (60 mL) and water (15 mL) was stirred at 60°C for 16 h. The reaction mixture was filtered and concentrated. The residue was extracted by AcOEt (50 mL x 3), the combined organic layer was washed with brine (50 mL x 2), dried over Na₂SO₄, filtered and concentrated. The residue was purified via chromatography (PE/EA=1/1, Rf=0.3) to afford the title compound (2.8 g, 8.73 mmol, 46.17% yield) as a yellow solid. MS (ESI, m/z): 321.0 [M+H]⁺.

### Step 4)

### tert-Butyl N-[2-[1-(2-methyl-4-nitro-benzoyl)-4-piperidyl]ethyl]carbamate

To a mixture of 2-methyl-4-nitro-benzoic acid (793 mg, 4.38 mmol, 1 eq), 4-(2-Boc-aminoethyl)piperidine (1000 mg, 4.38 mmol, 1 eq) and triethylamine (1.22 mL, 8.76 mmol, 2 eq) in DMF (20 mL) was added dropwise 1-propanephosphonic anhydride (2787 mg, 4.38 mmol, 1 eq) at 25°C under N₂. The mixture was stirred at 25°C for 3 h. The reaction mixture was poured into water (100 mL) and it was extracted by AcOEt (100 mL x 3). The combined organic layer was washed with brine (100 mL), dried over Na₂SO₄, then filtered and concentrated. The residue was purified via chemical flash (PE/EA=2/1, Rf=0.25) to afford the title compound (1700 mg, 4.34 mmol, 99.16% yield) as a colorless oil. MS (ESI, m/z): 292.1 [M+H-Boc]⁺.

### Step 5)

### [4-(2-aminoethyl)-1-piperidyl]-(2-methyl-4-nitro-phenyl)methanone

To a mixture of tert-butyl N-[2-[1-(2-methyl-4-nitro-benzoyl)-4-piperidyl]ethyl]carbamate (1.4 g, 3.58 mmol, 1 eq) in dichloromethane (30 mL) was added dropwise trifluoroacetic acid (3 mL, 38.94 mmol, 10.89 eq) at 25°C. The mixture was stirred at 25°C for 16 h. The reaction mixture was concentrated to afford the title compound (1 g, 3.43 mmol, 95.97% yield) as a colorless oil. MS (ESI, m/z): 292.1 [M+H]⁺.

### Step 6)

### Trimethyl-[2-[1-(2-methyl-4-nitro-benzoyl)-4-piperidyl]ethyl]ammonium formate

To a mixture of [4-(2-aminoethyl)-1-piperidyl]-(2-methyl-4-nitro-phenyl)methanone (1 g, 3.43 mmol, 1 eq) and potassium carbonate (3.79 g, 27.46 mmol, 8 eq) in acetonitrile (20 mL) was added iodomethane (2.92 g, 20.59 mmol, 6 eq) at 25 °C. The mixture was stirred at 25°C for 2 h. The reaction mixture was purified directly via prep-HPLC (FA) to afford the title compound (1 g, 2.99 mmol, 87.12% yield) as a yellow gum. MS (ESI, m/z): 334.1 [M]⁺.

### Step 7)

### 2-[1-(4-Amino-2-methyl-benzoyl)-4-piperidyl]ethyl-trimethyl-ammonium formate/acetate

A mixture of trimethyl-[2-[1-(2-methyl-4-nitro-benzoyl)-4-piperidyl]ethyl]ammonium formate (400 mg, 1.2 mmol, 1 eq) and 10% Pd/C : water 1:1 (0.04 mL, 0.380 mmol, 0.31 eq) in methanol (8 mL) was stirred under a hydrogen balloon at 25°C for 16 h. Two drops of acetic acid was added and the mixture was stirred at 25°C for another 16 h with a hydrogen balloon. The reaction mixture was filtered and concentrated to afford the title compound (360 mg, 1.18 mmol, 98.86% yield) as a yellow oil. MS (ESI, m/z): 305.1 [M]⁺.

### Step 8)

### 2-[1-[4-[[3-[4-(Cyanomethoxy)-2,3-difluoro-phenyl]imidazo[1,2-a]pyrazin-8-yl]amino]-2-methyl-benzoyl]-4-piperidyl]ethyl-trimethyl-ammonium formate

A mixture of 2-[4-(8-chloroimidazo[1,2-a]pyrazin-3-yl)-2,3-difluoro-phenoxy]acetonitrile (210.66 mg, 0.660 mmol, 1 eq) and 2-[1-(4-amino-2-methyl-benzoyl)-4-piperidyl]ethyl-trimethyl-ammonium formate/acetate (200 mg, 0.660 mmol, 1 eq) in acetonitrile (2 mL) and acetic acid (0.2 mL) was stirred at 60°C for 16 h. The reaction mixture was purified directly via prep-HPLC (FA) and then lyophilized to afford the title compound (31 mg, 0.050 mmol, 7.58% yield) as a yellow solid. MS (ESI, m/z): 294.7 [M+H]²⁺.

### Example 3

### N-[3-[[4-[[3-(2,3-difluoro-4-pyrimidin-2-yloxy-phenyl)imidazo[1,2-a]pyrazin-8-yl]amino]-2-ethyl-benzoyl]amino]propyl]-1-methyl-1-(pyrrolidin-3-ylmethyl)piperidin-1-ium-4-carboxamide formate

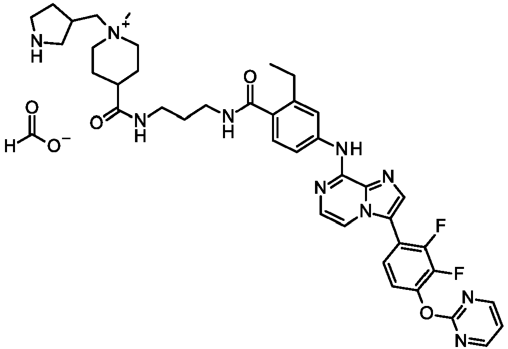

### Step 1) Intermediate 46

### 2-Ethyl-4-[(3-iodoimidazo[1,2-a]pyrazin-8-yl)amino]benzoic acid

To a solution of 8-chloro-3-iodoimidazo[1,2-a]pyrazine (Intermediate 1, 1g, 3.58 mmol, Eq: 1) in EtOH ( 10 mL) was added 4-amino-2-ethylbenzoic acid (709 mg, 4.29 mmol, Eq: 1.2), the reaction was stirred for 15 hours at 100 °C. The reaction mixture was cooled to room temperature and filtered. The filter cake was dried in vacuum to give the title compound (1.2 g, 2.94 mmol, 82.2 % yield). MS (ESI, m/z): 409.0 [M+H]⁺.

### Step 2)

### tert-Butyl N-[3-[[2-ethyl-4-[(3-iodoimidazo[1,2-a]pyrazin-8-yl)amino]benzoyl]amino]propyl]carbamate

To a solution of 2-ethyl-4-((3-iodoimidazo[1,2-a]pyrazin-8-yl)amino)benzoic acid ( 300 mg, 735 µmol, Eq: 1) in DMF ( 6 mL) was added *tert-*butyl (3-aminopropyl)carbamate (166 mg, 955 µmol, Eq: 1.3),2-(3H-[1,2,3]triazolo[4,5-b]pyridin-3-yl)-1,1,3,3-tetramethylisouronium hexafluorophosphate(V) (559 mg, 1.47 mmol, Eq: 2) and triethylamine (223 mg, 307 µL, 2.2 mmol, Eq: 3). The reaction was stirred for 20 minutes at room temperature. The reaction mixture was poured into water and filtered. The filter cake was dried in vacuum to give the title compound (400 mg, 709 µmol, 96.4 % yield). MS (ESI, m/z): 565.0 [M+H]⁺.

### Step 3)

### N-(3-Aminopropyl)-2-ethyl-4-[(3-iodoimidazo[1,2-a]pyrazin-8-yl)amino]benzamide 2,2,2-trifluoroacetate

To dissolve *tert*-butyl (3-(2-ethyl-4-((3-iodoimidazo[1,2-a]pyrazin-8-yl)amino)benzamido)propyl)carbamate (400 mg, 709 µmol, Eq: 1) in the mixture solvent of DCM (4 mL) and TFA (4 mL), the reaction was stirred for 10 minutes at room temperature. The reaction mixture was concentrated in vacuum to give the title compound (350 mg, 605 µmol, 85.4 % yield). MS (ESI, m/z): 465.0 [M+H]⁺.

### Step 4)

### tert-Butyl 4-[3-[[2-chloro-4-[(3-iodoimidazo[1,2-a]pyrazin-8-yl)amino]benzoyl]amino]propylcarbamoyl]piperidine-1-carboxylate

To a solution of N-(3-aminopropyl)-2-chloro-4-((3-iodoimidazo[1,2-a]pyrazin-8-yl)amino)benzamide 2,2,2-trifluoroacetate (350 mg, 599 µmol, Eq: 1)in DMF ( 5 mL) was added 1-(*tert*-butoxycarbonyl)piperidine-4-carboxylic acid (165 mg, 718 µmol, Eq: 1.2), triethylamine (182 mg, 250 µL, 1.8 mmol, Eq: 3) and 2-(3H-[1,2,3]triazolo[4,5-b]pyridin-3-yl)-1,1,3,3-tetramethylisouronium hexafluorophosphate(V) (341 mg, 898 µmol, Eq: 1.5). The reaction was stirred for 20 minutes at room temperature. The reaction mixture was poured onto water and filtered. The filter cake was dried in vacuum to give the title compound (380 mg, 557 µmol, 93.1 % yield). MS (ESI, m/z): 682.0 [M+H]⁺.

### Step 5)

### 2-(4-bromo-2,3-difluoro-phenoxy)pyrimidine

To a solution of 4-bromo-2,3-difluorophenol (2 g, 9.57 mmol, Eq: 1) in DMSO (20 mL) was added 2-chloropyrimidine (2.19 g, 19.1 mmol, Eq: 2) and potassium carbonate (3.97 g, 28.7 mmol, Eq: 3), the reaction was stirred for 5 hours at 110 °C. The reaction mixture was cooled to room temperature and poured into water. The mixture was filtered and the filter cake was dried in vacuum to give the title compound ( 2.1 g, 7.32 mmol, 76.4 % yield). MS (ESI, m/z): 287.0 [M+H]⁺.

### Step 6)

### 2-[2,3-Difluoro-4-(4,4,5,5-tetramethyl-1,3,2-dioxaborolan-2-yl)phenoxy]pyrimidine

To a solution of 2-(4-bromo-2,3-difluorophenoxy)pyrimidine (1 g, 3.48 mmol, Eq: 1) in dioxane (10 mL) was added 4,4,4',4',5,5,5',5'-octamethyl-2,2'-bi(1,3,2-dioxaborolane) (973 mg, 3.83 mmol, Eq: 1.1), potassium acetate (684 mg, 6.97 mmol, Eq: 2) and and 1,1'-bis(di-*tert-*butylphosphino)ferrocene palladium dichloride (284 mg, 348 µmol, Eq: 0.1), the reaction was stirred for 12 hours at 80 °C under atmosphere of argon. The reaction mixture was cooled to room temperature and filtered. The filtrate was concentrated in vacuum. The residue was purified by column chromatgraphy to give the crude title compound (1.3 g, 3.31 mmol, 94.9 % yield). MS (ESI, m/z): 335.0 [M+H]⁺.

### Step 7)

### tert-Butyl 4-[3-[[4-[[3-(2,3-difluoro-4-pyrimidin-2-yloxy-phenyl)imidazo[1,2-a]pyrazin-8-yl]amino]-2-ethyl-benzoyl]amino]propylcarbamoyl]piperidine-1-carboxylate

To a solution of *tert*-butyl 4-((3-(2-ethyl-4-((3-iodoimidazo[1,2-a]pyrazin-8-yl)amino)benzamido)propyl)carbamoyl)piperidine-1-carboxylate (420 mg, 622 µmol, Eq: 1) in the mixture solvent of dioxane (5 mL) and water (1 mL) was added 2-(2,3-difluoro-4-(4,4,5,5-tetramethyl-1,3,2-dioxaborolan-2-yl)phenoxy)pyrimidine (249 mg, 746 µmol, Eq: 1.2), sodium carbonate (198 mg, 1.87 mmol, Eq: 3) and 1,1'-bis(di-*tert*-butylphosphino)ferrocene palladium dichloride (40.5 mg, 62.2 µmol, Eq: 0.1), the reaction was stirred for 30 minutes at 100 °C under microwave irriation and atmosphere of nitrogen. The reaction mixture was filtered and the filtrate was concentrated in vacuum. The residue was purified by column chromatgraphy to give the title compound (300 mg, 397 µmol, 63.8 % yield). MS (ESI, m/z): 756.1 [M+H]⁺.

### Step 8)

### N-[3-[[4-[[3-(2,3-difluoro-4-pyrimidin-2-yloxy-phenyl)imidazo[1,2-a]pyrazin-8-yl]amino]-2-ethyl-benzoyl]amino]propyl]piperidine-4-carboxamide hydrochloride

To a solution of *tert*-butyl 4-((3-(4-((3-(2,3-difluoro-4-(pyrimidin-2-yloxy)phenyl)imidazo[1,2-a]pyrazin-8-yl)amino)-2-ethylbenzamido)propyl)carbamoyl)piperidine-1-carboxylate (320 mg, 423 µmol, Eq: 1) in MeOH ( 3 mL) was added HCl/dioxane (2 mol/L, 5mL), the reaction was stirred for 30 minutes at room temperature. The reaction mixture was dried in vacuum to give the title compound (290 mg, 419 µmol, 99.2 % yield). MS (ESI, m/z): 656.0 [M+H]⁺.

### Step 9)

### tert-Butyl 3-[[4-[3-[[4-[[3-(2,3-difluoro-4-pyrimidin-2-yloxy-phenyl)imidazo[1,2-a]pyrazin-8-yl]amino]-2-ethyl-benzoyl]amino]propylcarbamoyl]-1-piperidyl]methyl]pyrrolidine-1-carboxylate

To a solution of N-(3-(4-((3-(2,3-difluoro-4-(pyrimidin-2-yloxy)phenyl)imidazo[1,2-a]pyrazin-8-yl)amino)-2-ethylbenzamido)propyl)piperidine-4-carboxamide hydrochloride (170 mg, 246 µmol, Eq: 1) in MeOH ( 3 mL) was added *tert-*butyl 3-formylpyrrolidine-1-carboxylate (147 mg, 737 µmol, Eq: 3) and sodium cyanoborohydride (30.9 mg, 491 µmol, Eq: 2). The reaction was stirred for two hours at room temperature. The reaction mixture was quenched with water and extracted with EtOAc. The organic layer was dried over anhydrous Na₂SO₄ and concentrated in vacuum. The residue was purified by column chromatography to give the title compound (100 mg, 119 µmol, 48.5 % yield). MS (ESI, m/z): 839.1 [M+H]⁺.

### Step 10)

### tert-Butyl 3-[[4-[3-[[4-[[3-(2,3-difluoro-4-pyrimidin-2-yloxy-phenyl)imidazo[1,2-a]pyrazin-8-yl]amino]-2-ethyl-benzoyl]amino]propylcarbamoyl]-1-methyl-piperidin-1-ium-1-yl]methyl]pyrrolidine-1-carboxylate iodide

To a solution of *tert*-butyl 3-((4-((3-(4-((3-(2,3-difluoro-4-(pyrimidin-2-yloxy)phenyl)imidazo[1,2-a]pyrazin-8-yl)amino)-2-ethylbenzamido)propyl)carbamoyl)piperidin-1-yl)methyl)pyrrolidine-1-carboxylate ( 80 mg, 95.4 µmol, Eq: 1) in acetonitrile (3 mL) was added triethylamine (19.3 mg, 26.6 µL, 191 µmol, Eq: 2) and iodomethane (67.7 mg, 29.8 µL, 477 µmol, Eq: 5). The reaction was stirred for 16 hours at room temperature. The reaction mixture was concentrated in vacuum and the residue was directly used for the next step without further purification. MS (ESI, m/z): 853.0 [M]⁺.

### Step 11)

### N-[3-[[4-[[3-(2,3-Difluoro-4-pyrimidin-2-yloxy-phenyl)imidazo[1,2-a]pyrazin-8-yl]amino]-2-ethyl-benzoyl]amino]propyl]-1-methyl-1-(pyrrolidin-3-ylmethyl)piperidin-1-ium-4-carboxamide formate

To a solution of *tert*-butyl 3-[[4-[3-[[4-[[3-(2,3-difluoro-4-pyrimidin-2-yloxy-phenyl)imidazo[1,2-a]pyrazin-8-yl]amino]-2-ethyl-benzoyl]amino]propylcarbamoyl]-1-methyl-piperidin-1-ium-1-yl]methyl]pyrrolidine-1-carboxylate iodide ( 100 mg, 117 µmol, Eq: 1) in MeOH ( 3 mL) was added HCl/dioxane (2 mol/L, 3mL), the reaction was stirred for two hours at room temperature. The reaction mixture was concentrated in vacuum and the residue was purified by preparative HPLC to give the title compound (13 mg, 13.9 % yield). MS (ESI, m/z): 753.0 [M]⁺.

### Example 4

### 1-(Cyanomethyl)-1-(2-(2-(4-((3-(1-(cyanomethyl)-3-(trifluoromethyl)-1H-pyrazol-4-yl)imidazo[1,2-a]pyrazin-8-yl)amino)-2-ethylbenzamido)ethoxy)ethyl)pyrrolidin-1-ium formate

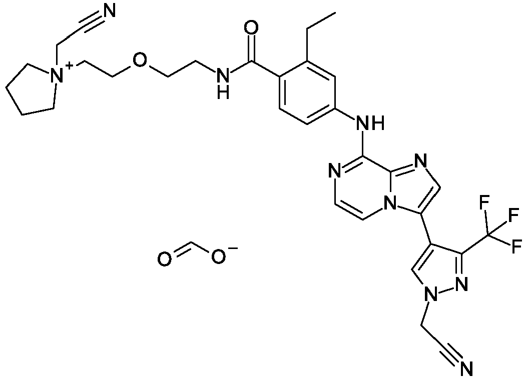

### Step 1)

### Methyl 4-amino-2-ethylbenzoate

Methyl 4-amino-2-bromobenzoate (20 g, 82.6 mmol, Eq: 1) was combined with DMF (80 ml). At room temperature, triethylborane solution 1.0 M in THF (125 ml, 125 mmol, Eq: 1.51) was added dropwise over 15 min. Potassium carbonate (17.1 g, 124 mmol, Eq: 1.5) was added. Under argon atmosphere, Pd(Ph₃P)₄ (477 mg, 413 µmol, Eq: 0.005) was added. The yellow suspension was heated to 85 °C and stirred for 2 h. The reaction mixture was poured into 400 mL of an ice/water mixture and was extracted with 600 ml and 300 ml of EtOAc. The organic layer was washed with 250 ml of brine and dried over Na₂SO₄. The crude material was purified by flash chromatography (silica gel, 330 g, 0% to 30% EtOAc in heptane) to afford the title compound (12.56 g, 70.1 mmol, 84.9% yield) as a white solid. MS (ESI, m/z): 180.1 [M+H]⁺.

### Step 2) Intermediate 31

### Methyl 2-ethyl-4-((3-iodoimidazo[1,2-a]pyrazin-8-yl)amino)benzoate hydrochloride

8-chloro-3-iodoimidazo[1,2-a]pyrazine (Intermediate 1, 16.05 g, 57.4 mmol, Eq: 1) and methyl 4-amino-2-ethylbenzoate (12.56 g, 70.1 mmol, Eq: 1.22) were combined with acetonitrile (70 ml) and acetic acid (7 ml). The reaction mixture was heated to 85 °C (reflux) and stirred for 15 h. The reaction was allowed to reach room temperature. The mixture was filtered through sintered glass and washed with 150 ml of ACN/MeOH 1:1. The obtained solid was dried in vacuo to afford the title compound (25.76 g, 56.2 mmol, 97.8% yield) as a light yellow crystalline solid. MS (ESI, m/z): 423.2 [M+H]⁺.

### Step 3)

### Methyl 2-ethyl-4-((3-(3-(trifluoromethyl)-1H-pyrazol-4-yl)imidazo[1,2-a]pyrazin-8-yl)amino)benzoate

To methyl 2-ethyl-4-((3-iodoimidazo[1,2-a]pyrazin-8-yl)amino)benzoate hydrochloride (2 g, 4.36 mmol, Eq: 1), (3-(trifluoromethyl)-1H-pyrazol-4-yl)boronic acid (1.41 g, 7.85 mmol, Eq: 1.8), 1,1'-bis(diphenylphosphino)ferrocene-palladium(ii)dichloride dichloromethane complex (534 mg, 654 µmol, Eq: 0.15) and Na₂CO₃ (1.06 g, 10 mmol, Eq: 2.3) was added a degazed solution of dioxane (35 ml)/water (8 ml). The mixture was stirred at 110°C overnight. The reaction mixture was poured into 75 mL of saturated NaCl and extracted with EtOAc (3 x 75 mL). The organic layers were combined, adsorbed on Isolute and purified by flash chromatography (silica gel, SiliCycle 20g cartridge (40-63 µm), 10% to 100% EtOAc in heptane) to afford the title compound (1.534 g, 2.74 mmol, 62.9% yield). MS (ESI, m/z): 431.3 [M+H]⁺.

### Step 4) Intermediate 32

### 2-Ethyl-4-((3-(3-(trifluoromethyl)-1H-pyrazol-4-yl)imidazo[1,2-a]pyrazin-8-yl)amino)benzoic acid

Methyl 2-ethyl-4-((3-(3-(trifluoromethyl)-1H-pyrazol-4-yl)imidazo[1,2-a]pyrazin-8-yl)amino)benzoate (1.534 g, 2.74 mmol, Eq: 1) was treated with LiOH.H₂O (576 mg, 13.7 mmol, Eq: 5) in a mixture of THF (40 ml)/MeOH (10 ml)/Water (10 ml) at 65°C overnight. The organic solvent was removed under vacuum. To the diluted solution in water and under stirring was added acetic acid (4 ml). The resulting white suspension was filtered off, washed with water and dried under vacuum to afford the title compound (1.168 g, 2.3 mmol, 83.8% yield) as white cristals. MS (ESI, m/z): 417.2 [M+H]⁺.

### Step 5)

### 2-Ethyl-N-(2-(2-(pyrrolidin-1-yl)ethoxy)ethyl)-4-((3-(3-(trifluoromethyl)-1H-pyrazol-4-yl)imidazo[1,2-a]pyrazin-8-yl)amino)benzamide

To a mixture of 2-ethyl-4-((3-(3-(trifluoromethyl)-1H-pyrazol-4-yl)imidazo[1,2-a]pyrazin-8-yl)amino)benzoic acid (125 mg, 300 µmol, Eq: 1), TBTU (119 mg, 360 µmol, Eq: 1.2) and TEA (209 µl, 1.5 mmol, Eq: 5) in DMF (10 ml) was added 2-(2-(pyrrolidin-1-yl)ethoxy)ethan-1-amine (61.7 mg, 390 µmol, Eq: 1.3). The mixture was stirred at room temperature overnight. The mixture was concentrated in vacuo and then diluted with 15 mL of 1M 0KHSO₄ and extracted with EtOAc (3 x 10 mL). The solution was concentrated in vacuo and the obtained material used without further purification. MS (ESI, m/z): 557.3 [M+H]⁺.

### Step 6)

### 1-(cyanomethyl)-1-(2-(2-(4-((3-(1-(cyanomethyl)-3-(trifluoromethyl)-1H-pyrazol-4-yl)imidazo[1,2-a]pyrazin-8-yl)amino)-2-ethylbenzamido)ethoxy)ethyl)pyrrolidin-1-ium formate

2-ethyl-N-(2-(2-(pyrrolidin-1-yl)ethoxy)ethyl)-4-((3-(3-(trifluoromethyl)-1H-pyrazol-4-yl)imidazo[1,2-a]pyrazin-8-yl)amino)benzamide (22.3 mg, 40 µmol, Eq: 1) was dissolved in DMF (2 ml). Potassium carbonate (13.8 mg, 100 µmol, Eq: 2.5) was added followed by 2-bromoacetonitrile (28.8 mg, 60 µmol, Eq: 1.5). The mixture was stirred at room temperature overnight. The reaction mixture was purified by preparative HPLC to afford the title compound (8.1 mg, 11.9 µmol, 29.7% yield). MS (ESI, m/z): 635.4 [M]⁺.

### Example 5

### 4-[[3-[4-(4-aminobut-2-ynoxy)phenyl]imidazo[1,2-a]pyrazin-8-yl]amino]-N-[2-(1,1-dimethylpiperidin-1-ium-4-yl)ethyl]-N,2-dimethyl-benzamide formate

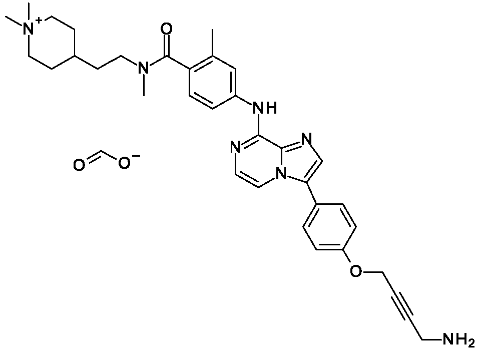

### Step 1)

### tert-Butyl N-(4-hydroxybut-2-ynyl)carbamate

A mixture of 4-hydroxybut-2-ynyl methanesulfonate (800.0 mg, 4.87 mmol, 1 eq) in 35% ammonium hydroxide (30.0 mL) was stirred at 30 °C for 8 h. Then di-t-butyldicarbonate (1276 mg, 5.85 mmol, 1.2 eq) was added. The resulting mixture was stirred for another 7 h. Then the mixture was concentrated and the residue was purified by silica gel chromatography eluting with PE:EA=3:1 to afford the title compound (180 mg, 0.970 mmol, 19.94% yield) as colorless oil. ¹H NMR (400 MHz, CDCl3) δ: 4.84 (br s, 1H), 4.24 (t, J = 1.9 Hz, 2H), 3.94 (br d, J = 4.4 Hz, 2H), 2.50 - 2.21 (m, 1H), 1.43 (s, 9H) ppm

### Step 2)

### 4-(tert-Butoxycarbonylamino)but-2-ynyl methanesulfonate

A solution of tert-butyl N-(4-hydroxybut-2-ynyl)carbamate (180.0 mg, 0.970 mmol, 1 eq) and triethylamine (0.2 mL, 1.46 mmol, 1.5 eq) in DCM (3 mL) was cooled to 0 °C and methanesulfonyl chloride (0.09 mL, 1.17 mmol, 1.2 eq) was added. The resulting mixture was stirred at 0 °C for 2 h. The mixture was diluted with water and DCM (50 mL) was added. The organic phase was separated and washed with brine, dried over sodium sulfate and concentrated to afford the title compound (250 mg, 0.950 mmol, 97.7% yield) as colorless oil, which was used without further purification.

### Step 3)

### 4-(8-Chloroimidazo[1,2-a]pyrazin-3-yl)phenol

A mixture of 8-chloro-3-iodo-imidazo[1,2-a]pyrazine (Intermediate 1, 1.0 g, 3.58 mmol, 1 eq), 4-hydroxyphenylboronic acid (592 mg, 4.29 mmol, 1.2 eq), sodium carbonate (758 mg, 7.16 mmol, 2 eq) and [1,1'-bis(diphenylphosphino)ferrocene]dichloropalladium(II) (130.91 mg, 0.180 mmol, 0.050 eq) in 1,4-dioxane (10 mL) and water (1 mL) was heated to 80 °C for 15 h under nitrogen atmosphere. Then the mixture was diluted with water, extracted with EtOAc (100 mL*2), dried over sodium sulfate and concentrated. The residue was triturated with MTBE (50 mL) to afford the title compound (500 mg, 2.04 mmol, 45.51% yield) as brown solid. MS (ESI, m/z): 264.0 [M+H]⁺

### Step 4)

### tert-Butyl N-[4-[4-(8-chloroimidazo[1,2-a]pyrazin-3-yl)phenoxy]but-2-ynyl]carbamate

A mixture of 4-(tert-butoxycarbonylamino)but-2-ynyl methanesulfonate (214.37 mg, 0.810 mmol, 1 eq) , 4-(8-chloroimidazo[1,2-a]pyrazin-3-yl)phenol (200.0 mg, 0.810 mmol, 1 eq) and potassium carbonate (112.52 mg, 0.810 mmol, 1 eq) in ACN (10 mL) was heated to 60 °C for 15 h. Then the mixture was filtered and the filtrate was concentrated. The residue was purified by flash column to afford the title compound (180 mg, 0.440 mmol, 48.2% yield) as orange oil. MS (ESI, m/z): 413.8 [M+H]⁺

### Step 5)

### N,2-Dimethyl-N-[2-(1-methyl-4-piperidyl)ethyl]-4-nitro-benzamide

To a solution of 2-methyl-4-nitro-benzoic acid (150.0 mg, 0.830 mmol, 1 eq) in DMF (5 mL) was added O-(7-azabenzotriazol-1-yl)-N,N,N',N'-tetramethyluronium hexafluorophosphate (486.63 mg, 1.28 mmol, 1.55 eq), followed by addtion of N,N-diisopropylethylamine (0.22 mL, 1.28 mmol, 1.55 eq). After being stirred at 30 °C for 10 min N-methyl-2-(1-methyl-4-piperidyl)ethanamine (200.0 mg, 1.28 mmol, 1.55 eq) was added. The resulting mixture was stirred at 30 °C for 2 h. The mixture was poured into water (50 mL), extracted with EtOAc (50 mL*2). The combined oragnic phases were washed with brined, dried over sodium sulfate and concentrated. The residue was purified by silica gel chromagraphy eluting with PE:EA=10:1 to 1:1 to the title compound (200 mg, 0.630 mmol, 48.93% yield) as yellow oil.

### Step 6)

### 4-Amino-N,2-dimethyl-N-[2-(1-methyl-4-piperidyl)ethyl]benzamide

To a solution of N,2-dimethyl-N-[2-(1-methyl-4-piperidyl)ethyl]-4-nitro-benzamide (200.0 mg, 0.630 mmol, 1 eq) in methanol (10 mL) was added 10% palladium on carbon (20.0 mg, 0.190 mmol, 0.030 eq). The resulting mixture was hydrogenated under 760 mmHg at 25 °C for 15 h. The catalyst was removed by filtration and the filtrate was concentrated to afford the title compound (150 mg, 0.520 mmol, 82.77% yield) as colorless oil, which was used without further purification. MS (ESI, m/z): 290.3 [M+H]⁺

### Step 7)

### tert-Butyl N-[4-[4-[8-[3-methyl-4-[methyl-[2-(1-methyl-4-piperidyl)ethyl]carbamoyl]anilino]imidazo[1,2-a]pyrazin-3-yl]phenoxy]but-2-ynyl]carbamate

To a solution of tert-butyl N-[4-[4-(8-chloroimidazo[1,2-a]pyrazin-3-yl)phenoxy]but-2-ynyl]carbamate (100.0 mg, 0.240 mmol, 1 eq) in ACN (4.5 mL) and acetic acid (0.500 mL) was added 4-amino-N,2-dimethyl-N-[2-(1-methyl-4-piperidyl)ethyl]benzamide (84.12 mg, 0.290 mmol, 1.2 eq). The resulting mixture was stirred at 80 °C for 15 h. The mixture was concentrated and the residue was purified by prep-HPLC to afford the title compound (70 mg, 0.110 mmol, 43.41% yield) as yellow oil. MS (ESI, m/z): 666.4 [M+H]⁺

### Step 8)

### tert-Butyl N-[4-[4-[8-[4-[2-(1,1-dimethylpiperidin-1-ium-4-yl)ethyl-methyl-carbamoyl]-3-methyl-anilino]imidazo[1,2-a]pyrazin-3-yl]phenoxy]but-2-ynyl]carbamate iodide

To a solution of tert-butyl N-[4-[4-[8-[3-methyl-4-[methyl-[2-(1-methyl-4-piperidyl)ethyl]carbamoyl]anilino]imidazo[1,2-a]pyrazin-3-yl]phenoxy]but-2-ynyl]carbamate (40.0 mg, 0.060 mmol, 1 eq) in ACN (2 mL) was added iodomethane (852.72 mg, 6.01 mmol, 100 eq). The resulting mixture was stirred at 25 °C for 15 h. Then the mixture was concentrated to afford the title compound (40 mg, 0.060 mmol, 97.79% yield) as yellow oil, which was used without further purification. MS (ESI, m/z): 680.4 [M]⁺

### Step 9)

### 4-[[3-[4-(4-aminobut-2-ynoxy)phenyl]imidazo[1,2-a]pyrazin-8-yl]amino]-N-[2-(1,1-dimethylpiperidin-1-ium-4-yl)ethyl]-N,2-dimethyl-benzamide formate

To a solution of tert-butyl N-[4-[4-[8-[4-[2-(1,1-dimethylpiperidin-1-ium-4-yl)ethyl-methylcarbamoyl]-3-methyl-anilino]imidazo[1,2-a]pyrazin-3-yl]phenoxy]but-2-ynyl]carbamate iodide (40.0 mg, 0.050 mmol, 1 eq) in methanol (2 mL) was added hydrogen chloride in MeOH (3.5 mL, 14 mmol, 282.66 eq), The resulting mixture was stirred at 25 °Cfor 15 h. LCMS indicated that most starting material was consumed up and and the mixture was concentrated. the residue was purified by prep-HPLC to afford the title compound (11.4 mg, 0.020 mmol, 30.58% yield) as colorless oil. MS (ESI, m/z): 580.5 [M]⁺

### Example 6

### 4-[[3-[2,3-difluoro-4-(2-methyltriazol-4-yl)oxy-phenyl]imidazo[1,2-a]pyrazin-8-yl]amino]-2-ethyl-N-[[1-methyl-1-(pyrrolidin-3-ylmethyl)piperidin-1-ium-4-yl]methyl]benzamide formate

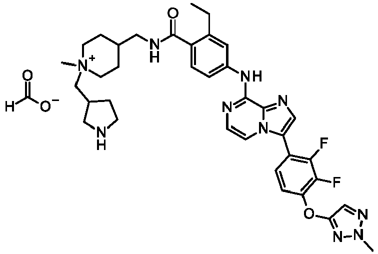

### Step 1)

### (1-Benzyl-5-iodo-triazol-4-yl)-trimethyl-silane

To a solution of (azidomethyl)benzene (2 g, 15 mmol, Eq: 1) in acetonitrile (30 mL) was added *tert*-butyl(ethynyl)silane (1.69 g, 15 mmol, Eq: 1), DIPEA (1.94 g, 2.62 ml, 15 mmol, Eq: 1), copper (I) iodide (2.86 g, 15 mmol, Eq: 1) and NBS (2.67 g, 15 mmol, Eq: 1), the reaction was stirred for 12 hours at room temperature. The reaction mixture was filtered and the filtrate was concentrated in vacuum. The residue was purified by column chromatography to give the title compound (2 g, 5.6 mmol, 37.3%). MS (ESI, m/z): 358.0 [M+H]⁺.

### Step 2)

### 1-Benzyl-5-(4-bromo-2,3-difluoro-phenoxy)triazole

To a solution of 4-bromo-2,3-difluorophenol (500 mg, 2.39 mmol, Eq: 1) in acetonitrile (20 mL) was added 1-benzyl-5-iodo-4-(trimethylsilyl)-1H-1,2,3-triazole (855 mg, 2.39 mmol, Eq: 1), ethyl 2-oxocyclohexanecarboxylate (81.4 mg, 478 µmol, Eq: 0.2),copper (I) bromide (34.3 mg, 239 µmol, Eq: 0.1) and Cs₂CO₃ (1.56 g, 4.78 mmol, Eq: 2), the reaction was stirred for 12 hours at 70 °C. The mixture was cooled to room temperature and filtered. The filtrate was concentrated and the residue was purified by column chromatography to give the title compound (612 mg, 1.7 mmol, 69.9%). MS (ESI, m/z): 366.0 [M+H]⁺.

### Step 3)

### 1-Benzyl-5-[2,3-difluoro-4-(4,4,5,5-tetramethyl-1,3,2-dioxaborolan-2-yl)phenoxy]triazole

To a solution of 1-benzyl-5-(4-bromo-2,3-difluorophenoxy)-1H-1,2,3-triazole ( 600 mg, 1.64 mmol, Eq: 1) in dioxane ( 15 mL) was added 4,4,4',4',5,5,5',5'-octamethyl-2,2'-bi(1,3,2-dioxaborolane) (416 mg, 1.64 mmol, Eq: 1), potassium acetate (322 mg, 3.28 mmol, Eq: 2) and 1,1'-bis(di-*tert*-butylphosphino)ferrocene palladium dichloride (134 mg, 164 µmol, Eq: 0.1), the reaction was stirred for 12 hours at 80 °C. The reaction mixture was cooled to room temperature and filtered. The filtrate was concentrated in vacuum and the residue was purified by column chromatography to give the title compound (520 mg, 1.26 mmol, 76.8 % yield). MS (ESI, m/z): 414.1 [M+H]⁺.

### Step 4)

### tert-Butyl 4-[[[4-[[3-[4-(3-benzyltriazol-4-yl)oxy-2,3-difluoro-phenyl]imidazo[1,2-a]pyrazin-8-yl]amino]-2-ethyl-benzoyl]amino]methyl]piperidine-1-carboxylate

To a solution of *tert*-butyl 4-((2-ethyl-4-((3-iodoimidazo[1,2-a]pyrazin-8-yl)amino)benzamido)methyl)piperidine-1-carboxylate ( 280mg, 463 µmol, Eq: 1) in the mixture solvent of dioxane (5 mL) and water (1 mL) was added 1-benzyl-5-(2,3-difluoro-4-(4,4,5,5-tetramethyl-1,3,2-dioxaborolan-2-yl)phenoxy)-1H-1,2,3-triazole (191 mg, 463 µmol, Eq: 1), sodium carbonate (98.2 mg, 926 µmol, Eq: 2) and 1,1'-bis(di-*tert*-butylphosphino)ferrocene palladium dichloride (30.2 mg, 46.3 µmol, Eq: 0.1). The reaction was stirred for 20 minutes at 100°C under microwave irradiation on and atmosphere of nitrogen. The reaction mixture was filtered and the filtrate was concentrated in vacuum. The residue was purified by column chromatography to give *tert*-butyl 4-[[[4-[[3-[4-(3-benzyltriazol-4-yl)oxy-2,3-difluorophenyl]imidazo[1,2-a]pyrazin-8-yl]amino]-2-ethyl-benzoyl]amino]methyl]piperidine-1-carboxylate (190 mg,249 µmol, 53.7 % yield). MS (ESI, m/z): 764.0 [M+H]⁺.

### Step 5)

### 4-[[3-[4-(3-Benzyltriazol-4-yl)oxy-2,3-difluoro-phenyl]imidazo[1,2-a]pyrazin-8-yl]amino]-2-ethyl-N-(4-piperidylmethyl)benzamide hydrochloride

To dissolve *tert*-butyl 4-((4-((3-(4-((1-benzyl-1H-1,2,3-triazol-5-yl)oxy)-2,3-difluorophenyl)imidazo[1,2-a]pyrazin-8-yl)amino)-2-ethylbenzamido)methyl)piperidine-1-carboxylate (190 mg, 249 µmol, Eq: 1) in MeOH (3mL) was added HCl/dioxane (2 mol/L, 3 mL), the reaction was stirred for 30 minutes at room temperature. The reaction mixture was concentrated in vacuum to give the title compound (170 mg, 97.5 % yield). MS (ESI, m/z): 664.0 [M+H]⁺.

### Step 6)

### tert-Butyl 3-[[4-[[[4-[[3-[4-(3-benzyltriazol-4-yl)oxy-2,3-difluoro-phenyl]imidazo[1,2-a]pyrazin-8-yl]amino]-2-ethyl-benzoyl]amino]methyl]-1-piperidyl]methyl]pyrrolidine-1-carboxylate

To a solution of 4-((3-(4-((1-benzyl-1H-1,2,3-triazol-5-yl)oxy)-2,3-difluorophenyl)imidazo[1,2-a]pyrazin-8-yl)amino)-2-ethyl-N-(piperidin-4-ylmethyl)benzamide hydrochloride (160 mg, 229 µmol, Eq: 1) in MeOH ( 3mL) was added *tert-*butyl 3-formylpyrrolidine-1-carboxylate (137 mg, 686 µmol, Eq: 3) and sodium cyanoborohydride (28.7 mg, 457 µmol, Eq: 2). The reaction was stirred for two hours at room temperature. The reaction mixture was quenched with water and extracted in EtOAc. The organic layer was dried over anhydrous Na₂SO₄ and concentrated in vacuum. The residue was purified by column chromatgraphy to give *tert*-butyl 3-[[4-[[[4-[[3-[4-(3-benzyltriazol-4-yl)oxy-2,3-difluoro-phenyl]imidazo[1,2-a]pyrazin-8-yl]amino]-2-ethyl-benzoyl]amino]methyl]-1-piperidyl]methyl]pyrrolidine-1-carboxylate (160 mg, 189 µmol, 82.5 % yield). MS (ESI, m/z): 847.0 [M+H]⁺.

### Step 7)

### tert-Butyl 3-[[4-[[[4-[[3-[2,3-difluoro-4-(1H-triazol-5-yloxy)phenyl]imidazo[1,2-a]pyrazin-8-yl]amino]-2-ethyl-benzoyl]amino]methyl]-1-piperidyl]methyl] pyrrolidine-1-carboxylate

To a solution of *tert*-butyl 3-((4-((4-((3-(4-((1-benzyl-1H-1,2,3-triazol-5-yl)oxy)-2,3-difluorophenyl)imidazo[1,2-a]pyrazin-8-yl)amino)-2-ethylbenzamido)methyl)piperidin-1-yl)methyl)pyrrolidine-1-carboxylate ( 160 mg, 189 µmol, Eq: 1) in EtOH (100 mL) was added Pd/C (50 mg), the reaction was stirred for 20 hours at room temperature under atmosphere of hydrogen. The reaction mixture was filtered and the filtrate was concentrated in vacuum. The residue was directly used in the next step without further purification. MS (ESI, m/z): 757.0 [M+H]⁺.

### Step 8)

### tert-Butyl 3-[[4-[[[4-[[3-[2,3-difluoro-4-(2-methyltriazol-4-yl)oxy-phenyl]imidazo[1,2-a]pyrazin-8-yl]amino]-2-ethyl-benzoyl]amino]methyl]-1-methyl-piperidin-1-ium-1-yl]methyl]pyrrolidine-1-carboxylate iodide

To a solution of *tert*-butyl 3-((4-((4-((3-(4-((2H-1,2,3-triazol-4-yl)oxy)-2,3-difluorophenyl)imidazol 1,2-a]pyrazin-8-yl)amino)-2-ethylbenzamido)methyl)piperidin-1-yl)methyl)pyrrolidine-1-carboxylate (50 mg, 66.1 µmol, Eq: 1) in MeOH (3 mL) was added iodomethane (46.9 mg, 20.7 µL, 330 µmol, Eq: 5) and TEA (20.1 mg, 27.6 µL, 198 µmol, Eq: 3). The reaction was stirred for 20 hours at room temperature. The reaction mixture was concentrated in vacuum and the residue was directly used for the next step without further purification. MS (ESI, m/z): 785.0 [M]⁺.

### Step 9)

### 4-[[3-[2,3-Difluoro-4-(2-methyltriazol-4-yl)oxy-phenyl]imidazo[1,2-a]pyrazin-8-yl]amino]-2-ethyl-N-[[1-methyl-1-(pyrrolidin-3-ylmethyl)piperidin-1-ium-4-yl]methyl]benzamide formate

To a solution of *tert*-butyl 3-[[4-[[[4-[[3-[2,3-difluoro-4-(2-methyltriazol-4-yl)oxyphenyl]imidazo[1,2-a]pyrazin-8-yl]amino]-2-ethyl-benzoyl]amino]methyl]-1-methyl-piperidin-1-ium-1-yl]methyl]pyrrolidine-1-carboxylate iodide (60 mg, 50 µmol, Eq: 1) in MeOH ( 5 mL) was added HCl/ EtOAC ( 1mol/L ,5 mL). The reaction was stirred for two hours at room temperature. The reaction mixture was concentrated in vacuum and the residue was purified by preparative HPLC to give the title compound (5 mg, 13.7% yield) . MS (ESI, m/z): 685.0 [M]⁺.

### Example 7

### [2-[3-[[2-Chloro-4-[[3-[3-(trifluoromethyl)-1H-pyrazol-4-yllimidazo[1,2-alpyrazin-8-yl]amino]benzoyl]amino]propylamino]-2-oxo-ethyl]-trimethyl-ammoniumformate

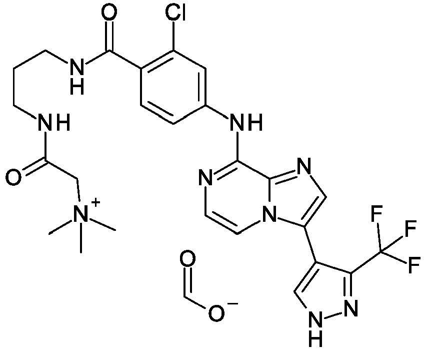

### Step 1) Intermediate 28

### 2-Chloro-4-[[3-[3-(trifluoromethyl)-1-trityl-pyrazol-4-yl]imidazo[1,2-a]pyrazin-8-yl]amino]benzoic acid

To a solution of methyl 2-chloro-4-[(3-iodoimidazo[1,2-a]pyrazin-8-yl)amino]benzoate (Intermediate 26, 250 mg, 0.580 mmol, 1 eq) and 4-(4,4,5,5-tetramethyl-1,3,2-dioxaborolan-2-yl)-3-(trifluoromethyl)-1-trityl-pyrazole (441.27 mg, 0.870 mmol, 1.5 eq) in 1,4-Dioxane (10 mL)/Water (1 mL) was added 1,1'-bis(diphenylphosphino)ferrocene-palladium(II)dichloride dichloromethane complex (23.8 mg, 0.030 mmol, 0.05 eq) and sodium carbonate (123.64 mg, 1.17 mmol, 2 eq). The reaction was stirred at 80 °C under N₂ for 12 h. Then sodium hydroxide (69.99 mg, 1.75 mmol, 3 eq) and water (3 mL) were added to the mixture and it was stirred at 80 °C for 4 h. To the reaction mixture was added water (20 mL) and it was adjusted to pH=3 with formic acid. Then the solids were filtered and the filter cake was collected and dried to afford the title compound (400 mg, 0.600 mmol, 103.11% yield) as a brown solid. MS (ESI, m/z): 665.3 [M+H]⁺.

### Step 2)

### tert-butyl N-[3-[[2-(dimethylamino)acetyl]amino]propyl]carbamate

To a solution of N,N-dimethylglycine (500 mg, 4.85 mmol, 1 eq) in DMF (5 mL) was added 1,1'-carbonyldiimidazole (786 mg, 4.85 mmol, 1 eq). The mixture was stirred at 50 °C for 1 h. Then N-Boc-1,3-diaminopropane (929 mg, 5.33 mmol, 1.1 eq) was added and the reaction mixture was stirred for 12 h. The reaction was diluted with water (30 mL) and extracted with DCM/MeOH=10:1 (30 mL x 3). The combined organic layer was washed with brine (50 mL), dried over Na₂SO₄, filtered and concentrated to afford the title compound (1.05 g, 4.05 mmol, 83.5% yield) as a colorless oil. MS (ESI, m/z): 260.3 [M+H]⁺.

### Step 3)

### [2-[3-(tert-butoxycarbonylamino)propylamino]-2-oxo-ethyl]-trimethyl-ammonium iodide

To a solution of tert-butyl N-[3-[[2-(dimethylamino)acetyl]amino]propyl]carbamate (1.05 g, 4.05 mmol, 1 eq) in ethyl acetate (8.33 mL) was added iodomethane (632 mg, 4.45 mmol, 1.1 eq). The reaction was stirred at 30 °C for 16 h. The reaction mixture was filtered and the filter cake was collected and dried to afford the title compound (1.08 g, 2.59 mmol, 63.92% yield) as a white solid. MS (ESI, m/z): 274.2 [M+H]⁺.

### Step 4)

### [2-(3-Aminopropylamino)-2-oxo-ethyl]-trimethyl-ammonium iodide hydrochloride

[2-[3-(tert-butoxycarbonylamino)propylamino]-2-oxo-ethyl]-trimethyl-ammonium iodide (1.08 g, 2.69 mmol, 1 eq) was added to 4M hydrogen chloride in methanol (10 mL, 40 mmol, 14.86 eq). The reaction was stirred at 30 °C for 1 h. The reaction mixture was concentrated to afford the title compound (900 mg, 2.67 mmol, 99.04% yield) as a yellow oil. MS (ESI, m/z): 174.1 [M+H]⁺.

### Step 5)

### [2-[3-[[2-Chloro-4-[[3-[3-(trifluoromethyl)-1-trityl-pyrazol-4-yl]imidazo[1,2-a]pyrazin-8-yl]amino]benzoyl]amino]propylamino]-2-oxo-ethyl]-trimethyl-ammonium formate

To a solution of 2-chloro-4-[[3-[3-(trifluoromethyl)-1-trityl-pyrazol-4-yl]imidazo[1,2-a]pyrazin-8-yl]amino]benzoic acid (200 mg, 0.300 mmol, 1 eq) and [2-(3-aminopropylamino)-2-oxoethyl]-trimethyl-ammonium iodide hydrochloride (122 mg, 0.360 mmol, 1.2 eq) in DMF (2 mL) were added N,N-diisopropylethylamine (0.16 mL, 0.900 mmol, 3 eq) and O-(7-azabenzotriazol-1-yl)-N,N,N',N'-tetramethyluronium hexafluorophosphate (172 mg, 0.450 mmol, 1.5 eq). The reaction was stirred at 30 °C for 12 h. The reaction mixture was purified by prep-HPLC (FA) to afford the title compound (100 mg, 0.120 mmol, 35.07% yield) as a yellow oil. MS (ESI, m/z): 820.3 [M+H]⁺.

### Step 6)

### [2-[3-[[2-Chloro-4-[[3-[3-(trifluoromethyl)-1H-pyrazol-4-yl]imidazo[1,2-a]pyrazin-8-yl]amino]benzoyl]amino]propylamino]-2-oxo-ethyl]-trimethyl-ammonium formate

To a solution of [2-[3-[[2-chloro-4-[[3-[3-(trifluoromethyl)-1-trityl-pyrazol-4-yl]imidazo[1,2-a]pyrazin-8-yl]amino]benzoyl]amino]propylamino]-2-oxo-ethyl]-trimethyl-ammonium formate (100 mg, 0.120 mmol, 1 eq) in DCM (3 mL) was added trifluoroacetic acid (1 mL, 12.98 mmol, 112.45 eq). The reaction was stirred at 30 °C for 12 h. The reaction mixture was adjusted to pH=7 with TEA and purified by prep-HPLC (FA) to afford the title compound (14.7 mg, 0.020 mmol, 20.27% yield) as a white solid. MS (ESI, m/z): 578.2 [M+H]⁺.

### Example 8

### azetidin-3-ylmethyl-(carboxymethyl)-[5-[[4-[[3-[1-(2,2-difluoroethyl)-3-(trifluoromethyl)pyrazol-4-yl]imidazo[1,2-a]pyrazin-8-yl]amino]-2-ethyl-benzoyl]amino]pentyl]-methyl-ammonium formate

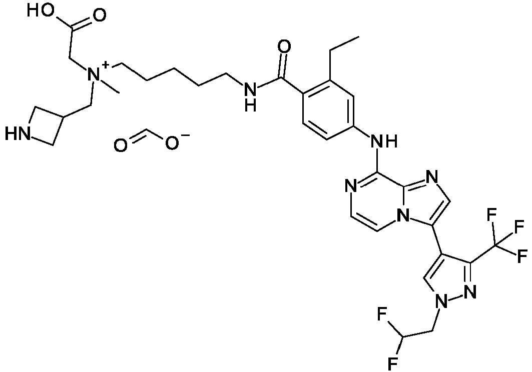

### Step 1)

### 4-Iodo-3-(trifluoromethyl)-1H-pyrazole

A solution of 3-(trifluoromethyl)pyrazole (200.0 g, 1470 mmol, 1 eq) in sulfuric acid (100.0 mL, 1470 mmol, 1 eq)/water (400 mL) was added N-iodosuccinimide (396.79 g, 1764 mmol, 1.2 eq) at 0°C and stirred for 10 min, then the solution was stirred at 20°C for 3h. Then the mixture was poured into water (3 L) and stirred for another 13h. The mixture was filtered and to the residue was added saturated Na₂SO₃ solution (1000 mL) , extracted with EtOAc (1000 mL*2), washed with brine (1000 mL), dried to afford the title compound (333 g, 1271 mmol, 86.49% yield) as white solid. MS (ESI, m/z): 262.8 [M+H]⁺

### Step 2)

### 4-Iodo-3-(trifluoromethyl)-1-trityl-pyrazole

To a solution of 4-iodo-3-(trifluoromethyl)-1H-pyrazole (110.0 g, 419.9 mmol, 1 eq) in THF (1100 mL) was added sodium hydride, 60% in oil (18.47 g, 461.88 mmol, 1.1 eq) at 0°C, the mixture was stirred for 0.5h at 0°C, then triphenylmethyl chloride (128.76 g, 461.88 mmol, 1.1 eq) was added to the solution. The mixture was stirred for 16 h at 10 °C. The mixture was quenched with sat. NH₄Cl (500 mL) and extracted with EtOAc (300 mL x 2), washed with brine (700 mL), dried over sodium sulfate, filtered and concentrated in vacuum to give crude product, which was purified by silica gel column chromatography eluting Petroluem ehter/EtOAc=100/1 to give the title compound (96 g, 190.37 mmol, 45.34% yield) as white solid. MS (ESI, m/z): 543.0 [M+K]⁺

### Step 3)

### [3-(trifluoromethyl)-1-trityl-pyrazol-4-yl]boronic acid

To a solution of 4-iodo-3-(trifluoromethyl)-1-trityl-pyrazole (45.0 g, 89.23 mmol, 1 eq) in THF (316.9 mL) was added dropwise butyllithium solution (42.83 mL, 107.08 mmol, 1.2 eq) at -70 °C under N2 and the mixture was stirred for 0.5h at -70 °C, then boron isopropoxide (30.89 mL, 133.85 mmol, 1.5 eq) was added dropwise to the mixture and stirred for 1 h at -70 °C for 0.5h. Then the mixture was poured into 300 mL sat.NH₄Cl was added and stirred for 0.5h, extracted with EtOAc (300 mL x 2), washed with brine (500 mL), dried over sodium sulfate, filtered and concentrated in vacuum and purified by silica column (PE/EA=4:1) to give the title compound (30 g, 71.05 mmol, 79.63% yield) as white solid. MS (ESI, m/z): 445.2 [M+Na]⁺

### Step 4)

### 8-chloro-3-[3-(trifluoromethyl)-1-trityl-pyrazol-4-yl]imidazo[1,2-a]pyrazine

A mixture of [3-(trifluoromethyl)-1-trityl-pyrazol-4-yl]boronic acid (9.06 g, 21.47 mmol, 1.2 eq), 8-chloro-3-iodo-imidazo[1,2-a]pyrazine (Intermediate 1, 5.0 g, 17.89 mmol, 1 eq), [1,1'-bis(diphenylphosphino)ferrocene]dichloropalladium(II) (0.65 g, 0.890 mmol, 0.050 eq), sodium carbonate (3.79 g, 35.78 mmol, 2 eq) in 1,4-dioxane (100 mL) was stirred under N2 at 80 °C for 16 h. The mixture was filtered, the filtrate was concentrated and purified by silica gel column chromatography eluting with Petroleum ehter/EtOAc=6:1 to 4:1 to afford the title compound (7 g, 13.21 mmol, 73.83% yield) as light yellow solid. MS (ESI, m/z): 530.1 [M+H]⁺

### Step 5) Intermediate 42

### 8-chloro-3-[3-(trifluoromethyl)-1H-pyrazol-4-yl]imidazo[1,2-a]pyrazine

A mixture of 8-chloro-3-[3-(trifluoromethyl)-1-trityl-pyrazol-4-yl]imidazo[1,2-a]pyrazine (7.0 g, 13.21 mmol, 1 eq) in 4M hydrochloric acid in dioxane (70.0 mL, 280 mmol, 21.2 eq) was stirred at 20 °C for 16 h. To the mixture MTBE (100 mL) was added, filtered to afford to afford the title compound as (3.5 g, 12.17 mmol, 92.12% yield) as white solid. MS (ESI, m/z): 288.0 [M+H]⁺

### Step 6) Intermediate 43

### 8-chloro-3-[1-(2,2-difluoroethyl)-3-(trifluoromethyl)pyrazol-4-yl]imidazo[1,2-a]pyrazine

To a mixture of 8-chloro-3-[3-(trifluoromethyl)-1H-pyrazol-4-yl]imidazo[1,2-a]pyrazine (1.5 g, 5.22 mmol, 1 eq) in ACN (30 mL) was added 2,2-difluoroethyl trifluoromethanesulfonate (1.34 g, 6.26 mmol, 1.2 eq) at -10°C, then the reaction was stirred at 0 °C for 16 h. The mixture was poured into water (50 mL), extracted with EtOAc (30 mL*2), washed by brine (50 mL), concentrated and purified by silica column (PE/EA=3:1 to 2:1) to afford the title compound (760 mg, 2.16 mmol, 41.44% yield) as white solid. MS (ESI, m/z): 352.1 [M+H]⁺

### Step 7)

### azetidin-3-ylmethyl-(carboxymethyl)-[5-[[4-[[3-[1-(2,2-difluoroethyl)-3-(trifluoromethyl)pyrazol-4-yl]imidazo [1,2-a]pyrazin-8-yl] amino]-2-ethyl-benzoyl]amino]pentyl]-methyl-ammonium formate

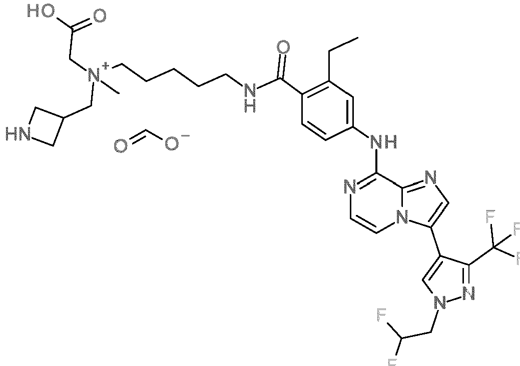

The title compound was prepared in analogy to example 104, using 8-chloro-3-[1-(2,2-difluoroethyl)-3-(trifluoromethyl)pyrazol-4-yl]imidazo[1,2-a]pyrazine in step 3, skipping step 5, using tert-butyl 3-formylazetidine-1-carboxylate in step 6. The title compound was obtained as whitel solid (26.4 mg). MS (ESI, m/z): 706.3 [M]⁺, 353.6 [M+H]²⁺

### Example 9

### 1-(2-amino-2-oxoethyl)-4-(4-(4-((3-(4-(difluoromethoxy)-2,3-difluorophenyl)imidazo[1,2-alpyrazin-8-yl)amino)-2-ethylbenzoyl)piperazine-1-carbonyl)-1-methylpiperidin-1-iumformate

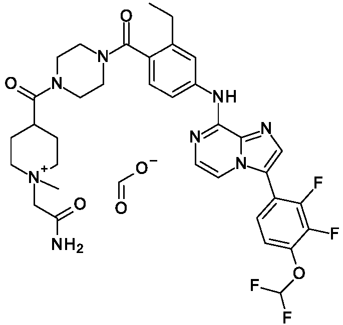

### Step 1) Intermediate 33

### 2-(4-(Difluoromethoxy)-2,3-difluorophenyl)-4,4,5,5-tetramethyl-1,3,2-dioxaborolane

1-Bromo-4-(difluoromethoxy)-2,3-difluorobenzene (1 g, 3.86 mmol, Eq: 1), 4,4,4',4',5,5,5',5'-octamethyl-2,2'-bi(1,3,2-dioxaborolane) (1.18 g, 4.63 mmol, Eq: 1.2), Pd(dppf)Cl₂ (315 mg, 386 µmol, Eq: 0.1) and potassium acetate (1.14 g, 11.6 mmol, Eq: 3) were placed in a microwave vial. The vessel was sealed, evacuated and backfilled with nitrogen for 5 times. DMSO (19.3 ml) was injected. The reaction was heated at 80 °C for 24h. The mixture was poured into 100 mL water. The aqueous phase was extracted with EtOAc (50 mL*3). The organic layers were combined, washed with brine, dried over sodium sulfate and concentrated in vacuo. The residue was purified by flash chromatography(silica gel; EtOAc:PE = 0:1 to 1:1). The title compound was obtained as light green oil (960 mg, 81% yield). 1H NMR (DMSO-d6, 400 MHz) δ 7.56 (s, 1H), 7.48 (ddd, 1H, J=2.3, 6.1, 8.5 Hz), 7.38 (s, 1H), 7.2-7.3 (m, 1H), 2.09 (s, 1H), 1.31 (s, 11H).

### Step 2) Intermediate 34

### 2-Ethyl-4-((3-iodoimidazo[1,2-a]pyrazin-8-yl)amino)benzoic acid

4-Amino-2-ethylbenzoic acid (1500 mg, 9.08 mmol, Eq: 1) and 8-chloro-3-iodoimidazo[1,2-a]pyrazine (Intermediate 1, 2.66 g, 9.53 mmol, Eq: 1.05) was suspended in MeCN (16.5 ml) and AcOH (1.65 ml). The mixture was heated in a sealed microwave tube at 100 °C for 18h and then cooled to rt. The precipitate was collected by filtration and washed with ether (30 mL*3). The cake was dried in vacuo to afford the title compond (3.6 g off white solid, 97% yield) which was used in the next step without purification. MS (ESI, m/z): 409.0 [M+H]⁺

### Step 3)

### tert-Butyl 4-(2-ethyl-4-((3-iodoimidazo[1,2-a]pyrazin-8-yl)amino)benzoyl)piperazine-1-carboxylate

2-Ethyl-4-((3-iodoimidazo[1,2-a]pyrazin-8-yl)amino)benzoic acid (500 mg, 1.22 mmol, Eq: 1), tert-butyl piperazine-1-carboxylate (274 mg, 1.47 mmol, Eq: 1.2) and HATU (559 mg, 1.47 mmol, Eq: 1.2) were suspended in DMF (3.06 ml). N-ethyl-N-isopropylpropan-2-amine (475 mg, 640 µl, 3.67 mmol, Eq: 3) was injected in one time. The stirring was continued for 1h, then 50 mL water was added. The aqueous phase was extracted with EtOAc (50 mL*3). The organic layers were washed with brine (50 mL), dried over sodium sulfate and concentrated in vacuo. The residue was purified by flash chromatography (silica gel; EtOAc:PE = 0:1 to 1:1). The title compound was obtained as light yellow foam (515 mg, 73% yield). MS (ESI, m/z): 577.1 [M+H]⁺

### Step 4) Intermediate 35

### tert-Butyl 4-(4-((3-(4-(difluoromethoxy)-2,3-difluorophenyl)imidazo[1,2-a]pyrazin-8-yl)amino)-2-ethylbenzoyl)piperazine-1-carboxylate

tert-Butyl 4-(2-ethyl-4-((3-iodoimidazo[1,2-a]pyrazin-8-yl)amino)benzoyl)piperazine-1-carboxylate (300 mg, 520 µmol, Eq: 1), 2-(4-(difluoromethoxy)-2,3-difluorophenyl)-4,4,5,5-tetramethyl-1,3,2-dioxaborolane (Intermediate 33, 159 mg, 520 µmol, Eq: 1), PdCl₂(dtbpf) (17 mg, 26 µmol, Eq: 0.05) were placed in a microwave vial. Dioxane (2.6 ml) and potassium carbonate (520 µl, 1.56 mmol, Eq: 3) were added. The vial was sealed immediatly and evacuated and backfilled with argon 5 times. The mixture was heated at 80 °C under nitrogen atmosphere for 18h. Then the mixture was cooled to rt, poured into 100 mL water and extracted with EtOAc (80 mL*3). The organic layers were combined, washed with 80 mL brine, dried over sodium sulfate, and concentrated in vacuo. The residue was purified by flash chromatography (silica gel; EtOAc:PE = 0;1 to 1:0). Rf = 0.3 (EtOAc:PE = 1:1). The title compound was obtained as light yellow foam (244 mg, 75% yield). MS (ESI, m/z): 629.2 [M]⁺

### Step 5)

### tert-Butyl 4-(4-(4-((3-(4-(difluoromethoxy)-2,3-difluorophenyl)imidazo[1,2-a]pyrazin-8-yl)amino)-2-ethylbenzoyl)piperazine-1-carbonyl)piperidine-1-carboxylate

tert-Butyl 4-(4-((3-(4-(difluoromethoxy)-2,3-difluorophenyl)imidazo[ 1,2-a]pyrazin-8-yl)amino)-2-ethylbenzoyl)piperazine-1-carboxylate (115 mg, 183 µmol, Eq: 1) was stirred in 3 mL 20% TFA/DCM (v/v) at rt for 1h. The solvent was removed in vacuo, and the residue was dissolved in dry DMF (915 µl). To this solution was added 1-(tert-butoxycarbonyl)piperidine-4-carboxylic acid (50.3 mg, 220 µmol, Eq: 1.2), N-ethyl-N-isopropylpropan-2-amine (118 mg, 159 µl, 915 µmol, Eq: 5) and HATU (83.5 mg, 220 µmol, Eq: 1.2) successively. The solution was stirred at rt for 1h. The solution was poured into 100 mL water and the aqueous phase was extracted with EtOAc (80 mL*3). The organic layers were combined, washed with 80 mL brine, dried over sodium sulfate and concentrated in vacuo. The title compound was used in the next step without purification (135 mg, quantitative yield). MS (ESI, m/z): 740.3 [M+H]⁺

### Step 6)

### (4-(4-((3-(4-(Difluoromethoxy)-2,3-difluorophenyl)imidazo[1,2-a]pyrazin-8-yl)amino)-2-ethylbenzoyl)piperazin-1-yl)(1-methylpiperidin-4-yl)methanone

tert-Butyl 4-(4-(4-((3-(4-(difluoromethoxy)-2,3-difluorophenyl)imidazo[1,2-a]pyrazin-8-yl)amino)-2-ethylbenzoyl)piperazine-1-carbonyl)piperidine-1-carboxylate (140 mg, 189 µmol, Eq: 1) was dissolved in 3 mL 20% TFA/DCM (v/v), and stirred at rt for 0.5h. The solvent was removed in vacuo, and the residue was suspended in 100 mL 1N NaOH, extracted with DCM/MeOH (v/v = 8:1) (80 mL*3). The organic layers were combined, washed with 80 mL brine, dried over sodium sulfate and concentrated in vacuo. The residue and paraformaldehyde (28.4 mg, 946 µmol, Eq: 5) were dissolved in dry THF (946 µl). To this solution was added NaBH(OAc)₃ (60.2 mg, 284 µmol, Eq: 1.5) in one portion. The mixture was stirred at rt for 18h. Silica gel was added, and the solvent was removed in vacuo to get the silica gel suported sample. The sample was purified by flash chromatography (MeOH:DCM = 0:1 to 1:7). The title compound was obtained as light yellow amorphous solid (70 mg, 56 % yield). MS (ESI, m/z): 654.3 [M+H]⁺

### Step 7)

### 1-(2-Amino-2-oxoethyl)-4-(4-(4-((3-(4-(difluoromethoxy)-2,3-difluorophenyl)imidazo[1,2-a]pyrazin-8-yl)amino)-2-ethylbenzoyl)piperazine-1-carbonyl)-1-methylpiperidin-1-ium formate

To a solution of (4-(4-((3-(4-(difluoromethoxy)-2,3-difluorophenyl)imidazo[1,2-a]pyrazin-8-yl)amino)-2-ethylbenzoyl)piperazin-1-yl)(1-methylpiperidin-4-yl)methanone (70 mg, 107 µmol, Eq: 1) in dry MeCN (535 µl) was added 2-iodoacetamide (99 mg, 535 µmol, Eq: 5) and 4 drops of DIPEA. The yellow solution was stirred for 18h. The solvent was removed in vacuo, and the residue was dissolved in 3 mL 20% TFA/DCM solution (v/v), 2-iodoacetamide (99 mg, 535 µmol, Eq: 5) and the resulting solution was stirred for 1h at rt. The solvent was removed in vacuo, and the residue was dissolved in MeCN, purified by preparative HPLC. The title compound was obtained as white amorphous freeze dried solid (34.5 mg, 42 % yield). MS (ESI, m/z): 711.2 [M]⁺

### Example 10

### N-[2-[1-(azetidin-3-ylmethyl)-1-methyl-piperidin-1-ium-4-yl]ethyl]-4-[[3-[4-(difluoromethoxy)-2,3-difluoro-phenyl]imidazo[1,2-a]pyrazin-8-yl]amino]-2-ethyl-benzamide diformate

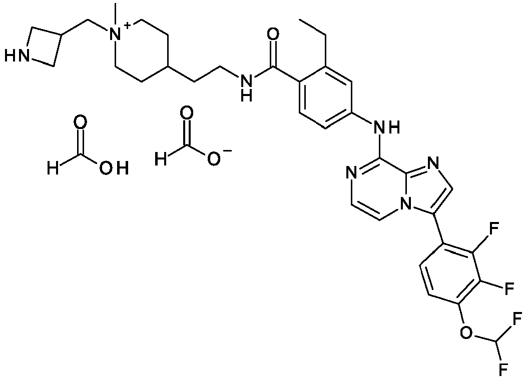

### Step 1)

### tert-butyl 4-(2-(4-((3-(4-(difluoromethoxy)-2,3-difluorophenyl)imidazo[1,2-a]pyrazin-8-yl)amino)-2-ethylbenzamido)ethyl)piperidine-1-carboxylate

In a 50 mL round-bottomed flask, 4-((3-(4-(difluoromethoxy)-2,3-difluorophenyl)imidazo[1,2-a]pyrazin-8-yl)amino)-2-ethylbenzoic acid (Intermediate 36, 80 mg, 174 µmol, Eq: 1), tert-butyl 4-(2-aminoethyl)piperidine-1-carboxylate (51.6 mg, 226 µmol, Eq: 1.3), 2-(3H-[1,2,3]triazolo[4,5-b]pyridin-3-yl)-1,1,3,3-tetramethylisouronium hexafluorophosphate(V) (85.9 mg, 226 µmol, Eq: 1.3) and DIPEA (67.4 mg, 91 µl, 521 µmol, Eq: 3) were combined with DMF (3 ml) to give a light brown solution. The reaction was stirred at room temperature for 1h. The reaction mixture was poured into 25 mL H₂O and extracted with EtOAc (3 x 25 mL).The organic layers were combined, washed with sat NaCl (1 x 25 mL), The organic layers were dried over Na₂SO₄ and concentrated in vacuo to afford the title compound (117 mg, 174 µmol, quantitative yield) which was used without further purification. MS (ESI, m/z): 671.6 [M+H]⁺

### Step 2)

### 4-((3-(4-(difluoromethoxy)-2,3-difluorophenyl)imidazo[1,2-a]pyrazin-8-yl)amino)-2-ethyl-N-(2-(piperidin-4-yl)ethyl)benzamide

In a 100 mL round-bottomed flask, tert-butyl 4-(2-(4-((3-(4-(difluoromethoxy)-2,3-difluorophenyl)imidazo[1,2-a]pyrazin-8-yl)amino)-2-ethylbenzamido)ethyl)piperidine-1-carboxylate (117 mg, 174 µmol, Eq: 1) was combined with THF (3 ml) to give a light brown solution. 12M aq HCl (1.45 ml, 17.4 mmol, Eq: 100) was added. The reaction was stirred at room temperature for 1h. The reaction mixture was concentrated in vacuo to afford the title compound (99 mg, 174 µmol, 99.5 % yield) which was used without further purification. MS (ESI, m/z): 571.3 [M+H]⁺

### Step 3)

### tert-butyl 3-((4-(2-(4-((3-(4-(difluoromethoxy)-2,3-difluorophenyl)imidazo[1,2-a]pyrazin-8-yl)amino)-2-ethylbenzamido)ethyl)piperidin-1-yl)methyl)azetidine-1-carboxylate

In a 50 mL round-bottomed flask, 4-((3-(4-(difluoromethoxy)-2,3-difluorophenyl)imidazo[1,2-a]pyrazin-8-yl)amino)-2-ethyl-N-(2-(piperidin-4-yl)ethyl)benzamide (99 mg, 174 µmol, Eq: 1), 1-(3,3-dimethylbutanoyl)azetidine-3-carbaldehyde (95.4 mg, 521 µmol, Eq: 3) and NaBH₃CN (54.5 mg, 868 µmol, Eq: 5) were combined with MeOH (6 ml) to give a light brown solution. The reaction mixture was heated to 45 °C and stirred for 2 h. The crude reaction mixture was concentrated in vacuo. The reaction mixture was poured into 25 mL H₂O and extracted with EtOAc (3 x 25 mL). The organic layers were combined, washed with sat NaCl (1 x 25 mL). The organic layers were dried over Na₂SO₄ and concentrated in vacuo to afford the title compound (128 mg, 173 µmol, 99.7 % yield) which was used without further purification. MS (ESI, m/z): 740.5 [M+H]⁺

### Step 4)

### 1-((1-(tert-butoxycarbonyl)azetidin-3-yl)methyl)-4-(2-(4-((3-(4-(difluoromethoxy)-2,3-difluorophenyl)imidazo[1,2-a]pyrazin-8-yl)amino)-2-ethylbenzamido)ethyl)-1-methylpiperidin-1-ium iodide

In a 50 mL round-bottomed flask, tert-butyl 3-((4-(2-(4-((3-(4-(difluoromethoxy)-2,3-difluorophenyl)imidazo[1,2-a]pyrazin-8-yl)amino)-2-ethylbenzamido)ethyl)piperidin-1-yl)methyl)azetidine-1-carboxylate (128 mg, 173 µmol, Eq: 1), MeI (123 mg, 54.1 µl, 865 µmol, Eq: 5) and DIPEA (112 mg, 151 µl, 865 µmol, Eq: 5) were combined with MeCN (6 ml) to give a light yellow solution. The reaction mixture was heated to 40 °C and stirred for 2 h. The reaction mixture was concentrated in vacuo to afford the title compound (131 mg, 174 µmol, quantitative yield), which was used without further purification. MS (ESI, m/z): 754.2.5 [M]⁺

### Step 5)

### N-[2-[1-(azetidin-3-ylmethyl)-1-methyl-piperidin-1-ium-4-yl]ethyl]-4-[[3-[4-(difluoromethoxy)-2,3-difluoro-phenyl]imidazo[1,2-a]pyrazin-8-yl]amino]-2-ethyl-benzamide diformate

In a 50 mL round-bottomed flask, 1-((1-(tert-butoxycarbonyl)azetidin-3-yl)methyl)-4-(2-(4-((3-(4-(difluoromethoxy)-2,3-difluorophenyl)imidazo[1,2-a]pyrazin-8-yl)amino)-2-ethylbenzamido)ethyl)-1-methylpiperidin-1-ium iodide (131 mg, 174 µmol, Eq: 1) was combined with DCM (3 ml) to give a light yellow solution. 2,2,2-trifluoroacetic acid (1.98 g, 17.4 mmol, Eq: 100) was added. The reaction was stirred at room temperature for 30min. The reaction mixture was concentrated in vacuo and the crude material was purified by preparative HPLC to afford the title compound as white powder (31 mg, 40.7 µmol, 23.5 % yield). MS (ESI, m/z): 654.3 [M]⁺

### Example 11

### 5-Aminopentyl-(carboxymethyl)-[5-[[4-[[3-(2,3-difluoro-4-methoxy-phenyl)imidazo[1,2-a]pyrazin-8-yl]amino]-2-ethyl-benzoyl]amino]pentyl]-methyl-ammonium 2,2,2-trifluoroacetate

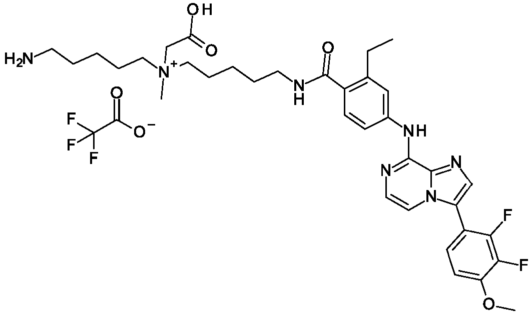

### Step 1)

### 5-(1,3-dioxoisoindolin-2-yl)pentanal

A suspension of 2-(5-hydroxypentyl)isoindoline-1,3-dione (300.0 mg, 1.29 mmol, 1 eq), tetrabutylammonium chloride (35.74 mg, 0.130 mmol, 0.100 eq), N-chlorosuccinimide (0.26 g, 1.93 mmol, 1.5 eq) and 2,2,6,6-tetramethyl-1-oxido-piperidine (20 mg, 0.1 mmol, 0.1eq) in a mixture 0.5N NaHCO₃ (4.5 eq) / 0.05N K₂CO₃ (0.5 eq) (10mL) and DCM (10mL) was stirred at room temperature for 0.5h. Then the mixture was poured into water (50mL) and extracted with DCM (30mL*2). The organic layers were dried over Na₂SO₄ and concentrated to afford the crude title compound (250 mg, 84% yield) as orange oil which was used without further purification.

### Step 2)

### 4-[[3-(2,3-difluoro-4-methoxy-phenyl)imidazo[1,2-a]pyrazin-8-yl]amino]-N-[5-[5-(1,3-dioxoisoindolin-2-yl)pentylamino]pentyl]-2-ethyl-benzamide

A mixture of N-(5-aminopentyl)-4-[[3-(2,3-difluoro-4-methoxy-phenyl)imidazo[1,2-a]pyrazin-8-yl]amino]-2-ethyl-benzamide (Intermediate 41, 250.0 mg, 0.490 mmol, 1 eq) , 5-(1,3-dioxoisoindolin-2-yl)pentanal (226.78 mg, 0.980 mmol, 1.99 eq) in Methanol (8 mL) was added sodium triacetoxyborohydride (208.37 mg, 0.980 mmol, 2 eq) and stirred at 35 °C for 16h. The mixture was concentrated and purified by prep-HPLC(FA condition) to the title compound (100 mg, 0.140 mmol, 28.1% yield) as white oil. MS (ESI, m/z): 724.2 [M+H]⁺

### Step 3)

### 4-[[3-(2,3-difluoro-4-methoxy-phenyl)imidazo[1,2-a]pyrazin-8-yl]amino]-N-[5-[5-(1,3-dioxoisoindolin-2-yl)pentyl-methyl-amino]pentyl]-2-ethyl-benzamide

A mixture of 4-[[3-(2,3-difluoro-4-methoxy-phenyl)imidazo[1,2-a]pyrazin-8-yl]amino]-N-[5-[5-(1,3-dioxoisoindolin-2-yl)pentylamino]pentyl]-2-ethyl-benzamide (100.0 mg, 0.140 mmol, 1 eq) , formaldehyde (1.0 mL, 0.280 mmol, 1.99 eq) in methanol (5 mL) was added sodium triacetoxyborohydride (58.56 mg, 0.280 mmol, 2 eq) and stirred at 35 °C for 16h. The mixture was concentrated and purified by prep-HPLC (FA condition) to afford the title compound (50 mg, 0.070 mmol, 49.05% yield) as white oil. MS (ESI, m/z): 738.2 [M+H]⁺

### Step 4)

### (2-tert-Butoxy-2-oxo-ethyl)-[5-[[4-[[3-(2,3-difluoro-4-methoxy-phenyl)imidazo[1,2-a]pyrazin-8-yl]amino]-2-ethyl-benzoyl]amino]pentyl]-[5-(1,3-dioxoisoindolin-2-yl)pentyl]-methyl-ammonium formate

A mixture of 4-[[3-(2,3-difluoro-4-methoxy-phenyl)imidazo[1,2-a]pyrazin-8-yl]amino]-N-[5-[5-(1,3-dioxoisoindolin-2-yl)pentyl-methyl-amino]pentyl]-2-ethyl-benzamide (50 mg, 1 eq) , triethylamine (0.01 mL, 0.070 mmol, 1 eq) in ACN (5 mL) was added tert-butyl bromoacetate (13.22 mg, 0.070 mmol, 1 eq) and stirred at 35 °C for 16h. The mixture was concentrated and purified by prep-HPLC (FA condition) to afford the title compound (40 mg, 0.050 mmol, 69.2% yield) as light yellow solid. MS (ESI, m/z): 852.2 [M]⁺

### Step 5)

### Carboxymethyl-[5-[[4-[[3-(2,3-difluoro-4-methoxy-phenyl)imidazo[1,2-a]pyrazin-8-yl]amino]-2-ethyl-benzoyl]amino]pentyl]-[5-(1,3-dioxoisoindolin-2-yl)pentyl]-methyl-ammonium formate

A mixture of (2-tert-butoxy-2-oxo-ethyl)-[5-[[4-[[3-(2,3-difluoro-4-methoxyphenyl)imidazo[1,2-a]pyrazin-8-yl]amino]-2-ethyl-benzoyl]amino]pentyl]-[5-(1,3-dioxoisoindolin-2-yl)pentyl]-methyl-ammonium formate (40.0 mg, 0.050 mmol, 1 eq) in DCM (5 mL) was added trifluoroacetic acid (5.0 mL, 64.9 mmol, 1384 eq) and stirred at 25 °C for 16h. The mixture was concentrated and purified by prep-HPLC (FA condition) to afford the title compound (20 mg, 0.030 mmol, 53.52% yield) as yellow oil. MS (ESI, m/z): 796.4 [M]⁺

### Step 6)

### 5-Aminopentyl-(carboxymethyl)-[5-[[4-[[3-(2,3-difluoro-4-methoxy-phenyl)imidazo[1,2-a]pyrazin-8-yl]amino]-2-ethyl-benzoyl]amino]pentyl]-methyl-ammonium 2,2,2-trifluoroacetate

A mixture of carboxymethyl-[5-[[4-[[3-(2,3-difluoro-4-methoxy-phenyl)imidazo[1,2-a]pyrazin-8-yl]amino]-2-ethyl-benzoyl]amino]pentyl]-[5-(1,3-dioxoisoindolin-2-yl)pentyl]-methyl-ammonium formate (20.0 mg, 0.030 mmol, 1 eq) in ethanol (5 mL) was added hydrazine hydrate (2.0 mL, 0.030 mmol, 1 eq) and the reaction stirred at 40 °C for 4h. The mixture was concentrated and purified by prep-HPLC (TFA condition) to afford the title compound (9.9 mg, 0.010 mmol, 46.79% yield) as white solid. MS (ESI, m/z): 666.3 [M]⁺

### Example 12

### 2-(4-(2-Chloro-4-((3-(2,3-difluoro-4-methoxyphenyl)imidazo[1,2-a]pyrazin-8-yl)amino)benzoyl)piperazin-1-yl)-N,N,N-trimethyl-2-oxoethan-1-aminium iodide

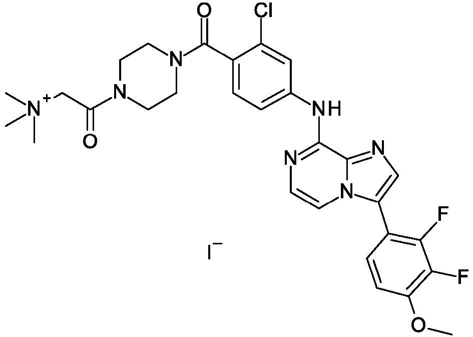

### Step 1)

### Benzyl 4-(N-(tert-butoxycarbonyl)-N-methylglycyl)piperazine-1-carboxylate

In a 25 mL round-bottomed flask, N-(tert-butoxycarbonyl)-N-methylglycine (1 g, 5.29 mmol, Eq: 1) was combined with DMF (10 ml). DIPEA (1.37 g, 1.85 ml, 10.6 mmol, Eq: 2.00) and HATU (3.01 g, 7.93 mmol, Eq: 1.50) were added and after stirring for 15 min benzyl piperazine-1-carboxylate (1.75 g, 7.93 mmol, Eq: 1.50) was added. The reaction mixture was stirred at RT for 20 h. H₂O was added to the reaction mixture and it was extracted with DCM two times. The crude was purified by flash chromatography (silica gel, 80 g, 60% to 100% EtOAc in heptane) to afford the title compound (1.54 g, 3.93 mmol, 52.1% yield) as a yellow liquid. MS (ESI, m/z): 292.3 [M+H-Boc]⁺.

### Step 2) Intermediate 24

### tert-Butyl methyl(2-oxo-2-(piperazin-1-yl)ethyl)carbamate

Benzyl 4-(N-(tert-butoxycarbonyl)-N-methylglycyl)piperazine-1-carboxylate (1.54 g, 3.93 mmol, Eq: 1) was combined with MeOH (15 ml). 10% Pd/C (41.9 mg, 393 µmol, Eq: 0.01) was added and the reaction mixture was stirred under H₂ for 4 h. The reaction mixture was put under Ar and filtered over dicalite. After removal of the volatiles, the oil obtained was dried under HV to afford the title compound (1.42 g, 5.5 mmol, 98.4% yield) as a light grey viscous oil. MS (ESI, m/z): 258.4 [M+H]⁺.

### Step 3) Intermediate 5

### 2-chloro-4-((3-(2,3-difluoro-4-methoxyphenyl)imidazo[1,2-a]pyrazin-8-yl)amino)benzoic acid

8-chloro-3-(2,3-difluoro-4-methoxyphenyl)imidazo[1,2-a]pyrazine (Intermediate 18, 330 mg, 1.12 mmol, Eq: 1) was combined with acetonitrile (8 ml) and AcOH (800 µl). 4-amino-2-chlorobenzoic acid (287 mg, 1.67 mmol, Eq: 1.5) was added and the reaction mixture was stirred at 90°C for 1 week. After cooling down to RT, ACN and AcOH were removed under vacou. Then the product was precipitated in HCl (1M). The solid was filtered, washed with HCl and dried under vacuo to afford the title compound (470.5 mg, 1.09 mmol, 93% yield) as a light brown solid. MS (ESI, m/z): 431.1 [M+H]⁺.

### Step 4)

### tert-Butyl (2-(4-(2-chloro-4-((3-(2,3-difluoro-4-methoxyphenyl)imidazo[1,2-a]pyrazin-8-yl)amino)benzoyl)piperazin-1-yl)-2-oxoethyl)(methyl)carbamate

2-Chloro-4-((3-(2,3-difluoro-4-methoxyphenyl)imidazo[1,2-a]pyrazin-8-yl)amino)benzoic acid (50 mg, 92.9 µmol, Eq: 1) was combined with DMF (1.3 ml). HATU (70.6 mg, 186 µmol, Eq: 2) and DIPEA (36 mg, 48.7 µl, 279 µmol, Eq: 3.00) were added and after stirring for 15 min, tert-butyl methyl(2-oxo-2-(piperazin-1-yl)ethyl)carbamate (35.8 mg, 139 µmol, Eq: 1.50) was added and the reaction mixture was stirred at RT for 6 h. The crude material was purified by prep HPLC to afford the title compound (42.9 mg, 64 µmol, 68.9% yield) as a light yellow waxy solid. MS (ESI, m/z): 671.3 [M+H]⁺.

### Step 5)

### 1-(4-(2-Chloro-4-((3-(2,3-difluoro-4-methoxyphenyl)imidazo[1,2-a]pyrazin-8-yl)amino)benzoyl)piperazin-1-yl)-2-(methylamino)ethan-1-one hydrochloride

Tert-butyl (2-(4-(2-chloro-4-((3-(2,3-difluoro-4-methoxyphenyl)imidazo[1,2-a]pyrazin-8-yl)amino)benzoyl)piperazin-1-yl)-2-oxoethyl)(methyl)carbamate (42.9 mg, 64 µmol, Eq: 1) was combined with 4M HCl in dioxane (160 µl, 640 µmol, Eq: 10). The reaction mixture was stirred at RT overnight. The solvent was evaporated and the residue dried under HV to afford the title compound (36.7 mg, 60.5 µmol, 94.5% yield) as a white solid. MS (ESI, m/z): 571.4 [M+H]⁺.

### Step 6)

### 2-(4-(2-Chloro-4-((3-(2,3-difluoro-4-methoxyphenyl)imidazo[1,2-a]pyrazin-8-yl)amino)benzoyl)piperazin-1-yl)-N,N,N-trimethyl-2-oxoethan-1-aminium iodide

1-(4-(2-Chloro-4-((3-(2,3-difluoro-4-methoxyphenyl)imidazo[1,2-a]pyrazin-8-yl)amino)benzoyl)piperazin-1-yl)-2-(methylamino)ethan-1-one hydrochloride (36.7 mg, 60.5 µmol, Eq: 1) was dissolved in 1,4-dioxane (386 µl) and DIPEA (39.3 mg, 53.2 µl, 304 µmol, Eq: 5.03) was added. The reaction mixture was stirred at room temperature for 15 min and iodomethane (85.9 mg, 37.7 µl, 605 µmol, Eq: 10) was added. The reaction mixture was stirred at room temperature overnight. The crude material was purified by preparative HPLC and the product was lyophilized to afford the title compound (27.6 mg, 46 µmol, 64.7% yield) as a white solid. MS (ESI, m/z): 600.5 [M+H]⁺.

### Example 13

### 2-(2-(4-((3-(2,3-difluoro-4-methoxyphenyl)imidazo[1,2-a]pyrazin-8-yl)amino)-2-ethylbenzamido)ethoxy)-N,N,N-trimethylethanaminium formate

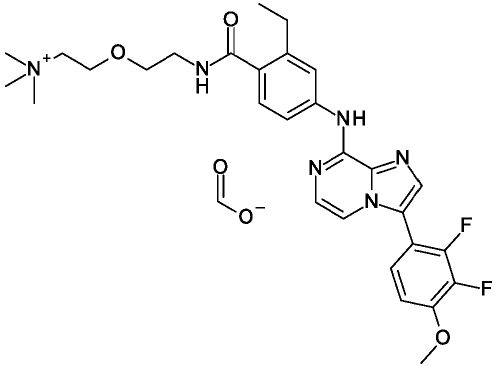

### Step 1)

### Methyl 4-amino-2-vinylbenzoate

To a microwave vial, methyl 4-amino-2-bromobenzoate (1 g, 4.35 mmol, Eq: 1), 4,4,5,5-tetramethyl-2-vinyl-1,3,2-dioxaborolane (1 g, 1.11 ml, 6.52 mmol, Eq: 1.5), sodium methoxide (470 mg, 484 µl, 8.69 mmol, Eq: 2) and tetrakis(triphenylphosphine)palladium (0) (251 mg, 217 µmol, Eq: 0.05) were added in THF (8 ml) and water (800 µl). The vial was capped and heated in the microwave at 110°C for 60 min. The reaction mixture was poured into 20 mL of H₂O and extracted with EtOAc (3 x 25 mL).The organic layers were dried over MgSO₄ and concentrated in vacuo. The crude material was purified by flash chromatography (silica gel, 12 g, 0% to 50% EtOAc in heptane) to afford the title compound (345 mg, 1.9 mmol, 44.9% yield) as an orange oil. MS (ESI, m/z): 178.2 [M+H]⁺.

### Step 2) Intermediate 7

### Methyl 4-amino-2-ethylbenzoate

Methyl 4-amino-2-vinylbenzoate (665 mg, 3.75 mmol, Eq: 1) was dissolved in MeOH (2 ml) and palladium on carbon (39.9 mg, 375 µmol, Eq: 0.1) was added. The reaction was stirred under hydrogen at room temperature for 4 h. The reaction mixture was carefully filtered under argon through celite and was concentrated in vacuo to afford the title compound (601.3 mg, 3.35 mmol, 89.4% yield) as a dark brown oil. MS (ESI, m/z): 180.2 [M+H]⁺.

### Step 3)

### Methyl 2-ethyl-4-((3-iodoimidazo[1,2-a]pyrazin-8-yl)amino)benzoate

8-chloro-3-iodoimidazo[1,2-a]pyrazine (Intermediate 1, 1.46 g, 5.23 mmol, Eq: 1) was combined with acetonitrile (30 ml). Methyl 4-amino-2-ethylbenzoate (937 mg, 5.23 mmol, Eq: 1) and acetic acid (3 ml) were added. The reaction mixture was stirred at 80 °C for 24 h. The reaction mixture was filtered and washed with MeOH and acetonitrile. The solid was dried under HV to afford the title compound (1.83 g, 4.34 mmol, 83.2% yield) as a white solid. MS (ESI, m/z): 423.1 [M+H]⁺.

### Step 4)

### Methyl 4-((3-(2,3-difluoro-4-methoxyphenyl)imidazo[1,2-a]pyrazin-8-yl)amino)-2-ethylbenzoate

Methyl 2-ethyl-4-((3-iodoimidazo[1,2-a]pyrazin-8-yl)amino)benzoate (450 mg, 1.07 mmol, Eq: 1), (2,3-difluoro-4-methoxyphenyl)boronic acid (300 mg, 1.6 mmol, Eq: 1.5), Na₂CO₃ (226 mg, 2.13 mmol, Eq: 2) and 1,1'-bis(diphenylphosphino)ferrocenedichloro palladium(II) dichloromethane complex (78 mg, 107 µmol, Eq: 0.1) were mixed in dioxane (4 ml) and water (400 µl). The vial was capped and heated in the microwave at 115°C for 30 min. The reaction mixture was poured into 20 ml of H₂O and extracted with EtOAc (3 x 30 ml). The organic layers were dried over MgSO₄ and concentrated in vacuo. The crude material was purified by flash chromatography (silica gel, 40 g, 0% to 50% EtOAc in heptane) to afford the title compound (367 mg, 0.84 mmol, 78.5% yield) as a light brown solid. MS (ESI, m/z): 439.2 [M+H]⁺.

### Step 5) Intermediate 8

### 4-((3-(2,3-difluoro-4-methoxyphenyl)imidazo[1,2-a]pyrazin-8-yl)amino)-2-ethylbenzoic acid

Methyl 4-((3-(2,3-difluoro-4-methoxyphenyl)imidazo[1,2-a]pyrazin-8-yl)amino)-2-ethylbenzoate (365 mg, 833 µmol, Eq: 1) and LiOH (1 ml, 1 mmol, Eq: 1.2) were dissolved in THF (2 ml) and MeOH (1 ml). The reaction mixture was heated to 50°C and stirred for 3 h. The reaction mixture was concentrated in vacuum and was acidified with 1M HCl. The solids were filtered through sintered glass and dried under HV for 2 h to afford the title compound (343 mg, 808 µmol, 97.2% yield) as a white solid. MS (ESI, m/z): 425.2 [M+H]⁺.

### Step 6)

### Tert-butyl (2-(2-(4-((3-(2,3-difluoro-4-methoxyphenyl)imidazo[1,2-a]pyrazin-8-yl)amino)-2-ethylbenzamido)ethoxy)ethyl)carbamate

4-((3-(2,3-difluoro-4-methoxyphenyl)imidazo[1,2-a]pyrazin-8-yl)amino)-2-ethylbenzoic acid (250 mg, 589 µmol, Eq: 1), DIPEA (305 mg, 412 µl, 2.36 mmol, Eq: 4) and HATU (336 mg, 884 µmol, Eq: 1.5) were dissolved in DMF (750 µl). The reaction mixture was stirred at room temperature for 30 minutes. Tert-butyl (2-(2-aminoethoxy)ethyl)carbamate (180 mg, 884 µmol, Eq: 1.5) was added and the reaction mixture was stirred at room temperature for 3 h. The reaction was not finished so DIPEA (152 mg, 206 µl, 1.18 mmol, Eq: 2), HATU (112 mg, 295 µmol, Eq: 0.5) and tert-butyl (2-(2-aminoethoxy)ethyl)carbamate (84.2 mg, 412 µmol, Eq: 0.7) were added again and the reaction mixture was stirred at room temperature for 30 minutes. Tert-butyl (2-(2-aminoethoxy)ethyl)carbamate (180 mg, 884 µmol, Eq: 1.5) was added and the reaction mixture was stirred at room temperature for 3 h. The crude material was purified by preparative HPLC to afford the title compound (quantitative yield) as a yellow oil. MS (ESI, m/z): 611.3 [M+H]⁺.

### Step 7) Intermediate 9

### N-(2-(2-aminoethoxy)ethyl)-4-((3-(2,3-difluoro-4-methoxyphenyl)imidazo[1,2-a]pyrazin-8-yl)amino)-2-ethylbenzamide hydrochloride

Tert-butyl (2-(2-(4-((3-(2,3-difluoro-4-methoxyphenyl)imidazo[1,2-a]pyrazin-8-yl)amino)-2-ethylbenzamido)ethoxy)ethyl)carbamate (238 mg, 390 µmol, Eq: 1) and HCl 4M in dioxane (457 mg, 381 µl, 1.52 mmol, Eq: 3.91) were combined. The reaction mixture was stirred at room temperature for 2 h. The reaction was concentrated under HV to afford the title compound (203.2 mg, 371 µmol, 95.3% yield) as a light brown solid. MS (ESI, m/z): 512.2 [M+H]⁺.

### Step 8)

### 2-(2-(4-((3-(2,3-difluoro-4-methoxyphenyl)imidazo[1,2-a]pyrazin-8-yl)amino)-2-ethylbenzamido)ethoxy)-N,N,N-trimethylethanaminium formate

N-(2-(2-aminoethoxy)ethyl)-4-((3-(2,3-difluoro-4-methoxyphenyl)imidazo[1,2-a]pyrazin-8-yl)amino)-2-ethylbenzamide hydrochloride (575 mg, 1.05 mmol, Eq: 1) was dissolved in 1,4-dioxane (5 ml) and DIPEA (1.11 g, 1.5 ml, 8.59 mmol, Eq: 8.17) was added. The reaction mixture was stirred at room temperature for 20 min and iodomethane (4.1 g, 1.8 ml, 28.9 mmol, Eq: 27.5) was added. The reaction mixture was stirred at room temperature overnight.

The crude material was purified by preparative HPLC and lyophilized to afford the title compound (117 mg, 195 µmol, 18.6% yield) as a white solid. MS (ESI, m/z): 553.3 [M]⁺.

### Example 14

### 2-[4-[2-Bromo-4-[[3-(3-fluoro-4-methoxy-phenyl)imidazo[1,2-a]pyrazin-8-yl]amino]benzoyl]piperazin-1-yl]ethyl-trimethyl-ammonium 2,2,2-trifluoroacetate

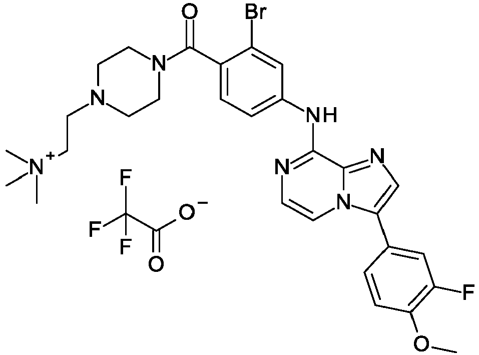

### Step 1)

### methyl 4-amino-2-bromo-benzoate

A solution of methyl 2-bromo-4-nitro-benzoate (5 g, 19.23 mmol, 1 eq), iron (6442.66 mg, 115.37 mmol, 6 eq) and ammonium chloride (1234.19 mg, 23.07 mmol, 1.2 eq) in 2-propanol (20 mL)/water (80 mL) was stirred at 100°C for 30 minutes. The mixture was cooled, filtered and concentrated under reduced pressure. The residue was partitioned between EtOAc (60 mL) and water (30 mL). The organic layer was washed with brine, dried over Na₂SO₄, filtered and concentrated to afford the title compound (4.2 g, 18.26 mmol, 94.95% yield) as a light yellow solid.

### Step 2) Intermediate 16

### Methyl 2-bromo-4-[(3-iodoimidazo[1,2-a]pyrazin-8-yl)amino]benzoate

To a solution of 8-chloro-3-iodo-imidazo[1,2-a]pyrazine (Intermediate 1, 2 g, 7.16 mmol, 1 eq) in ACN (18 mL)/acetic acid (2 mL) was added methyl 4-amino-2-bromo-benzoate (1646.4 mg, 7.16 mmol, 1 eq). The mixture was stirred at 90°C for 14 h. The mixture was filtered and the solid was washed by MeCN to afford the title compound (2.8 g, 5.92 mmol, 72.78% yield) as a white solid. MS (ESI, m/z): 472.0, 474.0 [M+H]⁺.

### Step 3)

### 2-(3-Fluoro-4-methoxy-phenyl)-4,4,5,5-tetramethyl-1,3,2-dioxaborolane

To a solution of 4-bromo-2-fluoroanisole (5 g, 24.39 mmol, 1 eq) in 1,4-dioxane (120 mL) /water (10 mL) was added [1,1'-bis(diphenylphosphino)ferrocene]dichloropalladium(II) (1784 mg, 2.44 mmol, 0.1 eq), potassium acetate (7180 mg, 73.16 mmol, 3 eq) and bis(pinacolato)diboron (6812 mg, 26.83 mmol, 1.1 eq). The mixture was degassed by N₂ for 5 min. The mixture was stirred at 100°C for 18 h. The mixture was filtered and evaporated. The residue was purified by column chromatography on silica (Petroleum ether/Ethyl acetate=1:0~20:1) to afford the title compound (4.2 g, 16.66 mmol, 68.32% yield) as a light yellow solid.

### Step 4)

### Methyl 2-bromo-4-[[3-(3-fluoro-4-methoxy-phenyl)imidazo[1,2-a]pyrazin-8-yl]amino]benzoate

To a solution of 2-(3-fluoro-4-methoxy-phenyl)-4,4,5,5-tetramethyl-1,3,2-dioxaborolane (245.13 mg, 0.970 mmol, 1.15 eq) in 1,4-dioxane (8 mL)/water (2 mL) was added methyl 2-bromo-4-[(3-iodoimidazo[1,2-a]pyrazin-8-yl)amino]benzoate (400 mg, 0.850 mmol, 1 eq), [1,1'-bis(diphenylphosphino)ferrocene]dichloropalladium(II) (61.87 mg, 0.080 mmol, 0.1 eq) and sodium hydrogen carbonate (213.1 mg, 2.54 mmol, 3 eq). The mixture was stirred at 70°C for 18 h. To the mixture was added 20 mL of THF and it was stirred for 10 min. The mixture was filtered and the organic layer was concentrated. The residue was purified by column (petroleum ether:EtOAc=5:1~1:1) to afford the title compound (280 mg, 0.590 mmol, 70.26% yield) as an off-white solid. MS (ESI, m/z): 471.0, 473.0 [M+H]⁺.

### Step 5) 2-bromo-4-[[3-(3-fluoro-4-methoxy-phenyl)imidazo[1,2-a]pyrazin-8-yl]amino]benzoic acid

A solution of methyl 2-bromo-4-[[3-(3-fluoro-4-methoxy-phenyl)imidazo[1,2-a]pyrazin-8-yl]amino]benzoate (280 mg, 0.590 mmol, 1 eq) in methanol (4 mL) was stirred at 80°C for 10 min. When the mixture was clear, sodium hydroxide (4 mL, 16 mmol, 26.93 eq) was added. The mixture was stirred at 80°C for 4 h. The reaction mixture was concentrated in vacuo, diluted with water and acidified with 2N HCl. The solid was collected and thoroughly dried to afford the title compound (240 mg, 0.520 mmol, 88.34% yield) as an off-white solid. MS (ESI, m/z): 457.0, 459.0 [M+H]⁺.

### Step 6)

### [2-Bromo-4-[[3-(3-fluoro-4-methoxy-phenyl)imidazo [1,2-a]pyrazin-8-yl]amino] phenyl]-[4-[2-(dimethylamino)ethyl]piperazin-1-yl]methanone formate

To a mixture of 1-(2-dimethylaminoethyl)piperazine (82 mg, 0.520 mmol, 1.5 eq) and 2-bromo-4-[[3-(3-fluoro-4-methoxy-phenyl)imidazo[1,2-a]pyrazin-8-yl]amino]benzoic acid (160 mg, 0.350 mmol, 1 eq) in DMF (4 mL) were added triethylamine (0.24 mL, 1.75 mmol, 5 eq) and 1-propanephosphonic anhydride (1113 mg, 1.75 mmol, 5 eq) (50% in EtOAc solution) slowly at 25°C. Then the mixture was stirred at 40 °C for 16 h. The solvent was removed and the residue was purified by prep-HPLC (FA) to afford the title compound (80 mg, 0.120 mmol, 37.56% yield) as a light yellow solid. MS (ESI, m/z): 596.0, 598.0 [M+H]⁺.

### Step 7)

### 2-[4-[2-Bromo-4-[[3-(3-fluoro-4-methoxy-phenyl)imidazo[1,2-a]pyrazin-8-yl]amino]benzoyl]piperazin-1-yl]ethyl-trimethyl-ammonium 2,2,2-trifluoroacetate

To a solution of [2-bromo-4-[[3-(3-fluoro-4-methoxy-phenyl)imidazo[1,2-a]pyrazin-8-yl]amino]phenyl]-[4-[2-(dimethylamino)ethyl]piperazin-1-yl]methanone formate? (58 mg, 0.100 mmol, 1 eq) in acetone (2 mL) was added iodomethane (83 mg, 0.590 mmol, 6 eq). The mixture was stirred at 25°C for 12 h. The solvent was removed and the residue was purified by prep-HPLC (TFA) to afford the title compound (17 mg, 0.020 mmol, 23.47% yield) as a light yellow solid. MS (ESI, m/z): 610.0, 612.0 [M]⁺.

### Example 15

### 2-[2-[[4-[[3-(2,3-Difluoro-4-prop-2-ynoxy-phenyl)imidazo[1,2-a]pyrazin-8-yl]amino]-2-ethyl-benzoyl]amino]ethoxy]ethyl-trimethyl-ammonium 2,2,2-trifluoroacetate

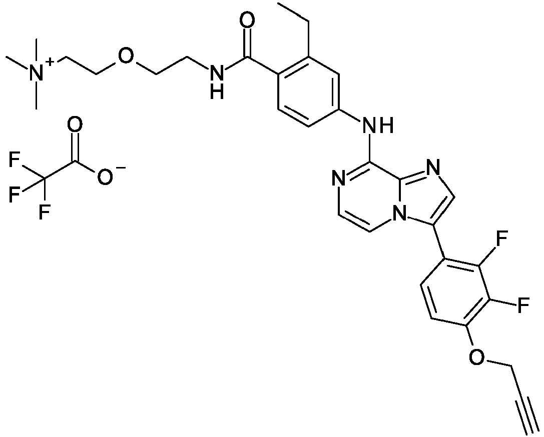

### Step 1)

### 2,3-Difluoro-4-(4,4,5,5-tetramethyl-1,3,2-dioxaborolan-2-yl)phenol

A mixture of 4-bromo-2,3-difluorophenol (6.5 g, 31.1 mmol, 1 eq), bis(pinacolato)diboron (7.9 g, 31.1 mmol, 1 eq), [1,1'-bis(diphenylphosphino)ferrocene]dichloropalladium(II) (2.28 g, 3.11 mmol, 0.1 eq) and potassium acetate (5.83 mL, 93.31 mmol, 3 eq) in 1,4-dioxane (500 mL) was stirred under N₂ at 70 °C for 2 h. The mixture was filtered and purified by silica column eluting with 10% DCM in petroleum ether to afford the title compound (3 g, 11.72 mmol, 37.67% yield) as a colorless oil.

### Step 2)

### 4-(8-Chloroimidazo[1,2-a]pyrazin-3-yl)-2,3-difluoro-phenol

To a solution of 8-chloro-3-iodo-imidazo[1,2-a]pyrazine (Intermediate 1, 546 mg, 1.95 mmol, 1 eq) and 2,3-difluoro-4-(4,4,5,5-tetramethyl-1,3,2-dioxaborolan-2-yl)phenol (500 mg, 1.95 mmol, 1 eq) in 1,4-dioxane (5 mL) and water (0.5 mL) was added 1,1'-bis(diphenylphosphino)ferrocene]dichloropalladium(II) (71 mg, 0.100 mmol, 0.05 eq) and sodium carbonate (621 mg, 5.86 mmol, 3 eq) at 20 °C. The mixture was stirred at 60 °C for 16 h. The mixture was filtered and concentrated under reduced pressure to afford the title compound (600 mg, 2.13 mmol, 94.37% yield) as a brown solid. MS (ESI, m/z): 282.1 [M+H]⁺.

### Step 3)

### 8-Chloro-3-(2,3-difluoro-4-prop-2-ynoxy-phenyl)imidazo[1,2-a]pyrazine

To a solution of 4-(8-chloroimidazo[1,2-a]pyrazin-3-yl)-2,3-difluoro-phenol (500 mg, 1.78 mmol, 1 eq) and propargyl bromide (211 mg, 1.78 mmol, 1 eq) in DMF (5 mL) was added potassium carbonate (245 mg, 1.78 mmol, 1 eq) at 20 °C. The reaction mixture was stirred at 20 °C for 16 h. The reaction was concentrated and purified by gel silica to afford the title compound (200 mg, 0.630 mmol, 35.24% yield) as a white solid. MS (ESI, m/z): 320.0 [M+H]⁺.

### Step 4)

### Methyl 4-nitro-2-vinyl-benzoate

A solution of methyl 2-bromo-4-nitro-benzoate (15 g, 57.68 mmol, 1 eq), vinylboronic acid pinacol ester (13.33 g, 86.53 mmol, 1.5 eq), phosphoric acid, tri-potassium salt (36.73 g, 173.05 mmol, 3 eq) and 1,1'-bis(di-tert-butylphosphino)ferrocene palladium dichloride (3.76 g, 5.77 mmol, 0.100 eq) in toluene (150 mL) and water (5 mL) was heated to 100 °C for 15 h under nitrogen. The reaction mixture was concentrated and the residue was purified by normal silica gel chromatography eluting with Petroluem ether/EtOAc=50:1 to afford the title compound (6.2 g, 29.93 mmol, 51.88% yield) as a light yellow solid.

### Step 5) Intermediate 22

### 4-Nitro-2-vinyl-benzoic acid

To a solution of methyl 4-nitro-2-vinyl-benzoate (2 g, 9.65 mmol, 1 eq) in THF (25 mL) and water (5 mL) was added lithium hydroxide. H₂O (0.81 g, 19.31 mmol, 2 eq). The resulting mixture was stirred at 20 °C for 15 h. Most of the solvent was removed and the mixture was extracted with MTBE (20 mL) and the water layer was neutralized with 3 N HCl, extracted with DCM (75 mL x 2), dried over sodium sulfate and concentrated to afford the title compound (1.68 g, 8.7 mmol, 90.1% yield) as a yellow solid.

### Step 6)

### tert-Butyl N-[2-[2-[(4-nitro-2-vinyl-benzoyl)amino]ethoxy]ethyl]carbamate

A solution of N-Boc-2-(2-amino-ethoxy)-ethylamine (1.06 g, 5.18 mmol, 1 eq), 4-nitro-2-vinylbenzoic acid (1 g, 5.18 mmol, 1 eq) and O-(7-azabenzotriazol-1-yl)-N,N,N',N'-tetramethyluronium hexafluorophosphate (2362 mg, 6.21 mmol, 1.2 eq) in DMF (15 mL) was stirred for 3 h at 20 °C. The solvent was removed and the residue was treated with water. The mixture was extracted with EtOAc (75 mL x 2). The organic layer was washed with brine (75 mL), dried over sodium sulfate, filtered and concentrated in vacuum to afford the title compound (1.95 g, 5.14 mmol, 99.28% yield) as a yellow oil. MS (ESI, m/z): 402.3 [M+Na]⁺.

### Step 7)

### tert-Butyl N-[2-[2-[(4-amino-2-ethyl-benzoyl)amino]ethoxy]ethyl]carbamate

To a solution of tert-butyl N-[2-[2-[(4-nitro-2-vinyl-benzoyl)amino]ethoxy]ethyl]carbamate (1.95 g, 5.14 mmol, 1 eq) in methanol (20 mL) was added 10% Pd/C : water 1:1 (547 mg, 0.510 mmol, 0.1 eq) and the mixture was stirred for 16 h at 30 °C under hydrogen atmosphere (2280 mm Hg). The solvent was removed in vacuum to afford the title compound (2.38 g, 6.77 mmol, 99.48% yield) as a light yellow oil. MS (ESI, m/z): 374.1 [M+Na]⁺.

### Step 8)

### tert-Butyl N-[2-[2-[[4-[[3-(2,3-difluoro-4-prop-2-ynoxy-phenyl)imidazo[1,2-a]pyrazin-8-yl]amino]-2-ethyl-benzoyl]amino]ethoxy]ethyl]carbamate

A mixture of 8-chloro-3-(2,3-difluoro-4-prop-2-ynoxy-phenyl)imidazo[1,2-a]pyrazine (150 mg, 0.470 mmol, 1 eq) and tert-butyl N-[2-[2-[(4-amino-2-ethyl-benzoyl)amino]ethoxy]ethyl]carbamate (198 mg, 0.560 mmol, 1.2 eq) in ACN (4.5 mL) and acetic acid (0.5 mL) was heated to 80 °C for 15 h. The mixture was purified by flash column to afford the title compound (170 mg, 0.270 mmol, 57.09% yield) as a white solid. MS (ESI, m/z): 635.5 [M+H]⁺.

### Step 9)

### N-[2-(2-Aminoethoxy)ethyl]-4-[[3-(2,3-difluoro-4-prop-2-ynoxy-phenyl)imidazo[1,2-a]pyrazin-8-yl]amino]-2-ethyl-benzamide

To a solution of tert-butyl N-[2-[2-[[4-[[3-(2,3-difluoro-4-prop-2-ynoxy-phenyl)imidazo[1,2-a]pyrazin-8-yl]amino]-2-ethyl-benzoyl]amino]ethoxy]ethyl]carbamate (150 mg, 0.240 mmol, 1 eq) in methanol (5 mL) was added hydrogen chloride in MeOH (6.25 mL, 25 mmol, 105.78 eq). The resulting mixture was stirred at 25 °C for 4 h. The residue was purified by prep-HPLC (FA) to afford the title compound (92 mg, 0.170 mmol, 72.38% yield) as a white solid. MS (ESI, m/z): 535.5 [M+H]⁺.

### Step 10)

### 2-[2-[[4-[[3-(2,3-Difluoro-4-prop-2-ynoxy-phenyl)imidazo[1,2-a]pyrazin-8-yl]amino]-2-ethyl-benzoyl]amino]ethoxy]ethyl-trimethyl-ammonium 2,2,2-trifluoroacetate

To a solution of N-[2-(2-aminoethoxy)ethyl]-4-[[3-(2,3-difluoro-4-prop-2-ynoxy-phenyl)imidazo[1,2-a]pyrazin-8-yl]amino]-2-ethyl-benzamide (70 mg, 0.130 mmol, 1 eq) in DMF (3 mL) was added iodomethane (372 mg, 2.62 mmol, 20 eq). The resulting mixture was stirred at 25 °C for 48 h. The mixture was concentrated under reduced pressure and purified by prep-HPLC (TFA) to afford the title compound (11.9 mg, 0.020 mmol, 12.84% yield) as a colorless oil. MS (ESI, m/z): 577.1 [M+H]⁺.

### Example 16

### 2-[2-[6-[[3-(2,3-difluoro-4-methoxy-phenyl)imidazo[1,2-a]pyrazin-8-yl]amino]-1-oxo-3,4-dihydroisoquinolin-2-yl]ethoxy]ethyl-trimethyl-ammoniumformate

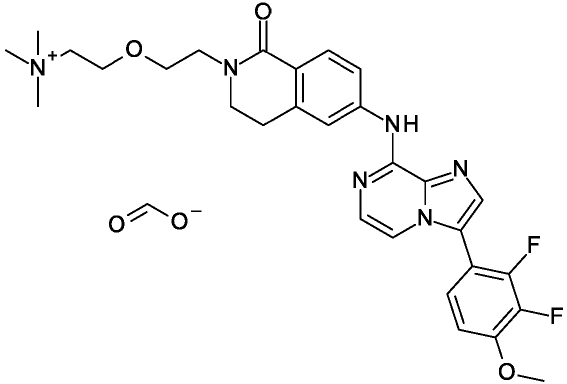

### Step 1)

### tert-Butyl N-[2-(2-hydroxyethoxy)ethyl]carbamate

To a mixture of 2-(2-aminoethoxy)ethanol (3000 mg, 28.53 mmol, 1 eq) in THF (60 mL) and sodium hydrogen carbonate in water (15 mL, 28.53 mmol, 1 eq) was added di-t-butyldicarbonate (6227 mg, 28.53 mmol, 1 eq) at 0 °C. The mixture was stirred for 4 h. The mixture was washed with 1M HCl (5 mL), then washed with brine, dried over Na₂SO₄, filtered and concentrated to afford the title compound (5000 mg, 24.36 mmol, 85.38% yield) as a white oil.

### Step 2)

### 2-[2-(tert-Butoxycarbonylamino)ethoxy]ethyl methanesulfonate

To a mixture of 2-(2-BOC-aminoethoxy)ethanol (3000 mg, 14.62 mmol, 1 eq) in DCM (20 mL) and methanesulfonyl chloride (2.03 mL, 26.19 mmol, 1.79 eq) was added triethylamine (4.07 mL, 29.23 mmol, 2 eq) at 0 °C. The mixture was stirred for 4 h. The mixture was quenched with aqueous NH₄Cl and extracted with AcOEt (10 mL x 2). The organic phase was concentrated and purified by column (PE:EA=5:1) to afford the title compound (2800 mg, 9.88 mmol, 67.61% yield) as a white oil.

### Step 3)

### tert-Butyl N-[2-[2-(6-bromo-1-oxo-3,4-dihydroisoquinolin-2-yl)ethoxy]ethyl]carbamate

To a mixture of 6-bromo-3,4-dihydro-2H-isoquinolin-1-one (399 mg, 1.76 mmol, 1 eq) in THF (10 mL) was added sodium hydride (105 mg, 2.65 mmol, 1.5 eq) at 0°C. The mixture was stirred at 25°C for 20 minutes. 2-[2-(tert-butoxycarbonylamino)ethoxy]ethyl methanesulfonate (500 mg, 1.76 mmol, 1 eq) was added and the mixture was stirred at 60°C for 16 h. The reaction mixture was poured into aqueous NH₄Cl (saturated, 50 mL). It was extracted by AcOEt (50 mL x 3), the combined layer was washed by brine (50 mL), dried with Na₂SO₄, filtered and concentrated. The residue was purified via prep-HPLC (FA), then lyophilized to afford the title compound (350 mg, 0.850 mmol, 47.99% yield) as a yellow solid. MS (ESI, m/z): 413.0, 415.0 [M+H]⁺.

### Step 4)

### 2-[2-(2-Aminoethoxy)ethyl]-6-bromo-3,4-dihydroisoquinolin-1-one

To a mixture of tert-butyl N-[2-[2-(6-bromo-1-oxo-3,4-dihydroisoquinolin-2-yl)ethoxy]ethyl]carbamate (120 mg, 0.290 mmol, 1 eq) in DCM (1 mL) was added trifluoroacetic acid (0.2 mL, 2.6 mmol, 8.94 eq) at 0°C. The mixture was stirred at 25°C for 1 h. The reaction mixture was concentrated to afford the title compound (90 mg, 0.290 mmol, 98.98% yield) as a colorless oil. MS (ESI, m/z): 313.0, 315.0 [M+H]⁺.

### Step 5)

### 2-[2-(6-bromo-1-oxo-3,4-dihydroisoquinolin-2-yl)ethoxy]ethyl-trimethyl-ammonium formate

To a mixture of 2-[2-(2-aminoethoxy)ethyl]-6-bromo-3,4-dihydroisoquinolin-1-one (90 mg, 0.290 mmol, 1 eq) and potassium carbonate (317.73 mg, 2.3 mmol, 8 eq) in acetonitrile (1 mL) was added methyl iodide (244.73 mg, 1.72 mmol, 6 eq) at 25°C. The mixture was stirred at 25°C for 16 h. Water (2 mL) was added and the mixture was purified via prep-HPLC (FA), then lyophilized to afford the title compound (80 mg, 0.220 mmol, 78.14% yield) as a white solid. MS (ESI, m/z): 355.0, 357.0 [M]⁺.

### Step 6) Intermediate 18

### 8-chloro-3-(2,3-difluoro-4-methoxy-phenyl)imidazo[1,2-a]pyrazine

To a solution of 8-chloro-3-iodo-imidazo[1,2-a]pyrazine (Intermediate 1, 2000 mg, 7.16 mmol, 1 eq) in 1,4-dioxane (50 mL)/water (12 mL) was added 2-(2,3-difluoro-4-methoxy-phenyl)-4,4,5,5-tetramethyl-1,3,2-dioxaborolane (Intermediate 17, 2512.64 mg, 9.3 mmol, 1.3 eq), [1,1'-bis(diphenylphosphino)ferrocene]dichloropalladium(II) (523.64 mg, 0.720 mmol, 0.1 eq) and sodium carbonate (2275.48 mg, 21.47 mmol, 3 eq). The mixture was stirred at 70°C for 18 h. The mixture was filtered and concentrated. The residue was purified by column chromatography on silica (Petroleum ether/Ethyl acetate=10:1~1:1) to afford the title compound (2 g, 6.76 mmol, 94.52% yield) as a light grey solid. MS (ESI, m/z): 296.0 [M+H]⁺.

### Step 7)

### 3-(2,3-difluoro-4-methoxy-phenyl)imidazo[1,2-a]pyrazin-8-amine

In a sealed tube to a solution of 8-chloro-3-(2,3-difluoro-4-methoxy-phenyl)imidazo[1,2-a]pyrazine (500 mg, 1.69 mmol, 1 eq) in 1,4-dioxane (5 mL) was added ammonium hydroxide (10 mL). The mixture was stirred at 100°C for 20 h. The solvent was removed to afford the title compound (495 mg, 1.79 mmol, 105.96% yield) as a crude light yellow solid. MS (ESI, m/z): 277.0 [M+H]⁺.

### Step 8)

### 2-[2-[6-[[3-(2,3-Difluoro-4-methoxy-phenyl)imidazo[1,2-a]pyrazin-8-yl]amino]-1-oxo-3,4-dihydroisoquinolin-2-yl]ethoxy]ethyl-trimethyl-ammonium formate

To a mixture of 2-[2-(6-bromo-1-oxo-3,4-dihydroisoquinolin-2-yl)ethoxy]ethyl-trimethyl-ammonium formate (40 mg, 0.110 mmol, 1 eq), 3-(2,3-difluoro-4-methoxy-phenyl)imidazo[1,2-a]pyrazin-8-amine (31.01 mg, 0.110 mmol, 1 eq) and potassium carbonate (46.55 mg, 0.340 mmol, 3 eq) in tert-butanol (1 mL) was added Brettphos Pd G3 (9.26 mg, 0.010 mmol, 0.1 eq) under N₂ at 25°C. The mixture was stirred at 130°C for 10 h in the microwave. The reaction mixture was filtered and concentrated. The residue was purified via prep-HPLC (FA), then lyophilized to afford the title compound (10 mg, 0.020 mmol, 14.41% yield) as a white solid. MS (ESI, m/z): 551.1 [M]⁺.

### Example 17

### 4-[2-[2-[[2-chloro-4-[[3-[4-(cyanomethoxy)-2,3-difluoro-phenyl]imidazo[1,2-a]pyrazin-8-yl]amino]benzoyl]amino]ethoxy]ethyl]-4-methyl-piperazin-4-ium-2-carboxylic acid formate

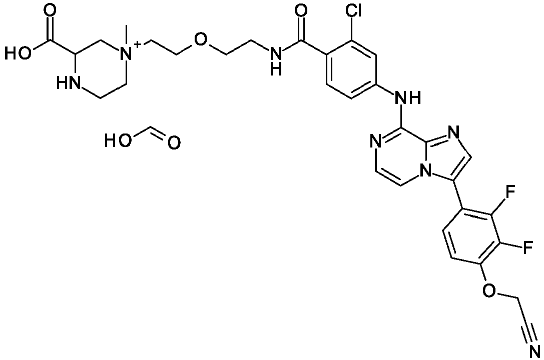

### Step 1)

### 2-chloro-N-[2-(2-hydroxyethoxy)ethyl]-4-[(3-iodoimidazo[1,2-a]pyrazin-8-yl)amino]benzamide

A mixture of 2-chloro-4-[(3-iodoimidazo[1,2-a]pyrazin-8-yl)amino]benzoic acid (Intermediate 26, 1.5 g, 3.62 mmol, 1 eq), O-(7-azabenzotriazol-1-yl)-N,N,N,N-tetramethyluronium hexafluorophosphate (2.75 g, 7.24 mmol, 2 eq)and triethylamine (1.51 mL, 10.85 mmol, 3 eq) in DMF (50 mL) was stirred at 25 °C for 0.5 hours. 2-(2-aminoethoxy)ethanol (0.57 g, 5.43 mmol, 1.5 eq) was added and the mixture was stirred at 25 °C for 16 hours. The reaction mixture was poured into water (200 mL), it was extracted with EA (100 mL x 3), the combined organic layers were washed with brine (100 mL x2), dried with Na₂SO₄, filtered and conentrated. The residue was purified via prep-HPLC(FA), then lyophilization to afford the title compound (0.800 g, 1.59 mmol, 38.93% yield) as a white solid. MS (ESI, m/z): 501.9, 503.9 [M+H]⁺

### Step 2)

### 2-[2-[[2-Chloro-4-[(3-iodoimidazo[1,2-a]pyrazin-8-yl)amino]benzoyl]amino]ethoxy]ethyl methanesulfonate

To a mixture of 2-chloro-N-[2-(2-hydroxyethoxy)ethyl]-4-[(3-iodoimidazo[1,2-a]pyrazin-8-yl)amino]benzamide (0.8 g, 1.59 mmol, 1 eq)and triethylamine (0.44 mL, 3.19 mmol, 2 eq) in DCM (15 mL) was added dropwise methylsufonyl chloride (0.27 g, 2.39 mmol, 1.5 eq) at 0°C under N2. The mixture was stired at 25°C for 16 hours. The reaction mixture was poured into water (30 mL), it was extracted with DCM (30 x 3 mL), the combined organic layer were washed with brine (30 mL x 2), dried by Na₂SO₄, filtered and concentrated. The residue was purified via prep-HPLC (FA), then lyophilization to afford the title compound (600 mg, 1.03 mmol, 64.9% yield) as a yellow solid. MS (ESI, m/z): 579.8, 581.8 [M+H]⁺

### Step 3)

### O1-tert-butyl O2-methyl 4-[2-[2-[[2-chloro-4-[(3-iodoimidazo[1,2-a]pyrazin-8-yl)amino]benzoyl]amino]ethoxy]ethyl]piperazine-1,2-dicarboxylate

A mixture of 2-[2-[[2-chloro-4-[(3-iodoimidazo[1,2-a]pyrazin-8-yl)amino]benzoyl]amino]ethoxy]ethyl methanesulfonate (300.0 mg, 0.520 mmol, 1 eq), 1-N-BOC-piperazine-2-carboxylic acid methyl ester (189.6 mg, 0.780 mmol, 1.5 eq) and N,N-diisopropylethylenediamine (223.93 mg, 1.55 mmol, 3 eq) in DMSO (10 mL) was stirred at 100°C for 16 hours. The reaction mixture was purified directly via prep-HPLC (FA), then lyophilization to afford the title compound (200 mg, 0.270 mmol, 44.59% yield) as a yellow solid. MS (ESI, m/z): 728.0, 730.0 [M+H]⁺

### Step 4)

### O1-tert-butyl O2-methyl 4-[2-[2-[[2-chloro-4-[[3-[4-(cyanomethoxy)-2,3-difluoro-

### phenyl]imidazo[1,2-a]pyrazin-8-yl]amino]benzoyl]amino]ethoxy]ethyl]piperazine-1,2-dicarboxylate

To a mixture of 2-[2,3-difluoro-4-(4,4,5,5-tetramethyl-1,3,2-dioxaborolan-2-yl)phenoxy]acetonitrile (Intermediate 15, 89.18 mg, 0.300 mmol, 1.1 eq), O1-tert-butyl O2-methyl 4-[2-[2-[[2-chloro-4-[(3-iodoimidazo[1,2-a]pyrazin-8-yl)amino]benzoyl]amino]ethoxy]ethyl]piperazine-1,2-dicarboxylate (200.0 mg, 0.270 mmol, 1 eq) and sodium bicarbonate (69.24 mg, 0.820 mmol, 3 eq) in 1,4-dioxane (5 mL) and water (1 mL) was added [1,1'-bis(diphenylphosphino)ferrocene]dichloropalladium(II) (20.1 mg, 0.030 mmol, 0.100 eq) at 25°C under N2. The mixture was stirred at 60°C for 2 hours. The reaction mixture was filtered and concentrated. The residue was purified via prep-HPLC(FA), then lyophilization to the title compound (130 mg, 0.170 mmol, 61.52% yield) as a white solid. MS (ESI, m/z): 769.1, 771.1 [M+H]⁺

### Step 5)

### O1-tert-butyl O2-methyl 4-[2-[2-[[2-chloro-4-[[3-[4-(cyanomethoxy)-2,3-difluorophenyl]imidazo[1,2-a]pyrazin-8-yl]amino]benzoyl]amino]ethoxy]ethyl]-4-methyl-piperazin-4-ium-1,2-dicarboxylate iodide

To a mixture of methyl 2-[tert-butoxycarbonyl(ethyl)amino]-3-[2-[2-[[2-chloro-4-[[3-[4-(cyanomethoxy)-2,3-difluoro-phenyl]imidazo[1,2-a]pyrazin-8-yl]amino]benzoyl]amino]ethoxy]ethyl-methyl-amino]propanoate (100.0 mg, 0.130 mmol, 1 eq)inacetonitrile (3 mL) was added dropwise methyl iodide (0.1 mL, 2.55 mmol, 20 eq) at 25°C under N2. The reaction mixture was purified directly via prep-HPLC, then lyophilization to afford the title compound (100 mg, 0.130 mmol, quant. yield) as a white solid. MS (ESI, m/z): 783.0, 785.0 [M]⁺

### Step 6)

### 4-[2-[2-[[2-chloro-4-[[3-[4-(cyanomethoxy)-2,3-difluoro-phenyl]imidazo[1,2-a]pyrazin-8-yl]amino]benzoyl]amino]ethoxy]ethyl]-4-methyl-piperazin-4-ium-2-carboxylic acid formate

A mixture of O1-tert-butyl O2-methyl 4-[2-[2-[[2-chloro-4-[[3-[4-(cyanomethoxy)-2,3-difluorophenyl]imidazo[1,2-a]pyrazin-8-yl]amino]benzoyl]amino]ethoxy]ethyl]-4-methyl-piperazin-4-ium-1,2-dicarboxylate iodide (40.0 mg, 0.050 mmol, 1 eq), lithium chloride (43.25 mg, 1.02 mmol, 20 eq) and triethylamine (0.1 mL, 0.720 mmol, 14.07 eq) in DMF (0.500 mL) was stirred at 100°C under N2 for 5 h. The reaction mixture was purified directly via prep-HPLC(FA), then lyophilization to afford the title compound (3.2 mg, 0 mmol, 8.39% yield) as a brown solid. MS (ESI, m/z): 669.0, 671.0 [M]⁺

### Example 18

### 2-(2-(4-((3-(2,3-Difluoro-4-methoxyphenyl)imidazo[1,2-a]pyrazin-8-yl)amino)-2-fluoro-6-methylbenzamido)ethoxy)-N,N,N-trimethylethan-1-aminium hydrogen carbonate

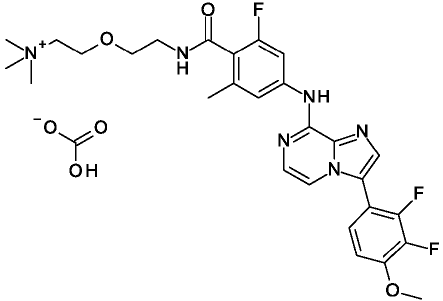

### Step 1)

### tert-Butyl 4-bromo-2-fluoro-6-methylbenzoate

In a pressure tube, 4-bromo-2-fluoro-6-methylbenzoic acid (700 mg, 3 mmol, Eq: 1) was suspended in dry toluene (1.88 ml) and N,N-dimethylformamide di-tert-butyl acetal (4.41 g, 5.19 ml, 19.5 mmol, Eq: 6.5) was added. The tube was sealed and the mixture heated to 80°C for 3 h. The reaction mixture was diluted with water, ethyl acetate and saturated aqueous NaHCO₃. The mixture was extracted with ethyl acetate and the organic layer was washed with saturated aqueous NaHCO₃ and brine. The combined organic layers were dried with sodium sulfate, filtered and concentrated in vacuo. The crude material was purified by silica gel chromatography (40 g column prepacked / gradient: Heptane, 0% to 5% ethyl acetate) to afford the title compound (794.9 mg, 2.69 mmol, 89.7% yield) as a colorless oil. MS (ESI, m/z): 290.0 [M+H]⁺.

### Step 2)

### 3-(2,3-Difluoro-4-methoxyphenyl)imidazo[1,2-a]pyrazin-8-amine

In a sealed pressure tube, a suspension of 8-chloro-3-(2,3-difluoro-4-methoxyphenyl)imidazo[1,2-a]pyrazine (Intermediate 18, 1.5 g, 5.07 mmol, Eq: 1) in isopropanol (20.3 ml) and 25% aqueous ammonia (31.8 g, 35.3 ml, 467 mmol, Eq: 92) was heated to 100°C for 2 days. The reaction mixture was diluted with water and the suspension was filtered and washed with water. The solid was collected and dried in vacuo to afford the title compound (1.23 g, 4.45 mmol, 87.8% yield) as a brown solid. MS (ESI, m/z): 277.1 [M+H]⁺.

### Step 3)

### tert-Butyl 4-((3-(2,3-difluoro-4-methoxyphenyl)imidazo[1,2-a]pyrazin-8-yl)amino)-2-fluoro-6-methylbenzoate

A suspension of 3-(2,3-difluoro-4-methoxyphenyl)imidazo[1,2-a]pyrazin-8-amine (300 mg, 1.09 mmol, Eq: 1), tert-butyl 4-bromo-2-fluoro-6-methylbenzoate (628 mg, 2.17 mmol, Eq: 2) and sodium tert-butoxide (157 mg, 1.63 mmol, Eq: 1.5) in THF (10.9 ml) in a pressure tube was sparged with argon for 5 minutes while sonicating in an ultra sonic bath. Then 1,1'-bis(diphenylphosphino)ferrocene (72.2 mg, 130 µmol, Eq: 0.12) and tris(dibenzylideneacetone)dipalladium (0) (39.8 mg, 43.4 µmol, Eq: 0.04) were added and the degassing was continued for 1 minute. The tube was sealed and heated to 130°C for 3 h.

The mixture was concentrated in vacuo. The crude material was purified by silica gel chromatography (70 g column prepacked, gradient: heptane, 0% to 60% ethyl acetate) to afford the title compound (367 mg, 758 µmol, 69.8% yield) as a yellow solid. MS (ESI, m/z): 485.2 [M+H]⁺.

### Step 4)

### 4-((3-(2,3-Difluoro-4-methoxyphenyl)imidazo[1,2-a]pyrazin-8-yl)amino)-2-fluoro-6-methylbenzoic acid hydrochloride

To a solution of tert-butyl 4-((3-(2,3-difluoro-4-methoxyphenyl)imidazo[1,2-a]pyrazin-8-yl)amino)-2-fluoro-6-methylbenzoate (367 mg, 758 µmol, Eq: 1) in dioxane (1.52 ml) was added 4M HCl in dioxane (11.4 ml, 45.5 mmol, Eq: 60) and the reaction was heated to 70°C for 4 h. The mixture was diluted with dioxane and concentrated in vacuo to afford the title compound (410 mg, 750 µmol, 99% yield) as a light brown solid. MS (ESI, m/z): 429.2 [M+H]⁺.

### Step 5)

### 4-((3-(2,3-Difluoro-4-methoxyphenyl)imidazo[1,2-a]pyrazin-8-yl)amino)-N-(2-(2-(dimethylamino)ethoxy)ethyl)-2-fluoro-6-methylbenzamide

To a solution of 4-((3-(2,3-difluoro-4-methoxyphenyl)imidazo[1,2-a]pyrazin-8-yl)amino)-2-fluoro-6-methylbenzoic acid hydrochloride (90 mg, 194 µmol, Eq: 1) and DIPEA (125 mg, 169 µl, 968 µmol, Eq: 5) in dry DMF (861 µl) was added HATU (73.6 mg, 194 µmol, Eq: 1) and the mixture was stirred at RT for 10 minutes. Then 2-(2-aminoethoxy)-N,N-dimethylethan-1-amine (38.4 mg, 290 µmol, Eq: 1.5) in dry DMF (430 µl) was added and the reaction mixture was stirred at RT for 1 h. The mixture was concentrated in vacuo. The crude material was purified by silica gel chromatography (4 g column prepacked, gradient: Heptan, 10% to 80% EtOAc/EtOH/NH₄OH 75:25:2) to afford the title compound (83.8 mg, 148 µmol, 76.6% yield) as an off-white solid. MS (ESI, m/z): 543.3 [M+H]⁺.

### Step 6)

### 2-(2-(4-((3-(2,3-Difluoro-4-methoxyphenyl)imidazo[1,2-a]pyrazin-8-yl)amino)-2-fluoro-6-methylbenzamido)ethoxy)-N,N,N-trimethylethan-1-aminium hydrogen carbonate

To a solution of 4-((3-(2,3-difluoro-4-methoxyphenyl)imidazo[1,2-a]pyrazin-8-yl)amino)-N-(2-(2-(dimethylamino)ethoxy)ethyl)-2-fluoro-6-methylbenzamide (38.2 mg, 70.4 µmol, Eq: 1) in dioxane (704 µl) was added a solution of iodomethane (12 mg, 5.26 µl, 84.5 µmol, Eq: 1.2) in dioxane (704 µl). The reaction mixture was stirred at RT overnight. The reaction mixture was concentrated in vacuo. Then the residue was dissolved in methanol and dichloromethane and filtrated through an ion exchange column Agilent Stratosphere SPE-HCO3- (exchange of the iodine to the hydrogene carbonate) and it was washed with methanol. The filtrate was concentrated in vacuo to afford the title compound (43.1 mg, 66.2 µmol, 94% yield) as a yellow solid. MS (ESI, m/z): 557.2526 [M]⁺.

### Example 19

### 4-(2-(4-((3-(4-(difluoromethoxy)phenyl)imidazo[1,2-a]pyrazin-8-yl)amino)-N,2-dimethylbenzamido)ethyl)-1,1-dimethylpiperidin-1-ium formate

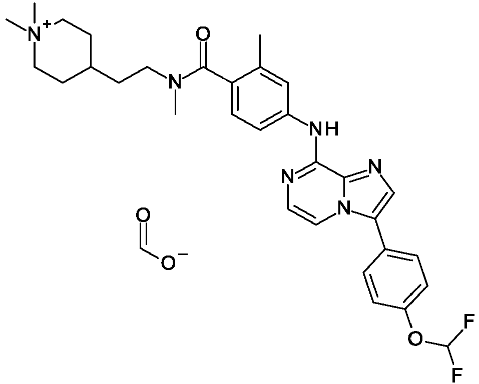

### Step 1)

### Tert-butyl 4-(2-(4-((3-(4-(difluoromethoxy)phenyl)imidazo[1,2-a]pyrazin-8-yl)amino)-N,2-dimethylbenzamido)ethyl)piperidine-1-carboxylate

4-((3-(4-(difluoromethoxy)phenyl)imidazo[1,2-a]pyrazin-8-yl)amino)-2-methylbenzoic acid (Intermediate 2, 500 mg, 1.22 mmol, Eq: 1) was combined with DMF (5 ml). Tert-butyl 4-(2-(methylamino)ethyl)piperidine-1-carboxylate (369 mg, 1.52 mmol, Eq: 1.25), DIPEA (472 mg, 638 µl, 3.66 mmol, Eq: 3.00) and HATU (695 mg, 1.83 mmol, Eq: 1.50) were added and the reaction mixture was stirred overnight at room temperature. Tert-butyl 4-(2-(methylamino)ethyl)piperidine-1-carboxylate (207 mg, 853 µmol, Eq: 0.70) was added again and the reaction was stirred overnight at room temperature. DMF was removed and the extraction was done with DCM and saturated ammonium chloride solution. After washing the organic layer with water and brine and drying over Na₂SO₄, the solution was evaporated and the crude material was purified by flash chromatographies (silica gel, 50 g, 0% to 10% MeOH in DCM) to afford the title compound (704.3 mg, 1.11 mmol, 91.1% yield) as a light brown foam. MS (ESI, m/z): 635.6 [M+H]⁺.

### Step 2) Intermediate 3

### 4-((3-(4-(difluoromethoxy)phenyl)imidazo[1,2-a]pyrazin-8-yl)amino)-N,2-dimethyl-N-(2-(piperidin-4-yl)ethyl)benzamide hydrochloride

Tert-butyl 4-(2-(4-((3-(4-(difluoromethoxy)phenyl)imidazo[1,2-a]pyrazin-8-yl)amino)-N,2-dimethylbenzamido)ethyl)piperidine-1-carboxylate (700 mg, 1.1 mmol, Eq: 1) was combined with HCl 4M in dioxane (4.14 ml, 16.5 mmol, Eq: 15) and it was stirred for 1 h at room temperature. MeOH was added to dissolve the suspension and the volatiles were removed. The solid obtained was triturated with ether, then filtered and dried under HV to afford the title compound (633 mg, 1.11 mmol, 95.6% yield) as a light brown solid. MS (ESI, m/z): 535.4 [M+H]⁺.

### Step 3)

### 4-(2-(4-((3-(4-(difluoromethoxy)phenyl)imidazo[1,2-a]pyrazin-8-yl)amino)-N,2-dimethylbenzamido)ethyl)-1,1-dimethylpiperidin-1-ium formate

4-((3-(4-(difluoromethoxy)phenyl)imidazo[1,2-a]pyrazin-8-yl)amino)-N,2-dimethyl-N-(2-(piperidin-4-yl)ethyl)benzamide hydrochloride (30 mg, 52.5 µmol, Eq: 1) was dissolved in 1,4-dioxane (2 ml) and DIPEA (14.8 mg, 20 µL, 115 µmol, Eq: 2.18) was added. The reaction mixture was stirred at room temperature for 20 min and iodomethane (30 µL) was added. The reaction mixture was stirred at room temperature overnight. The crude material was purified by preparative HPLC to afford the title compound (22.4 mg, 36.8 µmol, 70.1% yield). MS (ESI, m/z): 563.3 [M]⁺.

### Example 20

### 1-ethyl-4-(2-(4-((3-(4-methoxyphenyl)imidazo[1,2-a]pyrazin-8-yl)amino)-2-methylbenzamido)ethyl)-1-methylpiperidin-1-ium formate

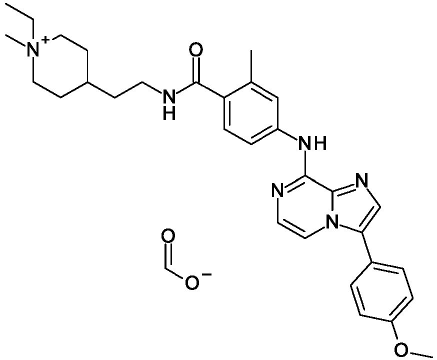

### Step 1)

### 8-chloro-3-(4-methoxyphenyl)imidazo[1,2-a]pyrazine

8-chloro-3-iodoimidazo[1,2-a]pyrazine (Intermediate 1, 2 g, 7.16 mmol, Eq: 1) was combined with dioxane (22 ml) and water (11 ml). 2-(4-methoxyphenyl)-4,4,5,5-tetramethyl-1,3,2-dioxaborolane (2.01 g, 8.59 mmol, Eq: 1.20), Na₂CO₃ (1.52 g, 14.3 mmol, Eq: 2.00) and 1,1'-bis(diphenylphosphino)ferrocenedichloro palladium(II) dichloromethane complex (262 mg, 358 µmol, Eq: 0.05) were added and the reaction mixture was heated at 80°C and stirred overnight. The reaction mixture was diluted with AcOEt and water (2:1) and the organic layer was separated. The aqueous layer was extracted with EtOAc several times. The combined organic layers were washed with brine, dried over Na₂SO₄ and concentrated in vacuo. The brown solid obtained was purified by flash chromatography (silica gel, 80 g, 0% to 80% EtOAc in heptane) to afford the title compound (1.31 g, 5.04 mmol, 70.5% yield) as a light yellow solid. MS (ESI, m/z): 260.1 [M+H]⁺.

### Step 2)

### 4-((3-(4-methoxyphenyl)imidazo[1,2-a]pyrazin-8-yl)amino)-2-methylbenzoic acid

8-chloro-3-(4-methoxyphenyl)imidazo[1,2-a]pyrazine (1.31 g, 5.04 mmol, Eq: 1) and 4-amino-2-methylbenzoic acid (915 mg, 6.05 mmol, Eq: 1.20) were combined with acetonitrile (21 ml). The reaction mixture was stirred at 90°C for 3 days. After cooling down to RT, acetonitrile and methanol (1:1) was added to the suspension and the solid was filtered off to afford the title compound (1.6 g, 4.27 mmol, 84.7% yield) as a light yellow solid. MS (ESI, m/z): 375.2 [M+H]⁺.

### Step 3) Intermediate 4

### 4-((3-(4-methoxyphenyl)imidazo[1,2-a]pyrazin-8-yl)amino)-2-methyl-N-(2-(1-methylpiperidin-4-yl)ethyl)benzamide

4-((3-(4-methoxyphenyl)imidazo[1,2-a]pyrazin-8-yl)amino)-2-methylbenzoic acid (800 mg, 2.14 mmol, Eq: 1) was combined with DMF (8 ml). 2-(1-methylpiperidin-4-yl)ethanamine (456 mg, 3.21 mmol, Eq: 1.50), DIPEA (828 mg, 1.12 ml, 6.41 mmol, Eq: 3.00) and HATU (1.22 g, 3.21 mmol, Eq: 1.50) were added and the reaction mixture was stirred at RT for 1 h. The reaction mixture was directly submitted to prep HPLC for purification to afford the title compound (587.3 mg, 1.18 mmol, 55.1% yield) as a white solid. MS (ESI, m/z): 499.5 [M+H]⁺.

### Step 4)

### 1-ethyl-4-(2-(4-((3-(4-methoxyphenyl)imidazo[1,2-a]pyrazin-8-yl)amino)-2-methylbenzamido)ethyl)-1-methylpiperidin-1-ium formate

4-((3-(4-methoxyphenyl)imidazo[1,2-a]pyrazin-8-yl)amino)-2-methyl-N-(2-(1-methylpiperidin-4-yl)ethyl)benzamide (30 mg, 60.2 µmol, Eq: 1) was dissolved in 1,4-dioxane (4 ml) and DIPEA (29.6 mg, 40 µl, 229 µmol, Eq: 3.81) was added. The reaction mixture was stirred at room temperature for 20 min and iodoethane (37.5 mg, 19.2 µl, 241 µmol, Eq: 4) was added. The reaction mixture was stirred at room temperature overnight. The crude material was purified by preparative HPLC to afford the title compound (27.7 mg, 48.37 µmol, 80.4% yield) as a colorless oil. MS (ESI, m/z): 527.3 [M]⁺.

### Example 21

### 2-chloro-4-[[3-(2,3-difluoro-4-methoxy-phenyl)imidazo[1,2-a]pyrazin-8-yl]amino]-N-[2-(4,4-dimethylpiperazin-4-ium-1-yl)ethyl]-N-methyl-benzamide 2,2,2-trifluoroacetate

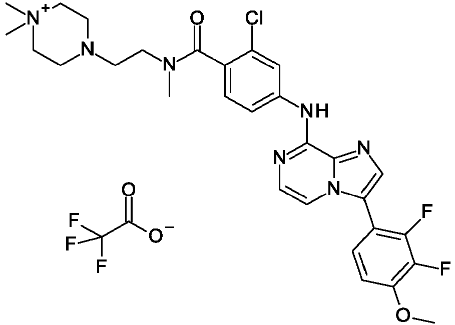

### Step 1)

### tert-butyl 4-(2-(2-chloro-4-((3-(2,3-difluoro-4-methoxyphenyl)imidazo[1,2-a]pyrazin-8-yl)amino)-N-methylbenzamido)ethyl)piperazine-1-carboxylate

A mixture of 2-chloro-4-((3-(2,3-difluoro-4-methoxyphenyl)imidazo[1,2-a]pyrazin-8-yl)amino)benzoic acid (Intermediate 5, 150 mg, 348 µmol, Eq: 1), tert-butyl 4-(2-(methylamino)ethyl)piperazine-1-carboxylate (84.7 mg, 348 µmol, Eq: 1), TEA (363 mg, 0.5 mL, 3.59 mmol, Eq: 10.3) and 2-(3H-[1,2,3]triazolo[4,5-b]pyridin-3-yl)-1,1,3,3-tetramethylisouronium hexafluorophosphate(V) (265 mg, 696 µmol, Eq: 2) in DMF (5 mL) was stirred at room temperature overnight. The mixture was diluted with water and extracted with DCM. The organic layer was dried and concentrated in vacuo to afford the title compound (250 mg, 381 µmol, 109% yield) which was used directly in the next step. MS (ESI, m/z): 656.4 [M+H]⁺.

### Step 2)

### 2-chloro-4-((3-(2,3-difluoro-4-methoxyphenyl)imidazo[1,2-a]pyrazin-8-yl)amino)-N-methyl-N-(2-(piperazin-1-yl)ethyl)benzamide

To a solution of tert-butyl 4-(2-(2-chloro-4-((3-(2,3-difluoro-4-methoxyphenyl)imidazo[1,2-a]pyrazin-8-yl)amino)-N-methylbenzamido)ethyl)piperazine-1-carboxylate (250 mg, 381 µmol, Eq: 1) in CH₂Cl₂ (10 mL) was added TFA (2.96 g, 2 mL, 26 mmol, Eq: 68.1). The mixture was stirred at room temperature for 3 h and then refluxed overnight. The volatiles were removed and the residue was purified by prep-HPLC to afford the title compound (100 mg, 180 µmol, 47.2% yield) as a light yellow solid. 1H NMR (400 MHz, METHANOL-d4) δ ppm 2.71 (s, 3 H) 3.00 (s, 3 H) 3.69 (br s, 9 H) 3.99 (s, 3 H) 7.13 (br t, J=7.83 Hz, 1 H) 7.27 - 7.42 (m, 1 H) 7.51 - 7.62 (m, 2 H) 7.68 - 7.86 (m, 2 H) 7.99 (s, 1 H) 8.12 (d, J=1.59 Hz, 1 H)

### Step 3)

### 2-chloro-4-[[3-(2,3-difluoro-4-methoxy-phenyl)imidazo[1,2-a]pyrazin-8-yl]amino]-N-[2-(4,4-dimethylpiperazin-4-ium-1-yl)ethyl]-N-methyl-benzamide 2,2,2-trifluoroacetate

A mixture of 2-chloro-4-((3-(2,3-difluoro-4-methoxyphenyl)imidazo[1,2-a]pyrazin-8-yl)amino)-N-methyl-N-(2-(piperazin-1-yl)ethyl)benzamide (60 mg, 108 µmol, Eq: 1), iodomethane (153 mg, 1.08 mmol, Eq: 10) and DIPEA (370 mg, 0.5 mL, 2.86 mmol, Eq: 26.5) in acetonitrile (5 mL) was heated to 50 °C with stirring overnight. The volatiles were removed and the residue was purified by prep-HPLC to afford the title compound (50 mg, 70.2 µmol, 65.1% yield) as a light yellow solid. 1H NMR (400 MHz, METHANOL-d4) δ ppm 3.04 (s, 2 H) 3.29 (s, 1 H) 3.37 (s, 4 H) 3.42 (s, 5 H) 3.52 - 3.64 (m, 2 H) 3.77 - 3.89 (m, 3 H) 3.90 - 3.97 (m, 4 H) 4.02 (s, 3 H) 7.14 - 7.23 (m, 1 H) 7.41 (td, J=8.19, 2.20 Hz, 1 H) 7.54 (d, J=5.26 Hz, 1 H) 7.65 (d, J=8.31 Hz, 1 H) 7.75 (dd, J=8.31, 2.08 Hz, 1 H) 7.88 (dd, J=5.20, 2.26 Hz, 1 H) 8.04 (s, 1 H) 8.09 (d, J=1.96 Hz, 1 H)

### Example 22

### 4-[[3-(2,3-Difluoro-4-methoxy-phenyl)imidazo[1,2-a]pyrazin-8-yl]amino]-N-[2-(1,1-dimethylpiperidin-1-ium-4-yl)ethyl]-N,2-dimethyl-benzamide 2,2,2-trifluoroacetate

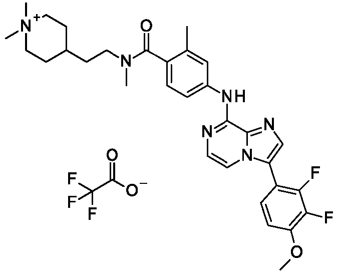

### Step 1)

### tert-Butyl 4-[2-[[4-[[3-(2,3-difluoro-4-methoxy-phenyl)imidazo[1,2-a]pyrazin-8-yl]amino]-2-methyl-benzoyl]-methyl-amino] ethyl] piperidine-1-carboxylate

To a solution of 4-((3-(2,3-difluoro-4-methoxyphenyl)imidazo[1,2-a]pyrazin-8-yl)amino)-2-methylbenzoic acid (Intermediate 6) (820 mg, 2 mmol, Eq: 1.0), *tert*-butyl 4-(2-(methylamino)ethyl)piperidine-1-carboxylate (582 mg, 2.4 mmol, Eq: 1.2) in anhydrous DMF (10 mL) was added DIPEA (516 mg,4 mmol, Eq: 2.0). Then the resultant mixture was stirred for 30 min at room temperature, HATU (1.52 g, 4.0 mmol, Eq: 2.0) was added in the mixture and stirred for extra 10 h. The mixture was poured into water (50 mL) and the aqueous solution was extracted with DCM (50 mL×2). The organic layers were combined and washed with water and brine, dried over anhydrous sodium sulfate and concentrated under reduced pressure to give a red oil, which was purified by flash column.to provide the title compound (1.0 g, 1.57 mmol, 78.5% yield) as a yellow solid. MS (ESI, m/z): 635.3 [M+H]⁺.

### Step 2)

### 4-[[3-(2,3-Difluoro-4-methoxy-phenyl)imidazo[1,2-a]pyrazin-8-yl]amino]-N,2-dimethyl-N-[2-(4-piperidyl)ethyl]benzamide

To a solution of *tert*-butyl 4-(2-(4-((3-(2,3-difluoro-4-methoxyphenyl)imidazo[1,2-a]pyrazin-8-yl)amino)-N,2-dimethylbenzamido)ethyl)piperidine-1-carboxylate(635 mg, 1 mmol) in MeOH (15 ml) was added 1 mol/L hydrochloric acid in EA (5 mL) at room temperature.

The resultant mixture was stirred for 4.0 h and then adjusted to pH=7-8 with 2M aqueous solution of sodium carbonate. The mixture was extracted with DCM (50 mL×2), the combined organic layers were washed with water and brine, dried over anhydrous sodium sulfate and concentrated to give a red solid, which was purified by Prep.HPLC.to afford the title compound (270 mg, 0.50 mmol, 50.0 % yield) as a yellow powder. MS (ESI, m/z): 535.3 [M+H]⁺.

### Step 3)

### 4-[[3-(2,3-Difluoro-4-methoxy-phenyl)imidazo[1,2-a]pyrazin-8-yl]amino]-N-[2-(1,1-dimethylpiperidin-1-ium-4-yl)ethyl]-N,2-dimethyl-benzamide 2,2,2-trifluoroacetate

To a solution of 4-((3-(2,3-difluoro-4-methoxyphenyl)imidazo[1,2-a]pyrazin-8-yl)amino)-N,2-dimethyl-N-(2-(piperidin-4-yl)ethyl)benzamide (270 mg, 0.5 mmol, Eq: 1.0) in anhydrous EtOH (10 mL) was added DIPEA (129 mg,1.0 mmol, Eq: 2.0). Then the resultant mixture was stirred for 10 min at room temperature, iodomethane (710 mg, 5.0 mmol, Eq: 10) was added in the mixture and stirred for extra 10 hr. Poured the mixture into water (20 mL) and the aqueous solution was extracted with DCM (50 mL×2). The organic layers were combined and washed with water and brine, dried over anhydrous sodium sulfate and concentrated under reduced pressure to give a red oil, which was purified by Prep. HPLC to provide the title compound (200 mg, 0.30 mmol, 60.0 % yield) MS (ESI, m/z): 563.3 [M]⁺.

### Example 23

### 1-(carboxymethyl)-4-(2-(4-((3-(4-methoxyphenyl)imidazo[1,2-a]pyrazin-8-yl)amino)-2-methylbenzamido)ethyl)-1-methylpiperidin-1-ium formate

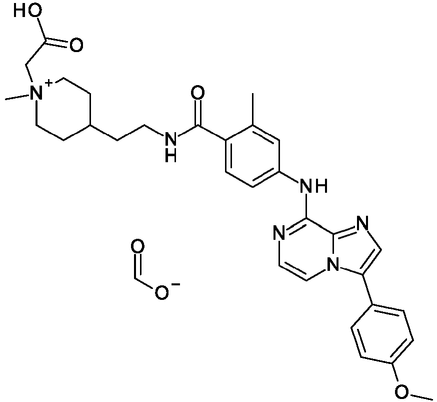

### Step 1) Example 155 1-(2-methoxy-2-oxoethyl)-4-(2-(4-((3-(4-methoxyphenyl)imidazo [1,2-a] pyrazin-8-yl)amino)-2-methylbenzamido)ethyl)-1-methylpiperidin-1-ium formate

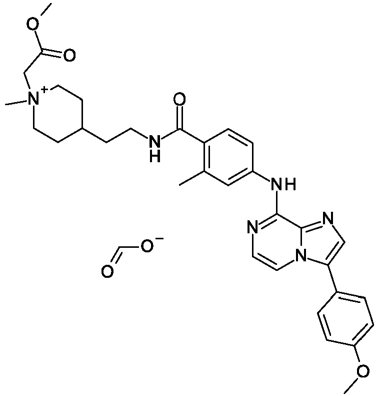

4-((3-(4-methoxyphenyl)imidazo[1,2-a]pyrazin-8-yl)amino)-2-methyl-N-(2-(1-methylpiperidin-4-yl)ethyl)benzamide (Intermediate 4, 30 mg, 60.2 µmol, Eq: 1) was dissolved in 1,4-dioxane (4 ml) and DIPEA (29.6 mg, 40 µl, 229 µmol, Eq: 3.81) was added. The reaction mixture was stirred at room temperature for 20 min and methyl 2-bromoacetate (36.8 mg, 22.1 µl, 241 µmol, Eq: 4) was added. The reaction mixture was stirred at room temperature overnight. The crude material was purified by preparative HPLC to afford the title compound (27 mg, 43.8 µmol, 72.8% yield) as a colorless oil. MS (ESI, m/z): 616.2 [M]⁺.

### Step 2)

### 1-(carboxymethyl)-4-(2-(4-((3-(4-methoxyphenyl)imidazo[1,2-a]pyrazin-8-yl)amino)-2-methylbenzamido)ethyl)-1-methylpiperidin-1-ium formate

1-(2-methoxy-2-oxoethyl)-4-(2-(4-((3-(4-methoxyphenyl)imidazo[1,2-a]pyrazin-8-yl)amino)-2-methylbenzamido)ethyl)-1-methylpiperidin-1-ium formate (27 mg, 43.8 µmol, Eq: 1) and LiOH (1 ml, 1 mmol, Eq: 22.8) were dissolved in THF (1 ml) and MeOH (500 µl). The reaction mixture was heated to 60 °C and stirred for 24 h. The reaction mixture was concentrated in vacuum and it was acidified with 1M HCl. The mixture was evaporated to dryness and the crude material was purified by preparative HPLC to afford the title compound (15.2 mg, 25.2 µmol, 57.6% yield) as a colorless oil. MS (ESI, m/z): 557.2 [M]⁺.

### Example 24

### 4-(2-(4-((3-(4-(difluoromethoxy)phenyl)imidazo[1,2-a]pyrazin-8-yl)amino)-N,2-dimethylbenzamido)ethyl)-1,1-diethylpiperidin-1-ium formate

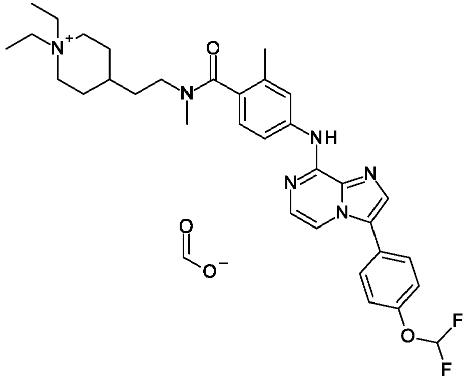

4-((3-(4-(difluoromethoxy)phenyl)imidazo[1,2-a]pyrazin-8-yl)amino)-N,2-dimethyl-N-(2-(piperidin-4-yl)ethyl)benzamide hydrochloride (Intermediate 3, 30 mg, 52.5 µmol, Eq: 1) was dissolved in 1,4-dioxane (4 ml) and DIPEA (29.6 mg, 40 µl, 229 µmol, Eq: 4.36) was added. The reaction mixture was stirred at room temperature for 20 min and iodoethane (78 mg, 40 µl, 500 µmol, Eq: 9.52) was added. The reaction mixture was stirred at room temperature overnight. Then iodoethane (117 mg, 60 µl, 750 µmol, Eq: 14.3) was added again and the reaction mixture was stirred at room temperature for 2 days. The reaction was incomplete with SM remaining so iodoethane (97.5 mg, 50 µL, 625 µmol, Eq: 11.9) and DIPEA (14.8 mg, 20 µL, 115 µmol, Eq: 2.18) were added and the reaction mixture was stirred at room temperature for 2 days again. The crude material was purified by preparative HPLC to afford the title compound (20.5 mg, 32.2 µmol, 61.3% yield) as a colorless solid. MS (ESI, m/z): 591.4 [M]⁺296.3 [M+H]²⁺.

### Example 25

### 6-(4-((3-(4-(difluoromethoxy)phenyl)imidazo[1,2-a]pyrazin-8-yl)amino)-2-methylbenzamido)-N,N,N-trimethylhexan-1-aminium formate

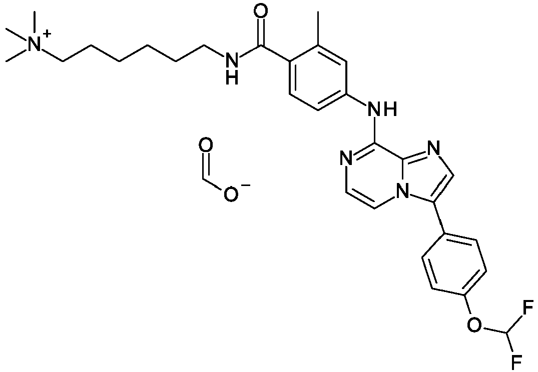

### Step 1)

### Tert-butyl (6-(4-((3-(4-(difluoromethoxy)phenyl)imidazo[1,2-a]pyrazin-8-yl)amino)-2-methylbenzamido)hexyl)carbamate

4-((3-(4-(difluoromethoxy)phenyl)imidazo[1,2-a]pyrazin-8-yl)amino)-2-methylbenzoic acid (Intermediate 2, 30 mg, 73.1 µmol, Eq: 1) was combined with DMF (750 µl). Tert-butyl (6-aminohexyl)carbamate (23.7 mg, 110 µmol, Eq: 1.50) was added, followed by DIPEA (28.3 mg, 38.3 µl, 219 µmol, Eq: 3.00) and HATU (41.7 mg, 110 µmol, Eq: 1.50) and the reaction mixture was stirred at RT for 2 h. The reaction mixture was purified via prep HPLC to afford the title compound (28.6 mg, 46.98 µmol, 64.3% yield) as an off-white solid. MS (ESI, m/z): 609.4 [M+H]⁺.

### Step 2)

### N-(6-aminohexyl)-4-((3-(4-(difluoromethoxy)phenyl)imidazo[1,2-a]pyrazin-8-yl)amino)-2-methylbenzamide hydrochloride

Tert-butyl (6-(4-((3-(4-(difluoromethoxy)phenyl)imidazo[1,2-a]pyrazin-8-yl)amino)-2-methylbenzamido)hexyl)carbamate (28 mg, 46 µmol, Eq: 1) was combined with HCl 4M in dioxane (173 µl, 690 µmol, Eq: 15). After stirring for 1 h, the reaction mixture was concentrated to dryness to afford the title compound (25.4 mg, 46.6 µmol, 99.3% yield) as an off-white solid. MS (ESI, m/z): 509.2 [M+H]⁺.

### Step 3)

### 6-(4-((3-(4-(difluoromethoxy)phenyl)imidazo[1,2-a]pyrazin-8-yl)amino)-2-methylbenzamido)-N,N,N-trimethylhexan-1-aminium formate

The title compound (12 mg, 20.1 µmol, 41.1% yield) was obtained as a white solid in analogy to Example **13,** Step 8. MS (ESI, m/z): 551.3 [M]⁺.

### Example 26

### 2-[4-[2-chloro-4-[[3-[4-(cyanomethoxy)-2,3-difluorophenyl]imidazo[1,2-a]pyrazin-8-yl]amino]benzoyl]piperazin-1-yl]ethyl-trimethylazanium iodide

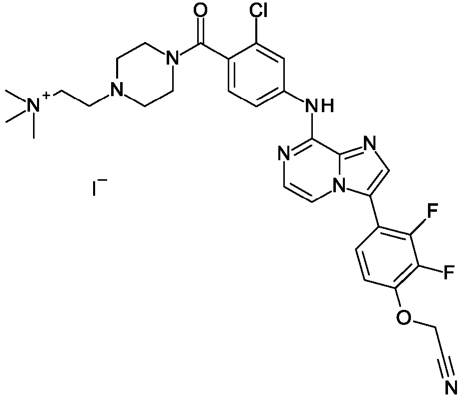

### Step 1) Intermediate 10

### 2-chloro-4-((3-(4-(cyanomethoxy)-2,3-difluorophenyl)imidazo[1,2-a]pyrazin-8-yl)amino)benzoic acid hydrochloride

2-(4-(8-chloroimidazo[1,2-a]pyrazin-3-yl)-2,3-difluorophenoxy)acetonitrile (Intermediate 11, 1.5 g, 4.68 mmol, Eq: 1) was dissolved in acetonitrile (40 ml) and acetic acid (4 ml). 4-Amino-2-chlorobenzoic acid (883 mg, 5.15 mmol, Eq: 1.1) was added and the reaction mixture was heated at reflux for 15 h. The reaction mixture was concentrated under vacuum, the residue was taken in acetonitrile and filtered. The cake was washed with acetonitrile and heptane and was dried for 2 h with the HV to afford the title compound (1.44 g, 2.9 mmol, 59.4% yield) as a light grey solid. MS (ESI, m/z): 456.1 [M+H]⁺.

### Step 2)

### 2-(4-(8-((3-chloro-4-(4-(2-(dimethylamino)ethyl)piperazine-1-carbonyl)phenyl)amino)imidazo[1,2-a]pyrazin-3-yl)-2,3-difluorophenoxy)acetonitrile

2-chloro-4-((3-(4-(cyanomethoxy)-2,3-difluorophenyl)imidazo[1,2-a]pyrazin-8-yl)amino)benzoic acid hydrochloride (700 mg, 1.42 mmol, Eq: 1) was combined with DMF (9 ml). HATU (811 mg, 2.13 mmol, Eq: 1.50) and Et₃N (576 mg, 793 µl, 5.69 mmol, Eq: 4.00) were added, followed by N,N-dimethyl-2-(piperazin-1-yl)ethan-1-amine (335 mg, 2.13 mmol, Eq: 1.50). The reaction mixture was stirred at RT. Then water was added to the reaction mixture and the product was extracted with DCM and dried over Na₂SO₄. The crude was purified by flash chromatography (silica gel, 80 g, 0% to 100% DCM:MeOH:NH₄OH(100:10:1) in DCM) to afford the title compound (642.5 mg, 1.08 mmol, 75.2% yield) as an off-white foam. MS (ESI, m/z): 593.3 [M+H]⁺.

### Step 3)

### 2-[4-[2-chloro-4-[[3-[4-(cyanomethoxy)-2,3-difluorophenyl]imidazo[1,2-a]pyrazin-8-yl]amino]benzoyl]piperazin-1-yl]ethyl-trimethylazanium iodide

2-(4-(8-((3-chloro-4-(4-(2-(dimethylamino)ethyl)piperazine-1-carbonyl)phenyl)amino)imidazo[1,2-a]pyrazin-3-yl)-2,3-difluorophenoxy)acetonitrile (630 mg, 1.06 mmol, Eq: 1) was combined with EtOH (6.4 ml). Iodomethane (180 mg, 79.4 µl, 1.27 mmol, Eq: 1.20) was added and the reaction was stirred overnight at RT. Iodomethane (75.1 mg, 33.1 µl, 529 µmol, Eq: 0.50) was added again and the stirring continued at RT for 4 h. Ether was added to the suspension formed and the solid was filtered, washed with ether and dried under HV to afford the title compound (629 mg, 853 µmol, 88.7% yield) as a white solid. MS (ESI, m/z): 609.3 [M]⁺.

### Example 27

### 2-(1-(4-((3-(3-Fluoro-4-methoxyphenyl)imidazo[1,2-a]pyrazin-8-yl)amino)-2-methylbenzoyl)piperidine-4-carboxamido)-N,N,N-trimethylethanaminium formate

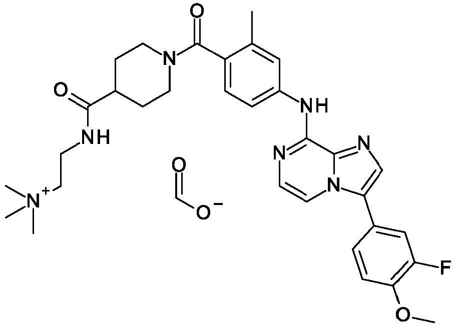

### Step 1)

### 8-Chloro-3-(3-fluoro-4-methoxyphenyl)imidazo[1,2-a]pyrazine

To a microwave vial were added 8-chloro-3-iodoimidazo[1,2-a]pyrazine (Intermediate 1, 2.5 g, 8.95 mmol, Eq: 1), (3-fluoro-4-methoxyphenyl)boronic acid (2.28 g, 13.4 mmol, Eq: 1.5), Na₂CO₃ (1.9 g, 17.9 mmol, Eq: 2) and 1,1'-bis(diphenylphosphino)ferrocenedichloro palladium(II) dichloromethane complex (655 mg, 895 µmol, Eq: 0.1) in dioxane (16 ml) and water (1.6 ml). The vial was capped and heated in the microwave at 120°C for 60 min. The reaction mixture was poured into 10 mL of H₂O and extracted with EtOAc (3 x 25 mL). The organic layers were dried over MgSO₄ and concentrated in vacuo. The crude material was purified by flash chromatography (silica gel, 120 g, 0% to 65% EtOAc in heptane) to afford the title compound (1.55 g, 5.58 mmol, 62.7% yield) as a brown solid. MS (ESI, m/z): 278.0 [M+H]⁺.

### Step 2) Intermediate 12

### 4-((3-(3-Fluoro-4-methoxyphenyl)imidazo[1,2-a]pyrazin-8-yl)amino)-2-methylbenzoic acid

8-chloro-3-(3-fluoro-4-methoxyphenyl)imidazo[1,2-a]pyrazine (1 g, 3.6 mmol, Eq: 1) was combined with acetonitrile (100 ml) and AcOH (1 ml). 4-Amino-2-methylbenzoic acid (680 mg, 4.5 mmol, Eq: 1.25) was added and the reaction mixture was stirred at 80°C overnight. After cooling down to RT, the solid was filtered, washed with acetonitrile and MeOH and dried under HV. The mother liquor, which contains starting material were put again in reaction with 0.5 eq of 4-amino-2-methylbenzoic acid in 20 ml of acetonitrile (+200 ul of AcOH). After stirring for 55 h, the reaction was cooled down and the solid formed was filtered, washed with acetonitrile and MeOH and dried under HV to afford in total the title compound (1.1 g, 2.9 mmol, 79% yield) as an off-white solid. MS (ESI, m/z): 393.2 [M+H]⁺.

### Step 3)

### Methyl 1-(4-((3-(3-fluoro-4-methoxyphenyl)imidazo[1,2-a]pyrazin-8-yl)amino)-2-methylbenzoyl)piperidine-4-carboxylate

4-((3-(3-fluoro-4-methoxyphenyl)imidazo[1,2-a]pyrazin-8-yl)amino)-2-methylbenzoic acid (500 mg, 1.27 mmol, Eq: 1) was combined with DMF (10 ml). DIPEA (494 mg, 668 µl, 3.82 mmol, Eq: 3) and HATU (969 mg, 2.55 mmol, Eq: 2.00) were added and the reaction mixture was stirred at room temperature for 30 min. Methyl piperidine-4-carboxylate (274 mg, 1.91 mmol, Eq: 1.5) was then added and it was stirred at room temperature overnight. The reaction mixture was purified by prep HPLC to afford the title compound (386 mg, 746 µmol, 58.5% yield) as a light brown solid. MS (ESI, m/z): 518.2 [M+H]⁺.

### Step 4)

### 1-(4-((3-(3-fluoro-4-methoxyphenyl)imidazo[1,2-a]pyrazin-8-yl)amino)-2-methylbenzoyl)piperidine-4-carboxylic acid

Methyl 1-(4-((3-(3-fluoro-4-methoxyphenyl)imidazo[1,2-a]pyrazin-8-yl)amino)-2-methylbenzoyl)piperidine-4-carboxylate (386mg, 746 µmol, Eq: 1) and 1M LiOH (3.52 ml, 3.52 mmol, Eq: 4.71) were dissolved in THF (5 ml) and MeOH (2.5 ml). The reaction mixture was heated to 60 °C and stirred overnight. The reaction mixture was concentrated in vacuum and acidified with 1M HCl. The mixture was poured into 1 mL of H₂O and extracted with DCM (2 x 2 mL). The organic layers were dried over Na₂SO₄ and concentrated in vacuo to afford the title compound (340.7 mg, 676 µmol, 86.2% yield) as a light brown solid. MS (ESI, m/z): 504.2 [M+H]⁺.

### Step 5)

### Tert-butyl (2-(1-(4-((3-(3-fluoro-4-methoxyphenyl)imidazo[1,2-a]pyrazin-8-yl)amino)-2-methylbenzoyl)piperidine-4-carboxamido)ethyl)(methyl)carbamate

1-(4-((3-(3-fluoro-4-methoxyphenyl)imidazo[1,2-a]pyrazin-8-yl)amino)-2-methylbenzoyl)piperidine-4-carboxylic acid (30 mg, 59.6 µmol, Eq: 1) was combined with DMF (600 µl). DIPEA (23.1 mg, 31.2 µl, 179 µmol, Eq: 3.00), HATU (34 mg, 89.4 µmol, Eq: 1.50) and tert-butyl (2-aminoethyl)(methyl)carbamate (15.6 mg, 89.4 µmol, Eq: 1.50) were added and the reaction mixture was stirred at room temperature for 3 h. The reaction mixture was directly purified by prep HPLC to afford the title compound (28.2 mg, 42.7 µmol, 71.7% yield) as a white solid. MS (ESI, m/z): 660.4 [M+H]⁺.

### Step 6)

### 1-(4-((3-(3-fluoro-4-methoxyphenyl)imidazo[1,2-a]pyrazin-8-yl)amino)-2-methylbenzoyl)-N-(2-(methylamino)ethyl)piperidine-4-carboxamide hydrochloride

Tert-butyl (2-(1-(4-((3-(3-fluoro-4-methoxyphenyl)imidazo[1,2-a]pyrazin-8-yl)amino)-2-methylbenzoyl)piperidine-4-carboxamido)ethyl)(methyl)carbamate (26 mg, 39.4 µmol, Eq: 1) was combined with HCl 4M in dioxane (98.5 µl, 394 µmol, Eq: 10.0). After stirring at RT for 4 h, the reaction mixture was concentrated to dryness and dried under HV to afford the title compound (24.9 mg, 41.8 µmol, 106% yield) as a light yellow solid. MS (ESI, m/z): 560.3 [M+H]⁺.

### Step 7)

### 2-(1-(4-((3-(3-fluoro-4-methoxyphenyl)imidazo[1,2-a]pyrazin-8-yl)amino)-2-methylbenzoyl)piperidine-4-carboxamido)-N,N,N-trimethylethanaminium formate

1-(4-((3-(3-fluoro-4-methoxyphenyl)imidazo[1,2-a]pyrazin-8-yl)amino)-2-methylbenzoyl)-N-(2-(methylamino)ethyl)piperidine-4-carboxamide hydrochloride (12 mg, 20.1 µmol, Eq: 1) was dissolved in 1,4-Dioxane (2 ml) and DIPEA (37 mg, 50 µl, 286 µmol, Eq: 14.2) was added. The reaction mixture was stirred at room temperature for 20 min and iodomethane (2.86 mg, 60 µL, 20.1 µmol, Eq: 1) was added. The reaction mixture was stirred at room temperature overnight.

The crude material was purified by preparative HPLC. The product was lyophilized to afford the title compound (8.6 mg, 13.6 µmol, 67.4% yield) as a white solid. MS (ESI, m/z): 588.3 [M]⁺.

### Example 28

### 3-(4-((3-(4-Chloro-2,3-difluorophenyl)imidazo[1,2-a]pyrazin-8-yl)amino)-2-ethylbenzamido)-N,N,N-trimethylpropan-1-aminium formate

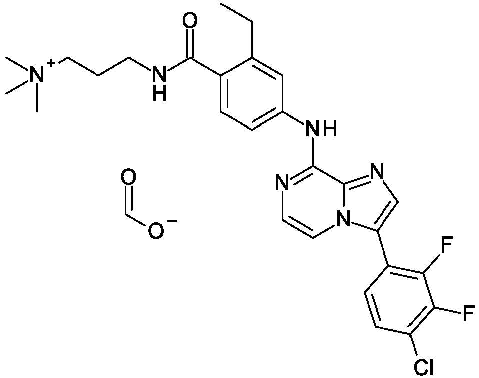

### Step 1)

### 8-Chloro-3-(4-chloro-2,3-difluorophenyl)imidazo[1,2-a]pyrazine

To a microwave vial was added 8-chloro-3-iodoimidazo[1,2-a]pyrazine (Intermediate 1, 500 mg, 1.79 mmol, Eq: 1), (4-chloro-2,3-difluorophenyl)boronic acid (413 mg, 2.15 mmol, Eq: 1.2), Na₂CO₃ (379 mg, 3.58 mmol, Eq: 2) and 1,1'-bis(diphenylphosphino)ferrocenedichloro palladium(II) dichloromethane complex (131 mg, 179 µmol, Eq: 0.1) in dioxane (5 ml) and water (500 µl). The vial was capped and heated in the microwave at 90°C for 60 min. The reaction mixture was poured into 15 ml of H₂O and extracted with EtOAc (3 x 15 ml). The organic layers were dried over MgSO₄ and concentrated in vacuo. The crude material was purified by flash chromatography (silica gel, 50 g, 0% to 45% EtOAc in heptane) to afford the title compound (280 mg, 933 µmol, 52.1% yield) as a brown solid. MS (ESI, m/z): 299.9 [M+H]⁺.

### Step 2)

### Methyl 4-((3-(4-chloro-2,3-difluorophenyl)imidazo[1,2-a]pyrazin-8-yl)amino)-2-ethylbenzoate

8-Chloro-3-(4-chloro-2,3-difluorophenyl)imidazo[1,2-a]pyrazine (130 mg, 433 µmol, Eq: 1) and methyl 4-amino-2-ethylbenzoate (Intermediate 7, 93.2 mg, 520 µmol, Eq: 1.2) were combined with acetonitrile (2 ml) and AcOH (200 µL). The reaction mixture was heated to 80°C and stirred for 2 days. The reaction mixture was filtered through sintered glass and washed with acetonitrile and methanol. The solid was dried under high vacuum for 30 min to afford the title compound (83.9 mg, 189 µmol, 43.7% yield) as an off-white solid. MS (ESI, m/z): 443.1 [M+H]⁺.

### Step 3)

### 4-((3-(4-Chloro-2,3-difluorophenyl)imidazo[1,2-a]pyrazin-8-yl)amino)-2-ethylbenzoic acid

Methyl 4-((3-(4-chloro-2,3-difluorophenyl)imidazo[1,2-a]pyrazin-8-yl)amino)-2-ethylbenzoate (143 mg, 323 µmol, Eq: 1) and 1M LiOH (1 ml, 1 mmol, Eq: 3.1) were dissolved in THF (1 ml) and MeOH (500 µl). The reaction mixture was heated to 60 °C and stirred for 24 h. The reaction mixture was concentrated in vacuum and acidified with 1M HCl. The solids were filtered through sintered glass and dried under HV to afford the title compound (100 mg, 233 µmol, 72.2% yield) as a brown solid. MS (ESI, m/z): 429.1 [M+H]⁺.

### Step 4)

### tert-Butyl (3-(4-((3-(4-chloro-2,3-difluorophenyl)imidazo[1,2-a]pyrazin-8-yl)amino)-2-ethylbenzamido)propyl)carbamate

The title compound (75.9 mg, 129 µmol, 46.4% yield) was obtained as a light yellow solid in analogy to Example **27,** Step 5 using tert-butyl (3-aminopropyl)carbamate. MS (ESI, m/z): 585.2 [M+H]⁺.

### Step 5)

### N-(3-Aminopropyl)-4-((3-(4-chloro-2,3-difluorophenyl)imidazo[1,2-a]pyrazin-8-yl)amino)-2-ethylbenzamide hydrochloride

The title compound (53.2 mg, 102 µmol, 78.6% yield) was obtained as a light brown solid in analogy to Example **27,** Step 6. MS (ESI, m/z): 485.1 [M+H]⁺.

### Step 8)

### 3-(4-((3-(4-Chloro-2,3-difluorophenyl)imidazo[1,2-a]pyrazin-8-yl)amino)-2-ethylbenzamido)-N,N,N-trimethylpropan-1-aminium formate

The title compound (13.4 mg, 23.4 µmol, 50.8% yield) was obtained as a white solid in analogy to Example **27,** Step 7. MS (ESI, m/z): 528.2 [M]⁺.

### Example 29

### 2-(1-(4-((3-(4-(Difluoromethoxy)phenyl)imidazo[1,2-a]pyrazin-8-yl)amino)-2-methylbenzoyl)piperidin-4-yl)-N,N,N-trimethylethanaminium formate

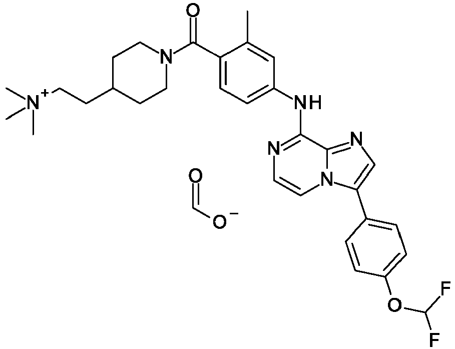

### Step 1)

### Tert-butyl (2-(1-(4-((3-(4-(difluoromethoxy)phenyl)imidazo[1,2-a]pyrazin-8-yl)amino)-2-methylbenzoyl)piperidin-4-yl)ethyl)carbamate

4-((3-(4-(Difluoromethoxy)phenyl)imidazo[1,2-a]pyrazin-8-yl)amino)-2-methylbenzoic acid (Intermediate 2, 30 mg, 73.1 µmol, Eq: 1) was combined with DMF (1 ml). Tert-butyl (2-(piperidin-4-yl)ethyl)carbamate (25 mg, 110 µmol, Eq: 1.5), DIPEA (28.3 mg, 38.3 µl, 219 µmol, Eq: 3) and HATU (55.6 mg, 146 µmol, Eq: 2) were added and it was stirred overnight at room temperature. The crude material was purified by preparative HPLC to afford the title compound (7.6 mg, 12.2 µmol, 16.7% yield) as a white solid. MS (ESI, m/z): 621.4 [M+H]⁺.

### Step 2)

### (4-(2-aminoethyl)piperidin-1-yl)(4-((3-(4-(difluoromethoxy)phenyl)imidazo[1,2-a]pyrazin-8-yl)amino)-2-methylphenyl)methanone hydrochloride

Tert-butyl (2-(1-(4-((3-(4-(difluoromethoxy)phenyl)imidazo[1,2-a]pyrazin-8-yl)amino)-2-methylbenzoyl)piperidin-4-yl)ethyl)carbamate (7.6 mg, 12.2 µmol, Eq: 1) and 4M HCl in dioxane (600 mg, 500 µl, 2 mmol, Eq: 163) were combined and it was stirred at room temperature for 30 min. The reaction mixture was evaporated and dried under HV to afford the title compound (8.1 mg, 14.5 µmol, 119% yield) as a light yellow solid. MS (ESI, m/z): 521.1 [M+H]⁺.

### Step 3)

### 2-(1-(4-((3-(4-(Difluoromethoxy)phenyl)imidazo[1,2-a]pyrazin-8-yl)amino)-2-methylbenzoyl)piperidin-4-yl)-N,N,N-trimethylethanaminium formate

The title compound (12.7 mg, 20.8 µmol, 58.1% yield) was obtained as a white solid in analogy to Example **27,** Step 7. MS (ESI, m/z): 563.3 [M]⁺.

### Example 30

### [4-[[3-[4-(Difluoromethoxy)phenyl]imidazo[1,2-a]pyrazin-8-yl]amino]-2-methyl-phenyl]-[4-(4,4-dimethylpiperazin-4-ium-1-carbonyl)piperazin-1-yl]methanone formate

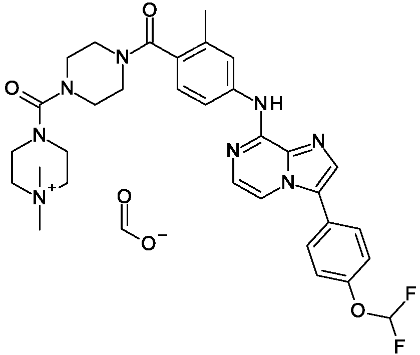

### Step 1)

### tert-Butyl 4-[4-[[3-[4-(difluoromethoxy)phenyl]imidazo[1,2-a]pyrazin-8-yl]amino]-2-methyl-benzoyl]piperazine-1-carboxylate

To a solution of 4-[[3-[4-(difluoromethoxy)phenyl]imidazo[1,2-a]pyrazin-8-yl]amino]-2-methylbenzoic acid (Intermediate 2, 1.2 g, 2.92 mmol, 1 eq) in DMF (10 mL) were added 1-Boc-piperazine (0.82 g, 4.39 mmol, 1.5 eq), N,N-diisopropylethylamine (1.53 mL, 8.77 mmol, 3 eq) and O-(7-azabenzotriazol-1-yl)-N,N,N',N'-tetramethyluronium hexafluorophosphate (2.22 g, 5.85 mmol, 2 eq). The reaction was stirred at 25 °C for 12 h. 40 mL of water was added and it was extracted with EtOAc (50 mL x 3). The combined organic layers were washed with brine (40 mL x 3), dried over Na₂SO₄, filtered and concentrated. 20 mL of MTBE was added to the residue and it was stirred for 1 h. The solids were filtered and dried to afford the title compound (1.7 g, 2.94 mmol, 90.43% yield) as a yellow solid. MS (ESI, m/z): 579.3 [M+H]⁺.

### Step 2) Intermediate 13

### [4-[[3-[4-(Difluoromethoxy)phenyl]imidazo[1,2-a]pyrazin-8-yl]amino]-2-methyl-phenyl]-piperazin-1-yl-methanone

To a solution of tert-butyl 4-[4-[[3-[4-(difluoromethoxy)phenyl]imidazo[1,2-a]pyrazin-8-yl]amino]-2-methyl-benzoyl]piperazine-1-carboxylate (1.7 g, 2.94 mmol, 1 eq) in methanol (10 mL) was added hydrochloric acid in MeOH (20 mL, 80 mmol, 27.23 eq) and it was stirred at 20 °C for 12 h. After concentration, 100 mL of aqueous saturated NaHCO₃ was added and it was extracted with DCM (50 mL x 3). The combined organic layers were washed with brine (100 mL), dried over Na₂SO₄, filtered and concentrated. The residue was purified by silica gel column chromatography eluted with DCM:MeOH=10:1 to afford the title compound (800 mg, 1.67 mmol, 56.91% yield) as a yellow solid. MS (ESI, m/z): 479.2 [M+H]⁺.

### Step 3)

### [4-[[3-[4-(Difluoromethoxy)phenyl]imidazo[1,2-a]pyrazin-8-yl]amino]-2-methyl-phenyl]-[4-(4-methylpiperazine-1-carbonyl)piperazin-1-yl]methanone

To a solution of [4-[[3-[4-(difluoromethoxy)phenyl]imidazo[1,2-a]pyrazin-8-yl]amino]-2-methyl-phenyl]-piperazin-1-yl-methanone (200 mg, 0.420 mmol, 1 eq) in DMF (5 mL) were added N,N-diisopropylethylamine (0.22 mL, 1.25 mmol, 3 eq), N,N'-carbonyldiimidazole (74.55 mg, 0.460 mmol, 1.1 eq) and 1-methylpiperazine (83.73 mg, 0.840 mmol, 2 eq), then the reaction was stirred at 60 °C for 60 h. The reaction mixture was purified by prep-HPLC (base) to afford the by-product 4-[4-[[3-[4-(difluoromethoxy)phenyl]imidazo[1,2-a]pyrazin-8-yl]amino]-2-methyl-benzoyl]-N,N-dimethyl-piperazine-1-carboxamide (44.4 mg, 0.080 mmol, 18.13% yield) as a white solid and the title compound (50 mg, 0.080 mmol, 19.78% yield) as a yellow solid. MS (ESI, m/z): 605.0 [M+H]⁺.

### Step 4)

### [4-[[3-[4-(difluoromethoxy)phenyl]imidazo[1,2-a]pyrazin-8-yl]amino]-2-methyl-phenyl]-[4-(4,4-dimethylpiperazin-4-ium-1-carbonyl)piperazin-1-yl]methanone formate

To a solution of [4-[[3-[4-(difluoromethoxy)phenyl]imidazo[1,2-a]pyrazin-8-yl]amino]-2-methyl-phenyl]-[4-(4-methylpiperazine-1-carbonyl)piperazin-1-yl]methanone (50 mg, 0.080 mmol, 1 eq) in ACN (2 mL) was added iodomethane (35.21 mg, 0.250 mmol, 3 eq) and it was stirred at 40 °C for 12 h. The reaction mixture was purified by prep-HPLC (FA) to afford the title compound (22 mg, 0.030 mmol, 39.18% yield) as a white solid. MS (ESI, m/z): 619.3 [M]⁺.

### Example 31

### [2-[4-[2-Chloro-4-[[3-(3-fluoro-4-methoxyphenyl)imidazo[1,2-a]pyrazin-8-yl]amino]benzoyl]piperazin-1-yl]-2-oxoethyl]-trimethylazanium formate

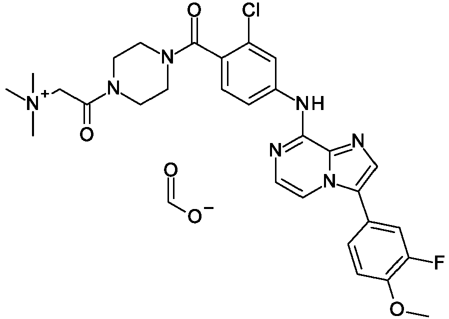

### Step 1)

### 2-(3-Fluoro-4-methoxy-phenyl)-4,4,5,5-tetramethyl-1,3,2-dioxaborolane

A mixture of 4-bromo-2-fluoroanisole (20 g, 97.55 mmol, 1 eq), bis(pinacolato)diboron (26.01 g, 102.43 mmol, 1.05 eq), potassium acetate (19148 mg, 195.1 mmol, 2 eq) and 1,1'-bis(diphenylphosphino)ferrocene-palladium(II)dichloride dichloromethane complex (4776 mg, 5.85 mmol, 0.06 eq) in 1,4-dioxane (300 mL) was stirred at 80 °C for 4 h under N₂. The reaction mixture was concentrated and purified by silica gel chromatography (petroleum ether/ ethyl acetate=20/1) to afford the title compound (18 g, 71.4 mmol, 65.88% yield) as a grey solid.

### Step 2)

### 8-Chloro-3-(3-fluoro-4-methoxy-phenyl)imidazo[1,2-a]pyrazine

A mixture of 8-chloro-3-iodo-imidazo[1,2-a]pyrazine (18 g, 64.41 mmol, 1 eq), 2-(3-fluoro-4-methoxy-phenyl)-4,4,5,5-tetramethyl-1,3,2-dioxaborolane (17048 mg, 67.63 mmol, 1.05 eq), sodium carbonate (13653 mg, 128.82 mmol, 2 eq) and 1,1'-bis(diphenylphosphino)ferrocene-palladium(II)dichloride dichloromethane complex (2628 mg, 3.22 mmol, 0.05 eq) in 1,4-dioxane (200 mL) and water (100 mL) was stirred at 80 °C for 2 h under N₂. To the mixture was added water (200 mL) and it was extracted with EtOAc (300 mL x 3). The organic layers were dried with Na₂SO₄ and concentrated. The crude was purified by silica gel column chromatography (DCM/ ethyl acetate=5/1) to afford the title compound (21.4 g, 77.07 mmol, 91.77% yield) as a pink solid. MS (ESI, m/z): 278.0 [M+H]⁺.

### Step 3)

### 2-Chloro-4-((3-(3-fluoro-4-methoxyphenyl)imidazo[1,2-a]pyrazin-8-yl)amino)benzoic acid

A mixture of 8-chloro-3-(3-fluoro-4-methoxy-phenyl)imidazo[1,2-a]pyrazine (3 g, 10.8 mmol, 1 eq) and 4-amino-2-chlorobenzoic acid (2780.57 mg, 16.21 mmol, 1.5 eq) in acetic acid (60 mL) and acetonitrile (6 mL) was stirred at 80 °C for 24 h. The reaction mixture was filtered. The white solid was triturated with DMF (20 mL) and MeCN (10 mL) at 60 °C for 1 h. The solids were filtered and washed with MeCN several times to afford the title compound (1100 mg, 2.66 mmol, 23.43% yield) as a white solid. MS (ESI, m/z): 413.0 [M+H]⁺.

### Step 4)

### Tert-butyl 4-(2-chloro-4-((3-(3-fluoro-4-methoxyphenyl)imidazo[1,2-a]pyrazin-8-yl)amino)benzoyl)piperazine-1-carboxylate

2-chloro-4-((3-(3-fluoro-4-methoxyphenyl)imidazo[1,2-a]pyrazin-8-yl)amino)benzoic acid (200 mg, 484 µmol, Eq: 1) was combined with DMF (4 ml). DIPEA (250 mg, 338 µl, 1.94 mmol, Eq: 4.00) and HATU (276 mg, 727 µmol, Eq: 1.50) were added and after stirring for 15 min tert-butyl piperazine-1-carboxylate (135 mg, 727 µmol, Eq: 1.50) was added. The reaction mixture was stirred at RT overnight. Water was added to the reaction and the product was extracted with AcOEt. The crude obtained was purified by flash chromatography (silica gel, 25 g, 0% to 10% MeOH in DCM) to afford the title compound (377.4 mg, 649 µmol, 130% yield) as an orange viscous oil. MS (ESI, m/z): 581.2 [M+H]⁺.

### Step 5)

### (2-Chloro-4-((3-(3-fluoro-4-methoxyphenyl)imidazo[1,2-a]pyrazin-8-yl)amino)phenyl)(piperazin-1-yl)methanone hydrochloride

Tert-butyl 4-(2-chloro-4-((3-(3-fluoro-4-methoxyphenyl)imidazo[1,2-a]pyrazin-8-yl)amino)benzoyl)piperazine-1-carboxylate (282 mg, 485 µmol, Eq: 1) was combined with HCl 4M in dioxane (1.21 ml, 4.85 mmol, Eq: 10). After 50 min, the reaction was evaporated to dryness to afford the title compound (300 mg, 579 µmol, 108% yield) as an off-white solid. MS (ESI, m/z): 479.4 [M-H]⁻.

### Step 6)

### Tert-butyl (2-(4-(2-chloro-4-((3-(3-fluoro-4-methoxyphenyl)imidazo[1,2-a]pyrazin-8-yl)amino)benzoyl)piperazin-1-yl)-2-oxoethyl)(methyl)carbamate

2-((tert-Butoxycarbonyl)(methyl)amino)acetic acid (110 mg, 580 µmol, Eq: 1.20) was combined with DMF (2.5 ml). DIPEA (250 mg, 338 µl, 1.93 mmol, Eq: 4.00) and HATU (276 mg, 725 µmol, Eq: 1.50) were added and after stirring for 15 min, (2-chloro-4-((3-(3-fluoro-4-methoxyphenyl)imidazo[1,2-a]pyrazin-8-yl)amino)phenyl)(piperazin-1-yl)methanone hydrochloride (250 mg, 483 µmol, Eq: 1) was added. After stirring for 2 h, water was added to the reaction mixture and it was extracted with AcOEt. The crude obtained after evaporation was purified by flash chromatography (silica gel, 20 g, 0% to 5% MeOH in DCM) to afford the title compound (224.6 mg, 344 µmol, 64.2% yield) as an off-white foam. MS (ESI, m/z): 652.2 [M+H]⁺.

### Step 7)

### 1-(4-(2-Chloro-4-((3-(3-fluoro-4-methoxyphenyl)imidazo[1,2-a]pyrazin-8-yl)amino)benzoyl)piperazin-1-yl)-2-(methylamino)ethanone

Tert-butyl (2-(4-(2-chloro-4-((3-(3-fluoro-4-methoxyphenyl)imidazo[1,2-a]pyrazin-8-yl)amino)benzoyl)piperazin-1-yl)-2-oxoethyl)(methyl)carbamate (220 mg, 337 µmol, Eq: 1) was combined with 4M HCl in dioxane (843 µl, 3.37 mmol, Eq: 10). After 1 h, the reaction was evaporated to dryness. The product was taken in DCM and basified with saturated Na₂CO₃. After extraction and removal of the solvent, the product was purified by prep HPLC to afford the title compound (48.2 mg, 87.3 µmol, 25.4% yield) as a light brown solid. MS (ESI, m/z): 552.2 [M+H]⁺.

### Step 8)

### [2-[4-[2-chloro-4-[[3-(3-fluoro-4-methoxyphenyl)imidazo[1,2-a]pyrazin-8-yl]amino]benzoyl]piperazin-1-yl]-2-oxoethyl]-trimethylazanium formate

1-(4-(2-chloro-4-((3-(3-fluoro-4-methoxyphenyl)imidazo[1,2-a]pyrazin-8-yl)amino)benzoyl)piperazin-1-yl)-2-(methylamino)ethanone (41 mg, 74.3 µmol, Eq: 1) was dissolved in 1,4-dioxane (69.6 µl) and DIPEA (29.6 mg, 40µl, 229 µmol, Eq: 3.08) was added. The reaction mixtures was stirred at room temperature for 20 min and iodomethane (105 mg, 46.2 µl, 743 µmol, Eq: 10) was added. The reaction mixture was stirred at room temperature for 24 h. The crude material was purified by preparative HPLC. The product was lyophilized to afford the title compound (28.7 mg, 45.8 µmol, 61.7% yield) as a white solid. MS (ESI, m/z): 580.4 [M]⁺.

### Example 32

### 2-(4-(4-((3-(3-Fluoro-4-methoxyphenyl)imidazo[1,2-a]pyrazin-8-yl)amino)-2-methylbenzoyl)piperazin-1-yl)-N,N,N-trimethyl-2-oxoethan-1-aminium iodide

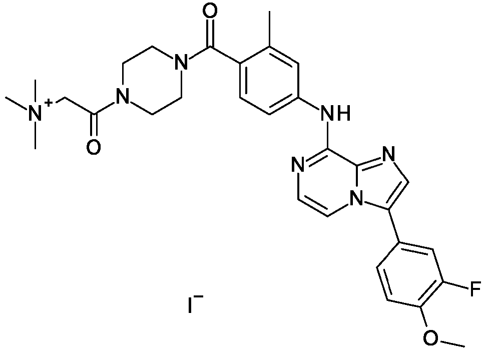

### Step 1)

### 2-(Dimethylamino)-1-(4-(4-((3-(3-fluoro-4-methoxyphenyl)imidazo[1,2-a]pyrazin-8-yl)amino)-2-methylbenzoyl)piperazin-1-yl)ethanone

4-((3-(3-Fluoro-4-methoxyphenyl)imidazo[1,2-a]pyrazin-8-yl)amino)-2-methylbenzoic acid (Intermediate 12, 30 mg, 76.5 µmol, Eq: 1), DIPEA (39.5 mg, 53.4 µl, 306 µmol, Eq: 4) and HATU (43.6 mg, 115 µmol, Eq: 1.5) were dissolved in DMF (500 µl). The reaction mixture was stirred at room temperature for 30 minutes. 2-(Dimethylamino)-1-(piperazin-1-yl)ethanone dihydrochloride (28 mg, 115 µmol, Eq: 1.5) was added and the reaction mixture was stirred at room temperature for 3 h. The crude material was purified by preparative HPLC to afford the title compound (23.5 mg, 43 µmol, 56.3% yield) as a light yellow oil. MS (ESI, m/z): 546.3 [M+H]⁺.

### Step 2)

### 2-(4-(4-((3-(3-Fluoro-4-methoxyphenyl)imidazo[1,2-a]pyrazin-8-yl)amino)-2-methylbenzoyl)piperazin-1-yl)-N,N,N-trimethyl-2-oxoethan-1-aminium iodide

2-(dimethylamino)-1-(4-(4-((3-(3-fluoro-4-methoxyphenyl)imidazo[1,2-a]pyrazin-8-yl)amino)-2-methylbenzoyl)piperazin-1-yl)ethanone (10 mg, 18.3 µmol, Eq: 1) was dissolved in 1,4-dioxane (1 ml) and iodomethane (52 mg, 22.8 µl, 367 µmol, Eq: 20) was added. The reaction mixture was stirred at room temperature for 1 h. The product was dried under HV and lyophilised to afford the title compound (13 mg, 18.9 µmol, 103% yield) as a white solid. MS (ESI, m/z): 560.5 [M]⁺.

### Example 33

### [4-[4-[4-[[3-[4-(Difluoromethoxy)phenyl]imidazo[1,2-a]pyrazin-8-yl]amino]-2-methylbenzoyl]piperazine-1-carbonyl]morpholin-2-yl]methyl-trimethyl-ammonium formate

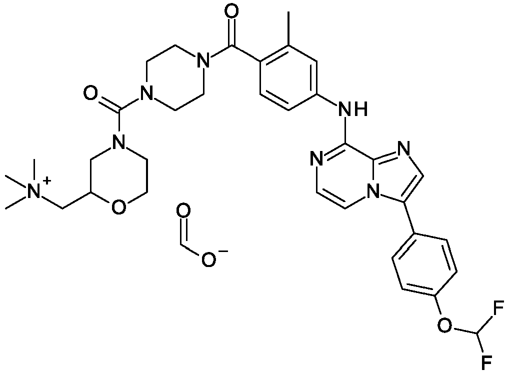

### Step 1)

### [4-[[3-[4-(difluoromethoxy)phenyl]imidazo[1,2-a]pyrazin-8-yl]amino]-2-methyl-phenyl]-[4-(imidazole-1-carbonyl)piperazin-1-yl]methanone

To a solution of N,N-diisopropylethylamine (0.11 mL, 0.630 mmol, 0.6 eq) in ACN (5 mL) was added [4-[[3-[4-(difluoromethoxy)phenyl]imidazo[1,2-a]pyrazin-8-yl]amino]-2-methyl-phenyl]-piperazin-1-yl-methanone (Intermediate 13, 500 mg, 1.04 mmol, 1 eq) and N.N'-carbonyldiimidazole (186.38 mg, 1.15 mmol, 1.1 eq). The reaction was stirred at 30 °C for 24 h. The reaction mixture was concentrated to afford the title compound (800 mg, 1.4 mmol, 133.71% yield) as a brown oil. MS (ESI, m/z): 573.2 [M+H]⁺.

### Step 2)

### [4-[[3-[4-(Difluoromethoxy)phenyl]imidazo[1,2-a]pyrazin-8-yl]amino]-2-methyl-phenyl]-[4-[2-[(dimethylamino)methyl]morpholine-4-carbonyl]piperazin-1-yl]methanone

To a solution of [4-[[4-[[3-[4-(difluoromethoxy)phenyl]imidazo[1,2-a]pyrazin-8-yl]amino]-2-methyl-phenyl]methyl]piperazin-1-yl]-imidazol-1-yl-methanone (320 mg, 0.570 mmol, 1 eq) in NMP (5 mL) was added N,N-dimethyl-2-morpholinmethanamine (99.14 mg, 0.690 mmol, 1.2 eq) and N,N-diisopropylethylamine (0.2 mL, 1.15 mmol, 2 eq). The reaction was stirred at 120 °C for 12 h. The mixture was purified by prep-HPLC (base) and lyophilized to afford the tile compound (80.5 mg, 0.120 mmol, 21.66% yield) as a yellow solid. MS (ESI, m/z): 649.3 [M+H]⁺.

### Step 3)

### [4-[4-[4-[[3-[4-(Difluoromethoxy)phenyl]imidazo[1,2-a]pyrazin-8-yl]amino]-2-methylbenzoyl]piperazine-1-carbonyl]morpholin-2-yl]methyl-trimethyl-ammonium formate

To a solution of [4-[[-[4-(difluoromethoxy)phenyl]imidazo[1,2-a]pyrazin-8-yl]amino]-2-methyl-phenyl]-[4-[2-[(dimethylamino)methyl]morpholine-4-carbonyl]piperazin-1-yl]methanone (60 mg, 0.090 mmol, 1 eq) in ACN (2 mL) was added iodomethane (39.38 mg, 0.280 mmol, 3 eq) and it was stirred at 25 °C for 12 h. The reaction mixture was purified by prep-HPLC (FA) to afford the title compound (20.1 mg, 0.030 mmol, 30.66% yield) as a white solid. MS (ESI, m/z): 665.3 [M]⁺.

### Example 34

### 2-Chloro-4-[[3-[4-(cyanomethoxy)-2,3-difluoro-phenyl]imidazo[1,2-a]pyrazin-8-yl]amino]-N-[2-(4,4-dimethylpiperazin-4-ium-1-yl)ethyl]-N-methyl-benzamide iodide

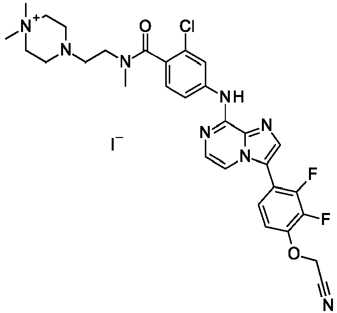

### Step 1)

### tert-Butyl 4-[2-[[2-chloro-4-[[3-[4-(cyanomethoxy)-2,3-difluoro-phenyl]imidazo[1,2-a]pyrazin-8-yl]amino]benzoyl]-methyl-amino]ethyl]piperazine-1-carboxylate

To a solution of 2-chloro-4-((3-(4-(cyanomethoxy)-2,3-difluorophenyl)imidazo[1,2-a]pyrazin-8-yl)amino)benzoic acid (Intermediate 10) (456 mg, 1.0 mmol, Eq: 1.0), *tert*-butyl 4-(2-(methylamino)ethyl)piperazine-1-carboxylate (300 mg, 1.2 mmol, Eq: 1.2) in anhydrous DMF (15 mL) was added DIPEA (258 mg, 2.0 mmol, Eq: 2.0). Then the resultant mixture was stirred for 30 min at room temperature, HATU (760 mg, 2.0 mmol, Eq: 2.0) was added in the mixture and stirred for extra 10 h. Poured the mixture into water (50 mL) and the aqueous solution was extracted with DCM (50 mL×2). Combined the organic layers and washed with water and brine. Dried over anhydrous sodium sulfate. Concentrated under reduced pressure to give the title compound, which used in next step without purification. (305 mg, 0.45 mmol, 45.0 % yield) as a yellow solid. MS (ESI, m/z): 681.2 [M+H]⁺.

### Step 2)

### 2-Chloro-4-[[3-[4-(cyanomethoxy)-2,3-difluoro-phenyl]imidazo[1,2-a]pyrazin-8-yl]amino]-N-methyl-N-(2-piperazin-1-ylethyl)benzamide

To a solution of *tert*-butyl 4-(2-(2-chloro-4-((3-(4-(cyanomethoxy)-2,3-difluorophenyl)imidazo[1,2-a]pyrazin-8-yl)amino)-N-methylbenzamido)ethyl)piperazine-1-carboxylate (305 mg, 0.45 mmol) in MeOH (10 ml) was added 3M hydrochloric acid (2.0 mL) at room temperature. The resultant mixture was stirred for 10 h and then adjusted to pH=7-8 with 2M aqueous solution of sodium carbonate. The mixture was extracted with DCM (50 mL×2), the combined organic layers were washed with water and brine, dried over anhydrous sodium sulfate and concentrated to give a red oil which was purified by Prep.HPLC to afford the title compound (100 mg, 0.17 mmol, 37.8 % yield) as an off-white powder. MS (ESI, m/z): 581.2 [M+H]⁺.

### Step 3)

### 2-Chloro-4-[[3-[4-(cyanomethoxy)-2,3-difluoro-phenyl]imidazo [1,2-a]pyrazin-8-yl]amino]-N[2-(4,4-dimethylpiperazin-4-ium-1-yl)ethyl]-N-methyl-benzamide;iodide

To a solution of 2-chloro-4-((3-(4-(cyanomethoxy)-2,3-difluorophenyl)imidazo[1,2-a]pyrazin-8-yl)amino)-N-methyl-N-(2-(piperazin-1-yl)ethyl)benzamidel (100 mg, 0.17 mmol, Eq: 1.0) in anhydrous EtOH (3 mL) was added DIPEA (44 mg, 0.34 mmol, Eq: 2.0). Then the resultant mixture was stirred for 10 min at room temperature, iodomethane (241 mg, 1.7 mmol, Eq: 10) was added in the mixture and stirred for extra 10 hr. The mixture was concentrated under reduced pressure to give a red oil, purified by Prep.HPLC to provide the title compound (60 mg, 0.081 mmol, 47.6 % yield) as a white powder. MS (ESI, m/z): 609.3 [M]⁺.

### Example 35

### 2-(2-(4-((3-(3-Fluoro-4-methoxyphenyl)imidazo[1,2-a]pyrazin-8-yl)amino)-N,2-dimethylbenzamido)ethoxy)-N,N,N-trimethylethan-1-aminium chloride

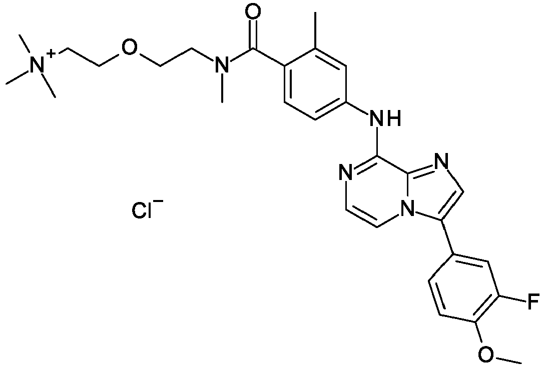

### Step 1)

### N-(2-(2-Chloroethoxy)ethyl)-4-((3-(3-fluoro-4-methoxyphenyl)imidazo[1,2-a]pyrazin-8-yl)amino)-N,2-dimethylbenzamide

4-((3-(3-fluoro-4-methoxyphenyl)imidazo[1,2-a]pyrazin-8-yl)amino)-2-methylbenzoic acid (Intermediate 12, 500 mg, 1.27 mmol, Eq: 1) was combined with DMF (5 ml). HATU (727 mg, 1.91 mmol, Eq: 1.50), DIPEA (659 mg, 890 µl, 5.1 mmol, Eq: 4.00) and 2-(2-chloroethoxy)-N-methylethanamine hydrochloride (333 mg, 1.91 mmol, Eq: 1.50) were added and the reaction mixture was stirred at RT. After 4 h, water was added to the reaction mixture and the extraction was done using AcOEt. The crude obtained was purified by prep. HPLC to afford the title compound (430 mg, 0.84 mmol, 65.9% yield) as a brown foam. MS (ESI, m/z): 512.2 [M+H]⁺.

### Step 2)

### 2-(2-(4-((3-(3-Fluoro-4-methoxyphenyl)imidazo[1,2-a]pyrazin-8-yl)amino)-N,2-dimethylbenzamido)ethoxy)-N,N,N-trimethylethan-1-aminium chloride

N-(2-(2-chloroethoxy)ethyl)-4-((3-(3-fluoro-4-methoxyphenyl)imidazo[1,2-a]pyrazin-8-yl)amino)-N,2-dimethylbenzamide (11.5 mg, 22.5 µmol, Eq: 1) was combined with dioxane (100 µl). TEA (11.4 mg, 15.7 µl, 112 µmol, Eq: 5.00) and 45% aqueous trimethylamine solution (6.64 mg, 112 µmol, Eq: 5.00) were added and the reaction was heated to 65°C. After 2 h stirring, again 45% aqueous trimethylamine (6.64 mg, 112 µmol, Eq: 5.00) was added and the stirring at 65°C was continued for 24 h. The reaction mixture was then transfered into a sealed tube, again 45% aqueous trimethylamine () was added and it was stirred at 90°C overnight. The reaction mixture was evaporated and dried under HV for 1 h to afford the title compound (8.7 mg, 15.2 µmol, 63% yield) as an off-white solid. MS (ESI, m/z): 535.3 [M]⁺.

### Example 36

### 2-[[4-[4-[[3-[4-(Difluoromethoxy)phenyl]imidazo[1,2-a]pyrazin-8-yl]amino]-2-methylbenzoyl]piperazine-1-carbonyl]amino]ethyl-trimethyl-ammonium formate

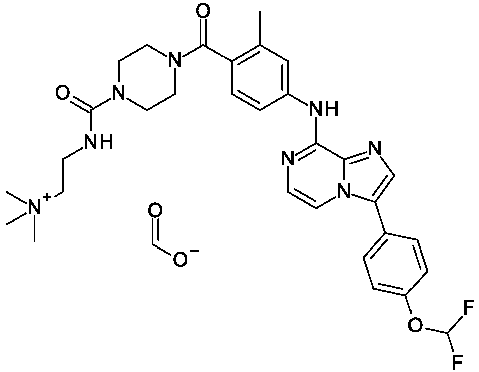

### Step 1)

### 4-[4-[[3-[4-(difluoromethoxy)phenyl]imidazo[1,2-a]pyrazin-8-yl]amino]-2-methyl-benzoyl]-N-[2-(dimethylamino)ethyl]piperazine-1-carboxamide

To a solution of [4-[[3-[4-(difluoromethoxy)phenyl]imidazo[1,2-a]pyrazin-8-yl]amino]-2-methyl-phenyl]-piperazin-1-yl-methanone (Intermediate 13, 200 mg, 0.420 mmol, 1 eq) in DCM (5 mL) was added N,N-diisopropylethylamine (0.36 mL, 2.09 mmol, 5 eq) and bis(trichloromethyl)carbonate (49.61 mg, 0.170 mmol, 0.4 eq) at 0 °C. The mixture was stirred at 0°C for 1 h, then N,N-dimethylethylenediamine (110.54 mg, 1.25 mmol, 3 eq) was added. The reaction mixture was stirred at 25 °C for 12 h. The mixture was concentrated and purified by prep-HPLC (base) and then lyophilized to afford the title compound (21.1 mg, 0.040 mmol, 8.52% yield) as a yellow solid. MS (ESI, m/z): 593.3 [M+H]⁺.

### Step 2)

### 2-[[4-[4-[[3-[4-(difluoromethoxy)phenyl]imidazo[1,2-a]pyrazin-8-yl]amino]-2-methylbenzoyl]piperazine-1-carbonyl]amino]ethyl-trimethyl-ammonium formate

The title compound (61.3 mg, 0.090 mmol, 70.55% yield) was obtained as a yellow solid in analogy to Example **33,** Step 3. MS (ESI, m/z): 607.2 [M]⁺.

### Example 37

### 2-[[(3S,4R)-1-[4-[[3-(3-fluoro-4-methoxy-phenyl)imidazo[1,2-a]pyrazin-8-yl]amino]-2-methylbenzoyl]-3-hydroxy-piperidine-4-carbonyl]amino]ethyl-trimethyl-ammonium formate

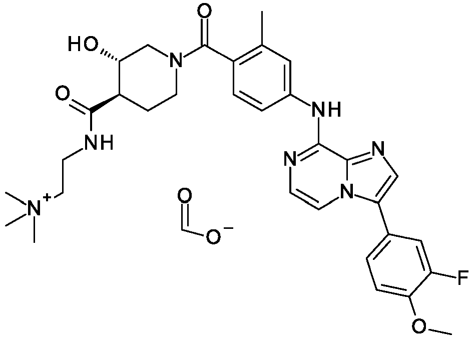

### Step 1)

### ethyl (3S,4R)-1-[4-[[3-(3-fluoro-4-methoxy-phenyl)imidazo[1,2-a]pyrazin-8-yl]amino]-2-methyl-benzoyl]-3-hydroxy-piperidine-4-carboxylate

To a solution of O-(7-azabenzotriazol-1-yl)-N,N,N',N'-tetramethyluronium hexafluorophosphate (1.16 g, 3.06 mmol, 2 eq) in DMF (20 mL) were added N,N-diisopropylethylamine (0.8 mL, 4.59 mmol, 3 eq), 4-[[3-(3-fluoro-4-methoxy-phenyl)imidazo[1,2-a]pyrazin-8-yl]amino]-2-methyl-benzoic acid (Intermediate 12, 600 mg, 1.53 mmol, 1 eq) and ethyl (3S,4R)-3-hydroxypiperidine-4-carboxylate (317.83 mg, 1.83 mmol, 1.2 eq). The reaction was stirred at 25 °C for 12 h. 50 mL of water was added and it was extracted with EtOAc (50 mL x 3). The combined organic layers were washed with 1N HCl (50 mL x 2), brine (50 mL), dried over Na₂SO₄, filtered and concentrated to afford the title compound (700 mg, 1.28 mmol, 83.6% yield) as a dark green oil. MS (ESI, m/z): 548.2 [M+H]⁺.

### Step 2)

### (3S,4R)-1-[4-[[3-(3-Fluoro-4-methoxy-phenyl)imidazo[1,2-a]pyrazin-8-yl]amino]-2-methylbenzoyl]-3-hydroxy-piperidine-4-carboxylic acid

To a solution of aqueous sodium hydroxide (10 mL, 20 mmol, 19.91 eq) in THF (10 mL)/methanol (10 mL) was added ethyl (3S,4R)-1-[4-[[3-(3-fluoro-4-methoxyphenyl)imidazo[1,2-a]pyrazin-8-yl]amino]-2-methyl-benzoyl]-3-hydroxy-piperidine-4-carboxylate (550 mg, 1 mmol, 1 eq) and it was stirred at 20 °C for 24 h. The reaction mixture was adjusted to pH=1~2 by 3N HCl addition and was extracted with DCM:MeOH=10:1 (60 mL x 3), dried over Na₂SO₄ and concentrated to afford the title compound (310 mg, 0.600 mmol, 59.41% yield) as a light yellow solid. MS (ESI, m/z): 520.2 [M+H]⁺.

### Step 3)

### (3S,4R)-N-[2-(Dimethylamino)ethyl]-1-[4-[[3-(3-fluoro-4-methoxy-phenyl)imidazo[1,2-a]pyrazin-8-yl]amino]-2-methyl-benzoyl]-3-hydroxy-piperidine-4-carboxamide

To a solution of O-(7-azabenzotriazol-1-yl)-N,N,N',N'-tetramethyluronium hexafluorophosphate (146.38 mg, 0.380 mmol, 2 eq) in DMF (10 mL) was added N,N-diisopropylethylamine (0.1 mL, 0.580 mmol, 3 eq), N,N-dimethylethylenediamine (20.36 mg, 0.230 mmol, 1.2 eq) and (3S,4R)-1-[4-[[3-(3-fluoro-4-methoxy-phenyl)imidazo[1,2-a]pyrazin-8-yl]amino]-2-methyl-benzoyl]-3-hydroxy-piperidine-4-carboxylic acid (100 mg, 0.190 mmol, 1 eq). The reaction was stirred at 25 °C for 12 h. 50 mL of water was added and it was extracted with EtOAc (50 mL x 3). The combined organic layers were washed with brine (50 mL), dried over Na₂SO₄, filtered and concentrated to afford the title compound (100 mg, 0.170 mmol, 88.11% yield) as a dark green oil. MS (ESI, m/z): 590.3 [M+H]⁺.

### Step 4)

### 2-[1(3S,4R)-1-[4-[[3-(3-fluoro-4-methoxy-phenyl)imidazo[1,2-a]pyrazin-8-yl]amino]-2-methyl-benzoyl]-3-hydroxy-piperidine-4-carbonyl]amino]ethyl-trimethyl-ammonium formate

To a solution of (3S,4R)-N-[2-(dimethylamino)ethyl]-1-[4-[[3-(3-fluoro-4-methoxyphenyl)imidazo[1,2-a]pyrazin-8-yl]amino]-2-methyl-benzoyl]-3-hydroxy-piperidine-4-carboxamide (100 mg, 0.170 mmol, 1 eq) in ACN (5 mL) was added methyl iodide (48.14 mg, 0.340 mmol, 2 eq) and the reaction mixture was stirred at 40 °C for 12 h. The reaction was concentrated under reduce pressure and purified by prep-HPLC (FA) to afford the title compound (21 mg, 0.030 mmol, 18.81% yield) as a white solid. MS (ESI, m/z): 604.3 [M]⁺.

### Example 38

### 2-Chloro-4-[[3-[4-(cyanomethoxy)-2,3-difluoro-phenyl]imidazo[1,2-a]pyrazin-8-yl]amino]-N-[2-(1,1-dimethylpiperidin-1-ium-4-yl)ethyl]-N-methyl-benzamide 2,2,2-trifluoroacetate

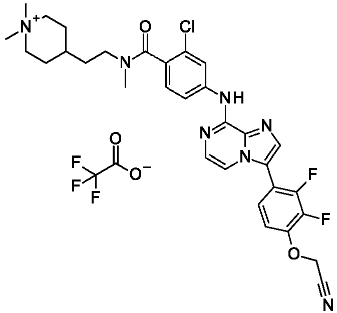

### Step 1)

### tert-Butyl 4-[2-[[2-chloro-4-[[3-[4-(cyanomethoxy)-2,3-difluoro-phenyl]imidazo[1,2-a]pyrazin-8-yl]amino]benzoyl]-methyl-amino]ethyl]piperidine-1-carboxylate

To a solution of 2-chloro-4-((3-(4-(cyanomethoxy)-2,3-difluorophenyl)imidazo[1,2-a]pyrazin-8-yl)amino) benzoic acid (Intermediate 10) (456 mg, 1.0 mmol, Eq: 1.0), *tert*-butyl 4-(2-(methylamino)ethyl)piperidine-1-carboxylate (291 mg, 1.2 mmol, Eq: 1.2) in anhydrous DMF (15 mL) was added DIPEA (258 mg, 2.0 mmol, Eq: 2.0). Then the resultant mixture was stirred for 30 min at room temperature, HATU (760 mg, 2.0 mmol, Eq: 2.0) was added in the mixture and stirred for extra 10 h. Poured the mixture into water (50 mL) and the aqueous solution was extracted with DCM (50 mL×2). Combined the organic layers and washed with water and brine, dried over anhydrous sodium sulfate and concentrated under reduced pressure to give the title compound, which used in next step without purification. (306 mg, 0.45 mmol, 45.0 % yield) as a yellow solid. MS (ESI, m/z): 680.2 [M+H]⁺.

### Step 2)

### 2-Chloro-4-[[3-[4-(cyanomethoxy)-2,3-difluoro-phenyl]imidazo[1,2-a]pyrazin-8-yl]amino]-N-methyl-N-[2-(4-piperidyl)ethyl]benzamide

To a solution of *tert*-butyl 4-(2-(2-chloro-4-((3-(4-(cyanomethoxy)-2,3-difluorophenyl)imidazo[1,2-a]pyrazin-8-yl)amino)-N-methylbenzamido)ethyl)piperidine-1-carboxylatete (306 mg, 0.45 mmol) in MeOH (10 ml) was added 3.0 M hydrochloric acid (2.0 mL) at room temperature. The resultant mixture was stirred for 10 h and then adjusted to pH=7-8 with 2M aqueous solution of sodium carbonate. The mixture was extracted with DCM (50 mL×2), the combined organic layers were washed with water and brine, dried over anhydrous sodium sulfate and concentrated to give a red oil which was purified by Prep.HPLC to afford the title compound (145 mg, 0.25 mmol, 55.6 % yield) as an off-white powder. MS (ESI, m/z): 580.3 [M+H]⁺.

### Step 3)

### 2-Chloro-4-[[3-[4-(cyanomethoxy)-2,3-difluoro-phenyl]imidazo[1,2-a]pyrazin-8-yl]amino]-N-[2-(1,1-dimethylpiperidin-1-ium-4-yl)ethyl]-N-methyl-benzamide 2,2,2-trifluoroacetate

To a solution of 2-chloro-4-((3-(4-(cyanomethoxy)-2,3-difluorophenyl)imidazo[1,2-a]pyrazin-8-yl)amino)-N-methyl-N-(2-(piperidin-4-yl)ethyl)benzamide (145 mg, 0.25 mmol, Eq: 1.0) in anhydrous EtOH (5 mL) was added DIPEA (64 mg,0.5 mmol, Eq: 2.0). Then the resultant mixture was stirred for 10 min at room temperature, Iodomethane (142 mg, 1.0 mmol, Eq: 4.0) was added in the mixture and stirred for extra 10 hr. The mixture was concentrated under reduced pressure to give a white solid which was purified by Prep.HPLC to provide the title compound (75 mg, 0.104 mmol, 41.6 % yield) MS (ESI, m/z): 608.3 [M]⁺.

### Example 39

### 4-(4-(2-(4-((3-(4-(Difluoromethoxy)phenyl)imidazo[1,2-a]pyrazin-8-yl)amino)-N,2-dimethylbenzamido)ethyl)piperidin-1-yl)-2-hydroxy-N,N,N-trimethyl-4-oxobutan-1-aminium formate

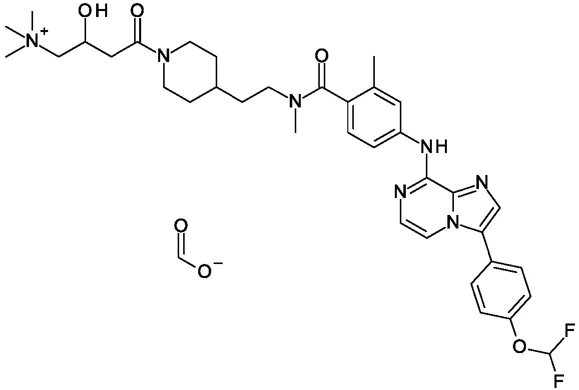

In a 10 ml round bottom flask 4-((3-(4-(difluoromethoxy)phenyl)imidazo[1,2-a]pyrazin-8-yl)amino)-N,2-dimethyl-N-(2-(piperidin-4-yl)ethyl)benzamide (Intermediate 3, 30 mg, 56.1 µmol, Eq: 1), 3-carboxy-2-hydroxy-N,N,N-trimethylpropan-1-aminium (13.7 mg, 84.2 µmol, Eq: 1.5) and DIPEA (29 mg, 39.2 µl, 224 µmol, Eq: 4) were dissolved in DMF (750 µl) to give a brown solution. The reaction mixture was stirred at room temperature for 1 minute. HATU (32 mg, 84.2 µmol, Eq: 1.5) was added and the reaction mixture was stirred at room temperature for 3 hours. The crude material was directly purified by preparative HPLC. The crude material was purified by preparative HPLC to afford the title compound (9.8 mg, 13.5 µmol, 24.1% yield) as a cololess oil. MS (ESI, m/z): 678.36 [M]⁺.

### Example 40

### 2-(2-(4-((3-(2,3-Difluoro-4-methoxyphenyl)imidazo[1,2-a]pyrazin-8-yl)amino)-2-ethylbenzamido)ethoxy)-N,N,N-triethylethan-1-aminium formate

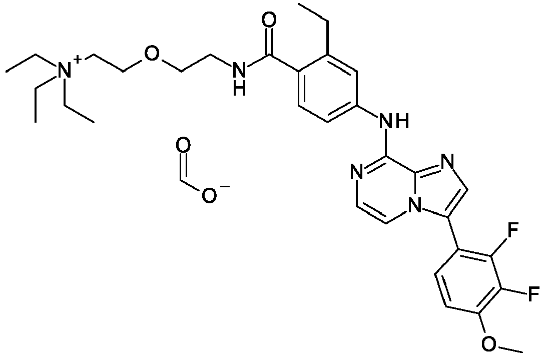

N-(2-(2-Aminoethoxy)ethyl)-4-((3-(2,3-difluoro-4-methoxyphenyl)imidazo[1,2-a]pyrazin-8-yl)amino)-2-ethylbenzamide hydrochloride (Intermediate 9, 30 mg, 54.8 µmol, Eq: 1) was dissolved in 1,4-dioxane (5 ml) and DIPEA (37 mg, 50 µL, 286 µmol, Eq: 5.22) was added. The reaction mixture was stirred at room temperature for 20 min and iodoethane (117 mg, 60 µL, 750 µmol, Eq: 13.7) was added. The reaction mixture was stirred at room temperature over the weekend. DIPEA (37 mg, 50 µL, 286 µmol, Eq: 5.22) and iodoethane (117 mg, 60 µL, 750 µmol, Eq: 13.7) were added again and the reaction mixture was stirred at room temperature for 4 h and then heated and stirred at 55°C. The crude material was purified by preparative HPLC. The product was lyophilized to afford the title compound (13 mg, 20.2 µmol, 37% yield) as a white solid. MS (ESI, m/z): 595.6 [M]⁺.

### Example 41

### 2-[2-[[4-[[3-[4-(Cyanomethoxy)-2,3-difluoro-phenyl]imidazo[1,2-a]pyrazin-8-yl]amino]-2-ethyl-benzoyl]amino]ethoxy]ethyl-trimethyl-ammonium iodide

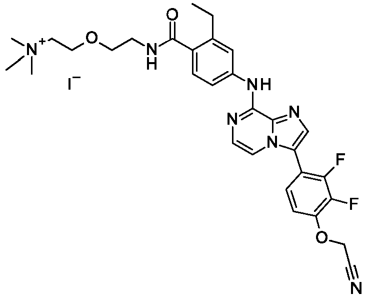

### Step 1)

### 2-[4-(8-chloroimidazo[1,2-a]pyrazin-3-yl)-2,3-difluoro-phenoxylacetonitrile

A mixture of 8-chloro-3-iodo-imidazo[1,2-a]pyrazine (2.79 g, 9.98 mmol, 1 eq), 2-[2,3-difluoro-4-(4,4,5,5-tetramethyl-1,3,2-dioxaborolan-2-yl)phenoxy]acetonitrile (4.42 g, 14.97 mmol, 1.5 eq), [1,1'-bis(diphenylphosphino)ferrocene]dichloropalladium(II) (0.73 g, 1 mmol, 0.100 eq), and phosphoric acid, potassium salt (2.48 mL, 29.95 mmol, 3 eq) in THF (50 mL) and water (25 mL) at 45 °C under nitrogen for 14 h. The mixture was added water (5 mL) and extracted with EA (10 mL × 3). The combined organic layers were concentrated to dryness. The crude was then purified by flash column chromatography eluting 20% EtOAc in PE to afford the title compound (2.59 g, 8.07 mmol, 80.81% yield) as a brown solid. MS (ESI, m/z): 321.7 [M+H]⁺.

### Step 2) Intermediate 14

### 4-[[3-[4-(Cyanomethoxy)-2,3-difluoro-phenyl]imidazo[1,2-a]pyrazin-8-yl]amino]-2-ethyl-benzoic acid

A mixture of 2-[4-(8-chloroimidazo[1,2-a]pyrazin-3-yl)-2,3-difluoro-phenoxy]acetonitrile (573.0 mg, 1.79 mmol, 1 eq) and 4-amino-2-ethyl-benzoic acid (354.2 mg, 2.14 mmol, 1.2 eq) in acetonitrile (20 mL) and acetic acid (4 mL) was stirred at 100 °C for 3 h. The mixture was concentrated to dryness. The residual was treated with EA (10 mL). The mixture was stirred at 25 °C for 5 min and filtered to collect the solid which was dried to the title compound (556 mg, 1.24 mmol, 69.24% yield) as a brown solid. MS (ESI, m/z): 448.5[M-H]⁻.

### Step 3)

### 4-[[3-[4-(Cyanomethoxy)-2,3-difluoro-phenyl]imidazo[1,2-a]pyrazin-8-yl]amino]-N-[2-[2-(dimethylamino)ethoxy]ethyl]-2-ethyl-benzamide

A mixture of 4-[[3-[4-(cyanomethoxy)-2,3-difluoro-phenyl]imidazo[1,2-a]pyrazin-8-yl]amino]- 2-ethyl-benzoic acid (220.0 mg, 0.490 mmol, 1 eq Intermediate 14), 2-[2-(dimethylamino)ethoxy]ethanamine (97.07 mg, 0.730 mmol, 1.5 eq), O-(7-azabenzotriazol-1-yl)-N,N,N',N'-tetramethyluronium hexafluorophosphate (279.2 mg, 0.730 mmol, 1.5 eq) and N,N-diisopropylethylamine (0.26 mL, 1.47 mmol, 3 eq) in DMF (10 mL) was stirred at 25 °C for 3 h. The mixture was added water (15 mL) and extracted with EA (10 mL × 3). The combined organic layers were washed by NH₄Cl (20 mL), dried over Na₂SO₄ and concentrated to dryness. The crude was then purified by flash column chromatography eluting 10% MeOH in DCM. The desired fractions were concentrated to dryness in vacuum to afford the title compound (111.8 mg, 39.71% yield) as a light grey powder. MS (ESI, m/z): 564.6 [M+H]⁺.

### Step 4)

### 2-[2-[[4-[[3-[4-(Cyanomethoxy)-2,3-difluoro-phenyl]imidazo[1,2-a]pyrazin-8-yl]amino]-2-ethyl-benzoyl]amino]ethoxy]ethyl-trimethyl-ammonium iodide

A mixture of 4-[[3-[4-(cyanomethoxy)-2,3-difluoro-phenyl]imidazo[1,2-a]pyrazin-8-yl]amino]-N-[2-[2-(dimethylamino)ethoxy]ethyl]-2-ethyl-benzamide (53.0 mg, 0.090 mmol, 1 eq), iodomethane (40.04 mg, 0.280 mmol, 3 eq) and N,N-diisopropylethylamine (0.03 mL, 0.190 mmol, 2 eq) in ethanol (6 mL) was stirred at 80 °C for 14 h. The mixture was concentrated to dryness. The crude was purified by prep-HPLC. The desired fractions were concentrated to dryness in vacuo to the title compouind (30 mg, 0.040 mmol, 44.94% yield) as a pink gum. MS (ESI, m/z): 578.6 [M]⁺.

### Example 42

### [2-[4-[4-[[3-[4-(Difluoromethoxy)phenyl]imidazo[1,2-a]pyrazin-8-yl]amino-2-methylbenzoyl[piperazin-1-yl]-2-oxo-ethyl]-trimethyl-ammonium chloride

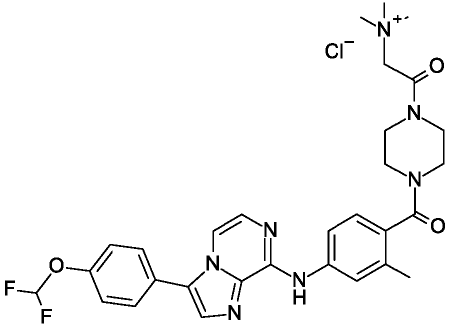

To a mixture of [4-[[3-[4-(difluoromethoxy)phenyl]imidazo[1,2-a]pyrazin-8-yl]amino]-2-methyl-phenyl]-piperazin-1-yl-methanone (Intermediate 13, 80 mg, 0.170 mmol, 1 eq) and carboxymethyl(trimethyl)ammonium chloride (19.75 mg, 0.170 mmol, 1 eq) in DMF (1 mL) was added 1-propanephosphonic anhydride (159.59 mg, 0.250 mmol, 1.5 eq) (50% in DMF solution) and triethylamine (0.07 mL, 0.500 mmol, 3 eq) slowly at 25 °C. The mixture was stirred at 25 °C for 48 h. Then the mixture was filtered and purified by prep-HPLC (TFA) to afford the title compound (15.32 mg, 0.020 mmol, 13.7% yield) as a white solid. MS (ESI, m/z): 578.2 [M]⁺.

### Example 43

### 2-[2-[[4-[[3-[2-chloro-4-(cyanomethoxy)-3-fluoro-phenyl]imidazo[1,2-a]pyrazin-8-yl]amino]-2-ethyl-benzoyl]amino]ethoxy]ethyl-trimethyl-ammonium iodide

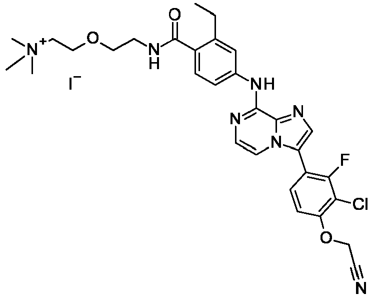

### Step 1)

### 2-[2-chloro-3-fluoro-4-(4,4,5,5-tetramethyl-1,3,2-dioxaborolan-2-yl)phenoxy]acetonitrile

This compound was prepared in analogy to Example 2, step 1 using 4-bromo-2-chloro-3-fluorophenol. The title compound was obtained as a pink solid. MS (ESI, m/z): 312.1 [M+H]⁺.

### Step 2)

### 2-[2-[[4-[[3-[2-chloro-4-(cyanomethoxy)-3-fluoro-phenyl]imidazo[1,2-a]pyrazin-8-yl]amino]-2-ethyl-benzoyl]amino]ethoxy]ethyl-trimethyl-ammonium iodide

This compound was prepared in analogy to example 41 using 2-[3-chloro-2-fluoro-4-(4,4,5,5-tetramethyl-1,3,2-dioxaborolan-2-yl)phenoxy]acetonitrile. The title compound was obtained as a light yellow solid. MS (ESI, m/z): 594.2 [M+]⁺.

### Example 44

### 2-[2-[[2-bromo-4-[[3-(2,3-difluoro-4-methoxy-phenyl)imidazo[1,2-a]pyrazin-8-yl]amino]benzoyl]amino]ethoxy]ethyl-trimethyl-ammonium 2,2,2-trifluoroacetate

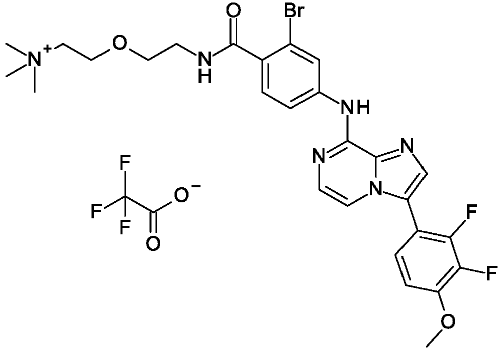

### Step 1) Intermediate 17

### 2-(2,3-difluoro-4-methoxy-phenyl)-4,4,5,5-tetramethyl-1,3,2-dioxaborolane

The title compound (3 g, 11.11 mmol, 22.52% yield) was obtained as a white solid in analogy to Example **14,** Step 3 using 1-bromo-2,3-difluoro-4-methoxy-benzene.

### Step 2)

### methyl 2-bromo-4-[[3-(2,3-difluoro-4-methoxy-phenyl)imidazo[1,2-a]pyrazin-8-yl]amino]benzoate

The title compound (260 mg, 0.530 mmol, 62.85% yield) was obtained as an off-white solid in analogy to Example **14,** Step 4 using Intermediate 16. MS (ESI, m/z): 489.0, 491.0 [M+H]⁺.

### Step 3)

### 2-bromo-4-[[3-(2,3-difluoro-4-methoxy-phenyl)imidazo[1,2-a]pyrazin-8-yl]amino]benzoic acid

The title compound (246 mg, 0.520 mmol, 97.58% yield) was obtained as a light yellow solid in analogy to Example **14,** Step 5. MS (ESI, m/z): 475.0, 477.0 [M+H]⁺.

### Step 4)

### N-[2-(2-aminoethoxy)ethyl]-2-bromo-4-[[3-(2,3-difluoro-4-methoxy-phenyl)imidazo[1,2-a]pyrazin-8-yl]amino]benzamide formate

The title compound (75 mg, 0.120 mmol, 31.11% yield) was obtained as a light yellow solid in analogy to Example **14,** Step 6. MS (ESI, m/z): 561.0, 563.0 [M+H]⁺.

### Step 5)

### 2-[2-[[2-bromo-4-[[3-(2,3-difluoro-4-methoxy-phenyl)imidazo[1,2-a]pyrazin-8-yl]amino]benzoyl]amino]ethoxy]ethyl-trimethyl-ammonium 2,2,2-trifluoroacetate

The title compound (11 mg, 0.020 mmol, 18.57% yield) was obtained as a white solid in analogy to Example **14,** Step 7. MS (ESI, m/z): 603.0, 605.0 [M]⁺.

### Example 45

### 2-[2-[[2-Chloro-4-[[3-[4-(cyanomethoxy)-2,3-difluoro-phenyl]imidazo[1,2-a]pyrazin-8-yl]amino]benzoyl]amino]ethoxy]ethyl-tripropyl-ammonium iodide

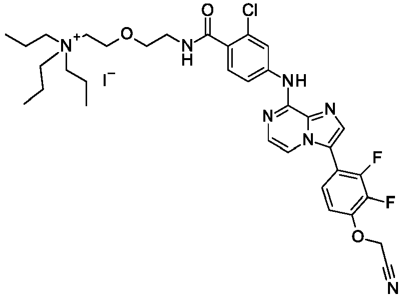

### Step 1)

### 2-Chloro-4-[[3-[4-(cyanomethoxy)-2,3-difluoro-phenyl]imidazo[1,2-a]pyrazin-8-yl]amino]-N-[2-[2-(dipropylamino)ethoxy]ethyl]benzamide

A mixture of N-[2-(2-aminoethoxy)ethyl]-N-propyl-propan-1-amine (0.001 mL, 0.490 mmol, 1.5 eq), 2-chloro-4-[[3-[4-(cyanomethoxy)-2,3-difluoro-phenyl]imidazo[1,2-a]pyrazin-8-yl]amino]benzoic acid (150.0 mg, 0.330 mmol, 1 eq, Intermediate 10), N,N-diisopropylethylamine (0.17 mL, 0.990 mmol, 3 eq) and 1-ethyl-3-(3-dimethylaminopropyl)carbodiimide hydrochloride (75.7 mg, 0.390 mmol, 1.2 eq) in DMF (3 mL) was stirred at 25 °C for 14 h. The mixture was added water (10 mL) and extracted with EA (10 mL × 3). The combined organic layers were concentrated to dryness. The crude was purified by column chromatography (DCM/MeOH from 100% to 10%) to afford the title compound (123 mg, 0.200 mmol, 59.7% yield) as a brown solid.

MS (ESI, m/z): 626.2, 628.2 [M+H]⁺.

### Step 2)

### 2-[2-[[2-Chloro-4-[[3-[4-(cyanomethoxy)-2,3-difluoro-phenyl]imidazo[1,2-a]pyrazin-8-yl]amino]benzoyl]amino]ethoxy]ethyl-tripropyl-ammonium iodide

A mixture of 2-chloro-4-[[3-[4-(cyanomethoxy)-2,3-difluoro-phenyl]imidazo[1,2-a]pyrazin-8-yl]amino]-N-[2-[2-(dipropylamino)ethoxy]ethyl]benzamide (123.0 mg, 0.200 mmol, 1 eq), N,N-diisopropylethylamine (0.07 mL, 0.390 mmol, 2 eq) and 1-iodopropane (100.19 mg, 0.590 mmol, 3 eq) in ethanol (5 mL) was stirred at 100 °C for 48 h. The mixture was concentrated to dryness. The crude was purified by prep-HPLC. The desired fractions was dried by lyophilization to afford the title compound (60.3 mg, 0.080 mmol, 37.7% yield) as a white solid. MS (ESI, m/z): 668.3, 670.2 [M]⁺.

### Example 46

### 2-Chloro-4-[[3-[4-(cyanomethoxy)-2,3-difluoro-phenyl]imidazo[1,2-a]pyrazin-8-yl]amino]-N-[2-[2-(1-methylpyrrolidin-1-ium-1-yl)ethoxy]ethyl]benzamide formate

A mixture of 2-[2-(1-methylpyrrolidin-1-ium-1-yl)ethoxy]ethanamine bromide (0.0 mL, 0.440 mmol, 2 eq), 2-chloro-4-[[3-[4-(cyanomethoxy)-2,3-difluoro-phenyl]imidazo[1,2-a]pyrazin-8-yl]amino]benzoic acid (Intermediate 10, 100.0 mg, 0.220 mmol, 1 eq), N,N-diisopropylethylamine (0.11 mL, 0.660 mmol, 3 eq), 1-hydroxybenzotrizaole (35.58 mg, 0.260 mmol, 1.2 eq) and N-(3-dimethylaminopropyl)-N'-ethylcarbodiimide hydrochloride (50.47 mg, 0.260 mmol, 1.2 eq) in DMF (3 mL) was stirred at 25 °C for 14 h. The mixture was purified by prep-HPLC to the title compound (47.9 mg, 0.070 mmol, 32.87% yield) as a white solid. MS (ESI, m/z): 610.2, 612.1 [M]⁺.

### Example 47

### 3-[[2-Chloro-4-[[3-(2,3-difluoro-4-methoxy-phenyl)imidazo[1,2-a]pyrazin-8-yl]amino]benzoyl]amino]propyl-trimethyl-ammonium formate

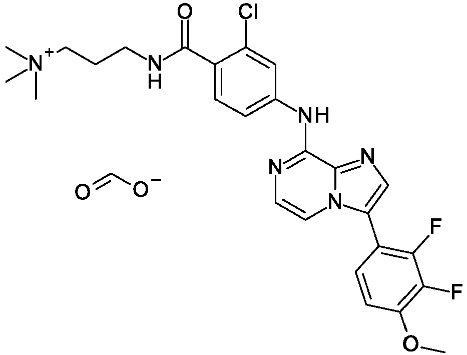

### Step 1)

### Methyl 2-chloro-4-[[3-(2,3-difluoro-4-methoxy-phenyl)imidazo[1,2-a]pyrazin-8-yl]amino]benzoate 2,2,2-trifluoroacetate

A mixture of methyl 4-amino-2-chlorobenzoate (1883 mg, 10.15 mmol, 1 eq), 8-chloro-3-(2,3-difluoro-4-methoxy-phenyl)imidazo[1,2-a]pyrazine (Intermediate 18, 3000 mg, 10.15 mmol, 1 eq) in toluene (10 mL) and trifluoroacetic acid (2.35 mL, 30.44 mmol, 3 eq) was heated to 100 °C for 16 h. The solvent was removed under reduced pressure and the solid was triturated with MeCN (30 mL) and filtered off to afford the title compound (3.5 g, 7.87 mmol, 48.68% yield) as an off-white solid. MS (ESI, m/z): 445.0, 447.0 [M+H]⁺.

### Step 2)

### 2-Chloro-4-[[3-(2,3-difluoro-4-methoxy-phenyl)imidazo[1,2-a]pyrazin-8-yl]amino]benzoic acid

To a solution of methyl 2-chloro-4-[[3-(2,3-difluoro-4-methoxy-phenyl)imidazo[1,2-a]pyrazin-8-yl]amino]benzoate (3.5 g, 7.87 mmol, 1 eq) in methanol (80 mL) was added sodium hydroxide (20 mL, 80 mmol, 10.17 eq). The mixture was stirred at 80°C for 12 h. The reaction mixture was concentrated in vacuo, diluted with water and acidified with 2N HCl. The solid was collected and thoroughly dried to afford the title compound (3.15 g, 7.31 mmol, 92.93% yield) as an off-white solid. MS (ESI, m/z): 431.0, 433.0 [M+H]⁺.

### Step 3)

### (2,5-Dioxopyrrolidin-1-yl) 2-chloro-4-[[3-(2,3-difluoro-4-methoxy-phenyl)imidazo[1,2-a]pyrazin-8-yl]amino]benzoate

To a solution of 2-chloro-4-[[3-(2,3-difluoro-4-methoxy-phenyl)imidazo[1,2-a]pyrazin-8-yl]amino]benzoic acid (500 mg, 1.16 mmol, 1 eq) in THF (6 mL)/DMF (2 mL) was added N-hydroxysuccinimide (173.65 mg, 1.51 mmol, 1.3 eq) and 1-ethyl-(3-(3-dimethylamino)propyl)-carbodiimide hydrochloride (244.75 mg, 1.28 mmol, 1.1 eq). The mixture was stirred at 25°C for 14 h. The precipitate was filtered and dried in vacuo to afford the title compound (530 mg, 1 mmol, 86.51% yield) as a white solid. MS (ESI, m/z): 528.0, 530.0 [M+H]⁺.

### Step 4) Intermediate 19

### 3-[[2-Chloro-4-[[3-(2,3-difluoro-4-methoxy-phenyl)imidazo[1,2-a]pyrazin-8-yl]amino]benzoyl]amino]propyl-trimethyl-ammonium formate

To a solution of (2,5-dioxopyrrolidin-1-yl) 2-chloro-4-[[3-(2,3-difluoro-4-methoxyphenyl)imidazo[1,2-a]pyrazin-8-yl]amino]benzoate (50 mg, 0.090 mmol, 1 eq) in THF (1 mL) was added trimethylamine (5.6 mg, 0.090 mmol, 1 eq) and 3-bromopropan-1-amine (19.61 mg, 0.140 mmol, 1.5 eq). The mixture was stirred at 25°C for 2 h then warmed to 60°C and stirred for 12 h. The mixture was filtered and the filtrate was concentrated. The residue was purified by prep-HPLC (FA) to afford the title compound (23 mg, 0.040 mmol, 45.82% yield) as a white solid. MS (ESI, m/z): 529.0, 531.0 [M]⁺.

### Example 48

### 3-[[2-Chloro-4-[[3-(2,3-difluoro-4-methoxy-phenyl)imidazo[1,2-a]pyrazin-8-yl]amino]benzoyl]amino]propyl-triethyl-ammonium formate

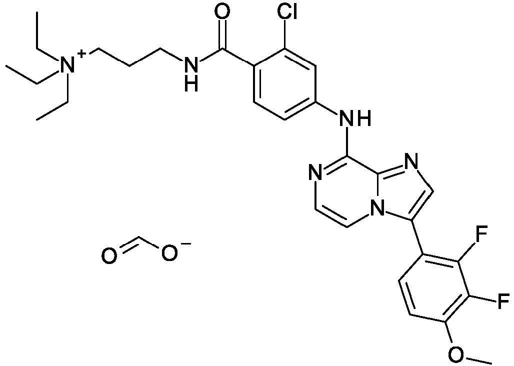

To a solution of (2,5-dioxopyrrolidin-1-yl) 2-chloro-4-[[3-(2,3-difluoro-4-methoxyphenyl)imidazo[1,2-a]pyrazin-8-yl]amino]benzoate (Intermediate 19, 50 mg, 0.090 mmol, 1 eq) in DMF (2 mL) was added 3-bromopropan-1-amine (19.61 mg, 0.140 mmol, 1.5 eq) and triethylamine (0.04 mL, 0.280 mmol, 3 eq). The mixture was stirred at 25°C for 16 h. The mixture was filtered and the filtrate was concentrated. The residue was purified by prep-HPLC (FA) to afford the title compound (20 mg, 0.030 mmol, 35.03% yield) as a white solid. MS (ESI, m/z): 571.0, 573.0 [M]⁺.

### Example 49

### Tributyl-[3-[[2-chloro-4-[[3-(2,3-difluoro-4-methoxy-phenyl)imidazo[1,2-a]pyrazin-8-yl]amino]benzoyl]amino]propyl]ammonium formate

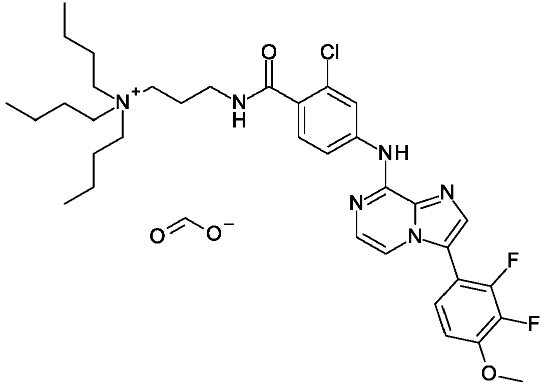

### Step 1)

### 3-(Benzyloxycarbonylamino)propyl-tributyl-ammonium formate

To a solution of benzyl N-(3-bromopropyl)carbamate (300.0 mg, 1.1 mmol, 1 eq) in o-xylene (1 mL) was added tributylamine (0.56 mL, 2.2 mmol, 2 eq). The mixture was stirred at 120 °C for 4 h. To the mixture was added water. The aqueous solution was lyophilized and the residue purified by prep-HPLC (FA) to give the title compound (150 mg, 0.400 mmol, 36.04% yield) as colorless oil which was used without further purification. MS (ESI, m/z): 377 [M]⁺.

### Step 2)

### 3-aminopropyl(tributyl)ammonium formate

To a solution of 3-(benzyloxycarbonylamino)propyl-tributyl-ammonium formate (150.0 mg, 0.400 mmol, 1 eq) in methanol (4 mL) was added Pd/C (20.0 mg, 0.400 mmol, 1 eq). The mixture was stirred at 25 °C for 14 h under H2 balloon. The mixture was filtered and the organic layer was concentrated to give 3-aminopropyl(tributyl)ammonium formate (50 mg, 0.210 mmol, 51.7% yield) as light yellow oil and was used in the next step without further purification.

### Step 3)

### Tributyl-[3-[[2-chloro-4-[[3-(2,3-difluoro-4-methoxy-phenyl)imidazo[1,2-a]pyrazin-8-yl]amino]benzoyl]amino]propyl]ammonium formate

To a solution of (2,5-dioxopyrrolidin-1-yl) 2-chloro-4-[[3-(2,3-difluoro-4-methoxyphenyl)imidazo[1,2-a]pyrazin-8-yl]amino]benzoate (Intermediate 19, 65.05 mg, 0.120 mmol, 0.6 eq) in THF (3 mL) was added 3-aminopropyl(tributyl)ammonium formate(50 mg, 0.210 mmol, 1 eq) and N,N-diisopropylethylamine (0.04 mL, 0.210 mmol, 1 eq). The mixture was stirred at 60°C for 2 h. The mixture was filtered and the filtrate was concentrated. The residue was purified by prep-HPLC (FA) to afford the title compound (11 mg, 0.020 mmol, 7.62% yield) as a white solid. MS (ESI, m/z): 655.0 [M]⁺.

### Example 50

### Carboxymethyl-[2-[2-[[4-[[3-(2,3-difluoro-4-methoxy-phenyl)imidazo[1,2-a]pyrazin-8-yl]amino]-2-ethyl-benzoyl]amino]ethoxy]ethyl]-dimethyl-ammonium formate

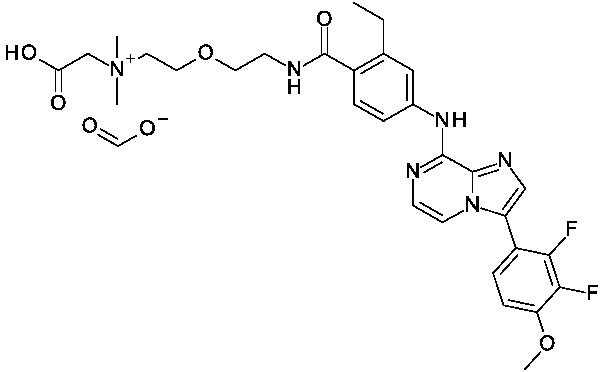

### Step 1)

### (2,5-Dioxopyrrolidin-1-yl) 4-[[3-(2,3-difluoro-4-methoxy-phenyl)imidazo[1,2-a]pyrazin-8-yl]amino]-2-ethyl-benzoate

The title compound (1.8 g, 3.45 mmol, 73.25% yield) was obtained as a light yellow solid in analogy to Example **47,** Step 3 using Intermediate 8. MS (ESI, m/z): 522.0 [M+H]⁺.

### Step 2) Intermediate 20

### N-[2-(2-aminoethoxy)ethyl]-4-[[3-(2,3-difluoro-4-methoxy-phenyl)imidazo[1,2-a]pyrazin-8-yl]amino]-2-ethyl-benzamide

To a solution of (2,5-dioxopyrrolidin-1-yl) 4-[[3-(2,3-difluoro-4-methoxy-phenyl)imidazo[1,2-a]pyrazin-8-yl]amino]-2-ethyl-benzoate (1100 mg, 2.11 mmol, 1 eq) in THF (20 mL) was added triethylamine (0.44 mL, 3.16 mmol, 1.5 eq) and 1,5-diamino-3-oxapentane (329 mg, 3.16 mmol, 1.5 eq). The mixture was stirred at 25°C for 3 h. The solvent was removed. The solid was triturated with water and filtered to afford the title compound (912 mg, 1.79 mmol, 84.68% yield) as an off-white solid. MS (ESI, m/z): 511.0 [M+H]⁺.

### Step 3)

### 2-[2-[2-[[4-[[3-(2,3-difluoro-4-methoxy-phenyl)imidazo[1,2-a]pyrazin-8-yl]amino]-2-ethyl-benzoyl]amino]ethoxy]ethylamino]acetate

To a solution of N-[2-(2-aminoethoxy)ethyl]-4-[[3-(2,3-difluoro-4-methoxy-phenyl)imidazo[1,2-a]pyrazin-8-yl]amino]-2-ethyl-benzamide (500 mg, 0.980 mmol, 1 eq) in DMF (5 mL) was added triethylamine (0.2 mL, 1.47 mmol, 1.5 eq) and methyl chloroacetate (138 mg, 1.27 mmol, 1.3 eq). The mixture was stirred at 25°C for 4 h. To the mixture was added water and the pH of mixture was adjusted to around 4 by 1M HCl. The mixture was extracted by EtOAc (5 mLx 3). The pH of the aqueous solution was adjusted to around 8. The resulting suspension was filtered and the solid was dried to afford the title compound (370 mg, 0.640 mmol, 65% yield) as a yellow solid. MS (ESI, m/z): 583.0 [M+H]⁺.

### Step 4) Intermediate 21

### 2-[2-[[4-[[3-(2,3-Difluoro-4-methoxy-phenyl)imidazo[1,2-a]pyrazin-8-yl]amino]-2-ethyl-benzoyl]amino]ethoxy]ethyl-(2-methoxy-2-oxo-ethyl)-dimethyl-ammonium

To a solution of methyl 2-[2-[2-[[4-[[3-(2,3-difluoro-4-methoxy-phenyl)imidazo[1,2-a]pyrazin-8-yl]amino]-2-ethyl-benzoyl]amino]ethoxy]ethylamino]acetate (100 mg, 0.170 mmol, 1 eq) in DMF (2 mL) was added iodomethane (146.18 mg, 1.03 mmol, 6 eq). The mixture was stirred at 25°C for 26 h. In order to improve purification in the next step, Boc₂O was added and stirred for another 1 h. The solvent was removed to afford the title compound (120 mg, 0.200 mmol, quantitative yield) as a yellow gum and was used without further purification. MS (ESI, m/z): 611.0 [M+H]⁺.

### Step 5)

### Carboxymethyl-[2-[2-[[4-[[3-(2,3-difluoro-4-methoxy-phenyl)imidazo[1,2-a]pyrazin-8-yl]amino]-2-ethyl-benzoyl]amino]ethoxy]ethyl]-dimethyl-ammonium formate

To a solution of 2-[2-[[4-[[3-(2,3-difluoro-4-methoxy-phenyl)imidazo[1,2-a]pyrazin-8-yl]amino]-2-ethyl-benzoyl]amino]ethoxy]ethyl-(2-methoxy-2-oxo-ethyl)-dimethyl-ammonium (110 mg, 0.180 mmol, 1 eq) in methanol (1 mL) was added sodium hydroxide (1 mL, 4 mmol, 22.24 eq). The mixture was stirred at 25°C for 2 h. The pH was adjusted to around 6 by 1M HCl. The solvent was removed and the residue was purified by prep-HPLC (FA) to afford the title compound (20 mg, 0.030 mmol, 17.13% yield) as a white solid. MS (ESI, m/z): 597.0 [M]⁺.

### Example 51

### 2-[2-[[4-[[3-(2,3-Difluoro-4-methoxy-phenyl)imidazo[1,2-a]pyrazin-8-yl]amino]-2-ethyl-benzoyl]amino]ethoxy]ethyl-(2-hydroxyethyl)-dimethyl-ammonium formate

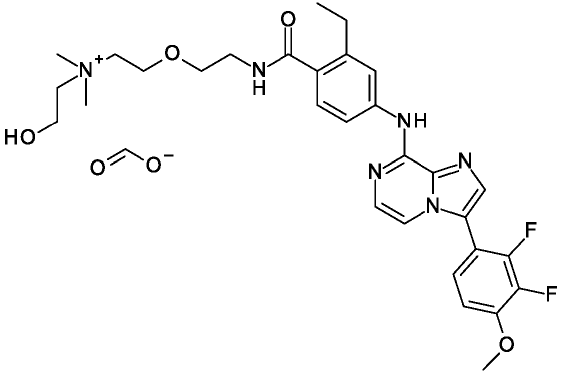

To a solution of lithium chloride (19 mg, 0.460 mmol, 4 eq) in THF/EtOH (1:1) (2 mL) was added sodium borohydride (17 mg, 0.460 mmol, 4 eq). The mixture was stirred for 10 minutes. Then 2-[2-[[4-[[3-(2,3-difluoro-4-methoxy-phenyl)imidazo[1,2-a]pyrazin-8-yl]amino]-2-ethyl-benzoyl]amino]ethoxy]ethyl-(2-methoxy-2-oxo-ethyl)-dimethyl-ammonium (Intermediate 21, 70 mg, 0.110 mmol, 1 eq) in THF (0.5 mL) was slowly added. The mixture was stirred for 2 h. The mixture was quenched by 1N HCl. The mixture was purified by prep-HPLC (FA) to afford the title compound (18 mg, 0.030 mmol, 25.02% yield) as a white solid. MS (ESI, m/z): 583.0 [M+H]⁺.

### Example 52

### (2-Amino-2-oxo-ethyl)-[2-[2-[[4-[[3-(2,3-difluoro-4-methoxy-phenyl)imidazo[1,2-a]pyrazin-8-yl]amino]-2-ethyl-benzoyl]amino]ethoxy]ethyl]-dimethyl-ammonium formate

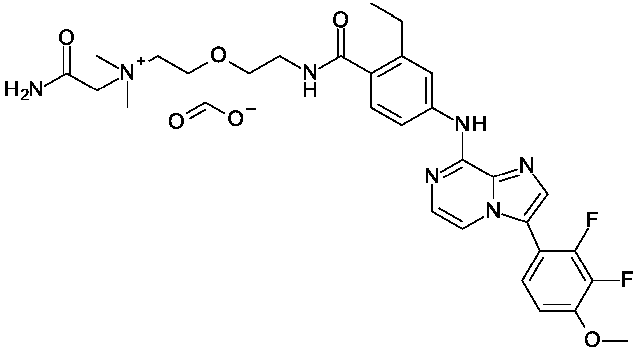

### Step 1)

### 4-[[3-(2,3-Difluoro-4-methoxy-phenyl)imidazo[1,2-a]pyrazin-8-yl]amino]-N-[2-[2-(dimethylamino)ethoxy]ethyl]-2-ethyl-benzamide

To solution of N-[2-(2-aminoethoxy)ethyl]-4-[[3-(2,3-difluoro-4-methoxy-phenyl)imidazo[1,2-a]pyrazin-8-yl]amino]-2-ethyl-benzamide (Intermediate 20, 100 mg, 0.200 mmol, 1 eq) in methanol (5 mL) was added aqueous formaldehyde (5 mL, 0.200 mmol, 1 eq) and sodium cyanoborohydride (25 mg, 0.390 mmol, 2 eq). The resulting mixture was stirred at 15 °C for 15 h. The mixture was concentrated and the residue was purified by flash column to afford the title compound (80 mg, 0.150 mmol, 75.83% yield) as a white solid. MS (ESI, m/z): 539.4 [M+H]⁺.

### Step 2)

### (2-Amino-2-oxo-ethyl)-[2-[2-[[4-[[3-(2,3-difluoro-4-methoxy-phenyl)imidazo[1,2-a]pyrazin-8-yl]amino]-2-ethyl-phenyl]methylamino]ethoxy]ethyl]-dimethyl-ammonium formate

To a solution of 4-[[3-(2,3-difluoro-4-methoxy-phenyl)imidazo[1,2-a]pyrazin-8-yl]amino]-N-[2-[2-(dimethylamino)ethoxy]ethyl]-2-ethyl-benzamide (20 mg, 0.040 mmol, 1 eq) in DMF (2 mL) was added 2-chloroacetamide (35 mg, 0.370 mmol, 10 eq). The resulting mixture was stirred at 20 °C for 15 h. The mixture was concentrated and the residue was purified by prep-HPLC (FA) to afford the title compound (9.9 mg, 0.020 mmol, 41.07% yield) as a white solid. MS (ESI, m/z): 596.4 [M+H]⁺.

### Example 53

### 3-Carboxypropyl-[2-[2-[[4-[[3-(2,3-difluoro-4-methoxy-phenyl)imidazo[1,2-a]pyrazin-8-yl]amino]-2-ethyl-benzoyl]amino]ethoxy]ethyl]-dimethyl-ammonium formate

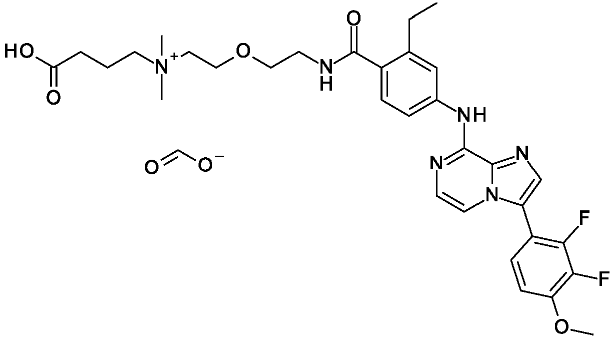

### Step 1)

### 2-[2-[[4-[[3-(2,3-Difluoro-4-methoxy-phenyl)imidazo[1,2-a]pyrazin-8-yl]amino]-2-ethylphenyl]methylamino]ethoxy]ethyl-(4-methoxy-4-oxo-butyl)-dimethyl-ammonium bromide

To a solution of 4-[[3-(2,3-difluoro-4-methoxy-phenyl)imidazo[1,2-a]pyrazin-8-yl]amino]-N-[2-[2-(dimethylamino)ethoxy]ethyl]-2-ethyl-benzamide (Intermediate 23, 60 mg, 0.110 mmol, 1 eq) in DMF (2 mL) was added methyl 4-bromobutyrate (101 mg, 0.560 mmol, 5 eq). The resulting mixture was stirred at 10 °C for 15 h. The mixture was concentrated and the residue was purified by prep-HPLC (FA) to afford the title compound (30 mg, 0.040 mmol, 38.16% yield) as a white solid. MS (ESI, m/z): 639.4 [M+H]⁺.

### Step 2)

### 3-Carboxypropyl-[2-[2-[[4-[[3-(2,3-difluoro-4-methoxy-phenyl)imidazo[1,2-a]pyrazin-8-yl]amino]-2-ethyl-benzoyl]amino]ethoxy]ethyl]-dimethyl-ammonium formate

To a solution of 2-[2-[[4-[[3-(2,3-difluoro-4-methoxy-phenyl)imidazo[1,2-a]pyrazin-8-yl]amino]-2-ethyl-benzoyl]amino]ethoxy]ethyl-(4-methoxy-4-oxo-butyl)-dimethyl-ammonium bromide (30 mg, 0.050 mmol, 1 eq) in methanol (2 mL) and water (1 mL) was added lithium hydroxide monohydrate (9.85 mg, 0.230 mmol, 5 eq). The resulting mixture was stirred at 10 °C for 15 h. The mixture was acidified with aqueous 1 N HCl to pH=3 and then purified by prep-HPLC (FA) to afford the title compound (10.3 mg, 0.020 mmol, 31.83% yield) as a white solid. MS (ESI, m/z): 625.4 [M+H]⁺.

### Example 54

### 2-(4-(2-Chloro-4-((3-(4-(cyanomethoxy)-2,3-difluorophenyl)imidazo[1,2-a]pyrazin-8-yl)amino)benzoyl)piperazin-1-yl)-N,N,N-trimethyl-2-oxoethan-1-aminiumformate

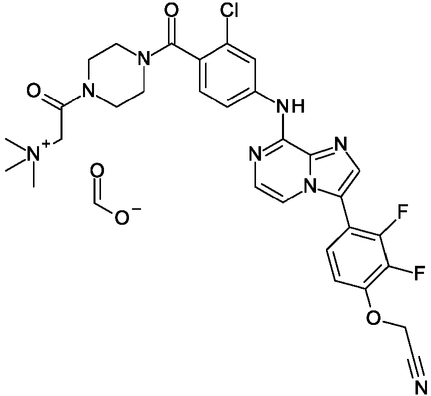

### Step 1)

### 2-(4-(8-((3-Chloro-4-(4-(dimethylglycyl)piperazine-1-carbonyl)phenyl)amino)imidazo[1,2-a]pyrazin-3-yl)-2,3-difluorophenoxy)acetonitrile

2-chloro-4-((3-(4-(cyanomethoxy)-2,3-difluorophenyl)imidazo[1,2-a]pyrazin-8-yl)amino)benzoic acid hydrochloride (Intermediate 10, 75 mg, 152 µmol, Eq: 1) were combined with DMF (1.3 ml). HATU (116 mg, 305 µmol, Eq: 2) and DIPEA (98.5 mg, 133 µl, 762 µmol, Eq: 5.00) were added and after stirring for 15 min, 2-(dimethylamino)-1-(piperazin-1-yl)ethan-1-one (39.1 mg, 229 µmol, Eq: 1.5) was added and the reaction mixture was stirred at RT for 1 h. The crude material was purified by flash chromatography (reverse phase, 12 g, 0% to 100% water: ACN) to afford the title compound (43.3 mg, 71 µmol, 46.7% yield) as a light brown solid. MS (ESI, m/z): 610.0 [M+H]⁺.

### Step 2)

### 2-(4-(2-Chloro-4-((3-(4-(cyanomethoxy)-2,3-difluorophenyl)imidazo[1,2-a]pyrazin-8-yl)amino)benzoyl)piperazin-1-yl)-N,N,N-trimethyl-2-oxoethan-1-aminium formate

2-(4-(8-((3-chloro-4-(4-(dimethylglycyl)piperazine-1-carbonyl)phenyl)amino)imidazo[1,2-a]pyrazin-3-yl)-2,3-difluorophenoxy)acetonitrile (43.3 mg, 71.1 µmol, Eq: 1) was dissolved in 1,4-dioxane (250 µl) and DIPEA (46.2 mg, 62.5 µl, 358 µmol, Eq: 5.03) was added. The mixture was stirred at room temperature for 15 min and iodomethane (50.5 mg, 22.1 µl, 355 µmol, Eq: 5) was added. The reaction mixture was stirred at room temperature overnight. The crude material was purified by preparative HPLC, then lyophilized to afford the title compound (23.5 mg, 35 µmol, 52.3% yield) as a white lyoph solid. MS (ESI, m/z): 624.5 [M+H]⁺.

### Example 55

### (2S,4R)-2-(4-(4-((3-(2,3-Difluoro-4-methoxyphenyl)imidazo[1,2-a]pyrazin-8-yl)amino)-2-methylbenzoyl)piperazine-1-carbonyl)-4-hydroxy-1,1-dimethylpyrrolidin-1-ium formate Chiral

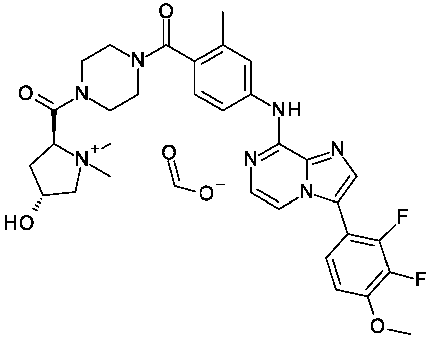

### Step 1) Intermediate 25

### Methyl 4-((3-iodoimidazo[1,2-a]pyrazin-8-yl)amino)-2-methylbenzoate hydrochloride

8-Chloro-3-iodoimidazo[1,2-a]pyrazine (Intermediate 1, 18.18 g, 61.8 mmol, Eq: 1) was suspended in acetonitrile (90 ml) and acetic acid (9 ml) was added. While stirring, methyl 4-amino-2-methylbenzoate (12.3 g, 74.2 mmol, Eq: 1.2) was added carefully and the reaction mixture was heated to 90 °C and stirred overnight. The reaction mixture was cooled to RT using an ice bath. The pale brown suspension was filtered and washed with 75 mL of cold ACN/MeOH (1: 1) and dried under HV overnight to afford the title compound (26.23 g, 59 mmol, 95.5% yield) as a white solid. MS (ESI, m/z): 409.1 [M+H]⁺.

### Step 2)

### Methyl 4-((3-(2,3-difluoro-4-methoxyphenyl)imidazo[1,2-a]pyrazin-8-yl)amino)-2-methylbenzoate

Methyl 4-((3-iodoimidazo[1,2-a]pyrazin-8-yl)amino)-2-methylbenzoate hydrochloride (5 g, 11.2 mmol, Eq: 1) and (2,3-difluoro-4-methoxyphenyl)boronic acid (2.22 g, 11.8 mmol, Eq: 1.05) were combined with dioxane (75 ml). Na₂CO₃ (2.62 g, 24.7 mmol, Eq: 2.2) was dissolved in water (7.5 ml) and added to the reaction mixture while stirring. Argon was sparged in the reaction and [1,1'-bis(diphenylphosphino)ferrocene]dichloropalladium(II), complex with dichloromethane (138 mg, 169 µmol, Eq: 0.015) was added. The reaction mixture was heated to 110 °C and stirred overnight. The suspension was filtered and the filter cake was washed with EtOAc and the solvent of the obtained solution was evaporated. The crude material was purified by flash chromatography (silica gel, 330 g, 0% to 50% DCM/MeOH/NH₃ (95:5:1)) to afford the title compound (4.39 g, 9.72 mmol, 86.5% yield) as an off-white solid. MS (ESI, m/z): 425.2 [M+H]⁺.

### Step 3) Intermediate 6

### 4-((3-(2,3-Difluoro-4-methoxyphenyl)imidazo[1,2-a]pyrazin-8-yl)amino)-2-methylbenzoic acid

Methyl 4-((3-(2,3-difluoro-4-methoxyphenyl)imidazo[1,2-a]pyrazin-8-yl)amino)-2-methylbenzoate (4.39 g, 9.72 mmol, Eq: 1) was combined with EtOH (20 mL) and water (10 mL). LiOH solution (1M in water) (19.4 ml, 19.4 mmol, Eq: 2) was added at RT. The reaction mixture was heated to 90 °C and stirred for 24 h. The organic solvents were evaporated and aqueous HCl (25%) was added until pH =0: a precipitate appeared so it was filtered off. The filter cake was washed with EtOAc and dried thoroughly in HV to afford the title compound (4.12 g, 10 mmol, quantitative yield) as a yellow powder. MS (ESI, m/z): 411.1 [M+H]⁺.

### Step 4)

### tert-Butyl 4-(4-((3-(2,3-difluoro-4-methoxyphenyl)imidazo[1,2-a]pyrazin-8-yl)amino)-2-methylbenzoyl)piperazine-1-carboxylate

4-((3-(2,3-Difluoro-4-methoxyphenyl)imidazo[1,2-a]pyrazin-8-yl)amino)-2-methylbenzoic acid (4.12 g, 10 mmol, Eq: 1) was combined with DMF (40 ml). 1-Boc-Piperazine (2.8 g, 15.1 mmol, Eq: 1.5) and HATU (7.63 g, 20.1 mmol, Eq: 2.0) were added. The reaction mixture was stirred at RT followed by the addition of DIPEA (6.49 g, 8.77 ml, 50.2 mmol, Eq: 5). The stirring was continued at RT overnight. The reaction mixture was poured into 100 mL of water (basified with 1M NaOH) and it was extracted with 150 mL of EtOAc. The organic layer was concentrated in vacuo. The crude material was purified by flash chromatography (silica gel, 120 g, 40% Heptane/EtOAc (isocratic) followed by 0% to 50% DCM/MeOH/NH₃ (95:5:1)) to afford the title compound (6.066 g, 10.2 mmol, 101% yield) as a light brown solid. MS (ESI, m/z): 579.3 [M+H]⁺.

### Step 5)

### (4-((3-(2,3-Difluoro-4-methoxyphenyl)imidazo[1,2-a]pyrazin-8-yl)amino)-2-methylphenyl)(piperazin-1-yl)methanone hydrochloride

Tert-butyl 4-(4-((3-(2,3-difluoro-4-methoxyphenyl)imidazo[1,2-a]pyrazin-8-yl)amino)-2-methylbenzoyl)piperazine-1-carboxylate (6.066 g, 10.2 mmol, Eq: 1) was combined with dioxane (80 ml) and MeOH (15 ml). Hydrogen chloride solution (4M in Dioxane) (12.7 ml, 50.8 mmol, Eq: 5) was added slowly. The stirring at RT was continued overnight. Addition of diethyl ether (60 mL) followed by sonication gave a precipitate which was filtered. The filter cake was washed with diethyl ether and dried in vacuum and high vacuum to afford the title compound (4.978 g, 9.67 mmol, 95.1 % yield) as a white solid. MS (ESI, m/z): 479.2 [M+H]⁺.

### Step 6)

### tert-Butyl (2S,4R)-2-(4-(4-((3-(2,3-difluoro-4-methoxyphenyl)imidazo[1,2-a]pyrazin-8-yl)amino)-2-methylbenzoyl)piperazine-1-carbonyl)-4-hydroxypyrrolidine-1-carboxylate

(4-((3-(2,3-Difluoro-4-methoxyphenyl)imidazo[1,2-a]pyrazin-8-yl)amino)-2-methylphenyl)(piperazin-1-yl)methanone hydrochloride (2.135 g, 4.02 mmol, Eq: 1) was combined with DMF (30 ml). (2S,4R)-1-(tert-butoxycarbonyl)-4-hydroxypyrrolidine-2-carboxylic acid (1.39 g, 6.03 mmol, Eq: 1.5) and HATU (3.06 g, 8.04 mmol, Eq: 2.0) were added. DIPEA (1.61 g, 2.17 ml, 12.1 mmol, Eq: 3.0) was added and the reaction mixture was stirred at RT for 3 h. The reaction was quenched with 10 mL of EtOAc, 10 mL of water and 10 mL of NaOH (1M). The solution was poured into 250 mL of water and 250 mL of EtOAc and was extracted. The organic layer was dried with MgSO₄, filtered and evaporated. The crude material was purified by flash chromatography (silica gel, 120 g, 0% to 100% DCM:(DCM/MeOH/NH₃ (95:5:1))) to afford the title compound (1.68 g, 2.43 mmol, 60.4% yield) as an off-white foam. MS (ESI, m/z): 692.43 [M+H]⁺.

### Step 7)

### (4-(4-((3-(2,3-difluoro-4-methoxyphenyl)imidazo[1,2-a]pyrazin-8-yl)amino)-2-methylbenzoyl)piperazin-1-yl)((2S,4R)-4-hydroxypyrrolidin-2-yl)methanone hydrochloride

Tert-butyl (2S,4R)-2-(4-(4-((3-(2,3-difluoro-4-methoxyphenyl)imidazo[1,2-a]pyrazin-8-yl)amino)-2-methylbenzoyl)piperazine-1-carbonyl)-4-hydroxypyrrolidine-1-carboxylate (1.68 g, 2.43 mmol, Eq: 1) was combined with dioxane. The mixture was stirred at RT and hydrogen chloride solution (4M in dioxane) (3.04 ml, 12.1 mmol, Eq: 5) was added very slowly. The reaction mixture was stirred at RT overnight. Addition of ether and sonication gave a suspension that was filtered. The filter cake obtained was dried in high vacuum at 50°C to afford the title compound (1.606 g, 2.56 mmol, 105% yield) as a light yellow/off-white solid. MS (ESI, m/z): 590.5 [M-H]⁻.

### Step 8)

### (2S,4R)-2-(4-(4-((3-(2,3-Difluoro-4-methoxyphenyl)imidazo[1,2-a]pyrazin-8-yl)amino)-2-methylbenzoyl)piperazine-1-carbonyl)-4-hydroxy-1,1-dimethylpyrrolidin-1-ium formate

To a white suspension of (4-(4-((3-(2,3-difluoro-4-methoxyphenyl)imidazo[1,2-a]pyrazin-8-yl)amino)-2-methylbenzoyl)piperazin-1-yl)((2S,4R)-4-hydroxypyrrolidin-2-yl)methanone hydrochloride (40 mg, 63.7 µmol, Eq: 1) in DCM (637 µl) were added iodomethane (22.6 mg, 9.96 µl, 159 µmol, Eq: 2.5) and Hunig's base (8.23 mg, 11.1 µl, 63.7 µmol, Eq: 1). The reaction mixture was stirred at room temperature overnight and then purified by prep HPLC to afford the title compound (30 mg, 45 µmol, 67.2% yield) as an amorphous white solid. MS (ESI, m/z): 620.3 [M+H]⁺.

### Example 56

### [2-[4-[4-[[3-[4-(Cyanomethoxy)-2,3-difluorophenyl]imidazo[1,2-a]pyrazin-8-yl]amino]-2-methylbenzoyl]piperazin-1-yl]-2-oxoethyl]-trimethylazanium iodide

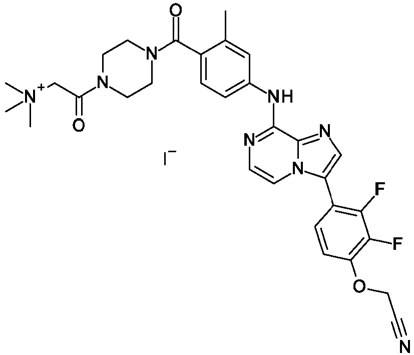

### Step 1)

### 4-((3-(4-(Cyanomethoxy)-2,3-difluorophenyl)imidazo[1,2-a]pyrazin-8-yl)amino)-2-methylbenzoic acid

2-(4-(8-chloroimidazo[1,2-a]pyrazin-3-yl)-2,3-difluorophenoxy)acetonitrile (Intermediate 11, 750 mg, 2.34 mmol, Eq: 1) was combined with acetonitrile (7.5 ml) and AcOH (750 µl). 4-Amino-2-methylbenzoic acid (371 mg, 2.46 mmol, Eq: 1.05) was added and the reaction mixture was stirred at 60°C over the weekend and then at 80°C for 7 h. After cooling back to RT, acetonitrile and a little amount of MeOH were added to the suspension and the solid was filtered and dried under HV to afford the title compound (896.6 mg, 2 mmol, 63.4% yield) as a light brown solid. MS (ESI, m/z): 436.1 [M+H]⁺.

### Step 2)

### Tert-butyl (2-(4-(4-((3-(4-(cyanomethoxy)-2,3-difluorophenyl)imidazo[1,2-a]pyrazin-8-yl)amino)-2-methylbenzoyl)piperazin-1-yl)-2-oxoethyl)(methyl)carbamate

4-((3-(4-(cyanomethoxy)-2,3-difluorophenyl)imidazo[1,2-a]pyrazin-8-yl)amino)-2-methylbenzoic acid (50 mg, 115 µmol, Eq: 1) was combined with DMF (1.3 ml). HATU (87.3 mg, 230 µmol, Eq: 2) and DIPEA (44.5 mg, 60.2 µl, 345 µmol, Eq: 3.00) were added, and after stirring for 15 min, tert-butyl methyl(2-oxo-2-(piperazin-1-yl)ethyl)carbamate (Intermediate 24, 44.3 mg, 172 µmol, Eq: 1.50) was added and the reaction mixture was stirred at RT overnight. The crude material was purified by prep HPLC to afford the title compound (40.2 mg, 59.5 µmol, 51.9% yield) as a white solid. MS (ESI, m/z): 675.3 [M+H]⁺.

### Step 3)

### 2-(2,3-Difluoro-4-(8-((3-methyl-4-(4-(methylglycyl)piperazine-1-carbonyl)phenyl)amino)imidazo[1,2-a]pyrazin-3-yl)phenoxy)acetonitrile

Tert-butyl (2-(4-(4-((3-(4-(cyanomethoxy)-2,3-difluorophenyl)imidazo[1,2-a]pyrazin-8-yl)amino)-2-methylbenzoyl)piperazin-1-yl)-2-oxoethyl)(methyl)carbamate (40.2 mg, 59.6 µmol, Eq: 1) was combined with THF (315 µl). 3.4M HCl (87.6 µl, 298 µmol, Eq: 5) was added and the reaction mixture was stirred at RT for 1 h. More 3.4M HCl (87.6 µl, 298 µmol, Eq: 5) was added and the reaction mixture was stirred at RT overnight. The resultant mixture was poured into water and then adjusted to pH=10-11 with ammonia solution. The aqueous solution was extracted with DCM and isopropanol (6:1) and after evaporation of the volatiles, the crude material was purified by preparative HPLC to afford the title compound (24.6 mg, 42.8 µmol, 71.9% yield) as an off-white solid. MS (ESI, m/z): 575.3 [M+H]⁺.

### Step 4)

### [2-[4-[4-[[3-[4-(Cyanomethoxy)-2,3-difluorophenyl]imidazo[1,2-a]pyrazin-8-yl]amino]-2-methylbenzoyl]piperazin-1-yl]-2-oxoethyl]-trimethylazanium iodide

2-(2,3-difluoro-4-(8-((3-methyl-4-(4-(methylglycyl)piperazine-1-carbonyl)phenyl)amino)imidazo[1,2-a]pyrazin-3-yl)phenoxy)acetonitrile (20 mg, 34.8 µmol, Eq: 1) was dissolved in 1,4-dioxane (250 µl) and DIPEA (22.6 mg, 30.6 µl, 175 µmol, Eq: 5.03) was added. The mixture was stirred at room temperature for 15 min and iodomethane (24.7 mg, 10.8 µl, 174 µmol, Eq: 5) was added. The reaction mixture was stirred at room temperature overnight. The crude material was purified by preparative HPLC and the product was lyophilized to afford the title compound (11.7 mg, 18 µmol, 51.8% yield) as a white solid. MS (ESI, m/z): 604.2 [M+H]⁺.

### Example 57

### 4-[4-[2-[[4-[[3-[4-(Difluoromethoxy)phenyl]imidazo[1,2-a]pyrazin-8-yl]amino]-2-methylbenzoyl]-methylamino]ethyl]-1-methylpiperidin-1-ium-1-yl]butanoic acid hydrochloride

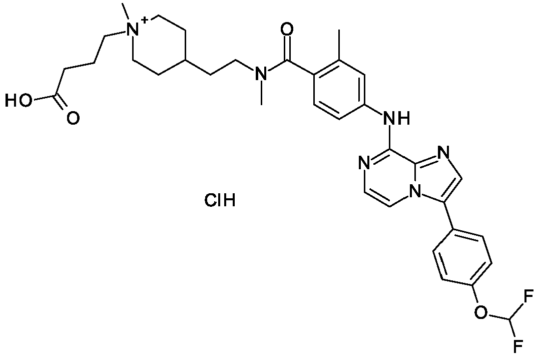

### Step 1)

### Tert-butyl 4-(4-(2-(4-((3-(4-(difluoromethoxy)phenyl)imidazo[1,2-a]pyrazin-8-yl)amino)-N,2-dimethylbenzamido)ethyl)piperidin-1-yl)butanoate

4-((3-(4-(Difluoromethoxy)phenyl)imidazo[1,2-a]pyrazin-8-yl)amino)-N,2-dimethyl-N-(2-(piperidin-4-yl)ethyl)benzamide hydrochloride (Intermediate 3, 113 mg, 198 µmol, Eq: 1) was combined with n-BuOH (3 ml). Tert-butyl 4-bromobutanoate (66.2 mg, 297 µmol, Eq: 1.5), Na₂CO₃ (83.9 mg, 791 µmol, Eq: 4) and KI (3.28 mg, 19.8 µmol, Eq: 0.1) were added and the reaction mixture was stirred at 105°C overnight. The reaction mixture was poured into 5 mL of H₂O and was extracted with EtOAc (2 x 5 mL). The organic layers were concentrated in vacuo. The crude material was purified by preparative HPLC to afford the title compound (55.9 mg, 82.5 µmol, 41.7% yield) as a light yellow solid. MS (ESI, m/z): 677.6 [M+H]⁺.

### Step 2)

### 1-(4-(tert-Butoxy)-4-oxobutyl)-4-(2-(4-((3-(4-(difluoromethoxy)phenyl)imidazo[1,2-a]pyrazin-8-yl)amino)-N,2-dimethylbenzamido)ethyl)-1-methylpiperidin-1-ium formate

Tert-butyl 4-(4-(2-(4-((3-(4-(difluoromethoxy)phenyl)imidazo[1,2-a]pyrazin-8-yl)amino)-N,2-dimethylbenzamido)ethyl)piperidin-1-yl)butanoate (55.9 mg, 82.6 µmol, Eq: 1) was dissolved in 1,4-dioxane (250 µl) and DIPEA (53.7 mg, 72.6 µl, 415 µmol, Eq: 5.03) was added. The mixture was stirred at room temperature for 15 min and iodomethane (58.6 mg, 25.7 µl, 413 µmol, Eq: 5) was added. The reaction mixture was stirred at room temperature overnight. The crude material was purified by preparative HPLC to afford the title compound (30.4 mg, 41.2 µmol, 50% yield) as an off-white waxy solid. MS (ESI, m/z): 692.4 [M+H]⁺.

### Step 3)

### 4-[4-[2-[[4-[[3-[4-(Difluoromethoxy)phenyl]imidazo[1,2-a]pyrazin-8-yl]amino]-2-methylbenzoyl]-methylamino]ethyl]-1-methylpiperidin-1-ium-1-yl]butanoic acid hydrochloride

1-(4-(tert-butoxy)-4-oxobutyl)-4-(2-(4-((3-(4-(difluoromethoxy)phenyl)imidazo[1,2-a]pyrazin-8-yl)amino)-N,2-dimethylbenzamido)ethyl)-1-methylpiperidin-1-ium formate (30.4 mg, 41.3 µmol, Eq: 1) was dissolved in dioxane (100 µL) and then was added 4M HCl in dioxane (206 µL, 826 µmol, Eq: 20). The reaction mixture was stirred at RT over the weekend. The volatiles were removed and the product was lyophilized to afford the title compound (21.8 mg, 32.4 µmol, 77% yield) as a yellow solid. MS (ESI, m/z): 635.3 [M+H]⁺.

### Example 58

### [2-[2-[[2-Chloro-4-[[3-[3-(trifluoromethyl)-1H-pyrazol-4-yl]imidazo[1,2-a]pyrazin-8-yl]amino]benzoyl]amino]ethylamino]-2-oxo-ethyl]-trimethyl-ammonium formate

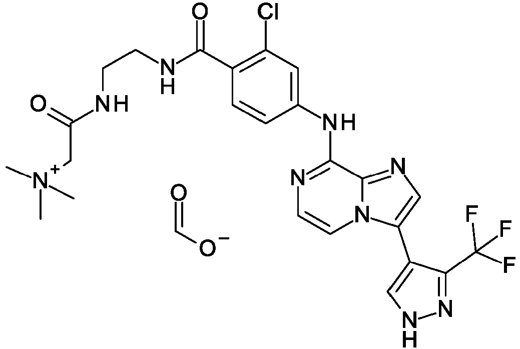

### Step 1) Intermediate 26

### Methyl 2-chloro-4-[(3-iodoimidazo[1,2-a]pyrazin-8-yl)amino]benzoate

To a solution of 8-chloro-3-iodo-imidazo[1,2-a]pyrazine (Intermediate 1, 5 g, 17.89 mmol, 1 eq) in ACN (27.5 mL)/acetic acid (27.5 mL) was added methyl 4-amino-2-chlorobenzoate (3.98 g, 21.47 mmol, 1.2 eq) and the reaction was stirred at 80°C for 12 h. After cooled to room temperature, the reaction mixture was filtered, washed with ACN:MeOH=10:1 and dried to afford the title compound (6.52 g, 15.2 mmol, 87.83% yield) as an off-white solid.

### Step 2)

### Methyl 2-chloro-4-[[3-[3-(trifluoromethyl)-1H-pyrazol-4-yl]imidazo[1,2-a]pyrazin-8-yl]amino]benzoate

To a solution of 4-(4,4,5,5-tetramethyl-1,3,2-dioxaborolan-2-yl)-3-(trifluoromethyl)-1H-pyrazole (1.59 g, 6.07 mmol, 1.3 eq) in water (115 mL)/1,4-dioxane (115 mL) was added methyl 2-chloro-4-[(3-iodoimidazo[1,2-a]pyrazin-8-yl)amino]benzoate (2 g, 4.67 mmol, 1 eq), 1,1'-bis(diphenylphosphino)ferrocene-palladium(II)dichloride dichloromethane complex (1.14 g, 1.4 mmol, 0.300 eq) and sodium carbonate (1.48 g, 14 mmol, 3 eq) at 25 °C. The reaction mixture was stirred at 90 °C for 12 h under N₂. The reaction was poured into water (100 ml) and extracted with DCM (50 ml x 3). The organic phase was combined and concentrated under reduce pressure. The target compound was purified by silica chromatography column (PE: EtOAc=50:1 to 1:1) to afford the title compound (0.400 g, 0.920 mmol, 19.63% yield) as a dark green solid. MS (ESI, m/z): 437.0 [M+H]⁺.

### Step 3)

### 2-Chloro-4-[[3-[3-(trifluoromethyl)-1H-pyrazol-4-yl]imidazo[1,2-a]pyrazin-8-yl]amino]benzoic acid

To a solution of methyl 2-chloro-4-[[3-[3-(trifluoromethyl)-1H-pyrazol-4-yl]imidazo[1,2-a]pyrazin-8-yl]amino]benzoate (400 mg, 0.920 mmol, 1 eq) in THF (5 mL) was added aqueous sodium hydroxide (5 mL, 10 mmol, 10.92 eq). The reaction mixture was stirred at 60°C for 12 h. The reaction was adjusted to pH=6~7 by 1N HCl addition and then it was concentrated under reduce pressure to afford the title compound (1 g, 2.37 mmol, quantitative yield) as an off-white solid. MS (ESI, m/z): 423.0 [M+H]⁺.

### Step 4)

### tert-Butyl N-[2-[[2-(dimethylamino)acetyl]amino]ethyl]carbamate

To a solution of N,N-dimethylglycine (1 g, 9.7 mmol, 1 eq) in DMF (10 mL) was added 1,1'-carbonyldiimidazole (1.57 g, 9.7 mmol, 1 eq). The mixture was stirred at 50 °C for 1 h. Then N-Boc-ethylenediamine (1.71 g, 10.67 mmol, 1.1 eq) was added to the mixture and it was stirred for 12 h. The reaction mixture was diluted with water (30 mL) and extracted with DCM/MeOH=10:1 (30 mL x 3).The combined organic layer was washed with brine (50 mL), dried over Na₂SO₄, filtered and concentrated to afford the title compound (3.5 g, 14.27 mmol, 147.12% yield) as a white solid. MS (ESI, m/z): 246.2 [M+H]⁺.

### Step 5)

### [2-[2-(tert-Butoxycarbonylamino)ethylamino]-2-oxo-ethyl]-trimethyl-ammonium iodide

To a solution of tert-butyl N-[2-[[2-(dimethylamino)acetyl]amino]ethyl]carbamate (73 mg, 2.04 mmol, 1 eq) in ethyl acetate (10 mL) was added iodomethane (318.1 mg, 2.24 mmol, 1.1 eq). The reaction mixture was stirred at 30 °C for 16 h. Then it was filtered and the filter cake was collected to afford the title compound (450 mg, 1.16 mmol, 57.04% yield) as a light yellow solid. MS (ESI, m/z): 260.3 [M+H]⁺.

### Step 6)

### [2-(2-Aminoethylamino)-2-oxo-ethyl]-trimethyl-ammonium iodide hydrochloride

[2-[2-(tert-butoxycarbonylamino)ethylamino]-2-oxo-ethyl]-trimethyl-ammonium iodide (450 mg, 1.16 mmol, 1 eq) was added to hydrogen chloride in methanol (5 mL, 20 mmol, 17.21 eq). The reaction mixture was stirred at 30 °C for 1 h. The reaction was concentrated to afford the title compound (370 mg, 1.14 mmol, 98.4% yield) as a yellow oil. MS (ESI, m/z): 160.1 [M+H]⁺.

### Step 7)

### [2-[2-[[2-Chloro-4-[[3-[3-(trifluoromethyl)-1H-pyrazol-4-yl]imidazo[1,2-a]pyrazin-8-yl]amino]benzoyl]amino]ethylamino]-2-oxo-ethyl]-trimethyl-ammonium formate

To a solution of O-(7-azabenzotriazol-1-yl)-N,N,N',N'-tetramethyluronium hexafluorophosphate (270 mg, 0.710 mmol, 3 eq) and N,N-diisopropylethylamine (0.06 mL, 0.350 mmol, 1.5 eq) in DMF (4 mL) was added [2-(2-aminoethylamino)-2-oxo-ethyl]-trimethyl-ammonium iodide hydrochloride (92 mg, 0.280 mmol, 1.2 eq) and 2-chloro-4-[[3-[3-(trifluoromethyl)-1H-pyrazol-4-yl]imidazo[1,2-a]pyrazin-8-yl]amino]benzoic acid (250 mg, 0.240 mmol, 1 eq). The reaction was stirred at 30 °C for 12 h. The mixture product was purified by prep-HPLC (FA) and lyophilised to afford the title compound (60 mg, 0.100 mmol, 41.58% yield) as a white solid. MS (ESI, m/z): 564.2 [M+H]⁺.

### Example 59

### [2-[[3-[[2-Chloro-4-[[3-(2,3-difluoro-4-methoxy-phenyl)imidazo[1,2-a]pyrazin-8-yl]amino]benzoyl]amino]cyclobutyl]amino]-2-oxo-ethyl]-trimethyl-ammonium formate

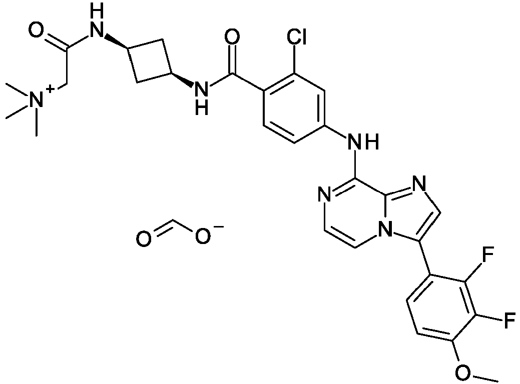

### Step 1)

### tert-Butyl N-[3-[[2-(dimethylamino)acetyl]amino]cyclobutyl]carbamate

To a solution of N,N-dimethylglycine (500 mg, 4.85 mmol, 1 eq) in DMF (5 mL) was added 1,1'-carbonyldiimidazole (786 mg, 4.85 mmol, 1 eq). The reaction mixture was stirred at 50 °C for 1 h. Then tert-butyl N-(3-aminocyclobutyl)carbamate (903 mg, 4.85 mmol, 1 eq) was added to the mixture and it was stirred at 50 °C for 12 h. The reaction mixture was diluted with water (20 mL) and extracted with DCM/MeOH=10:1 (20 mL x 3). The combined organic layer was washed with brine (50 mL), dried over Na₂SO₄, filtered and concentrated to afford the title compound (1.31 g, 4.83 mmol, 99.56% yield) as a colorless oil. MS (ESI, m/z): 272.3 [M+H]⁺.

### Step 2)

### [2-[[3-(tert-Butoxycarbonylamino)cyclobutyl]amino]-2-oxo-ethyl]-trimethyl-ammonium iodide

To a solution of tert-butyl N-[3-[[2-(dimethylamino)acetyl]amino]cyclobutyl]carbamate (1.31 g, 4.83 mmol, 1 eq) in ethyl acetate (15 mL) was added iodomethane (822 mg, 5.79 mmol, 1.2 eq). The reaction mixture was stirred at 30 °C for 16 h. The reaction was filtered and the filter cake was collected and dried to afford the title compound (1.3 g, 3.15 mmol, 65.16% yield) as a yellow solid. MS (ESI, m/z): 286.3 [M+H]⁺.

### Step 3) Intermediate 27

### [2-[(3-Aminocyclobutyl)amino]-2-oxo-ethyl]-trimethyl-ammonium iodide hydrochloride

[2-[[3-(tert-butoxycarbonylamino)cyclobutyl]amino]-2-oxo-ethyl]-trimethyl-ammonium iodide (1.3 g, 3.15 mmol, 1 eq) was added to 4M hydrogen chloride in methanol (15 mL, 60 mmol, 19.07 eq). The reaction was stirred at 30 °C for 1 h. The reaction mixture was concentrated to afford the title compound (1.1 g, 3.15 mmol, quantitative yield) as a yellow oil. MS (ESI, m/z): 186.2 [M+H]⁺.

### Step 4)

### [2-[[3-[[2-Chloro-4-[[3-(2,3-difluoro-4-methoxy-phenyl)imidazo[1,2-a]pyrazin-8-yl]amino]benzoyl]amino]cyclobutyl]amino]-2-oxo-ethyl]-trimethyl-ammonium formate

To a solution of 2-chloro-4-[[3-(2,3-difluoro-4-methoxy-phenyl)imidazo[1,2-a]pyrazin-8-yl]amino]benzoic acid (Intermediate 5, 100 mg, 0.230 mmol, 1 eq) and [2-[(3-aminocyclobutyl)amino]-2-oxo-ethyl]-trimethyl-ammonium iodide hydrochloride (97.4 mg, 0.280 mmol, 1.2 eq) in DMF (2 mL) was added N,N-diisopropylethylamine (0.12 mL, 0.700 mmol, 3 eq) and O-(7-azabenzotriazol-1-yl)-N,N,N',N'-tetramethyluronium hexafluorophosphate (132.4 mg, 0.350 mmol, 1.5 eq). The reaction was stirred at 30 °C for 16 h. After adjusted to pH=4~5 with formic acid, the mixture was purified by prep-HPLC (FA) and lyophilized to afford the title compound (81.3 mg, 0.130 mmol, 54.38% yield) as a grey solid. MS (ESI, m/z): 598.3 [M+H]⁺.

### Example 60

### 2-(4-(4-((3-(2,3-Difluoro-4-methoxyphenyl)imidazo[1,2-a]pyrazin-8-yl)amino)-2-methylbenzamido)piperidin-1-yl)-N,N,N-trimethyl-2-oxoethan-1-aminium chloride

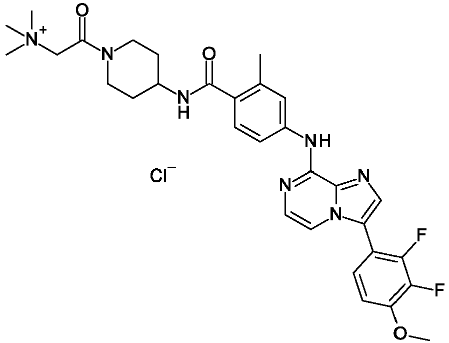

### Step 1)

### tert-Butyl 4-(4-((3-(2,3-difluoro-4-methoxyphenyl)imidazo[1,2-a]pyrazin-8-yl)amino)-2-methylbenzamido)piperidine-1-carboxylate

Under argon, 4-((3-(2,3-difluoro-4-methoxyphenyl)imidazo[1,2-a]pyrazin-8-yl)amino)-2-methylbenzoic acid (Intermediate 6, 200 mg, 487 µmol, Eq: 1) was suspended in DMF (2 ml). DIPEA (315 mg, 414 µl, 2.44 mmol, Eq: 5) was added followed by tert-butyl 4-aminopiperidine-1-carboxylate (117 mg, 584 µmol, Eq: 1.2) and 2-(3H-[1,2,3]triazolo[4,5-b]pyridin-3-yl)-1,1,3,3-tetramethylisouronium hexafluorophosphate(V) (HATU) (370 mg, 974 µmol, Eq: 2). The brown solution was stirred at RT over 2 h. The solvent was evaporated and the crude material was purified by flash chromatography (silica gel, 20 g, 0% to 100% DCM/MeOH/NH₄OH (95/5/1) / DCM) to afford the title compound (389 mg, 656 µmol, 135% yield) as a light brown solid. MS (ESI, m/z): 593.3 [M+H]⁺.

### Step 2)

### 4-((3-(2,3-Difluoro-4-methoxyphenyl)imidazo[1,2-a]pyrazin-8-yl)amino)-2-methyl-N-(piperidin-4-yl)benzamide

Under argon, tert-butyl 4-(4-((3-(2,3-difluoro-4-methoxyphenyl)imidazo[1,2-a]pyrazin-8-yl)amino)-2-methylbenzamido)piperidine-1-carboxylate (178 mg, 300 µmol, Eq: 1) was dissolved in DCM (1.5 ml). TFA (1.2 g, 804 µl, 10.5 mmol, Eq: 35) was added and the reaction mixture was stirred at RT over 1 h. DCM/MeOH/NH₄OH was added till the TFA was neutralised and the reaction mixture was evaporated. The crude was purified by flash chromatography (silica gel, 20 g, 0% to 100% DCM/MeOH/NH₄OH (90/10/1)) to afford the title compound (165 mg, 335 µmol, 100% yield) as an off-white solid. MS (ESI, m/z): 493.3 [M+H]⁺.

### Step 3)

### 2-(4-(4-((3-(2,3-Difluoro-4-methoxyphenyl)imidazo[1,2-a]pyrazin-8-yl)amino)-2-methylbenzamido)piperidin-1-yl)-N,N,N-trimethyl-2-oxoethan-1-aminium chloride

Under argon, 4-((3-(2,3-difluoro-4-methoxyphenyl)imidazo[1,2-a]pyrazin-8-yl)amino)-2-methyl-N-(piperidin-4-yl)benzamide (104 mg, 211 µmol, Eq: 1) was dissolved in DMF (2.08 ml). Betaine hydrochloride (35.7 mg, 232 µmol, Eq: 1.1) and 2-(3H-[1,2,3]triazolo[4,5-b]pyridin-3-yl)-1,1,3,3-tetramethylisouronium hexafluorophosphate(V) HATU (88.3 mg, 232 µmol, Eq: 1.1) were added followed by DIPEA (54.6 mg, 73.8 µl, 422 µmol, Eq: 2). The reaction was stirred at RT over 1 h. The solvent was evaporated and the crude was then purified by reverse phase preparative HPLC (FA) to afford the title compound (72 mg, 114 µmol, 54.3% yield) as light yellow solid. MS (ESI, m/z): 592.4 [M+H]⁺.

### Example 61

### [2-[[3-[[2-Chloro-4-[[3-[3-(trifluoromethyl)-1H-pyrazol-4-yl]imidazo[1,2-a]pyrazin-8-yl]amino]benzoyl]amino]cyclobutyl]amino]-2-oxo-ethyl]-trimethyl-ammonium formate

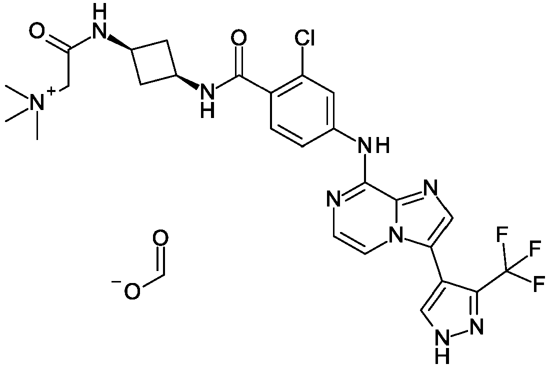

### Step 1)

### [2-[[3-[[2-chloro-4-[[3-[3-(trifluoromethyl)-1-trityl-pyrazol-4-yl]imidazo[1,2-a]pyrazin-8-yl]amino]benzoyl]amino]cyclobutyl]amino]-2-oxo-ethyl]-trimethyl-ammonium iodide

The title compound (105 mg, 0.110 mmol, 42.78% yield) was obtained as a yellow solid in analogy to Example 7, Step 5 using Intermediate 28and Intermediate 27. MS (ESI, m/z): 832.3 [M+H]⁺.

### Step 2)

### [2-[[3-[[2-chloro-4-[[3-[3-(trifluoromethyl)-1H-pyrazol-4-yl]imidazo[1,2-a]pyrazin-8-yl]amino]benzoyl]amino]cyclobutyl]amino]-2-oxo-ethyl]-trimethyl-ammonium formate

The title compound (13.5 mg, 0.020 mmol, 18.64% yield) was obtained as a white solid in analogy to Example 7, Step 6. MS (ESI, m/z): 590.2 [M+H]⁺.

### Example 62

### [2-[4-[2-Chloro-4-[[3-[3-(trifluoromethyl)-1H-pyrazol-4-yl]imidazo[1,2-a]pyrazin-8-yl]amino]benzoyl]piperazin-1-yl]-2-oxo-ethyl]-trimethyl-ammonium formate

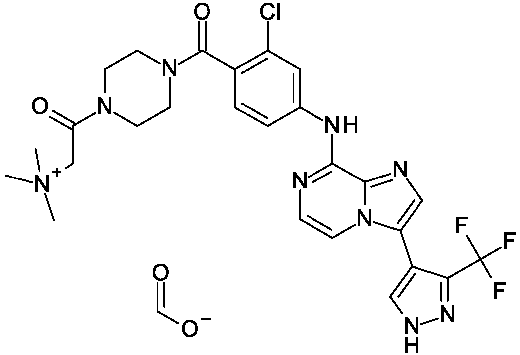

### Step 1)

### Methyl 2-chloro-4-[(3-iodoimidazo[1,2-a]pyrazin-8-yl)amino]benzoate

To a solution of 8-chloro-3-iodo-imidazo[1,2-a]pyrazine (Intermediate 1, 5 g, 17.89 mmol, 1 eq) in ACN (50 mL) was added acetic acid (5 mL, 17.89 mmol, 1 eq) and methyl 4-amino-2-chlorobenzoate (3.98 g, 21.47 mmol, 1.2 eq). The reaction was stirred at 80°C for 48 h. After cooled to room temperature, the reaction mixture was filtered and washed with ACN:MeOH=10:1 and then dried to afford the title compound (6.8 g, 15.87 mmol, 88.68% yield) as an off-white solid. MS (ESI, m/z): 428.9 [M+H]⁺.

### Step 2)

### 2-Chloro-4-[(3-iodoimidazo[1,2-a]pyrazin-8-yl)amino]benzoic acid

To a solution of methyl 2-chloro-4-[(3-iodoimidazo[1,2-a]pyrazin-8-yl)amino]benzoate (6.5 g, 15.17 mmol, 1 eq) in THF (40 mL) was added 2M aqueous sodium hydroxide (40 mL, 80 mmol, 5.28 eq) and it was stirred at 60°C for 12 h. The reaction mixture was adjusted to pH=1~2 with 3N HCl and filtered. The filtrate was extracted with DCM (10 mL x 3), dried over Na₂SO₄ and concentrated to afford the title compound (6 g, 14.47 mmol, 95.43% yield) as a white solid. MS (ESI, m/z): 414.9 [M+H]⁺.

### Step 3)

### tert-Butyl 4-[2-chloro-4-[(3-iodoimidazo[1,2-a]pyrazin-8-yl)amino]benzoyl]piperazine-1-carboxylate

To a solution of N,N-diisopropylethylamine (7.56 mL, 43.42 mmol, 3 eq), 2-chloro-4-[(3-iodoimidazo[1,2-a]pyrazin-8-yl)amino]benzoic acid (6 g, 14.47 mmol, 1 eq) and 1-Boc-piperazine (0.08 mL, 15.92 mmol, 1.1 eq) in DMF (50 mL) was added O-(7-azabenzotriazol-1-yl)-N,N,N',N'-tetramethyluronium hexafluorophosphate (11.01 g, 28.94 mmol, 2 eq) at 30 °C. The reaction mixture was stirred at 30 °C for 12 h. 100 mL of water was added and the mixture was filtered to afford the title compound (6 g, 10.29 mmol, 71.14% yield) as an off-white solid. MS (ESI, m/z): 583.0 [M+H]⁺.

### Step 4)

### tert-Butyl 4-[2-chloro-4-[[3-[3-(trifluoromethyl)-1H-pyrazol-4-yl]imidazo[1,2-a]pyrazin-8-yl]amino]benzoyl]piperazine-1-carboxylate

To a solution of tert-butyl 4-[2-chloro-4-[(3-iodoimidazo[1,2-a]pyrazin-8-yl)amino]benzoyl]piperazine-1-carboxylate (3 g, 5.15 mmol, 1 eq) in water (9 mL)/1,4-dioxane (90 mL) was added 4-(4,4,5,5-tetramethyl-1,3,2-dioxaborolan-2-yl)-3-(trifluoromethyl)-1H-pyrazole (1.75 g, 6.69 mmol, 1.3 eq), 1,1'-bis(diphenylphosphino)ferrocene-palladium(II)dichloride dichloromethane complex (0.42 g, 0.510 mmol, 0.1 eq) and sodium carbonate (1637 mg, 15.44 mmol, 3 eq) at 25 °C. The mixture was stirred at 90 °C for 12 h under N₂. The reaction mixture was poured into water (100 ml) and extracted with EtOAc (50 ml x 3). The organic phase was combined and concentrated under reduced pressure and then purified by silica chromatography column (PE: EtOAc=1:1 to EtOAc) to afford the title compound (1.4 g, 2.37 mmol, 36.82% yield) as a light brown solid. MS (ESI, m/z): 591.1 [M+H]⁺.

### Step 5)

### [2-Chloro-4-[[3-[3-(trifluoromethyl)-1H-pyrazol-4-yl]imidazo[1,2-a]pyrazin-8-yl]amino]phenyl]-piperazin-1-yl-methanone hydrochloride

Tert-butyl 4-[2-chloro-4-[[3-[3-(trifluoromethyl)-1H-pyrazol-4-yl]imidazo[1,2-a]pyrazin-8-yl]amino]benzoyl]piperazine-1-carboxylate (1.4 g, 2.37 mmol, 1 eq) was added hydrochloric acid in dioxane (35.1 mL, 140.43 mmol, 59.28 eq) and then it was stirred at 20 °C for 2 h. The mixture was concentrated in vacuum and purified by prep-HPLC (HCl) to afford the title compound (900 mg, 1.83 mmol, 77.4% yield) as an off-white solid. MS (ESI, m/z): 491.2 [M+H]⁺.

### Step 6)

### [2-[4-[2-Chloro-4-[[3-[3-(trifluoromethyl)-1H-pyrazol-4-yl]imidazo[1,2-a]pyrazin-8-yl]amino]benzoyl]piperazin-1-yl]-2-oxo-ethyl]-trimethyl-ammonium formate

To a solution of N,N-diisopropylethylamine (0.11 mL, 0.610 mmol, 3 eq), betaine (33.32 mg, 0.280 mmol, 1.5 eq) and [2-chloro-4-[[3-[3-(trifluoromethyl)-1H-pyrazol-4-yl]imidazo[1,2-a]pyrazin-8-yl]amino]phenyl]-piperazin-1-yl-methanone (100 mg, 0.200 mmol, 1 eq) in DMF (1.92 mL) was added O-(7-azabenzotriazol-1-yl)-N,N,N',N'-tetramethyluronium hexafluorophosphate (154.92 mg, 0.410 mmol, 2 eq). The reaction mixture was stirred at 20 °C for 12 h. The mixture was acidified with HCOOH and then purified by prep-HPLC (FA) to afford the title compound (28.7 mg, 0.050 mmol, 21.8% yield) as an off-white solid. MS (ESI, m/z): 590.3 [M+H]⁺.

### Example 63

### 2-(((1s,4s)-4-(4((3-(2,3-Difluoro-4-methoxyphenyl)imidazo[1,2-a]pyrazin-8-yl)amino)-2-methylbenzamido)cyclohexyl)amino)-N,N,N-trimethyl-2-oxoethan-1-aminium chloride

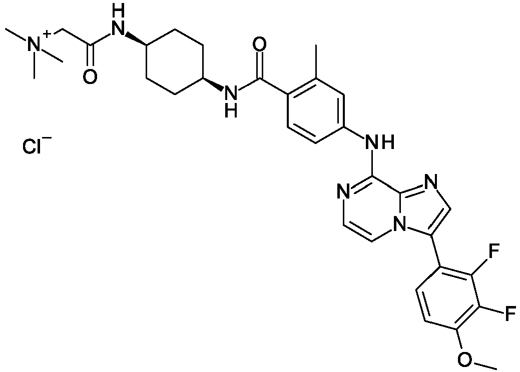

### Step 1)

### tert-Butyl ((1s,4s)-4-(4-((3-(2,3-difluoro-4-methoxyphenyl)imidazo[1,2-a]pyrazin-8-yl)amino)-2-methylbenzamido)cyclohexyl)carbamate

Under argon 4-((3-(2,3-difluoro-4-methoxyphenyl)imidazo[1,2-a]pyrazin-8-yl)amino)-2-methylbenzoic acid (Intermediate 6, 200 mg, 487 µmol, Eq: 1) was suspended in DMF (2 ml). N-ethyl-N-isopropylpropan-2-amine (DIPEA) (315 mg, 414 µl, 2.44 mmol, Eq: 5) was added followed by tert-butyl ((1s,4s)-4-aminocyclohexyl)carbamate (125 mg, 584 µmol, Eq: 1.2) and 2-(3H-[1,2,3]triazolo[4,5-b]pyridin-3-yl)-1,1,3,3-tetramethylisouronium hexafluorophosphate(V) (HATU) (370 mg, 974 µmol, Eq: 2). The solution was stirred at RT over 2 h. The solvent was evaporated and the crude material was purified by flash chromatography (silica gel, 20 g, 0% to 100% DCM/(DCM/MeOH/NH₄OH (95/5/1))) to afford the title compound (366 mg, 603 µmol, 124% yield) as a light red solid. MS (ESI, m/z): 607.3 [M+H]⁺.

### Step 2)

### N-((1s,4s)-4-Aminocyclohexyl)-4-((3-(2,3-difluoro-4-methoxyphenyl)imidazo[1,2-a]pyrazin-8-yl)amino)-2-methylbenzamide

Under argon, tert-butyl ((1s,4s)-4-(4-((3-(2,3-difluoro-4-methoxyphenyl)imidazo[1,2-a]pyrazin-8-yl)amino)-2-methylbenzamido)cyclohexyl)carbamate (182 mg, 300 µmol, Eq: 1) was dissolved in DCM (1.5 ml). TFA (1.2 g, 804 µl, 10.5 mmol, Eq: 35) was added and the reaction mixture was stirred at RT over 1 h. DCM/MeOH/NH₄OH was added till the TFA was neutralised and the mixture was evaporated. The crude material was purified by flash chromatography (silica gel, 20 g, 0% to 100% DCM/MeOH/NH₄OH (90/10/1)) to afford the title compound (131 mg, 259 µmol, 86% yield) as a white solid. MS (ESI, m/z): 507.3 [M+H]⁺.

### Step 3)

### 2-(((1s,4s)-4-(4-((3-(2,3-difluoro-4-methoxyphenyl)imidazo[1,2-a]pyrazin-8-yl)amino)-2-methylbenzamido)cyclohexyl)amino)-N,N,N-trimethyl-2-oxoethan-1-aminium chloride

Under argon, 2-(trimethylammonio)acetate (18.6 mg, 159 µmol, Eq: 1.15) was suspended in DCM (1 ml). 1-Chloro-N,N,2-trimethyl-1-propenylamine (23.1 mg, 22.9 µl, 173 µmol, Eq: 1.25) was added and the reaction mixture was stirred at RT over 30 minutes. A suspension containing N-((1s,4s)-4-aminocyclohexyl)-4-((3-(2,3-difluoro-4-methoxyphenyl)imidazo[1,2-a]pyrazin-8-yl)amino)-2-methylbenzamide (70 mg, 138 µmol, Eq: 1) and N-ethyl-N-isopropylpropan-2-amine (44.7 mg, 58.8 µl, 345 µmol, Eq: 2.5) was added and the reaction mixture was stirred at RT over 1 h. The reaction was not finished so 1-carboxy-N,N,N-trimethylmethanaminium chloride (21.2 mg, 138 µmol, Eq: 1) was dissolved in 1 ml of DMA. 1-Chloro-N,N,2-trimethyl-1-propenylamine (23.1 mg, 22.9 µl, 173 µmol, Eq: 1.25) was added and after 30 minutes at RT, this solution was added to the reaction mixture and it was stirred at RT over 1 h. The solvent was evaporated and the crude material was purified by reverse phase chromatography under acidic conditions (FA) to afford the title compound (5 mg, 7.7 µmol, 5.63% yield) as an off-white solid. MS (ESI, m/z): 606.3 [M+H]⁺.

### Example 64

### [2-[4-[4-[[3-[1-(Cyanomethyl)-3-(trifluoromethyl)pyrazol-4-yl]imidazo[1,2-a]pyrazin-8-yl]amino]-2-methyl-benzoyl]piperazin-1-yl]-2-oxo-ethyl]-trimethyl-ammonium formate

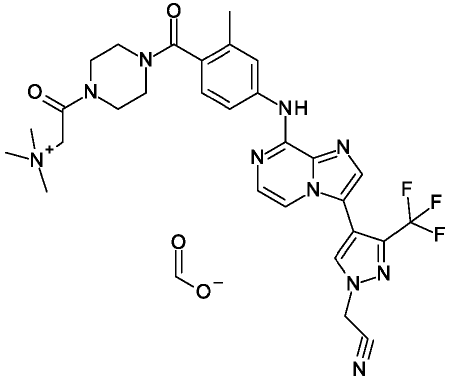

### Step 1)

### Methyl 4-[(3-iodoimidazo[1,2-a]pyrazin-8-yl)amino]-2-methyl-benzoate

To a solution of 8-chloro-3-iodo-imidazo[1,2-a]pyrazine (Intermediate 1, 5 g, 17.89 mmol, 1 eq) in ACN (50 mL)/acetic acid (5 mL) was added methyl 4-amino-2-methylbenzoate (3.55 g, 21.47 mmol, 1.2 eq) and the reaction mixture was stirred at 80°C for 12 h. After cooled to room temperature, the reaction was filtered, washed with ACN:MeOH=10:1 and dried to afford the title compound (6.1 g, 14.94 mmol, 83.53% yield) as an off-white solid.

### Step 2)

### 4-[(3-Iodoimidazo[1,2-a]pyrazin-8-yl)amino]-2-methyl-benzoic acid

To a solution of methyl 4-[(3-iodoimidazo[1,2-a]pyrazin-8-yl)amino]-2-methyl-benzoate (6.1 g, 14.94 mmol, 1 eq) in THF (20 mL)/methanol (20 mL) was added 2M aqueous sodium hydroxide (20 mL, 40 mmol, 2.68 eq) and it was stirred at 60 °C for 16 h. After cooled to room temperature, the reaction mixture was adjusted to pH=1~2 with 3N HCl, filtered and dried to afford the title compound (5.8 g, 14.71 mmol, 98.46% yield) as an off-white solid. MS (ESI, m/z): 394.9 [M+H]⁺.

### Step 3)

### tert-Butyl 4-[4-[(3-iodoimidazo[1,2-a]pyrazin-8-yl)amino]-2-methyl-benzoyl]piperazine-1-carboxylate

To a solution of 4-[(3-iodoimidazo[1,2-a]pyrazin-8-yl)amino]-2-methyl-benzoic acid (3 g, 7.61 mmol, 1 eq) in DMF (30 mL) was added 1-Boc-piperazine (1.7 g, 9.13 mmol, 1.2 eq), N,N-diisopropylethylamine (3.98 mL, 22.83 mmol, 3 eq) and O-(7-azabenzotriazol-1-yl)-N,N,N',N'-tetramethyluronium hexafluorophosphate (5.79 g, 15.22 mmol, 2 eq). The reaction mixture was stirred at 30 °C for 16 h. 160 mL of water were added. This mixture was filtered and the filter cake was dried to afford the title compound (3 g, 5.33 mmol, 70.09% yield) as an off-white solid. MS (ESI, m/z): 563.2 [M+H]⁺.

### Step 4)

### 2-[4-Iodo-3-(trifluoromethyl)pyrazol-1-yl] acetonitrile

To a mixture of 4-iodo-3-(trifluoromethyl)-1H-pyrazole (10 g, 38.17 mmol, 1 eq) in acetone (200 mL) was added potassium carbonate (6.33 g, 45.81 mmol, 1.2 eq) and 2-bromoacetonitrile (6.87 g, 57.26 mmol, 1.5 eq) at -60 °C . The mixture was stirred at 0 °C for 4 h. The mixture was poured into water (50 mL). The aqueous phase was extracted with EtOAc (50 mL x 2). The combined organic phase was washed with brine (100 mL), dried over anhydrous Na₂SO₄ and concentrated in vacuum. The residue was purified by silical gel column chromatography (petroleum ether/EtOAc=20/1) to afford the title compound (10 g, 33.2 mmol, 87.03% yield) as a colorless oil. 1H NMR (400 MHz, CHLOROFORM-d) Shift 7.74 (d, J=0.73 Hz, 1H), 5.14 (s, 2H).

### Step 5) Intermediate 29

### 2-[4-(4,4,5,5-Tetramethyl-1,3-dioxolan-2-yl)-3-(trifluoromethyl)pyrazol-1-yl]acetonitrile

To a solution of 2-[4-iodo-3-(trifluoromethyl)pyrazol-1-yl]acetonitrile (8.45 g, 28.07 mmol, 1 eq) in dimethylsulfoxide (90 mL) was added 1,1'-bis(diphenylphosphino)ferrocene-palladium(II)dichloride dichloromethane complex (1.15 g, 1.4 mmol, 0.05 eq), potassium acetate (5.26 mL, 84.22 mmol, 3 eq) and bis(pinacolato)diboron (10.69 g, 42.11 mmol, 1.5 eq) under N₂. The reaction was stirred at 70°C for 16 h. The reaction mixture was poured into water (100 ml) and extracted with EtOAc (60 ml x 2). The combined organic phase was washed with brine, dried over anhydrous Na₂SO₄, filtered and concentrated under reduce pressure to afford the title compound (18.8 g, 62.44 mmol, 222.44% yield) as a dark brown oil. MS (ESI, m/z): 299.9 [M+H]⁺.

### Step 6)

### tert-butyl 4-[4-[[3-[1-(cyanomethyl)-3-(trifluoromethyl)pyrazol-4-yl]imidazo[1,2-a]pyrazin-8-yl]amino]-2-methyl-benzoyl]piperazine-1-carboxylate

To a solution of tert-butyl 4-[4-[(3-iodoimidazo[1,2-a]pyrazin-8-yl)amino]-2-methylbenzoyl]piperazine-1-carboxylate (1.3 g, 2.31 mmol, 1 eq) in water (3 mL)/1,4-dioxane (30 mL) were added 2-[4-(4,4,5,5-tetramethyl-1,3-dioxolan-2-yl)-3-(trifluoromethyl)pyrazol-1-yl]acetonitrile (7.67 g, 22.76 mmol, 9.85 eq), 1,1'-bis(diphenylphosphino)ferrocene-palladium(II)dichloride dichloromethane complex (188.62 mg, 0.230 mmol, 0.10 eq) and sodium carbonate (0.73 g, 6.93 mmol, 3 eq) at 25 °C. The mixture was stirred at 100 °C for 12 h. The mixture was poured into water (50 mL). The aqueous phase was extracted with EtOAc (50 mL x 2). The combined organic phase was washed with brine (61 mL) and concentrated in vacuum. The residue was purified by silical gel column chromatography (petroleum ether/EtOAc=1/90) to afford the title compound (900 mg, 1.48 mmol, 63.87% yield) as a brown solid. MS (ESI, m/z): 610.3 [M+H]⁺.

### Step 7)

### 2-[4-[8-[3-Methyl-4-(piperazine-1-carbonyl)anilino]imidazo[1,2-a]pyrazin-3-yl]-3-(trifluoromethyl)pyrazol-1-yl]acetonitrile

To a solution of tert-butyl 4-[4-[[3-[1-(cyanomethyl)-3-(trifluoromethyl)pyrazol-4-yl]imidazo[1,2-a]pyrazin-8-yl]amino]-2-methyl-benzoyl]piperazine-1-carboxylate (0.9 g, 1.48 mmol, 1 eq) in DCM (45 mL) was added trifluoroacetic acid (9 mL, 116.82 mmol, 79.13 eq) and it was stirred at 20 °C for 2 h. The pH was adjusted to 8-9 by the addition of aqueous saturated Na₂CO₃. The aqueous phase was extracted with DCM (40 mL x 2). The combined organic phase was dried over Na₂SO₄, filtered and concentrated to afford the title compound (540 mg, 1.06 mmol, 71.79% yield) as a light brown solid. MS (ESI, m/z): 510.1 [M+H]⁺.

### Step 8)

### [2-[4-[4-[[3-[1-(Cyanomethyl)-3-(trifluoromethyl)pyrazol-4-yl]imidazo[1,2-a]pyrazin-8-yl]amino]-2-methyl-benzoyl]piperazin-1-yl]-2-oxo-ethyl]-trimethyl-ammonium formate

To a solution of N,N-diisopropylethylamine (0.11 mL, 0.610 mmol, 3 eq), betaine (35.8 mg, 0.310 mmol, 1.5 eq) and 2-[4-[8-[3-methyl-4-(piperazine-1-carbonyl)anilino]imidazo[1,2-a]pyrazin-3-yl]-3-(trifluoromethyl)pyrazol-1-yl]acetonitrile (100 mg, 0.200 mmol, 1 eq) in DMF (1.85 mL) was added O-(7-azabenzotriazol-1-yl)-N,N,N',N'-tetramethyluronium hexafluorophosphate (154.92 mg, 0.410 mmol, 2 eq) and the reaction mixture was stirred at 20 °C for 12 h. The mixture was acidified with HCOOH and then purified by prep-HPLC (FA) to afford the title compound (26.8 mg, 0.040 mmol, 20.1% yield) as an off-white solid. MS (ESI, m/z): 609.3 [M]⁺.

### Example 65

### [2-[4-[2-Chloro-4-[[3-[1-(cyanomethyl)-3-(trifluoromethyl)pyrazol-4-yl]imidazo[1,2-a]pyrazin-8-yl]amino]benzoyl]piperazin-1-yl]-2-oxo-ethyl]-trimethyl-ammonium formate

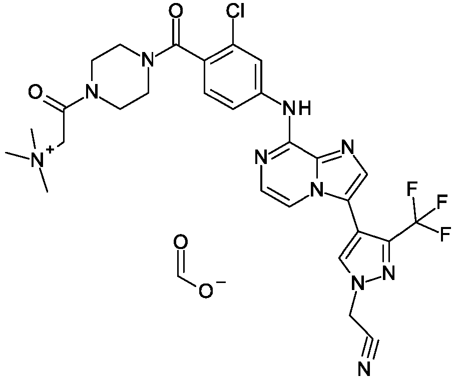

### Step 1)

### 2-Chloro-4-[(3-iodoimidazo[1,2-a]pyrazin-8-yl)amino]benzoic acid

To a solution of methyl 2-chloro-4-[(3-iodoimidazo[1,2-a]pyrazin-8-yl)amino]benzoate (Intermediate 26, 2 g, 4.67 mmol, 1 eq) in THF (7 mL)/methanol (7 mL) was added aqueous sodium hydroxide (7 mL, 14 mmol, 3 eq) and it was stirred at 60 °C for 12 h. After cooled to room temperature, the reaction mixture was adjusted to pH=6~7 with 3N HCl, filtered and dried to afford the title compound (1.5 g, 3.62 mmol, 77.54% yield) as an off-white solid. MS (ESI, m/z): 414.9 [M+H]⁺.

### Step 2)

### tert-Butyl 4-[2-chloro-4-[(3-iodoimidazo[1,2-a]pyrazin-8-yl)amino]benzoyl]piperazine-1-carboxylate

The title compound (1 g, 1.72 mmol, 71.14% yield) was obtained as a brown solid in analogy to Example **64,** Step 3. MS (ESI, m/z): 583.0 [M+H]⁺.

### Step 3)

### tert-butyl 4-[2-chloro-4-[[3-[1-(cyanomethyl)-3-(trifluoromethyl)pyrazol-4-yl]imidazo[1,2-a]pyrazin-8-yl]amino]benzoyl]piperazine-1-carboxylate

The title compound (300 mg, 0.480 mmol, 55.5% yield) was obtained as a brown solid in analogy to Example **64,** Step 6 using Intermediate 29. MS (ESI, m/z): 630.2 [M+H]⁺.

### Step 4)

### 2-[4-[8-[3-Chloro-4-(piperazine-1-carbonyl)anilino]imidazo[1,2-a]pyrazin-3-yl]-3-(trifluoromethyl)pyrazol-1-yl]acetonitrile

To a solution of tert-butyl 4-[2-chloro-4-[[3-[1-(cyanomethyl)-3-(trifluoromethyl)pyrazol-4-yl]imidazo[1,2-a]pyrazin-8-yl]amino]benzoyl]piperazine-1-carboxylate (300 mg, 0.480 mmol, 1 eq) in DCM (5 mL) was added trifluoroacetic acid (1 mL, 12.98 mmol, 27.26 eq) and the reaction mixture was stirred at 20 °C for 2 h. The reaction mixture was concentrated to afford the title compound (177 mg, 0.330 mmol, 70% yield) as a brown solid. MS (ESI, m/z): 530.2 [M+H]⁺.

### Step 5)

### [2-[4-[2-chloro-4-[[3-[1-(cyanomethyl)-3-(trifluoromethyl)pyrazol-4-yl]imidazo[1,2-a]pyrazin-8-yl]amino]benzoyl]piperazin-1-yl]-2-oxo-ethyl]-trimethyl-ammonium formate

The title compound (38.1 mg, 0.060 mmol, 16.9% yield) was obtained as an off-white solid in analogy to Example **64,** Step 8. MS (ESI, m/z): 629.2 [M]⁺.

### Example 66

### 2-[[3-[[2-Chloro-4-[[3-(2,3-difluoro-4-methoxy-phenyl)imidazo[1,2-a]pyrazin-8-yl]amino]benzoyl]amino]cyclobutyl]amino]ethyl-trimethyl-ammonium formate

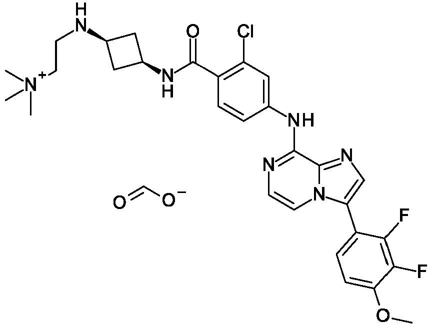

### Step 1)

### 2-[[3-(tert-Butoxycarbonylamino)cyclobutyl]amino]ethyl-trimethyl-ammonium formate

To a solution of tert-butyl N-(3-aminocyclobutyl)carbamate (500 mg, 2.68 mmol, 1 eq) and 2-bromoethyl(trimethyl)ammonium bromide (2.98 g, 12.08 mmol, 4.5 eq) in ACN (5 mL) was added sodium iodide (0.12 g, 0.810 mmol, 0.3 eq) and diisopropyl ethylamine (2.11 mL, 12.08 mmol, 4.5 eq). The reaction was stirred at 80 °C for 24 h. The reaction mixture was concentrated under reduce pressure and was purified by prep-HPLC (FA) to afford the title compound (500 mg, 1.58 mmol, 68.37% yield) as a dark green oil.

### Step 2) Intermediate 30

### 2-[(3-Aminocyclobutyl)amino]ethyl-trimethyl-ammonium chloride

To a solution of 2-[[3-(tert-butoxycarbonylamino)cyclobutyl]amino]ethyl-trimethyl-ammonium formate (500 mg, 1.84 mmol, 1 eq) was added HCl in MeOH (2.0 mL, 8 mmol, 4.36 eq) and the reaction was stirred at 20 °C for 12 h. The reaction mixture was concentrated under reduced pressure to afford the title compound (500 mg, 2.41 mmol, 95.48% yield) as a light yellow oil, which was used without further purification. MS (ESI, m/z): 172.2 [M]⁺.

### Step 3)

### 2-[[3-[[2-Chloro-4-[[3-(2,3-difluoro-4-methoxy-phenyl)imidazo[1,2-a]pyrazin-8-yl]amino]benzoyl]amino]cyclobutyl]amino]ethyl-trimethyl-ammonium formate

To a solution of 2-chloro-4-[[3-(2,3-difluoro-4-methoxy-phenyl)imidazo[1,2-a]pyrazin-8-yl]amino]benzoic acid (Intermediate 5, 100 mg, 0.230 mmol, 1 eq) and 2-[(3-aminocyclobutyl)amino]ethyl-trimethyl-ammonium chloride (57.87 mg, 0.280 mmol, 1.2 eq) in DMF (2 mL) was added N,N-diisopropylethylamine (0.12 mL, 0.700 mmol, 3 eq) and O-(7-azabenzotriazol-1-yl)-N,N,N',N'-tetramethyluronium hexafluorophosphate (132.4 mg, 0.350 mmol, 1.5 eq). The reaction was stirred at 30 °C for 16 h. After adjusted to pH=4~5 with formic acid, the mixture was purified by prep-HPLC (FA) and lyophilized to afford the title compound (17.1 mg, 0.030 mmol, 11.38% yield) as an off-white solid. MS (ESI, m/z): 584.3 [M]⁺.

### Example 67

### (S)-2-(3-(4-((3-(2,3-Difluoro-4-methoxyphenyl)imidazo[1,2-a]pyrazin-8-yl)amino)-2-methylbenzamido)pyrrolidin-1-yl)-N,N,N-trimethyl-2-oxoethan-1-aminium chloride

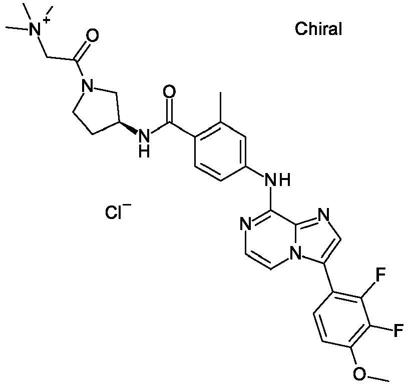

### Step 1)

### tert-Butyl (S)-3-(4-((3-(2,3-difluoro-4-methoxyphenyl)imidazo[1,2-a]pyrazin-8-yl)amino)-2-methylbenzamido)pyrrolidine-1-carboxylate

The title compound (148 mg, 256 µmol, 52.5% yield) was obtained as a light brown solid in analogy to Example **63,** Step 1 using tert-butyl (S)-3-aminopyrrolidine-1-carboxylate and Intermediate 6. MS (ESI, m/z): 579.3 [M+H]⁺.

### Step 2)

### (S)-4-((3-(2,3-Difluoro-4-methoxyphenyl)imidazo[1,2-a]pyrazin-8-yl)amino)-2-methyl-N-(pyrrolidin-3-yl)benzamide

The title compound (122 mg, 255 µmol, 85% yield) was obtained as a white solid in analogy to Example **63,** Step 2. MS (ESI, m/z): 479.3 [M+H]⁺.

### Step 3)

### (S)-2-(3-(4-((3-(2,3-difluoro-4-methoxyphenyl)imidazo[1,2-a]pyrazin-8-yl)amino)-2-methylbenzamido)pyrrolidin-1-yl)-N,N,N-trimethyl-2-oxoethan-1-aminium chloride

Under argon, (S)-4-((3-(2,3-difluoro-4-methoxyphenyl)imidazo[1,2-a]pyrazin-8-yl)amino)-2-methyl-N-(pyrrolidin-3-yl)benzamide (70 mg, 146 µmol, Eq: 1) was dissolved in DMF (1.43 ml). Betaine hydrochloride (24.7 mg, 161 µmol, Eq: 1.1) and 2-(3H-[1,2,3]triazolo[4,5-b]pyridin-3-yl)-1,1,3,3-tetramethylisouronium hexafluorophosphate(V) (61.2 mg, 161 µmol, Eq: 1.1) were added. DIPEA (37.8 mg, 51.1 µl, 293 µmol, Eq: 2) was added. The reaction mixture was stirred at RT over 1 h. The solvent was evaporated and the crude was then purified by reverse phase to afford the title compound (17 mg, 27.6 µmol, 18.9% yield) as a light brown solid. MS (ESI, m/z): 578.5 [M]⁺.

### Example 68

### 2-(3-(4-((3-(2,3-Difluoro-4-methoxyphenyl)imidazo[1,2-a]pyrazin-8-yl)amino)-2-methylbenzamido)piperidin-1-yl)-N,N,N-trimethyl-2-oxoethan-1-aminium chloride

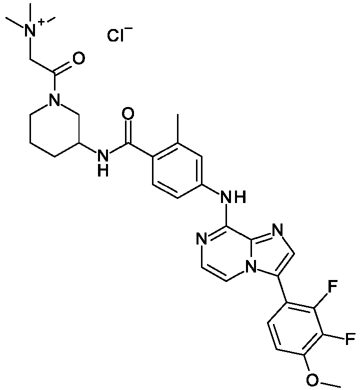

### Step 1)

### tert-Butyl 3-(4-((3-(2,3-difluoro-4-methoxyphenyl)imidazo[1,2-a]pyrazin-8-yl)amino)-2-methylbenzamido)piperidine-1-carboxylate

The title compound (323 mg, 545 µmol, 112 % yield) was obtained as a light red solid in analogy to Example **63,** Step 1 using tert-butyl 3-aminopiperidine-1-carboxylate and Intermediate 6. MS (ESI, m/z): 593.3 [M+H]⁺.

### Step 2)

### 4-((3-(2,3-Difluoro-4-methoxyphenyl)imidazo[1,2-a]pyrazin-8-yl)amino)-2-methyl-N-(piperidin-3-yl)benzamide

The title compound (144 mg, 292 µmol, 97% yield) was obtained as a light yellow solid in analogy to Example **63,** Step 2. MS (ESI, m/z): 493.3 [M+H]⁺.

### Step 3)

### 2-(3-(4-((3-(2,3-Difluoro-4-methoxyphenyl)imidazo[1,2-a]pyrazin-8-yl)amino)-2-methylbenzamido)piperidin-1-yl)-N,N,N-trimethyl-2-oxoethan-1-aminium chloride

The title compound (30 mg, 47.8 µmol, 29.4% yield) was obtained as a white solid in analogy to Example 67, Step 3. MS (ESI, m/z): 592.5 [M]⁺.

### Example 69

### 2-(((1r,4r)-4-(4-((3-(2,3-Difluoro-4-methoxyphenyl)imidazo[1,2-a]pyrazin-8-yl)amino)-2-methylbenzamido)cyclohexyl)amino)-N,N,N-trimethyl-2-oxoethan-1-aminium chloride

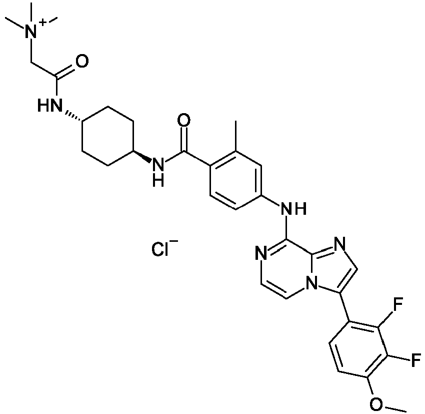

### Step 1)

### tert-Butyl ((1r,4r)-4-(4-((3-(2,3-difluoro-4-methoxyphenyl)imidazo[1,2-a]pyrazin-8-yl)amino)-2-methylbenzamido)cyclohexyl)carbamate

The title compound (145 mg, 239 µmol, 49% yield) was obtained as a light brown solid in analogy to Example **63,** Step 1 using tert-butyl ((1r,4r)-4-aminocyclohexyl)carbamate and Intermediate 6. MS (ESI, m/z): 607.4 [M+H]⁺.

### Step 2)

### N-((1r,4r)-4-aminocyclohexyl)-4-((3-(2,3-difluoro-4-methoxyphenyl)imidazo[1,2-a]pyrazin-8-yl)amino)-2-methylbenzamide

The title compound (106 mg, 209 µmol, 69% yield) was obtained as a white solid in analogy to Example **63,** Step 2. MS (ESI, m/z): 507.3 [M+H]⁺.

### Step 3)

### 2-(((1r,4r)-4-(4-((3-(2,3-difluoro-4-methoxyphenyl)imidazo[1,2-a]pyrazin-8-yl)amino)-2-methylbenzamido)cyclohexyl)amino)-N,N,N-trimethyl-2-oxoethan-1-aminium chloride

The title compound (35 mg, 54.5 µmol, 48.4% yield) was obtained as a white solid in analogy to Example **67,** Step 3. MS (ESI, m/z): 606.5 [M]⁺.

### Example 70

### (R)-2-(3-(4-((3-(2,3-difluoro-4-methoxyphenyl)imidazo[1,2-a]pyrazin-8-yl)amino)-2-methylbenzamido)pyrrolidin-1-yl)-N,N,N-trimethyl-2-oxoethan-1-aminium chloride

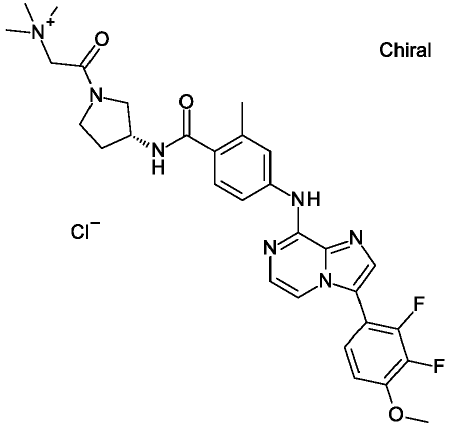

### Step 1)

### tert-Butyl (R)-3-(4-((3-(2,3-difluoro-4-methoxyphenyl)imidazo[1,2-a]pyrazin-8-yl)amino)-2-methylbenzamido)pyrrolidine-1-carboxylate

The title compound (146 mg, 252 µmol, 51.8% yield) was obtained as a light brown solid in analogy to Example **63,** Step 1 using tert-butyl (R)-3-aminopyrrolidine-1-carboxylate and Intermediate 6. MS (ESI, m/z): 579.3 [M+H]⁺.

### Step 2)

### (R)-4-((3-(2,3-difluoro-4-methoxyphenyl)imidazo[1,2-a]pyrazin-8-yl)amino)-2-methyl-N-(pyrrolidin-3-yl)benzamide

The title compound (108 mg, 226 µmol, 75% yield) was obtained as a white solid in analogy to Example **63,** Step 2. MS (ESI, m/z): 479.3 [M+H]⁺.

### Step 3)

### (R)-2-(3-(4-((3-(2,3-difluoro-4-methoxyphenyl)imidazo[1,2-a]pyrazin-8-yl)amino)-2-methylbenzamido)pyrrolidin-1-yl)-N,N,N-trimethyl-2-oxoethan-1-aminium chloride

The title compound (43 mg, 70 µmol, 55.8% yield) was obtained as a white solid in analogy to Example **67,** Step 3. MS (ESI, m/z): 578.5 [M]⁺.

### Example 71

### 2-Amino-N-(2-(3-(4-((3-(2,3-difluoro-4-methoxyphenyl)imidazo[1,2-a]pyrazin-8-yl)amino)-2-ethylbenzamido)azetidin-1-yl)-2-oxoethyl)-N,N-dimethyl-2-oxoethan-1-aminium formate

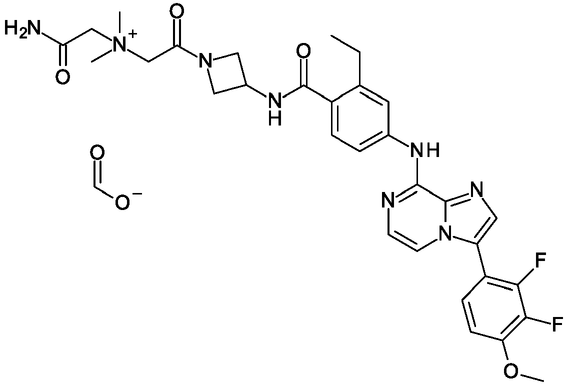

### Step 1)

### tert-Butyl 3-(4-((3-(2,3-difluoro-4-methoxyphenyl)imidazo[1,2-a]pyrazin-8-yl)amino)-2-ethylbenzamido)azetidine-1-carboxylate

Tert-butyl 3-aminoazetidine-1-carboxylate (89.3 mg, 518 µmol, Eq: 1.1), 4-((3-(2,3-difluoro-4-methoxyphenyl)imidazo[1,2-a]pyrazin-8-yl)amino)-2-ethylbenzoic acid (Intermediate 8, 200 mg, 471 µmol, Eq: 1) and DIPEA (122 mg, 165 µl, 942 µmol, Eq: 2) were combined with DMF (6 ml). HATU (269 mg, 707 µmol, Eq: 1.5) was added. The reaction was stirred at room temperature for 2 h. The reaction mixture was poured into 50 mL of H₂O and extracted with EtOAc (3 x 25 mL). The organic layers were combined, washed with saturated NaCl (25 mL) and evaporated to afford the title compound (244 mg, 422 µmol, 89.5% yield) as a white solid. MS (ESI, m/z): 579.8 [M+H]⁺.

### Step 2)

### N-(Azetidin-3-yl)-4-((3-(2,3-difluoro-4-methoxyphenyl)imidazo[1,2-a]pyrazin-8-yl)amino)-2-ethylbenzamide

tert-Butyl 3-(4-((3-(2,3-difluoro-4-methoxyphenyl)imidazo[1,2-a]pyrazin-8-yl)amino)-2-ethylbenzamido)azetidine-1-carboxylate (244 mg, 422 µmol, Eq: 1) was combined with THF (5 ml). 4M HCl (3.16 ml, 12.7 mmol, Eq: 30) in water was added. The reaction was stirred at room temperature for 10 min. The crude reaction mixture was concentrated in vacuo to afford the title compound (202 mg, 422 µmol, 100% yield) as a white solid. MS (ESI, m/z): 479.5 [M+H]⁺.

### Step 3)

### 4-((3-(2,3-Difluoro-4-methoxyphenyl)imidazo[1,2-a]pyrazin-8-yl)amino)-N-(1-(dimethylglycyl)azetidin-3-yl)-2-ethylbenzamide

N,N-dimethylglycine (22.4 mg, 217 µmol, Eq: 1.3), N-(azetidin-3-yl)-4-((3-(2,3-difluoro-4-methoxyphenyl)imidazo[1,2-a]pyrazin-8-yl)amino)-2-ethylbenzamide (80 mg, 167 µmol, Eq: 1) and TEA (84.6 mg, 117 µl, 836 µmol, Eq: 5) were combined with DMF (5 ml). 1-propanephosphonic anhydride (160 mg, 251 µmol, Eq: 1.5) was added. The reaction was stirred at room temperature overnight. The reaction mixture was poured into 50 mL of H₂O and extracted with EtOAc (3 x 25 mL). The organic layers were dried over Na₂SO₄ and concentrated in vacuo to afford the title compound (61 mg, 108 µmol, 64.7% yield) as a white solid. MS (ESI, m/z): 564.6 [M+H]⁺.

### Step 4)

### 2-Amino-N-(2-(3-(4-((3-(2,3-difluoro-4-methoxyphenyl)imidazo[1,2-a]pyrazin-8-yl)amino)-2-ethylbenzamido)azetidin-1-yl)-2-oxoethyl)-N,N-dimethyl-2-oxoethan-1-aminium formate

2-iodoacetamide (40 mg, 216 µmol, Eq: 2) and 4-((3-(2,3-difluoro-4-methoxyphenyl)imidazo[1,2-a]pyrazin-8-yl)amino)-N-(1-(dimethylglycyl)azetidin-3-yl)-2-ethylbenzamide (61 mg, 108 µmol, Eq: 1) were combined with CH₂Cl₂ (6 ml). The reaction was stirred at room temperature overnight. The crude reaction mixture was concentrated in vacuo and was purified by preparative HPLC to afford the title compound (25 mg, 36.7 µmol, 34% yield) as a white powder. MS (ESI, m/z): 621.6 [M]⁺.

### Example 72

### 2-(((1s,3s)-3-(4-((3-(2,3-Difluoro-4-methoxyphenyl)imidazo[1,2-a]pyrazin-8-yl)amino)-2-methylbenzamido)cyclobutyl)amino)-N,N,N-trimethyl-2-oxoethan-1-aminium chloride

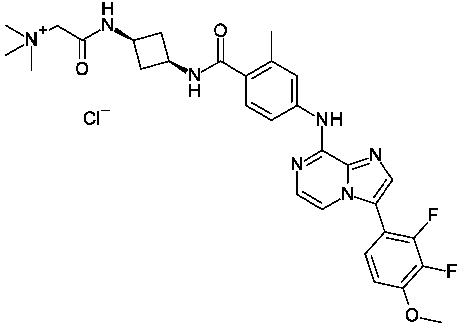

### Step 1)

### tert-Butyl ((1s,3s)-3-(4-((3-(2,3-difluoro-4-methoxyphenyl)imidazo[1,2-a]pyrazin-8-yl)amino)-2-methylbenzamido)cyclobutyl)carbamate

Under argon, 4-((3-(2,3-difluoro-4-methoxyphenyl)imidazo[1,2-a]pyrazin-8-yl)amino)-2-methylbenzoic acid hydrochloride (Intermediate 6, 400 mg, 895 µmol, Eq: 1) was suspended in DMF (4 ml). N-ethyl-N-isopropylpropan-2-amine (DIPEA) (578 mg, 761 µl, 4.48 mmol, Eq: 5) was added. tert-Butyl ((1s,3s)-3-aminocyclobutyl)carbamate (200 mg, 1.07 mmol, Eq: 1.2) and 2-(3H-[1,2,3]triazolo[4,5-b]pyridin-3-yl)-1,1,3,3-tetramethylisouronium hexafluorophosphate(V) (HATU) (681 mg, 1.79 mmol, Eq: 2) were added. The reaction mixture was stirred at RT over 2 h. Then N-ethyl-N-isopropylpropan-2-amine (DIPEA) (144 mg, 190µl, 1.12 mmol, Eq: 1.25) and tert-butyl ((1s,3s)-3-aminocyclobutyl)carbamate (50 mg, 268 µmol, Eq: 0.3) were added and the reaction was stirred at RT over 30 min. The solvent was evaporated and the crude material was purified by flash chromatography (silica gel, 20 g, 0% to 100% DCM/MeOH/NH₄OH (95/5/1)) to afford the title compound (416 mg, 719 µmol, 80.3% yield) as a light brown solid. MS (ESI, m/z): 579.4 [M+H]⁺.

### Step 2)

### N-((1s,3s)-3-aminocyclobutyl)-4-((3-(2,3-difluoro-4-methoxyphenyl)imidazo[1,2-a]pyrazin-8-yl)amino)-2-methylbenzamide

Under argon, tert-butyl ((1s,3s)-3-(4-((3-(2,3-difluoro-4-methoxyphenyl)imidazo[1,2-a]pyrazin-8-yl)amino)-2-methylbenzamido)cyclobutyl)carbamate (416 mg, 719 µmol, Eq: 1) was suspended in DCM (11 ml). TFA (2.87 g, 1.94 ml, 25.2 mmol, Eq: 35) was added and the solution was stirred at RT over 1 h. DCM/MeOH/NH₃ was added till the TFA was neutralised and the RM was evaporated. The crude was purified by flash chromatography (silica gel, 20 g, 0% to 100% DCM(DCM/MeOH/NH₄OH (90/10/1))) to afford the title compound (272 mg, 568 µmol, 79.1% yield) as a light yellow solid. MS (ESI, m/z): 479.4 [M+H]⁺.

### Step 3)

### 2-(((1s,3s)-3-(4-((3-(2,3-difluoro-4-methoxyphenyl)imidazo[1,2-a]pyrazin-8-yl)amino)-2-methylbenzamido)cyclobutyl)amino)-N,N,N-trimethyl-2-oxoethan-1-aminium chloride

Under argonr, N-((1s,3s)-3-aminocyclobutyl)-4-((3-(2,3-difluoro-4-methoxyphenyl)imidazo[1,2-a]pyrazin-8-yl)amino)-2-methylbenzamide (60 mg, 125 µmol, Eq: 1) was dissolved in DMF (1.23 ml). Betaine (21.2 mg, 138 µmol, Eq: 1.1) and 2-(3H-[1,2,3]triazolo[4,5-b]pyridin-3-yl)-1,1,3,3-tetramethylisouronium hexafluorophosphate(V) (HATU) (52.4 mg, 138 µmol, Eq: 1.1) were added. DIPEA (32.4 mg, 43.8 µl, 251 µmol, Eq: 2) was added. The reaction mixture was stirred at RT over 1 h. The solvent was evaporated and the crude was purified by preparative reverse phase HPLC under acidic conditions to afford the title compound (18 mg, 29.3 µmol, 23.4% yield) as a white solid. MS (ESI, m/z): 578.5 [M]⁺.

### Example 73

### 3-(2-chloro-4-((3-(2,3-difluoro-4-methoxyphenyl)imidazo[1,2-a]pyrazin-8-yl)amino)benzamido)-N-(2-hydroxyethyl)-N,N-dimethylpropan-1-aminium formate

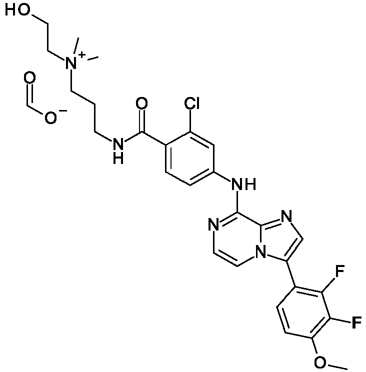

### Step 1)

### 2-Chloro-4-((3-(2,3-difluoro-4-methoxyphenyl)imidazo[1,2-a]pyrazin-8-yl)amino)-N-(3-((2-hydroxyethyl)amino)propyl)benzamide

2-Chloro-4-((3-(2,3-difluoro-4-methoxyphenyl)imidazo[1,2-a]pyrazin-8-yl)amino)benzoic acid (Intermediate 5, 75 mg, 174 µmol, Eq: 1) and HATU (79.4 mg, 209 µmol, Eq: 1.2) were dissolved in DMF (1.74 ml). DIPEA (90 mg, 122 µl, 696 µmol, Eq: 4) was added and the reaction mixture was stirred for 20 min. 2-((3-aminopropyl)amino)ethan-1-ol (24.7 mg, 24.5 µl, 209 µmol, Eq: 1.2) was added and it was stirred for 1 h. The mixture was purified by prep. HPLC and the solution was concentrated under vacuum to afford the title compound (55 mg, 103 µmol, 56.5% yield) as a white amorph solid. MS (ESI, m/z): 531.4 [M+H]⁺.

### Step 2)

### 3-(2-Chloro-4-((3-(2,3-difluoro-4-methoxyphenyl)imidazo[1,2-a]pyrazin-8-yl)amino)benzamido)-N-(2-hydroxyethyl)-N,N-dimethylpropan-1-aminium formate

2-chloro-4-((3-(2,3-difluoro-4-methoxyphenyl)imidazo[1,2-a]pyrazin-8-yl)amino)-N-(3-((2-hydroxyethyl)amino)propyl)benzamide (27 mg, 50.9 µmol, Eq: 1), MeI (18 mg, 7.95 µl, 127 µmol, Eq: 2.5) and DIPEA (9.86 mg, 13.3 µl, 76.3 µmol, Eq: 1.5) were mixed together in DMF (500 µl). The reaction mixture was stirred at room temperature for 16 h and then purified by prep. HPLC to afford the title compound (18 mg, 29.7 µmol, 55.6% yield) as an off-white amorph solid. MS (ESI, m/z): 559.4 [M]⁺.

### Example 74

### N-(Cyanomethyl)-2-(2-(4-((3-(2,3-difluoro-4-methoxyphenyl)imidazo[1,2-a]pyrazin-8-yl)amino)-2-ethylbenzamido)ethoxy)-N,N-dimethylethan-1-aminium formate

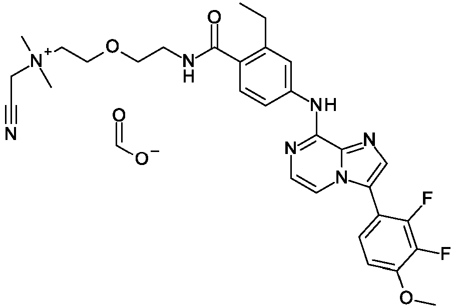

### Step 1) Intermediate 23

### 4-((3-(2,3-Difluoro-4-methoxyphenyl)imidazo[1,2-a]pyrazin-8-yl)amino)-N-(2-(2-(dimethylamino)ethoxy)ethyl)-2-ethylbenzamide

4-((3-(2,3-Difluoro-4-methoxyphenyl)imidazo[1,2-a]pyrazin-8-yl)amino)-2-ethylbenzoic acid (Intermediate 8, 400 mg, 942 µmol, Eq: 1) was combined with DMF (8 ml). DIPEA (365 mg, 494 µl, 2.83 mmol, Eq: 3) and HATU (717 mg, 1.88 mmol, Eq: 2) were added. It was stirred at RT for 15 min. Then 2-(2-aminoethoxy)-N,N-dimethylethan-1-amine (187 mg, 1.41 mmol, Eq: 1.5) was added. The reaction mixture was stirred at RT for 1.5 h. The reaction mixture was diluted with H₂O and it was extracted with DCM (2 x 100 mL). The organic layers were washed with 50 ml of brine, dried over Na₂SO₄, filtered and concentrated in vacuo. The crude material was purified by flash chromatography (silica gel, 25 g, 0% to 100%

DCM/MeOH/NH₄OH(100:10:1): DCM) to afford the title compound (395.4 mg, 734 µmol, 77.9% yield) as a light brown solid. MS (ESI, m/z): 539.3 [M+H]⁺.

### Step 2)

### N-(Cyanomethyl)-2-(2-(4-((3-(2,3-difluoro-4-methoxyphenyl)imidazo[1,2-a]pyrazin-8-yl)amino)-2-ethylbenzamido)ethoxy)-N,N-dimethylethan-1-aminium formate

4-((3-(2,3-difluoro-4-methoxyphenyl)imidazo[1,2-a]pyrazin-8-yl)amino)-N-(2-(2-(dimethylamino)ethoxy)ethyl)-2-ethylbenzamide (25 mg, 46.4 µmol, Eq: 1) and 2-bromoacetonitrile (27.8 mg, 16.2 µl, 232 µmol, Eq: 5) were mixed together in MeCN (500 µl). The reaction mixture was refluxed for 3 h. The mixture was then diluted with DCM, concentrated under vacuum and purified by prep. HPLC to afford the title compound (16 mg, 25.6 µmol, 52.5% yield) as a white amorph solid. MS (ESI, m/z): 578.4 [M]⁺.

### Example 75

### N-(2-(2-(4-((3-(2,3-Difluoro-4-methoxyphenyl)imidazo[1,2-a]pyrazin-8-yl)amino)-2-ethylbenzamido)ethoxy)ethyl)-2-ethoxy-N,N-dimethyl-2-oxoethan-1-aminium formate

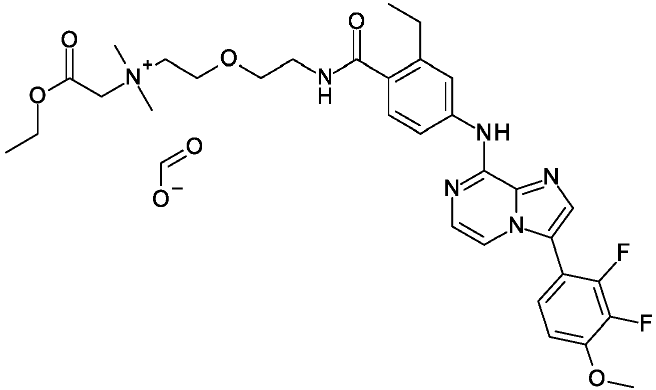

4-((3-(2,3-Difluoro-4-methoxyphenyl)imidazo[1,2-a]pyrazin-8-yl)amino)-N-(2-(2-(dimethylamino)ethoxy)ethyl)-2-ethylbenzamide (Intermediate 23, 25 mg, 46.4 µmol, Eq: 1) and ethyl 2-iodoacetate (72.4 mg, 40 µl, 338 µmol, Eq: 7.29) were mixed together in MeCN (1 ml). The reaction mixture was refluxed for 4 h. It was then concentrated and purified by prep. HPLC to afford the title compound (14 mg, 20.8 µmol, 42.7% yield) as a white amorph solid. MS (ESI, m/z): 625.5 [M]⁺.

### Example 76

### Mono(N-(2-amino-2-oxoethyl)-5-(4-((3-(2,3-difluoro-4-methoxyphenyl)imidazo[1,2-a]pyrazin-8-yl)amino)-2-ethylbenzamido)-N,N-dimethylpentan-1-aminium) monoformate monoiodide

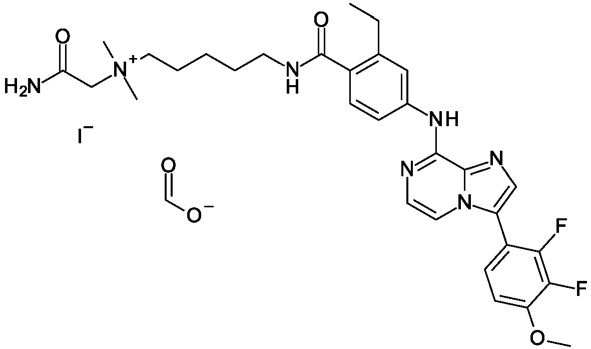

### Step 1)

### 4-((3-(2,3-difluoro-4-methoxyphenyl)imidazo[1,2-a]pyrazin-8-yl)amino)-N-(5-(dimethylamino)pentyl)-2-ethylbenzamide

4-((3-(2,3-difluoro-4-methoxyphenyl)imidazo[1,2-a]pyrazin-8-yl)amino)-2-ethylbenzoic acid (Intermediate 8, 200 mg, 471 µmol, Eq: 1) and HATU (215 mg, 565 µmol, Eq: 1.2) were dissolved in DMF (2 ml). N1,N1-dimethylpentane-1,5-diamine (73.6 mg, 85.6 µl, 565 µmol, Eq: 1.2) and DIPEA (128 mg, 173 µl, 990 µmol, Eq: 2.1) were successively added. The reaction mixture was stirred at room temperature for 30 min. Water was added and the reaction was extracted with 20 ml of DCM 3 times. The organic layers were combined, dried with MgSO₄ and concentrated under vacuum. The crude was purified by flash chromatography (25 g, EtOAc // EtOAc/MeOH/NH₃ (75/23/2) 0 to 100%). The solution was concentrated under vacuum to afford the title compound (217 mg, 404 µmol, 81.5% yield) as an off-white solid. MS (ESI, m/z): 537.4 [M+H]⁺.

### Step 2)

### Mono(N-(2-amino-2-oxoethyl)-5-(4-((3-(2,3-difluoro-4-methoxyphenyl)imidazo[1,2-a]pyrazin-8-yl)amino)-2-ethylbenzamido)-N,N-dimethylpentan-1-aminium) monoformate monoiodide

4-((3-(2,3-difluoro-4-methoxyphenyl)imidazo[1,2-a]pyrazin-8-yl)amino)-N-(5-(dimethylamino)pentyl)-2-ethylbenzamide (66 mg, 123 µmol, Eq: 1) was dissolved in DCM (1.5 ml). 2-iodoacetamide (114 mg, 49.5 µl, 615 µmol, Eq: 5) was added and the reaction mixture was stirred at room temperature for 4 h. Then the reaction was concentrated under vacuum and purified by prep. HPLC to afford the title compound (45 mg, 66.1 µmol, 51.5% yield) as a white amorph solid (mixture of formate and iodide (50/50)). MS (ESI, m/z): 594.5 [M]⁺.

### Example 77

### Trans-[2-[[4-[[4-[[3-(2,3-Difluoro-4-methoxyphenyl)imidazo[1,2-a]pyrazin-8-yl]amino]-2-ethylbenzoyl]amino]cyclohexyl]amino]-2-oxoethyl]-trimethylazanium formate

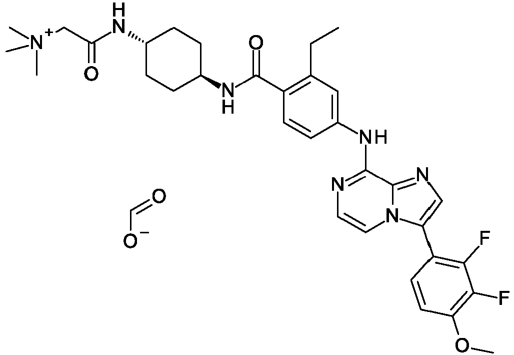

### Step 1)

### tert-Butyl ((1r,4r)-4-(4-((3-(2,3-difluoro-4-methoxyphenyl)imidazo[1,2-a]pyrazin-8-yl)amino)-2-ethylbenzamido)cyclohexyl)carbamate

Under argon, 4-((3-(2,3-difluoro-4-methoxyphenyl)imidazo[1,2-a]pyrazin-8-yl)amino)-2-ethylbenzoic acid hydrochloride (Intermediate 8, 295 mg, 640 µmol, Eq: 1) was dissolved in DMF (3.69 ml). N-ethyl-N-isopropylpropan-2-amine (414 mg, 544 µl, 3.2 mmol, Eq: 5), trans-tert-butyl ((1r,4r)-4-aminocyclohexyl)carbamate (165 mg, 768 µmol, Eq: 1.2) and 2-(3H-[1,2,3]triazolo[4,5-b]pyridin-3-yl)-1,1,3,3-tetramethylisouronium hexafluorophosphate(V) (HATU) (487 mg, 1.28 mmol, Eq: 2) were added and the yellow solution was stirred at room temperature over 1 h. The solvent was evaporated and the crude material was purified by flash chromatography (silica gel, 20 g, 0% to 100% DCM:DCM/MeOH/NH₄OH (90/10/1)) to afford the title compound (448 mg, 722 µmol, 101% yield) as a yellow solid.

### Step 2)

### N-((1r,4r)-4-aminocyclohexyl)-4-((3-(2,3-difluoro-4-methoxyphenyl)imidazo[1,2-a]pyrazin-8-yl)amino)-2-ethylbenzamide

Under argon, tert-butyl ((1r,4r)-4-(4-((3-(2,3-difluoro-4-methoxyphenyl)imidazo[1,2-a]pyrazin-8-yl)amino)-2-ethylbenzamido)cyclohexyl)carbamate (397 mg, 640 µmol, Eq: 1) was suspended in MeOH (4.3 ml). 4M HCl in dioxane (1.6 ml, 6.4 mmol, Eq: 10) was added and the reaction mixture was stirred at RT over 2 days. 4M HCl in dioxane (1.6 ml, 6.4 mmol, Eq: 10) was added again and the mixture was stirred at RT over the weekend. DCM/MeOH/NH₃ was added till the HCl was neutralised and the reaction mixture was evaporated. The crude was purified by reverse phase chromatography under basic conditions to afford the title compound (55 mg, 105 µmol, 16.5% yield) as an off-white solid. MS (ESI, m/z): 521.4 [M+H]⁺.

### Step 3)

### Trans-[2-[[4-[[4-[[3-(2,3-difluoro-4-methoxy-phenyl)imidazo[1,2-a]pyrazin-8-yl]amino]-2-ethyl-benzoyl]amino]cyclohexyl]amino]-2-oxo-ethyl]-trimethyl-ammonium formate

Under argon, betaine hydrochloride (17.9 mg, 116 µmol, Eq: 1.1) was combined in DMF (1.04 ml). 2-(3H-[1,2,3]triazolo[4,5-b]pyridin-3-yl)-1,1,3,3-tetramethylisouronium hexafluorophosphate(V) (HATU) (44.2 mg, 116 µmol, Eq: 1.1) and DIPEA (27.3 mg, 36.9 µl, 211 µmol, Eq: 2) were added. The reaction mixture was stirred at room temperature over 30 minutes. N-((1r,4r)-4-aminocyclohexyl)-4-((3-(2,3-difluoro-4-methoxyphenyl)imidazo[1,2-a]pyrazin-8-yl)amino)-2-ethylbenzamide (55 mg, 106 µmol, Eq: 1) was added and the reaction was stirred at RT over 1 h. Then betaine hydrochloride (17.9 mg, 116 µmol, Eq: 1.1), DIPEA (27.3 mg, 36.9 µl, 211 µmol, Eq: 2) and 2-(3H-[1,2,3]triazolo[4,5-b]pyridin-3-yl)-1,1,3,3-tetramethylisouronium hexafluorophosphate(V) (HATU) (44.2 mg, 116 µmol, Eq: 1.1) were added again and the reaction mixture was stirred at RT over 30 minutes. The solvent was evaporated and the crude was then purified by preparative reverse phase HPLC under acidic conditions (formic acid) to afford the title compound (27 mg, 40.5 µmol, 38% yield) as an off-white powder. MS (ESI, m/z): 620.5 [M]⁺.

### Example 78

### 4-(Cyanomethyl)-4-(2-(2-(4-((3-(1-(cyanomethyl)-3-(trifluoromethyl)-1H-pyrazol-4-yl)imidazo[1,2-a]pyrazin-8-yl)amino)-2-ethylbenzamido)ethoxy)ethyl)morpholin-4-ium formate

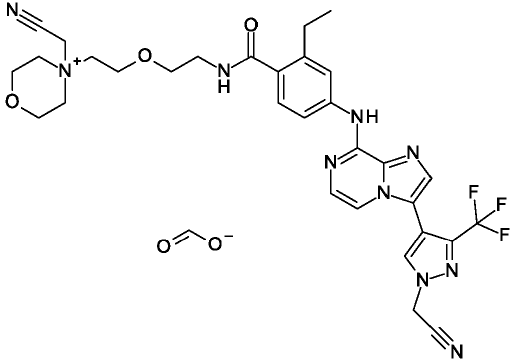

### Step 1)

### 2-Ethyl-N-(2-(2-morpholinoethoxy)ethyl)-4-((3-(3-(trifluoromethyl)-1H-pyrazol-4-yl)imidazo[1,2-a]pyrazin-8-yl)amino)benzamide

The title compound (7 mg, 12 µmol, 1.7% yield) was obtained in analogy to Example **4,** Step 5 using 2-(2-morpholinoethoxy)ethan-1-amine and Intermediate 32. MS (ESI, m/z): 573.4 [M+H]⁺.

### Step 2)

### 4-(Cyanomethyl)-4-(2-(2-(4-((3-(1-(cyanomethyl)-3-(trifluoromethyl)-1H-pyrazol-4-yl)imidazo[1,2-a]pyrazin-8-yl)amino)-2-ethylbenzamido)ethoxy)ethyl)morpholin-4-ium formate

The title compound (4.35 mg, 6 µmol, 15.6% yield) was obtained in analogy to Example 4, Step 6. MS (ESI, m/z): 651.4 [M]⁺.

### Example 79

### 5-[[4-[[3-[4-(Difluoromethoxy)-2,3-difluoro-phenyl]imidazo[1,2-a]pyrazin-8-yl]amino]-2-ethyl-benzoyl]amino]pentyl-dimethyl-(2-oxo-2-piperazin-1-yl-ethyl)ammonium bis(2,2,2-trifluoroacetate)

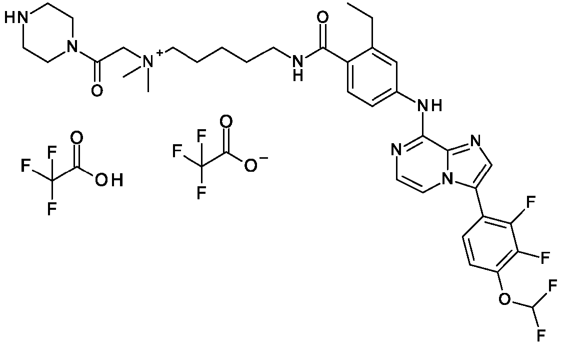

### Step 1)

### 8-chloro-3-(4-(difluoromethoxy)-2,3-difluorophenyl)imidazo[1,2-a]pyrazine

To a 5 mL microwave vial was added 8-chloro-3-iodoimidazo[1,2-a]pyrazine (Intermediate 1, 1 g, 3.58 mmol, Eq: 1), 2-(4-(difluoromethoxy)-2,3-difluorophenyl)-4,4,5,5-tetramethyl-1,3,2-dioxaborolane (Intermediate 33, 1.1 g, 3.58 mmol, Eq: 1), 1,1'-bis(di-tert-butylphosphino)ferrocene palladium dichloride (233 mg, 358 µmol, Eq: 0.1) and Na₂CO₃ (1.14 g, 10.7 mmol, Eq: 3) in dioxane (3 ml). The vial was capped and heated in the microwave at 100 °C for 30 min under N2. The crude reaction mixture was concentrated in vacuo. The reaction mixture was poured into 50 mL H2O and extracted with EtOAc (3 x 50 mL). The organic layers were combined, washed with sat NaCl (1 x 50 mL), The organic layers were dried over Na₂SO₄ and concentrated in vacuo. The crude material was purified by flash chromatography (silica gel, 40 g, 0% to 5% MeOH in DCM) to afford the title compound as ?? (1.19 g, 3.59 mmol, quantitative yield). 332.1 [M+H]⁺

### Step 2) Intermediate 36

### 4-((3-(4-(difluoromethoxy)-2,3-difluorophenyl)imidazo[1,2-a]pyrazin-8-yl)amino)-2-ethylbenzoic acid

The title compound was prepared in analogy to example 9, step 2 using 8-chloro-3-(4-(difluoromethoxy)-2,3-difluorophenyl)imidazo[1,2-a]pyrazine (1.17 g, 3.53 mmol, Eq: 1) and 4-amino-2-ethylbenzoic acid (758 mg, 4.59 mmol, Eq: 1.3). (750 mg, 1.63 mmol, 46.2 % yield). MS (ESI, m/z): 461.1 [M+H]⁺

### Step 3)

### tert-Butyl (5-(4-((3-(4-(difluoromethoxy)-2,3-difluorophenyl)imidazo[1,2-a]pyrazin-8-yl)amino)-2-ethylbenzamido)pentyl)carbamate

In a 50 mL round-bottomed flask, 4-((3-(4-(difluoromethoxy)-2,3-difluorophenyl)imidazo[1,2-a]pyrazin-8-yl)amino)-2-ethylbenzoic acid (80 mg, 174 µmol, Eq: 1), tert-butyl (5-aminopentyl)carbamate (45.7 mg, 226 µmol, Eq: 1.3), 2-(3H-[1,2,3]triazolo[4,5-b]pyridin-3-yl)-1,1,3,3-tetramethylisouronium hexafluorophosphate(V) (85.9 mg, 226 µmol, Eq: 1.3) and DIPEA (67.4 mg, 91 µl, 521 µmol, Eq: 3) were combined with DMF (3 ml) to give a light brown solution. The reaction was stirred at room temperature for 1 h. The reaction mixture was poured into 25 mL H₂O and extracted with EtOAc (3 x 25 mL).The organic layers were combined, washed with sat NaCl (1 x 25 mL), The organic layers were dried over Na₂SO₄ and concentrated in vacuo and the title compound was obtained (112 mg, 174 µmol, 100 % yield). MS (ESI, m/z): 645.3 [M+H]⁺

### Step 4)

### N-(5-Aminopentyl)-4-((3-(4-(difluoromethoxy)-2,3-difluorophenyl)imidazo[1,2-a]pyrazin-8-yl)amino)-2-ethylbenzamide hydrochloride

In a 50 mL round-bottomed flask, tert-butyl (5-(4-((3-(4-(difluoromethoxy)-2,3-difluorophenyl)imidazo[1,2-a]pyrazin-8-yl)amino)-2-ethylbenzamido)pentyl)carbamate (110 mg, 171 µmol, Eq: 1) was combined with THF (3 ml) to give a light brown solution. 12M aq HCl (1.14 ml, 13.7 mmol, Eq: 80) was added. The reaction was stirred at room temperature for 30min. The reaction mixture was concentrated in vacuo and the crude title compound was obtained (92.9 mg, 171 µmol, 100 % yield). MS (ESI, m/z): 545.5 [M+H]⁺

### Step 5) Intermediate 37

### 4-((3-(4-(Difluoromethoxy)-2,3-difluorophenyl)imidazo[1,2-a]pyrazin-8-yl)amino)-N-(5-(dimethylamino)pentyl)-2-ethylbenzamide

In a 50 mL round-bottomed flask, N-(5-aminopentyl)-4-((3-(4-(difluoromethoxy)-2,3-difluorophenyl)imidazo[1,2-a]pyrazin-8-yl)amino)-2-ethylbenzamide hydrochloride (92 mg, 169 µmol, Eq: 1), NaBH₃CN (63.7 mg, 1.01 mmol, Eq: 6) and formaldehyde (33% in methanol) (461 mg, 5.07 mmol, Eq: 30) were combined with MeOH (6 ml) to give a light brown solution. The reaction mixture was heated to 50 °C and stirred for 2 h. The reaction mixture was poured into 25 mL H₂O and extracted with EtOAc (3 x 25 mL).The organic layers were combined and washed with sat NaCl (1 x 25 mL). The organic layers were dried over Na₂SO₄ and concentrated in vacuo to afford the title compound (96.7 mg, 169 µmol, 100 % yield). MS (ESI, m/z): 573.4 [M+H]⁺, 287.4 [M+2H]²⁺

### Step 6)

### N-(2-(4-(tert-Butoxycarbonyl)piperazin-1-yl)-2-oxoethyl)-5-(4-((3-(4-(difluoromethoxy)-2,3-difluorophenyl)imidazo[1,2-a]pyrazin-8-yl)amino)-2-ethylbenzamido)-N,N-dimethylpentan-1-aminium bromide

To a 5 mL microwave vial was added 4-((3-(4-(difluoromethoxy)-2,3-difluorophenyl)imidazo[1,2-a]pyrazin-8-yl)amino)-N-(5-(dimethylamino)pentyl)-2-ethylbenzamide (50 mg, 87.3 µmol, Eq: 1), tert-butyl 4-(2-bromoacetyl)piperazine-1-carboxylate (40.2 mg, 131 µmol, Eq: 1.5) and DIPEA (33.9 mg, 45.8 µl, 262 µmol, Eq: 3) in MeCN (3 ml). The vial was capped and heated in the microwave at 60°C for 30 min. The reaction mixture was concentrated in vacuo. The crude product was directly used to the next step (69.8 mg, 87.3 µmol, quantitative yield). MS (ESI, m/z): 799.2 [M]⁺

### Step 7)

### 5-[[4-[[3-[4-(Difluoromethoxy)-2,3-difluoro-phenyl]imidazo[1,2-a]pyrazin-8-yl]amino]-2-ethyl-benzoyl]amino]pentyl-dimethyl-(2-oxo-2-piperazin-1-yl-ethyl)ammonium di-2,2,2-trifluoroacetate

In a 50 mL round-bottomed flask, N-(2-(4-(tert-butoxycarbonyl)piperazin-1-yl)-2-oxoethyl)-5-(4-((3-(4-(difluoromethoxy)-2,3-difluorophenyl)imidazo[1,2-a]pyrazin-8-yl)amino)-2-ethylbenzamido)-N,N-dimethylpentan-1-aminium bromide (69 mg, 86.3 µmol, Eq: 1) was combined with DCM (3 ml). 2,2,2-trifluoroacetic acid (1.18 g, 10.4 mmol, Eq: 120) was added. The reaction was stirred at room temperature for 30min.. The reaction mixture was concentrated in vacuo. The crude material was purified by preparative HPLC to afford the title compound (9 mg, 9.23 µmol, 10.7 % yield) as white powder. MS (ESI, m/z): 699.2 [M]⁺

### Example 80

### N-[[3-(azetidin-3-ylmethyl)-3-methyl-3-azoniabicyclo[3.1.0]hexan-6-yl]methyl]-4-[[3-[4-(difluoromethoxy)-2,3-difluoro-phenyl]imidazo[1,2-a]pyrazin-8-yl]amino]-2-ethyl-benzamide bis(2,2,2-trifluoroacetate)

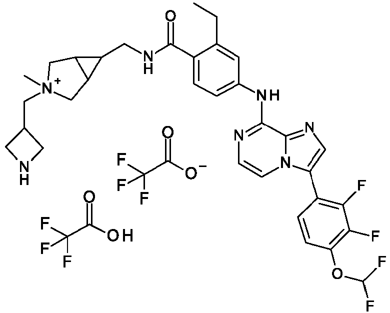

### Step 1)

### tert-Butyl 6-((4-((3-(4-(difluoromethoxy)-2,3-difluorophenyl)imidazo[1,2-a]pyrazin-8-yl)amino)-2-ethylbenzamido)methyl)-3-azabicyclo[3.1.0]hexane-3-carboxylate

In a 50 mL round-bottomed flask, 4-((3-(4-(difluoromethoxy)-2,3-difluorophenyl)imidazo[1,2-a]pyrazin-8-yl)amino)-2-ethylbenzoic acid (Intermediate 36, 50 mg, 109 µmol, Eq: 1), tert-butyl 6-(aminomethyl)-3-azabicyclo[3.1.0]hexane-3-carboxylate (30 mg, 141 µmol, Eq: 1.3), 2-(3H-[1,2,3]triazolo[4,5-b]pyridin-3-yl)-1,1,3,3-tetramethylisouronium hexafluorophosphate(V) (53.7 mg, 141 µmol, Eq: 1.3) and DIPEA (42.1 mg, 56.9 µl, 326 µmol, Eq: 3) were combined with DMF (3 ml) to give a light brown solution. The reaction was stirred at room temperature for 30min. The reaction mixture was poured into 25 mL H₂O and extracted with EtOAc (3 x 25 mL).The organic layers were combined, washed with sat NaCl (1 x 25 mL), The organic layers were dried over Na2SO4 and concentrated in vacuo to afford the title compound as ?? (71 mg, 108 µmol, 99.9 % yield) which was used without further purification. MS (ESI, m/z): 655.0 [M+H]⁺

### Step 2)

### N-((3-Azabicyclo[3.1.0]hexan-6-yl)methyl)-4-((3-(4-(difluoromethoxy)-2,3-difluorophenyl)imidazo[1,2-a]pyrazin-8-yl)amino)-2-ethylbenzamide

In a 50 mL round-bottomed flask, tert-butyl 6-((4-((3-(4-(difluoromethoxy)-2,3-difluorophenyl)imidazo[1,2-a]pyrazin-8-yl)amino)-2-ethylbenzamido)methyl)-3-azabicyclo[3.1.0]hexane-3-carboxylate (71 mg, 108 µmol, Eq: 1) was combined with THF (3 ml). 12M aq HCl (904 µl, 10.8 mmol, Eq: 100) was added. The reaction was stirred at room temperature for 30 min. The crude reaction mixture was concentrated in vacuo to afford N-((3-azabicyclo[3.1.0]hexan-6-yl)methyl)-4-((3-(4-(difluoromethoxy)-2,3-difluorophenyl)imidazo[1,2-a]pyrazin-8-yl)amino)-2-ethylbenzamide as ?? (60.1 mg, 108 µmol, 99.9 % yield), which was used without further purification. MS (ESI, m/z): 555.0 [M+H]⁺

### Step 3)

### tert-Butyl 3-((6-((4-((3-(4-(difluoromethoxy)-2,3-difluorophenyl)imidazo[1,2-a]pyrazin-8-yl)amino)-2-ethylbenzamido)methyl)-3-azabicyclo[3.1.0]hexan-3-yl)methyl)azetidine-1-carboxylate

In a 50 mL round-bottomed flask, N-((3-azabicyclo[3.1.0]hexan-6-yl)methyl)-4-((3-(4-(difluoromethoxy)-2,3-difluorophenyl)imidazo[1,2-a]pyrazin-8-yl)amino)-2-ethylbenzamide (61 mg, 110 µmol, Eq: 1), tert-butyl 3-formylazetidine-1-carboxylate (61.1 mg, 330 µmol, Eq: 3) and NaBH₃CN (34.6 mg, 550 µmol, Eq: 5) were combined with MeOH (6 ml) to give a light brown solution. The reaction mixture was heated to 45 °C and stirred for 15 h. The crude reaction mixture was concentrated in vacuo and the residue diluted with 25 mL H₂O and extracted with EtOAc (3 x 25 mL). The organic layers were combined, washed with sat NaCl (1 x 25 mL), The organic layers were dried over Na₂SO₄ and concentrated in vacuo afford the title compound (79.6 mg, 110 µmol, quantitative yield) which was used without further purification . MS (ESI, m/z): 724.3 [M+H]⁺

### Step 4)

### 3-((1-(tert-Butoxycarbonyl)azetidin-3-yl)methyl)-6-((4-((3-(4-(difluoromethoxy)-2,3-difluorophenyl)imidazo[1,2-a]pyrazin-8-yl)amino)-2-ethylbenzamido)methyl)-3-methyl-3-azabicyclo[3.1.0]hexan-3-ium iodide

In a 50 mL round-bottomed flask, tert-butyl 3-((6-((4-((3-(4-(difluoromethoxy)-2,3-difluorophenyl)imidazo[1,2-a]pyrazin-8-yl)amino)-2-ethylbenzamido)methyl)-3-azabicyclo[3.1.0]hexan-3-yl)methyl)azetidine-1-carboxylate (79.6 mg, 110 µmol, Eq: 1), MeI (78.1 mg, 34.4 µl, 550 µmol, Eq: 5) and DIPEA (71.1 mg, 96 µl, 550 µmol, Eq: 5) were combined with MeCN (6 ml). The reaction was stirred at room temperature for 15h. The reaction mixture was concentrated in vacuo to afford the title compound (81.3 mg, 110 µmol, 100 % yield) which was used without further purification. MS (ESI, m/z): 738.4 [M+H]⁺

### Step 5)

### N-[[3-(azetidin-3-ylmethyl)-3-methyl-3-azoniabicyclo[3.1.0]hexan-6-yl]methyl]-4-[[3-[4-(difluoromethoxy)-2,3-difluoro-phenyl]imidazo[1,2-a]pyrazin-8-yl]amino]-2-ethyl-benzamide bis(2,2,2-trifluoroacetate)

In a 50 mL round-bottomed flask, 3-((1-(tert-butoxycarbonyl)azetidin-3-yl)methyl)-6-((4-((3-(4-(difluoromethoxy)-2,3-difluorophenyl)imidazo[1,2-a]pyrazin-8-yl)amino)-2-ethylbenzamido)methyl)-3-methyl-3-azabicyclo[3.1.0]hexan-3-ium iodide (81.3 mg, 110 µmol, Eq: 1) was combined with DCM (3 ml) to give a light brown solution. 2,2,2-trifluoroacetic acid (1.25 g, 11 mmol, Eq: 100) was added. The reaction was stirred at room temperature for 30min. The crude reaction mixture was concentrated in vacuo purified by preparative HPLC. The title compound was obtained as white powder (19 mg, 21.5 µmol, 19.5 % yield). MS (ESI, m/z): 638.4 [M]⁺

### Example 81

### 1-(Azetidin-3-ylmethyl)-4-(4-(4-((3-(4-(difluoromethoxy)-2,3-difluorophenyl)imidazo[1,2-a]pyrazin-8-yl)amino)-2-ethylbenzoyl)piperazine-1-carbonyl)-1-methylpiperidin-1-ium 2,2,2-trifluoroacetate

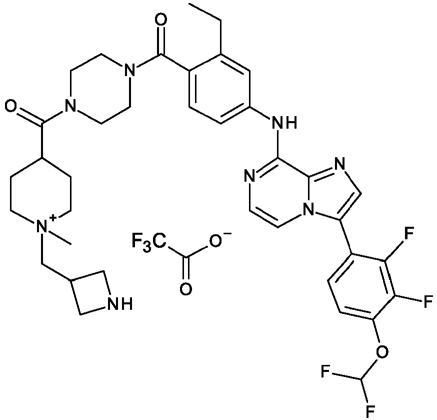

### Step 1) Intermediate 38

### tert-Butyl 4-(4-(4-((3-(4-(difluoromethoxy)-2,3-difluorophenyl)imidazo[1,2-a]pyrazin-8-yl)amino)-2-ethylbenzoyl)piperazine-1-carbonyl)piperidine-1-carboxylate

tert-Butyl 4-(4-((3-(4-(difluoromethoxy)-2,3-difluorophenyl)imidazo[1,2-a]pyrazin-8-yl)amino)-2-ethylbenzoyl)piperazine-1-carboxylate (Intermediate 35, 115 mg, 183 µmol, Eq: 1) was stirred in 3 mL 20% TFA/DCM (v/v) at rt for 1h, at which time LC-MS indicated a full conversion of the substrate. The solvent was removed in vacuo, and the residual film was dissolved in dry DMF (915 µl). To this solution was added 1-(tert-butoxycarbonyl)piperidine-4-carboxylic acid (50.3 mg, 220 µmol, Eq: 1.2), N-ethyl-N-isopropylpropan-2-amine (118 mg, 159 µl, 915 µmol, Eq: 5) and HATU (83.5 mg, 220 µmol, Eq: 1.2) successively. The solution was stirred at rt for 1h. Then the solution was poured into 100 mL water and the aqueous phase was extracted with EtOAc (80 mL*3). The organic layers were combined, washed with 80 mL brine, dried over sodium sulfate and concentrated in vacuo. The residue was purified by flash chromatography (silica gel; MeOH:DCM = 0:1 to 1:10) to afford the title compound as light yellow amorphous solid. MS (ESI, m/z): 740.3 [M+H]⁺ Rf = 0.6 (MeOH:DCM = 1:10).

### Step 2)

### tert-Butyl 3-((4-(4-(4-((3-(4-(difluoromethoxy)-2,3-difluorophenyl)imidazo[1,2-a]pyrazin-8-yl)amino)-2-ethylbenzoyl)piperazine-1-carbonyl)piperidin-1-yl)methyl)azetidine-1-carboxylate

tert-Butyl 4-(4-(4-((3-(4-(difluoromethoxy)-2,3-difluorophenyl)imidazo[1,2-a]pyrazin-8-yl)amino)-2-ethylbenzoyl)piperazine-1-carbonyl)piperidine-1-carboxylate (198 mg, 268 µmol, Eq: 1) was dissolved in 2 mL dioxane. To a stirred 4M HCl dioxane solution was added the above solution dropwise. The stirring was continued for 1h after addition. The solvent was removed in vacuo, and the residue was azeotroped with toluene (5 mL) to remove residual HCl. The remaining solid and tert-butyl 3-formylazetidine-1-carboxylate (59.5 mg, 321 µmol, Eq: 1.2) were dissolved in Ethanol (122 µl) and CH2Cl2 (1.22 ml). To this stirred solution was added NaBH(OAc)₃ (68.1 mg, 321 µmol, Eq: 1.2) in one portion. The stirrig was continued for 1h. Then silica gel was added, and the solvent was removed in vacuo. The residue was purified by flash chromatography (silica gel; MeOH:DCM = 0:1 to 1:10). to afford the title compound as light yellow solid (76 mg, 35% yield. MS (ESI, m/z): 405.4 [M+2H]²⁺ Rf = 0.6(MeOH:DCM = 1:10)

### Step 3) 1-(Azetidin-3-ylmethyl)-4-(4-(4-((3-(4-(difluoromethoxy)-2,3-difluorophenyl)imidazo[1,2-a]pyrazin-8-yl)amino)-2-ethylbenzoyl)piperazine-1-carbonyl)-1-methylpiperidin-1-ium

### 2,2,2-trifluoroacetate

To a solution of tert-butyl 3-((4-(4-(4-((3-(4-(difluoromethoxy)-2,3-difluorophenyl)imidazo[1,2-a]pyrazin-8-yl)amino)-2-ethylbenzoyl)piperazine-1-carbonyl)piperidin-1-yl)methyl)azetidine-1-carboxylate (75 mg, 92.7 µmol, Eq: 1) in dry MeCN (464 µl) was added iodomethane (65.8 mg, 28.9 µl, 464 µmol, Eq: 5). The yellow solution was stirred for 18h. The solvent was removed in vacuo, and the residue was dissolved in 3 mL 20% TFA/DCM solution (v/v), and the resulting solution was stirred for 1h at rt. The solvent was removed in vacuo, the residue was dissolved in MeOH and purified by preparative HPLC. The title compound was obtained as white amorphous solid (18 mg, 95% yield). MS (ESI, m/z): 723.2 [M]⁺

### Example 82

### N-[3-[[4-[[3-[4-(Difluoromethoxy)-2,3-difluoro-phenyl]imidazo[1,2-a]pyrazin-8-yl]amino]-2-ethyl-benzoyl]amino]propyl]-1-methyl-1-(pyrrolidin-3-ylmethyl)piperidin-1-ium-4-carboxamide bis (2,2,2-trifluoroacetate)

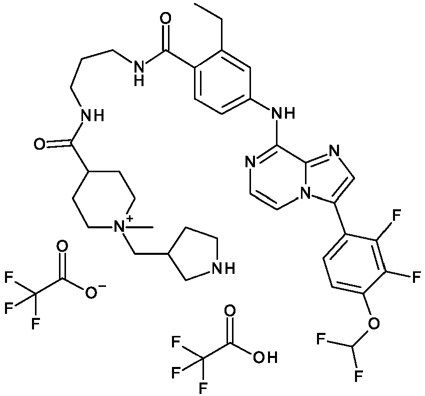

### Step 1)

### tert-Butyl N-[3-[[2-ethyl-4-[(3-iodoimidazo[1,2-a]pyrazin-8-yl)amino]benzoyl]amino]propyl]carbamate

2-Ethyl-4-((3-iodoimidazo[1,2-a]pyrazin-8-yl)amino)benzoic acid (Intermediate 34, 500 mg, 1.22 mmol, Eq: 1), tert-butyl (3-aminopropyl)carbamate (256 mg, 1.47 mmol, Eq: 1.2) and HATU (559 mg, 1.47 mmol, Eq: 1.2) was suspended in DMF (3.06 ml). N-ethyl-N-isopropylpropan-2-amine (475 mg, 640 µl, 3.67 mmol, Eq: 3) was injected in one time. The stirring was continued for 1h, and then 50 mL water was added. The aqueous phase was extracted by EtOAc (50 mL X 3). The organic layers were washed with brine (50 mL), dried over sodium sulfate and concentrated in vacuo. The residue was purified by flash chromatography (silica gel; EtOAc:PE = 0:1 to 1:1). The title compound was obtained as white solid (580 mg, 83.9% yield). MS (ESI, m/z): 565.1 [M+H]⁺.

### Step 2)

### tert-butyl N-[3-[[4-[[3-[4-(difluoromethoxy)-2,3-difluoro-phenyl]imidazo[1,2-a]pyrazin-8-yl]amino]-2-ethyl-benzoyl]amino]propyl]carbamate

*tert*-butyl (3-(2-ethyl-4-((3-iodoimidazo[1,2-a]pyrazin-8-yl)amino)benzamido)propyl)carbamate (295 mg, 523 µmol, Eq: 1), 2-(4-(difluoromethoxy)-2,3-difluorophenyl)-4,4,5,5-tetramethyl-1,3,2-dioxaborolane (Intermediate 33, 160 mg, 523 µmol, Eq: 1), Pd-118 (17 mg, 26.1 µmol, Eq: 0.05) were placed in a microwave vial. Dioxane (2.61 ml) and potassium carbonate (523 µl, 1.57 mmol, Eq: 3) were added. The vial was sealed immediatly and degassed for 5 times. The mixture was heated at 80 °C under nitrogen atmosphere for 18 h, LC-MS indicated a full conversion of

the substrate. The mixture was cooled to rt, poured into 100 mL water and extracted with EtOAc (80 mL X 3). The organic layers were combined, washed with 80 mL brine, dried over sodium sulfate, and concentrated in vacuo. The residue was purified by flash chromatography (silica gel; EtOAc:PE = 0; 1 to 1:0). The title compound was obtained as yellow solid (256 mg, 79.4% yield). R*_{f}* = 0.3 (EtOAc:PE = 1:1). MS (ESI, m/z): 617.2 [M+H]⁺.

### Step 3)

### tert-Butyl 4-((3-(4-((3-(4-(difluoromethoxy)-2,3-difluorophenyl)imidazo[1,2-a]pyrazin-8-yl)amino)-2-ethylbenzamido)propyl)carbamoyl)piperidine-1-carboxylate

*tert*-Butyl (3-(4-((3-(4-(Difluoromethoxy)-2,3-difluorophenyl)imidazo[1,2-a]pyrazin-8-yl)amino)-2-ethylbenzamido)propyl)carbamate (115 mg, 187 µmol, Eq: 1) was stirred in 3 mL 20% TFA/DCM (v/v) at rt for 1h. The solvent was removed in vacuo, and the residual film was dissolved in dry DMF (933 µl). To this solution was added 1-(*tert*-butoxycarbonyl)piperidine-4-carboxylic acid (51.3 mg, 224 µmol, Eq: 1.2), N-ethyl-N-isopropylpropan-2-amine (121 mg, 162 µl, 933 µmol, Eq: 5) and HATU (85.1 mg, 224 µmol, Eq: 1.2) successively. The solution was stirred at rt for 1h. Then the solution was poured into 100 mL water and the aqueous phase was extracted with EtOAc (80 mL X 3). The organic layers were combined, washed with 80 mL brine, dried over sodium sulfate and concentrated in vacuo to afford the title compound as light yellow oil (130mg, 96% yield) and was used in the next step without purification. The title compound was obtained as light yellow oil (130 mg, 95.8% yield). MS (ESI, m/z): 728.3 [M+H]⁺.

### Step 4)

### tert-Butyl 3-((4-((3-(4-((3-(4-(difluoromethoxy)-2,3-difluorophenyl)imidazo[1,2-a]pyrazin-8-yl)amino)-2-ethylbenzamido)propyl)carbamoyl)piperidin-1-yl)methyl)pyrrolidine-1-carboxylate

*tert*-Butyl 4-((3-(4-((3-(4-(difluoromethoxy)-2,3-difluorophenyl)imidazo[1,2-a]pyrazin-8-yl)amino)-2-ethylbenzamido)propyl)carbamoyl)piperidine-1-carboxylate (126 mg, 173 µmol, Eq: 1) was dissolved in 3 mL 20% TFA/DCM (v/v), and stirred at rt for 0.5h. The solvent was removed in vacuo, and the residue was suspended in 100 mL 1N NaOH, extracted with DCM/MeOH (v/v = 8:1) (80 mL X 3). The organic layers were combined, washed with 80 mL brine, dried over sodium sulfate and concentrated in vacuo. The residue and *tert-*butyl 3-formylpyrrolidine-1-carboxylate (103 mg, 519 µmol, Eq: 3) were dissolved in dry THF (866 µl). To this solution was added NaBH(OAc)₃ (55 mg, 260 µmol, Eq: 1.5) in one portion. The mixture was stirred at rt for 30 min. Silica gel was added, and the solvent was removed in vacuo to get the silica gel supported sample. The sample was purified by flash chromatography (MeOH:DCM = 0:1 to 1:10) to afford the title compound as light yellow amorphous solid (130 mg, 93% yield). MS (ESI, m/z): 406.3 [M+2H]²⁺.

### Step 5)

### N-[3-[[4-[[3-[4-(difluoromethoxy)-2,3-difluoro-phenyl]imidazo[1,2-a]pyrazin-8-yl]amino]-2-ethyl-benzoyl]amino]propyl]-1-methyl-1-(pyrrolidin-3-ylmethyl)piperidin-1-ium-4-carboxamide bis(2,2,2-trifluoroacetate)

To a solution of *tert*-butyl 3-((4-((3-(4-((3-(4-(difluoromethoxy)-2,3-difluorophenyl)imidazo[1,2-a]pyrazin-8-yl)amino)-2-ethylbenzamido)propyl)carbamoyl)piperidin-1-yl)methyl)pyrrolidine-1-carboxylate (130 mg, 160 µmol, Eq: 1) in dry MeCN (802 µl) was added iodomethane (114 mg, 49.9 µl, 802 µmol, Eq: 5) and 4 drops of DIPEA. The yellow solution was stirred overnight for 18h. The solvent was removed in vacuo, and the residue was dissolved in 3 mL 20% TFA/DCM solution (v/v), and the resulting solution was stirred for 1h at rt. The solvent was removed in vacuo, and the residue was dissolved in MeCN and purified by preparative HPLC to afford the title compound as white amorphous freeze-dried solid (44 mg, 28% yield). MS (ESI, m/z): 725.5 [M]⁺.

### Example 83

### [4-[1-(2-aminoethyl)-1-methyl-piperidin-1-ium-4-carbonyl[piperazin-1-yl]-[4-[[3-[4-(difluoromethoxy)-2,3-difluoro-phenyl]imidazo[1,2-a]pyrazin-8-yl]amino]-2-ethyl-phenyl]methanone bis(2,2,2-trifluoroacetate)

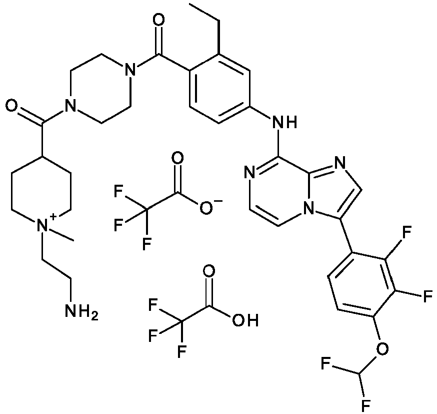

### Step 1)

### tert-Butyl (2-(4-(4-(4-((3-(4-(difluoromethoxy)-2,3-difluorophenyl)imidazo[1,2-a]pyrazin-8-yl)amino)-2-ethylbenzoyl)piperazine-1-carbonyl)piperidin-1-yl)ethyl)carbamate

tert-Butyl 4-(4-(4-((3-(4-(difluoromethoxy)-2,3-difluorophenyl)imidazo[1,2-a]pyrazin-8-yl)amino)-2-ethylbenzoyl)piperazine-1-carbonyl)piperidine-1-carboxylate (Intermediate 38, 150 mg, 203 µmol, Eq: 1) was dissolved in 3 mL 20% TFA/DCM (v/v), and stirred at rt for 0.5h. The solvent was removed in vacuo, and the residue was suspended in 100 mL 1N NaOH, extracted with DCM/MeOH (v/v = 8:1) (80 mL*3). The organic layers were combined, washed with 80 mL brine, dried over sodium sulfate and concentrated in vacuo. The above residue and tert-butyl (2-oxoethyl)carbamate (64.6 mg, 406 µmol, Eq: 2) were dissolved in dry THF (2.03 ml). To this solution was added NaBH(OAc)₃ (64.5 mg, 304 µmol, Eq: 1.5) in one portion. The mixture was stirred at rt for 30 min, and LC-MS indicated a complete conversion. Silica gel was added, and the solvent was removed in vacuo to get the silica gel suported sample. The sample was purified by flash chromatography (MeOH:DCM = 0:1 to 1:10) to affor the title compound as light brown oil (105 mg, 66% yield). MS (ESI, m/z): 783.3 [M+H]⁺

### Step 2)

### [4-[1-(2-aminoethyl)-1-methyl-piperidin-1-ium-4-carbonyl]piperazin-1-yl]-[4-[[3-[4-(difluoromethoxy)-2,3-difluoro-phenyl]imidazo[1,2-a]pyrazin-8-yl]amino]-2-ethylphenyl]methanone bis(2,2,2-trifluoroacetate)

To a solution of tert-butyl (2-(4-(4-(4-((3-(4-(difluoromethoxy)-2,3-difluorophenyl)imidazo[1,2-a]pyrazin-8-yl)amino)-2-ethylbenzoyl)piperazine-1-carbonyl)piperidin-1-yl)ethyl)carbamate (105 mg, 134 µmol, Eq: 1) in dry MeCN (671 µl) was added iodomethane (95.2 mg, 41.7 µl, 671 µmol, Eq: 5) and 4 drops of DIPEA. The yellow solution was stirred overnight for 18h. The solvent was removed in vacuo, and the residue was dissolved in 3 mL 20% TFA/DCM solution (v/v), and the resulting solution was stirred for 1h at rt. The solvent was removed in vacuo, and the residue was dissolved in MeCN purified by preparative HPLC to afford the title compound as off white amorphous freeze dried solid and a puritiy of 91% (32 mg, 23% yield). MS (ESI, m/z): 697.3 [M]⁺

### Example 84

### [4-[[3-[4-(difluoromethoxy)-2,3-difluoro-phenyl]imidazo[1,2-a]pyrazin-8-yl]amino]-2-ethyl-phenyl]-[4-[1-methyl-1-(pyrrolidin-3-ylmethyl)piperidin-1-ium-4-carbonyl]piperazin-1-yl]methanone bis(2,2,2-trifluoroacetate)

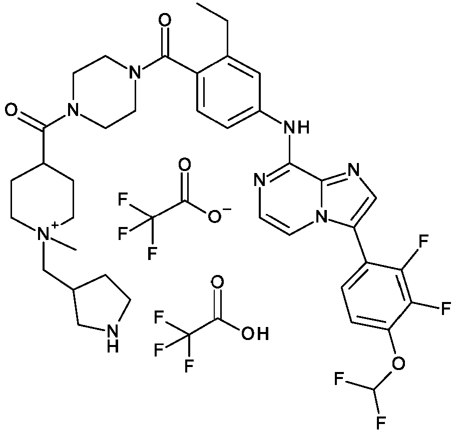

The title compound was prepared in analogy to example 83 using tert-butyl 3-formylpyrrolidine-1-carboxylate in step 1 and was obtained as white amorphous freeze dried solid. MS (ESI, m/z): 737.2 [M]⁺

### Example 85

### [4-[[3-[4-(difluoromethoxy)-2,3-difluoro-phenyl]imidazo[1,2-a]pyrazin-8-yl]amino]-2-ethyl-phenyl]-[4-[1-methyl-1-[2-(methylamino)ethyl]piperidin-1-ium-4-carbonyl]piperazin-1-yl]methanone bis(2,2,2-trifluoroacetate)

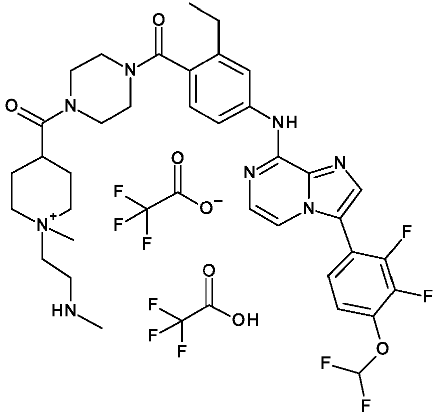

The title compound was prepared in analogy to example 83 using tert-butyl methyl(2-oxoethyl)carbamate in step 1 and was obtained as white amorphous freeze dried solid. MS (ESI, m/z): 711.6 [M]⁺

### Example 86

### [4-[1-[(3-amino-1-bicyclo[1.1.1]pentanyl)methyl]-1-methyl-piperidin-1-ium-4-carbonyl]piperazin-1-yl]-[4-[[3-[4-(difluoromethoxy)-2,3-difluoro-phenyl]imidazo[1,2-a]pyrazin-8-yl]amino]-2-ethyl-phenyl]methanone bis(2,2,2-trifluoroacetate)

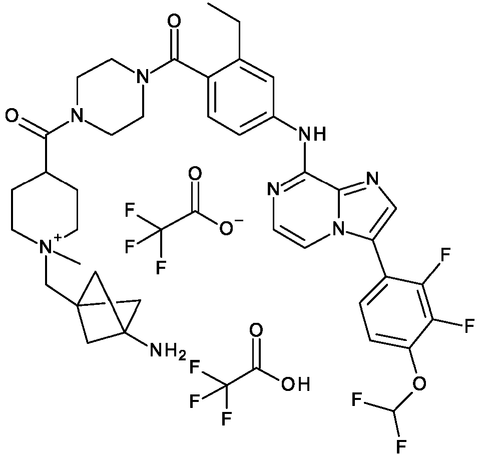

The title compound was prepared in analogy to example 83 using tert-butyl (3-formylbicyclo[1.1.1]pentan-1-yl)carbamate in step 1 and was obtained as off white amorphous freeze dried solid. MS (ESI, m/z): 749.6 [M]⁺

### Example 87

### N-[[1-(azetidin-3-ylmethyl)-1-methyl-piperidin-1-ium-4-yl]methyl]-4-[[3-[4-(difluoromethoxy)-2,3-difluoro-phenyllimidazo[1,2-a]pyrazin-8-yl]amino]-2-ethyl-benzamide diformate

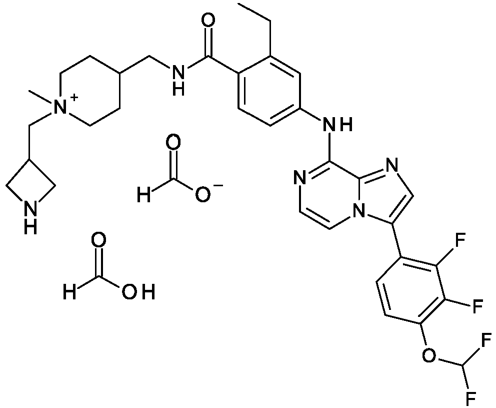

This compound was prepared in analogy to example 10 using tert-butyl 4-(aminomethyl)piperidine-1-carboxylate in step 1. The title compound was obtained as white powder. MS (ESI, m/z): 640.4 [M]⁺

### Example 88

### N-[[1-(azetidin-3-ylmethyl)-1-methyl-pyrrolidin-1-ium-3-yl]methyl]-4-[[3-[4-(difluoromethoxy)-2,3-difluoro-phenyl]imidazo[1,2-a]pyrazin-8-yl]amino]-2-ethyl-benzamide bis(2,2,2-trifluoroacetate)

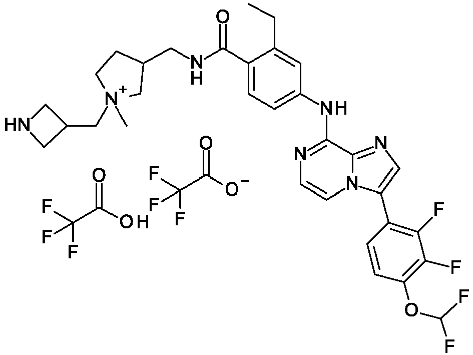

This compound was prepared in analogy to example 10 using tert-butyl 3-(aminomethyl)pyrrolidine-1-carboxylate in step 1. The title compound was obtained as white powder. MS (ESI, m/z): 626.2 [M]⁺

### Example 89

### Bis(azetidin-3-ylmethyl)-[5-[[4-[[3-[4-(difluoromethoxy)-2,3-difluoro-phenyl]imidazo[1,2-a]pyrazin-8-yl]amino]-2-ethyl-benzoyl]amino]pentyl]-methyl-ammonium bis(2,2,2-trifluoroacetate)

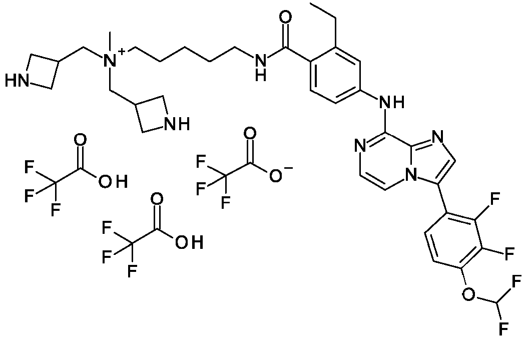

This compound was prepared in analogy to example 10 using tert-butyl (5-aminopentyl)carbamate in step 1 and using 4 eq aldehyde and 6 eq sodium cyanoborohydride in step 3 (double reductive amination). The title compound was obtained as white powder. MS (ESI, m/z): 697.1 [M]⁺

### Example 90

### N-[2-[1-(azetidin-3-ylmethyl)-1-methyl-azetidin-1-ium-3-yl]ethyl]-4-[[3-[4-(difluoromethoxy)-2,3-difluoro-phenyl]imidazo[1,2-a]pyrazin-8-yl]amino]-2-ethyl-benzamide diformate

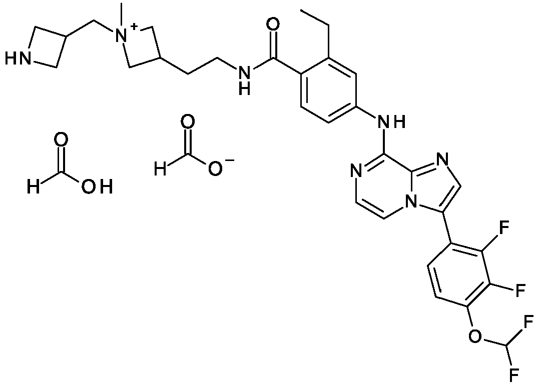

This compound was prepared in analogy to example 10 using tert-butyl 3-(2-aminoethyl)azetidine-1-carboxylate in step 1. The title compound was obtained as white powder. MS (ESI, m/z): 626.3 [M]⁺

### Example 91

### N-[2-[1-(azetidin-3-ylmethyl)-1-methyl-piperidin-1-ium-4-yl]ethyl]-4-[[3-[4-(difluoromethoxy)-2,3-difluoro-phenyl]imidazo[1,2-a]pyrazin-8-yl]amino]-2-ethyl-benzamide diformate

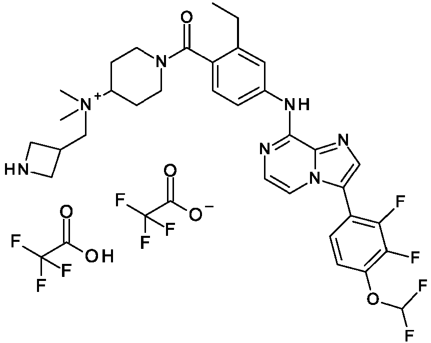

This compound was prepared in analogy to example 10 using tert-butyl N-(4-piperidyl)carbamate in step 1, double alkylation in step 4. The title compound was obtained as white powder. MS (ESI, m/z): 640.4 [M]⁺

### Example 92

### N-[3-[4-(Azetidin-3-ylmethyl)-4-methyl-piperazin-4-ium-1-yl]-3-oxo-propyl]-4-[[3-[4-(difluoromethoxy)-2,3-difluoro-phenyl]imidazo[1,2-a]pyrazin-8-yl]amino]-2-ethyl-benzamide bis(2,2,2-trifluoroacetate)

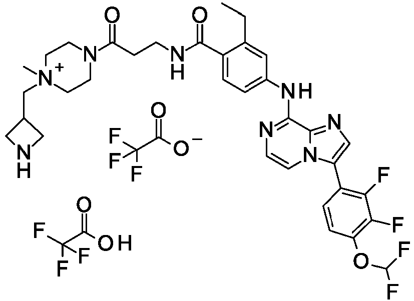

### Step 1)

### tert-Butyl 3-[[2-ethyl-4-[(3-iodoimidazo[1,2-a]pyrazin-8-yl)amino]benzoyl]amino]propanoate

In a 100 mL round-bottomed flask, 2-ethyl-4-((3-iodoimidazo[1,2-a]pyrazin-8-yl)amino)benzoic acid (100 mg, 245 µmol), tert-butyl 3-aminopropanoate (42.7 mg, 294 µmol.2) and DIPEA (95 mg, 128 µl, 735 µmol) were combined with DMF (4 mL) to give a light brown solution. HATU (112 mg, 294 µmol) was added. The reaction was stirred at room temperature for 1h. The reaction mixture was poured into 25 mL H₂O and extracted with EtOAc (3 x 25 mL). The organic layers were combined, washed with sat NaCl (1 x 25 mL). The organic layers were dried over Na₂SO₄ and concentrated in vacuum to afford the title compound (128 mg, 239 µmol, 97.6 % yield). MS (ESI, m/z): 536 [M+H]⁺.

### Step 2)

### 3-[[2-Ethyl-4-[(3-iodoimidazo[1,2-a]pyrazin-8-yl)amino]benzoyl]amino]propanoic acid

In a 100 mL round-bottomed flask, tert-butyl 3-(2-ethyl-4-((3-iodoimidazo[1,2-a]pyrazin-8-yl)amino)benzamido)propanoate (128 mg, 239 µmol) was combined with THF (3 mL) to give a light brown solution. HCl(in water) (19 mL, 16.7 mmol, Eq: 70) was added. The reaction was stirred at room temperature for 30min. The crude reaction mixture was concentrated in vacuum amd the crude title compound directly used in the next step (115 mg, 240 µmol, 100 % yield). MS (ESI, m/z): 480 [M+H]⁺.

### Step 3)

### tert-butyl 4-[3-[[2-ethyl-4-[(3-iodoimidazo[1,2-a]pyrazin-8-yl)amino[benzoyl]amino] propanoyl]piperazine-1-carboxylate

In a 100 mL round-bottomed flask, tert-butyl piperazine-1-carboxylate (62.6 mg, 336 µmol.4), 3-(2-ethyl-4-((3-iodoimidazo[1,2-a]pyrazin-8-yl)amino)benzamido)propanoic acid (115 mg, 240 µmol) and DIPEA (93 mg, 126 µl, 720 µmol) were combined with DMF (4 mL) to give a light yellow solution. HATU (128 mg, 336 µmol.4) was added. The reaction was stirred at room temperature for 1h. The reaction mixture was poured into 50 mL H₂O and extracted with EtOAc (3 x 25 mL). The organic layers were combined, washed with sat NaCl (1 x 25 mL). The organic layers were dried over Na₂SO₄ and concentrated in vacuum to afford the title compound (113 mg, 175 µmol, 72.7 % yield). MS (ESI, m/z): 648.1 [M+H]⁺.

### Step 4)

### tert-Butyl 4-[3-[[4-[[3-[4-(difluoromethoxy)-2,3-difluoro-phenyl]imidazo[1,2-a]pyrazin-8-yl]amino]-2-ethyl-benzoyl]amino]propanoyl]piperazine-1-carboxylate

To a 5 mL microwave vial was added *tert-butyl* 4-(3-(2-ethyl-4-((3-iodoimidazo[1,2-a]pyrazin-8-yl)amino)benzamido)propanoyl)piperazine-1-carboxylate (113 mg, 175 µmol), 2-(4-(difluoromethoxy)-2,3-difluorophenyl)-4,4,5,5-tetramethyl-1,3,2-dioxaborolane (Intermediate 33, 64.1 mg, 209 µmol.2), 1,1'-bis(di-tert-butylphosphino)ferrocene palladium dichloride (11.4 mg, 17.5 µmol, Eq: 0.1) and Na₂CO₃ (55.5 mg, 524 µmol) in dioxane (3 mL)/water (0.3 mL). The vial was capped and heated in the microwave at 100°C for 30 min under N₂. The crude reaction mixture was concentrated in vacuum. The crude material was purified by flash chromatography (silica gel, 12 g, 0% to 10% MeOH in DCM) to afford the title compound (95 mg, 136 µmol, 77.8 % yield). MS (ESI, m/z): 700.2 [M+H]⁺.

### Step 5)

### 4-[[3-[4-(Difluoromethoxy)-2,3-difluoro-phenyl]imidazo[1,2-a]pyrazin-8-yl]amino]-2-ethyl-N-(3-oxo-3-piperazin-1-yl-propyl)benzamide

In a 50 mL round-bottomed flask, tert-butyl 4-(3-(4-((3-(4-(difluoromethoxy)-2,3-difluorophenyl)imidazo[1,2-a]pyrazin-8-yl)amino)-2-ethylbenzamido)propanoyl)piperazine-1-carboxylate (95 mg, 136 µmol) was combined with THF (3 mL) to give a light brown solution. HCl(in water) (1.13 mL, 13.6 mmo) was added. The reaction was stirred at room temperature for 30min. The crude reaction mixture was concentrated in vacuum. The crude title compound was directly used to the next step (81.4 mg, 136 µmol, 100 % yield). MS (ESI, m/z): 600.2 [M+H]⁺.

### Step 6)

### tert-Butyl 3-[[4-[3-[[4-[[3-[4-(difluoromethoxy)-2,3-difluoro-phenyl]imidazo[1,2-a]pyrazin-8-yl]amino]-2-ethyl-benzoyl]amino]propanoyl]piperazin-1-yl]methyl]azetidine-1-carboxylate

In a 100 mL round-bottomed flask, 4-((3-(4-(difluoromethoxy)-2,3-difluorophenyl)imidazo[1,2-a]pyrazin-8-yl)amino)-2-ethyl-N-(3-oxo-3-(piperazin-1-yl)propyl)benzamide (60 mg, 100 µmol), *tert-butyl* 3-formylazetidine-1-carboxylate (37.1 mg, 200 µmol) and NaBH₃CN (37.7 mg, 600 µmol, Eq: 6) were combined with MeOH (6 mL) to give a light yellow solution. The reaction mixture was heated to 40 °C for 15 h. The crude reaction mixture was concentrated in vacuum. The reaction mixture was poured into 25 mL sat NaHCO₃ and extracted with EtOAc (3 x 25 mL). The organic layers were combined, washed with sat NaCl (1 x 25 mL), The organic layers were dried over Na₂SO₄ and concentrated in vacuum to afford the title compound (76.9 mg, 100 µmol, 100 % yield). MS (ESI, m/z): 769 [M+H]⁺.

### Step 7)

### tert-Butyl 3-[[4-[3-[[4-[[3-[4-(difluoromethoxy)-2,3-difluoro-phenyl]imidazo[1,2-a]pyrazin-8-yl]amino]-2-ethyl-benzoyl]amino]propanoyl]-1-methyl-piperazin-1-ium-1-yl]methyl]azetidine-1-carboxylate iodide

In a 100 mL round-bottomed flask, tert-butyl 3-((4-(3-(4-((3-(4-(difluoromethoxy)-2,3-difluorophenyl)imidazo[1,2-a]pyrazin-8-yl)amino)-2-ethylbenzamido)propanoyl)piperazin-1-yl)methyl)azetidine-1-carboxylate (76.9 mg, 100 µmol), MeI (142 mg, 62.5 µl, 1 mmol) and DIPEA (129 mg, 175 µl, 1 mmol) were combined with MeCN (4 mL) to give a light yellow solution. The reaction mixture was heated to 40 °C for 2 h. The crude reaction mixture was concentrated in vacuum. The crude title compound was directly used in the next step (78.4 mg, 100 µmol, 100 % yield). MS (ESI, m/z): 783.4 [M]⁺.

### Step 8)

### N-[3-[4-(Azetidin-3-ylmethyl)-4-methyl-piperazin-4-ium-1-yl]-3-oxo-propyl]-4-[[3-[4-(difluoromethoxy)-2,3-difluoro-phenyl]imidazo[1,2-a]pyrazin-8-yl]amino]-2-ethyl-benzamide bis(2,2,2-trifluoroacetate)

In a 50 mL round-bottomed flask, 1-((1-(tert-butoxycarbonyl)azetidin-3-yl)methyl)-4-(3-(4-((3-(4-(difluoromethoxy)-2,3-difluorophenyl)imidazo[1,2-a]pyrazin-8-yl)amino)-2-ethylbenzamido)propanoyl)-1-methylpiperazin-1-ium (78 mg, 99.5 µmol) was combined with DCM (4 mL) to give a light yellow solution. 2,2,2-trifluoroacetic acid (1.13 g, 9.95 mmol) was added. The reaction was stirred at room temperature for 30min. The crude reaction mixture was concentrated in vacuum. The crude material was purified by preparative HPLC to afford the title compound (33 mg, 34.4 µmol, 34.6 % yield). MS (ESI, m/z): 683.1 [M]⁺.

### Example 93

### Methyl 4-[1-(azetidin-3-ylmethyl)-1-methyl-piperidin-1-ium-4-carbonyl]-1-[4-[[3-[4-(difluoromethoxy)-2,3-difluoro-phenyl]imidazo[1,2-a]pyrazin-8-yl]amino]-2-ethyl-benzoyl]piperazine-2-carboxylate bis(2,2,2-trifluoroacetate)

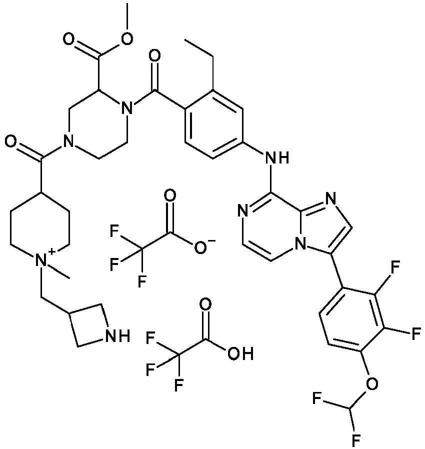

### Step 1)

### 1-(tert-Butyl) 3-methyl 4-(2-ethyl-4-((3-iodoimidazo[1,2-a]pyrazin-8-yl)amino)benzoyl)piperazine-1,3-dicarboxylate

1-(tert-butyl) 3-methyl piperazine-1,3-dicarboxylate (215 mg, 882 µmol, Eq: 1.2), 2-ethyl-4-((3-iodoimidazo[1,2-a]pyrazin-8-yl)amino)benzoic acid (Intermediate 31, 300 mg, 735 µmol, Eq: 1) and HATU (335 mg, 882 µmol, Eq: 1.2) was suspended in DMA (3.67 ml). N-ethyl-N-isopropylpropan-2-amine (475 mg, 640 µl, 3.67 mmol, Eq: 5) was injected in one time. The stirring was continued for 1h, then 50 mL water was added. The precipitate was collected by filtration and washed with water (20 mL*3). The cake was dried in vacuo to afford the crude product which was purified by flash chromatography (silica gel; MeOH:DCM = 0:1 to 1:10) to afford the title compound (230 mg, 49% yield). MS (ESI, m/z): 634.8 [M+H]⁺

### Step 2)

### 1-(tert-Butyl) 3-methyl 4-(4-((3-(4-(difluoromethoxy)-2,3-difluorophenyl)imidazo[1,2-a]pyrazin-8-yl)amino)-2-ethylbenzoyl)piperazine-1,3-dicarboxylate

1-(tert-butyl) 3-methyl 4-(2-ethyl-4-((3-iodoimidazo[1,2-a]pyrazin-8-yl)amino)benzoyl)piperazine-1,3-dicarboxylate (230 mg, 363 µmol, Eq: 1), 2-(4-(difluoromethoxy)-2,3-difluorophenyl)-4,4,5,5-tetramethyl-1,3,2-dioxaborolane (Intermediate 33, 111 mg, 363 µmol, Eq: 1), Pd-118 (11.8 mg, 18.1 µmol, Eq: 0.05) were placed in a microwave vial. Dioxane (1.81 ml) and potassium carbonate (363 µl, 1.09 mmol, Eq: 3) were added. The vial was sealed immediatly and evacuated and backfilled with argon 5 times. The mixture was heated at 80 °C under nitrogen atmosphere for 18h. The mixture was cooled to rt, poured into 100 mL water and extracted with EtOAc (80 mL*3). The organic layers were combined, washed with 80 mL brine, dried over sodium sulfate, and concentrated in vacuo. The residue was purified by flash chromatography (silica gel; EtOAc:PE = 0;1 to 1:0). To afford the title compound as light brown oil (120 mg, 48% yield). Rf = 0.3 (EtOAc:PE = 1: 1). MS (ESI, m/z): 687.1 [M+H]⁺

### Step 3)

### Methyl 4-(1-tert-butoxycarbonylpiperidine-4-carbonyl)-1-[4-[[3-[4-(difluoromethoxy)-2,3-difluoro-phenyl]imidazo[1,2-a]pyrazin-8-yl]amino]-2-ethyl-benzoyl]piperazine-2-carboxylate

1-(tert-Butyl) 3-methyl 4-(4-((3-(4-(difluoromethoxy)-2,3-difluorophenyl)imidazo[1,2-a]pyrazin-8-yl)amino)-2-ethylbenzoyl)piperazine-1,3-dicarboxylate (156 mg, 227 µmol, Eq: 1) was stirred in 3 mL 20% TFA/DCM (v/v) at rt for 1h. The solvent was removed in vacuo, and the residue was dissolved in dry DMA (1.14 ml). To this solution was added 1-(tert-butoxycarbonyl)piperidine-4-carboxylic acid (62.5 mg, 273 µmol, Eq: 1.2), N-ethyl-N-isopropylpropan-2-amine (147 mg, 198 µl, 1.14 mmol, Eq: 5) and HATU (104 mg, 273 µmol, Eq: 1.2) successively. The solution was stirred at rt for 1h. After completion, the solution was poured into 100 mL water and the aqueous phase was extracted with EtOAc (80 mL*3). The organic layers were combined, washed with 80 mL brine, dried over sodium sulfate and concentrated in vacuo to afford the title compound as yellow oil (188 mg, quantitative yield) which was used in the next step without further purification. MS (ESI, m/z): 798.5 [M+H]⁺

### Step 4)

### methyl 4-[1-(azetidin-3-ylmethyl)-1-methyl-piperidin-1-ium-4-carbonyl]-1-[4-[[3-[4-(difluoromethoxy)-2,3-difluoro-phenyl]imidazo[1,2-a]pyrazin-8-yl]amino]-2-ethyl-benzoyl]piperazine-2-carboxylate bis(2,2,2-trifluoroacetate)

The title compound was prepared in analogy to example 81, steps 2-3 and was obtained as white amorphous freeze dried solid (28 mg). MS (ESI, m/z): 781.0 [M]⁺

### Example 94

### methyl 1-[1-(azetidin-3-ylmethyl)-1-methyl-piperidin-1-ium-4-carbonyl]-4-[4-[[3-[4-(difluoromethoxy)-2,3-difluoro-phenyl]imidazo[1,2-a]pyrazin-8-yl]amino]-2-ethyl-benzoyl]piperazine-2-carboxylate tris(2,2,2-trifluoroacetate)

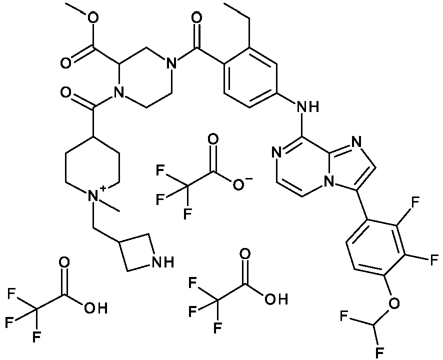

The title compound was prepared in analogy to example 93 using 1-(tert-butyl) 2-methyl piperazine-1,2-dicarboxylate in step 1. The compound was obtained as white amorphous freeze dried solid (21 mg). MS (ESI, m/z): 781.3 [M]⁻, 391.3 [M+H]²⁺

### Example 95

### [4-[[3-[4-(difluoromethoxy)-2,3-difluoro-phenyl]imidazo[1,2-a]pyrazin-8-yl]amino]-2-ethyl-phenyl]-[2-[1-methyl-1-(pyrrolidin-3-ylmethyl)piperidin-1-ium-4-carbonyl]-2,6-diazaspiro[3.3]heptan-6-yl]methanone bis(2,2,2-trifluoroacetate)

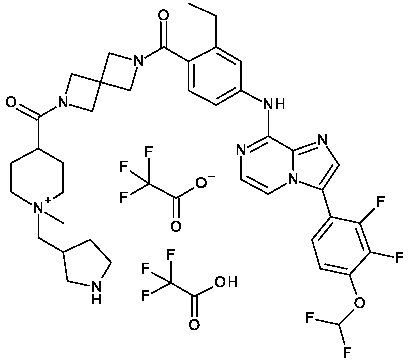

The title compound was prepared in analogy to example 93 using tert-butyl 2,6-diazaspiro[3.3]heptane-2-carboxylate in step 1 and tert-butyl 3-formylpyrrolidine-1-carboxylate in step 4 and was obtained as white amorphous freeze dried solid. MS (ESI, m/z): 749.3 [M]⁺

### Example 96

### Azetidin-3-ylmethyl-[5-[[4-[[3-[4-(difluoromethoxy)-2,3-difluoro-phenyl]imidazo[1,2-a]pyrazin-8-yl]amino]-2-ethyl-benzoyl]amino]pentyl]-dimethyl-ammonium bis(2,2,2-trifluoroacetate)

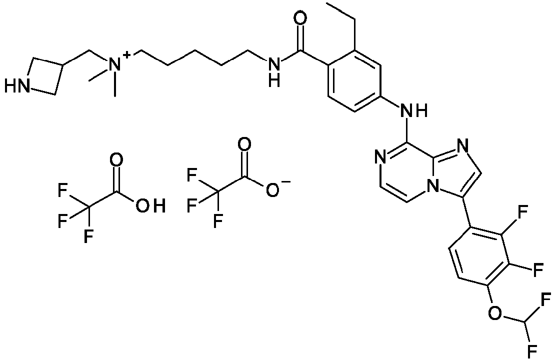

The title compound was prepared in analogy to example 79 using tert-butyl 3-(iodomethyl)azetidine-1-carboxylate in step 6 and was obtained as white powder. MS (ESI, m/z): 642.4 [M]⁺

### Example 97

### [4-[[3-[4-(Difluoromethoxy)-2,3-difluoro-phenyl]imidazo[1,2-a]pyrazin-8-yl]amino]-2-ethyl-phenyl]-[4-[4-methyl-4-(pyrrolidin-3-ylmethyl)piperazin-4-ium-1-carbonyl]-1-piperidyl]methanone 2,2,2-trifluoroacetate

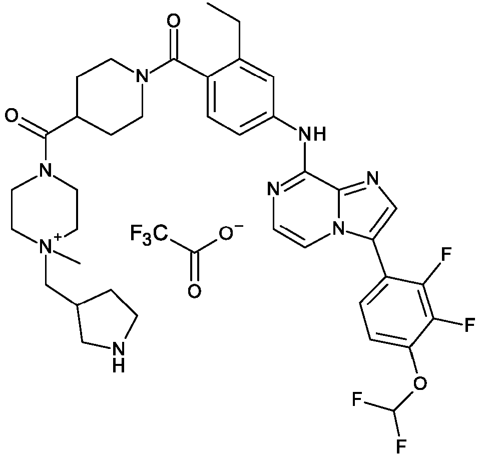

### Step 1) Intermediate 39

### Methyl 1-[2-ethyl-4-[(3-iodoimidazo[1,2-a]pyrazin-8-yl)amino]benzoyl]piperidine-4-carboxylate

2-Ethyl-4-((3-iodoimidazo[1,2-a]pyrazin-8-yl)amino)benzoic acid (Intermediate 34, 500 mg, 1.22 mmol, Eq: 1), methyl piperidine-4-carboxylate (210 mg, 1.47 mmol, Eq: 1.2) and HATU (559 mg, 1.47 mmol, Eq: 1.2) were suspended in DMF (3.06 ml). N-ethyl-N-isopropylpropan-2-amine (475 mg, 640 µl, 3.67 mmol, Eq: 3) was injected in one time. The stirring was continued for 1h, and then 50 mL water was added. The aqueous phase was extracted by EtOAc (50 mL X 3). The organic layers were washed with brine (50 mL), dried over sodium sulfate and concentrated in vacuo. The residue was purified by flash chromatography (silica gel; EtOAc:PE = 0:1 to 1:1) to afford the title compound as yellow oil (650 mg, 99.5% yield). MS (ESI, m/z): 534.0 [M+H]⁺

### Step 2)

### Methyl 1-[4-[[3-[4-(difluoromethoxy)-2,3-difluoro-phenyl]imidazo[1,2-a]pyrazin-8-yl]amino]-2-ethyl-benzoyl]piperidine-4-carboxylate

Methyl 1-(2-ethyl-4-((3-iodoimidazo[1,2-a]pyrazin-8-yl)amino)benzoyl)piperidine-4-carboxylate (279 mg, 523 µmol, Eq: 1), 2-(4-(difluoromethoxy)-2,3-difluorophenyl)-4,4,5,5-tetramethyl-1,3,2-dioxaborolane (Intermediate 33, 160 mg, 523 µmol, Eq: 1), Pd-118 (17 mg, 26.1 µmol, Eq: 0.05) were placed in a microwave vial. Dioxane (2.61 ml) and potassium carbonate (523 µl, 1.57 mmol, Eq: 3) were added. The vial was sealed immediatly and degassed for 5 times. The mixture was heated at 80 °C under nitrogen atmosphere for 18 h. The mixture was cooled to rt, poured into 100 mL water and extracted with EtOAc (80 mL X 3). The organic layers were combined, washed with 80 mL brine, dried over sodium sulfate, and concentrated in vacuo. The residue was purified by flash chromatography (silica gel; EtOAc:PE = 0;1 to 1:0) to afford the title compound as light yellow oil (207 mg, 67.6% yield). R*_{f}*= 0.3 (EtOAc:PE = 1:1). MS (ESI, m/z): 586.1 [M+H]⁺

### Step 3)

### tert-Butyl 4-(1-(4-((3-(4-(difluoromethoxy)-2,3-difluorophenyl)imidazo[1,2-a]pyrazin-8-yl)amino)-2-ethylbenzoyl)piperidine-4-carbonyl)piperazine-1-carboxylate

Methyl 1-(4-((3-(4-(difluoromethoxy)-2,3-difluorophenyl)imidazo[1,2-a]pyrazin-8-yl)amino)-2-ethylbenzoyl)piperidine-4-carboxylate (115 mg, 196 µmol, Eq: 1) was dissolved in 3 mL MeOH. To this solution was added 100 mg LiOH and 1 mL water. The resulting mixture was heated at 65°C for 1h. The mixture was cooled to rt, poured into 100 mL water and acidified to pH = 5-6 with 3N HCl. The aqueous phase was extracted with DCM (80 mL*3). The organic layers were combined, washed with 80 mL brine, dried over sodium sulfate and concentrated in vacuo. The residue was dissolved in dry DMF (982 µl). To this solution was added 1-(*tert-*butoxycarbonyl)piperidine-4-carboxylic acid (54 mg, 236 µmol, Eq: 1.2), N-ethyl-N-isopropylpropan-2-amine (127 mg, 171 µl, 982 µmol, Eq: 5) and HATU (89.6 mg, 236 µmol, Eq: 1.2) successively. The solution was stirred at rt for 1h. Then the solution was poured into 100 mL water and the aqueous phase was extracted with EtOAc (80 mL X 3). The organic layers were combined, washed with 80 mL brine, dried over sodium sulfate and concentrated in vacuo to afford the title compound as light yellow oil (145 mg, quantitative yield), which was used without further purification. MS (ESI, m/z): 740.2 [M+H]⁺

### Step 4)

### tert-Butyl 3-((4-(1-(4-((3-(4-(difluoromethoxy)-2,3-difluorophenyl)imidazo[1,2-a]pyrazin-8-yl)amino)-2-ethylbenzoyl)piperidine-4-carbonyl)piperazin-1-yl)methyl)pyrrolidine-1-carboxylate

The title compound was prepared in analogy to example 83 step 1 from tert-butyl 4-(1-(4-((3-(4-(difluoromethoxy)-2,3-difluorophenyl)imidazo[1,2-a]pyrazin-8-yl)amino)-2-ethylbenzoyl)piperidine-4-carbonyl)piperazine-1-carboxylate (210 mg, 284 µmol, Eq: 1) using *tert-butyl* 3-formylpyrrolidine-1-carboxylate and was obtained as light yellow amorphous solid (196 mg, 84% yield). MS (ESI, m/z): 384.2 [M-*^{t}*Bu+2H]²⁺

### Step 5)

**[4-[[3-[4-(difluoromethoxy)-2,3-difluoro-phenyl]imidazo[1,2-a]pyrazin-8-yl]amino]-2-ethylphenyl]-[4-[4-methyl-4-(pyrrolidin-3-ylmethyl)piperazin-4-ium-1-carbonyl]-1-piperidyl]methanone 2,2,2-trifluoroacetate**

The title compound was prepared in analogy to example 10 steps 4 and 5 from *tert*-butyl 3-((4-(1-(4-((3-(4-(difluoromethoxy)-2,3-difluorophenyl)imidazo[1,2-a]pyrazin-8-yl)amino)-2-ethylbenzoyl)piperidine-4-carbonyl)piperazin-1-yl)methyl)pyrrolidine-1-carboxylate (196 mg, 238 µmol, Eq: 1) and was obtained as while amorphous freeze dried solid (68 mg, 33% yield). MS (ESI, m/z): 737.2 [M]⁺

### Example 98

### N-[[1-(Azetidin-3-ylmethyl)-1-methyl-pyrrolidin-1-ium-3-yl]methyl]-1-[4-[[3-[4-(difluoromethoxy)-2,3-difluoro-phenyl]imidazo[1,2-a]pyrazin-8-yl]amino]-2-ethyl-benzoyl]piperidine-4-carboxamide formate / 2,2,2-trifluoroacetate (mixed salt)

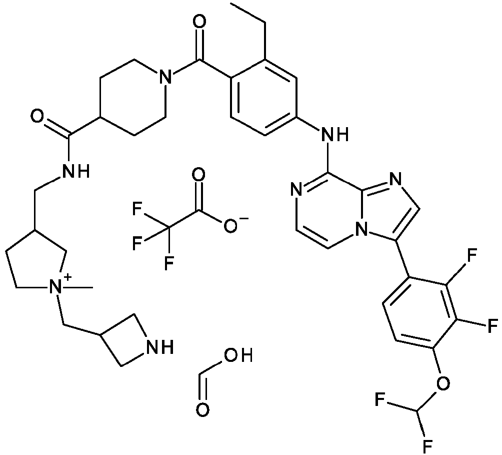

### Step 1)

### 1-[4-[[3-[4-(difluoromethoxy)-2,3-difluoro-phenyl]imidazo[1,2-a]pyrazin-8-yl]amino]-2-ethyl-benzoyl]piperidine-4-carboxylic acid

Methyl 1-(4-((3-(4-(difluoromethoxy)-2,3-difluorophenyl)imidazo[1,2-a]pyrazin-8-yl)amino)-2-ethylbenzoyl)piperidine-4-carboxylate (Intermediate 39, 1.23 g, 2.1 mmol, Eq: 1) was dissolved in MeOH (10.5 ml). To this solution was added lithium hydroxide (503 mg, 21 mmol, Eq: 10) and 1 mL water. The resulting mixture was heated at 65 °C for 1h. The mixture was cooled to rt, poured into 100 mL water and acidified to pH = 5-6 with 3N HCl. The aqueous phase was extracted with DCM (80 mL X 3). The organic layers were combined, washed with 80 mL brine, dried over sodium sulfate and concentrated in vacuo to afford the title compound as off white powder (1.07 g, 89.1% yield). MS (ESI, m/z): 572.4 [M+H]⁺

### Step 2)

### 1-[4-[[3-[4-(Difluoromethoxy)-2,3-difluoro-phenyl]imidazo[1,2-a]pyrazin-8-yl]amino]-2-ethyl-benzoyl]-N-(pyrrolidin-3-ylmethyl)piperidine-4-carboxamide formate 2,2,2-trifluoroacetate (mixed salt)

1-(4-((3-(4-(difluoromethoxy)-2,3-difluorophenyl)imidazo[1,2-a]pyrazin-8-yl)amino)-2-ethylbenzoyl)piperidine-4-carboxylic acid (150 mg, 262 µmol, Eq: 1) was stirred in 3 mL 20% TFA/DCM (v/v) at rt for 1h. The solvent was removed in vacuo, and the residual film was dissolved in dry DMF (1.31 ml). To this solution was added *tert-*butyl 3-(aminomethyl)pyrrolidine-1-carboxylate (63.1 mg, 315 µmol, Eq: 1.2), N-ethyl-N-isopropylpropan-2-amine (170 mg, 229 µl, 1.31 mmol, Eq: 5) and HATU (120 mg, 315 µmol, Eq: 1.2) successively. The solution was stirred at rt for 3h. After completion, the solution was poured into 100 mL water and the aqueous phase was extracted with EtOAc (80 mL X 3). The organic layers were combined, washed with 80 mL brine, dried over sodium sulfate and concentrated in vacuo. The residue was dissolved in 5 mL 20% TFA/DCM, the solution was stirred at rt for 1h. The solvent was removed in vacuo and the residue was purified by preparative HPLC. The title compound was obtained as white amorphous freeze dried solid (32.0 mg, 14.8% yield). MS (ESI, m/z): 653.9 [M+H]⁺

### Step 3)

### tert-Butyl 3-[[3-[[[1-[4-[[3-[4-(difluoromethoxy)-2,3-difluoro-phenyl]imidazo[1,2-a]pyrazin-8-yl]amino]-2-ethyl-benzoyl]piperidine-4-carbonyl]amino]methyl]pyrrolidin-1-yl]methyl]azetidine-1-carboxylate

1-(4-((3-(4-(difluoromethoxy)-2,3-difluorophenyl)imidazo[1,2-a]pyrazin-8-yl)amino)-2-ethylbenzoyl)-N-(pyrrolidin-3-ylmethyl)piperidine-4-carboxamide (100 mg, 153 µmol, Eq: 1) was suspended in 100 mL 1N NaOH, extracted with DCM/MeOH (v/v = 8:1) (80 mL X 3). The organic layers were combined, washed with 80 mL brine, dried over sodium sulfate and concentrated in vacuo. The above residue and *tert-butyl* 3-formylazetidine-1-carboxylate (34 mg, 184 µmol, Eq: 1.2) were dissolved in dry THF (1.53 ml). To this solution was added NaBH(OAc)3 (48.6 mg, 229 µmol, Eq: 1.5) in one portion. The mixture was stirred at rt for 30 min. Silica gel was added, and the solvent was removed in vacuo to get the silica gel supported sample. The sample was purified by flash chromatography (MeOH:DCM = 0:1 to 1:10) to get off-white oil (35 mg, 27.8% yield). MS (ESI, m/z): 723.5 [M+H]⁺

### Step 4)

### N-[[1-(Azetidin-3-ylmethyl)-1-methyl-pyrrolidin-1-ium-3-yl]methyl]-1-[4-[[3-[4-(difluoromethoxy)-2,3-difluoro-phenyl]imidazo[1,2-a]pyrazin-8-yl]amino]-2-ethyl-benzoyl]piperidine-4-carboxamide formate / 2,2,2-trifluoroacetate (mixed salt)

To a solution of tert-butyl (3-((4-(4-(4-((3-(4-(difluoromethoxy)-2,3-difluorophenyl)imidazo[1,2-a]pyrazin-8-yl)amino)-2-ethylbenzoyl)piperazine-1-carbonyl)piperidin-1-yl)methyl)bicyclo[1.1.1]pentan-1-yl)carbamate (120 mg, 144 µmol, Eq: 1) in dry MeCN (719 µl) was added iodomethane (102 mg, 44.7 µl, 719 µmol, Eq: 5) and 4 drops of DIPEA. The yellow solution was stirred for 18h. The solvent was removed in vacuo, and the residue was dissolved in 3 mL 20% TFA/DCM solution (v/v), and the resulting solution was stirred for 1h at rt. The solvent was removed in vacuo, and the residue was dissolved in MeCN, and purified by preparative HPLC. The title compound was obtained as white amorphous freeze dried solid (6.8 mg, 17% yield). MS (ESI, m/z): 737.0 [M]⁺

### Example 99

### [4-[2-(azetidin-3-ylmethyl)-2-methyl-6-aza-2-azoniaspiro[3.3]-6-carbonyl]-1-piperidyl]-[4-[[3-[4-(difluoromethoxy)-2,3-difluoro-phenyl]imidazo[1,2-a]pyrazin-8-yl]amino]-2-ethylphenyl]methanone formic acid 2,2,2-trifluoroacetate (mixed salt)

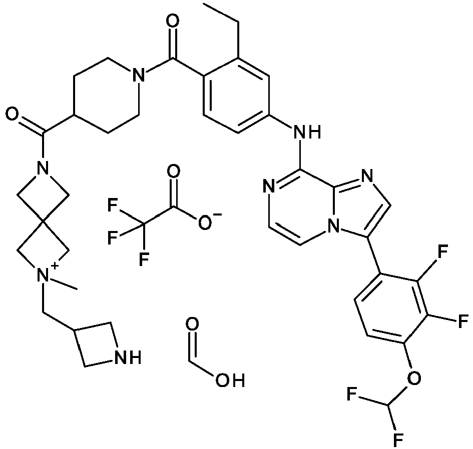

The title compound was prepared in analogy to example 98 steps 3 and 4 by using *tert-*butyl 3-(aminomethyl)pyrrolidine-1-carboxylate in step 2 and was obtained as white amorphous freeze dried solid (5.6 mg, 9.42% yield). MS (ESI, m/z): 753.1 [M+H₂O]⁺

### Example 100

### [4-[[3-[4-(Difluoromethoxy)-2,3-difluoro-phenyl]imidazo[1,2-a]pyrazin-8-yl]amino]-2-ethylphenyl]-[4-[rac-(1S,4S)-5-(azetidin-3-ylmethyl)-5-methyl-2-aza-5-azoniabicyclo[2.2.1]heptane-2-carbonyl]-1-piperidyl]methanone;formic acid;2,2,2-trifluoroacetate (mixed salt)

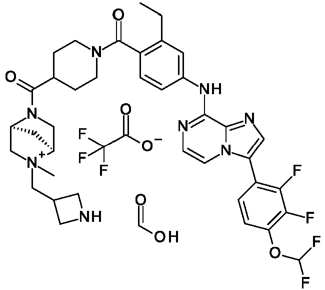

The title compound was prepared in analogy to example 98 steps 3 and 4 by using tert-butyl (1S,4S)-2,5-diazabicyclo[2.2.1]heptane-2-carboxylate in step 2 and was obtained as white amorphous freeze dried solid (7.4 mg, 10.3% yield). MS (ESI, m/z): 735.0 [M]⁺

### Example 101

### [4-[6-(Azetidin-3-ylmethyl)-6-methyl-3-aza-6-azoniabicyclo[3.1.1]heptane-3-carbonyl]-1-piperidyl]-[4-[[3-[4-(difluoromethoxy)-2,3-difluoro-phenyl]imidazo[1,2-a]pyrazin-8-yl]amino]-2-ethyl-phenyl]methanone formate 2,2,2-trifluoroacetate (mixed salt)

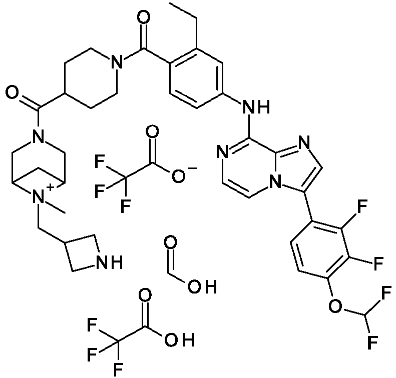

The title compound was prepared in analogy to example 98 steps 3 and 4 by using tert-butyl (1S,4S)-2,5-diazabicyclo[2.2.1]heptane-2-carboxylate in step 2 and was obtained as white amorphous freeze dried solid (5.6 mg, 6.8% yield). MS (ESI, m/z): 753.0 [M+H₂O]⁺

### Example 102

### N-[2-[[4-[[3-[4-(Difluoromethoxy)-2,3-difluoro-phenyl]imidazo[1,2-a]pyrazin-8-yl]amino]-2-ethyl-benzoyl]amino]ethyl]-1-methyl-1-(pyrrolidin-3-ylmethyl)piperidin-1-ium-4-carboxamide bis(2,2,2-trifluoroacetate)

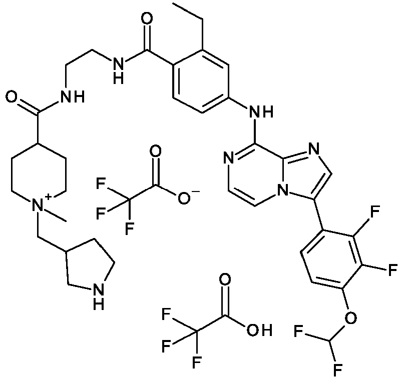

The title compound was prepared in analogy to example 82 steps 1-5 by using *tert-*butyl (3-aminopropyl)carbamate in step 1 and was obtained as white amorphous freeze dried solid (64 mg, 52.2% yield). MS (ESI, m/z): 711.2 [M]⁺

### Example 103

### [4-[[3-[4-(Difluoromethoxy)-2,3-difluoro-phenyl]imidazo[1,2-a]pyrazin-8-yl]amino]-2-fluorophenyl]-[4-[1-methyl-1-(pyrrolidin-3-ylmethyl)piperidin-1-ium-4-carbonyl]piperazin-1-yljmethanone 2,2,2-trifluoroacetate

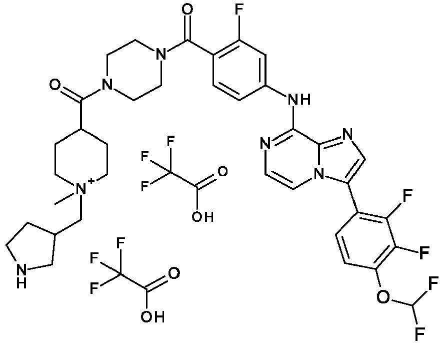

### Step 1)

### tert-butyl 4-(2-fluoro-4-((3-iodoimidazo[1,2-a]pyrazin-8-yl)amino)benzoyl)piperazine-1-carboxylate

The title compound was prepared in analogy to example 9, step 2 and 3, using 4-amino-2-fluorobenzoic acid and was obtained as brown solid (520 mg). MS (ESI, m/z): 567.0 [M+H]⁺

### Step 2)

### tert-butyl 4-(4-(2-fluoro-4-((3-iodoimidazo[1,2-a]pyrazin-8-yl)amino)benzoyl)piperazine-1-carbonyl)piperidine-1-carboxylate

The title compound was prepared in analogy to example 81 step 1 and was obtained as brown solid (570 mg). MS (ESI, m/z): 678.0 [M+H]⁺

### Step 3)

### tert-butyl 4-(4-(4-((3-(4-(difluoromethoxy)-2,3-difluorophenyl)imidazo[1,2-a]pyrazin-8-yl)amino)-2-fluorobenzoyl)piperazine-1-carbonyl)piperidine-1-carboxylate

The title compound was prepared in analogy to Example 92, step 4 and was obtained as brown oil (200 mg). MS (ESI, m/z): 730.0 [M+H]⁺

### Step 4)

### (4-(4-((3-(4-(difluoromethoxy)-2,3-difluorophenyl)imidazo[1,2-a]pyrazin-8-yl)amino)-2-fluorobenzoyl)piperazin-1-yl)(piperidin-4-yl)methanone

A mixture of tert-butyl 4-(4-(4-((3-(4-(difluoromethoxy)-2,3-difluorophenyl)imidazo[1,2-a]pyrazin-8-yl)amino)-2-fluorobenzoyl)piperazine-1-carbonyl)piperidine-1-carboxylate (200 mg, 274 µmol, Eq: 1) in DCM (6 mL) and TFA (2 mL) was stirred at r.t for 1h. Then the mixture was concentrated and the residue was basified by NH3.H2O to pH 8-9. The water layer was extracted with DCM. The organic layer was dried and concentrated to give the title compound (160 mg) as a brown solid. MS (ESI, m/z): 630.6 [M+H]⁺

### Step 5)

### tert-butyl 3-((4-(4-(4-((3-(4-(difluoromethoxy)-2,3-difluorophenyl)imidazo[1,2-a]pyrazin-8-yl)amino)-2-fluorobenzoyl)piperazine-1-carbonyl)piperidin-1-yl)methyl)pyrrolidine-1-carboxylate

(4-(4-((3-(4-(difluoromethoxy)-2,3-difluorophenyl)imidazo[1,2-a]pyrazin-8-yl)amino)-2-fluorobenzoyl)piperazin-1-yl)(piperidin-4-yl)methanone (160 mg, 254 µmol, Eq: 1) and tert-butyl 3-formylpyrrolidine-1-carboxylate (65.8 mg, 330 µmol, Eq: 1.3) were dissolved in dry THF (5 ml). To this solution was added sodium triacetoxyborohydride (80.8 mg, 381 µmol, Eq: 1.5) in one portion. The mixture was stirred at rt for 30 min. Then silica gel was added, and the solvent was removed in vacuo to get the silica gel suported sample. The sample was purified by flash chromatography (MeOH:DCM = 0:1 to 1:10) to give the title compound (190 mg) as a brown solid. MS (ESI, m/z): 813.3 [M+H]⁺

### Step 6)

### [4-[[3-[4-(Difluoromethoxy)-2,3-difluoro-phenyl]imidazo[1,2-a]pyrazin-8-yl]amino]-2-fluoro-phenyl]-[4-[1-methyl-1-(pyrrolidin-3-ylmethyl)piperidin-1-ium-4-carbonyl]piperazin-1-yl]methanone 2,2,2-trifluoroacetate

The title compound was prepared in analogy to example 81, step 3 and 4 and was obtained as light yellow solid (80 mg). MS (ESI, m/z): 727.5 [M]⁺

### Example 104

### 3-Aminopropyl-(carboxymethyl)-[5-[[4-[[3-(2,3-difluoro-4-methoxy-phenyl)imidazo[1,2-a]pyrazin-8-yl]amino]-2-ethyl-benzoyl]amino]pentyl]-methyl-ammonium formate

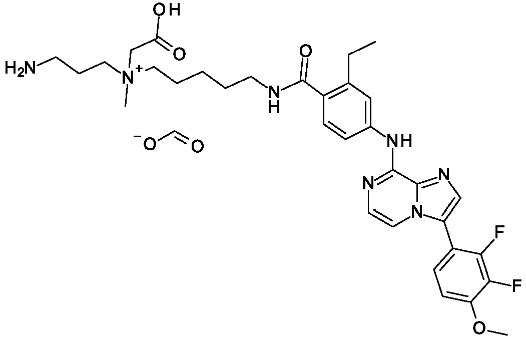

### Step 1)

### tert-Butyl N-[5-[(4-nitro-2-vinyl-benzoyl)amino]pentyl]carbamate

A mixture of 4-nitro-2-vinyl-benzoic acid (Intermediate 22, 4.3 g, 22.26 mmol, 1 eq), N-Boc-1,5-diaminopentane (4.95 g, 24.49 mmol, 1.1 eq), triethylamine (9.31 mL, 66.78 mmol, 3 eq) and propylphosphonic anhydride (19.86 mL, 33.39 mmol, 1.5 eq) in THF (50 mL) and was strred at 25 °C for 16 h.

Then the mixture was poured into water, extracted with EtOAc (50ml*3), washed with brine, dried over sodium sulfate and concentrated. The crude was purified by preparative-HPLC (FA) to afford the title compound (6 g, 15.9 mmol, 71.41% yield) as light yellow oil. MS (ESI, m/z): 278.1 [M-Boc+H]⁺

### Step 2) Intermediate 40

### tert-Butyl N-[5-[(4-amino-2-ethyl-benzoyl)amino]pentyl]carbamate

A mixture of tert-butyl N-[5-[(4-nitro-2-vinyl-benzoyl)amino]pentyl]carbamate (9.0 g, 23.85 mmol, 1 eq), 10% palladium on actived carbon (1.24 mL, 1.19 mmol, 0.050 eq) in methanol (90 mL) was stirred under H₂ (1540 mmHg) at 25 °C for 16 h. The mixture was poured into water (50ml*3), extracted with EtOAc (50ml*3), washed with brine, dried over sodium sulfate and concentrated. The crude material was purified by silica column (DCM/EA=2:1) to afford title compound (7.9 g, 22.61 mmol, 94.8% yield) as light yellow oil. MS (ESI, m/z): 350.2 [M+H]⁺, 250.2 [M-Boc+H]⁺

### Step 3)

### tert-Butyl N-[5-[[4-[[3-(2,3-difluoro-4-methoxy-phenyl)imidazo[1,2-a]pyrazin-8-yl]amino]-2-ethyl-benzoyl]amino]pentyl]carbamate

A mixture of tert-butyl N-[5-[(4-amino-2-ethyl-benzoyl)amino]pentyl]carbamate (6.0 g, 17.17 mmol, 1 eq), 8-chloro-3-(2,3-difluoro-4-methoxy-phenyl)imidazo[1,2-a]pyrazine (1.24 mL, 17.17 mmol, 1 eq) in ACN (66.67 mL)/acetic acid (3.33 mL) was stirred at 60 °C for 16 h. The mixture was concentrated and the crude material purified by prep-HPLC (FA) to afford the title compound (4.3 g, 7.06 mmol, 41.15% yield) as light yellow solid. MS (ESI, m/z): 609.3 [M+H]⁺

### Step 4) Intermediate 41

### N-(5-Aminopentyl)-4-[[3-(2,3-difluoro-4-methoxy-phenyl)imidazo[1,2-a]pyrazin-8-yl]amino]-2-ethyl-benzamide

A mixture of tert-butyl N-[5-[[4-[[3-(2,3-difluoro-4-methoxy-phenyl)imidazo[1,2-a]pyrazin-8-yl]amino]-2-ethyl-benzoyl]amino]pentyl]carbamate (4.3 g, 7.06 mmol, 1 eq) in hydrochloric acid in MeOH (20.0 mL, 80 mmol, 11.32 eq) was stirred at 20 °C for 1 h. The mixture was concentrated and the crude material purified by prep-HPLC (FA) to afford the title compound as (2.2 g, 4.33 mmol, 61.24% yield) as white solid. MS (ESI, m/z): 509.2 [M+H]⁺

### Step 5)

### tert-Butyl N-(3-oxopropyl)carbamate

A suspension of 3-(Boc-amino)-1-propanol (600.0 mg, 3.42 mmol, 1 eq), 2,2,6,6-tetramethyl-1-oxido-piperidine (53.5 mg, 0.340 mmol, 0.100 eq), N-chlorosuccinimide (685.82 mg, 5.14 mmol, 1.5 eq) and N-chlorosuccinimide (685.82 mg, 5.14 mmol, 1.5 eq) in a mixture 0.5N NaHCO₃ (4.5 eq) /0.05N K₂CO₃ (0.5 eq) (10mL) and DCM(10mL) was stirred at roomtemperature for 2h. Then the mixture was poured into water (20mL) and extracted with DCM (30mL*2). The organic layers were dried over Na₂SO₄ and concentrated to afford the crude title compound (450 mg, 76% yield) as orange oil which was used without further purificationfor next step.

### Step 6)

### tert-Butyl N-[3-[5-[[4-[[3-(2,3-difluoro-4-methoxy-phenyl)imidazo[1,2-a]pyrazin-8-yl]amino]-2-ethyl-benzoyl]amino]pentylamino]propyl]carbamate

A mixture of N-(5-aminopentyl)-4-[[3-(2,3-difluoro-4-methoxy-phenyl)imidazo[1,2-a]pyrazin-8-yl]amino]-2-ethyl-benzamide (obtained in step 4, 250.0 mg, 0.490 mmol, 1 eq), tert-butyl N-(3-oxopropyl)carbamate (obtained in step 5, 1.0 mL, 0.540 mmol, 1.1 eq) in methanol (3 mL) was added sodium triacetoxyborohydride (208.37 mg, 0.980 mmol, 2 eq) and stirred at 40 °C for 16 h. Then the mixture was concentrated and purified by prep-HPLC (FA) to afford the title compound (200 mg, 0.300 mmol, 60.62% yield) as white solid. MS (ESI, m/z): 666.4 [M+H]⁺

### Step 7)

### tert-Butyl N-[3-[5-[[4-[[3-(2,3-difluoro-4-methoxy-phenyl)imidazo[1,2-a]pyrazin-8-yl]amino]-2-ethyl-benzoyl]amino]pentyl-methyl-amino]propyl]carbamate

A mixture of tert-butyl N-[3-[5-[[4-[[3-(2,3-difluoro-4-methoxy-phenyl)imidazo[1,2-a]pyrazin-8-yl]amino]-2-ethyl-benzoyl]amino]pentylamino]propyl]carbamate (100.0 mg, 0.150 mmol, 1 eq), formaldehyde in water (0.03 mL, 0.750 mmol, 5 eq) in methanol (2 mL) was added sodium triacetoxyborohydride (159.17 mg, 0.750 mmol, 5 eq) and stirred at 35°C for 16 h. Then the mixture was concentrated and purified by prep-HPLC (FA) to afford the title compound (60 mg, 0.090 mmol, 52.42% yield) as yellow oil. MS (ESI, m/z): 680.4 [M+H]⁺

### Step 8)

### 3-(tert-Butoxycarbonylamino)propyl-(2-tert-butoxy-2-oxo-ethyl)-[5-[[4-[[3-(2,3-difluoro-4-methoxy-phenyl)imidazo[1,2-a]pyrazin-8-yl]amino]-2-ethyl-benzoyl]amino]pentyl]-methyl-ammonium bromide

To a mixture of tert-butyl N-[3-[5-[[4-[[3-(2,3-difluoro-4-methoxy-phenyl)imidazo[1,2-a]pyrazin-8-yl]amino]-2-ethyl-benzoyl]amino]pentyl-methyl-amino]propyl]carbamate (60.0 mg, 0.090 mmol, 1 eq) and triethylamine (0.01 mL, 0.090 mmol, 1 eq) in ACN (2 mL) was added tert-butyl bromoacetate (17.22 mg, 0.090 mmol, 1 eq) and the reaction mixture stirred at 35 °C for 16h. Then the mixture was concentrated to afford crude title compound (50 mg, 0.060 mmol, 71.26% yield) as yellow oil which was used without further purification. MS (ESI, m/z): 794.6 [M]⁺

### Step 9)

### 3-aminopropyl-(carboxymethyl)-[5-[[4-[[3-(2,3-difluoro-4-methoxy-phenyl)imidazo[1,2-a]pyrazin-8-yl]amino]-2-ethyl-benzoyl]amino]pentyl]-methyl-ammonium formate

A mixture of 3-(tert-butoxycarbonylamino)propyl-(2-tert-butoxy-2-oxo-ethyl)-[5-[[4-[[3-(2,3-difluoro-4-methoxy-phenyl)imidazo[1,2-a]pyrazin-8-yl]amino]-2-ethyl-benzoyl]amino]pentyl]-methyl-ammonium bromide (50.0 mg, 0.060 mmol, 1 eq) and trifluoroacetic acid (3.0 mL, 38.94 mmol, 619.12 eq) in DCM (3 mL) was stirred at 25 °C for 16h. The mixture was concentrated and purified by prep-HPLC (FA) to afford the title compound (30.6 mg, 0.040 mmol, 64.55% yield) as white solid. MS (ESI, m/z): 638.2 [M]⁺, 319.6 [M+H]²⁺

### Example 105

### 4-aminobutyl-(carboxymethyl)-[5-[[4-[[3-(2,3-difluoro-4-methoxy-phenyl)imidazo[1,2-a]pyrazin-8-yl]amino]-2-ethyl-benzoyl]amino]pentyl]-methyl-ammonium formate

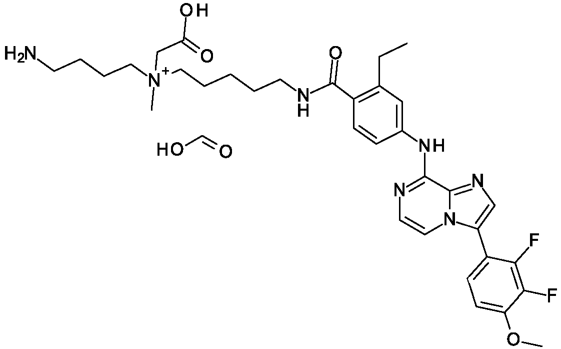

The title compound was prepared in analogy to example 104 using tert-butyl N-(4-hydroxybutyl)carbamate in step 5 and was obtained as white solid (6.7 mg). MS (ESI, m/z): 652.3 [M]⁺

### Example 106

### Azetidin-3-ylmethyl-(carboxymethyl)-[5-[[4-[[3-(2,3-difluoro-4-methoxy-phenyl)imidazo[1,2-a]pyrazin-8-yl]amino]-2-ethyl-benzoyl]amino]pentyl]-methyl-ammonium formate

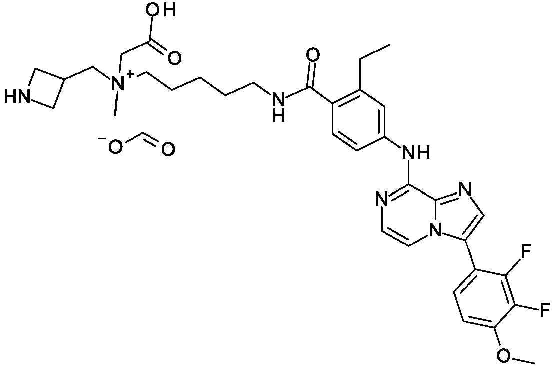

The title compound was prepared in analogy to example 104 using skipping step 5 and using tert-butyl 3-formylazetidine-1-carboxylate in step 6 and was obtained as white solid (31 mg). MS (ESI, m/z): 650.4 [M]⁺

### Example 107

### Azetidin-3-ylmethyl-(3-carboxypropyl)-[5-[[4-[[3-(2,3-difluoro-4-methoxy-phenyl)imidazo[1,2-a]pyrazin-8-yl]amino]-2-ethyl-benzoyl]amino]pentyl]-methyl-ammonium 2,2,2-trifluoroacetate

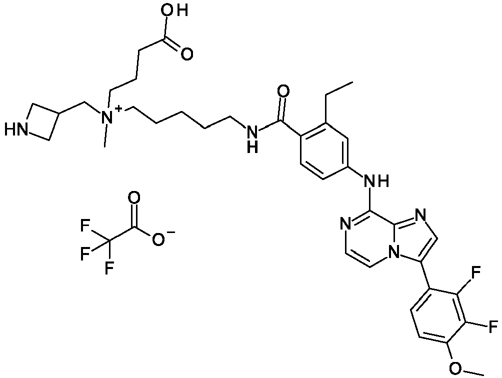

The title compound was prepared in analogy to example 104 using skipping step 5, using tert-butyl 3-formylazetidine-1-carboxylate in step 6 and tert-butyl-4-bromo-butanoate in step 8 and was obtained as white solid (16 mg). MS (ESI, m/z): 678.3 [M]⁺

### Example 108

### 2-[[3-[[2-chloro-4-[[3-[3-(trifluoromethyl)-1H-pyrazol-4-yl]imidazo[1,2-a]pyrazin-8-yl]amino]benzoyl]amino]cyclobutyl]amino]ethyl-trimethyl-ammonium formate

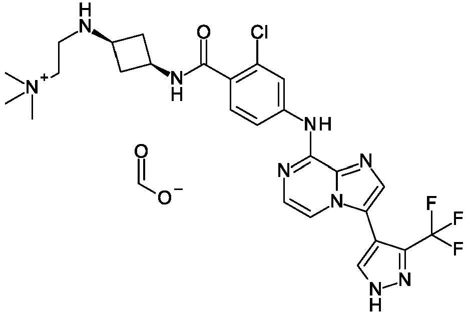

### Step 1)

### Methyl 2-chloro-4-[[3-[3-(trifluoromethyl)-1H-pyrazol-4-yl]imidazo[1,2-a]pyrazin-8-yl]amino]benzoate

To a solution of methyl 2-chloro-4-[(3-iodoimidazo[1,2-a]pyrazin-8-yl)amino]benzoate (Intermediate 26, 1.2 g, 2.8 mmol, 1 eq) in water (5 mL)/1,4-dioxane (50 mL) was added 4-(4,4,5,5-tetramethyl-1,3,2-dioxaborolan-2-yl)-3 -(trifluoromethyl)-1H-pyrazole (0.95 g, 3.64 mmol, 1.3 eq), 1,1'-bis(diphenylphosphino)ferrocene-palladium(II)dichloride dichloromethane complex (0.69 g, 0.840 mmol, 0.300 eq) and sodium carbonate (0.89 g, 8.4 mmol, 3 eq) at 25 °C, the mixture was stirred at 90 °C for 12 h under N2. The reaction mixture was poured into water (100.0 ml) and extracted with DCM(50.0 ml*3), the organic phase was combined and concentrated under reduced pressure. The crude material was purified by silica chromatography column (PE: EtOAc=50:1 to 1:1) to provide methyl 2-chloro-4-[[3-[3-(trifluoromethyl)-1H-pyrazol-4-yl]imidazo[1,2-a]pyrazin-8-yl]amino]benzoate (0.400 g, 0.920 mmol, 32.71% yield) as off white solid. MS (ESI, m/z): 437.0 [M+H]⁺

### Step 2)

### 2-chloro-4-[[3-[3-(trifluoromethyl)-1H-pyrazol-4-yl]imidazo[1,2-a]pyrazin-8-yl]amino]benzoic acid

To a solution of methyl 2-chloro-4-[[3-[3-(trifluoromethyl)-1H-pyrazol-4-yl]imidazo[1,2-a]pyrazin-8-yl]amino]benzoate (400.0 mg, 0.920 mmol, 1 eq) in THF (5 mL) was added 2M aqueous sodium hydroxide (5.0 mL, 10 mmol, 10.92 eq) and then stirred at 60°C for 12 h. The reaction mixture was adjusted to pH=6~7 by 1N HCl, and then the mixture was concentracted under reduce pressure to provide the title compound (1 g, 2.37 mmol, 103.32% yield) as off white solid which was used without further purification. MS (ESI, m/z): 423.0 [M+H]⁺

### Step 3)

### 2-[[3-[[2-chloro-4-[[3-[3-(trifluoromethyl)-1H-pyrazol-4-yl]imidazo[1,2-a]pyrazin-8-yl]amino]benzoyl]amino]cyclobutyl]amino]ethyl-trimethyl-ammonium formate

To a solution of O-(7-azabenzotriazol-1-yl)-N,N,N',N'-tetramethyluronium hexafluorophosphate (485.69 mg, 1.28 mmol, 3 eq) and N,N-diisopropylethylamine (0.11 mL, 0.640 mmol, 1.5 eq) in DMF (5 mL) was added 2-chloro-4-[[3-[3-(trifluoromethyl)-1H-pyrazol-4-yl]imidazo[1,2-a]pyrazin-8-yl]amino]benzoic acid (180.0 mg, 0.430 mmol, 1 eq) and 2-[(3-aminocyclobutyl)amino]ethyl-trimethyl-ammonium chloride (Intermediate 30, 106.14 mg, 0.510 mmol, 1.2 eq). The reaction was stirred at 30 °C for 12 h. Then the mixture was purified by Prep-HPLC(FA) to provide title compound (12.6 mg, 0.020 mmol, 4.58% yield) as off white solid. MS (ESI, m/z): 576.2 [M]⁺

### Example 109

### Azetidin-3-ylmethyl-(carboxymethyl)-[5-[[4-[[3-[1-(cyanomethyl)-3-(trifluoromethyl)pyrazol-4-yl]imidazo[1,2-a]pyrazin-8-yl]amino]-2-ethyl-benzoyl]amino]pentyl]-methyl-ammonium formate

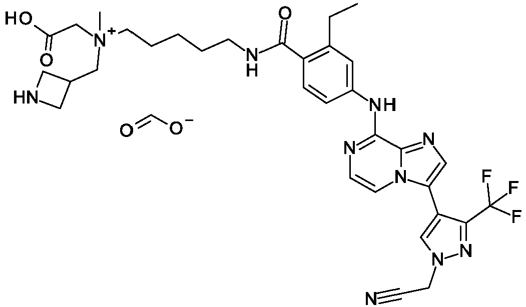

### Step 1) Intermediate 44

### 2-[4-(8-chloroimidazo[1,2-a]pyrazin-3-yl)-3-(trifluoromethyl)pyrazol-1-yl]acetonitrile

A mixture of 8-chloro-3-[3-(trifluoromethyl)-1H-pyrazol-4-yl]imidazo[1,2-a]pyrazine (Intermediate 42, 1.0 g, 3.48 mmol, 1 eq) in ACN (20 mL) was added bromoacetonitrile (0.5 g, 4.17 mmol, 1.2 eq) at 0°C , then the mixture was stirred at 0 °C for 16 h. The mixture was filtered and concentrated, purified by silica column (PE/EA=2:1 to 1:1) to afford the title compound (630 mg, 1.93 mmol, 55.47% yield) as light yellow solid. MS (ESI, m/z): 327.1 [M+H]⁺

### Step 2)

### Azetidin-3-ylmethyl-(carboxymethyl)-[5-[[4-[[3-[1-(cyanomethyl)-3-(trifluoromethyl)pyrazol-4-yl]imidazo[1,2-a]pyrazin-8-yl]amino]-2-ethyl-benzoyl]amino]pentyl]-methyl-ammonium formate

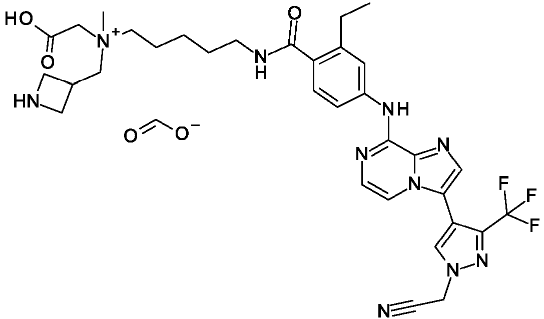

The title compound was prepared in analogy to example 104 using 2-[4-(8-chloroimidazo[1,2-a]pyrazin-3-yl)-3-(trifluoromethyl)pyrazol-1-yl]acetonitrile in step 3, using TFA in DCM for deprotection in step 4, skipping step 5, using tert-butyl 3-formylazetidine-1-carboxylate in step 6 and was obtained as white solid (4.1 mg). MS (ESI, m/z): 681.2 [M]⁺

### Example 110

### Carboxymethyl-[5-[[4-[[3-[1-(cyanomethyl)-3-(trifluoromethyl)pyrazol-4-yl]imidazo[1,2-a]pyrazin-8-yl]amino]-2-ethyl-benzoyl]amino]pentyl]-dimethyl-ammonium formate

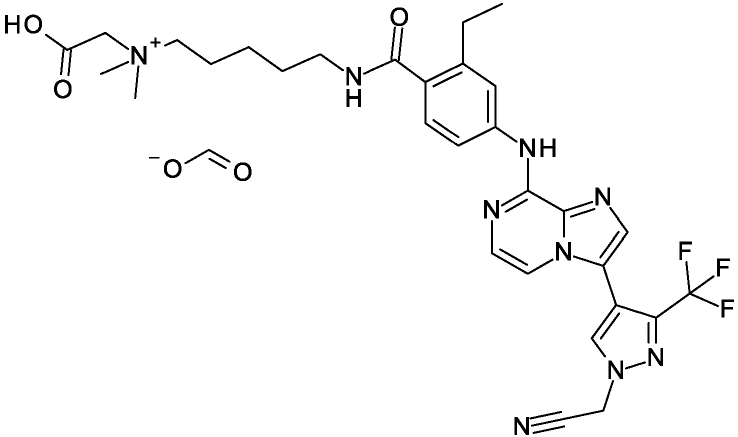

### Step 1)

### tert-Butyl N-[5-[[4-[[3-[1-(cyanomethyl)-3-(trifluoromethyl)pyrazol-4-yl]imidazo[1,2-a]pyrazin-8-yl]amino]-2-ethyl-benzoyl]amino]pentyl]carbamate

The title compound was prepared in analogy to example 104, step 3 using 2-[4-(8-chloroimidazo[1,2-a]pyrazin-3-yl)-3-(trifluoromethyl)pyrazol-1-yl]acetonitrile (Intermediate 44) and was obtained as light yellow solid (530 mg, 0.830 mmol, 60.15% yield). MS (ESI, m/z): 640.4 [M+H]⁺

### Step 2)

### N-(5-Aminopentyl)-4-[[3-[1-(cyanomethyl)-3-(trifluoromethyl)pyrazol-4-yl]imidazo[1,2-a]pyrazin-8-yl]amino]-2-ethyl-benzamide

A solution of tert-butyl N-[5-[[4-[[3-[1-(cyanomethyl)-3-(trifluoromethyl)pyrazol-4-yl]imidazo[1,2-a]pyrazin-8-yl]amino]-2-ethyl-benzoyl]amino]pentyl]carbamate (530.0 mg, 0.830 mmol, 1 eq) in DCM (4.81 mL) was added trifluoroacetic acid (0.5 mL, 6.49 mmol, 7.83 eq) and stirred at 20 °C for 16 h. The mixture was concentrated and purified by prep-HPLC (FA) to afford the title compound (360 mg, 0.670 mmol, 80.53% yield) as white solid. MS (ESI, m/z): 540.2 [M+H]⁺

### Step 3)

### 4-[[3-[1-(cyanomethyl)-3-(trifluoromethyl)pyrazol-4-yl]imidazo[1,2-a]pyrazin-8-yl]amino]-N-[5-(dimethylamino)pentyl]-2-ethyl-benzamide

To a mixture of N-(5-aminopentyl)-4-[[3-[1-(cyanomethyl)-3-(trifluoromethyl)pyrazol-4-yl]imidazo[1,2-a]pyrazin-8-yl]amino]-2-ethyl-benzamide (70.0 mg, 0.130 mmol, 1 eq), formaldehyde in water (0.05 mL, 0.650 mmol, 5 eq) in methanol (2 mL) was added sodium triacetoxyborohydride (137.48 mg, 0.650 mmol, 5 eq) and stirred at 50 °C for 4 h. The solution was filtered and purified by prep-HPLC (FA) to afford the title compound (33 mg, 0.060 mmol, 44.81% yield) as white solid. MS (ESI, m/z): 568.3 [M+H]⁺

### Step 4)

### (2-tert-Butoxy-2-oxo-ethyl)-[5-[[4-[[3-[1-(cyanomethyl)-3-(trifluoromethyl)pyrazol-4-yl]imidazo[1,2-a]pyrazin-8-yl]amino]-2-ethyl-benzoyl]amino]pentyl]-dimethyl-ammonium bromide

A solution of 4-[[3-[1-(cyanomethyl)-3-(trifluoromethyl)pyrazol-4-yl]imidazo[1,2-a]pyrazin-8-yl]amino]-N-[5-(dimethylamino)pentyl]-2-ethyl-benzamide (33.0 mg, 0.060 mmol, 1 eq) and triethylamine (0.02 mL, 0.120 mmol, 2 eq) in ACN (1.1 mL) was added tert-butyl bromoacetate (56.7 mg, 0.290 mmol, 5 eq) and stirred at 40 °C for 3 h. The solution was purified by prep-HPLC (FA) to afford the title compound (21 mg, 0.030 mmol, 47.36% yield) as white solid. MS (ESI, m/z): 682.4 [M]⁺

### Step 5)

### Carboxymethyl-[5-[[4-[[3-[1-(cyanomethyl)-3-(trifluoromethyl)pyrazol-4-yl]imidazo[1,2-a]pyrazin-8-yl]amino]-2-ethyl-benzoyl]amino]pentyl]-dimethyl-ammonium formate

A solution of (2-tert-butoxy-2-oxo-ethyl)-[5-[[4-[[3-[1-(cyanomethyl)-3-(trifluoromethyl)pyrazol-4-yl]imidazo[1,2-a]pyrazin-8-yl]amino]-2-ethyl-benzoyl]amino]pentyl]-dimethyl-ammonium bromide (21.0 mg, 0.030 mmol, 1 eq) in DCM (0.636 mL) was added trifluoroacetic acid (0.47 mL, 6.15 mmol, 223.26 eq) and stirred at 20 °C for 16 h. The solution was purified by prep-HPLC (FA) to afford the title compound (4.6 mg, 0.010 mmol, 23.95% yield) as white solid. MS (ESI, m/z): 626.3 [M]⁺

### Example 111

### Trimethyl-[2-[4-[2-methyl-4-[[3-[1-methyl-3-(trifluoromethyl)pyrazol-4-yl[imidazo[1,2-a]pyrazin-8-yl]amino]benzoyl]piperazin-1-yl]-2-oxo-ethyl]ammonium formate

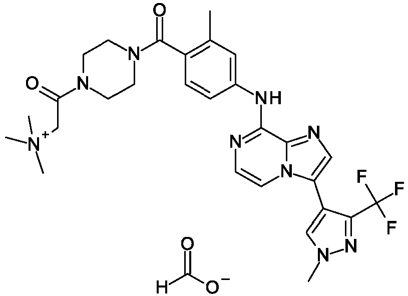

### Step 1)

### 4-[(3-iodoimidazo[1,2-a]pyrazin-8-yl)amino]-2-methyl-benzoic acid

To a solution of methyl 4-[(3-iodoimidazo[1,2-a]pyrazin-8-yl)amino]-2-methyl-benzoate (Intermediate 25, 13.5 g, 33.07 mmol, 1 eq) in THF (30 mL)/methanol (30 mL) was added 2M aqueous sodium hydroxide (60.0 mL, 120 mmol, 3.63 eq) and then stirred at 60 °C for 16 h. After cooling to room temperature, the reaction mixture was adjusted to pH=1~2 by 3N HCl, filtered and dried to give the title compound (13 g, 32.98 mmol, 99.72% yield) as off-white solid. MS (ESI, m/z): 394.9 [M+H]⁺

### Step 2)

### tert-butyl 4-[4-[(3-iodoimidazo[1,2-a]pyrazin-8-yl)amino]-2-methyl-benzoyl]piperazine-1-carboxylate

To a solution of 4-[(3-iodoimidazo[1,2-a]pyrazin-8-yl)amino]-2-methyl-benzoic acid (5.0 g, 12.68 mmol, 1 eq) in DMF (50 mL) was added 1-BOC-piperazine (2.84 g, 15.22 mmol, 1.2 eq), N,N-diisopropylethylamine (6.63 mL, 38.05 mmol, 3 eq) and O-(7-azabenzotriazol-1-yl)-N,N,N',N'-tetramethyluronium hexafluorophosphate (9.65 g, 25.37 mmol, 2 eq), then the reaction was stirred at 30 °C for 16 h. Then 160 mL of water were added and the resulting mixture was filtered. The filter cake was dried to give the title compound (6.2 g, 11.02 mmol, 91.13% yield) as light brown solid. MS (ESI, m/z): 563.1 [M+H]⁺

### Step 3)

### tert-butyl 4-[2-methyl-4-[[3-[1-methyl-3-(trifluoromethyl)pyrazol-4-yl]imidazo[1,2-a]pyrazin-8-yl]amino]benzoyl]piperazine-1-carboxylate

To a solution of tert-butyl 4-[4-[(3-iodoimidazo[1,2-a]pyrazin-8-yl)amino]-2-methylbenzoyl]piperazine-1-carboxylate (1.5 g, 2.67 mmol, 1 eq) in water (1 mL)/1,4-dioxane (10 mL) was added [1-methyl-3-(trifluoromethyl)pyrazol-4-yl]boronic acid (620.65 mg, 3.2 mmol, 1.2 eq), 1,1'-bis(diphenylphosphino)ferrocene-palladium(II)dichloride dichloromethane complex (652.92 mg, 0.800 mmol, 0.300 eq) and sodium carbonate (848.05 mg, 8 mmol, 3 eq) at 25 °C, the mixture was stirred at 80 °C for 12 h. The mixture was poured into water (100 mL) and was extracted with EtOAc (100 mL x3), the combined organic phase was washed with brine (100 mL), dried over anhydrous Na₂SO₄, and concentrated. The residue was purified by flash column chromatography (PE:EtOAc=1:1 ~ EtOAc) to give the title compound (1.3 g, 2.22 mmol, 83.38% yield) as light brown solid. MS (ESI, m/z): 585.3 [M+H]⁺

### Step 4)

### [2-methyl-4-[[3-[1-methyl-3-(trifluoromethyl)pyrazol-4-yl]imidazo[1,2-a]pyrazin-8-yl]amino]phenyl]-piperazin-1-yl-methanone hydrochloride

tert-Butyl 4-[2-methyl-4-[[3-[1-methyl-3-(trifluoromethyl)pyrazol-4-yl]imidazo[1,2-a]pyrazin-8-yl]amino]benzoyl]piperazine-1-carboxylate (1.3 g, 2.22 mmol, 1 eq) was added 4M hydrochloric acid in dioxane (15.0 mL, 60 mmol, 26.98 eq) and then stirred at 20 °C for 3 h. The mixture was concentrated in vacuum and dried to give the title compound [2-methyl-4-[[3-[1-methyl-3-(trifluoromethyl)pyrazol-4-yl]imidazo[1,2-a]pyrazin-8-yl]amino]phenyl]-piperazin-1-yl-methanone (1.2 g, 2.48 mmol, 97% yield) as light brown solid, which was used without further purification.

### Step 5)

### Trimethyl-[2-[4-[2-methyl-4-[[3-[1-methyl-3-(trifluoromethyl)pyrazol-4-yl]imidazo[1,2-a]pyrazin-8-yl]amino]benzoyl]piperazin-1-yl]-2-oxo-ethyl]ammonium formate

To a solution of N,N-diisopropylethylamine (0.16 mL, 0.930 mmol, 3 eq), [2-methyl-4-[[3-[1-methyl-3-(trifluoromethyl)pyrazol-4-yl]imidazo[1,2-a]pyrazin-8-yl]amino]phenyl]-piperazin-1-yl-methanone (150.0 mg, 0.310 mmol, 1 eq) and betaine (40.48 mg, 0.350 mmol, 1.2 eq) in DMF (10 mL) was added O-(7-azabenzotriazol-1-yl)-N,N,N',N'-tetramethyluronium hexafluorophosphate (235.45 mg, 0.620 mmol, 2 eq) then the reaction was stirred at 20 °C for 12 hThe mixture was acidified with FA to pH about 6-7 and purified by prep-HPLC (FA) to give the title compound (50 mg, 0.080 mmol, 26.66% yield) as off-white solid. MS (ESI, m/z): 584.2 [M]⁺, 292.7 [M+H]²⁺
1H NMR (400 MHz, METHANOL-d4) Shift 8.12 (s, 1H), 8.08 (s, 1H), 7.83-7.90 (m, 2H), 7.62-7.67 (m, 2H), 7.51 (d, J=4.77 Hz, 1H), 7.25 (d, J=8.19 Hz, 1H), 4.51 (br s, 1H), 4.45-4.54 (m, 1H), 4.42 (br s, 1H), 4.08 (s, 3H), 3.33-3.95 (m, 17H), 2.35 (s, 3H).

### Example 112

### bis(carboxymethyl)-[5-[[4-[[3-(2,3-difluoro-4-methoxy-phenyl)imidazo[1,2-a]pyrazin-8-yl]amino]-2-ethyl-benzoyl]amino]pentyl]-methyl-ammonium 2,2,2-trifluoroacetate

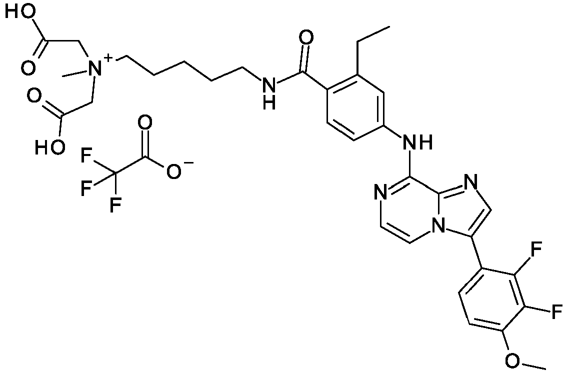

### Step 1)

### tert-butyl 2-[(2-tert-butoxy-2-oxo-ethyl)-[5-[[4-[[3-(2,3-difluoro-4-methoxyphenyl)imidazo[1,2-a]pyrazin-8-yl]amino]-2-ethyl-benzoyl]amino]pentyl]amino]acetate

A solution of N-(5-aminopentyl)-4-[[3-(2,3-difluoro-4-methoxy-phenyl)imidazo[1,2-a]pyrazin-8-yl]amino]-2-ethyl-benzamide (Intermediate 41, 200.0 mg, 0.390 mmol, 1 eq) in ACN (5 mL) was added triethylamine (0.06 mL, 0.450 mmol, 1.15 eq) and tert-butyl bromoacetate (1.0 mL, 0.450 mmol, 1.15 eq), stirred at 25 °C for 16 h.

The reaction detected by LCMS which showed the reaction was completed. The reaction was concentrated and purified by prep-HPLC (FA) to afford the title compound (190 mg, 0.260 mmol, 65.57% yield) as yellow oil. MS (ESI, m/z): 737.5 [M+H]⁺

### Step 2)

### bis(2-tert-butoxy-2-oxo-ethyl)-[5-[[4-[[3-(2,3-difluoro-4-methoxy-phenyl)imidazo[1,2-a]pyrazin-8-yl]amino]-2-ethyl-benzoyl]amino]pentyl]-methyl-ammonium iodide

A solution of tert-butyl 2-[(2-tert-butoxy-2-oxo-ethyl)-[5-[[4-[[3-(2,3-difluoro-4-methoxyphenyl)imidazo[1,2-a]pyrazin-8-yl]amino]-2-ethyl-benzoyl]amino]pentyl]amino]acetate (100.0 mg, 0.140 mmol, 1 eq) in DCM (10 mL) was added iodomethane (0.32 mL, 5.21 mmol, 38.42 eq) and stirred at 30 °C for 16 h. The reaction was concentrated and purified by prep-HPLC (FA) to afford the title compound (70 mg, 0.080 mmol, 58.69% yield) as light yellow oil. MS (ESI, m/z): 751.4 [M]⁺

### Step 3)

### bis(carboxymethyl)-[5-[[4-[[3-(2,3-difluoro-4-methoxy-phenyl)imidazo[1,2-a]pyrazin-8-yl]amino]-2-ethyl-benzoyl]amino]pentyl]-methyl-ammonium 2,2,2-trifluoroacetate

A solution of bis(2-tert-butoxy-2-oxo-ethyl)-[5-[[4-[[3-(2,3-difluoro-4-methoxyphenyl)imidazo[1,2-a]pyrazin-8-yl]amino]-2-ethyl-benzoyl]amino]pentyl]-methyl-ammonium (70.0 mg, 0.090 mmol, 1 eq) in DCM (8 mL) was added trifluoroacetic acid (0.28 mL, 3.58 mmol, 38.42 eq) and stirred at 25 °C for 16 h. The reaction was concentrated and purified by prep-HPLC (FA condition) to afford the title compound (43 mg, 0.060 mmol, 61.18% yield) as white solid. MS (ESI, m/z): 639.4 [M]⁺

### Example 113

### Carboxymethyl-[5-[[4-[[3-[1-(2,2-difluoroethyl)-3-(trifluoromethyl)pyrazol-4-yl]imidazo[1,2-a]pyrazin-8-yl]amino]-2-ethyl-benzoyl]amino]pentyl]-dimethyl-ammonium formate

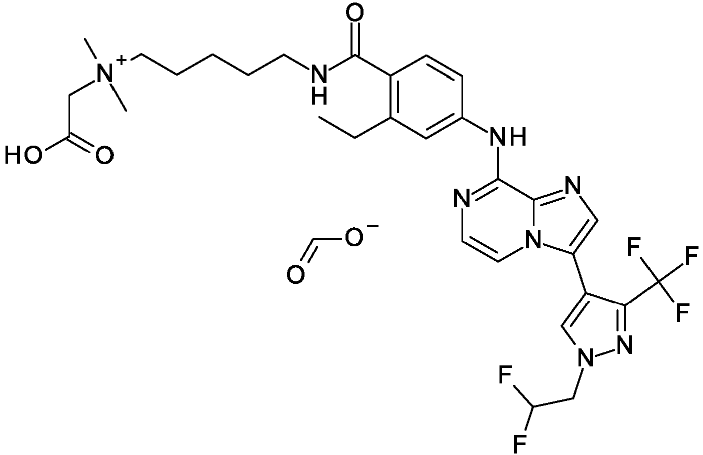

### Step 1)

### tert-Butyl N-[5-[[4-[[3-[1-(2,2-difluoroethyl)-3-(trifluoromethyl)pyrazol-4-yl]imidazo[1,2-a]pyrazin-8-yl]amino]-2-ethyl-benzoyl]amino]pentyl]carbamate

A mixture of tert-butyl N-[5-[(4-amino-2-ethyl-benzoyl)amino]pentyl]carbamate (Intermediate 40, 496.89 mg, 1.42 mmol, 1 eq), 8-chloro-3-[1-(2,2-difluoroethyl)-3-(trifluoromethyl)pyrazol-4-yl]imidazo[1,2-a]pyrazine (Intermediate 43, 1.24 mL, 1.42 mmol, 1 eq) in ACN (8 mL)/acetic acid (0.500 mL) was stirred at 60 °C for 16 h. The mixture was purified by prep-HPLC (FA) to the title compound (640 mg, 0.960 mmol, 67.72% yield) as white solid. MS (ESI, m/z): 665.3 [M+H]⁺

### Step 2)

### N-(5-aminopentyl)-4-[[3-[1-(2,2-difluoroethyl)-3-(trifluoromethyl)pyrazol-4-yl]imidazo[1,2-a]pyrazin-8-yl]amino]-2-ethyl-benzamide

A solution of tert-butyl N-[5-[[4-[[3-[1-(2,2-difluoroethyl)-3-(trifluoromethyl)pyrazol-4-yl]imidazo[1,2-a]pyrazin-8-yl]amino]-2-ethyl-benzoyl]amino]pentyl]carbamate (640.0 mg, 0.960 mmol, 1 eq) in DCM (5.59 mL) was added trifluoroacetic acid (0.58 mL, 7.54 mmol, 7.83 eq) and stirred at 20 °C for 16 h. The mixture was concentrated and purified by prep-HPLC (FA) to afford the title compound (420 mg, 0.740 mmol, 77.26% yield) as white solid. MS (ESI, m/z): 565.3 [M+H]⁺

### Step 3)

### 4-[[3-[1-(2,2-difluoroethyl)-3-(trifluoromethyl)pyrazol-4-yl]imidazo[1,2-a]pyrazin-8-yl]amino]-N-[5-(dimethylamino)pentyl]-2-ethyl-benzamide

A mixture of N-(5-aminopentyl)-4-[[3-[1-(2,2-difluoroethyl)-3-(trifluoromethyl)pyrazol-4-yl]imidazo[1,2-a]pyrazin-8-yl]amino]-2-ethyl-benzamide (70.0 mg, 0.120 mmol, 1 eq), 33% aqueous formaldehyde (0.06 mL, 0.620 mmol, 5 eq) in methanol (2 mL) was added sodium triacetoxyborohydride (131.4 mg, 0.620 mmol, 5 eq) and stirred at 50 °C for 4 h. Then the mixture was filtered and purified by prep-HPLC (FA) to afford the title compound (40 mg, 0.070 mmol, 54.44% yield) as white solid. MS (ESI, m/z): 593.3 [M+H]⁺

### Step 4)

### (2-tert-butoxy-2-oxo-ethyl)-[5-[[4-[[3-[1-(2,2-difluoroethyl)-3-(trifluoromethyl)pyrazol-4-yl]imidazo[1,2-a]pyrazin-8-yl]amino]-2-ethyl-benzoyl]amino]pentyl]-dimethyl-ammonium bromide

A solution of 4-[[3-[1-(2,2-difluoroethyl)-3-(trifluoromethyl)pyrazol-4-yl]imidazo[1,2-a]pyrazin-8-yl]amino]-N-[5-(dimethylamino)pentyl]-2-ethyl-benzamide (40.0 mg, 0.070 mmol, 1 eq) and triethylamine (0.02 mL, 0.130 mmol, 2 eq) in ACN (1.28 mL) was added tert-butyl bromoacetate (65.83 mg, 0.340 mmol, 5 eq) and stirred at 40 °C for 3 h. Then the solution was concentrated to afford the title compound (50 mg, 0.060 mmol, 94.05% yield) as light yellow oil, which was used without further purification. MS (ESI, m/z): 707.3 [M+H]⁺

### Step 5)

### Carboxymethyl-[5-[[4-[[3-[1-(2,2-difluoroethyl)-3-(trifluoromethyl)pyrazol-4-yl]imidazo[1,2-a]pyrazin-8-yl]amino]-2-ethyl-benzoyl]amino]pentyl]-dimethyl-ammonium formate

A solution of (2-tert-butoxy-2-oxo-ethyl)-[5-[[4-[[3-[1-(2,2-difluoroethyl)-3-(trifluoromethyl)pyrazol-4-yl]imidazo[1,2-a]pyrazin-8-yl]amino]-2-ethyl-benzoyl]amino]pentyl]-dimethyl-ammonium bromide (50.0 mg, 0.060 mmol, 1 eq) in trifluoroacetic acid (2.0 mL, 25.96 mmol, 408.96 eq) was stirred at 20 °C for 3 h.Then the solution was concentrated and purified by prep-HPLC (FA) to the title compound (29.2 mg, 0.040 mmol, 65.83% yield) as white solid. MS (ESI, m/z): 651.3 [M+H]⁺

### Example 114

### 1-(Azetidin-3-ylmethyl)-N-[(2S)-2-[[4-[[3-[4-(difluoromethoxy)-2,3-difluoro-phenyl]imidazo[1,2-a]pyrazin-8-yl]amino]-2-ethyl-benzoyl]amino]propyl]-1-methyl-piperidin-1-ium-4-carboxamide formate

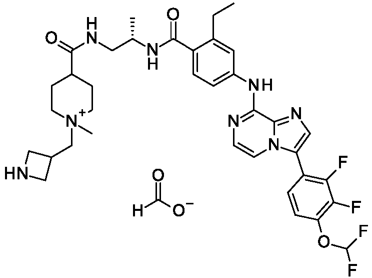

### Step 1)

### tert-butyl N-[(2S)-2-[[4-[[3-[4-(difluoromethoxy)-2,3-difluoro-phenyl]imidazo[1,2-a]pyrazin-8-yl]amino]-2-ethyl-benzoyl]amino]propyl]carbamate

To a solution of 4-((3-(4-(difluoromethoxy)-2,3-difluorophenyl)imidazo[1,2-a]pyrazin-8-yl)amino)-2-ethylbenzoic acid (Intermediate 36, 1.43 g, 3.11 mmol, Eq: 1.0), *tert-butyl* (S)-(2-aminopropyl)carbamate (600 mg, 3.42 mmol, Eq: 1.1) in anhydrous DMF (25 mL) was added DIPEA (1.2 g, 9.33 mmol, Eq: 3.0). Then the resultant mixture was stirred for 10 min at room temperature, HATU (2.4 g, 6.22 mmol, Eq: 2.0) was added in the mixture and stirred for extra 10 hr. The mixture poured into water (50 mL) and the aqueous solution extracted with DCM (50 mL×2). The organic layers were combined and washed with water and brine, dried over anhydrous sodium sulfate and concentrated under reduced pressure to give the title compound (1.6 g, 2.6 mmol, 83.5% yield) as a red oil which was used in next step without purification. MS (ESI, m/z): 617.0 [M+H]⁺.

### Step 2)

### N-[(1S)-2-Amino-1-methyl-ethyl]-4-[[3-[4-(difluoromethoxy)-2,3-difluorophenyl]imidazo[1,2-a]pyrazin-8-yl]amino]-2-ethyl-benzamide

To a solution of tert-butyl (S)-(2-(4-((3-(4-(difluoromethoxy)-2,3-difluorophenyl)imidazo[1,2-a]pyrazin-8-yl)amino)-2-ethylbenzamido)propyl)carbamate (1.23 g, 2.0 mmol) in DCM (20 ml) was added TFA (4.0 mL) at room temperature. The resultant mixture was stirred for 4.0 h and then adjusted to pH=7-8 with aqueous ammonia. The mixture was poured into water (15 mL) and then extracted with dichloromethane / isopropanol (100/10 mL), the organic layer was concentrated to give a red oil which was purified by flash column to afford the title compound (520 mg, 1.0 mmol, 50.0 % yield) as an off-white solid. MS (ESI, m/z): 517.0 [M+H]⁺.

### Step 3)

### tert-Butyl 4-[[(2S)-2-[[4-[[3-[4-(difluoromethoxy)-2,3-difluoro-phenyl]imidazo[1,2-a]pyrazin-8-yl]amino]-2-ethyl-benzoyl]amino]propyl]carbamoyl]piperidine-1-carboxylate

To a solution of (S)-N-(1-aminopropan-2-yl)-4-((3-(4-(difluoromethoxy)-2,3-difluorophenyl)imidazo[1,2-a]pyrazin-8-yl)amino)-2-ethylbenzamide (517 mg, 1.0 mmol, Eq: 1.0), 1-(tert-butoxycarbonyl)piperidine-4-carboxylic acid (275 mg, 1.2 mmol, Eq: 1.2) in anhydrous DMF (10 mL) was added DIPEA (258 mg, 2 mmol, Eq: 2.0). Then the resultant mixture was stirred for 10 min at room temperature, HATU (760 mg, 2.0 mmol, Eq: 2.0) was added in the mixture and stirred for extra 10 hr. The mixture was poured into water (50 mL) and the aqueous solution was extracted with DCM (50 mL×2). The organic layers were combined and washed with water and brine, dried over anhydrous sodium sulfate and concentrated under reduced pressure to give a red oil which was used in next step without purification (600 mg, 0.82 mmol, 82.4 % yield) MS (ESI, m/z): 728.0 [M+H]⁺.

### Step 4)

### N-[(2S)-2-[[4-[[3-[4-(Difluoromethoxy)-2,3-difluoro-phenyl]imidazo[1,2-a]pyrazin-8-yl]amino]-2-ethyl-benzoyl]amino]propyl]piperidine-4-carboxamide

To a solution of tert-butyl (S)-4-((2-(4-((3-(4-(difluoromethoxy)-2,3-difluorophenyl)imidazo[1,2-a]pyrazin-8-yl)amino)-2-ethylbenzamido)propyl)carbamoyl)piperidine-1-carboxylate (580 mg, 0.8 mmol) in DCM (20 ml) was added TFA (4.0 mL) at room temperature. The resultant mixture was stirred for 4.0 h and then adjusted to pH=7-8 with aqueous ammonia. The mixture was poured into water (15 mL) and then extracted with dichloromethane / isopropanol (100/10 mL), the organic layer was concentrated to give a red oil, which was purified by Prep.HPLC to afford the title compound (300 mg, 1.35 mmol, 85.7% yield) as a white powder. MS (ESI, m/z): 628.4 [M+H]⁺.

### Step 5)

### tert-Butyl 3-[[4-[[(2S)-2-[[4-[[3-[4-(difluoromethoxy)-2,3-difluoro-phenyl]imidazo[1,2-a]pyrazin-8-yl]amino]-2-ethyl-benzoyl]amino]propyl]carbamoyl]-1-piperidyl]methyl]azetidine-1-carboxylate

In a 25 mL round-bottomed flask, (S)-N-(2-(4-((3-(4-(difluoromethoxy)-2,3-difluorophenyl)imidazo[1,2-a]pyrazin-8-yl)amino)-2-ethylbenzamido)propyl)piperidine-4-carboxamide (314 mg, 0.5 mmol, Eq: 1), *tert*-butyl 3-formylpyrrolidine-1-carboxylate (300 mg, 1.5 mmol, Eq: 3.0) and sodium cyanoborohydride (125 mg, 2.0 mmol, Eq: 4) were combined with MeOH (10 ml) to give a colorless solution. Stirred the reaction mixture for 4.0 h. The mixture was poured into water (50 mL) and then extracted with DCM (100 mLx2). The organic layers were combined and washed with water and brine, dried over anhydrous sodium sulfate and concentrated under reduced pressure to give a red oil, which was purified by Flash to provide the desired compound (300 mg, 0.37 mmol, 74.0 % yield) as a yellow solid. MS (ESI, m/z): 797.1 [M+H]⁺.

### Step 6)

### tert-butyl 3-[[4-[[(2S)-2-[[4-[[3-[4-(difluoromethoxy)-2,3-difluoro-phenyl]imidazo[1,2-a]pyrazin-8-yl]amino]-2-ethyl-benzoyl]amino]propyl]carbamoyl]-1-methyl-piperidin-1-ium-1-yl]methyl]azetidine-1-carboxylate iodide

To a solution of tert-butyl (S)-3-((4-((2-(4-((3-(4-(difluoromethoxy)-2,3-difluorophenyl)imidazo[1,2-a]pyrazin-8-yl)amino)-2-ethylbenzamido)propyl)carbamoyl)piperidin-1-yl)methyl)azetidine-1-carboxylate (100 mg, 0.125 mmol, Eq: 1.0) in acetonitrile (5 mL) was added DIPEA( 65 mg, 0.5 mmol, Eq: 4.0), methyl iodide (71 mg, 0.5 mmol, Eq: 4.0) at room temperature. The solvent was concentrated to provide the title compound (100 mg, 0.12 mmol, 96.0 % yield) as a yellow oil, which used in next step without purification. MS (ESI, m/z): 811.4 [M]⁺.

### Step 7)

### 1-(Azetidin-3-ylmethyl)-N-[(2S)-2-[[4-[[3-[4-(difluoromethoxy)-2,3-difluorophenyl]imidazo[1,2-a]pyrazin-8-yl]amino]-2-ethyl-benzoyl]amino]propyl]-1-methyl-piperidin-1-ium-4-carboxamide formate

To a solution of *tert*-butyl 3-[[4-[[(2S)-2-[[4-[[3-[4-(difluoromethoxy)-2,3-difluorophenyl]imidazo[1,2-a]pyrazin-8-yl]amino]-2-ethyl-benzoyl]amino]propyl]carbamoyl]-1-methyl-piperidin-1-ium-1-yl]methyl]azetidine-1-carboxylate iodide (100 mg, 0.105 mmol) in DCM (10 ml) was added TFA (2.0 mL) at room temperature. The resultant mixture was stirred for 5.0 h and then adjusted to pH=7-8 with aqueous ammonia.

The mixture was poured into water (25 mL) and then extracted with dichloromethane / isopropanol (100/10 mL), the organic layer was concentrated to give a red oil, which was purified by prep. HPLC to afford the title compound (3.0 mg, 4.0 µmol, 3.8% yield) as a yellow powder. MS (ESI, m/z): 711.4 [M]⁺.

### Example 115

### 2-[4-[[[4-[[3-(2,3-Difluoro-4-methoxy-phenyl)imidazo[1,2-a]pyrazin-8-yl]amino]-2-ethyl-benzoyl]amino]methyl]-1-methyl-piperidin-1-ium-1-yl]acetic acid 2,2,2-trifluoroacetate

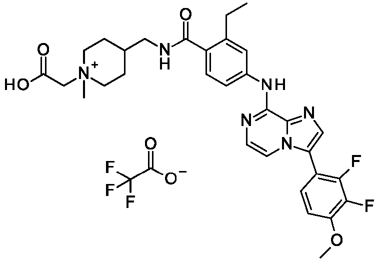

### Step 1)

### tert-Butyl 4-[[[4-[[3-(2,3-difluoro-4-methoxy-phenyl)imidazo[1,2-a]pyrazin-8-yl]amino]-2-ethyl-benzoyl]amino]methyl]piperidine-1-carboxylate

To a solution of 4-((3-(2,3-difluoro-4-methoxyphenyl)imidazo[1,2-a]pyrazin-8-yl)amino)-2-ethylbenzoic acid (Intermediate 8) (0.85 g, 2.0 mmol, Eq: 1.0), *tert*-butyl 4-(aminomethyl)piperidine-1-carboxylate (0.51 g, 2.4 mmol, Eq: 1.2) in anhydrous DMF (25 mL) was added DIPEA (516 mg, 4.0 mmol, Eq: 2.0). Then the resultant mixture was stirred for 10 min at room temperature, HATU (1.52 g, 4.0 mmol, Eq: 2.0) was added in the mixture and stirred for extra 4.0 hr. Poured the mixture into water (50 mL) and the aqueous solution was extracted with DCM (50 mL×2). The organic layers were combined and washed with water and brine, dried over anhydrous sodium sulfate and concentrated under reduced pressure to give a red oil which was purified by Flash column to provide the title compound (1.0 g, 1.61 mmol, 80.5% yield) as a yellow solid. MS (ESI, m/z): 621.4 [M+H]⁺.

### Step 2)

### 4-[[3-(2,3-Difluoro-4-methoxy-phenyl)imidazo[1,2-a]pyrazin-8-yl]amino]-2-ethyl-N-(4-piperidylmethyl)benzamide

To a solution of *tert-*butyl 4-[[[4-[[3-(2,3-difluoro-4-methoxy-phenyl)imidazo[1,2-a]pyrazin-8-yl]amino]-2-ethyl-benzoyl]amino]methyl]piperidine-1-carboxylate (1.0 g, 1.61 mmol) in DCM (25 ml) was added TFA (5.0 mL) at room temperature. The resultant mixture was stirred for 4.0 h and then adjusted to pH=7-8 with aqueous ammonia. The mixture was poured into water (25 mL) and then extracted with dichloromethane / isopropanol (100/10 mL), the organic layer was concentrated to give a red oil which was purified by flash column to afford the title compound (780 mg, 1.5 mmol, 93.2 % yield) as a yellow solid. MS (ESI, m/z): 521.5 [M+H]⁺.

### Step 3)

### tert-butyl 2-[4-[[[4-[[3-(2,3-difluoro-4-methoxy-phenyl)imidazo[1,2-a]pyrazin-8-yl]amino]-2-ethyl-benzoyl]amino]methyl]-1-piperidyl]acetate

To a solution of 4-((3-(2,3-difluoro-4-methoxyphenyl)imidazo[1,2-a]pyrazin-8-yl)amino)-2-ethyl-N-(piperidin-4-ylmethyl)benzamide (780 mg, 1.5 mmol, Eq: 1.0 ) and DIPEA (580 mg, 4.5 mmol, Eq: 3.0) in acetonitrile (15 ml) was added *tert-butyl* 2-bromoacetate (290 mg, 1.5 mmol, Eq: 1.0) at room temperature, and then the resultant mixture was stirred overnight.

The mixture was poured into water (30 mL) and then extracted with dichloromethane / isopropanol (100/10 mL), the organic layer was concentrated to give a red oil, which was purified by flash column to provide the title compound (850 mg, 1.34 mmol, 89.4 % yield) MS (ESI, m/z): 635.5 [M+H]⁺.

### Step 4)

### tert-Butyl 2-[4-[[[4-[[3-(2,3-difluoro-4-methoxy-phenyl)imidazo[1,2-a]pyrazin-8-yl]amino]-2-ethyl-benzoyl]amino]methyl]-1-methyl-piperidin-1-ium-1-yl]acetate iodide

To a solution of tert-butyl 2-(4-((4-((3-(2,3-difluoro-4-methoxyphenyl)imidazo[1,2-a]pyrazin-8-yl)amino)-2-ethylbenzamido)methyl)piperidin-1-yl)acetate (315 mg, 0.5 mmol, Eq: 1.0 ) and DIPEA (258 mg, 2.0 mmol, Eq: 4.0) in acetonitrile (10 ml) was added iodomethane (284 mg, 2.0 mmol, Eq: 4.0) at room temperature, and then the resultant mixture was stirred overnight. The solvent was concentrated to give a yellow solid (260 mg, 0.35 mmol, 70.0% yield) which was used in next step without purification. MS (ESI, m/z): 649.5 [M]⁺.

### Step 5)

### 2-[4-[[[4-[[3-(2,3-difluoro-4-methoxy-phenyl)imidazo[1,2-a]pyrazin-8-yl]amino]-2-ethyl-benzoyl]amino]methyl]-1-methyl-piperidin-1-ium-1-yl]acetic acid;2,2,2-trifluoroacetate3-[[4-[[(2S)-2-[[4-[[3-[4-(difluoromethoxy)-2,3-difluoro-phenyl]imidazo[1,2-a]pyrazin-8-yl]amino]-2-ethyl-benzoyl]amino]propyl]carbamoyl]-1-piperidyl]methyl]azetidine-1-carboxylate

To a solution of tert-butyl 2-[4-[[[4-[[3-(2,3-difluoro-4-methoxy-phenyl)imidazo[1,2-a]pyrazin-8-yl]amino]-2-ethyl-benzoyl]amino]methyl]-1-methyl-piperidin-1-ium-1-yl]acetate;iodide (260 mg, 0.35 mmol) in DCM (10 mL) was added TFA (3.0 mL) and the resultant solution was stirred for 4.0 h at room temperature.

The mixture was adjusted to pH=6-7 with aqueous ammonia and then poured into water (50 mL) and then extracted with dichloromethane / isopropanol (100/10 mL), the organic layer was concentrated to give a red oil which was purified by Prep. HPLC to provide the desired compound (75 mg, 0.106 mmol, 30.0 % yield) as a white powder. MS (ESI, m/z): 593.5 [M]⁺.

### Example 116

### [2-Chloro-4-[[3-[4-(difluoromethoxy)-2,3-difluoro-phenyl]imidazo[1,2-a]pyrazin-8-yl]amino]phenyl]-[4-[1-methyl-1-(pyrrolidin-3-ylmethyl)piperidin-1-ium-4-carbonyl]piperazin-1-yl]methanone formate

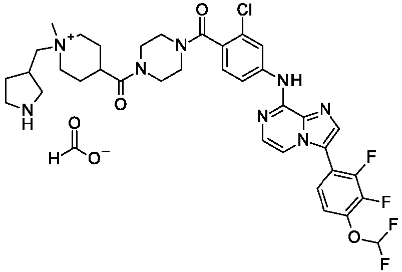

### Step 1)

### 8-Chloro-3-[4-(difluoromethoxy)-2,3-difluoro-phenyl]imidazo[1,2-a]pyrazine

To a solution of 8-chloro-3-iodoimidazo[1,2-a]pyrazine (Intermediate 1 600 mg, 2.15 mmol, Eq: 1) in the mixture solvent of dioxane (5 mL) and water (1 mL) was added 2-(4-(difluoromethoxy)-2,3-difluorophenyl)-4,4,5,5-tetramethyl-1,3,2-dioxaborolane (Intermediate 33 789 mg, 2.58 mmol, Eq: 1.2), sodium carbonate (683 mg, 6.44 mmol, Eq: 3) and 1,1'-bis(di-*tert-*butylphosphino)ferrocene palladium dichloride (140 mg, 215 µmol, Eq: 0.1). The reaction was stirred for 30 minutes at 100 °C under microwave irriation and atmosphere of nitrogen. The reaction mixture was filtered and the filtrate was concentrated in vacuum. The residue was purified by column chromatography to give the title compound ( 410 mg, 1.24 mmol, 57.6 % yield). MS (ESI, m/z): 332.1 [M+H]⁺.

### Step 2) Intermediate 45

### 2-Chloro-4-[[3-[4-(difluoromethoxy)-2,3-difluoro-phenyl]imidazo[1,2-a]pyrazin-8-yl]amino]benzoic acid

To a solution of 8-chloro-3-(4-(difluoromethoxy)-2,3-difluorophenyl)imidazo[1,2-a]pyrazine (900mg, 2.71 mmol, Eq: 1) in EtOH ( 10 mL) was added 4-amino-2-chlorobenzoic acid (559 mg, 3.26 mmol, Eq: 1.2), the reaction was stirred for 15 hours at 100 °C. The reaction mixture was cooled to room temperature and filtered. The filter cake was dried in vacuum to give the title compound (600 mg, 1.29 mmol, 47.4 % yield). MS (ESI, m/z): 467.2 [M+H]⁺.

### Step 3)

### tert-Butyl 4-[2-chloro-4-[[3-[4-(difluoromethoxy)-2,3-difluoro-phenyl]imidazo[1,2-a]pyrazin-8-yl]amino]benzoyl]piperazine-1-carboxylate

To a solution of 2-chloro-4-((3-(4-(difluoromethoxy)-2,3-difluorophenyl)imidazo[1,2-a]pyrazin-8-yl)amino)benzoic acid ( 300 mg, 643 µmol, Eq: 1) in DMF ( 6 mL) was added tert-butyl piperazine-1-carboxylate (156 mg, 836 µmol, Eq: 1.3), HATU (489 mg, 1.29 mmol, Eq: 2) and triethylamine (195 mg, 269 µl, 1.93 mmol, Eq: 3). The reaction was stirred for 20 minutes at room temperature. The reaction mixture was poured into water and filtered. The filter cake was dried in vacuum to give the title compound (400 mg, 630 µmol, 98 % yield). MS (ESI, m/z): 635.1 [M+H]⁺.

### Step 4)

### [2-Chloro-4-[[3-[4-(difluoromethoxy)-2,3-difluoro-phenyl]imidazo[1,2-a]pyrazin-8-yl]amino]phenyl]-piperazin-1-yl-methanone 2,2,2-trifluoroacetate

To dissolve tert-butyl 4-(2-chloro-4-((3-(4-(difluoromethoxy)-2,3-difluorophenyl)imidazo[1,2-a]pyrazin-8-yl)amino)benzoyl)piperazine-1-carboxylate ( 400 mg, 630 µmol, Eq: 1) in the mixture solvent of DCM (4 mL) and TFA (4 mL), the reaction was stirred for 10 minutes at room temperature. The reaction mixture was concentrated in vacuum to give the title compound (450 mg, 617 µmol, 98 % yield). MS (ESI, m/z): 535.1 [M+H]⁺.

### Step 5)

### tert-Butyl 4-[4-[2-chloro-4-[[3-[4-(difluoromethoxy)-2,3-difluoro-phenyl]imidazo[1,2-a]pyrazin-8-yl]amino]benzoyl]piperazine-1-carbonyl]piperidine-1-carboxylate

To a solution of (2-chloro-4-((3-(4-(difluoromethoxy)-2,3-difluorophenyl)imidazo[1,2-a]pyrazin-8-yl)amino)phenyl)(piperazin-1-yl)methanone 2,2,2-trifluoroacetate ( 450 mg, 693 µmol, Eq: 1) in DMF ( 5 mL) was added 1-(*tert*-butoxycarbonyl)piperidine-4-carboxylic acid (191 mg, 832 µmol, Eq: 1.2), triethylamine (211 mg, 290 µl, 2.08 mmol, Eq: 3) and HATU (396 mg, 1.04 mmol, Eq: 1.5). The reaction was stirred for 20 minutes at room temperature. The reaction mixture was poured into water and filtered. The filter cake was dried in vacuum to give the title compound ( 500 mg, 670 µmol, 96.6 % yield). MS (ESI, m/z): 746.1 [M+H]⁺.

### Step 6)

### [2-Chloro-4-[[3-[4-(difluoromethoxy)-2,3-difluoro-phenyl]imidazo[1,2-a]pyrazin-8-yl]amino]phenyl]-[4-(piperidine-4-carbonyl)piperazin-1-yl]methanone hydrochloride

To dissolve tert-butyl 4-(4-(2-chloro-4-((3-(4-(difluoromethoxy)-2,3-difluorophenyl)imidazo[1,2-a]pyrazin-8-yl)amino)benzoyl)piperazine-1-carbonyl)piperidine-1-carboxylate ( 500 mg, 670 µmol, Eq: 1) in MeOH ( 3 mL) was added HCl/dioxane (2 mol/L, 5mL), the reaction was stirred for 30 minutes at room temperature. The reaction mixture was dried in vacuum to give the title compound (400 mg, 586 µmol, 87.5 % yield). MS (ESI, m/z): 646.2 [M+H]⁺.

### Step 7)

### tert-Butyl 3-[[4-[4-[2-chloro-4-[[3-[4-(difluoromethoxy)-2,3-difluoro-phenyl]imidazo[1,2-a]pyrazin-8-yl]amino]benzoyl]piperazine-1-carbonyl]-1-piperidyl]methyl]pyrrolidine-1-carboxylate

To a solution of (4-(2-chloro-4-((3-(4-(difluoromethoxy)-2,3-difluorophenyl)imidazo[1,2-a]pyrazin-8-yl)amino)benzoyl)piperazin-1-yl)(piperidin-4-yl)methanone hydrochloride (100 mg, 147 µmol, Eq: 1) in MeOH ( 3 mL) was added *tert-butyl* 3-formylpyrrolidine-1-carboxylate (87.6 mg, 440 µmol, Eq: 3) and sodium cyanoborohydride (18.4 mg, 293 µmol, Eq: 2). The reaction was stirred for two hours at room temperature. The reaction mixture was quenched with water and extracted in EtOAc. The organic layer was dried over anhydrous Na₂SO₄ and concentrated in vacuum. The residue was purified by column chromatography to give the title compound (50 mg, 60 µmol, 41.1 % yield). MS (ESI, m/z): 829.2 [M+H]⁺.

### Step 8)

### tert-Butyl 3-[[4-[4-[2-chloro-4-[[3-[4-(difluoromethoxy)-2,3-difluoro-phenyl]imidazo[1,2-a]pyrazin-8-yl]amino]benzoyl]piperazine-1-carbonyl]-1-methyl-piperidin-1-ium-1-yl]methyl]pyrrolidine-1-carboxylate iodide

To a solution of tert-butyl 3-((4-(4-(2-chloro-4-((3-(4-(difluoromethoxy)-2,3-difluorophenyl)imidazo[1,2-a]pyrazin-8-yl)amino)benzoyl)piperazine-1-carbonyl)piperidin-1-yl)methyl)pyrrolidine-1-carboxylate ( 50 mg, 60.3 µmol, Eq: 1) in acetonitrile ( 3 mL) was added triethylamine (12.2 mg, 16.8 µl, 121 µmol, Eq: 2) and iodomethane (42.8 mg, 18.8 µl, 301 µmol, Eq: 5). The reaction was stirred for 16 hours at room temperature. The reaction mixture was concentrated in vacuum and the residue was directly used for the next step without further purification. MS (ESI, m/z): 843.3 [M]⁺.

### Step 9)

### [2-Chloro-4-[[3-[4-(difluoromethoxy)-2,3-difluoro-phenyl]imidazo[1,2-a]pyrazin-8-yl]amino]phenyl]-[4-[1-methyl-1-(pyrrolidin-3-ylmethyl)piperidin-1-ium-4-carbonyl]piperazin-1-yl]methanone formate

To a solution of tert-butyl 3-[[4-[4-[2-chloro-4-[[3-[4-(difluoromethoxy)-2,3-difluorophenyl]imidazo[1,2-a]pyrazin-8-yl]amino]benzoyl]piperazine-1-carbonyl]-1-methyl-piperidin-1-ium-1-yl]methyl]pyrrolidine-1-carboxylate;iodide ( 50 mg, 59.2 µmol, Eq: 1) in MeOH (3 mL) was added HCl/dioxane (2 mol/L, 3mL), the reaction was stirred for two hours at room temperature. The reaction mixture was concentrated in vacuum and the residue was purified by preparative HPLC to give the title compound (24 mg, 51.4% yield). MS (ESI, m/z): 743.0 [M]⁺.

### Example 117

### N-[3-[[2-Chloro-4-[[3-[4-(difluoromethoxy)-2,3-difluoro-phenyl]imidazo[1,2-a]pyrazin-8-yl]amino]benzoyl]amino]propyl]-1-methyl-1-(pyrrolidin-3-ylmethyl)piperidin-1-ium-4-carboxamide formate

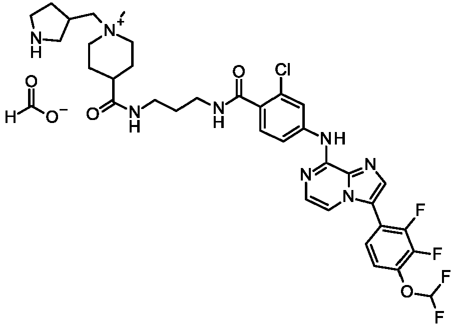

### Step 1)

### tert-Butyl N-[3-[[2-chloro-4-[[3-[4-(difluoromethoxy)-2,3-difluoro-phenyl]imidazo[1,2-a]pyrazin-8-yl]amino]benzoyl]amino]propyl] carbamate

To a solution of 2-chloro-4-((3-(4-(difluoromethoxy)-2,3-difluorophenyl)imidazo[1,2-a]pyrazin-8-yl)amino)benzoic acid (Intermediate 45, 300 mg, 643 µmol, Eq: 1) in DMF ( 6 mL) was added tert-butyl (3-aminopropyl)carbamate (146 mg, 836 µmol, Eq: 1.3),2-(3H-[1,2,3]triazolo[4,5-b]pyridin-3-yl)-1,1,3,3-tetramethylisouronium hexafluorophosphate(V) (489 mg, 1.29 mmol, Eq: 2) and triethylamine (195 mg, 269 µl, 1.93 mmol, Eq: 3), the reaction was stirred for 20 minutes at room temperature. The reaction mixture was poured into water and filtered. The filter cake was dried in vacuum to give the title compound (360 mg, 578 µmol, 89.9 % yield). MS (ESI, m/z): 623.1 [M+H]⁺.

### Step 2)

### N-(3-Aminopropyl)-2-chloro-4-[[3-[4-(difluoromethoxy)-2,3-difluoro-phenyl]imidazo[1,2-a]pyrazin-8-yl]amino]benzamide 2,2,2-trifluoroacetate

To dissolve tert-butyl (3-(2-chloro-4-((3-(4-(difluoromethoxy)-2,3-difluorophenyl)imidazo[1,2-a]pyrazin-8-yl)amino)benzamido)propyl)carbamate (360 mg, 578 µmol, Eq: 1) in the mixture solvent of DCM (4 mL) and TFA (4 mL), the reaction was stirred for 10 minutes at room temperature. The reaction mixture was concentrated in vacuum to give the title compound (320 mg, 502 µmol, 86.9 % yield). MS (ESI, m/z): 523.1 [M+H]⁺.

### Step 3)

### tert-Butyl 4-[3-[[2-chloro-4-[[3-[4-(difluoromethoxy)-2,3-difluoro-phenyl]imidazo[1,2-a]pyrazin-8-yl]amino]benzoyl]amino]propylcarbamoyl]piperidine-1-carboxylate

To a solution of N-(3-aminopropyl)-2-chloro-4-((3-(4-(difluoromethoxy)-2,3-difluorophenyl)imidazo[1,2-a]pyrazin-8-yl)amino)benzamide 2,2,2-trifluoroacetate (320 mg, 502 µmol, Eq: 1) in DMF ( 5 mL) was added 1-(tert-butoxycarbonyl)piperidine-4-carboxylic acid (138 mg, 603 µmol, Eq: 1.2), triethylamine (153 mg, 210 µL, 1.51 mmol, Eq: 3) and 2-(3H-[1,2,3]triazolo[4,5-b]pyridin-3-yl)-1,1,3,3-tetramethylisouronium hexafluorophosphate(V) (287 mg, 754 µmol, Eq: 1.5). The reaction was stirred for 20 minutes at room temperature. The reaction mixture was poured into water and filtered. The filter cake was dried in vacuum to give the title compound (320 mg, 436 µmol, 86.8 % yield). MS (ESI, m/z): 734.0 [M+H]⁺.

### Step 4)

### N-[3-[[2-Chloro-4-[[3-[4-(difluoromethoxy)-2,3-difluoro-phenyl]imidazo[1,2-a]pyrazin-8-yl]amino]benzoyl]amino]propyl]piperidine-4-carboxamide; hydrochloride

To dissolve tert-butyl 4-((3-(2-chloro-4-((3-(4-(difluoromethoxy)-2,3-difluorophenyl)imidazo[1,2-a]pyrazin-8-yl)amino)benzamido)propyl)carbamoyl)piperidine-1-carboxylate (320 mg) in MeOH ( 3 mL) was added HCl/Dioxane (2 mol/L, 5mL), the reaction was stirred for 30 minutes at room temperature. The reaction mixture was concentrated in vacuum to give the title compound (286 mg, 427 µmol, 97.9 % yield). MS (ESI, m/z): 634.0 [M+H]⁺.

### Step 5)

### tert-Butyl 3-[[4-[3-[[2-chloro-4-[[3-[4-(difluoromethoxy)-2,3-difluoro-phenyl]imidazo[1,2-a]pyrazin-8-yl]amino]benzoyl]amino]propylcarbamoyl]-1-piperidyl]methyl]pyrrolidine-1-carboxylate

To a solution of N-(3-(2-chloro-4-((3-(4-(difluoromethoxy)-2,3-difluorophenyl)imidazo[1,2-a]pyrazin-8-yl)amino)benzamido)propyl)piperidine-4-carboxamide hydrochloride ( 150 mg, 224 µmol, Eq: 1) in MeOH ( 3 mL) was added *tert-butyl* 3-formylpyrrolidine-1-carboxylate (134 mg, 671 µmol, Eq: 3) and sodium cyanoborohydride (28.1 mg, 447 µmol, Eq: 2). The reaction was stirred for two hours at room temperature. The reaction mixture was quenched with water and extracted in EtOAc. The organic layer was dried over anhydrous Na₂SO₄ and concentrated in vacuum. The residue was purified by column chromatography to give the title compound (90 mg, 110 µmol, 49.2 % yield) . MS (ESI, m/z): 817.0 [M+H]⁺.

### Step 6)

### tert-Butyl 3-[[4-[3-[[2-chloro-4-[[3-[4-(difluoromethoxy)-2,3-difluoro-phenyl]imidazo[1,2-a]pyrazin-8-yl]amino]benzoyl]amino]propylcarbamoyl]-1-methyl-piperidin-1-ium-1-yl]methyl]pyrrolidine-1-carboxylate iodide

To a solution of tert-butyl 3-((4-((3-(2-chloro-4-((3-(4-(difluoromethoxy)-2,3-difluorophenyl)imidazo[1,2-a]pyrazin-8-yl)amino)benzamido)propyl)carbamoyl)piperidin-1-yl)methyl)pyrrolidine-1-carboxylate ( 80 mg, 97.9 µmol, Eq: 1) in acetonitrile ( 3 mL) was added triethylamine (19.8 mg, 27.3 µL, 196 µmol, Eq: 2) and iodomethane (69.5 mg, 30.6 µL, 489 µmol, Eq: 5). The reaction was stirred for 16 hours at room temperature. The reaction mixture was concentrated in vacuum and the residue was directly used for the next step without further purification. MS (ESI, m/z): 831.1 [M]⁻.

### Step 7)

### N-[3-[[2-Chloro-4-[[3-[4-(difluoromethoxy)-2,3-difluoro-phenyl]imidazo[1,2-a]pyrazin-8-yl]amino]benzoyl]amino]propyl]-1-methyl-1-(pyrrolidin-3-ylmethyl)piperidin-1-ium-4-carboxamide formate

To a solution of tert-butyl 3-[[4-[3-[[2-chloro-4-[[3-[4-(difluoromethoxy)-2,3-difluorophenyl]imidazo[1,2-a]pyrazin-8-yl]amino]benzoyl]amino]propylcarbamoyl]-1-methyl-piperidin-1-ium-1-yl]methyl]pyrrolidine-1-carboxylate iodide (50 mg, 60.1 µmol, Eq: 1) in MeOH (3 mL) was added HCl/Dioxane (2 mol/L, 2 mL), the reaction was stirred for two hours at room temperature. The reaction mixture was concentrated in vacuum and the residue was purified by preparative HPLC to give the title compound (40 mg, 85.7 % yield). MS (ESI, m/z): 731.0 [M]⁺.

### Example 118

### 4-[[3-(2,3-Difluoro-4-pyrimidin-2-yloxy-phenyl)imidazo[1,2-a]pyrazin-8-yl]amino]-2-ethyl-N-[[1-methyl-1-(pyrrolidin-3-ylmethyl)piperidin-1-ium-4-yl]methyl]benzamide formate

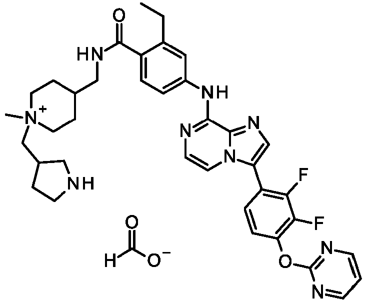

### Step 1) Intermediate 47

### tert-Butyl 4-[[[2-ethyl-4-[(3-iodoimidazo[1,2-a]pyrazin-8-yl)amino]benzoyl]amino]methyl]piperidine-1-carboxylate

To a solution of 2-ethyl-4-((3-iodoimidazo[1,2-a]pyrazin-8-yl)amino)benzoic acid (Intermediate 46, 300 mg, 735 µmol, Eq: 1) in DMF (6 mL) was added *tert-butyl* 4-(aminomethyl)piperidine-1-carboxylate (158 mg, 735 µmol, Eq: 1), 2-(3H-[1,2,3]triazolo[4,5-b]pyridin-3-yl)-1,1,3,3-tetramethylisouronium hexafluorophosphate(V) (559 mg, 1.47 mmol, Eq: 2) and triethylamine (223 mg, 307 µL, 2.2 mmol, Eq: 3), the reaction was stirred for 20 minutes at room temperature. The reaction mixture was poured into water and filtered. The filter cake was dried in vacuum to give the title compound (390mg, 645 µmol, 87.8 % yield). MS (ESI, m/z): 605.0 [M+H]⁺.

### Step 2)

### tert-Butyl 4-[[[4-[[3-(2,3-difluoro-4-pyrimidin-2-yloxy-phenyl)imidazo[1,2-a]pyrazin-8-yl]amino]-2-ethyl-benzoyl]amino]methyl]piperidine-1-carboxylate

To a solution of tert-butyl 4-((2-ethyl-4-((3-iodoimidazo[1,2-a]pyrazin-8-yl)amino)benzamido)methyl)piperidine-1-carboxylate (350 mg, 579 µmol, Eq: 1) in the mixture solvent of dioxane (5 mL) and water (1 mL) was added 2-(2,3-difluoro-4-(4,4,5,5-tetramethyl-1,3,2-dioxaborolan-2-yl)phenoxy)pyrimidine (232 mg, 695 µmol, Eq: 1.2), sodium carbonate (184 mg, 1.74 mmol, Eq: 3) and 1,1'-bis(di-*tert*-butylphosphino)ferrocene palladium dichloride (37.7 mg , 57.9 µmol, Eq: 0.1). The reaction was stirred for 30 minutes at 100 °C under microwave irriation and atmosphere of nitrogen. The reaction mixture was filtered and the filtrate was concentrated in vacuum. The residue was purified by column chromatography to give the title compound (290 mg, 424 µmol, 73.2 % yield). MS (ESI, m/z): 685.2 [M+H]⁺.

### Step 3)

### 4-[[3-(2,3-Difluoro-4-pyrimidin-2-yloxy-phenyl)imidazo[1,2-a]pyrazin-8-yl]amino]-2-ethyl-N-(4-piperidylmethyl)benzamide hydrochloride

To a solution of tert-butyl 4-((4-((3-(2,3-difluoro-4-(pyrimidin-2-yloxy)phenyl)imidazo[1,2-a]pyrazin-8-yl)amino)-2-ethylbenzamido)methyl)piperidine-1-carboxylate (300 mg, 438 µmol, Eq: 1) in MeOH ( 3 mL) was added HCl/dioxane (2 mol/L, 5 mL), the reaction was stirred for 30 minutes at room temperature. The reaction mixture was concentrated in vacuum to give the title compound (270 mg, 435 µmol, 99.3 % yield). MS (ESI, m/z): 585.0 [M+H]⁺.

### Step 4)

### tert-Butyl 3-[[4-[[[4-[[3-(2,3-difluoro-4-pyrimidin-2-yloxy-phenyl)imidazo[1,2-a]pyrazin-8-yl]amino]-2-ethyl-benzoyl]amino]methyl]-1-piperidyl]methyl]pyrrolidine-1-carboxylate

To a solution of 4-((3-(2,3-difluoro-4-(pyrimidin-2-yloxy)phenyl)imidazo[1,2-a]pyrazin-8-yl)amino)-2-ethyl-N-(piperidin-4-ylmethyl)benzamide hydrochloride (300 mg, 483 µmol, Eq: 1) in MeOH ( 3 mL) was added tert-butyl 3-formylpyrrolidine-1-carboxylate (289 mg, 1.45 mmol, Eq: 3) and sodium cyanoborohydride (60.7 mg, 966 µmol, Eq: 2), the reaction was stirred for two hours at room temperature. The reaction mixture was quenched with water and extracted in EtOAc. The organic layer was dried over anhydrous Na₂SO₄ and concentrated in vacuum. The residue was purified by column chromatography to give the title compound (200 mg, 260 µmol, 81.0 % yield). MS (ESI, m/z): 768.0 [M+H]⁺.

### Step 5)

### tert-Butyl 3-[[4-[[[4-[[3-(2,3-difluoro-4-pyrimidin-2-yloxy-phenyl)imidazo[1,2-a]pyrazin-8-yl]amino]-2-ethyl-benzoyl]amino]methyl]-1-methyl-piperidin-1-ium-1-yl]methyl]pyrrolidine-1-carboxylate iodide

To a solution of tert-butyl 3-((4-((4-((3-(2,3-difluoro-4-(pyrimidin-2-yloxy)phenyl)imidazo[1,2-a]pyrazin-8-yl)amino)-2-ethylbenzamido)methyl)piperidin-1-yl)methyl)pyrrolidine-1-carboxylate ( 200 mg, 260 µmol, Eq: 1) in MeOH ( 3 mL) was added iodomethane (185 mg, 81.4 µL, 1.3 mmol, Eq: 5) and TEA (79.1 mg, 109 µL, 781 µmol, Eq: 3). The reaction was stirred for 20 hours at room temperature. The reaction mixture was concentrated in vacuum and the residue was directly used for the next step without further purification. MS (ESI, m/z): 782.1 [M]⁺.

### Step 6)

### 4-[[3-(2,3-difluoro-4-pyrimidin-2-yloxy-phenyl)imidazo[1,2-a]pyrazin-8-yl]amino]-2-ethyl-N-[[1-methyl-1-(pyrrolidin-3-ylmethyl)piperidin-1-ium-4-yl]methyl]benzamide formate

To a tert-butyl 3-[[4-[[[4-[[3-(2,3-difluoro-4-pyrimidin-2-yloxy-phenyl)imidazo[1,2-a]pyrazin-8-yl]amino]-2-ethyl-benzoyl]amino]methyl]-1-methyl-piperidin-1-ium-1-yl]methyl]pyrrolidine-1-carboxylate iodide (100 mg, 117 µmol, Eq: 1) in MeOH ( 3 mL) was added HCl / dioxane (2 mol/L, 3mL), the reaction was stirred for 2 hours at room temperature. The reaction mixture was concentarted in vacuum and the residue was purified by preparative HPLC to give the title compound (25mg, 27.9 % yield). MS (ESI, m/z): 682.0 [M]⁺.

### Example 119

### [4-[[3-[4-(Difluoromethoxy)-2,3-difluoro-phenyl]imidazo[1,2-a]pyrazin-8-yl]amino]-2-ethylphenyl]-[4-methyl-4-(pyrrolidin-3-ylmethyl)piperazin-4-ium-1-yl]methanone formate

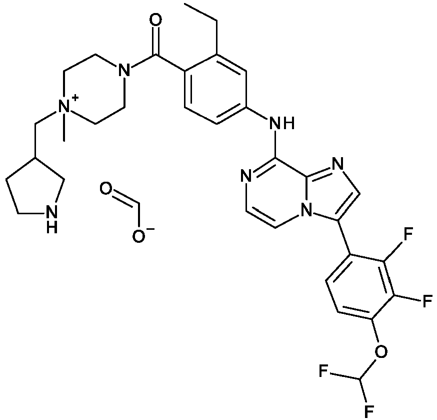

### Step 1)

### tert-Butyl 3-[[4-[4-[[3-[4-(difluoromethoxy)-2,3-difluoro-phenyl]imidazo[1,2-a]pyrazin-8-yl]amino]-2-ethyl-benzoyl]piperazin-1-yl]methyl]pyrrolidine-1-carboxylate

tert-butyl 4-(4-(4-((3-(4-(difluoromethoxy)-2,3-difluorophenyl)imidazo[1,2-a]pyrazin-8-yl)amino)-2-ethylbenzoyl)piperazine-1-carbonyl)piperidine-1-carboxylate (Intermediate 38, 148 mg, 200 µmol, Eq: 1) was dissolved in 2 mL dioxane. To a stirred 4M HCl dioxane solution was added the above solution dropwise. The stirring was continued for 1h after addition. LC-MS indicated the substrate was completely converted. The solvent was removed in vacuo, and the residue was azeotroped with toluene (5 mL) to remove residual HCl. The solid remained and *tert-butyl* 3-formylpyrrolidine-1-carboxylate (47.8 mg, 240 µmol, Eq: 1.2) were dissolved in Ethanol (90.9 µl) and CH₂Cl₂ (909 µl). To this stirred solution was added NaBH(OAc)₃ (50.9 mg, 240 µmol, Eq: 1.2) in one portion. The stirring was continued for 1h. After completion, silica gel was added, and the solvent was removed in vacuo. The residue was purified by flash chromatography (silica gel; MeOH:DCM = 0:1 to 1:10) to give the title compound as light yellow amorphous (141 mg, 99% yield). R*_{f}* = 0.6(MeOH:DCM = 1:10). MS (ESI, m/z): 712.1 [M+H]⁺

### Step 2)

### [4-[[3-[4-(Difluoromethoxy)-2,3-difluoro-phenyl]imidazo[1,2-a]pyrazin-8-yl]amino]-2-ethyl-phenyl]-[4-methyl-4-(pyrrolidin-3-ylmethyl)piperazin-4-ium-1-yl]methanone formate

To a solution of tert-butyl 3-((4-(4-((3-(4-(difluoromethoxy)-2,3-difluorophenyl)imidazo[1,2-a]pyrazin-8-yl)amino)-2-ethylbenzoyl)piperazin-1-yl)methyl)pyrrolidine-1-carboxylate (140 mg, 197 µmol, Eq: 1) in dry MeCN (983 µl) was added iodomethane (140 mg, 61.2 µl, 983 µmol, Eq: 5). The yellow solution was stirred overnight for 18h. The solvent was removed in vacuo, and the residue was dissolved in 3 mL 20% TFA/DCM solution (v/v), and the resulting solution was stirred for 1h at rt. The solvent was removed in vacuo, and the residue was dissolved in MeOH, and submitted for HPLC purification directly. The title compound was obtained as white amorphous freeze-dried solid (56.1 mg, 95% yield). MS (ESI, m/z): 626.5 [M]⁺

### Example 120

### Methyl (2S)-2-[[1-(azetidin-3-ylmethyl)-1-methyl-piperidin-1-ium-4-carbonyl]amino]-3-[[4-[[3-(2,3-difluoro-4-methoxy-phenyl)imidazo[1,2-a]pyrazin-8-yl]amino]-2-ethyl-benzoyl]amino]propanoate formate 2,2,2-trifluoroacetate (mixed salt)

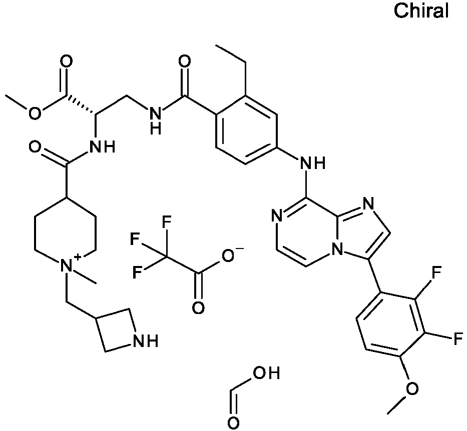

### Step 1)

### Methyl (2S)-3-amino-2-(9H-fluoren-9-ylmethoxycarbonylamino)propanoate

Methyl (S)-2-((((9H-fluoren-9-yl)methoxy)carbonyl)amino)-3-((*tert-*butoxycarbonyl)amino)propanoate (500 mg, 1.14 mmol, Eq: 1) was dissolved in 5 mL 20% (v/v) solution of TFA in DCM. The solution was stirred for 1h at rt and then concentrated in vacuo. The residue was used in the coming step without further purification, (386 mg). MS (ESI, m/z): 340.8 [M+H]⁺

### Step 2)

### methyl (2R)-3-[[2-ethyl-4-[(3-iodoimidazo[1,2-a]pyrazin-8-yl)amino]benzoyl]amino]-2-(9H-fluoren-9-ylmethoxycarbonylamino)propanoate

Methyl (S)-2-((((9H-fluoren-9-yl)methoxy)carbonyl)amino)-3-aminopropanoate (386 mg, 1.13 mmol, Eq: 1), 2-ethyl-4-((3-iodoimidazo[1,2-a]pyrazin-8-yl)amino)benzoic acid (Intermediate 34, 463 mg, 1.13 mmol, Eq: 1) and HATU (517 mg, 1.36 mmol, Eq: 1.2) was suspended in DMA (5.67 ml). N-ethyl-N-isopropylpropan-2-amine (733 mg, 988 µl, 5.67 mmol, Eq: 5) was injected in one time. The stirring was continued for 1h, then 50 mL water was added. The precipitate was collected by filtration and washed with water (20 mL X 3). The cake was dried in vacuo to afford the crude product which was purified by flash chromatography (silica gel; MeOH:DCM = 0:1 to 1:10) to get light yellow oil (414 mg, 50% yield). MS (ESI, m/z): 731.1 [M+H]⁺.

### Step 3)

### Methyl (2S)-3-[[4-[[3-(2,3-difluoro-4-methoxy-phenyl)imidazo[1,2-a]pyrazin-8-yl]amino]-2-ethyl-benzoyl]amino]-2-(9H-fluoren-9-ylmethoxycarbonylamino)propanoate

Methyl (S)-2-((((9H-fluoren-9-yl)methoxy)carbonyl)amino)-3-(2-ethyl-4-((3-iodoimidazo[1,2-a]pyrazin-8-yl)amino)benzamido)propanoate (414 mg, 567 µmol, Eq: 1), (2,3-difluoro-4-methoxyphenyl)boronic acid (213 mg, 1.13 mmol, Eq: 2), Pd-118 (18.5 mg, 28.3 µmol, Eq: 0.05) were placed in a microwave vial. Dioxane (2.83 ml) and potassium carbonate (567 µl, 1.7 mmol, Eq: 3) were added. The vial was sealed immediatly and degassed for 5 times. The mixture was heated at 60 °C under nitrogen atmosphere for 18h, The mixture was cooled to rt, poured into 100 mL water and extracted with EtOAc (80 mL X 3). The organic layers were combined, washed with 80 mL brine, dried over sodium sulfate, and concentrated in vacuo. The residue was purified by flash chromatography (silica gel; EtOAc:PE = 0;1 to 1:0) to get light yellow solid (202 mg, 47.7% yield) . R*_{f}*= 0.3 (EtOAc:PE = 1:1). MS (ESI, m/z): 747.3 [M+H]⁺.

### Step 4)

### tert-Butyl 4-[[(1S)-1-[[[4-[[3-(2,3-difluoro-4-methoxy-phenyl)imidazo[1,2-a]pyrazin-8-yl]amino]-2-ethyl-benzoyl]amino]methyl]-2-methoxy-2-oxo-ethyl]carbamoyl]piperidine-1-carboxylate

Methyl (S)-2-((((9H-fluoren-9-yl)methoxy)carbonyl)amino)-3-(4-((3-(2,3-difluoro-4-methoxyphenyl)imidazo[1,2-a]pyrazin-8-yl)amino)-2-ethylbenzamido)propanoate (200 mg, 268 µmol, Eq: 1) was dissolved in 5 mL MeCN and diethylamine (v/v = 4:1). The solution was stirred at rt for 1h. The solvent was removed in vacuo, and the residue was used in the next coupling reaction without further purification. The above residue, 1-(*tert-*butoxycarbonyl)piperidine-4-carboxylic acid (73.7 mg, 321 µmol, Eq: 1.2) and HATU (122 mg, 321 µmol, Eq: 1.2) was suspended in DMA (1.34 ml). N-ethyl-N-isopropylpropan-2-amine (173 mg, 233 µl, 1.34 mmol, Eq: 5) was injected in one time. The stirring was continued for 1h, and then 50 mL water was added. The precipitate was collected by filtration and washed with water (20 mL X 3). The cake was dried in vacuo to afford the crude product which was purified by flash chromatography (silica gel; EtOAc:PE = 1:1 to 1:0) to get light yellow foam (147 mg, 74.6% yield). MS (ESI, m/z): 736.5 [M+H]⁺.

### Step 5)

### tert-Butyl 3-[[4-[[(1S)-1-[[[4-[[3-(2,3-difluoro-4-methoxy-phenyl)imidazo[1,2-a]pyrazin-8-yl]amino]-2-ethyl-benzoyl]amino]methyl]-2-methoxy-2-oxo-ethyl]carbamoyl]-1-piperidyl]methyl]azetidine-1-carboxylate

*tert*-Butyl (S)-4-((3-(4-((3-(2,3-difluoro-4-methoxyphenyl)imidazo[1,2-a]pyrazin-8-yl)amino)-2-ethylbenzamido)-1-methoxy-1-oxopropan-2-yl)carbamoyl)piperidine-1-carboxylate (147 mg, 200 µmol, Eq: 1) was dissolved in 3 mL 20% TFA/DCM (v/v), and stirred at rt for 0.5 h. The solvent was removed in vacuo, and the residue was suspended in 100 mL 1N NaOH, extracted with DCM/MeOH (v/v = 8:1) (80 mL X 3). Combined the organic layers, washed with 80 mL brine, dried over sodium sulfate and concentrated in vacuo. The above residue and *tert-*butyl 3-formylazetidine-1-carboxylate (44.4 mg, 240 µmol, Eq: 1.2) were dissolved in dry THF (2 ml). To this solution was added NaBH(OAc)₃ (63.5 mg, 300 µmol, Eq: 1.5) in one portion. The mixture was stirred at rt for 30 min, and LC-MS indicated a complete conversion. Silica gel was added, and the solvent was removed in vacuo to get the silica gel suported sample. The sample was purified by flash chromatography (MeOH:DCM = 0:1 to 1:10) to give the product as yellow gum (133 mg, 82.7% yield). MS (ESI, m/z): 705.5 [M-Boc+H]⁺

### Step 6)

### Methyl (2S)-2-[[1-(azetidin-3-ylmethyl)-1-methyl-piperidin-1-ium-4-carbonyl]amino]-3-[[4-[[3-(2,3-difluoro-4-methoxy-phenyl)imidazo[1,2-a]pyrazin-8-yl]amino]-2-ethyl-benzoyl]amino]propanoate formate 2,2,2-trifluoroacetate (mixed salt)

To a solution of tert-butyl (S)-3-((4-((3-(4-((3-(2,3-difluoro-4-methoxyphenyl)imidazo[1,2-a]pyrazin-8-yl)amino)-2-ethylbenzamido)-1-methoxy-1-oxopropan-2-yl)carbamoyl)piperidin-1-yl)methyl)azetidine-1-carboxylate (133 mg, 165 µmol, Eq: 1) in dry MeCN (826 µl) was added iodomethane (117 mg, 51.4 µl, 826 µmol, Eq: 5) and 4 drops of DIPEA. The yellow solution was stirred overnight for 18 h. The solvent was removed in vacuo, and the residue was dissolved in 3 mL 20% TFA/DCM solution (v/v), and the resulting solution was stirred for 1h at rt. The solvent was removed in vacuo, and the residue was dissolved in MeCN, and submitted for HPLC purification directly. The title compound was obtained as light yellow amorphous freeze-dried solid (28 mg, 19.3% yield). MS (ESI, m/z): 719.2 [M]⁺

### Example 121

### Methyl (2S)-2-[[1-(azetidin-3-ylmethyl)-1-methyl-piperidin-1-ium-4-carbonyl]amino]-4-[[4-[[3-(2,3-difluoro-4-methoxy-phenyl)imidazo[1,2-a]pyrazin-8-yl]amino]-2-ethyl-benzoyl]amino]butanoate formate 2,2,2-trifluoroacetate (mixed salt)

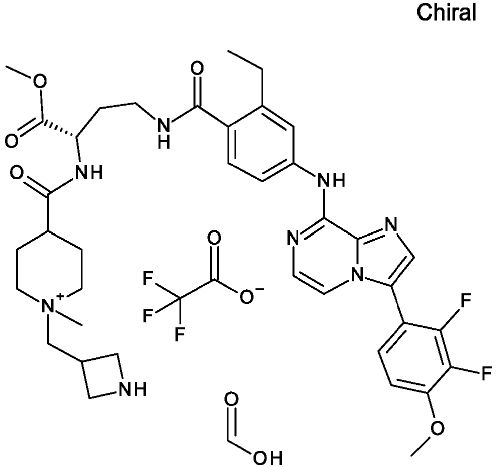

The title compound was prepared in analogy to example 120 steps 1-6 by using methyl (S)-2-((((9H-fluoren-9-yl)methoxy)carbonyl)amino)-4-((tert-butoxycarbonyl)amino)butanoate in step 1 and was obtained as white amorphous freeze dried solid (28.5 mg, 12.3% yield). MS (ESI, m/z): 733.1 [M]⁺

### Example 122

### Methyl (2S)-3-[[1-(azetidin-3-ylmethyl)-1-methyl-piperidin-1-ium-4-carbonyl]amino]-2-[[4-[[3-(2,3-difluoro-4-methoxy-phenyl)imidazo[1,2-a]pyrazin-8-yl]amino]-2-ethyl-benzoyl]amino]propanoate formate 2,2,2-trifluoroacetate (mixed salt)

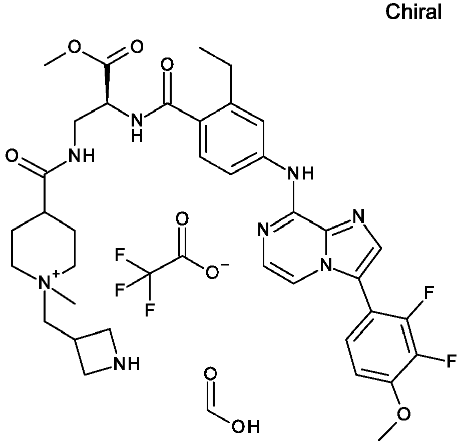

### Step 1)

### Methyl (2S)-3-(tert-butoxycarbonylamino)-2-(9H-fluoren-9-ylmethoxycarbonylamino)propanoate

(S)-2-((((9H-fluoren-9-yl)methoxy)carbonyl)amino)-3-((*tert*-butoxycarbonyl)amino)propanoic acid (1 g, 2.34 mmol, Eq: 1) was combined with finely ground potassium carbonate (648 mg, 4.69 mmol, Eq: 2) in dry DMA (11.7 ml). The resulting mixture was cooled to 0 °C in an ice bath. iodomethane (666 mg, 292 µl, 4.69 mmol, Eq: 2) was added slowly via syringe, and the resulting solution was stirred for 3 hours. Water (10 mL) was added, and a cloudy white mixture was obtained. The mixture was extracted with EtOAc (3 x 70 mL), and the organic layer was washed with saturated NaCl solution (1 x 50 mL). Next, the organic layer was dried over Na₂SO₄ and evaporated under reduced pressure to afford the product as a yellow solid (1.0 g, 97% yield). MS (ESI, m/z): 441.0 [M+H]⁺

### Step 2)

### Methyl (2S)-2-amino-3-(tert-butoxycarbonylamino)propanoate

Methyl (S)-2-((((9H-fluoren-9-yl)methoxy)carbonyl)amino)-3-((*tert-*butoxycarbonyl)amino)propanoate (500 mg, 1.14 mmol, Eq: 1) was dissolved in 10 mL 20% solution of diethylamine in MeCN. The solution was stirred for 1h at rt and then concentrated in vacuo and the residue (248 mg, 100% yield) was used directly in the following coupling reaction. MS (ESI, m/z): 219.3 [M+H]⁺

### Step 3)

### Methyl (2R)-3-(tert-butoxycarbonylamino)-2-[[2-ethyl-4-[(3-iodoimidazo[1,2-a]pyrazin-8-yl)amino]benzoyl]amino]propanoate

Methyl (S)-2-amino-3-((*tert*-butoxycarbonyl)amino)propanoate (248 mg, 1.14 mmol, Eq: 1), 2-ethyl-4-((3-iodoimidazo[1,2-a]pyrazin-8-yl)amino)benzoic acid (464 mg, 1.14 mmol, Eq: 1) and HATU (518 mg, 1.36 mmol, Eq: 1.2) was suspended in DMA (5.68 ml). N-ethyl-N-isopropylpropan-2-amine (734 mg, 990 µl, 5.68 mmol, Eq: 5) was injected in one time. The stirring was continued for 1h, and then 50 mL water was added. The precipitate was collected by filtration and washed with water (20 mL X 3). The cake was dried in vacuo to afford the crude product which was purified by flash chromatography (silica gel; MeOH:DCM = 0:1 to 1:10) to get yellow foam (202 mg, 29.2% yield). MS (ESI, m/z): 609.1 [M+H]⁺

### Step 4)

### Methyl (2S)-3-(tert-butoxycarbonylamino)-2-[[4-[[3-(2,3-difluoro-4-methoxyphenyl)imidazo[1,2-a]pyrazin-8-yl]amino]-2-ethyl-benzoyl]amino]propanoate

Methyl (S)-3-((*tert*-butoxycarbonyl)amino)-2-(2-ethyl-4-((3-iodoimidazo[1,2-a]pyrazin-8-yl)amino)benzamido)propanoate (394 mg, 648 µmol, Eq: 1), (2,3-difluoro-4-methoxyphenyl)boronic acid (183 mg, 971 µmol, Eq: 1.5), Pd-118 (21.1 mg, 32.4 µmol, Eq: 0.05) were placed in a microwave vial. Dioxane (3.24 ml) and potassium carbonate (648 µl, 1.94 mmol, Eq: 3) were added. The vial was sealed immediatly and degassed for 5 times. The mixture was heated at 50 °C under nitrogen atmosphere for 8 h, The mixture was cooled to rt, poured into 100 mL water and extracted with EtOAc (80 mL X 3). The organic layers were combined, washed with 80 mL brine, dried over sodium sulfate, and concentrated in vacuo. The residue was purified by flash chromatography (silica gel; EtOAc:PE = 0; 1 to 1:0) to get yellow oil (237 mg, 58.6% yield). R*_{f}* = 0.3 (EtOAc:PE = 1:1). MS (ESI, m/z): 625.4 [M+H]⁺

### Step 5)

### tert-Butyl 4-[[(2S)-2-[[4-[[3-(2,3-difluoro-4-methoxy-phenyl)imidazo[1,2-a]pyrazin-8-yl]amino]-2-ethyl-benzoyl]amino]-3-methoxy-3-oxo-propyl]carbamoyl]piperidine-1-carboxylate

Methyl (S)-3-((*tert*-butoxycarbonyl)amino)-2-(4-((3-(2,3-difluoro-4-methoxyphenyl)imidazo[1,2-a]pyrazin-8-yl)amino)-2-ethylbenzamido)propanoate (237 mg, 379 µmol, EQ: 1.0), 1-(*tert*-butoxycarbonyl)piperidine-4-carboxylic acid (104 mg, 455 µmol, Eq: 1.2) and HATU (173 mg, 455 µmol, Eq: 1.2) were suspended in DMA (1.9 ml). N-ethyl-N-isopropylpropan-2-amine (245 mg, 330 µl, 1.9 mmol, Eq: 5) was injected in one time. The stirring was continued for 1h, and then 50 mL water was added. The precipitate was collected by filtration and washed with water (20 mL X 3). The cake was dried in vacuo to afford the crude product which was purified by flash chromatography (silica gel; MeOH:DCM = 0:1 to 1:10) to get yellow oil (279 mg, 99% yield). MS (ESI, m/z): 736.1 [M+H].

### Step 6)

### tert-Butyl 3-[[4-[[(2S)-2-[[4-[[3-(2,3-difluoro-4-methoxy-phenyl)imidazo[1,2-a]pyrazin-8-yl]amino]-2-ethyl-benzoyl]amino]-3-methoxy-3-oxo-propyl]carbamoyl]-1-piperidyl]methyl]azetidine-1-carboxylate

*tert-*Butyl (S)-4-((2-(4-((3-(2,3-difluoro-4-methoxyphenyl)imidazo[1,2-a]pyrazin-8-yl)amino)-2-ethylbenzamido)-3-methoxy-3-oxopropyl)carbamoyl)piperidine-1-carboxylate (279 mg, 379 µmol, Eq: 1) was dissolved in 3 mL 20% TFA/DCM (v/v), and stirred at rt for 0.5h. LC-MS indicated a complete conversion of the substrate. The solvent was removed in vacuo, and the residue was suspended in 100 mL 1N NaOH, extracted with DCM/MeOH (v/v = 8:1) (80 mL X 3). The organic layers were combined, washed with 80 mL brine, dried over sodium sulfate and concentrated in vacuo. The above residue and *tert-*butyl 3-formylazetidine-1-carboxylate (84.3 mg, 455 µmol, Eq: 1.2) were dissolved in dry THF (3.79 ml). To this solution was added NaBH(OAc)₃ (121 mg, 569 µmol, Eq: 1.5) in one portion. The mixture was stirred at rt for 30 min. Silica gel was added, and the solvent was removed in vacuo to get the silica gel suported sample. The sample was purified by flash chromatography (MeOH:DCM = 0:1 to 1:10) to get yellow foam (220 mg, 72% yield). MS (ESI, m/z): 403.3 [M+H]²⁺.

### Step 7)

### Methyl (2S)-3-[[1-(azetidin-3-ylmethyl)-1-methyl-piperidin-1-ium-4-carbonyl]amino]-2-[[4-[[3-(2,3-difluoro-4-methoxy-phenyl)imidazo[1,2-a]pyrazin-8-yl]amino]-2-ethyl-benzoyl]amino]propanoate formate 2,2,2-trifluoroacetate (mixed salt)

To a solution of tert-butyl (S)-3-((4-((2-(4-((3-(2,3-difluoro-4-methoxyphenyl)imidazo[1,2-a]pyrazin-8-yl)amino)-2-ethylbenzamido)-3-methoxy-3-oxopropyl)carbamoyl)piperidin-1-yl)methyl)azetidine-1-carboxylate (220 mg, 273 µmol, Eq: 1) in dry MeCN (1.37 ml) was added iodomethane (194 mg, 85.1 µl, 1.37 mmol, Eq: 5) and 4 drops of DIPEA. The yellow solution was stirred for 18h. The solvent was removed in vacuo, and the residue was dissolved in 3 mL 20% TFA/DCM solution (v/v), and the resulting solution was stirred for 1h at rt. The solvent was removed in vacuo, and the residue was dissolved in MeCN, and submitted for HPLC purification directly. The title compound was obtained as light yellow amorphous freeze-dried solid (39.8 mg, 16.4% yield). MS (ESI, m/z): 719.1 [M]⁺

### Example 123

### Methyl (2S)-2-[[1-(azetidin-3-ylmethyl)-1-methyl-piperidin-1-ium-4-carbonyl]amino]-4-[[4-[[3-(2,3-difluoro-4-methoxy-phenyl)imidazo[1,2-a]pyrazin-8-yl]amino]-2-ethyl-benzoyl]amino]butanoate formate 2,2,2-trifluoroacetate (mixed salt)

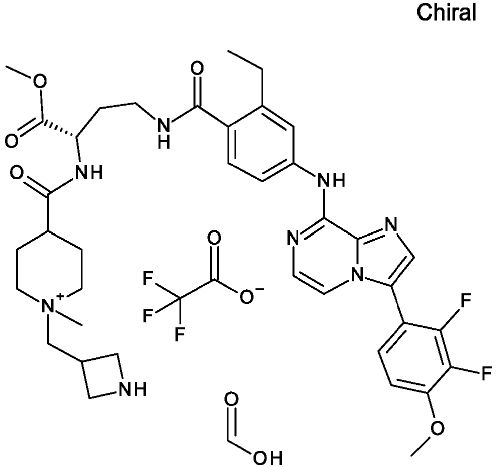

The title compound was prepared in analogy to example 122 steps 1-7 by using (S)-2-((((9H-fluoren-9-yl)methoxy)carbonyl)amino)-4-((tert-butoxycarbonyl)amino)butanoic acid in step 1 and was obtained as light yellow amorphous freeze dried solid (45.9 mg, 19.8% yield). MS (ESI, m/z): 733.1 [M]⁺

### Example 124

### Methyl 1-[1-(azetidin-3-ylmethyl)-1-methyl-piperidin-1-ium-4-carbonyl]-4-[[[4-[[3-(2,3-difluoro-4-methoxy-phenyl)imidazo[1,2-a]pyrazin-8-yl]amino]-2-ethyl-benzoyl]amino]methyl]piperidine-4-carboxylate formate 2,2,2-trifluoroacetate (mixed salt)

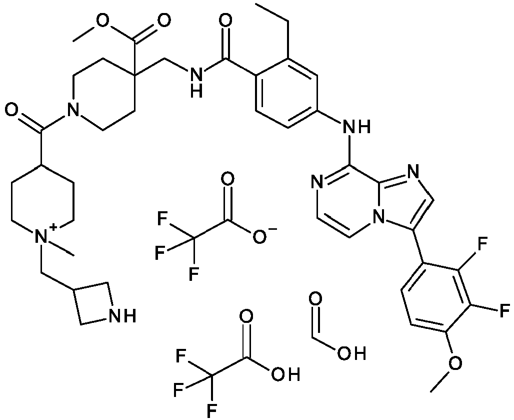

### Step 1)

### O1-tert-butyl O4-methyl 4-[[[4-[[3-(2,3-difluoro-4-methoxy-phenyl)imidazo[1,2-a]pyrazin-8-yl]amino]-2-ethyl-benzoyl]amino]methyl]piperidine-1,4-dicarboxylate

4-((3-(2,3-difluoro-4-methoxyphenyl)imidazo[1,2-a]pyrazin-8-yl)amino)-2-ethylbenzoic acid (Intermediate 8, 500 mg, 1.18 mmol, Eq: 1) and 1-(*tert*-butyl) 4-methyl 4-(aminomethyl)piperidine-1,4-dicarboxylate (385 mg, 1.41 mmol, Eq: 1.2) were dissolved in dry DMA (11.8 ml). To this solution was added HATU (672 mg, 1.77 mmol, Eq: 1.5) in one portion. The mixture was stirred at rt for 30 min. The solution was poured into 100 mL water and extracted with EtOAc (50 mL X 3). The organic layers were combined, washed with 100 mL brine, dried over sodium sulfate and concentrated in vacuo. The 800 mg yellow oily crude was used in the coming step without further purification. MS (ESI, m/z): 679.2 [M+H]⁺

### Step 2)

### Methyl 1-(1-tert-butoxycarbonylpiperidine-4-carbonyl)-4-[[[4-[[3-(2,3-difluoro-4-methoxyphenyl)imidazo[1,2-a]pyrazin-8-yl]amino]-2-ethyl-benzoyl]amino]methyl]piperidine-4-carboxylate

1-(tert-butyl) 4-methyl 4-((4-((3-(2,3-difluoro-4-methoxyphenyl)imidazo[1,2-a]pyrazin-8-yl)amino)-2-ethylbenzamido)methyl)piperidine-1,4-dicarboxylate (400 mg, 589 µmol, Eq: 1), 1-(tert-butoxycarbonyl)piperidine-4-carboxylic acid (162 mg, 707 µmol, Eq: 1.2) and HATU (269 mg, 707 µmol, Eq: 1.2) was suspended in DMA (2.95 ml). N-ethyl-N-isopropylpropan-2-amine (381 mg, 513 µl, 2.95 mmol, Eq: 5) was injected in one time. The stirring was continued for 1h, and then 50 mL water was added. The solution was poured into 100 mL water and extracted with EtOAc (50 mL X 3). The organic layers were combined, washed with 100 mL brine, dried over sodium sulfate and concentrated in vacuo. The crude yellow oil was used in the coming step without further purification, 466 mg, 100%. MS (ESI, m/z): 790.0 [M+H]⁺

### Step 3)

### Methyl 1-[1-[(1-tert-butoxycarbonylazetidin-3-yl)methyl]piperidine-4-carbonyl]-4-[[[4-[[3-(2,3-difluoro-4-methoxy-phenyl)imidazo[1,2-a]pyrazin-8-yl]amino]-2-ethyl-benzoyl]amino]methyl]piperidine-4-carboxylate

*tert*-Butyl 4-(4-((4-((3-(2,3-difluoro-4-methoxyphenyl)imidazo[1,2-a]pyrazin-8-yl)amino)-2-ethylbenzamido)methyl)-4-(methoxycarbonyl)piperidine-1-carbonyl)piperidine-1-carboxylate (466 mg, 590 µmol, Eq: 1) was dissolved in 3 mL 20% TFA/DCM (v/v), and stirred at rt for 0.5h. LC-MS indicated a complete conversion of the substrate. The solvent was removed in vacuo, and the residue was suspended in 100 mL 1N NaOH, extracted with DCM/MeOH (v/v = 8:1) (80 mL X 3). The organic layers were combined, washed with 80 mL brine, dried over sodium sulfate and concentrated in vacuo. The above residue and *tert*-butyl 3-formylazetidine-1-carboxylate (131 mg, 708 µmol, Eq: 1.2) were dissolved in dry THF (5.9 ml). To this solution was added NaBH(OAc)₃ (188 mg, 885 µmol, Eq: 1.5) in one portion. The mixture was stirred at rt for 30 min, and LC-MS indicated a complete conversion. Silica gel was added, and the solvent was removed in vacuo to get the silica gel supported sample. The sample was purified by flash chromatography (MeOH:DCM = 0:1 to 1:10) to get light yellow foam (330 mg, 65% yield). MS (ESI, m/z): 430.5 [M+H]²⁺

### Step 4)

### tert-Butyl 3-[[4-[[(2S)-2-[[4-[[3-(2,3-difluoro-4-methoxy-phenyl)imidazo[1,2-a]pyrazin-8-yl]amino]-2-ethyl-benzoyl]amino]-3-methoxy-3-oxo-propyl]carbamoyl]-1-piperidyl]methyl]azetidine-1-carboxylate formate 2,2,2-trifluoroacetate (mixed salt)

To a solution of methyl 1-(1-((1-(*tert*-butoxycarbonyl)azetidin-3-yl)methyl)piperidine-4-carbonyl)-4-((4-((3-(2,3-difluoro-4-methoxyphenyl)imidazo[1,2-a]pyrazin-8-yl)amino)-2-ethylbenzamido)methyl)piperidine-4-carboxylate (330 mg, 384 µmol, Eq: 1) in dry acetonitrile (19.2 ml) was added iodomethane (273 mg, 120 µl, 1.92 mmol, Eq: 5) and 4 drops of DIPEA. The yellow solution was stirred overnight for 18h. The solvent was removed in vacuo, and the residue was dissolved in 3 mL 20% TFA/DCM solution (v/v), and the resulting solution was stirred for 1h at rt. The solvent was removed in vacuo, and the residue was dissolved in MeCN, and submitted for HPLC purification directly. The title compound was obtained as white amorphous freeze-dried solid (74 mg, 16.3% yield). MS (ESI, m/z): 773.2 [M]⁺

### Example 125

### Methyl 2-[1-[1-(azetidin-3-ylmethyl)-1-methyl-piperidin-1-ium-4-carbonyl]-4-piperidyl]-2-[[4-[[3-(2,3-difluoro-4-methoxy-phenyl)imidazo[1,2-a]pyrazin-8-yl]amino]-2-ethyl-benzoyl]amino]acetate formate 2,2,2-trifluoroacetate (mixed salt)

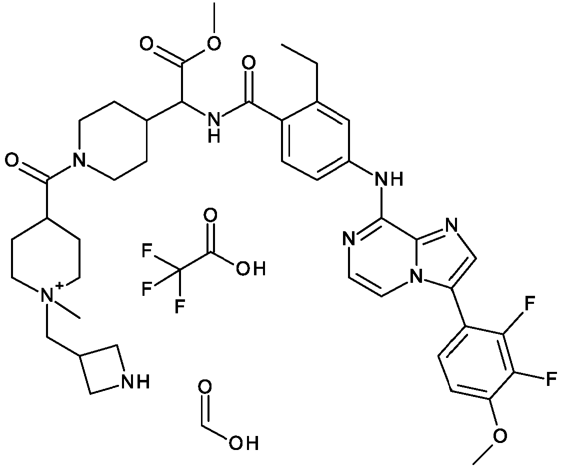

The title compound was prepared in analogy to example 124 steps 1-4 by using *tert*-butyl 4-(1-amino-2-methoxy-2-oxoethyl)piperidine-1-carboxylate in step 1 and was obtained as white amorphous freeze dried solid (36.0 mg, 19.7% yield). MS (ESI, m/z): 733.2 [M]⁺

### Example 126

### 1-(Azetidin-3-ylmethyl)-N-[3-[[4-[[3-(2,3-difluoro-4-methoxy-phenyl)imidazo[1,2-a]pyrazin-8-yl]amino]-2-ethyl-benzoyl]amino]cyclobutyl]-1-methyl-piperidin-1-ium-4-carboxamide formate 2,2,2-trifluoroacetate (mixed salt)

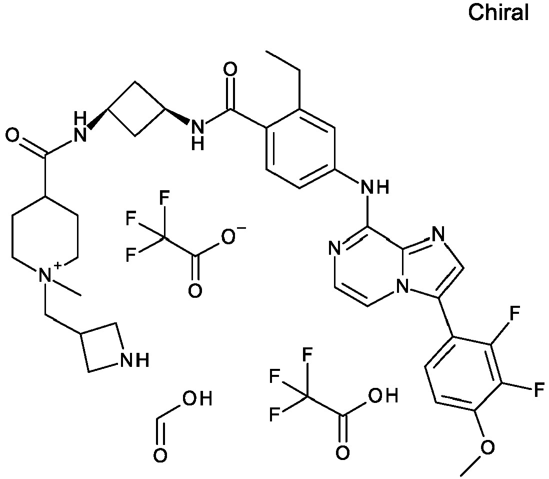

The title compound was prepared in analogy to example 124 steps 1-4 by using tert-butyl ((1s,3s)-3-aminocyclobutyl)carbamate in step 1 and was obtained as white amorphous freeze dried solid (32.4 mg, 38.1% yield). MS (ESI, m/z): 687.3 [M]⁺

### Example 127

### 1-(Azetidin-3-ylmethyl)-N-[3-[[[4-[[3-(2,3-difluoro-4-methoxy-phenyl)imidazo[1,2-a]pyrazin-8-yl]amino]-2-ethyl-benzoyl]amino]methyl]cyclobutyl]-1-methyl-piperidin-1-ium-4-carboxamide formate 2,2,2-trifluoroacetate (mixed salt)

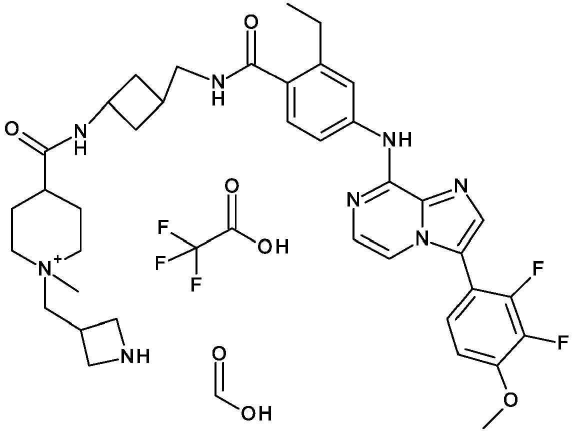

The title compound was prepared in analogy to example 124 steps 1-4 by using *tert*-butyl (3-(aminomethyl)cyclobutyl)carbamate in step 1 and was obtained as white amorphous freeze dried solid (36.5 mg, 37.2% yield). MS (ESI, m/z): 701.0 [M]⁺

### Example 128

### 2-[1-(Azetidin-3-ylmethyl)-4-[[[4-[[3-(2,3-difluoro-4-methoxy-phenyl)imidazo[1,2-a]pyrazin-8-yl]amino]-2-ethyl-benzoyl]amino]methyl]piperidin-1-ium-1-yl]acetic acid bis(2,2,2-trifluoroacetate)

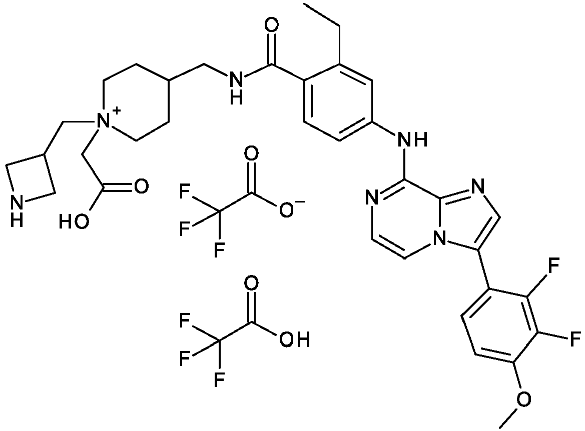

### Step 1)

### tert-Butyl 4-[[[4-[[3-(2,3-difluoro-4-methoxy-phenyl)imidazo[1,2-a]pyrazin-8-yl]amino]-2-ethyl-benzoyl]amino]methyl]piperidine-1-carboxylate

In a 100 mL round-bottomed flask, 4-((3-(2,3-difluoro-4-methoxyphenyl)imidazo[1,2-a]pyrazin-8-yl)amino)-2-ethylbenzoic acid (277 mg, 653 µmol, Eq: 1), *tert*-butyl 4-(aminomethyl)piperidine-1-carboxylate (154 mg, 718 µmol, Eq: 1.1) and DIPEA (253 mg, 342 µl, 1.96 mmol, Eq: 3) were combined with DMF (5 ml) to give a light brown solution. HATU (298 mg, 783 µmol, Eq: 1.2) was added. The reaction was stirred at room temperature for 1h. The reaction mixture was poured into 25 mL H₂O and extracted with EtOAc (3 x 25 mL). The organic layers were combined, washed with sat NaCl (1 x 25 mL). The organic layers were dried over Na₂SO₄ and concentrated in vacuo to afford the title compound (400 mg, 644 µmol, 98.7 % yield). MS (ESI, m/z): 621.0 [M+H]⁺

### Step 2)

### 4-[[3-(2,3-Difluoro-4-methoxy-phenyl)imidazo[1,2-a]pyrazin-8-yl]amino]-2-ethyl-N-(4-piperidylmethyl)benzamide

In a 50 mL round-bottomed flask, *tert*-butyl 4-((4-((3-(2,3-difluoro-4-methoxyphenyl)imidazo[1,2-a]pyrazin-8-yl)amino)-2-ethylbenzamido)methyl)piperidine-1-carboxylate (405 mg, 652 µmol, Eq: 1) was combined with THF (4 ml) to give a light yellow solution. HCl (in water) (2.17 ml, 26.1 mmol, Eq: 40) was added. The reaction was stirred at room temperature for 30min. The crude reaction mixture was concentrated in vacuo. The obtained crude title compound was used without further purification (340 mg, 653 µmol, 100 % yield). MS (ESI, m/z): 521.2 [M+H]⁺

### Step 3)

### tert-Butyl 3-[[4-[[[4-[[3-(2,3-difluoro-4-methoxy-phenyl)imidazo[1,2-a]pyrazin-8-yl]amino]-2-ethyl-benzoyl]amino]methyl]-1-piperidyl]methyl]azetidine-1-carboxylate

In a 50 mL round-bottomed flask, 4-((3-(2,3-difluoro-4-methoxyphenyl)imidazo[1,2-a]pyrazin-8-yl)amino)-2-ethyl-N-(piperidin-4-ylmethyl)benzamide (90 mg, 173 µmol, Eq: 1), *tert*-butyl 3-formylazetidine-1-carboxylate (48 mg, 259 µmol, Eq: 1.5) and NaBH₃CN (43.5 mg, 692 µmol, Eq: 4) were combined with MeOH (5 ml) to give a light yellow solution. The reaction mixture was heated to 50 °C for 2 h. The crude reaction mixture was concentrated in vacuo. The reaction mixture was poured into 50 mL sat. NaHCO₃ and extracted with EtOAc (3 x 25 mL). The organic layers were combined, washed with sat NaCl (1 x 25 mL). The organic layers were dried over Na₂SO₄ and concentrated in vacuo to afford the title compound (119 mg, 173 µmol, 99.8 % yield). MS (ESI, m/z): 690.2 [M+H]⁺

### Step 4)

### 2-[1-(Azetidin-3-ylmethyl)-4-[[[4-[[3-(2,3-difluoro-4-methoxy-phenyl)imidazo[1,2-a]pyrazin-8-yl]amino]-2-ethyl-benzoyl]amino]methyl]piperidin-1-ium-1-yl]acetic acid bis(2,2,2-trifluoroacetate)

The crude *tert*-butyl 3-((4-((4-((3-(2,3-difluoro-4-methoxyphenyl)imidazo[1,2-a]pyrazin-8-yl)amino)-2-ethylbenzamido)methyl)piperidin-1-yl)methyl)azetidine-1-carboxylate (70 mg, 101 µmol, Eq: 1) was dissolved in acetonitrile (1.01 ml). *tert*-Butyl 2-bromoacetate (198 mg, 1.01 mmol, Eq: 10) was added along with 4 drops of DIPEA. The solution was stirred at rt for 30 h. The solvent was removed in vacuo, and the residue was dissolved in 20%(v) TFA/DCM solution (10 mL) and stirred for 4h at rt. The solvent was removed in vacuo, and the residue was purified by preparative HPLC. The title compound was obtained as white amorphous freeze-dried solid (45.6 mg, 45.9% yield). MS (ESI, m/z): 648.7 [M]⁺

### Example 129

### 1-(Azetidin-3-ylmethyl)-N-[3-[[4-[[3-(2,3-difluoro-4-methoxy-phenyl)imidazo[1,2-a]pyrazin-8-yl]amino]-2-ethyl-benzoyl]amino]propyl]-1-methyl-piperidin-1-ium-4-carboxamide bis(trifluoroacetate)

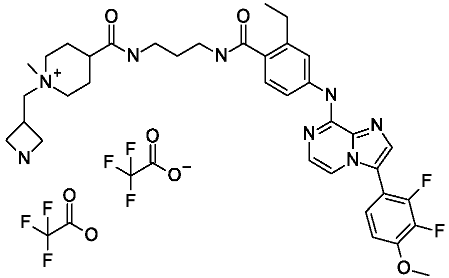

### Step 1)

### tert-Butyl N-[3-[[4-[[3-(2,3-difluoro-4-methoxy-phenyl)imidazo[1,2-a]pyrazin-8-yl]amino]-2-ethyl-benzoyl]amino]propyl]carbamate

In a 100 mL round-bottomed flask, 4-((3-(2,3-difluoro-4-methoxyphenyl)imidazo[1,2-a]pyrazin-8-yl)amino)-2-ethylbenzoic acid (200 mg, 471 µmol), *tert-*butyl (3-aminopropyl)carbamate (90 mg, 518 µmol) and DIPEA (183 mg, 247 µl, 1.41 mmol) were combined with DMF (5 mL) to give a light brown solution. HATU (215 mg, 565 µmol) was added. The reaction was stirred at room temperature for 1h. The reaction mixture was poured into 25 mL H₂O and extracted with EtOAc (3 x 25 mL). The organic layers were combined, washed with sat NaCl (1 x 25 mL). The organic layers were dried over Na₂SO₄ and concentrated in vacuum. The crude material was purified by flash chromatography (silica gel, 12 g, 0% to 5% MeOH in DCM) to afford the title compound (260 mg, 448 µmol, 95 % yield). MS (ESI, m/z): 581.2 [M+H]⁺.

### Step 2)

### N-(3-Aminopropyl)-4-[[3-(2,3-difluoro-4-methoxy-phenyl)imidazo[1,2-a]pyrazin-8-yl]amino]-2-ethyl-benzamide

In a 100 mL round-bottomed flask, *tert*-butyl (3-(4-((3-(2,3-difluoro-4-methoxyphenyl)imidazo[1,2-a]pyrazin-8-yl)amino)-2-ethylbenzamido)propyl)carbamate (260 mg, 448 µmol) was combined with THF (4 mL) to give a light brown solution. HCl (in water) (1.87 mL, 22.4 mmol) was added. The reaction was stirred at room temperature for 1h. The crude reaction mixture was concentrated in vacuum to afford the title compound (215 mg, 447 µmol, 99.9 % yield). MS (ESI, m/z): 481.2 [M+H]⁺.

### Step 3)

### tert-Butyl 4-[3-[[4-[[3-(2,3-difluoro-4-methoxy-phenyl)imidazo[1,2-a]pyrazin-8-yl]amino]-2-ethyl-benzoyl]amino]propylcarbamoyl]piperidine-1-carboxylate

In a 50 mL round-bottomed flask, N-(3-aminopropyl)-4-((3-(2,3-difluoro-4-methoxyphenyl)imidazo[1,2-a]pyrazin-8-yl)amino)-2-ethylbenzamide (70 mg, 146 µmol), 1-(*tert*-butoxycarbonyl)piperidine-4-carboxylic acid (43.4 mg, 189 µmol), 2-(3H-[1,2,3]triazolo[4,5-b]pyridin-3-yl)-1,1,3,3-tetramethylisouronium hexafluorophosphate(V) (72 mg, 189 µmol) and DIPEA (56.5 mg, 76 µl, 437 µmol) were combined with DMF (3 mL) to give a light yellow solution. The reaction was stirred at room temperature for 1h. The reaction mixture was poured into 25 mL H₂O and extracted with EtOAc (3 x 25 mL). The organic layers were combined, washed with sat NaCl (1 x 25 mL). The organic layers were dried over Na₂SO₄ and concentrated in vacuum to afford the title compound (101 mg, 146 µmol, 100 % yield). MS (ESI, m/z): 692.2 [M+H]⁺.

### Step 4)

### N-[3-[[4-[[3-(2,3-Difluoro-4-methoxy-phenyl)imidazo[1,2-a]pyrazin-8-yl]amino]-2-ethyl-benzoyl]amino]propyl]piperidine-4-carboxamide

In a 50 mL round-bottomed flask, *tert*-butyl 4-((3-(4-((3-(2,3-difluoro-4-methoxyphenyl)imidazo[1,2-a]pyrazin-8-yl)amino)-2-ethylbenzamido)propyl) carbamoyl)piperidine-1-carboxylate (101 mg, 146 µmol) was combined with THF (3 mL) to give a light yellow solution. HCl (in water) (1.22 mL, 14.6 mmol) was added. The reaction was stirred at room temperature for 1h. The crude reaction mixture was concentrated in vacuum. The crude title compound was used without further purification (86.4 mg, 146 µmol, 100 % yield). MS (ESI, m/z): 592.2 [M+H]⁺.

### Step 5)

### tert-Butyl 3-[[4-[3-[[4-[[3-(2,3-difluoro-4-methoxy-phenyl)imidazo[1,2-a]pyrazin-8-yl]amino]-2-ethyl-benzoyl]amino]propylcarbamoyl]-1-piperidyl]methyl]azetidine-1-carboxylate

In a 50 mL round-bottomed flask, N-(3-(4-((3-(2,3-difluoro-4-methoxyphenyl)imidazo[1,2-a]pyrazin-8-yl)amino)-2-ethylbenzamido)propyl)piperidine-4-carboxamide (86 mg, 145 µmol), *tert-*butyl 3-formylazetidine-1-carboxylate (53.8 mg, 291 µmol) and NaBH₃CN (45.7 mg, 727 µmol) were combined with MeOH (4 mL) to give a light yellow solution. The reaction mixture was heated to 45 °C for 15 h. The crude reaction mixture was concentrated in vacuum. The reaction mixture was poured into 25 mL sat NaHCO₃ and extracted with EtOAc (3 x 25 mL). The organic layers were combined, washed with sat NaCl (1 x 25 mL), The organic layers were dried over Na₂SO₄ and concentrated in vacuum to afford the title compound (110 mg, 145 µmol, 99.5 % yield). MS (ESI, m/z): 761.4 [M+H]⁺.

### Step 6)

### tert-Butyl 3-[[4-[3-[[4-[[3-(2,3-difluoro-4-methoxy-phenyl)imidazo[1,2-a]pyrazin-8-yl]amino]-2-ethyl-benzoyl]amino]propylcarbamoyl]-1-methyl-piperidin-1-ium-1-ylmethyl]azetidine-1-carboxylate iodide

In a 50 mL round-bottomed flask, *tert*-butyl 3-((4-((3-(4-((3-(2,3-difluoro-4-methoxyphenyl)imidazo[1,2-a]pyrazin-8-yl)amino)-2-ethylbenzamido)propyl)carbamoyl) piperidin-1-yl)methyl)azetidine-1-carboxylate (110 mg, 145 µmol), MeI (103 mg, 45.2 µl, 723 µmol) and DIPEA (93.4 mg, 126 µl, 723 µmol) were combined with MeCN (6 mL) to give a light yellow solution. The reaction mixture was heated to 40 °C and stirred for 1 h. The crude reaction mixture was concentrated in vacuum. The crude title compound was used without further purification (112 mg, 144 µmol, 99.8 % yield). MS (ESI, m/z): 775.2 [M]⁺.

### Step 7)

### 1-(Azetidin-3-ylmethyl)-N-[3-[[4-[[3-(2,3-difluoro-4-methoxy-phenyl)imidazo[1,2-a]pyrazin-8-yl]amino]-2-ethyl-benzoyl]amino]propyl]-1-methyl-piperidin-1-ium-4-carboxamide bis(2,2,2-trifluoroacetate)

In a 50 mL round-bottomed flask, 1-((1-(*tert*-butoxycarbonyl)azetidin-3-yl)methyl)-4-((3-(4-((3-(2,3-difluoro-4-methoxyphenyl)imidazo[1,2-a]pyrazin-8-yl)amino)-2-ethylbenzamido)propyl) carbamoyl)-1-methylpiperidin-1-ium iodide (112 mg, 144 µmol) was combined with THF (3 mL) to give a light yellow solution. HCl(in water) (1.2 mL, 14.4 mmo) was added. The reaction was stirred at room temperature for 30min. The crude reaction mixture was concentrated in vacuum. The crude material was purified by preparative HPLC. to afford the title compound (37 mg, 40.2 µmol, 27.8 % yield). MS (ESI, m/z): 675.4 [M]⁺.

### Example 130

### N-[3-[[2-[(3S)-1-(Azetidin-3-ylmethyl)-1-methyl-pyrrolidin-1-ium-3-yl]acetyl]amino]propyl]-4-[[3-(2,3-difluoro-4-methoxy-phenyl)imidazo[1,2-a]pyrazin-8-yl]amino]-2-ethyl-benzamide bis(2,2,2-trifluoroacetate)

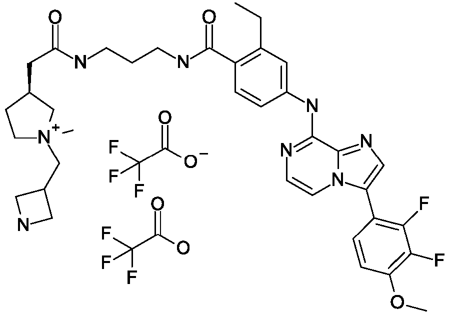

The title compound was prepared in analogy to example 129 using tert-butyl 4-((3-(4-((3-(2,3-difluoro-4-methoxyphenyl)imidazo[1,2-a]pyrazin-8-yl)amino)-2-ethylbenzamido)propyl)carbamoyl)piperidine-1-carboxylate and 2-[(3S)-1*-tert-*butoxycarbonylpyrrolidin-3-yl]acetic acid in step 1 and was obtained as off white amorphous freeze dried solid. MS (ESI, m/z): 675.4 [M]⁺.

### Example 131

### N-[3-[[2-[1-(Azetidin-3-ylmethyl)-1-methyl-piperidin-1-ium-4-yl]acetyl]amino]propyl]-4-[[3-(2,3-difluoro-4-methoxy-phenyl)imidazo[1,2-a]pyrazin-8-yl]amino]- 2-ethyl-benzamide bis(2,2,2-trifluoroacetate)

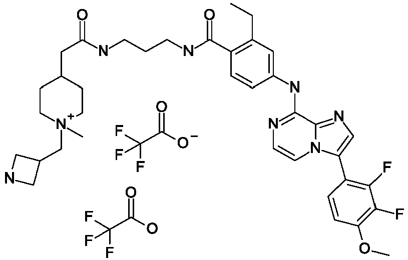

The title compound was prepared in analogy to example 129 using tert-butyl 4-((3-(4-((3-(2,3-difluoro-4-methoxyphenyl)imidazo[1,2-a]pyrazin-8-yl)amino)-2-ethylbenzamido)propyl)carbamoyl)piperidine-1-carboxylate and 2-(1-*tert*-butoxycarbonyl-4-piperidyl)acetic acid in step 1 and was obtained as off white amorphous freeze dried solid. MS (ESI, m/z): 689.4 [M]⁺.

### Example 132

### 4-[[3-(2,3-Difluoro-4-methoxy-phenyl)imidazo[1,2-a]pyrazin-8-yl]amino]-2-ethyl-N-[[1-methyl-1-[2-oxo-2-(pyrrolidin-3-ylmethylamino)ethyl]piperidin-1-ium-4-yl]methyl]benzamide bis(2,2,2-trifluoroacetate)

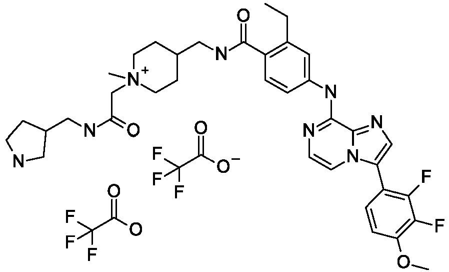

### Step 1)

### tert-Butyl 4-[[[4-[[3-(2,3-difluoro-4-methoxy-phenyl)imidazo[1,2-a]pyrazin-8-yl]amino]-2-ethyl-benzoyl]amino]methyl]piperidine-1-carboxylate

In a 100 mL round-bottomed flask, 4-((3-(2,3-difluoro-4-methoxyphenyl)imidazo[1,2-a]pyrazin-8-yl)amino)-2-ethylbenzoic acid (277 mg, 653 µmol), *tert-*butyl 4-(aminomethyl) piperidine-1-carboxylate (154 mg, 718 µmol.1) and DIPEA (253 mg, 342 µl, 1.96 mmol) were combined with DMF (5 mL) to give a light brown solution. HATU (298 mg, 783 µmol.2) was added. The reaction was stirred at room temperature for 1h. The reaction mixture was poured into 25 mL H₂O and extracted with EtOAc (3 x 25 mL). The organic layers were combined, washed with sat NaCl (1 x 25 mL). The organic layers were dried over Na₂SO₄ and concentrated in vacuum to afford the title compound (400 mg, 644 µmol, 98.7 % yield). MS (ESI, m/z): 621.0 [M+H]⁺.

### Step 2)

### 4-[[3-(2,3-Difluoro-4-methoxy-phenyl)imidazo[1,2-a]pyrazin-8-yl]amino]-2-ethyl-N-(4-piperidylmethyl)benzamide

In a 50 mL round-bottomed flask, *tert*-butyl 4-((4-((3-(2,3-difluoro-4-methoxyphenyl) imidazo[1,2-a]pyrazin-8-yl)amino)-2-ethylbenzamido)methyl)piperidine-1-carboxylate (405 mg, 652 µmol) was combined with THF (4 mL) to give a light yellow solution. HCl (in water) (2.17 mL, 26.1 mmol, Eq: 40) was added. The reaction was stirred at room temperature for 30 min and the reaction mixture was concentrated in vacuum. The crude title compound was was directly used to the next step (340 mg, 653 µmol, 100 % yield). MS (ESI, m/z): 521.2 [M+H]⁺.

### Step 3)

### tert-Butyl 2-[4-[[[4-[[3-(2,3-difluoro-4-methoxy-phenyl)imidazo[1,2-a]pyrazin-8-yl]amino]-2-ethyl-benzoyl]amino]methyl]-1-piperidyl]acetate

In a 50 mL round-bottomed flask, 4-((3-(2,3-difluoro-4-methoxyphenyl)imidazo[1,2-a]pyrazin-8-yl)amino)-2-ethyl-N-(piperidin-4-ylmethyl)benzamide (120 mg, 231 µmol), DIPEA (29.8 mg, 40 µl, 231 µmol) and *tert*-butyl 2-bromoacetate (89.9 mg, 461 µmol) were combined with MeCN (5 mL) to give a light yellow solution. The reaction was stirred at room temperature for 1h. The crude title compound was directly used in the next step (146 mg, 230 µmol, 99.8 % yield). MS (ESI, m/z): 635.1 [M+H]⁺.

### Step 4)

### 2-[4-[[[4-[[3-(2,3-Difluoro-4-methoxy-phenyl)imidazo[1,2-a]pyrazin-8-yl]amino]-2-ethyl-benzoyl]amino]methyl]-1-piperidyl]acetic acid

In a 100 mL round-bottomed flask, *tert*-butyl 2-(4-((4-((3-(2,3-difluoro-4-methoxyphenyl) imidazo[1,2-a]pyrazin-8-yl)amino)-2-ethylbenzamido)methyl)piperidin-1-yl)acetate (146 mg, 230 µmol) was combined with CH₂Cl₂ (2 mL) to give a light yellow solution. TFA (2.62 g, 1.77 mL, 23 mmo) was added. The reaction was stirred at room temperature for 1h. The crude reaction mixture was concentrated in vacuum. The crude product was directly used into the next step (133 mg, 230 µmol, 99.9 % yield). MS (ESI, m/z): 579.0 [M+H]⁺.

### Step 5)

### tert-Butyl 3-[[[2-[4-[[[4-[[3-(2,3-difluoro-4-methoxy-phenyl)imidazo[1,2-a]pyrazin-8-yl]amino]-2-ethyl-benzoyl]amino]methyl]-1-piperidyl]acetyl]amino]methyl]pyrrolidine-1-carboxylate

In a 50 mL round-bottomed flask, 2-(4-((4-((3-(2,3-difluoro-4-methoxyphenyl)imidazo[1,2-a]pyrazin-8-yl)amino)-2-ethylbenzamido)methyl)piperidin-1-yl)acetic acid (133 mg, 230 µmol), *tert-*butyl 3-(aminomethyl)pyrrolidine-1-carboxylate (64.5 mg, 322 µmol.4), HATU (122 mg, 322 µmol.4) and DIPEA (89.1 mg, 120 µl, 690 µmol) were combined with DMF (3 mL) to give a colorless solution. The reaction was stirred at room temperature for 1h. The reaction mixture was poured into 50 mL H₂O and extracted with EtOAc (3 x 25 mL).The organic layers were combined, washed with sat NaCl (1 x 25 mL), The organic layers were dried over Na₂SO₄ and concentrated in vacuum. The crude title compound was directly used in the next step (175 mg, 230 µmol, 100 % yield). ). MS (ESI, m/z): 761.2 [M+H]⁺.

### Step 6)

### tert-Butyl 3-[[[2-[4-[[[4-[[3-(2,3-difluoro-4-methoxy-phenyl)imidazo[1,2-a]pyrazin-8-yl]amino]-2-ethyl-benzoyl]amino]methyl]-1-methyl-piperidin-1-ium-1-yl]acetyl]amino]methyl]pyrrolidine-1-carboxylate iodide

In a 50 mL round-bottomed flask, *tert*-butyl 3-((2-(4-((4-((3-(2,3-difluoro-4-methoxyphenyl)imidazo[1,2-a]pyrazin-8-yl)amino)-2-ethylbenzamido)methyl)piperidin-1-yl)acetamido)methyl)pyrrolidine-1-carboxylate (100 mg, 131 µmol), MeI (93 mg, 41.1 µl, 657 µmol) and DIPEA (84.9 mg, 115 µl, 657 µmol) were combined with MeCN (6 mL) to give a light yellow solution. The reaction mixture was heated to 40 °C for 15 h. The reaction mixture was concentrated in vacuum and the crude title compounddirectly used in the next step (102 mg, 131 µmol, 100 % yield). MS (ESI, m/z): 775.0 [M]⁺.

### Step 7)

### 4-[[3-(2,3-Difluoro-4-methoxy-phenyl)imidazo[1,2-a]pyrazin-8-yl]amino]-2-ethyl-N-[[1-methyl-1-[2-oxo-2-(pyrrolidin-3-ylmethylamino)ethyl]piperidin-1-ium-4-yl]methyl]benzamide bis(2,2,2-trifluoroacetate)

In a 100 mL round-bottomed flask, 1-(2-(((1-(*tert*-butoxycarbonyl)pyrrolidin-3-yl)methyl)amino)-2-oxoethyl)-4-((4-((3-(2,3-difluoro-4-methoxyphenyl)imidazo[1,2-a]pyrazin-8-yl)amino)-2-ethylbenzamido)methyl)-1-methylpiperidin-1-ium iodide (80 mg, 103 µmol) was combined with DCM (5 mL) to give a light brown solution. 2,2,2-trifluoroacetic acid (1.18 g, 10 mmol) was added. The reaction was stirred at room temperature for 20min. The crude reaction mixture was concentrated in vacuum. The crude material was purified by preparative HPLC to afford the title compound (26 mg, 28.2 µmol, 27.4 % yield) MS (ESI, m/z): 675.2 [M]⁺.

### Example 133

### N-[[1-[1-(Azetidin-3-ylmethyl)-1-methyl-piperidin-1-ium-4-carbonyl]-4-piperidyl]methyl]-4-[[3-(2,3-difluoro-4-methoxy-phenyl)imidazo[1,2-a]pyrazin-8-yl]amino]-2-ethyl-benzamide bis(2,2,2-trifluoroacetate)

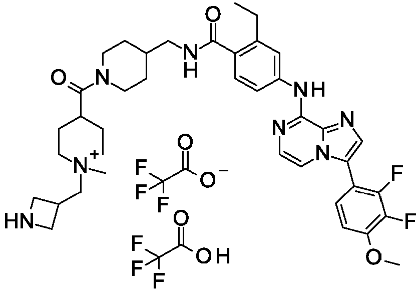

The title compound was prepared in analogy to example 129 using 4-[[3-(2,3-difluoro-4-methoxy-phenyl)imidazo[1,2-a]pyrazin-8-yl]amino]-2-ethyl-benzoic acid and *tert-*butyl *4-*(aminomethyl)piperidine-1-carboxylate in step 1 and was obtained as off white amorphous freeze dried solid. MS (ESI, m/z): 683.1 [M]⁺.

### Example 134

### N-[[1-(Azetidin-3-ylmethyl)-1-methyl-piperidin-1-ium-4-yl]methyl]-4-[[3-(2,3-difluoro-4-methoxy-phenyl)imidazo[1,2-a]pyrazin-8-yl]amino]-2-ethyl-benzamide;2,2,2-trifluoroacetate bis(2,2,2-trifluoroacetate)

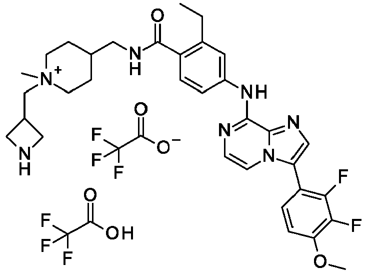

### Step 1)

### tert-Butyl 4-[[[4-[[3-(2,3-difluoro-4-methoxy-phenyl)imidazo[1,2-a]pyrazin-8-yl]amino]-2-ethyl-benzoyl]amino]methyl]piperidine-1-carboxylate

In a 100 mL round-bottomed flask, 4-((3-(2,3-difluoro-4-methoxyphenyl)imidazo[1,2-a]pyrazin-8-yl)amino)-2-ethylbenzoic acid (Intermediate 8, 277 mg, 653 µmol ), tert-butyl 4-(aminomethyl)piperidine-1-carboxylate (154 mg, 718 µmol) and DIPEA (253 mg, 342 µl, 1.96 mmol ) were combined with DMF (5 mL) to give a light brown solution. HATU (298 mg, 783 µmol ) was added. The reaction was stirred at room temperature for 1 h. The reaction mixture was poured into 25 mL H₂O and extracted with EtOAc (3 x 25 mL). The organic layers were combined, washed with sat NaCl (1 x 25 mL), The organic layers were dried over Na₂SO₄ and concentrated in vacuum to the title compound (400 mg, 644 µmol, 98.7 % yield). MS (ESI, m/z): 621.0 [M+H]⁺.

### Step 2)

### 4-[[3-(2,3-Difluoro-4-methoxy-phenyl)imidazo[1,2-a]pyrazin-8-yl]amino]-2-ethyl-N-(4-piperidylmethyl)benzamide

In a 50 mL round-bottomed flask, tert-butyl 4-((4-((3-(2,3-difluoro-4-methoxyphenyl) imidazo[1,2-a]pyrazin-8-yl)amino)-2-ethylbenzamido)methyl)piperidine-1-carboxylate (405 mg, 652 µmol ) was combined with THF (4 mL) to give a light yellow solution. HCl (in water) (2.17 mL, 26.1 mmol ) was added. The reaction was stirred at room temperature for 30 min, then the reaction mixture was concentrated in vacuum to afford the title compound (340 mg, 653 µmol, 100 % yield) which was used without further purification. MS (ESI, m/z): 521.2 [M+H]⁺.

### Step 3)

### tert-Butyl 3-[[4-[[[4-][3-(2,3-difluoro-4-methoxy-phenyl)imidazo[1,2-a]pyrazin-8-yl]amino]-2-ethyl-benzoyl]amino]methyl]-1-piperidyl]methyl]azetidine-1-carboxylate

In a 50 mL round-bottomed flask, 4-((3-(2,3-difluoro-4-methoxyphenyl)imidazo[1,2-a]pyrazin-8-yl)amino)-2-ethyl-N-(piperidin-4-ylmethyl)benzamide (90 mg, 173 µmol), tert-butyl 3-formylazetidine-1-carboxylate (48 mg, 259 µmol ) and NaBH₃CN (43.5 mg, 692 µmol ) were combined with MeOH (5 mL) to give a light yellow solution. The reaction mixture was heated to 50 °C and stirred for 2 h. The crude reaction mixture was concentrated in vacuum. The reaction mixture was poured into 50 mL sat NaHCO₃ and extracted with EtOAc (3 x 25 mL). The organic layers were combined, washed with sat NaCl (1 x 25 mL). The organic layers were dried over Na₂SO₄ and concentrated in vacuum to afford the title compound (119 mg, 173 µmol, 99.8 % yield). MS (ESI, m/z): 690.2 [M+H]⁺.

### Step 4)

### tert-Butyl 3-[[4-[[[4-[[3-(2,3-difluoro-4-methoxy-phenyl)imidazo[1,2-a]pyrazin-8-yl]amino]-2-ethyl-benzoyl]amino]methyl]-1-methyl-piperidin-1-ium-1-yl]methyl]azetidine-1-carboxylate iodide

In a 50 mL round-bottomed flask, tert-butyl 3-((4-((4-((3-(2,3-difluoro-4-methoxyphenyl) imidazo[1,2-a]pyrazin-8-yl)amino)-2-ethylbenzamido)methyl)piperidin-1-yl)methyl)azetidine-1-carboxylate (119 mg, 173 µmol ), MeI (245 mg, 108 µl, 1.73 mmol ) and DIPEA (223 mg, 301 µl, 1.73 mmol ) were combined with MeCN (6 mL) to give a light yellow solution. The reaction mixture was heated to 40 °C and stirred for 2 h. Then the reaction mixture was concentrated in vacuum to afford the title compound (122 mg, 173 µmol, 100 % yield) which was used without further purification. MS (ESI, m/z): 704.2 [M]⁻.

### Step 5)

### N-[[1-(Azetidin-3-ylmethyl)-1-methyl-piperidin-1-ium-4-yl]methyl]-4-[[3-(2,3-difluoro-4-methoxy-pheny)imidazo[1,2-a]pyrazin-8-yl]amino]-2-ethyl-benzamide;2,2,2-trifluoroacetate bis(2,2,2-trifluoroacetate)

In a 100 mL round-bottomed flask, 1-((1-(tert-butoxycarbonyl)azetidin-3-yl)methyl)-4-((4-((3-(2,3-difluoro-4-methoxyphenyl)imidazo[1,2-a]pyrazin-8-yl)amino)-2-ethylbenzamido)methyl)-1-methylpiperidin-1-ium (122 mg, 173 µmol) was combined with DCM (5 mL) to give a light brown solution. 2,2,2-trifluoroacetic acid (1.58 g, 13.8 mmol) was added. The reaction was stirred at room temperature for 1 h. The crude reaction mixture was concentrated in vacuum. The crude material was purified by preparative HPLC to afford the title compound (61 mg, 71.9 µmol, 41.5 % yield). MS (ESI, m/z): 604.4 [M]⁺.

### Example 135

### 4-[[3-(2,3-Difluoro-4-methoxy-phenyl)imidazo[1,2-a]pyrazin-8-yl]amino]-N-[[1-[(1,1-dimethylpyrrolidin-1-ium-3-yl)methyl]-1-methyl-piperidin-1-ium-4-yl]methyl]-2-ethyl-benzamide diformate

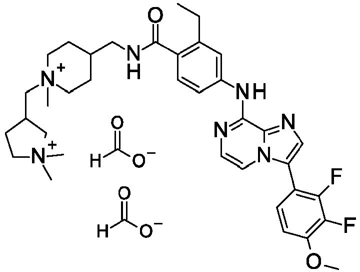

### Step 1)

### tert-Butyl 3-[[4-[[[4-[[3-(2,3-difluoro-4-methoxy-phenyl)imidazo[1,2-a]pyrazin-8-yl]amino]-2-ethyl-benzoyl]amino]methyl]-1-piperidyl]methyl]pyrrolidine-1-carboxylate

In a 100 mL round-bottomed flask, 4-((3-(2,3-difluoro-4-methoxyphenyl)imidazo[1,2-a]pyrazin-8-yl)amino)-2-ethyl-N-(piperidin-4-ylmethyl)benzamide (70 mg, 134 µmol), tert-butyl 3-formylpyrrolidine-1-carboxylate (53.6 mg, 269 µmol) and NaBH₃CN (25.3 mg, 403 µmol) were combined with MeOH (6 mL) to give a light brown solution. The reaction was stirred at room temperature for 1 h. The crude reaction mixture was concentrated in vacuum. The reaction mixture was poured into 25 mL H₂O and extracted with EtOAc (3 x 25 mL). The organic layers were combined, washed with sat NaCl (1 x 25 mL). The organic layers were dried over Na₂SO₄ and concentrated in vacuo to afford the title compound (94.6 mg, 134 µmol, 100 % yield). MS (ESI, m/z): 704.3 [M+H]⁺.

### Step 2)

### 4-[[3-(2,3-Difluoro-4-methoxy-phenyl)imidazo[1,2-a]pyrazin-8-yl]amino]-2-ethyl-N-[[1-(pyrrolidin-3-ylmethyl)-4-piperidyl]methyl]benzamide

In a 50 mL round-bottomed flask, tert-butyl 3-((4-((4-((3-(2,3-difluoro-4-methoxyphenyl) imidazo[1,2-a]pyrazin-8-yl)amino)-2-ethylbenzamido)methyl)piperidin-1-yl)methyl)pyrrolidine-1-carboxylate (94 mg, 134 µmol ) was combined with THF (3 mL) to give a light brown solution. HCl(in water) (1.11 mL, 13.4 mmol ) was added. The reaction was stirred at room temperature for 30 min. The crude reaction mixture was concentrated in vacuo to afford the title compound (80.6 mg, 134 µmol, 100 % yield) which was used without further purification. MS (ESI, m/z): 604.4 [M+H]⁺.

### Step 3)

### 4-[[3-(2,3-Difluoro-4-methoxy-phenyl)imidazo[1,2-a]pyrazin-8-yl]amino]-N-[[1-[(1,1-dimethylpyrrolidin-1-ium-3-yl)methyl]-1-methyl-piperidin-1-ium-4-yl]methyl]-2-ethyl-benzamide diformate

In a 100 mL round-bottomed flask, 4-((3-(2,3-difluoro-4-methoxyphenyl)imidazo[1,2-a]pyrazin-8-yl)amino)-2-ethyl-N-((1-(pyrrolidin-3-ylmethyl)piperidin-4-yl)methyl)benzamide (70 mg, 116 µmol ), MeI (82.3 mg, 36.3 µl, 580 µmol ) and DIPEA (74.9 mg, 101 µl, 580 µmol ) were combined with MeCN (6 mL) to give a light brown solution. The reaction was stirred at room temperature for 1 h. The crude reaction mixture was concentrated in vacuum. The crude material was purified by preparative HPLC. to afford 4-[[3-(2,3-difluoro-4-methoxy-phenyl)imidazo[1,2-a]pyrazin-8-yl]amino]-N-[[1-[(1,1-dimethylpyrrolidin-1-ium-3-yl)methyl]-1-methyl-piperidin-1-ium-4-yl]methyl]-2-ethyl-benzamide;diformate(37 mg, 47.6 µmol, 41.1 % yield). MS (ESI, m/z): 323.8 [M]⁺ (half of QAC mass)

### Example 136

### N-[1-(Azetidin-3-ylmethyl)-1-methyl-piperidin-1-ium-4-yl]-4-[[3-(2,3-difluoro-4-methoxy-phenyl)imidazo[1,2-a]pyrazin-8-yl]amino]-2-ethyl-benzamide bis(2,2,2-trifluoroacetate)

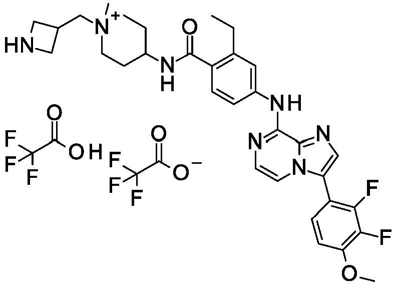

This compound was prepared in analogy to example 134 using *tert-*butyl 4-aminopiperidine-1-carboxylate in step 1. The title compound was obtained as white powder. MS (ESI, m/z): 590.2 [M]⁺

### Example 137

### 4-[[3-(2,3-Difluoro-4-methoxy-phenyl)imidazo[1,2-a]pyrazin-8-yl]amino]-2-ethyl-N-[rac-(1S,5R)-3-(azetidin-3-ylmethyl)-3-methyl-3-azoniabicyclo[3.1.0]hexan-6-yl]benzamide bis(2,2,2-trifluoroacetate)

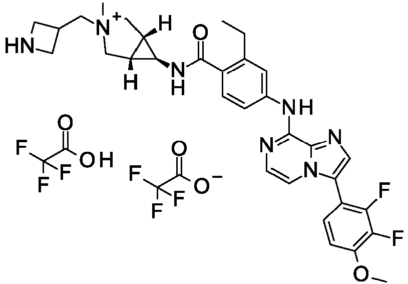

This compound was prepared in analogy to example 134 using *tert-butyl* (1R,5S)-6-amino-3-azabicyclo[3.1.0]hexane-3-carboxylate in step 1. The title compound was obtained as white powder. MS (ESI, m/z): 588.2 [M]⁺

### Example 138

### N-[[1-(Azetidin-3-ylmethyl)-1-methyl-pyrrolidin-1-ium-3-yl]methyl]-2-ethyl-4-[[3-[3-(trifluoromethyl)-1H-pyrazol-4-yl]imidazo[1,2-a]pyrazin-8-yl]amino]benzamide bis(2,2,2-trifluoroacetate)

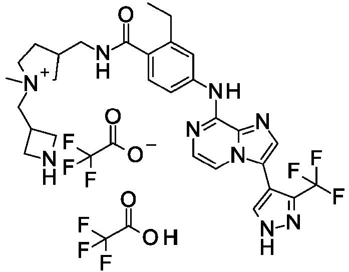

### Step 1)

### Trimethyl-[2-[[4-(4,4,5,5-tetramethyl-1,3,2-dioxaborolan-2-yl)-3-(trifluoromethyl)pyrazol-1-yl]methoxy]ethyl]silane

In a 100 mL round-bottomed flask, 4-(4,4,5,5-tetramethyl-1,3,2-dioxaborolan-2-yl)-3-(trifluoromethyl)-1 h-pyrazole (500 mg, 1.91 mmol ), SEM-Cl (414 mg, 440 µl, 2.48 mmol ) and DIPEA (740 mg, 1000 µl, 5.72 mmol ) were combined with DCM (20 mL) to give a colorless solution. The reaction was stirred at room temperature for 2 h. The crude reaction mixture was concentrated in vacuum. The crude material was purified by flash chromatography (silica gel, 40 g, 0% to 60% DCM in PE) to afford the title compound (700 mg, 1.78 mmol, 93.5 % yield). MS (ESI, m/z): 393.1 [M+H]⁺.

### Step 2)

### tert-Butyl 3-[[[2-ethyl-4-[(3-iodoimidazo[1,2-a]pyrazin-8-yl)amino]benzoyl]amino] methyl]pyrrolidine-1-carboxylate

In a 50 mL round-bottomed flask, 2-ethyl-4-((3-iodoimidazo[1,2-a]pyrazin-8-yl)amino)benzoic acid (Intermediate 34, 150 mg, 367 µmol), tert-butyl 3-(aminomethyl)pyrrolidine-1-carboxylate (95.7 mg, 478 µmol), HATU (182 mg, 478 µmol) and DIPEA (142 mg, 193 µl, 1.1 mmol) were combined with DMF (5 mL) to give a light brown solution. The reaction was stirred at room temperature for 30min. The reaction mixture was poured into 25 mL H₂O and extracted with EtOAc (3 x 25 mL). The organic layers were combined, washed with sat NaCl (1 x 25 mL). The organic layers were dried over Na₂SO₄ and concentrated in vacuum. The crude material was purified by flash chromatography (silica gel, 12 g, 0% to 5% MeOH in DCM). to afford *tert-*butyl 3-((2-ethyl-4-((3-iodoimidazo[1,2-a]pyrazin-8-yl)amino)benzamido)methyl)pyrrolidine-1-carboxylate (165 mg, 279 µmol, 76 % yield). MS (ESI, m/z): 591 [M+H]⁺.

### Step 3)

### tert-Butyl 3-[[[4-[[3-[1-[2-(2,2-dimethylpropoxy)ethyl]-3-(trifluoromethyl)pyrazol-4-yl]imidazo[1,2-a]pyrazin-8-yl]amino]-2-ethyl-benzoyl]amino]methyl]pyrrolidine-1-carboxylate

To a 5 mL microwave vial was added *tert-*butyl 3-((2-ethyl-4-((3-iodoimidazo[1,2-a]pyrazin-8-yl)amino)benzamido)methyl)pyrrolidine-1-carboxylate (120 mg, 203 µmol), 4-(4,4,5,5-tetramethyl-1,3,2-dioxaborolan-2-yl)-3-(trifluoromethyl)-1-((2-(trimethylsilyl)ethoxy)methyl)-1H-pyrazole (120 mg, 305 µmol.5), 1,1'-bis(di-*tert*-butylphosphino)ferrocene palladium dichloride (13.2 mg, 20 µmol, Eq: 0.1) and Na₂CO₃ (64.6 mg, 610 µmol) in dioxane (3 mL)/ water (0.3 mL). The vial was capped and heated in the microwave at 120 °C for 2 h under N₂. The crude reaction mixture was concentrated in vacuum. The crude material was purified by flash chromatography (silica gel, 12 g, 0% to 5% MeOH in DCM) to afford the title compound (120 mg, 165 µmol, 81 % yield). MS (ESI, m/z): 729.6 [M+H]⁺.

### Step 4)

### 2-Ethyl-N-(pyrrolidin-3-ylmethyl)-4-[[3-[3-(trifluoromethyl)-1H-pyrazol-4-yl]imidazo[1,2-a]pyrazin-8-yl]amino]benzamide

In a 100 mL round-bottomed flask, *tert*-butyl 3-((2-ethyl-4-((3-(3-(trifluoromethyl)-1-((2-(trimethylsilyl)ethoxy)methyl)-1H-pyrazol-4-yl)imidazo[1,2-a]pyrazin-8-yl)amino)benzamido)methyl)pyrrolidine-1-carboxylate (120 mg, 165 µmol) was combined with THF (3 mL) to give a light brown solution. HCl (in water) (17 mL, 16.5 mmol) was added. The reaction was stirred at room temperature for 1h. The crude reaction mixture was concentrated in vacuum. The crude title compound directly used in the next step (82.1 mg, 165 µmol, 100 % yield). MS (ESI, m/z): 499 [M+H]⁺.

### Step 5)

### tert-Butyl 3-[[3-[[[2-ethyl-4-[[3-[3-(trifluoromethyl)-1H-pyrazol-4-yl]imidazo[1,2-a]pyrazin-8-yl]amino]benzoyl]amino]methyl]pyrrolidin-1-yl]methyl]azetidine-1-carboxylate

In a 50 mL round-bottomed flask, *tert*-butyl 3-formylazetidine-1-carboxylate (91.4 mg, 493 µmol), 2-ethyl-N-(pyrrolidin-3-ylmethyl)-4-((3-(3-(trifluoromethyl)-1H-pyrazol-4-yl)imidazo[1,2-a]pyrazin-8-yl)amino)benzamide (82 mg, 164 µmol) and NaBH₃CN (51.7 mg, 822 µmol) were combined with MeOH (6 mL) to give a light brown solution. The reaction was stirred at room temperature for 15h. The crude reaction mixture was concentrated in vacuum. The reaction mixture was poured into 25 mL H₂O and extracted with EtOAc (3 x 25 mL). The organic layers were combined, washed with sat NaCl (1 x 25 mL). The organic layers were dried over Na₂SO₄ and concentrated in vacuum to afford the title compound (110 mg, 165 µmol, 100 % yield). MS (ESI, m/z): 668.5 [M+H]⁺.

### Step 6)

### tert-butyl 4-[8-[4-[[1-[(1-tert-butoxycarbonylazetidin-3-yl)methyl]pyrrolidin-3-yl]methylcarbamoyl]-3-ethyl-anilino]imidazo[1,2-a]pyrazin-3-yl]-3-(trifluoromethyl)pyrazole-1-carboxylate

In a 50 mL round-bottomed flask, *tert*-butyl 3-((3-((2-ethyl-4-((3-(3-(trifluoromethyl)-1H-pyrazol-4-yl)imidazo[1,2-a]pyrazin-8-yl)amino)benzamido)methyl)pyrrolidin-1-yl)methyl) azetidine-1-carboxylate (80 mg, 120 µmol), Boc₂O (34 mg, 36.2 µl, 156 µmol), DIPEA (31 mg, 41.9 µl, 240 µmol) and DMAP (49 mg, 35.9 µmol, Eq: 0) were combined with DCM (6 mL) to give a light brown solution. The reaction was stirred at room temperature for 1h. The crude reaction mixture was concentrated in vacuum. The reaction mixture was poured into 25 mL H₂O and extracted with EtOAc (3 x 25 mL). The organic layers were combined, washed with sat NaCl (1 x 25 mL). The organic layers were dried over Na₂SO₄ and concentrated in vacuum to afford the title compound (92 mg, 120 µmol, 100 % yield). MS (ESI, m/z): 768.7 [M+H]⁺.

### Step 7)

### tert-Butyl 4-[8-[4-[[1-[(1-tert-butoxycarbonylazetidin-3-yl)methyl]-1-methyl-pyrrolidin-1-ium-3-yl]methylcarbamoyl]-3-ethyl-anilino]imidazo[1,2-a]pyrazin-3-yl]-3-(trifluoromethyl)pyrazole-1-carboxylate

In a 50 mL round-bottomed flask, *tert*-butyl 4-(8-((4-(((1-((1-(*tert*-butoxycarbonyl)azetidin-3-yl)methyl)pyrrolidin-3-yl)methyl)carbamoyl)-3-ethylphenyl)amino)imidazo[1,2-a]pyrazin-3-yl)-3-(trifluoromethyl)-1H-pyrazole-1-carboxylate (85 mg, 111 µmol), MeI (78.6 mg, 34.6 µl, 553 µmol) and DIPEA (71.5 mg, 96.7 µl, 553 µmol) were combined with MeCN (6 mL) to give a light brown solution. The reaction was stirred at room temperature for 15h. The reaction mixture was concentrated in vacuum. The crude product was directly used to the next step, to afford the title compound (86.7 mg, 111 µmol, 100 % yield). MS (ESI, m/z): 782.6 [M]⁺.

### Step 8)

### N-[[1-(Azetidin-3-ylmethyl)-1-methyl-pyrrolidin-1-ium-3-yl]methyl]-2-ethyl-4-[[3-3-(trifluoromethyl)-1H-pyrazol-4-yllimidazo[1,2-a]pyrazin-8-yl]amino]benzamide bis(2,2,2-trifluoroacetate)

In a 50 mL round-bottomed flask, 3-((4-((3-(1-(tert-butoxycarbonyl)-3-(trifluoromethyl)-1H-pyrazol-4-yl)imidazo[1,2-a]pyrazin-8-yl)amino)-2-ethylbenzamido)methyl)-1-((1-(*tert-*butoxycarbonyl)azetidin-3-yl)methyl)-1-methylpyrrolidin-1-ium (86 mg, 110 µmol) was combined with DCM (3 mL) to give a light brown solution. 2,2,2-trifluoroacetic acid (1.25 g, 11 mmo) was added. The reaction was stirred at room temperature for 1h. The crude reaction mixture was concentrated in vacuum. The crude material was purified by preparative HPLC to afford the title compound (20 mg, 24.2 µmol, 22 % yield), MS (ESI, m/z): 582.6 [M]⁺.

### Example 139

### Bis(azetidin-3-ylmethyl)-[3-[[4-[[3-(2,3-difluoro-4-methoxy-phenyl)imidazo[1,2-a]pyrazin-8-yl]amino]-2-ethyl-benzoyl]amino]propyl]-methyl-ammonium bis(2,2,2-trifluoroacetate)

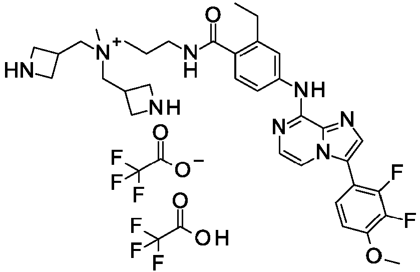

### Step 1)

### tert-Butyl N-[3-[[4-[[3-(2,3-difluoro-4-methoxy-phenyl)imidazo[1,2-a]pyrazin-8-yl]amino]-2-ethyl-benzoyl]amino]propyl]carbamate

In a 50 mL round-bottomed flask, 4-((3-(2,3-difluoro-4-methoxyphenyl)imidazo[1,2-a]pyrazin-8-yl)amino)-2-ethylbenzoic acid (Intermediate 8, 100 mg, 236 µmol), tert-butyl (3-aminopropyl)carbamate (49.3 mg, 283 µmol), 2-(3H-[1,2,3]triazolo[4,5-b]pyridin-3-yl)-1,1,3,3-tetramethylisouronium hexafluorophosphate(V) (108 mg, 283 µmol) and DIPEA (91.4 mg, 123 µl, 707 µmol) were combined with DMF (3 mL) to give a light brown solution. The reaction was stirred at room temperature for 30min. The reaction mixture was poured into 25 mL H₂O and extracted with EtOAc (3 x 25 mL). The organic layers were combined, washed with sat NaCl (1 x 25 mL). The organic layers were dried over Na₂SO₄ and concentrated in vacuum. The crude material was purified by flash chromatography (silica gel, 12 g, 0% to 10% MeOH in DCM) to afford the title compound (137 mg, 236 µmol, 100 % yield). MS (ESI, m/z): 581.4 [M+H]⁺.

### Step 2)

### N-(3-Aminopropyl)-4-[[3-(2,3-difluoro-4-methoxy-phenyl)imidazo[1,2-a]pyrazin-8-yl]amino]-2-ethyl-benzamide

In a 100 mL round-bottomed flask, tert-butyl (3-(4-((3-(2,3-difluoro-4-ethoxyphenyl)imidazo[1,2-a]pyrazin-8-yl)amino)-2-ethylbenzamido)propyl)carbamate (130 mg, 224 µmol) was combined with THF (3 mL) to give a light brown solution. HCl(in water) (1.49 mL, 17.9 mmol) was added. The reaction was stirred at room temperature for 1 h. The reaction mixture was concentrated in vacuum to afford the title compound(108 mg, 225 µmol, 100 % yield) which was used without further purification. MS (ESI, m/z): 481.3 [M+H]⁺.

### Step 3)

### tert-Butyl 3-[[(1-tert-butoxycarbonylazetidin-3-yl)methyl-[3-[[4-[[3-(2,3-difluoro-4-methoxy-phenyl)imidazo[1,2-a]pyrazin-8-yl]amino]-2-ethyl-benzoyl]amino]propyl]amino] methyl]azetidine-1-carboxylate

In a 50 mL round-bottomed flask, N-(3-aminopropyl)-4-((3-(2,3-difluoro-4-methoxyphenyl)imidazo[1,2-a]pyrazin-8-yl)amino)-2-ethylbenzamide (108 mg, 225 µmol), *tert-*butyl 3-formylazetidine-1-carboxylate (125 mg, 674 µmol) and NaBH₃CN (42.4 mg, 674 µmol) were combined with MeOH (6 mL) to give a light brown solution. The reaction mixture was heated to 40 °C for 3 h. The crude reaction mixture was concentrated in vacuum. The reaction mixture was poured into 50 mL H₂O and extracted with EtOAc (3 x 25 mL). The organic layers were combined, washed with sat NaCl (1 x 25 mL). The organic layers were dried over Na₂SO₄ and concentrated in vacuum to afford the title compound (184 mg, 225 µmol, 100 % yield). MS (ESI, m/z): 819.6 [M+H]⁺.

### Step 4)

### Bis[(1-tert-butoxycarbonylazetidin-3-yl)methyl]-[3-[[4-[[3-(2,3-difluoro-4-methoxyphenyl)imidazo[1,2-a]pyrazin-8-yl]amino]-2-ethyl-benzoyl]amino]propyl]-methyl-ammonium iodide

In a 100 mL round-bottomed flask, di-*tert*-butyl 3,3'-(((3-(4-((3-(2,3-difluoro-4-methoxyphenyl)imidazo[1,2-a]pyrazin-8-yl)amino)-2-ethylbenzamido)propyl)azanediyl)bis (methylene))bis(azetidine-1-carboxylate) (183 mg, 223 µmol), MeI (159 mg, 69.9 µl, 1.12 mmol) and DIPEA (144 mg, 195 µl, 1.12 mmol) were combined with MeCN (6 mL) to give a light brown solution. The reaction was stirred at room temperature for 15h. The reaction mixture was concentrated in vacuum. The crude title compound was directly used in the next step (186 mg, 223 µmol, 99.8 % yield). MS (ESI, m/z): 833 [M]⁺.

### Step 5)

### Bis(azetidin-3-ylmethyl)-[3-[[4-[[3-(2,3-difluoro-4-methoxy-phenyl)imidazo[1,2-a]pyrazin-8-yl]amino]-2-ethyl-benzoyl]amino]propyl]-methyl-ammonium bis(2,2,2-trifluoroacetate)

In a 100 mL round-bottomed flask, N,N-bis((1-(*tert*-butoxycarbonyl)azetidin-3-yl)methyl)-3-(4-((3-(2,3-difluoro-4-methoxyphenyl)imidazo[1,2-a]pyrazin-8-yl)amino)-2-ethylbenzamido)-N-methylpropan-1-aminium iodide (186 mg, 223 µmol) was combined with DCM (4 mL) to give a light yellow solution. 2,2,2-trifluoroacetic acid (2.54 g, 22 mmo) was added. The reaction was stirred at room temperature for 1h. The crude reaction mixture was concentrated in vacuum. The crude material was purified by preparative HPLC to afford the title compound (55 mg, 55 µmol, 24.8 % yield). MS (ESI, m/z): 633 [M]⁺.

### Example 140

### 4-[[3-(2,3-difluoro-4-methoxy-phenyl)imidazo[1,2-a]pyrazin-8-yl]amino]-2-ethyl-N-[[1-methyl-1-(3-pyridylmethyl)piperidin-1-ium-4-yl]methyl]benzamide;2,2,2-trifluoroacetate

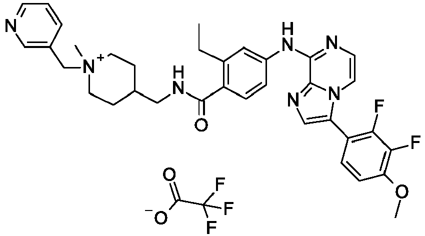

This compound was prepared in analogy to example 134 using *tert-*butyl 4-(aminomethyl)piperidine-1-carboxylate and pyridine-3-carbaldehyde. The title compound was obtained as white powder. MS (ESI, m/z): 626.3 [M]⁺

### Example 141

### N-[[1-(Azetidin-3-ylmethyl)-1-(2-hydroxyethyl)piperidin-1-ium-4-yl]methyl]-4-[[3-(2,3-difluoro-4-methoxy-phenyl)imidazo[1,2-a]pyrazin-8-yl]amino]-2-ethyl-benzamide 2,2,2-trifluoroacetate

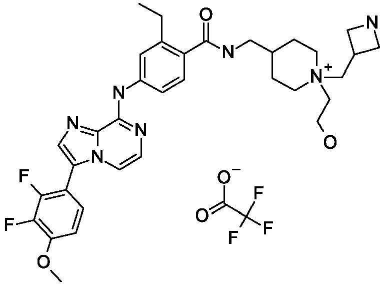

This compound was prepared in analogy to example 134 using *tert-*butyl 4-(aminomethyl)piperidine-1-carboxylate and 2-bromoethanol. The title compound was obtained as white powder. MS (ESI, m/z): 634.3[M]⁺

### Example 142

### 4-[[3-(2,3-Difluoro-4-methoxy-phenyl)imidazo[1,2-a]pyrazin-8-yl]amino]-2-ethyl-N-[[1-methyl-1-(pyrrolidin-2-ylmethyl)piperidin-1-ium-4-yl]methyl]benzamide 2,2,2-trifluoroacetate

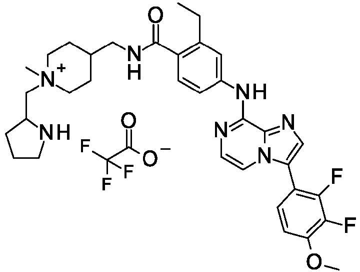

This compound was prepared in analogy to example 134 using *tert-*butyl 4-(aminomethyl)piperidine-1-carboxylate and *tert-*butyl 2-formylpyrrolidine-1-carboxylate. The title compound was obtained as white powder. MS (ESI, m/z): 618.3 [M]⁺

### Example 143

### N-[[1-(Azetidin-3-ylmethyl)-1-ethyl-piperidin-1-ium-4-yl]methyl]-4-[[3-(2,3-difluoro-4-methoxy-phenyl)imidazo[1,2-a]pyrazin-8-yl]amino]-2-ethyl-benzamide formate

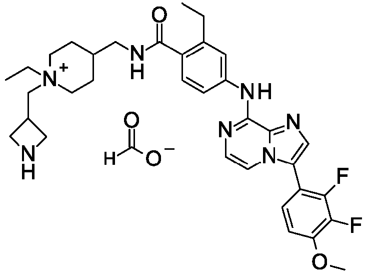

This compound was prepared in analogy to example 134 using *tert-*butyl 4-(aminomethyl)piperidine-1-carboxylate and iodoethane. The title compound was obtained as white powder. MS (ESI, m/z): 618.4 [M]⁺

### Example 144

### 1-[2-[[4-[[3-(2,3-Difluoro-4-methoxy-phenyl)imidazo[1,2-a]pyrazin-8-yl]amino]-2-ethyl-benzoyl]amino]ethyl]-1-methyl-pyrrolidin-1-ium-3-carboxamide 2,2,2-trifluoroacetate

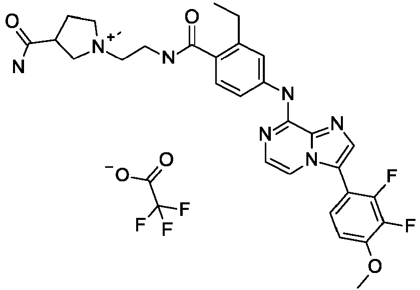

### Step 1)

### Methyl 1-[2-[[4-[[3-(2,3-difluoro-4-methoxy-phenyl)imidazo[1,2-a]pyrazin-8-yl]amino]-2-ethyl-benzoyl]amino]ethyl]pyrrolidine-3-carboxylate

To a solution of 4-[[3-(2,3-difluoro-4-methoxy-phenyl)imidazo[1,2-a]pyrazin-8-yl]amino]-2-ethyl-benzoic acid (Intermediate 8, 1626.43 mg, 3.83 mmol, 1.2 eq) and HATU (2428.66 mg, 6.39 mmol, 2 eq) in DMF (15 mL) was added methyl 1-(2-aminoethyl)pyrrolidine-3-carboxylate (550.0 mg, 3.19 mmol, 1 eq), DIPEA (1.58 mL, 9.58 mmol, 3 eq.) to the solution. The mixture was stirred at 25 °C under N₂ for 16 h. The mixture was added water (50 mL) and extracted with EA (30 mL × 3). The combined organic layers were washed by sat. aq. NH₄Cl (30 mL) and sat. aq. Na₂CO₃ (30 mL) and sat. LiCl (30 mL) and concentrated to dryness. The crude was then purified by flash column chromatography eluting 45% EtOAc in PE to afford a white solid. MS (ESI, m/z): 579.6 [M+H]⁺

### Step 2)

### 1-[2-[[4-[[3-(2,3-Difluoro-4-methoxy-phenyl)imidazo[1,2-a]pyrazin-8-yl]amino]-2-ethyl-benzoyl]amino]ethyl]pyrrolidine-3-carboxamide

A mixture of ammonia in MeOH (8.24 mg, 0.480 mmol, 1 eq), methyl 1-[2-[[4-[[3-(2,3-difluoro-4-methoxy-phenyl)imidazo[1,2-a]pyrazin-8-yl]amino]-2-ethyl-benzoyl]amino]ethyl]pyrrolidine-3-carboxylate (280.0 mg, 0.480 mmol, 1 eq.) in MeOH was stirred at 25 °C for 48 h. To the mixture was added water (50 mL) and extracted with EA (30 mL × 3). The combined organic layers were dried over Na₂SO₄ and concentrated to dryness. The crude was then purified by flash column chromatography eluting 35% EtOAc in PE to afford the title compound (265 mg, 0.470 mmol, 89.4% yield) as a white solid. MS (ESI, m/z): 564 [M+H]⁺

### Step 3)

### 1-[2-[[4-[[3-(2,3-Difluoro-4-methoxy-phenyl)imidazo[1,2-a]pyrazin-8-yl]amino]-2-ethyl-benzoyl]amino]ethyl]-1-methyl-pyrrolidin-1-ium-3-carboxamide;2,2,2-trifluoroacetate

A mixture of DIEA (188.88 mg, 1.33 mmol, 15 eq), 1-[2-[[4-[[3-(2,3-difluoro-4-methoxyphenyl)imidazo[1,2-a]pyrazin-8-yl]amino]-2-ethyl-benzoyl]amino]ethyl]pyrrolidine-3-carboxamide (50.0 mg, 0.09 mmol, 1 eq), and iodomethane (125.92 mg, 0.890 mmol, 10 eq) in MeCN (2 mL) was stirred at 80 °C for 16 h. The mixture was added water (50 mL) and extracted with EA (30 mL × 3) . The combined organic layers were dried over Na₂SO₄ and concentrated to dryness. The crude was then purified by flash column chromatography eluting 35% EtOAc in PE to afford 1-[2-[[4-[[3-(2,3-difluoro-4-methoxy-phenyl)imidazo[1,2-a]pyrazin-8-yl]amino]-2-ethyl-benzoyl]amino]ethyl]-1-methyl-pyrrolidin-1-ium-3-carboxamide 2,2,2-trifluoroacetate (8.2 mg, 0.010 mmol, 12.7% yield) as a yellow oil. (ESI, m/z): 578.27 [M]⁺

### Example 145

### 4-[[3-(2,3-Difluoro-4-phenoxy-phenyl)imidazo[1,2-a]pyrazin-8-yl]amino]-2-ethyl-N-[[1-methyl-1-(pyrrolidin-3-ylmethyl)piperidin-1-ium-4-yl]methyl]benzamide formate

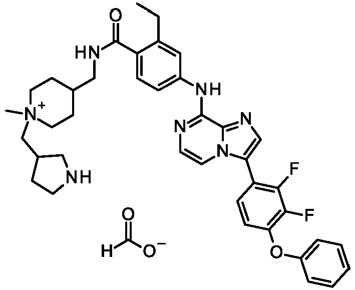

### Step 1)

### 1-Bromo-2,3-difluoro-4-phenoxy-benzene

To a solution of 4-bromo-2,3-difluorophenol ( 2 g, 9.57 mmol, Eq: 1) in DCM ( 160 mL) was added phenylboronic acid (2.33 g, 19.1 mmol, Eq: 2), copper (II) acetate (3.48 g, 19.1 mmol, Eq: 2) and TEA (1.94 g, 2.67 ml, 19.1 mmol, Eq: 2), the reaction was stirred for 48 hours at room temperature. The reaction mixture was filtered and the filtrate was concentrated in vacuum. The residue was purified by column chromatography to give the title compound (1 g, 3.5 mmol, 36.6 % yield). MS (ESI, m/z): 285.0 [M+H]⁺.

### Step 2)

### 2-(2,3-Difluoro-4-phenoxy-phenyl)-4,4,5,5-tetramethyl-1,3,2-dioxaborolane

To a solution of 1-bromo-2,3-difluoro-4-phenoxybenzene (1 g, 3.51 mmol, Eq: 1) in dioxane ( 10 mL) was added 4,4,4',4',5,5,5',5'-octamethyl-2,2'-bi(1,3,2-dioxaborolane) (980 mg, 3.86 mmol, Eq: 1.1), potassium acetate (689 mg, 7.02 mmol, Eq: 2) and 1,1'-bis(di-*tert-*butylphosphino)ferrocene palladium dichloride (286 mg, 351 µmol, Eq: 0.1), the reaction was stirred for 12 hours at 80 °C under atmosphere of argon. The reaction mixture was cooled to room temperature and filtered. The filtrate was concentrated in vacuum. The residue was purified by column chromatography to give the title compound (0.8 g, 2.4 mmol, 68.4 % yield). MS (ESI, m/z): 333.0 [M+H]⁺.

### Step 3)

### tert-Butyl 4-[[[4-[[3-(2,3-difluoro-4-phenoxy-phenyl)imidazo[1,2-a]pyrazin-8-yl]amino]-2-ethyl-benzoyl]amino]methyl]piperidine-1-carboxylate

To a solution of *tert*-butyl 4-((2-ethyl-4-((3-iodoimidazo[1,2-a]pyrazin-8-yl)amino)benzamido)methyl)piperidine-1-carboxylate (Intermediate 47 350 mg, 579 µmol, Eq: 1) in the mixture solvent of dioxane (5 mL) and water (1 mL) was added 2-(2,3-difluoro-4-phenoxyphenyl)-4,4,5,5-tetramethyl-1,3,2-dioxaborolane (231 mg, 695 µmol, Eq: 1.2), sodium carbonate (184 mg, 1.74 mmol, Eq: 3) and 1,1'-bis(di-*tert-*butylphosphino)ferrocene palladium dichloride (37.7 mg, 57.9 µmol, Eq: 0.1), the reaction was stirred for 30 minutes at 100 °C under microwave irradiation and atmosphere of nitrogen. The reaction mixture was filtered and the filtrate was concentrated in vacuum. The residue was purified by column chromatography to give the title compound.

### Step 4)

### 4-[[3-(2,3-Difluoro-4-phenoxy-phenyl)imidazo[1,2-a]pyrazin-8-yl]amino]-2-ethyl-N-(4-piperidylmethyl)benzamide hydrochloride

To a solution of *tert*-butyl 4-((4-((3-(2,3-difluoro-4-phenoxyphenyl)imidazo[1,2-a]pyrazin-8-yl)amino)-2-ethylbenzamido)methyl)piperidine-1-carboxylate (300 mg, 439 µmol, Eq: 1) in MeOH ( 3mL) was added HCl/Dioxane (2 mol/L, 5mL), the reaction was stirred for 30 minutes at room temperature. The reaction mixture was concentrated in vacuum to afford the title compound (270 mg, 429 µmol, 97.8 % yield). MS (ESI, m/z): 583.3 [M+H]⁺.

### Step 5)

### tert-Butyl 3-[[4-[[[4-[[3-(2,3-difluoro-4-phenoxy-phenyl)imidazo[1,2-a] pyrazin-8-yl] amino]-2-ethyl-benzoyl]amino]methyl]-1-piperidyl]methyl]pyrrolidine-1-carboxylate

To a solution of 4-((3-(2,3-difluoro-4-phenoxyphenyl)imidazo[1,2-a]pyrazin-8-yl)amino)-2-ethyl-N-(piperidin-4-ylmethyl)benzamide hydrochloride (270 mg, 436 µmol, Eq: 1) in MeOH ( 3 mL) was added *tert*-butyl 3-formylpyrrolidine-1-carboxylate (261 mg, 1.31 mmol, Eq: 3) and sodium cyanoborohydride (54.8 mg, 872 µmol, Eq: 2), the reaction was stirred for two hours at room temperature. The reaction mixture was quenched with water and extracted in EtOAc. The organic layer was dried over anhydrous Na₂SO₄ and concentrated in vacuum. The residue was purified by column chromatography to give the title compuond (300 mg, 392 µmol, 89.8 % yield). MS (ESI, m/z): 766.0 [M+H]⁺.

### Step 6)

### tert-Butyl 3-[[4-[[[4-[[3-(2,3-difluoro-4-phenoxy-phenyl)imidazo[1,2-a]pyrazin-8-yl]amino]-2-ethyl-benzoyl]amino]methyl]-1-methyl-piperidin-1-ium-1-yl]methyl]pyrrolidine-1-carboxylate iodide

To a solution of *tert*-butyl 3-((4-((4-((3-(2,3-difluoro-4-phenoxyphenyl)imidazo[1,2-a]pyrazin-8-yl)amino)-2-ethylbenzamido)methyl)piperidin-1-yl)methyl)pyrrolidine-1-carboxylate (200 mg, 261 µmol, Eq: 1) in MeOH ( 3 mL) was added iodomethane (185 mg, 81.6 µL, 1.31 mmol, Eq: 5) and TEA (79.3 mg, 109 µL, 783 µmol, Eq: 3). The reaction was stirred for 20 hours at room temperature. The reaction mixture was concentrated in vacuum and the residue was directly used for the next step without further purification. MS (ESI, m/z): 780.0 [M]⁺.

### Step 7)

### 4-((4-((3-(2,3-Difluoro-4-phenoxyphenyl)imidazo[1,2-a]pyrazin-8-yl)amino)-2-ethylbenzamido)methyl)-1-methyl-1-(pyrrolidin-3-ylmethyl)piperidin-1-ium formate

To a solution of 1-(tert-butoxycarbonyl)-3-((4-((4-((3-(2,3-difluoro-4-phenoxyphenyl)imidazo[1,2-a]pyrazin-8-yl)amino)-2-ethylbenzamido)methyl)piperidin-1-yl)methyl)-1-methylpyrrolidin-1-ium iodide (100mg, 128 µmol, Eq: 1) in MeOH (3mL) was added HCl /Dioxane and the reaction was stirred for 2 hours at room temperature. The reaction mixture was concentrated in vacuum and purified by preparative HPLC to afford the title compound (40 mg, 39% yield). MS (ESI, m/z): 680.6 [M]⁺.

### Assay procedures

### Antimicrobial susceptibility testing:

### 90% Growth Inhibitory Concentration (IC90) determination

The in vitro antimicrobial activity of the compounds was determined according to the following procedure:

The assay used a 10-points Iso-Sensitest broth medium to measure quantitatively the *in vitro* activity of the compounds against *Acinetobacter baumannii* ATCC17961 and ATCC17978.

Stock compounds in DMSO were serially twofold diluted (e.g. range from 50 to 0.097 µM final concentration) in 384 wells microtiter plates and inoculated with 49 µl the bacterial suspension in Iso-Sensitest medium to have a final cell concentration of ~ 5x10⁽⁵⁾ CFU/ml in a final volume/well of 50 ul/well. Microtiter plates were incubated at 35 ± 2 °C.

Bacterial cell growth was determined with the measurement of optical density at λ=600nm each 20 minutes over a time course of 16h. Growth inhibition was calculated during the logarithmic growth of the bacterial cells with determination of the concentration inhibiting 50% (IC50) and 90% (IC90) of the growth.

Table 1 provides the 90% growth inhibitory concentrations (IC90) in micromoles per liter of the compounds of present invention obtained against the strain *Acinetobacter baumannii* ATCC17978.

Table 2 provides the 90% growth inhibitory concentrations (IC90) in micromoles per liter of the compounds of present invention obtained against the strain *Acinetobacter baumannii* ATCC17961.

Particular compounds of the present invention exhibit an IC90 (*Acinetobacter baumannii* ATCC17961 or ATCC17978) ≤ 25 µmol/l.

More particular compounds of the present invention exhibit an IC90 (*Acinetobacter baumannii* ATCC17961 or ATCC17978) ≤ 5 µmol/l.

Most particular compounds of the present invention exhibit an IC90 (*Acinetobacter baumannii* ATCC17961 or ATCC17978) ≤ 1 µmol/l.

### Minimal Inhibitory Concentration (MIC) and MIC90 Determination:

The MIC of antibacterial compounds against multiple species and strains was determined using the broth microdilution method according to M07-A10 Clinical and Laboratory Standards Institute (CLSI) guidelines (CLSI (2015) Methods for dilution antimicrobial susceptibility tests for bacteria that grow aerobically; Approved Standard, Tenth Edition. CLSI Document M7-A10 (ISBN 1-56238-987-4). Wayne, PA: Clinical and Laboratory Standards Institute, 19087, USA.), using the appropriate broth medium. In short, the compound plates were prepared by dispensing 100µl of each stock solution in the first well of a 96 well microtiter plate (MTP) at a concentration 100-fold higher than the final concentration desired in broth. Eleven serial 2-fold dilutions of the highest concentration were made in DMSO for new compounds and in water (or appropriate solution) for reference compounds (CLSI (2017) Performance Standards for Antimicrobial Susceptibility Testing; Twenty-Fifth Informational Supplement. CLSI Document M100-S27, CLSI, Wayne, Pennsylvania 19087, USA.). 1µL of each well was transferred in a new MTP, which served as the test plate by subsequent inoculation. This bacterial suspension was prepared from strains that were first sub-cultured on agar plates and incubated for 18-24 hours as appropriate. Following incubation, the inoculum was prepared from isolated colonies and adjusted to 0.5 McFarland turbidity standard (1 to 2x10⁸ Colony Forming Units (CFU)/mL) in 0.9% saline. The bacterial suspension was then diluted 1:200 in appropriate sterile medium and within 15 minutes 100µL/well were dispensed. Negative controls (lack of bacterial cells) and growth control wells (lack of compound) were included in all plates. MTPs were incubated for 18-24 hours at 35±2°C in ambient air. The MIC value of each compound, expressed as µg/mL, was determined as the lowest concentration required for complete growth inhibition (no visible growth). MIC₉₀ and MIC₅₀ values were defined as the lowest concentration of the antibacterial compound at which 90% and 50% of the strains were inhibited.

Table 3 provides the MIC's of particular compounds of the present invention obtained against the strain *Acinetobacter baumannii* ATCC 17978, expressed as µg/mL.

**Table 1**

| Example | ATCC 17978 IC90 (uM) |
|---|---|
| 1 | 0.658 |
| 2 | 0.214 |
| 5 | 2.13 |
| 13 | 0.109 |
| 14 | 0.476 |
| 15 | 0.238 |
| 16 | 0.396 |
| 17 | 1.29 |
| 19 | 0.446 |
| 20 | 0.958 |
| 21 | 0.48 |
| 22 | 0.26 |
| 23 | 2.22 |
| 24 | 0.742 |
| 25 | 0.591 |
| 26 | 0.156 |
| 27 | 0.249 |
| 28 | 0.605 |
| 29 | 0.293 |
| 30 | 0.497 |
| 31 | 0.12 |
| 32 | 0.307 |
| 33 | 0.996 |
| 34 | 0.246 |
| 35 | 1.05 |
| 36 | 0.431 |
| 37 | 4.77 |
| 38 | 0.213 |
| 39 | 2.38 |
| 40 | 0.165 |
| 41 | 0.132 |
| 42 | 0.414 |
| 43 | 0.115 |
| 44 | 0.148 |
| 45 | 0.321 |
| 46 | 0.161 |
| 47 | 0.383 |
| 48 | 0.566 |
| 49 | 0.968 |
| 50 | 0.394 |
| 51 | 0.0628 |
| 52 | 0.0388 |
| 53 | 0.405 |
| 54 | 0.0452 |
| 146 | 0.703 |
| 147 | 0.138 |
| 148 | 0.135 |
| 149 | 0.182 |
| 150 | 0.357 |

**Table 2**

| Example | ATCC 17961 IC90 (uM) |
|---|---|
| 3 | 0.484 |
| 4 | 0.267 |
| 7 | 1.16 |
| 8 | 0.6 |
| 9 | 0.0635 |
| 10 | 0.157 |
| 11 | 0.131 |
| 12 | 0.0447 |
| 18 | 0.336 |
| 55 | 0.132 |
| 56 | 0.0596 |
| 57 | 2.18 |
| 58 | 1.58 |
| 59 | 0.041 |
| 60 | 0.166 |
| 61 | 0.478 |
| 62 | 0.449 |
| 63 | 0.0811 |
| 64 | 0.78 |
| 65 | 0.546 |
| 66 | 0.0572 |
| 67 | 0.0448 |
| 68 | 0.168 |
| 69 | 0.263 |
| 70 | 0.0445 |
| 71 | 0.0677 |
| 72 | 0.0403 |
| 73 | 0.121 |
| 74 | 0.031 |
| 75 | 0.0658 |
| 76 | 0.0274 |
| 77 | 0.342 |
| 78 | 0.467 |
| 79 | 0.0822 |
| 80 | 0.0959 |
| 81 | 0.107 |
| 82 | 0.11 |
| 83 | 0.127 |
| 84 | 0.132 |
| 85 | 0.147 |
| 86 | 0.168 |
| 87 | 0.318 |
| 88 | 0.485 |
| 89 | 0.634 |
| 90 | 0.32 |
| 91 | 0.834 |
| 92 | 0.332 |
| 93 | 0.909 |
| 94 | 0.457 |
| 95 | 0.725 |
| 96 | 0.171 |
| 97 | 0.205 |
| 102 | 0.223 |
| 103 | 0.0779 |
| 104 | 0.118 |
| 105 | 0.144 |
| 106 | 0.241 |
| 107 | 0.434 |
| 108 | 1.59 |
| 109 | 3.83 |
| 110 | 4.3 |
| 111 | 2.8 |
| 112 | 2.56 |
| 113 | 2.48 |
| 115 | 0.205 |
| 116 | 0.0811 |
| 117 | 0.157 |
| 118 | 0.832 |
| 119 | 1.7 |
| 120 | 0.682 |
| 121 | 0.854 |
| 122 | 1.11 |
| 123 | 5 |
| 124 | 1.55 |
| 125 | 1.55 |
| 126 | 0.111 |
| 127 | 0.258 |
| 128 | 0.343 |
| 129 | 0.0862 |
| 130 | 0.108 |
| 131 | 0.114 |
| 132 | 0.32 |
| 133 | 0.334 |
| 134 | 0.35 |
| 135 | 0.479 |
| 136 | 0.955 |
| 137 | 1.22 |
| 138 | 4.14 |
| 139 | 4.82 |
| 140 | 0.115 |
| 141 | 0.169 |
| 142 | 0.336 |
| 143 | 0.609 |
| 145 | 1.78 |
| 151 | 0.0857 |
| 152 | 0.0788 |
| 153 | 0.0434 |
| 154 | 0.0465 |

**Table 3**

| Example | ATCC 17978 MIC (ug/ml) |
|---|---|
| 6 | 8.57 |
| 98 | 0.92 |
| 99 | 0.92 |
| 100 | 1.84 |
| 101 | 1.84 |
| 114 | 17.8 |
| 144 | 3.62 |

### Example 156

A compound of formula (II) or (III) can be used in a manner known per se as the active ingredient for the production of tablets of the following composition:

### Per tablet

| | |
|---|---|
| Active ingredient | 200 mg |
| Microcrystalline cellulose | 155 mg |
| Corn starch | 25 mg |
| Talc | 25 mg |
| Hydroxypropylmethylcellulose | 20 mg |
| | 425 mg |

### Example 157

A compound of formula (II) or (III) can be used in a manner known per se as the active ingredient for the production of capsules of the following composition:

### Per capsule

| | |
|---|---|
| Active ingredient | 100.0 mg |
| Corn starch | 20.0 mg |
| Lactose | 95.0 mg |
| Talc | 4.5 mg |
| Magnesium stearate | 0.5 mg |
| | 220.0 mg |

### Example 158

A compound of formula (II) or (III) can be used in a manner known per se as the active ingredient for the production of an infusion solution of the following composition:

| | |
|---|---|
| Active ingredient | 100 mg |
| Lactic acid 90% | 100 mg |
| NaOH q.s. or HCl q.s. for adjustment to pH | 4.0 |
| Sodium chloride q.s. or glucose q.s. for adjustment of the osmolality to | 290 mOsm/kg |
| Water for injection (WFI) | ad 100 ml |

### Example 159

A compound of formula (II) or (III) can be used in a manner known per se as the active ingredient for the production of an infusion solution of the following composition:

| | |
|---|---|
| Active ingredient | 100 mg |
| Hydroxypropyl-beta-cyclodextrin | 10 g |
| NaOH q.s. or HCl q.s. for adjustment to | pH 7.4 |
| Sodium chloride q.s. or glucose q.s. for adjustment of the osmolality to | 290 mOsm/kg |
| Water for injection (WFI) | ad 100 ml |

## Claims

1. A compound of formula (IIa) or (IIIa) or a pharmaceutically acceptable salt thereof 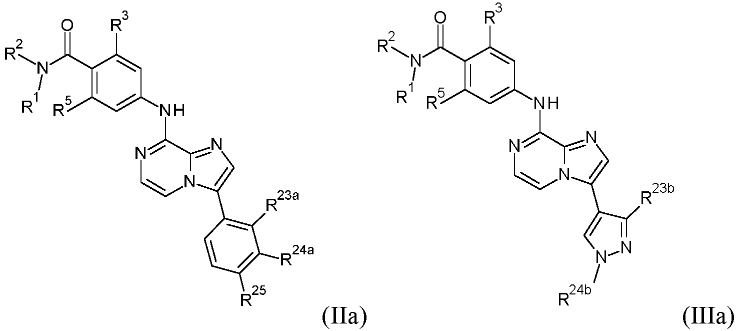 wherein:
R¹ is selected from R⁶-C₁-C₆-alkyl-, R⁶-C₁-C₆-alkyl-CH(C₁-C₆-alkoxycarbonyl)-, R⁷-C₁-C₆-alkyl-X-C₁-C₆-alkyl-, a group 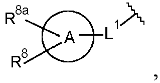
and a group 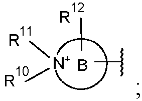 and
R² is selected from hydrogen and C₁-C₆-alkyl;
or R¹ and R², taken together with the nitrogen atom to which they are attached, form a 3- to 14-membered heterocycle that is substituted with R⁹ and R^{9a};
R³ is selected from hydrogen, halogen, and C₁-C₆-alkyl; or
R³ and R², taken together with the atoms to which they are attached, form a 3- to 14-membered heterocycle;
R⁵ is selected from hydrogen, C₁-C₆-alkyl, and halogen;
R⁶ is a group R¹⁶-L²-;
R⁷ is a group R¹⁶-L³-;
R⁸ is a group R¹⁶-L⁴-;
R^{8a}, R^{9a}, R^{11a}, R¹², R¹⁸, R¹⁹, R²⁰, R²¹, and R²² are independently selected from hydrogen, halogen, hydroxy, cyano, amino, carbamoyl, carboxy, C₁-C₆-alkyl, C₁-C₆-alkoxy, halo-C₁-C₆-alkyl, cyano-C₁-C₆-alkyl, carboxy-C₁-C₆-alkyl, hydroxy-C₁-C₆-alkyl, halo-C₁-C₆-alkoxy, cyano-C₁-C₆-alkoxy, C₂-C₆-alkynyloxy, amino-C₂-C₆-alkynyloxy, aryloxy, (5- to 14-membered heteroaryl)oxy, C₁-C₆-alkyl-(5-to 14-membered heteroaryl)oxy, and C₁-C₆-alkoxycarbonyl;
R⁹ is a group R¹⁶-L⁵- or a group 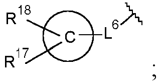
R¹⁰ is selected from C₁-C₆-alkyl, C₁-C₆-alkoxycarbonyl-C₁-C₆-alkyl, C₁-C₆-alkyl-NH-C₁-C₆-alkyl, amino-C₁-C₆-alkyl, carbamoyl, carbamoyl-C₁-C₆-alkyl, carboxy-C₁-C₆-alkyl, cyano-C₁-C₆-alkyl, halo-C₁-C₆-alkyl, hydroxy-C₁-C₆-alkyl, and a group 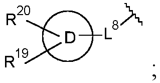
R¹¹ is selected from C₁-C₆-alkyl, carbamoyl, carboxy, carboxy-C₁-C₆-alkyl, and hydroxy-C₁-C₆-alkyl;
R¹³ and R¹⁴ are independently selected from C₁-C₆-alkyl, amino-C₁-C₆-alkyl, carbamoyl-C₁-C₆-alkyl, carboxy-C₁-C₆-alkyl, cyano-C₁-C₆-alkyl, hydroxy-C₁-C₆-alkyl, halo-C₁-C₆-alkyl, C₁-C₆-alkoxycarbonyl-C₁-C₆-alkyl, and a group 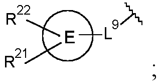
or R¹³ and R¹⁴, taken together with the nitrogen atom to which they are attached, form a 3- to 14-membered heterocycle which is optionally substituted with 1-3 substituents selected from halogen, amino, hydroxy, cyano, C₁-C₆-alkyl, halo-C₁-C₆-alkyl, C₁-C₆-alkoxy, and halo-C₁-C₆-alkoxy;
R¹⁵ is C₁-C₆-alkyl;
R¹⁶ is selected from a group 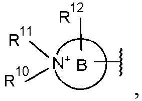 a group 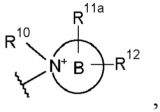 and a group 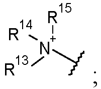
R¹⁷ is a group R¹⁶-L⁷-;
R^{23a} is hydrogen or halogen;
R^{23b} is halo-C₁-C₆-alkyl;
R^{24a} is hydrogen or halogen;
R^{24b} is selected from hydrogen, C₁-C₆-alkyl, cyano-C₁-C₆-alkyl, and halo-C₁-C₆-alkyl;
R²⁵ selected from halogen, C₁-C₆-alkoxy, cyano-C₁-C₆-alkoxy, halo-C₁-C₆-alkoxy, C₂-C₆-alkynyloxy, amino-C₂-C₆-alkynyloxy, aryloxy, (5- to 14-membered heteroaryl)oxy, and C₁-C₆-alkyl-(5- to 14-membered heteroaryl)oxy;
L¹ is selected from a covalent bond, carbonyl, and -C₁-C₆-alkyl-;
L² and L³ are independently selected from a covalent bond, carbonyl, -C₁-C₆-alkyl-C(O)-NH-, -C(O)-NH-, -NH-C(O)-, -C(O)-NH-CH(C₁-C₆-alkoxycarbonyl)-, - C₁-C₆-alkyl-CH(OH)-C₁-C₆-alkyl-C(O)-, and -C₁-C₆-alkyl-NH-;
L⁴, L⁵, L⁶, L⁷, and L⁸ are independently selected from a covalent bond, carbonyl, - C₁-C₆-alkyl-, -C₁-C₆-alkyl-C(O)-, -C₁-C₆-alkyl-NH-C(O)-, -C₁-C₆-alkyl-C(O)-NH-, -C₁-C₆-alkyl-NH-C(O)-C₁-C₆-alkyl-, -C₁-C₆-alkyl-C(O)-NH-C₁-C₆-alkyl-, -C(O)-NH-, -NH-C(O)-, -C(O)-NH-CH(C₁-C₆-alkoxycarbonyl)-, -C₁-C₆-alkyl-CH(OH)-C₁-C₆-alkyl-C(O)-, and -C₁-C₆-alkyl-NH-;
L⁹ is -C₁-C₆-alkyl- or -C(O)-C₁-C₆-alkyl-;
A, C, D, and E are each independently selected from C₆-C₁₀-aryl, C₃-C₁₀-cycloalkyl, 3- to 14-membered heterocyclyl, and 5- to 14-membered heteroaryl;
B is a 3- to 14-membered heterocycle; and
X is selected from O, S, SO, SO₂, NH, and N(C₁-C₆-alkyl).

2. The compound of formula (IIa) or (IIIa) according to claim 1, or a pharmaceutically acceptable salt thereof, wherein:
R¹ is selected from R⁶-C₁-C₆-alkyl-, R⁶-C₁-C₆-alkyl-CH(C₁-C₆-alkoxycarbonyl)-, R⁷-C₁-C₆-alkyl-O-C₁-C₆-alkyl-, a group 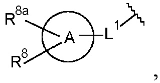 and a group 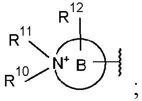
R² is selected from hydrogen and C₁-C₆-alkyl;
R⁶ is a group R¹⁶-L²-;
R⁷ is a group R¹⁶-L³-;
R⁸ is a group R¹⁶-L⁴-;
R^{8a} is hydrogen or C₁-C₆-alkoxycarbonyl;
R¹⁰ is selected from C₁-C₆-alkyl, C₁-C₆-alkoxycarbonyl-C₁-C₆-alkyl, C₁-C₆-alkyl-NH-C₁-C₆-alkyl, amino-C₁-C₆-alkyl, carbamoyl-C₁-C₆-alkyl, carboxy-C₁-C₆-alkyl, cyano-C₁-C₆-alkyl, and a group 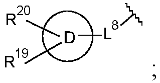
R¹¹ is selected from C₁-C₆-alkyl, carbamoyl, carboxy-C₁-C₆-alkyl, and hydroxy-C₁-C₆-alkyl;
R^{11a} is hydrogen or carboxy;
R¹² is hydrogen or hydroxy;
R¹³ is selected from C₁-C₆-alkyl, amino-C₁-C₆-alkyl, carbamoyl-C₁-C₆-alkyl, carboxy-C₁-C₆-alkyl, cyano-C₁-C₆-alkyl, hydroxy-C₁-C₆-alkyl, C₁-C₆-alkoxycarbonyl-C₁-C₆-alkyl, and a group 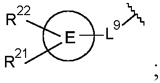 and
R¹⁴ is selected from C₁-C₆-alkyl, carboxy-C₁-C₆-alkyl, a group 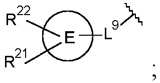 or R¹³ and R¹⁴, taken together with the nitrogen atom to which they are attached, form a 3- to 14-membered heterocycle;
R¹⁵ is C₁-C₆-alkyl;
R¹⁶ is selected from a group 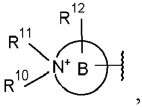 a group 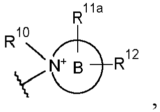 and a group 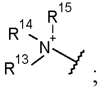
R¹⁷ is a group R¹⁶-L⁷-;
R¹⁸ is hydrogen;
R¹⁹ is selected from hydrogen, amino, and C₁-C₆-alkyl;
R²⁰ is hydrogen or C₁-C₆-alkyl;
R²¹ and R²² are both hydrogen;
L¹ is a covalent bond or -C₁-C₆-alkyl-;
L² is selected from a covalent bond, carbonyl, -C₁-C₆-alkyl-C(O)-NH-, -C(O)-NH-, -C(O)-NH-CH(C₁-C₆-alkoxycarbonyl)-, -C₁-C₆-alkyl-CH(OH)-C₁-C₆-alkyl-C(O)-, and -C₁-C₆-alkyl-NH-;
L³ is a covalent bond;
L⁴ is selected from carbonyl, -C₁-C₆-alkyl-C(O)-, -C₁-C₆-alkyl-C(O)-NH-, -C(O)-NH-, -C₁-C₆-alkyl-CH(OH)-C₁-C₆-alkyl-C(O)-, and -C₁-C₆-alkyl-NH-;
L⁷ is -C₁-C₆-alkyl-;
L⁸ is selected from a covalent bond, -C₁-C₆-alkyl-, and -C₁-C₆-alkyl-NH-C(O)-C₁-C₆-alkyl-;
L⁹ is -C₁-C₆-alkyl- or -C(O)-C₁-C₆-alkyl-;
A is C₃-C₁₀-cycloalkyl or a 3- to 14-membered heterocycle;
B is a 3- to 14-membered heterocycle;
D is selected from C₃-C₁₀-cycloalkyl, 3- to 14-membered heterocyclyl, and 5- to 14-membered heteroaryl; and
E is 3- to 14-membered heterocyclyl.

3. The compound of formula (IIa) or (IIIa) according to claim 1, or a pharmaceutically acceptable salt thereof, wherein:
R¹ is selected from R⁶-C₁-C₆-alkyl-, R⁷-C₁-C₆-alkyl-O-C₁-C₆-alkyl-, and a group 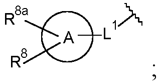
R² is selected from hydrogen and C₁-C₆-alkyl;
R⁶ is a group R¹⁶-L²-;
R⁷ is a group R¹⁶-L³-;
R⁸ is a group R¹⁶-L⁴-;
R^{8a} is hydrogen;
R¹⁰ is selected from carbamoyl-C₁-C₆-alkyl, cyano-C₁-C₆-alkyl, C₁-C₆-alkyl, carboxy-C₁-C₆-alkyl, and a group 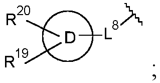
R¹¹ is C₁-C₆-alkyl or carboxy-C₁-C₆-alkyl;
R^{11a} and R¹² are both hydrogen;
R¹³ is selected from C₁-C₆-alkyl, amino-C₁-C₆-alkyl, carbamoyl-C₁-C₆-alkyl, and a group 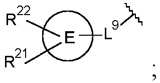
R¹⁴ is C₁-C₆-alkyl or carboxy-C₁-C₆-alkyl;
R¹⁵ is C₁-C₆-alkyl;
R¹⁶ is selected from a group 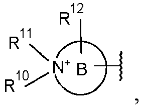 a group 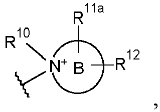 and a group 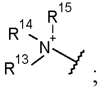
R¹⁹ and R²⁰ are both hydrogen;
R²¹ and R²² are both hydrogen;
L¹, L², and L³ are a covalent bond;
L⁴ is -C₁-C₆-alkyl-C(O)- or -C₁-C₆-alkyl-C(O)-NH-;
L⁸ and L⁹ are -C₁-C₆-alkyl-;
A is C₃-C₁₀-cycloalkyl or a 3- to 14-membered heterocycle;
B is a 3- to 14-membered heterocycle;
D is 3- to 14-membered heterocyclyl; and
E is 3- to 14-membered heterocyclyl.

4. The compound of formula (IIa) or (IIIa) according to claim 1, or a pharmaceutically acceptable salt thereof, wherein:
R¹ is selected from R⁶-(CH₂)ₙ-, R⁷-(CH₂)₂-O-(CH₂)₂-, and a group 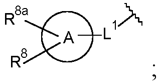
R² is selected from hydrogen and methyl;
R⁶ is a group R¹⁶-L²-;
R⁷ is a group R¹⁶-L³-;
R⁸ is a group R¹⁶-L⁴-;
R^{8a} is hydrogen;
R¹⁰ is selected from carbamoyl-CH₂-, methyl, carboxy-CH₂-, cyano-CH₂-, and a group 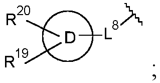
R¹¹ is methyl or carboxy-CH₂-;
R^{11a} and R¹² are both hydrogen;
R¹³ is selected from methyl, aminopropyl, carbamoyl-CH₂-, and a group 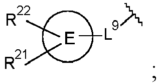
R¹⁴ is methyl or carboxymethyl;
R¹⁵ is methyl;
R¹⁶ is selected from a group 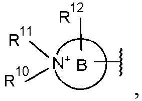 a group 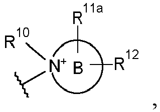 and a group 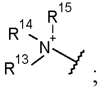
R¹⁹ and R²⁰ are both hydrogen;
R²¹ and R²² are both hydrogen;
L¹, L², and L³ are a covalent bond;
L⁴ is -CH₂-C(O)- or -CH₂-C(O)-NH-;
L⁸ and L⁹ are both -CH₂-;
A is cyclohexyl or pyrrolidinyl;
B is pyrrolidinyl, piperidyl, 3-azabicyclo[3.1.0]hexan-6-yl;
D is pyrrolidin or azetidin;
E is azetidine; and
n is 1, 2 or 5.

5. The compound of formula (IIa) or (IIIa) according to claim 1, or a pharmaceutically acceptable salt thereof, wherein:
R¹ and R², taken together with the nitrogen atom to which they are attached, form a 3- to 14-membered heterocycle that is substituted with R⁹ and R^{9a};
R⁹ is a group R¹⁶-L⁵- or a group 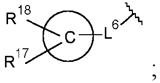
R^{9a} is selected from hydrogen, hydroxy, and C₁-C₆-alkoxycarbonyl;
R¹⁰ is selected from C₁-C₆-alkyl, C₁-C₆-alkyl-NH-C₁-C₆-alkyl, amino-C₁-C₆-alkyl, carbamoyl-C₁-C₆-alkyl, and a group 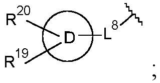
R¹¹ is C₁-C₆-alkyl;
R¹² is hydrogen or hydroxy;
R¹³ is C₁-C₆-alkyl or a group 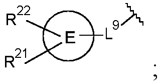 and
R¹⁴ and R¹⁵ are both C₁-C₆-alkyl;
R¹⁶ is a group 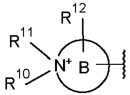 or a group 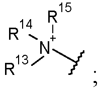
R¹⁷ is a group R¹⁶-L⁷-;
R¹⁸ is hydrogen;
R¹⁹ is hydrogen or amino;
R²⁰, R²¹, R²² are hydrogen;
B is a 3- to 14-membered heterocycle;
C is a 3- to 14-membered heterocycle;
D is C₃-C₁₀-cycloalkyl or 3- to 14-membered heterocyclyl;
E is a 3- to 14-membered heterocycle;
L⁵ is selected from a covalent bond, carbonyl, -C₁-C₆-alkyl-, -C₁-C₆-alkyl-C(O)-, and -C₁-C₆-alkyl-NH-C(O)-;
L⁶ is carbonyl;
L⁷ is -C₁-C₆-alkyl-; and
L⁸ and L⁹ are both -C₁-C₆-alkyl-.

6. The compound of formula (IIa) or (IIIa) according to claim 1, or a pharmaceutically acceptable salt thereof, wherein:
R¹ and R², taken together with the nitrogen atom to which they are attached, form a 3- to 14-membered heterocycle that is substituted with R⁹ and R^{9a};
R⁹ is a group R¹⁶-L⁵-;
R^{9a} is hydrogen;
R¹⁰ is carbamoyl-C₁-C₆-alkyl or a group 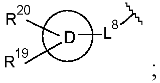
R¹¹ is C₁-C₆-alkyl;
R¹³, R¹⁴, and R¹⁵ are C₁-C₆-alkyl;
R¹⁶ is a group 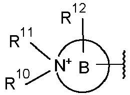 or a group 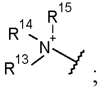
R¹², R¹⁹, and R²⁰ are hydrogen;
B is a 3- to 14-membered heterocycle;
D is a 3- to 14-membered heterocycle;
L⁵ is selected from carbonyl, -C₁-C₆-alkyl-, and -C₁-C₆-alkyl-C(O)-; and
L⁸ is -C₁-C₆-alkyl-.

7. The compound of formula (IIa) or (IIIa) according to claim 1, or a pharmaceutically acceptable salt thereof, wherein:
R¹ and R², taken together with the nitrogen atom to which they are attached, form a piperazinyl ring that is substituted with R⁹ and R^{9a};
R⁹ is a group R¹⁶-L⁵-;
R^{9a} is hydrogen;
R¹⁰ is carbamoylmethyl or a group 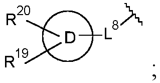
R¹¹ is methyl;
R¹³, R¹⁴, and R¹⁵ are methyl;
R¹⁶ is a group 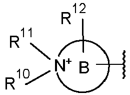 or a group 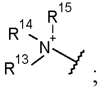
R¹², R¹⁹, and R²⁰ are hydrogen;
B is piperidyl;
D is pyrrolidinyl;
L⁵ is selected from carbonyl, -(CH₂)₂-, and -CH₂-C(O)-; and
L⁸ is -CH₂-.

8. The compound of formula (IIa) or (IIIa) according to any one of claims 1 to 7, or a pharmaceutically acceptable salt thereof, wherein R³ is selected from fluoro, chloro, methyl and ethyl;
or R³ and R², taken together with the atoms to which they are attached, form a 6-oxo-2,3-dihydropyridinyl ring.

9. The compound of formula (IIa) or (IIIa) according to any one of claims 1 to 8, or a pharmaceutically acceptable salt thereof, wherein R⁵ is hydrogen or C₁-C₆-alkyl.

10. The compound of formula (IIa) or (IIIa) according to any one of claims 1 to 8, or a pharmaceutically acceptable salt thereof, wherein R⁵ is hydrogen or methyl.

11. The compound of formula (IIa) or (IIIa) according to claim 1, or a pharmaceutically acceptable salt thereof, wherein the compound of formula (IIa) or (IIIa) is selected from:
2-(2-(4-((3-(4-(difluoromethoxy)phenyl)imidazo[1,2-a]pyrazin-8-yl)amino)-2-methylbenzamido)ethoxy)-N,N,N-trimethylethanaminium iodide;
4-(2-(4-((3-(4-(difluoromethoxy)phenyl)imidazo[1,2-a]pyrazin-8-yl)amino)-N,2-dimethylbenzamido)ethyl)-1,1-dimethylpiperidin-1-ium formate;
1-ethyl-4-(2-(4-((3-(4-methoxyphenyl)imidazo[1,2-a]pyrazin-8-yl)amino)-2-methylbenzamido)ethyl)-1-methylpiperidin-1-ium formate;
2-chloro-4-[[3-(2,3-difluoro-4-methoxy-phenyl)imidazo[1,2-a]pyrazin-8-yl]amino]-N-[2-(4,4-dimethylpiperazin-4-ium-1-yl)ethyl]-N-methyl-benzamide 2,2,2-trifluoroacetate;
4-[[3-(2,3-Difluoro-4-methoxy-phenyl)imidazo[1,2-a]pyrazin-8-yl]amino]-N-[2-(1,1-dimethylpiperidin-1-ium-4-yl)ethyl]-N,2-dimethyl-benzamide 2,2,2-trifluoroacetate;
1-(carboxymethyl)-4-(2-(4-((3-(4-methoxyphenyl)imidazo[1,2-a]pyrazin-8-yl)amino)-2-methylbenzamido)ethyl)-1-methylpiperidin-1-ium formate;
4-(2-(4-((3-(4-(difluoromethoxy)phenyl)imidazo[1,2-a]pyrazin-8-yl)amino)-N,2-dimethylbenzamido)ethyl)-1, 1-diethylpiperidin-1-ium formate;
2-(2-(4-((3-(2,3-difluoro-4-methoxyphenyl)imidazo[1,2-a]pyrazin-8-yl)amino)-2-ethylbenzamido)ethoxy)-N,N,N-trimethylethanaminium formate;
6-(4-((3-(4-(difluoromethoxy)phenyl)imidazo[1,2-a]pyrazin-8-yl)amino)-2-methylbenzamido)-N,N,N-trimethylhexan-1-aminium formate;
2-[4-[2-chloro-4-[[3-[4-(cyanomethoxy)-2,3-difluorophenyl]imidazo[1,2-a]pyrazin-8-yl]amino]benzoyl]piperazin-1-yl]ethyl-trimethylazanium iodide;
2-(1-(4-((3-(3-Fluoro-4-methoxyphenyl)imidazo[1,2-a]pyrazin-8-yl)amino)-2-methylbenzoyl)piperidine-4-carboxamido)-N,N,N-trimethylethanaminium formate;
3-(4-((3-(4-Chloro-2,3-difluorophenyl)imidazo[1,2-a]pyrazin-8-yl)amino)-2-ethylbenzamido)-N,N,N-trimethylpropan-1-aminium formate;
2-(1-(4-((3-(4-(Difluoromethoxy)phenyl)imidazo[1,2-a]pyrazin-8-yl)amino)-2-methylbenzoyl)piperidin-4-yl)-N,N,N-trimethylethanaminium formate;
[4-[[3-[4-(Difluoromethoxy)phenyl]imidazo[1,2-a]pyrazin-8-yl]amino]-2-methylphenyl]-[4-(4,4-dimethylpiperazin-4-ium-1-carbonyl)piperazin-1-yl]methanone formate;
[2-[4-[2-Chloro-4-[[3-(3-fluoro-4-methoxyphenyl)imidazo[1,2-a]pyrazin-8-yl]amino]benzoyl]piperazin-1-yl]-2-oxoethyl]-trimethylazanium formate;
2-(4-(4-((3-(3-Fluoro-4-methoxyphenyl)imidazo[1,2-a]pyrazin-8-yl)amino)-2-methylbenzoyl)piperazin-1-yl)-N,N,N-trimethyl-2-oxoethan-1-aminium iodide;
[4-[4-[4-[[3-[4-(Difluoromethoxy)phenyl]imidazo[1,2-a]pyrazin-8-yl]amino]-2-methyl-benzoyl]piperazine-1-carbonyl]morpholin-2-yl]methyl-trimethyl-ammonium formate;
2-Chloro-4-[[3-[4-(cyanomethoxy)-2,3-difluoro-phenyl]imidazo[1,2-a]pyrazin-8-yl]amino]-N-[2-(4,4-dimethylpiperazin-4-ium-1-yl)ethyl]-N-methyl-benzamide iodide;
2-(2-(4-((3-(3-Fluoro-4-methoxyphenyl)imidazo[1,2-a]pyrazin-8-yl)amino)-N,2-dimethylbenzamido)ethoxy)-N,N,N-trimethylethan-1-aminium chloride;
2-[[4-[4-[[3-[4-(Difluoromethoxy)phenyl]imidazo[1,2-a]pyrazin-8-yl]amino]-2-methyl-benzoyl]piperazine-1-carbonyl] amino] ethyl-trimethyl-ammonium formate;
2-[[(3S,4R)-1-[4-[[3-(3-fluoro-4-methoxy-phenyl)imidazo[1,2-a]pyrazin-8-yl]amino]-2-methyl-benzoyl]-3-hydroxy-piperidine-4-carbonyl]amino]ethyl-trimethyl-ammonium formate;
2-Chloro-4-[[3-[4-(cyanomethoxy)-2,3-difluoro-phenyl]imidazo[1,2-a]pyrazin-8-yl]amino]-N-[2-(1,1-dimethylpiperidin-1-ium-4-yl)ethyl]-N-methyl-benzamide 2,2,2-trifluoroacetate;
4-(4-(2-(4-((3-(4-(Difluoromethoxy)phenyl)imidazo[1,2-a]pyrazin-8-yl)amino)-N,2-dimethylbenzamido)ethyl)piperidin-1-yl)-2-hydroxy-N,N,N-trimethyl-4-oxobutan-1-aminium formate;
2-(2-(4-((3-(2,3-Difluoro-4-methoxyphenyl)imidazo[1,2-a]pyrazin-8-yl)amino)-2-ethylbenzamido)ethoxy)-N,N,N-triethylethan-1-aminium formate;
2-[2-[[4-[[3-[4-(Cyanomethoxy)-2,3-difluoro-phenyl]imidazo[1,2-a]pyrazin-8-yl]amino]-2-ethyl-benzoyl]amino]ethoxy]ethyl-trimethyl-ammonium iodide;
[2-[4-[4-[[3-[4-(Difluoromethoxy)phenyl]imidazo[1,2-a]pyrazin-8-yl]amino]-2-methyl-benzoyl]piperazin-1-yl]-2-oxo-ethyl]-trimethyl-ammonium chloride;
2-[2-[[4-[[3-[2-chloro-4-(cyanomethoxy)-3-fluoro-phenyl]imidazo[1,2-a]pyrazin-8-yl]amino]-2-ethyl-benzoyl]amino]ethoxy]ethyl-trimethyl-ammonium iodide;
2-[1-[4-[[3-[4-(Cyanomethoxy)-2,3-difluoro-phenyl]imidazo[1,2-a]pyrazin-8-yl]amino]-2-methyl-benzoyl]-4-piperidyl]ethyl-trimethyl-ammonium formate;
2-[4-[2-Bromo-4-[[3-(3-fluoro-4-methoxy-phenyl)imidazo[1,2-a]pyrazin-8-yl]amino]benzoyl]piperazin-1-yl]ethyl-trimethyl-ammonium 2,2,2-trifluoroacetate;
2-[2-[[2-bromo-4-[[3-(2,3-difluoro-4-methoxy-phenyl)imidazo[1,2-a]pyrazin-8-yl]amino]benzoyl]amino]ethoxy]ethyl-trimethyl-ammonium 2,2,2-trifluoroacetate;
2-[2-[6-[[3-(2,3-difluoro-4-methoxy-phenyl)imidazo[1,2-a]pyrazin-8-yl]amino]-1-oxo-3,4-dihydroisoquinolin-2-yl]ethoxy]ethyl-trimethyl-ammonium formate;
2-[2-[[2-Chloro-4-[[3-[4-(cyanomethoxy)-2,3-difluoro-phenyl]imidazo[1,2-a]pyrazin-8-yl]amino]benzoyl]amino]ethoxy]ethyl-tripropyl-ammonium iodide;
2-Chloro-4-[[3-[4-(cyanomethoxy)-2,3-difluoro-phenyl]imidazo[1,2-a]pyrazin-8-yl]amino]-N-[2-[2-(1-methylpyrrolidin-1-ium-1-yl)ethoxy]ethyl]benzamide formate;
3-[[2-Chloro-4-[[3-(2,3-difluoro-4-methoxy-phenyl)imidazo[1,2-a]pyrazin-8-yl]amino]benzoyl]amino]propyl-trimethyl-ammonium formate;
3-[[2-Chloro-4-[[3-(2,3-difluoro-4-methoxy-phenyl)imidazo[1,2-a]pyrazin-8-yl]amino]benzoyl]amino]propyl-triethyl-ammonium formate;
Tributyl-[3-[[2-chloro-4-[[3-(2,3-difluoro-4-methoxy-phenyl)imidazo[1,2-a]pyrazin-8-yl]amino]benzoyl]amino]propyl]ammonium formate;
Carboxymethyl-[2-[2-[[4-[[3-(2,3-difluoro-4-methoxy-phenyl)imidazo[1,2-a]pyrazin-8-yl]amino]-2-ethyl-benzoyl]amino]ethoxy]ethyl]-dimethyl-ammonium formate;
2-[2-[[4-[[3-(2,3-Difluoro-4-methoxy-phenyl)imidazo[1,2-a]pyrazin-8-yl]amino]-2-ethyl-benzoylamino]ethoxy]ethyl-(2-hydroxyethyl)-dimethyl-ammonium formate;
2-[2-[[4-[[3-(2,3-Difluoro-4-prop-2-ynoxy-phenyl)imidazo[1,2-a]pyrazin-8-yl]amino]-2-ethyl-benzoyl]amino]ethoxy]ethyl-trimethyl-ammonium 2,2,2-trifluoroacetate;
(2-Amino-2-oxo-ethyl)-[2-[2-[[4-[[3-(2,3-difluoro-4-methoxy-phenyl)imidazo[1,2-a]pyrazin-8-yl]amino]-2-ethyl-benzoyl]amino]ethoxy]ethyl]-dimethyl-ammonium formate;
3-Carboxypropyl-[2-[2-[[4-[[3-(2,3-difluoro-4-methoxy-phenyl)imidazo[1,2-a]pyrazin-8-yl]amino]-2-ethyl-benzoyl]amino]ethoxy]ethyl]-dimethyl-ammonium formate;
2-(4-(2-Chloro-4-((3-(4-(cyanomethoxy)-2,3-difluorophenyl)imidazo[1,2-a]pyrazin-8-yl)amino)benzoyl)piperazin-1-yl)-N,N,N-trimethyl-2-oxoethan-1-aminium formate;
2-(4-(2-Chloro-4-((3-(2,3-difluoro-4-methoxyphenyl)imidazo[1,2-a]pyrazin-8-yl)amino)benzoyl)piperazin-1-yl)-N,N,N-trimethyl-2-oxoethan-1-aminium iodide;
(2S,4R)-2-(4-(4-((3-(2,3-Difluoro-4-methoxyphenyl)imidazo[1,2-a]pyrazin-8-yl)amino)-2-methylbenzoyl)piperazine-1-carbonyl)-4-hydroxy-1,1-dimethylpyrrolidin-1-ium formate;
[2-[4-[4-[[3-[4-(Cyanomethoxy)-2,3-difluorophenyl]imidazo[1,2-a]pyrazin-8-yl]amino ]-2-methylbenzoyl]piperazin-1-yl]-2-oxoethyl]-trimethylazanium iodide;
4-[4-[2-[[4-[[3-[4-(Difluoromethoxy)phenyl]imidazo[1,2-a]pyrazin-8-yl]amino]-2-methylbenzoyl]-methylamino]ethyl]-1-methylpiperidin-1-ium-1-yl]butanoic acid hydrochloride;
[2-[2-[[2-Chloro-4-[[3-[3-(trifluoromethyl)-1H-pyrazol-4-yl]imidazo[1,2-a]pyrazin-8-yl]amino]benzoyl]amino]ethylamino]-2-oxo-ethyl]-trimethyl-ammonium formate;
[2-[[3-[[2-Chloro-4-[[3-(2,3-difluoro-4-methoxy-phenyl)imidazo[1,2-a]pyrazin-8-yl]amino]benzoyl]amino]cyclobutyl]amino]-2-oxo-ethyl]-trimethyl-ammonium formate;
2-(4-(4-((3-(2,3-Difluoro-4-methoxyphenyl)imidazo[1,2-a]pyrazin-8-yl)amino)-2-methylbenzamido)piperidin-1-yl)-N,N,N-trimethyl-2-oxoethan-1-aminium chloride;
[2-[3-[[2-Chloro-4-[[3-[3-(trifluoromethyl)-1H-pyrazol-4-yl]imidazo[1,2-a]pyrazin-8-yl]amino]benzoyl]amino]propylamino]-2-oxo-ethyl]-trimethyl-ammonium formate;
[2-[[3-[[2-Chloro-4-[[3-[3-(trifluoromethyl)-1H-pyrazol-4-yl]imidazo[1,2-a]pyrazin-8-yl]amino]benzoyl]amino]cyclobutyl]amino]-2-oxo-ethyl]-trimethyl-ammonium formate;
[2-[4-[2-Chloro-4-[[3-[3-(trifluoromethyl)-1H-pyrazol-4-yl]imidazo[1,2-a]pyrazin-8-yl]amino]benzoyl]piperazin-1-yl]-2-oxo-ethyl]-trimethyl-ammonium formate;
2-(((1s,4s)-4-(4-((3-(2,3-Difluoro-4-methoxyphenyl)imidazo[1,2-a]pyrazin-8-yl)amino)-2-methylbenzamido)cyclohexyl)amino)-N,N,N-trimethyl-2-oxoethan-1-aminium chloride;
[2-[4-[4-[[3-[1-(Cyanomethyl)-3-(trifluoromethyl)pyrazol-4-yl]imidazo[1,2-a]pyrazin-8-yl]amino]-2-methyl-benzoyl]piperazin-1-yl]-2-oxo-ethyl]-trimethyl-ammonium formate;
[2-[4-[2-Chloro-4-[[3-[1-(cyanomethyl)-3-(trifluoromethyl)pyrazol-4-yl]imidazo[1,2-a]pyrazin-8-yl]amino]benzoyl]piperazin-1-yl]-2-oxo-ethyl]-trimethyl-ammonium formate;
2-(2-(4-((3-(2,3-Difluoro-4-methoxyphenyl)imidazo[1,2-a]pyrazin-8-yl)amino)-2-fluoro-6-methylbenzamido)ethoxy)-N,N,N-trimethylethan-1-aminium hydrogen carbonate;
2-[[3-[[2-Chloro-4-[[3-(2,3-difluoro-4-methoxy-phenyl)imidazo[1,2-a]pyrazin-8-yl]amino]benzoyl]amino]cyclobutyl]amino]ethyl-trimethyl-ammonium formate;
(S)-2-(3-(4-((3-(2,3-Difluoro-4-methoxyphenyl)imidazo[1,2-a]pyrazin-8-yl)amino)-2-methylbenzamido)pyrrolidin-1-yl)-N,N,N-trimethyl-2-oxoethan-1-aminium chloride;
2-(3-(4-((3-(2,3-Difluoro-4-methoxyphenyl)imidazo[1,2-a]pyrazin-8-yl)amino)-2-methylbenzamido)piperidin-1-yl)-N,N,N-trimethyl-2-oxoethan-1-aminium chloride;
2-(((1r,4r)-4-(4-((3-(2,3-Difluoro-4-methoxyphenyl)imidazo[1,2-a]pyrazin-8-yl)amino)-2-methylbenzamido)cyclohexyl)amino)-N,N,N-trimethyl-2-oxoethan-1-aminium chloride;
(R)-2-(3-(4-((3-(2,3-difluoro-4-methoxyphenyl)imidazo[1,2-a]pyrazin-8-yl)amino)-2-methylbenzamido)pyrrolidin-1-yl)-N,N,N-trimethyl-2-oxoethan-1-aminium chloride;
2-Amino-N-(2-(3-(4-((3-(2,3-difluoro-4-methoxyphenyl)imidazo[1,2-a]pyrazin-8-yl)amino)-2-ethylbenzamido)azetidin-1-yl)-2-oxoethyl)-N,N-dimethyl-2-oxoethan-1-aminium formate;
2-(((1s,3s)-3-(4-((3-(2,3-Difluoro-4-methoxyphenyl)imidazo[1,2-a]pyrazin-8-yl)amino)-2-methylbenzamido)cyclobutyl)amino)-N,N,N-trimethyl-2-oxoethan-1-aminium chloride;
3-(2-chloro-4-((3-(2,3-difluoro-4-methoxyphenyl)imidazo[1,2-a]pyrazin-8-yl)amino)benzamido)-N-(2-hydroxyethyl)-N,N-dimethylpropan-1-aminium formate;
N-(Cyanomethyl)-2-(2-(4-((3-(2,3-difluoro-4-methoxyphenyl)imidazo[1,2-a]pyrazin-8-yl)amino)-2-ethylbenzamido)ethoxy)-N,N-dimethylethan-1-aminium formate;
N-(2-(2-(4-((3-(2,3-Difluoro-4-methoxyphenyl)imidazo[1,2-a]pyrazin-8-yl)amino)-2-ethylbenzamido)ethoxy)ethyl)-2-ethoxy-N,N-dimethyl-2-oxoethan-1-aminium formate;
Mono(N-(2-amino-2-oxoethyl)-5-(4-((3-(2,3-difluoro-4-methoxyphenyl)imidazo[1,2-a]pyrazin-8-yl)amino)-2-ethylbenzamido)-N,N-dimethylpentan-1-aminium) monoformate monoiodide;
Trans-[2-[[4-[[4-[[3-(2,3-Difluoro-4-methoxyphenyl)imidazo[1,2-a]pyrazin-8-yl]amino]-2-ethylbenzoyl]amino]cyclohexyl]amino]-2-oxoethyl]-trimethylazanium formate;
1-(Cyanomethyl)-1-(2-(2-(4-((3-(1-(cyanomethyl)-3-(trifluoromethyl)-1H-pyrazol-4-yl)imidazo[1,2-a]pyrazin-8-yl)amino)-2-ethylbenzamido)ethoxy)ethyl)pyrrolidin-1-ium formate;
4-(Cyanomethyl)-4-(2-(2-(4-((3-(1-(cyanomethyl)-3-(trifluoromethyl)-1H-pyrazol-4-yl)imidazo[1,2-a]pyrazin-8-yl)amino)-2-ethylbenzamido)ethoxy)ethyl)morpholin-4-ium formate;
1-(2-amino-2-oxoethyl)-4-(4-(4-((3-(4-(difluoromethoxy)-2,3-difluorophenyl)imidazo[1,2-a]pyrazin-8-yl)amino)-2-ethylbenzoyl)piperazine-1-carbonyl)-1-methylpiperidin-1-ium formate;
5-[[4-[[3-[4-(Difluoromethoxy)-2,3-difluoro-phenyl]imidazo[1,2-a]pyrazin-8-yl]amino]-2-ethyl-benzoyl]amino]pentyl-dimethyl-(2-oxo-2-piperazin-l-yl-ethyl)ammonium bis(2,2,2-trifluoroacetate);
N-[[3-(azetidin-3-ylmethyl)-3-methyl-3-azoniabicyclo[3.1.0]hexan-6-yl]methyl]-4-[[3-[4-(difluoromethoxy)-2,3-difluoro-phenyl]imidazo[1,2-a]pyrazin-8-yl]amino]-2-ethyl-benzamide bis(2,2,2-trifluoroacetate);
1-(Azetidin-3-ylmethyl)-4-(4-(4-((3-(4-(difluoromethoxy)-2,3-difluorophenyl)imidazo[1,2-a]pyrazin-8-yl)amino)-2-ethylbenzoyl)piperazine-1-carbonyl)-1-methylpiperidin-1-ium 2,2,2-trifluoroacetate;
N-[3-[[4-[[3-[4-(Difluoromethoxy)-2,3-difluoro-phenyl]imidazo[1,2-a]pyrazin-8-yl]amino]-2-ethyl-benzoyl]amino]propyl]-1-methyl-1-(pyrrolidin-3-ylmethyl)piperidin-1-ium-4-carboxamide bis (2,2,2-trifluoroacetate);
[4-[1-(2-aminoethyl)-1-methyl-piperidin-1-ium-4-carbonyl]piperazin-1-yl]-[4-[[3-[4-(difluoromethoxy)-2,3-difluoro-phenyl]imidazo[1,2-a]pyrazin-8-yl]amino]-2-ethyl-phenyl]methanone bis(2,2,2-trifluoroacetate);
[4-[[3-[4-(difluoromethoxy)-2,3-difluoro-phenyl]imidazo[1,2-a]pyrazin-8-yl]amino]-2-ethyl-phenyl]-[4-[1-methyl-1-(pyrrolidin-3-ylmethyl)piperidin-1-ium-4-carbonyl]piperazin-1-yl]methanone bis(2,2,2-trifluoroacetate);
[4-[[3-[4-(difluoromethoxy)-2,3-difluoro-phenyl]imidazo[1,2-a]pyrazin-8-yl]amino]-2-ethyl-phenyl]-[4-[1-methyl-1-[2-(methylamino)ethyl]piperidin-1-ium-4-carbonyl]piperazin-1-yl]methanone bis(2,2,2-trifluoroacetate);
[4-[1-[(3-amino-1-bicyclo[1.1.1]pentanyl)methyl]-1-methyl-piperidin-1-ium-4-carbonyl]piperazin-1-yl]-[4-[[3-[4-(difluoromethoxy)-2,3-difluorophenyl]imidazo[1,2-a]pyrazin-8-yl]amino]-2-ethyl-phenyl]methanone bis(2,2,2-trifluoroacetate);
N-[2-[1-(azetidin-3-ylmethyl)-1-methyl-piperidin-1-ium-4-yl]ethyl]-4-[[3-[4-(difluoromethoxy)-2,3-difluoro-phenyl]imidazo[1,2-a]pyrazin-8-yl]amino]-2-ethyl-benzamide diformate;
N-[[1-(azetidin-3-ylmethyl)-1-methyl-piperidin-1-ium-4-yl]methyl]-4-[[3-[4-(difluoromethoxy)-2,3-difluoro-phenyl]imidazo[1,2-a]pyrazin-8-yl]amino]-2-ethyl-benzamide diformate;
N-[[1-(azetidin-3-ylmethyl)-1-methyl-pyrrolidin-1-ium-3-yl]methyl]-4-[[3-[4-(difluoromethoxy)-2,3-difluoro-phenyl]imidazo[1,2-a]pyrazin-8-yl]amino]-2-ethyl-benzamide bis(2,2,2-trifluoroacetate);
Bis(azetidin-3-ylmethyl)-[5-[[4-[[3-[4-(difluoromethoxy)-2,3-difluorophenyl]imidazo[1,2-a]pyrazin-8-yl]amino]-2-ethyl-benzoyl]amino]pentyl]-methyl-ammonium bis(2,2,2-trifluoroacetate);
N-[2-[1-(azetidin-3-ylmethyl)-1-methyl-azetidin-1-ium-3-yl]ethyl]-4-[[3-[4-(difluoromethoxy)-2,3-difluoro-phenyl]imidazo[1,2-a]pyrazin-8-yl]amino]-2-ethyl-benzamide diformate;
N-[2-[1-(azetidin-3-ylmethyl)-1-methyl-piperidin-1-ium-4-yl]ethyl]-4-[[3-[4-(difluoromethoxy)-2,3-difluoro-phenyl]imidazo[1,2-a]pyrazin-8-yl]amino]-2-ethyl-benzamide diformate;
N-[3-[4-(Azetidin-3-ylmethyl)-4-methyl-piperazin-4-ium-1-yl]-3-oxo-propyl]-4-[[3-[4-(difluoromethoxy)-2,3-difluoro-phenyl]imidazo[1,2-a]pyrazin-8-yl]amino]-2-ethyl-benzamide bis(2,2,2-trifluoroacetate);
Methyl 4-[1-(azetidin-3-ylmethyl)-1-methyl-piperidin-1-ium-4-carbonyl]-1-[4-[[3-[4-(difluoromethoxy)-2,3-difluoro-phenyl]imidazo[1,2-a]pyrazin-8-yl]amino]-2-ethyl-benzoyl]piperazine-2-carboxylate bis(2,2,2-trifluoroacetate);
methyl 1-[1-(azetidin-3-ylmethyl)-1-methyl-piperidin-1-ium-4-carbonyl]-4-[4-[[3-[4-(difluoromethoxy)-2,3-difluoro-phenyl]imidazo[1,2-a]pyrazin-8-yl]amino]-2-ethyl-benzoyl]piperazine-2-carboxylate tris(2,2,2-trifluoroacetate);
[4-[[3-[4-(difluoromethoxy)-2,3-difluoro-phenyl]imidazo[1,2-a]pyrazin-8-yl]amino]-2-ethyl-phenyl]-[2-[1-methyl-1-(pyrrolidin-3-ylmethyl)piperidin-1-ium-4-carbonyl]-2,6-diazaspiro[3.3]heptan-6-yl]methanone bis(2,2,2-trifluoroacetate);
Azetidin-3-ylmethyl-[5-[[4-[[3-[4-(difluoromethoxy)-2,3-difluorophenyl]imidazo[1,2-a]pyrazin-8-yl]amino]-2-ethyl-benzoyl]amino]pentyl]-dimethyl-ammonium bis(2,2,2-trifluoroacetate);
[4-[[3-[4-(Difluoromethoxy)-2,3-difluoro-phenyl]imidazo[1,2-a]pyrazin-8-yl]amino]-2-ethyl-phenyl]-[4-[4-methyl-4-(pyrrolidin-3-ylmethyl)piperazin-4-ium-1-carbonyl]-1-piperidyl]methanone 2,2,2-trifluoroacetate;
N-[[1-(Azetidin-3-ylmethyl)-1-methyl-pyrrolidin-1-ium-3-yl]methyl]-1-[4-[[3-[4-(difluoromethoxy)-2,3-difluoro-phenyl]imidazo[1,2-a]pyrazin-8-yl]amino]-2-ethyl-benzoyl]piperidine-4-carboxamide formate / 2,2,2-trifluoroacetate (mixed salt);
[4-[2-(azetidin-3-ylmethyl)-2-methyl-6-aza-2-azoniaspiro[3.3]-6-carbonyl]-1-piperidyl]-[4-[[3-[4-(difluoromethoxy)-2,3-difluoro-phenyl]imidazo[1,2-a]pyrazin-8-yl]amino]-2-ethyl-phenyl]methanone formic acid 2,2,2-trifluoroacetate (mixed salt);
[4-[[3-[4-(Difluoromethoxy)-2,3-difluoro-phenyl]imidazo[1,2-a]pyrazin-8-yl]amino]-2-ethyl-phenyl]-[4-[rac-(1S,4S)-5-(azetidin-3-ylmethyl)-5-methyl-2-aza-5-azoniabicyclo[2.2.1]heptane-2-carbonyl]-1-piperidyl]methanone;formic acid;2,2,2-trifluoroacetate (mixed salt);
[4-[6-(Azetidin-3-ylmethyl)-6-methyl-3-aza-6-azoniabicyclo[3.1.1]heptane-3-carbonyl]-1-piperidyl]-[4-[[3-[4-(difluoromethoxy)-2,3-difluorophenyl]imidazo[1,2-a]pyrazin-8-yl]amino]-2-ethyl-phenyl]methanone formate 2,2,2-trifluoroacetate (mixed salt);
N-[2-[[4-[[3-[4-(Difluoromethoxy)-2,3-difluoro-phenyl]imidazo[1,2-a]pyrazin-8-yl]amino]-2-ethyl-benzoyl]amino]ethyl]-1-methyl-1-(pyrrolidin-3-ylmethyl)piperidin-1-ium-4-carboxamide bis(2,2,2-trifluoroacetate);
[4-[[3-[4-(Difluoromethoxy)-2,3-difluoro-phenyl]imidazo[1,2-a]pyrazin-8-yl]amino]-2-fluoro-phenyl]-[4-[1-methyl-1-(pyrrolidin-3-ylmethyl)piperidin-1-ium-4-carbonyl]piperazin-1-yl]methanone 2,2,2-trifluoroacetate;
3-Aminopropyl-(carboxymethyl)-[5-[[4-[[3-(2,3-difluoro-4-methoxyphenyl)imidazo[1,2-a]pyrazin-8-yl]amino]-2-ethyl-benzoyl]amino]pentyl]-methyl-ammonium formate;
5-Aminopentyl-(carboxymethyl)-[5-[[4-[[3-(2,3-difluoro-4-methoxyphenyl)imidazo[1,2-a]pyrazin-8-yl]amino]-2-ethyl-benzoyl]amino]pentyl]-methyl-ammonium 2,2,2-trifluoroacetate;
4-aminobutyl-(carboxymethyl)-[5-[[4-[[3-(2,3-difluoro-4-methoxyphenyl)imidazo[1,2-a]pyrazin-8-yl]amino]-2-ethyl-benzoyl]amino]pentyl]-methyl-ammonium formate;
Azetidin-3-ylmethyl-(carboxymethyl)-[5-[[4-[[3-(2,3-difluoro-4-methoxyphenyl)imidazo[1,2-a]pyrazin-8-yl]amino]-2-ethyl-benzoyl]amino]pentyl]-methyl-ammonium formate;
Azetidin-3-ylmethy1-(3-carboxypropyl)-[5-[[4-[[3-(2,3-difluoro-4-methoxyphenyl)imidazo[1,2-a]pyrazin-8-yl]amino]-2-ethyl-benzoyl]amino]pentyl]-methyl-ammonium 2,2,2-trifluoroacetate;
azetidin-3-ylmethyl-(carboxymethyl)-[5-[[4-[[3-[1-(2,2-difluoroethyl)-3-(trifluoromethyl)pyrazol-4-yl]imidazo[1,2-a]pyrazin-8-yl]amino]-2-ethyl-benzoyl]amino]pentyl]-methyl-ammonium formate;
2-[[3-[[2-chloro-4-[[3-[3-(trifluoromethyl)-1H-pyrazol-4-yl]imidazo[1,2-a]pyrazin-8-yl]amino]benzoyl]amino]cyclobutyl]amino]ethyl-trimethyl-ammonium formate;
Azetidin-3-ylmethyl-(carboxymethyl)-[5-[[4-[[3-[1-(cyanomethyl)-3-(trifluoromethyl)pyrazol-4-yl]imidazo[1,2-a]pyrazin-8-yl]amino]-2-ethyl-benzoyl]amino]pentyl]-methyl-ammonium formate;
Carboxymethyl-[5-[[4-[[3-[1-(cyanomethyl)-3-(trifluoromethyl)pyrazol-4-yl]imidazo[1,2-a]pyrazin-8-yl]amino]-2-ethyl-benzoyl]amino]pentyl]-dimethyl-ammonium formate;
Trimethyl-[2-[4-[2-methyl-4-[[3-[1-methyl-3-(trifluoromethyl)pyrazol-4-yl]imidazo[1,2-a]pyrazin-8-yl]amino]benzoyl]piperazin-1-yl]-2-oxoethyl]ammonium formate;
4-[2-[2-[[2-chloro-4-[[3-[4-(cyanomethoxy)-2,3-difluoro-phenyl]imidazo[1,2-a]pyrazin-8-yl]amino]benzoyl]amino]ethoxy]ethyl]-4-methyl-piperazin-4-ium-2-carboxylic acid formate;
bis(carboxymethyl)-[5-[[4-[[3-(2,3-difluoro-4-methoxy-phenyl)imidazo[1,2-a]pyrazin-8-yl]amino]-2-ethyl-benzoyl]amino]pentyl]-methyl-ammonium 2,2,2-trifluoroacetate;
4-[[3-[4-(4-aminobut-2-ynoxy)phenyl]imidazo[1,2-a]pyrazin-8-yl]amino]-N-[2-(1,1-dimethylpiperidin-1-ium-4-yl)ethyl]-N,2-dimethyl-benzamide formate;
Carboxymethy1-[5-[[4-[[3-[1-(2,2-difluoroethyl)-3-(trifluoromethyl)pyrazol-4-yl]imidazo[1,2-a]pyrazin-8-yl]amino]-2-ethyl-benzoyl]amino]pentyl]-dimethyl-ammonium formate;
1-(Azetidin-3-ylmethyl)-N-[(2S)-2-[[4-[[3-[4-(difluoromethoxy)-2,3-difluorophenyl]imidazo[1,2-a]pyrazin-8-yl]amino]-2-ethyl-benzoyl]amino]propyl]-1-methyl-piperidin-1-ium-4-carboxamide formate;
2-[4-[[[4-[[3-(2,3-Difluoro-4-methoxy-phenyl)imidazo[1,2-a]pyrazin-8-yl]amino]-2-ethyl-benzoyl]amino]methyl]-1-methyl-piperidin-1-ium-1-yl]acetic acid 2,2,2-trifluoroacetate;
[2-Chloro-4-[[3-[4-(difluoromethoxy)-2,3-difluoro-phenyl]imidazo[1,2-a]pyrazin-8-yl]amino]phenyl]-[4-[1-methyl-1-(pyrrolidin-3-ylmethyl)piperidin-1-ium-4-carbonyl]piperazin-1-yl]methanone formate;
N-[3-[[2-Chloro-4-[[3-[4-(difluoromethoxy)-2,3-difluoro-phenyl]imidazo[1,2-a]pyrazin-8-yl]amino]benzoyl]amino]propyl]-1-methyl-1-(pyrrolidin-3-ylmethyl)piperidin-1-ium-4-carboxamide formate;
N-[3-[[4-[[3-(2,3-difluoro-4-pyrimidin-2-yloxy-phenyl)imidazo[1,2-a]pyrazin-8-yl]amino]-2-ethyl-benzoyl]amino]propyl]-1-methyl-1-(pyrrolidin-3-ylmethyl)piperidin-1-ium-4-carboxamide formate;
4-[[3-(2,3-Difluoro-4-pyrimidin-2-yloxy-phenyl)imidazo[1,2-a]pyrazin-8-yl]amino]-2-ethyl-N-[[1-methyl-1-(pyrrolidin-3-ylmethyl)piperidin-1-ium-4-yl]methyl]benzamide formate;
4-[[3-[2,3-difluoro-4-(2-methyltriazol-4-yl)oxy-phenyl]imidazo[1,2-a]pyrazin-8-yl]amino]-2-ethyl-N-[[1-methyl-1-(pyrrolidin-3-ylmethyl)piperidin-1-ium-4-yl]methyl]benzamide formate;
[4-[[3-[4-(Difluoromethoxy)-2,3-difluoro-phenyl]imidazo[1,2-a]pyrazin-8-yl]amino]-2-ethyl-phenyl]-[4-methyl-4-(pyrrolidin-3-ylmethyl)piperazin-4-ium-1-yl]methanone formate;
Methyl (2S)-2-[[1-(azetidin-3-ylmethyl)-1-methyl-piperidin-1-ium-4-carbonyl]amino]-3-[[4-[[3-(2,3-difluoro-4-methoxy-phenyl)imidazo[1,2-a]pyrazin-8-yl]amino]-2-ethyl-benzoyl]amino]propanoate formate 2,2,2-trifluoroacetate (mixed salt);
Methyl (2S)-2-[[1-(azetidin-3-ylmethyl)-1-methyl-piperidin-1-ium-4-carbonyl]amino]-4-[[4-[[3-(2,3-difluoro-4-methoxy-phenyl)imidazo[1,2-a]pyrazin-8-yl]amino]-2-ethyl-benzoyl]amino]butanoate formate 2,2,2-trifluoroacetate (mixed salt);
Methyl (2S)-3-[[1-(azetidin-3-ylmethyl)-1-methyl-piperidin-1-ium-4-carbonyl]amino]-2-[[4-[[3-(2,3-difluoro-4-methoxy-phenyl)imidazo[1,2-a]pyrazin-8-yl]amino]-2-ethyl-benzoyl]amino]propanoate formate 2,2,2-trifluoroacetate (mixed salt);
Methyl (2S)-2-[[1-(azetidin-3-ylmethyl)-1-methyl-piperidin-1-ium-4-carbonyl]amino]-4-[[4-[[3-(2,3-difluoro-4-methoxy-phenyl)imidazo[1,2-a]pyrazin-8-yl]amino]-2-ethyl-benzoyl]amino]butanoate formate 2,2,2-trifluoroacetate (mixed salt);
Methyl 1-[1-(azetidin-3-ylmethyl)-1-methyl-piperidin-1-ium-4-carbonyl]-4-[[[4-[[3-(2,3-difluoro-4-methoxy-phenyl)imidazo[1,2-a]pyrazin-8-yl]amino]-2-ethyl-benzoyl]amino]methyl]piperidine-4-carboxylate formate 2,2,2-trifluoroacetate (mixed salt);
Methyl 2-[1-[1-(azetidin-3-ylmethyl)-1-methyl-piperidin-1-ium-4-carbonyl]-4-piperidyl]-2-[[4-[[3-(2,3-difluoro-4-methoxy-phenyl)imidazo[1,2-a]pyrazin-8-yl]amino]-2-ethyl-benzoyl]amino]acetate formate 2,2,2-trifluoroacetate (mixed salt);
1-(Azetidin-3-ylmethyl)-N-[3-[[4-[[3-(2,3-difluoro-4-methoxy-phenyl)imidazo[1,2-a]pyrazin-8-yl]amino]-2-ethyl-benzoyl]amino]cyclobutyl]-1-methyl-piperidin-1-ium-4-carboxamide formate 2,2,2-trifluoroacetate (mixed salt);
1-(Azetidin-3-ylmethyl)-N-[3-[[[4-[[3-(2,3-difluoro-4-methoxy-phenyl)imidazo[1,2-a]pyrazin-8-yl]amino]-2-ethyl-benzoyl]amino]methyl]cyclobutyl]-1-methyl-piperidin-1-ium-4-carboxamide formate 2,2,2-trifluoroacetate (mixed salt);
2-[1-(Azetidin-3-ylmethyl)-4-[[[4-[[3-(2,3-difluoro-4-methoxy-phenyl)imidazo[1,2-a]pyrazin-8-yl]amino]-2-ethyl-benzoyl]amino]methyl]piperidin-1-ium-1-yl]acetic acid bis(2,2,2-trifluoroacetate);
1-(Azetidin-3-ylmethyl)-N-[3-[[4-[[3-(2,3-difluoro-4-methoxy-phenyl)imidazo[1,2-a]pyrazin-8-yl]amino]-2-ethyl-benzoyl]amino]propyl]-1-methyl-piperidin-1-ium-4-carboxamide bis(trifluoroacetate);
N-[3-[[2-[(3S)-1-(Azetidin-3-ylmethyl)-1-methyl-pyrrolidin-1-ium-3-yl]acetyl]amino]propyl]-4-[[3-(2,3-difluoro-4-methoxy-phenyl)imidazo[1,2-a]pyrazin-8-yl]amino]-2-ethyl-benzamide bis(2,2,2-trifluoroacetate);
N-[3-[[2-[1-(Azetidin-3-ylmethyl)-1-methyl-piperidin-1-ium-4-yl]acetyl]amino]propyl]-4-[[3-(2,3-difluoro-4-methoxy-phenyl)imidazo[1,2-a]pyrazin-8-yl]amino]-2-ethyl-benzamide bis(2,2,2-trifluoroacetate);
4-[[3-(2,3-Difluoro-4-methoxy-phenyl)imidazo[1,2-a]pyrazin-8-yl]amino]-2-ethyl-N-[[1-methyl-1-[2-oxo-2-(pyrrolidin-3-ylmethylamino)ethyl]piperidin-1-ium-4-yl]methyl]benzamide bis(2,2,2-trifluoroacetate);
N-[[1-[1-(Azetidin-3-ylmethyl)-1-methyl-piperidin-1-ium-4-carbonyl]-4-piperidyl]methyl]-4-[[3-(2,3-difluoro-4-methoxy-phenyl)imidazo[1,2-a]pyrazin-8-yl]amino]-2-ethyl-benzamide bis(2,2,2-trifluoroacetate);
N-[[1-(Azetidin-3-ylmethyl)-1-methyl-piperidin-1-ium-4-yl]methyl]-4-[[3-(2,3-difluoro-4-methoxy-phenyl)imidazo[1,2-a]pyrazin-8-yl]amino]-2-ethyl-benzamide;2,2,2-trifluoroacetate bis(2,2,2-trifluoroacetate);
4-[[3-(2,3-Difluoro-4-methoxy-phenyl)imidazo[1,2-a]pyrazin-8-yl]amino]-N-[[1-[(1,1-dimethylpyrrolidin-1-ium-3-yl)methyl]-1-methyl-piperidin-1-ium-4-yl]methyl]-2-ethyl-benzamide diformate;
N-[1-(Azetidin-3-ylmethyl)-1-methyl-piperidin-1-ium-4-yl]-4-[[3-(2,3-difluoro-4-methoxy-phenyl)imidazo[1,2-a]pyrazin-8-yl]amino]-2-ethyl-benzamidebis(2,2,2-trifluoroacetate);
4-[[3-(2,3-Difluoro-4-methoxy-phenyl)imidazo[1,2-a]pyrazin-8-yl]amino]-2-ethyl-N-[rac-(1S,5R)-3-(azetidin-3-ylmethyl)-3-methyl-3-azoniabicyclo[3.1.0]hexan-6-yl]benzamide bis(2,2,2-trifluoroacetate);
N-[[1-(Azetidin-3-ylmethyl)-1-methyl-pyrrolidin-1-ium-3-yl]methyl]-2-ethyl-4-[[3-[3-(trifluoromethyl)-1H-pyrazol-4-yl]imidazo[1,2-a]pyrazin-8-yl]amino]benzamide bis(2,2,2-trifluoroacetate);
Bis(azetidin-3-ylmethyl)-[3-[[4-[[3-(2,3-difluoro-4-methoxy-phenyl)imidazo[1,2-a]pyrazin-8-yl]amino]-2-ethyl-benzoyl]amino]propyl]-methyl-ammonium bis(2,2,2-trifluoroacetate);
4-[[3-(2,3-difluoro-4-methoxy-phenyl)imidazo[1,2-a]pyrazin-8-yl]amino]-2-ethyl-N-[[1-methyl-1-(3-pyridylmethyl)piperidin-1-ium-4-yl]methyl]benzamide;2,2,2-trifluoroacetate;
N-[[1-(Azetidin-3-ylmethyl)-1-(2-hydroxyethyl)piperidin-1-ium-4-yl]methyl]-4-[[3-(2,3-difluoro-4-methoxy-phenyl)imidazo[1,2-a]pyrazin-8-yl]amino]-2-ethyl-benzamide 2,2,2-trifluoroacetate;
4-[[3-(2,3-Difluoro-4-methoxy-phenyl)imidazo[1,2-a]pyrazin-8-yl]amino]-2-ethyl-N-[[1-methyl-1-(pyrrolidin-2-ylmethyl)piperidin-1-ium-4-yl]methyl]benzamide 2,2,2-trifluoroacetate;
N-[[1-(Azetidin-3-ylmethyl)-1-ethyl-piperidin-1-ium-4-yl]methyl]-4-[[3-(2,3-difluoro-4-methoxy-phenyl)imidazo[1,2-a]pyrazin-8-yl]amino]-2-ethyl-benzamide formate;
1-[2-[[4-[[3-(2,3-Difluoro-4-methoxy-phenyl)imidazo[1,2-a]pyrazin-8-yl]amino]-2-ethyl-benzoyl]amino]ethyl]-1-methyl-pyrrolidin-1-ium-3-carboxamide 2,2,2-trifluoroacetate;
4-[[3-(2,3-Difluoro-4-phenoxy-phenyl)imidazo[1,2-a]pyrazin-8-yl]amino]-2-ethyl-N-[[1-methyl-1-(pyrrolidin-3-ylmethyl)piperidin-1-ium-4-yl]methyl]benzamide formate; and
1-(2-methoxy-2-oxoethyl)-4-(2-(4-((3-(4-methoxyphenyl)imidazo[1,2-a]pyrazin-8-yl)amino)-2-methylbenzamido)ethyl)-1-methylpiperidin-1-ium formate.

12. A compound of formula (IIa) or (IIIa) according to any one of claims 1 to 11, or a pharmaceutically acceptable salt thereof, for use as therapeutically active substance.

13. A pharmaceutical composition comprising a compound of formula (IIa) or (IIIa) according to any one of claims 1 to 11, or a pharmaceutically acceptable salt thereof, and a therapeutically inert carrier.

14. A compound of formula (IIa) or (IIIa) according to any of claims 1 to 11, or a pharmaceutically acceptable salt thereof, for use as antibiotic.

15. A compound of formula (IIa) or (IIIa) according to any of claims 1 to 11, or a pharmaceutically acceptable salt thereof, for use in the treatment or prevention of infections and resulting diseases caused by *Acinetobacter baumannii.*

## Patentansprüche

1. Verbindung der Formel (IIa) oder (IIIa) oder ein pharmazeutisch unbedenkliches Salz davon, 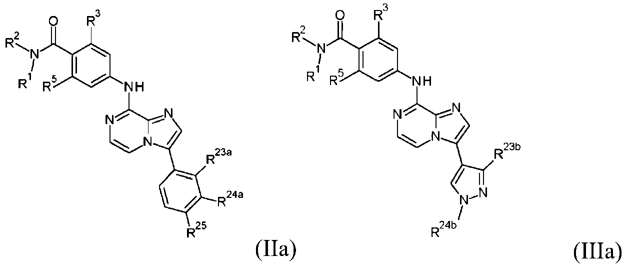 wobei:
R¹ ausgewählt ist aus P⁶-C₁-C₆-Alkyl-, R⁶-C₁-C₆-Alkyl-CH(C₁-C₆-alkoxycarbonyl)-,
R⁷-C₁-C₆-Alkyl-X-C₁-C₆-alkyl-, einer Gruppe 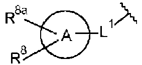 und einer Gruppe 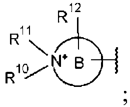 und
R² ausgewählt ist aus Wasserstoff und C₁-C₆-Alkyl;
oder R¹ und R² zusammen mit dem Stickstoffatom, an das sie gebunden sind, einen 3- bis 14-gliedrigen Heterocyclus bilden, der mit R⁹ und R^{9a} substituiert ist;
R³ ausgewählt ist aus Wasserstoff, Halogen und C₁-C₆-Alkyl; oder
R³ und R² zusammen mit den Atomen, an die sie gebunden sind, einen 3- bis 14-gliedrigen Heterocyclus bilden;
R⁵ ausgewählt ist aus Wasserstoff, C₁-C₆-Alkyl und Halogen;
R⁶ eine Gruppe R¹⁶-L²- ist;
R⁷ eine Gruppe R¹⁶-L³- ist;
R⁸ eine Gruppe R¹⁶-L⁴- ist;
R^{8a}, R^{9a}, R^{11a}, R¹², R¹⁸, R¹⁹, R²⁰, R²¹ und R²² unabhängig voneinander ausgewählt sind aus Wasserstoff, Halogen, Hydroxy, Cyano, Amino, Carbamoyl, Carboxy, C₁-C₆-Alkyl, C₁-C₆-Alkoxy, Halogen-C₁-C₆-alkyl, Cyano-C₁-C₆-alkyl, Carboxy-C₁-C₆-alkyl, Hydroxy-C₁-C₆-alkyl, Halogen-C₁-C₆-alkoxy, Cyano-C₁-C₆-alkoxy, C₂-C₆-Alkinyloxy, Amino-C₂-C₆-alkinyloxy, Aryloxy, (5- bis 14-gliedrigem Heteroaryl)oxy, C₁-C₆-Alkyl-(5- bis 14-gliedrigem heteroaryl)oxy und C₁-C₆-Alkoxycarbonyl;
R⁹ eine Gruppe R¹⁶-L⁵- oder eine Gruppe 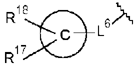 ist;
R¹⁰ ausgewählt ist aus C₁-C₆-Alkyl, C₁-C₆-Alkoxycarbonyl-C₁-C₆-alkyl, C₁-C₆-Alkyl-NH-C₁-C₆-alkyl, Amino-C₁-C₆-alkyl, Carbamoyl, Carbamoyl-C₁-C₆-alkyl, Carboxy-C₁-C₆-alkyl, Cyano-C₁-C₆-alkyl, Halogen-C₁-C₆-alkyl, Hydroxy-C₁-C₆-alkyl und einer Gruppe 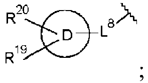
R¹¹ ausgewählt ist aus C₁-C₆-Alkyl, Carbamoyl, Carboxy, Carboxy-C₁-C₆-alkyl und Hydroxy-C₁-C₆-alkyl;
R¹³ und R¹⁴ unabhängig voneinander ausgewählt sind aus C₁-C₆-Alkyl, Amino-C₁-C₆-alkyl, Carbamoyl-C₁-C₆-alkyl, Carboxy-C₁-C₆-alkyl, Cyano-C₁-C₆-alkyl, Hydroxy-C₁-C₆-alkyl, Halogen-C₁-C₆-alkyl, C₁-C₆-Alkoxycarbonyl-C₁-C₆-alkyl und einer Gruppe 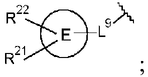
oder R¹³ und R¹⁴ zusammen mit dem Stickstoffatom, an das sie gebunden sind, einen 3-bis 14-gliedrigen Heterocyclus bilden, der gegebenenfalls mit 1-3 Substituenten substituiert ist, ausgewählt aus Halogen, Amino, Hydroxy, Cyano, C₁-C₆-Alkyl, Halogen-C₁-C₆-alkyl, C₁-C₆-Alkoxy und Halogen-C₁-C₆-alkoxy;
R¹⁵ C₁-C₆-Alkyl ist;
R¹⁶ ausgewählt ist aus einer Gruppe 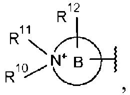 einer Gruppe 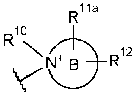 und einer Gruppe 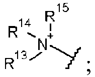
R¹⁷ eine Gruppe R¹⁶-L⁷- ist;
R^{23a} Wasserstoff oder Halogen ist;
R^{23b} Halogen-C₁-C₆-alkyl ist;
R^{24a} Wasserstoff oder Halogen ist;
R^{24b} ausgewählt ist aus Wasserstoff, C₁-C₆-Alkyl, Cyano-C₁-C₆-alkyl und Halogen-C₁-C₆-alkyl;
R²⁵ ausgewählt ist aus Halogen, C₁-C₆-Alkoxy, Cyano-C₁-C₆-alkoxy, Halogen-C₁-C₆-alkoxy, C₂-C₆-Alkinyloxy, Amino-C₂-C₆-alkinyloxy, Aryloxy, (5- bis 14-gliedrigem Heteroaryl)oxy und C₁-C₆-Alkyl-(5- bis 14-gliedrigem heteroaryl)oxy;
L¹ ausgewählt ist aus einer kovalenten Bindung, Carbonyl und -C₁-C₆-Alkyl-;
L² und L³ unabhängig voneinander ausgewählt sind aus einer kovalenten Bindung, Carbonyl, -C₁-C₆-Alkyl-C(O)-NH-, -C(O)-NH-, -NH-C(O)-, -C(O)-NH-CH(C₁-C₆-Alkoxycarbonyl)-, -C₁-C₆-Alkyl-CH(OH)-C₁-C₆-alkyl-C(O)- und - C₁-C₆-Alkyl-NH-;
L⁴, L⁵, L⁶, L⁷ und L⁸ unabhängig voneinander ausgewählt sind aus einer kovalenten Bindung, Carbonyl, -C₁-C₆-Alkyl-, -C₁-C₆-Alkyl-C(O)-, -C₁-C₆-Alkyl-NH-C(O)-, - C₁-C₆-Alkyl-C(O)-NH-, -C₁-C₆-Alkyl-NH-C(O)-C₁-C₆-alkyl-, -C₁-C₆-Alkyl-C(O)-NH-C₁-C₆-alkyl-, -C(O)-NH-, -NH-C(O)-, -C(O)-NH-CH(C₁-C₆-Alkoxycarbonyl)-, -C₁-C₆-Alkyl-CH(OH)-C₁-C₆-alkyl-C(O)- und - C₁-C₆-Alkyl-NH-;
L⁹ -C₁-C₆-Alkyl- oder -C(O)-C₁-C₆-Alkyl- ist;
A, C, D und E jeweils unabhängig voneinander ausgewählt sind aus C₆-C₁₀-Aryl, C₃-C₁₀-Cycloalkyl, 3- bis 14-gliedrigem Heterocyclyl und 5- bis 14-gliedrigem Heteroaryl;
B ein 3- bis 14-gliedriger Heterocyclus ist; und
X ausgewählt ist aus O, S, SO, SO₂, NH und N(C₁-C₆-Alkyl).

2. Verbindung der Formel (IIa) oder (IIIa) nach Anspruch 1 oder ein pharmazeutisch unbedenkliches Salz davon, wobei:
R¹ ausgewählt ist aus R⁶-C₁-C₆-Alkyl-, R⁶-C₁-C₆-Alkyl-CH(C₁-C₆-alkoxycarbonyl)-,
R⁷-C₁-C₆-Alkyl-O-C₁-C₆-alkyl-, einer Gruppe 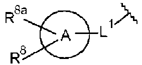 und einer Gruppe 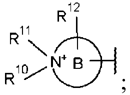
R² ausgewählt ist aus Wasserstoff und C₁-C₆-Alkyl;
R⁶ eine Gruppe R¹⁶-L²- ist;
R⁷ eine Gruppe R¹⁶-L³- ist;
R⁸ eine Gruppe R¹⁶ -L⁴- ist;
R^{8a} Wasserstoff oder C₁-C₆-Alkoxycarbonyl ist;
R¹⁰ ausgewählt ist aus C₁-C₆-Alkyl, C₁-C₆-Alkoxycarbonyl-C₁-C₆-alkyl, C₁-C₆-Alkyl-NH-C₁-C₆-alkyl, Amino-C₁-C₆-alkyl, Carbamoyl-C₁-C₆-alkyl, Carboxy-C₁-C₆-alkyl, Cyano-C₁-C₆-alkyl und einer Gruppe 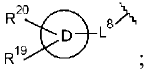
R¹¹ ausgewählt ist aus C₁-C₆-Alkyl, Carbamoyl, Carboxy-C₁-C₆-alkyl und Hydroxy-C₁-C₆-alkyl;
R^{11a} Wasserstoff oder Carboxy ist;
R¹² Wasserstoff oder Hydroxy ist;
R¹³ ausgewählt ist aus C₁-C₆-Alkyl, Amino-C₁-C₆-alkyl, Carbamoyl-C₁-C₆-alkyl, Carboxy-C₁-C₆-alkyl, Cyano-C₁-C₆-alkyl, Hydroxy-C₁-C₆-alkyl, C₁-C₆-Alkoxycarbonyl-C₁-C₆-alkyl und einer Gruppe 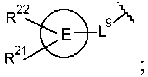 und
R¹⁴ ausgewählt ist aus C₁-C₆-Alkyl, Carboxy-C₁-C₆-alkyl, einer Gruppe 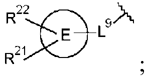
oder R¹³ und R¹⁴ zusammen mit dem Stickstoffatom, an das sie gebunden sind, einen 3-bis 14-gliedrigen Heterocyclus bilden;
R¹⁵ C₁-C₆-Alkyl ist;
R¹⁶ ausgewählt ist aus einer Gruppe 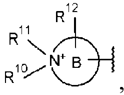 einer Gruppe 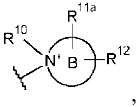 und einer Gruppe 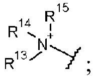
R¹⁷ eine Gruppe R¹⁶-L⁷- ist;
R¹⁸ Wasserstoff ist;
R¹⁹ ausgewählt ist aus Wasserstoff, Amino und C₁-C₆-Alkyl;
R²⁰ Wasserstoff oder C₁-C₆-Alkyl ist;
R²¹ und R²² beide Wasserstoff sind;
L¹ eine kovalente Bindung oder -C₁-C₆-Alkyl- ist;
L² ausgewählt ist aus einer kovalenten Bindung, Carbonyl, -C₁-C₆-Alkyl-C(O)-NH-, - C(O)-NH-, -C(O)-NH-CH(C₁-C₆-Alkoxycarbonyl)-, -C₁-C₆-Alkyl-CH(OH)-C₁-C₆-alkyl-C(O)- und -C₁-C₆-Alkyl-NH-;
L³ eine kovalente Bindung ist;
L⁴ ausgewählt ist aus Carbonyl, -C₁-C₆-Alkyl-C(O)-, -C₁-C₆-Alkyl-C(O)-NH-, -C(O)-NH-, -C₁-C₆-Alkyl-CH(OH)-C₁-C₆-alkyl-C(O)- und -C₁-C₆-Alkyl-NH-;
L⁷ -C₁-C₆-Alkyl- ist;
L⁸ ausgewählt ist aus einer kovalenten Bindung, -C₁-C₆-Alkyl- und -C₁-C₆-Alkyl-NH-C(O)-C₁-C₆-alkyl-;
L⁹ -C₁-C₆-Alkyl- oder -C(O)-C₁-C₆-Alkyl- ist;
A C₃-C₁₀-Cycloalkyl oder ein 3- bis 14-gliedriger Heterocyclus ist;
B ein 3- bis 14-gliedriger Heterocyclus ist;
D ausgewählt ist aus C₃-C₁₀-Cycloalkyl, 3- bis 14-gliedrigem Heterocyclyl und 5- bis 14-gliedrigem Heteroaryl; und
E 3- bis 14-gliedriges Heterocyclyl ist.

3. Verbindung der Formel (IIa) oder (IIIa) nach Anspruch 1 oder ein pharmazeutisch unbedenkliches Salz davon, wobei:
R¹ ausgewählt ist aus R⁶-C₁-C₆-Alkyl-, R⁷-C₁-C₆-Alkyl-O-C₁-C₆-alkyl- und einer Gruppe 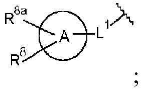
R² ausgewählt ist aus Wasserstoff und C₁-C₆-Alkyl;
R⁶ eine Gruppe R¹⁶-L²- ist;
R⁷ eine Gruppe R¹⁶-L³- ist;
R⁸ eine Gruppe R¹⁶-L⁴- ist;
R^{8a} Wasserstoff ist;
R¹⁰ ausgewählt ist aus Carbamoyl-C₁-C₆-alkyl, Cyano-C₁-C₆-alkyl, C₁-C₆-Alkyl, Carboxy-C₁-C₆-Alkyl und einer Gruppe 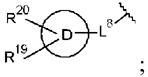
R¹¹ C₁-C₆-Alkyl oder Carboxy-C₁-C₆-alkyl ist;
R^{11a} und R¹² beide Wasserstoff sind;
R¹³ ausgewählt ist aus C₁-C₆-Alkyl, Amino-C₁-C₆-alkyl, Carbamoyl-C₁-C₆-alkyl und einer Gruppe 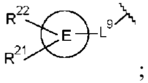
R¹⁴ C₁-C₆-Alkyl oder Carboxy-C₁-C₆-Alkyl ist;
R¹⁵ C₁-C₆-Alkyl ist;
R¹⁶ ausgewählt ist aus einer Gruppe 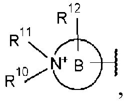 einer Gruppe 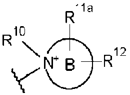 und einer Gruppe 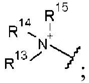
R¹⁹ und R²⁰ beide Wasserstoff sind;
R²¹ und R²² beide Wasserstoff sind;
L¹, L² und L³ eine kovalente Bindung sind;
L⁴ -C₁-C₆-Alkyl-C(O)- oder -C₁-C₆-Alkyl-C(O)-NH- ist;
L⁸ und L⁹ -C₁-C₆-Alkyl- sind;
A C₃-C₁₀-Cycloalkyl oder ein 3- bis 14-gliedriger Heterocyclus ist;
B ein 3- bis 14-gliedriger Heterocyclus ist;
D 3- bis 14-gliedriges Heterocyclyl ist; und
E 3- bis 14-gliedriges Heterocyclyl ist.

4. Verbindung der Formel (IIa) oder (IIIa) nach Anspruch 1 oder ein pharmazeutisch unbedenkliches Salz davon, wobei:
R¹ ausgewählt ist aus R⁶-(CH₂)ₙ-, R⁷-(CH₂)₂-O-(CH₂)₂- und einer Gruppe 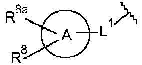 ;
R² ausgewählt ist aus Wasserstoff und Methyl;
R⁶ eine Gruppe R¹⁶-L²- ist;
R⁷ eine Gruppe R¹⁶-L³- ist;
R⁸ eine Gruppe R^{I6}-L ⁴- ist;
R^{8a} Wasserstoff ist;
R¹⁰ ausgewählt ist aus Carbamoyl-CH₂-, Methyl, Carboxy-CH₂-, Cyano-CH₂- und einer Gruppe 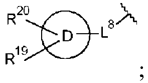
R¹¹ Methyl oder Carboxy-CH₂- ist;
R^{11a} und R¹² beide Wasserstoff sind;
R¹³ ausgewählt ist aus Methyl, Aminopropyl, Carbamoyl-CH₂- und einer Gruppe 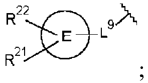
R¹⁴ Methyl oder Carboxymethyl ist;
R¹⁵ Methyl ist;
R¹⁶ ausgewählt ist aus einer Gruppe 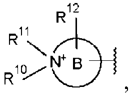 einer Gruppe 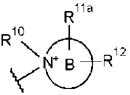 und einer Gruppe 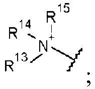
R¹⁹ und R²⁰ beide Wasserstoff sind;
R²¹ und R²² beide Wasserstoff sind;
L¹, L² und L³ eine kovalente Bindung sind;
L⁴ -CH₂-C(O)- oder -CH₂-C(O)-NH- ist;
L⁸ und L⁹ beide -CH₂- sind;
A Cyclohexyl oder Pyrrolidinyl ist;
B Pyrrolidinyl, Piperidyl, 3-Azabicyclo[3.1.0]hexan-6-yl ist;
D Pyrrolidin oder Azetidin ist;
E Azetidin ist; und
n 1, 2 oder 5 ist.

5. Verbindung der Formel (IIa) oder (IIIa) nach Anspruch 1 oder ein pharmazeutisch unbedenkliches Salz davon, wobei:
R¹ und R² zusammen mit dem Stickstoffatom, an das sie gebunden sind, einen 3- bis 14-gliedrigen Heterocyclus bilden, der mit R⁹ und R^{9a} substituiert ist;
R⁹ eine Gruppe R¹⁶-L⁵- oder eine Gruppe 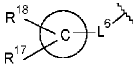 ist;
R^{9a} ausgewählt ist aus Wasserstoff, Hydroxy und C₁-C₆-Alkoxycarbonyl;
R¹⁰ ausgewählt ist aus C₁-C₆-Alkyl, C₁-C₆-Alkyl-NH-C₁-C₆-alkyl, Amino-C₁-C₆-alkyl, Carbamoyl-C₁-C₆-alkyl und einer Gruppe 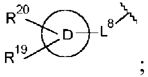
R¹¹ C₁-C₆-Alkyl ist;
R¹² Wasserstoff oder Hydroxy ist;
R¹³ C₁-C₆-Alkyl oder eine Gruppe 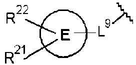 ist; und
R¹⁴ und R¹⁵ beide C₁-C₆-Alkyl sind;
R¹⁶ eine Gruppe 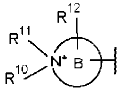 oder eine Gruppe 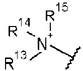 ist;
R¹⁷ eine Gruppe R¹⁶-L⁷- ist;
R¹⁸ Wasserstoff ist;
R¹⁹ Wasserstoff oder Amino ist;
R²⁰, R²¹, R²² Wasserstoff sind;
B ein 3- bis 14-gliedriger Heterocyclus ist;
C ein 3- bis 14-gliedriger Heterocyclus ist;
D C₃-C₁₀-Cycloalkyl oder 3- bis 14-gliedriges Heterocyclyl ist;
E ein 3- bis 14-gliedriger Heterocyclus ist;
L⁵ ausgewählt ist aus einer kovalenten Bindung, Carbonyl, -C₁-C₆-Alkyl-, - C₁-C₆-Alkyl-C(O)- und -C₁-C₆-Alkyl-NH-C(O)-;
L⁶ Carbonyl ist;
L⁷ -C₁-C₆-Alkyl- ist; und
L⁸ und L⁹ beide -C₁-C₆-Alkyl- sind.

6. Verbindung der Formel (IIa) oder (IIIa) nach Anspruch 1 oder ein pharmazeutisch unbedenkliches Salz davon, wobei:
R¹ und R² zusammen mit dem Stickstoffatom, an das sie gebunden sind, einen 3- bis 14-gliedrigen Heterocyclus bilden, der mit R⁹ und R^{9a} substituiert ist;
R⁹ eine Gruppe R¹⁶-L⁵- ist;
R^{9a} Wasserstoff ist;
R¹⁰ Carbamoyl-C₁-C₆-Alkyl oder eine Gruppe 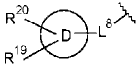 ist;
R¹¹ C₁-C₆-Alkyl ist;
R¹³, R¹⁴ und R¹⁵ C₁-C₆-Alkyl sind;
R¹⁶ eine Gruppe 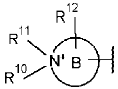 oder eine Gruppe 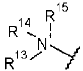 ist;
R¹², R¹⁹ und R²⁰ Wasserstoff sind;
B ein 3- bis 14-gliedriger Heterocyclus ist;
D ein 3- bis 14-gliedriger Heterocyclus ist;
L⁵ ausgewählt ist aus Carbonyl, -C₁-C₆-Alkyl- und -C₁-C₆-Alkyl-C(O)-; und
L⁸ -C₁-C₆-Alkyl- ist.

7. Verbindung der Formel (IIa) oder (IIIa) nach Anspruch 1 oder ein pharmazeutisch unbedenkliches Salz davon, wobei:
R¹ und R² zusammen mit dem Stickstoffatom, an das sie gebunden sind, einen Piperazinylring bilden, der mit R⁹ und R^{9a} substituiert ist;
R⁹ eine Gruppe R¹⁶-L⁵- ist;
R^{9a} Wasserstoff ist;
R¹⁰ Carbamoylmethyl oder eine Gruppe 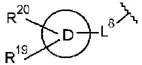 ist;
R¹¹ Methyl ist;
R¹³, R¹⁴ und R¹⁵ Methyl sind;
R¹⁶ eine Gruppe 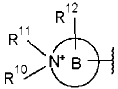 oder eine Gruppe 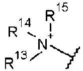 ist;
R¹², R¹⁹ und R²⁰ Wasserstoff sind;
B Piperidyl ist;
D Pyrrolidinyl ist;
L⁵ ausgewählt ist aus Carbonyl, -(CH₂)₂- und -CH₂-C(O)-; und
L⁸ -CH₂- ist.

8. Verbindung der Formel (IIa) oder (IIIa) nach einem der Ansprüche 1 bis 7 oder ein pharmazeutisch unbedenkliches Salz davon, wobei R³ ausgewählt ist aus Fluor, Chlor, Methyl und Ethyl;
oder R³ und R² zusammen mit den Atomen, an die sie gebunden sind, einen 6-Oxo-2,3-dihydropyridinylring bilden.

9. Verbindung der Formel (IIa) oder (IIIa) nach einem der Ansprüche 1 bis 8 oder ein pharmazeutisch unbedenkliches Salz davon, wobei R⁵ Wasserstoff oder C₁-C₆-Alkyl ist.

10. Verbindung der Formel (IIa) oder (IIIa) nach einem der Ansprüche 1 bis 8 oder ein pharmazeutisch unbedenkliches Salz davon, wobei R⁵ Wasserstoff oder Methyl ist.

11. Verbindung der Formel (IIa) oder (IIIa) nach Anspruch 1 oder ein pharmazeutisch unbedenkliches Salz davon, wobei die Verbindung der Formel (IIa) oder (IIIa) ausgewählt ist aus:
2-(2-(4-((3-(4-(Difluormethoxy)phenyl)imidazo[1,2-a]pyrazin-8-yl)amino)-2-methylbenzamido)ethoxy)-N,N,N-trimethylethanaminiumiodid;
4-(2-(4-((3-(4-(Difluormethoxy)phenyl)imidazo[1,2-a]pyrazin-8-yl)amino)-N,2-dimethylbenzamido)ethyl)-1,1-dimethylpiperidin-1-iumformiat;
1-Ethyl-4-(2-(4-((3-(4-methoxyphenyl)imidazo[1,2-a]pyrazin-8-yl)amino)-2-methylbenzamido)ethyl)-1-methylpiperidin-1-iumformiat;
2-Chlor-4-[[3-(2,3-difluor-4-methoxyphenyl)imidazo[1,2-a]pyrazin-8-yl]amino]-N-[2-(4,4-dimethylpiperazin-4-ium-1-yl)ethyl]-N-methylbenzamid-2,2,2-trifluoracetat;
4-[[3-(2,3-Difluor-4-methoxyphenyl)imidazo[1,2-a]pyrazin-8-yl]amino]-N-[2-(1,1-dimethylpiperidin-1-ium-4-yl)ethyl]-N,2-dimethylbenzamid-2,2,2-trifluoracetat;
1-(Carboxymethyl)-4-(2-(4-((3-(4-methoxyphenyl)imidazo[1,2-a]pyrazin-8-yl)amino)-2-methylbenzamido)ethyl)-1-methylpiperidin-1-iumformiat;
4-(2-(4-((3-(4-(Difluormethoxy)phenyl)imidazo[1,2-a]pyrazin-8-yl)amino)-N,2-dimethylbenzamido)ethyl)-1,1-diethylpiperidin-1-iumformiat;
2-(2-(4-((3-(2,3-Difluor-4-methoxyphenyl)imidazo[1,2-a]pyrazin-8-yl)amino)-2-ethylbenzamido)ethoxy)-N,N,N-trimethylethanaminiumformiat;
6-(4-((3-(4-(Difluormethoxy)phenyl)imidazo[1,2-a]pyrazin-8-yl)amino)-2-methylbenzamido)-N,N,N-trimethylhexan-1-aminiumformiat;
2-[4-[2-Chlor-4-[[3-[4-(cyanomethoxy)-2,3-difluorphenyl]imidazo[1,2-a]pyrazin-8-yl]amino]benzoyl]piperazin-1-yl]ethyltrimethylazaniumiodid;
2-(1-(4-((3-(3-Fluor-4-methoxyphenyl)imidazo[1,2-a]pyrazin-8-yl)amino)-2-methylbenzoyl)piperidin-4-carboxamido)-N,N,N-trimethylethanaminiumformiat;
3-(4-((3-(4-Chlor-2,3-difluorphenyl)imidazo[1,2-a]pyrazin-8-yl)amino)-2-ethylbenzamido)-N,N,N-trimethylpropan-1-aminiumformiat;
2-(1-(4-((3-(4-(Difluormethoxy)phenyl)imidazo[1,2-a]pyrazin-8-yl)amino)-2-methylbenzoyl)piperidin-4-yl)-N,N,N-trimethylethanaminiumformiat;
[4-[[3-[4-(Difluormethoxy)phenyl]imidazo[1,2-a]pyrazin-8-yl]amino]-2-methylphenyl]-[4-(4,4-dimethylpiperazin-4-ium-1-carbonyl)piperazin-1-yl]methanonformiat;
[2-[4-[2-Chlor-4-[[3-(3-fluor-4-methoxyphenyl)imidazo[1,2-a]pyrazin-8-yl]amino]benzoyl]piperazin-1-yl]-2-oxoethyl]trimethylazaniumformiat;
2-(4-(4-((3-(3-Fluor-4-methoxyphenyl)imidazo[1,2-a]pyrazin-8-yl)amino)-2-methylbenzoyl)piperazin-1-yl)-N,N,N-trimethyl-2-oxoethan-1-aminiumiodid;
[4-[4-[4-[[3-[4-(Difluormethoxy)phenyl]imidazo[1,2-a]pyrazin-8-yl]amino]-2-methylbenzoyl]piperazin-1-carbonyl]morpholin-2-yl]methyltrimethylammoniumformiat;
2-Chlor-4-[[3-[4-(cyanomethoxy)-2,3-difluorphenyl]imidazo[1,2-a]pyrazin-8-yl]amino]-N-[2-(4,4-dimethylpiperazin-4-ium-1-yl)ethyl]-N-methylbenzamidiodid;
2-(2-(4-((3-(3-Fluor-4-methoxyphenyl)imidazo[1,2-a]pyrazin-8-yl)amino)-N,2-dimethylbenzamido)ethoxy)-N,N,N-trimethylethan-1-aminiumchlorid;
2-[[4-[4-[[3-[4-(Difluormethoxy)phenyl]imidazo[1,2-a]pyrazin-8-yl]amino]-2-methylbenzoyl]piperazin-1-carbonyl]amino]ethyltrimethylammoniumformiat;
2-[[(3S,4R)-1-[4-[[3-(3-Fluor-4-methoxyphenyl)imidazo[1,2-a]pyrazin-8-yl]amino]-2-methylbenzoyl]-3-hydroxypiperidin-4-carbonyl]amino]ethyltrimethylammoniumformiat;
2-Chlor-4-[[3-[4-(cyanomethoxy)-2,3-difluorphenyl]imidazo[1,2-a]pyrazin-8-yl]amino]-N-[2-(1,1-dimethylpiperidin-1-ium-4-yl)ethyl]-N-methylbenzamid-2,2,2-trifluoracetat;
4-(4-(2-(4-((3-(4-(Difluormethoxy)phenyl)imidazo[1,2-a]pyrazin-8-yl)amino)-N,2-dimethylbenzamido)ethyl)piperidin-1-yl)-2-hydroxy-N,N,N-trimethyl-4-oxobutan-1-aminiumformiat;
2-(2-(4-((3-(2,3-Difluor-4-methoxyphenyl)imidazo[1,2-a]pyrazin-8-yl)amino)-2-ethylbenzamido)ethoxy)-N,N,N-triethylethan-1-aminiumformiat;
2-[2-[[4-[[3-[4-(Cyanomethoxy)-2,3-difluorphenyl]imidazo[1,2-a]pyrazin-8-yl]amino]-2-ethylbenzoyl]amino]ethoxy]ethyltrimethylammoniumiodid;
[2-[4-[4-[[3-[4-(Difluormethoxy)phenyl]imidazo[1,2-a]pyrazin-8-yl]amino]-2-methylbenzoyl]piperazin-1-yl]-2-oxoethyl]trimethylammoniumchlorid;
2-[2-[[4-[[3-[2-Chlor-4-(cyanomethoxy)-3-fluorphenyl]imidazo[1,2-a]pyrazin-8-yl]amino]-2-ethylbenzoyl]amino]ethoxy]ethyltrimethylammoniumiodid;
2-[1-[4-[[3-[4-(Cyanomethoxy)-2,3-difluorphenyl]imidazo[1,2-a]pyrazin-8-yl]amino]-2-methylbenzoyl]-4-piperidyl]ethyltrimethylammoniumformiat;
2-[4-[2-Brom-4-[[3-(3-fluor-4-methoxyphenyl)imidazo[1,2-a]pyrazin-8-yl]amino]benzoyl]piperazin-1-yl]ethyltrimethylammonium-2,2,2-trifluoracetat;
2-[2-[[2-Brom-4-[[3-(2,3-difluor-4-methoxyphenyl)imidazo[1,2-a]pyrazin-8-yl]amino]benzoyl]amino]ethoxy]ethyltrimethylammonium-2,2,2-trifluoracetat;
2-[2-[6-[[3-(2,3-Difluor-4-methoxyphenyl)imidazo[1,2-a]pyrazin-8-yl]amino]-1-oxo-3,4-dihydroisochinolin-2-yl]ethoxy]ethyltrimethylammoniumformiat;
2-[2-[[2-Chlor-4-[[3-[4-(cyanomethoxy)-2,3-difluorphenyl]imidazo[1,2-a]pyrazin-8-yl]amino]benzoyl]amino]ethoxy]ethyltripropylammoniumiodid;
2-Chlor-4-[[3-[4-(cyanomethoxy)-2,3-difluorphenyl]imidazo[1,2-a]pyrazin-8-yl]amino]-N-[2-[2-(1-methylpyrrolidin-1-ium-1-yl)ethoxy]ethyl]benzamidformiat;
3-[[2-Chlor-4-[[3-(2,3-difluor-4-methoxyphenyl)imidazo[1,2-a]pyrazin-8-yl]amino]benzoyl]amino]propyltrimethylammoniumformiat;
3-[[2-Chlor-4-[[3-(2,3-difluor-4-methoxyphenyl)imidazo[1,2-a]pyrazin-8-yl]amino]benzoyl]amino]propyltriethylammoniumformiat;
Tributyl-[3-[[2-chlor-4-[[3-(2,3-difluor-4-methoxyphenyl)imidazo[1,2-a]pyrazin-8-yl]amino]benzoyl]amino]propyl]ammoniumformiat;
Carboxymethyl-[2-[2-[[4-[[3-(2,3-difluor-4-methoxyphenyl)imidazo[1,2-a]pyrazin-8-yl]amino]-2-ethylbenzoyl]amino]ethoxy]ethyl]dimethylammoniumformiat;
2-[2-[[4-[[3-(2,3-Difluor-4-methoxyphenyl)imidazo[1,2-a]pyrazin-8-yl]amino]-2-ethylbenzoyl]amino]ethoxy]ethyl-(2-hydroxyethyl)dimethylammoniumformiat;
2-[2-[[4-[[3-(2,3-Difluor-4-prop-2-inoxyphenyl)imidazo[1,2-a]pyrazin-8-yl]amino]-2-ethylbenzoyl]amino]ethoxy]ethyltrimethylammonium-2,2,2-trifluoracetat;
(2-Amino-2-oxoethyl)-[2-[2-[[4-[[3-(2,3-difluor-4-methoxyphenyl)imidazo[1,2-a]pyrazin-8-yl]amino]-2-ethylbenzoyl]amino]ethoxy]ethyl]dimethylammoniumformiat;
3-Carboxypropyl-[2-[2-[[4-[[3-(2,3-difluor-4-methoxyphenyl)imidazo[1,2-a]-pyrazin-8-yl]amino]-2-ethylbenzoyl]amino]ethoxy]ethyl]dimethylammoniumformiat;
2-(4-(2-Chlor-4-((3-(4-(cyanomethoxy)-2,3-difluorphenyl)imidazo[1,2-a]pyrazin-8-yl)amino)benzoyl)piperazin-1-yl)-N,N,N-trimethyl-2-oxoethan-1-aminiumformiat;
2-(4-(2-Chlor-4-((3-(2,3-difluor-4-methoxyphenyl)imidazo[1,2-a]pyrazin-8-yl)amino)benzoyl)piperazin-1-yl)-N,N,N-trimethyl-2-oxoethan-1-aminiumiodid;
(2S,4R)-2-(4-(4-((3-(2,3-Difluor-4-methoxyphenyl)imidazo[1,2-a]pyrazin-8-yl)amino)-2-methylbenzoyl)piperazin-1-carbonyl)-4-hydroxy-1,1-dimethylpyrrolidin-1-iumformiat;
[2-[4-[4-[[3-[4-(Cyanomethoxy)-2,3-difluorphenyl]imidazo[1,2-a]pyrazin-8-yl]amino]-2-methylbenzoyl]piperazin-1-yl]-2-oxoethyl]trimethylazaniumiodid;
4-[4-[2-[[4-[[3-[4-(Difluormethoxy)phenyl]imidazo[1,2-a]pyrazin-8-yl]amino]-2-methylbenzoyl]methylamino]ethyl]-1-methylpiperidin-1-ium-1-yl]butansäure-hydrochlorid;
[2-[2-[[2-Chlor-4-[[3-[3-(trifluormethyl)-1H-pyrazol-4-yl]imidazo[1,2-a]pyrazin-8-yl]amino]benzoyl]amino]ethylamino]-2-oxoethyl]trimethylammoniumformiat;
[2-[[3-[[2-Chlor-4-[[3-(2,3-difluor-4-methoxyphenyl)imidazo[1,2-a]pyrazin-8-yl]amino]benzoyl]amino]cyclobutyl]amino]-2-oxoethyl]trimethylammoniumformiat;
2-(4-(4-((3-(2,3-Difluor-4-methoxyphenyl)imidazo[1,2-a]pyrazin-8-yl)amino)-2-methylbenzamido)piperidin-1-yl)-N,N,N-trimethyl-2-oxoethan-1-aminiumchlorid;
[2-[3-[[2-Chlor-4-[[3-[3-(trifluormethyl)-1H-pyrazol-4-yl]imidazo[1,2-a]pyrazin-8-yl]amino]benzoyl]amino]propylamino]-2-oxoethyl]trimethylammoniumformiat;
[2-[[3-[[2-Chlor-4-[[3-[3-(trifluormethyl)-1H-pyrazol-4-yl]imidazo[1,2-a]pyrazin-8-yl]amino]benzoyl]amino]cyclobutyl]amino]-2-oxoethyl]trimethylammonium-formiat;
[2-[4-[2-Chlor-4-[[3-[3-(trifluormethyl)-1H-pyrazol-4-yl]imidazo[1,2-a]pyrazin-8-yl]amino]benzoyl]piperazin-1-yl]-2-oxoethyl]trimethylammoniumformiat;
2-(((1s,4s)-4-(4-((3-(2,3-Difluor-4-methoxyphenyl)imidazo[1,2-a]pyrazin-8-yl)amino)-2-methylbenzamido)cyclohexyl)amino)-N,N,N-trimethyl-2-oxoethan-1-aminiumchlorid;
[2-[4-[4-[[3-[1-(Cyanomethyl)-3-(trifluormethyl)pyrazol-4-yl]imidazo[1,2-a]pyrazin-8-yl]amino]-2-methylbenzoyl]piperazin-1-yl]-2-oxoethyl]trimethylammoniumformiat;
[2-[4-[2-Chlor-4-[[3-[1-(cyanomethyl)-3-(trifluormethyl)pyrazol-4-yl]imidazo-[1,2-a]pyrazin-8-yl]amino]benzoyl]piperazin-1-yl]-2-oxoethyl]trimethylammoniumformiat;
2-(2-(4-((3-(2,3-Difluor-4-methoxyphenyl)imidazo[1,2-a]pyrazin-8-yl)amino)-2-fluor-6-methylbenzamido)ethoxy)-N,N,N-trimethylethan-1-aminiumhydrogencarbonat;
2-[[3-[[2-Chlor-4-[[3-(2,3-difluor-4-methoxyphenyl)imidazo[1,2-a]pyrazin-8-yl]amino]benzoyl]amino]cyclobutyl]amino]ethyltrimethylammoniumformiat;
(S)-2-(3-(4-((3-(2,3-Difluor-4-methoxyphenyl)imidazo[1,2-a]pyrazin-8-yl)amino)-2-methylbenzamido)pyrrolidin-1-yl)-N,N,N-trimethyl-2-oxoethan-1-aminiumchlorid;
2-(3-(4-((3-(2,3-Difluor-4-methoxyphenyl)imidazo[1,2-a]pyrazin-8-yl)amino)-2-methylbenzamido)piperidin-1-yl)-N,N,N-trimethyl-2-oxoethan-1-aminiumchlorid;
2-(((1r,4r)-4-(4-((3-(2,3-Difluor-4-methoxyphenyl)imidazo[1,2-a]pyrazin-8-yl)amino)-2-methylbenzamido)cyclohexyl)amino)-N,N,N-trimethyl-2-oxoethan-1-aminiumchlorid;
(R)-2-(3-(4-((3-(2,3-Difluor-4-methoxyphenyl)imidazo[1,2-a]pyrazin-8-yl)amino)-2-methylbenzamido)pyrrolidin-1-yl)-N,N,N-trimethyl-2-oxoethan-1-aminiumchlorid;
2-Amino-N-(2-(3-(4-((3-(2,3-difluor-4-methoxyphenyl)imidazo[1,2-a]pyrazin-8-yl)amino)-2-ethylbenzamido)azetidin-1-yl)-2-oxoethyl)-N,N-dimethyl-2-oxoethan-1-aminiumformiat;
2-(((1s,3s)-3-(4-((3-(2,3-Difluor-4-methoxyphenyl)imidazo[1,2-a]pyrazin-8-yl)amino)-2-methylbenzamido)cyclobutyl)amino)-N,N,N-trimethyl-2-oxoethan-1-aminiumchlorid;
3-(2-Chlor-4-((3-(2,3-difluor-4-methoxyphenyl)imidazo[1,2-a]pyrazin-8-yl)amino)benzamido)-N-(2-hydroxyethyl)-N,N-dimethylpropan-1-aminiumformiat;
N-(Cyanomethyl)-2-(2-(4-((3-(2,3-difluor-4-methoxyphenyl)imidazo[1,2-a]pyrazin-8-yl)amino)-2-ethylbenzamido)ethoxy)-N,N-dimethylethan-1-aminiumformiat;
N-(2-(2-(4-((3-(2,3-Difluor-4-methoxyphenyl)imidazo[1,2-a]pyrazin-8-yl)amino)-2-ethylbenzamido)ethoxy)ethyl)-2-ethoxy-N,N-dimethyl-2-oxoethan-1-aminiumformiat;
Mono(N-(2-amino-2-oxoethyl)-5-(4-((3-(2,3-difluor-4-methoxyphenyl)imidazo[1,2-a]-pyrazin-8-yl)amino)-2-ethylbenzamido)-N,N-dimethylpentan-1-aminium)monoformiatmonoiodid;
trans-[2-[[4-[[4-[[3-(2,3-Difluor-4-methoxyphenyl)imidazo[1,2-a]pyrazin-8-yl]amino]-2-ethylbenzoyl]amino]cyclohexyl]amino]-2-oxoethyl]trimethylazaniumformiat;
1-(Cyanomethyl)-1-(2-(2-(4-((3-(1-(cyanomethyl)-3-(trifluormethyl)-1H-pyrazol-4-yl)imidazo[1,2-a]pyrazin-8-yl)amino)-2-ethylbenzamido)ethoxy)ethyl)pyrrolidin-1-iumformiat;
4-(Cyanomethyl)-4-(2-(2-(4-((3-(1-(cyanomethyl)-3-(trifluormethyl)-1H-pyrazol-4-yl)imidazo[1,2-a]pyrazin-8-yl)amino)-2-ethylbenzamido)ethoxy)ethyl)-morpholin-4-iumformiat;
1-(2-Amino-2-oxoethyl)-4-(4-(4-((3-(4-(difluormethoxy)-2,3-difluorphenyl)imidazo-[1,2-a]pyrazin-8-yl)amino)-2-ethylbenzoyl)piperazin-1-carbonyl)-1-methylpiperidin-1-iumformiat;
5-[[4-[[3-[4-(Difluormethoxy)-2,3-difluorphenyl]imidazo[1,2-a]pyrazin-8-yl]amino]-2-ethylbenzoyl]amino]pentyldimethyl-(2-oxo-2-piperazin-1-ylethyl)ammonium-bis(2,2,2-trifluoracetat);
N-[[3-(Azetidin-3-ylmethyl)-3-methyl-3-azoniabicyclo[3.1.0]hexan-6-yl]methyl]-4-[[3-[4-(difluormethoxy)-2,3-difluorphenyl]imidazo[1,2-a]pyrazin-8-yl]amino]-2-ethylbenzamidbis(2,2,2-trifluoracetat);
1-(Azetidin-3-ylmethyl)-4-(4-(4-((3-(4-(difluormethoxy)-2,3-difluorphenyl)imidazo[1,2-a]pyrazin-8-yl)amino)-2-ethylbenzoyl)piperazin-1-carbonyl)-1-methylpiperidin-1-ium-2,2,2-trifluoracetat;
N-[3-[[4-[[3-[4-(Difluormethoxy)-2,3-difluorphenyl]imidazo[1,2-a]pyrazin-8-yl]amino]-2-ethylbenzoyl]amino]propyl]-1-methyl-1-(pyrrolidin-3-ylmethyl)-piperidin-1-ium-4-carboxamid-bis(2,2,2-trifluoracetat);
[4-[1-(2-Aminoethyl)-1-methylpiperidin-1-ium-4-carbonyl]piperazin-1-yl]-[4-[[3-[4-(difluormethoxy)-2,3-difluorphenyl]imidazo[1,2-a]pyrazin-8-yl]amino]-2-ethylphenyl]methanon-bis(2,2,2-trifluoracetat);
[4-[[3-[4-(Difluormethoxy)-2,3-difluorphenyl]imidazo[1,2-a]pyrazin-8-yl]amino]-2-ethylphenyl]-[4-[1-methyl-1-(pyrrolidin-3-ylmethyl)piperidin-1-ium-4-carbonyl]piperazin-1-yl]methanon-bis(2,2,2-trifluoracetat);
[4-[[3-[4-(Difluormethoxy)-2,3-difluorphenyl]imidazo[1,2-a]pyrazin-8-yl]amino]-2-ethylphenyl]-[4-[1-methy1-1-[2-(methylamino)ethyl]piperidin-1-ium-4-carbonyl]piperazin-1-yl]methanon-bis(2,2,2-trifluoracetat);
[4-[1-[(3-Amino-1-bicyclo[1.1.1]pentanyl)methyl]-1-methylpiperidin-1-ium-4-carbonyl]piperazin-1-yl]-[4-[[3-[4-(difluormethoxy)-2,3-difluorphenyl]imidazo[1,2-a]pyrazin-8-yl]amino]-2-ethylphenyl]methanon-bis(2,2,2-trifluoracetat);
N-[2-[1-(Azetidin-3-ylmethyl)-1-methylpiperidin-1-ium-4-yl]ethyl]-4-[[3-[4-(difluormethoxy)-2,3-difluorphenyl]imidazo[1,2-a]pyrazin-8-yl]amino]-2-ethylbenzamiddiformiat;
N-[[1-(Azetidin-3-ylmethyl)-1-methylpiperidin-1-ium-4-yl]methyl]-4-[[3-[4-(difluormethoxy)-2,3-difluorphenyl]imidazo[1,2-a]pyrazin-8-yl]amino]-2-ethylbenzamiddiformiat;
N-[[1-(Azetidin-3-ylmethyl)-1-methylpyrrolidin-1-ium-3-yl]methyl]-4-[[3-[4-(difluormethoxy)-2,3-difluorphenyl]imidazo[1,2-a]pyrazin-8-yl]amino]-2-ethylbenzamid-bis(2,2,2-trifluoracetat);
Bis(azetidin-3-ylmethyl)-[5-[[4-[[3-[4-(difluormethoxy)-2,3-difluorphenyl]imidazo-[1,2-a]pyrazin-8-yl]amino]-2-ethylbenzoyl]amino]pentyl]methylammonium-bis(2,2,2-trifluoracetat);
N-[2-[1-(Azetidin-3-ylmethyl)-1-methylazetidin-1-ium-3-yl]ethyl]-4-[[3-[4-(difluormethoxy)-2,3-difluorphenyl]imidazo[1,2-a]pyrazin-8-yl]amino]-2-ethylbenzamiddiformiat;
N-[2-[1-(Azetidin-3-ylmethyl)-1-methylpiperidin-1-ium-4-yl]ethyl]-4-[[3-[4-(difluormethoxy)-2,3-difluorphenyl]imidazo[1,2-a]pyrazin-8-yl]amino]-2-ethylbenzamiddiformiat;
N-[3-[4-(Azetidin-3-ylmethyl)-4-methylpiperazin-4-ium-1-yl]-3-oxopropyl]-4-[[3-[4-(difluormethoxy)-2,3-difluorphenyl]imidazo[1,2-a]pyrazin-8-yl]amino]-2-ethylbenzamid-bis(2,2,2-trifluoracetat);
Methyl-4-[1-(azetidin-3-ylmethyl)-1-methylpiperidin-1-ium-4-carbonyl]-1-[4-[[3-[4-(difluormethoxy)-2,3-difluorphenyl]imidazo[1,2-a]pyrazin-8-yl]amino]-2-ethylbenzoyl]piperazin-2-carboxylat-bis(2,2,2-trifluoracetat);
Methyl-1-[1-(azetidin-3-ylmethyl)-1-methylpiperidin-1-ium-4-carbonyl]-4-[4-[[3-[4-(difluormethoxy)-2,3-difluorphenyl]imidazo[1,2-a]pyrazin-8-yl]amino]-2-ethylbenzoyl]piperazin-2-carboxylat-tris(2,2,2-trifluoracetat);
[4-[[3-[4-(Difluormethoxy)-2,3-difluorphenyl]imidazo[1,2-a]pyrazin-8-yl]amino]-2-ethylphenyl]-[2-[1-methyl-1-(pyrrolidin-3-ylmethyl)piperidin-1-ium-4-carbonyl]-2,6-diazaspiro[3.3]heptan-6-yl]methanon-bis(2,2,2-trifluoracetat);
Azetidin-3-ylmethyl-[5-[[4-[[3-[4-(difluormethoxy)-2,3-difluorphenyl]imidazo-[ 1,2-a]pyrazin-8-yl]amino]-2-ethylbenzoyl]amino]pentyl]-dimethylammonium-bis(2,2,2-trifluoracetat);
[4-[[3-[4-(Difluormethoxy)-2,3-difluorphenyl]imidazo[1,2-a]pyrazin-8-yl]amino]-2-ethylphenyl]-[4-[4-methyl-4-(pyrrolidin-3-ylmethyl)piperazin-4-ium-1-carbonyl]-1-piperidyl]methanon-2,2,2-trifluoracetat;
N-[[1-(Azetidin-3-ylmethyl)-1-methylpyrrolidin-1-ium-3-yl]methyl]-1-[4-[[3-[4-(difluormethoxy)-2,3-difluorphenyl]imidazo[1,2-a]pyrazin-8-yl]amino]-2-ethylbenzoyl]piperidin-4-carboxamidformiat/2,2,2-trifluoracetat (Mischsalz);
[4-[2-(Azetidin-3-ylmethyl)-2-methyl-6-aza-2-azoniaspiro[3.3]-6-carbonyl]-1-piperidyl]-[4-[[3-[4-(difluormethoxy)-2,3-difluorphenyl]imidazo[1,2-a]pyrazin-8-yl]amino]-2-ethylphenyl]methanonameisensäure-2,2,2-trifluoracetat (Mischsalz);
[4-[[3-[4-(Difluormethoxy)-2,3-difluorphenyl]imidazo[1,2-a]pyrazin-8-yl]amino]-2-ethylphenyl]-[4-[rac-(1S,4S)-5-(azetidin-3-ylmethyl)-5-methyl-2-aza-5-azoniabicyclo[2.2.1]heptan-2-carbonyl]-1-piperidyl]methanonameisensäure-2,2,2-trifluoracetat (Mischsalz);
[4-[6-(Azetidin-3-ylmethyl)-6-methyl-3-aza-6-azoniabicyclo[3.1.1]heptan-3-carbonyl]-1-piperidyl]-[4-[[3-[4-(difluormethoxy)-2,3-difluorphenyl]imidazo[1,2-a]pyrazin-8-yl]amino]-2-ethylphenyl]methanonformiat-2,2,2-trifluoracetat (Mischsalz);
N-[2-[[4-[[3-[4-(Difluormethoxy)-2,3-difluorphenyl]imidazo[1,2-a]pyrazin-8-yl]amino]-2-ethylbenzoyl]amino]ethyl]-1-methyl-1-(pyrrolidin-3-ylmethyl)piperidin-1-ium-4-carboxamid-bis(2,2,2-trifluoracetat);
[4-[[3-[4-(Difluormethoxy)-2,3-difluorphenyl]imidazo[1,2-a]pyrazin-8-yl]amino]-2-fluorphenyl]-[4-[1-methyl-1-(pyrrolidin-3-ylmethyl)piperidin-1-ium-4-carbonyl]piperazin-1-yl]methanon-2,2,2-trifluoracetat;
3-Aminopropyl-(carboxymethyl)-[5-[[4-[[3-(2,3-difluor-4-methoxyphenyl)imidazo[1,2-a]pyrazin-8-yl]amino]-2-ethylbenzoyl]amino]pentyl]methylammoniumformiat;
5-Aminopentyl-(carboxymethyl)-[5-[[4-[[3-(2,3-difluor-4-methoxyphenyl)imidazo-[1,2-a]pyrazin-8-yl]amino]-2-ethylbenzoyl]amino]pentyl]methylammonium-2,2,2-trifluoracetat;
4-Aminobutyl-(carboxymethyl)-[5-[[4-[[3-(2,3-difluor-4-methoxyphenyl)imidazo[1,2-a]pyrazin-8-yl]amino]-2-ethylbenzoyl]amino]pentyl]methylammoniumformiat;
Azetidin-3-ylmethyl-(carboxymethyl)-[5-[[4-[[3-(2,3-difluor-4-methoxyphenyl)imidazo[1,2-a]pyrazin-8-yl]amino]-2-ethylbenzoyl]amino]pentyl]methylammoniumformiat;
Azetidin-3-ylmethyl-(3-carboxypropyl)-[5-[[4-[[3-(2,3-difluor-4-methoxyphenyl)imidazo[1,2-a]pyrazin-8-yl]amino]-2-ethylbenzoyl]amino]pentyl]methylammonium-2,2,2-trifluoracetat;
Azetidin-3-ylmethyl-(carboxymethyl)-[5-[[4-[[3-[1-(2,2-difluorethyl)-3-(trifluormethyl)pyrazol-4-yl]imidazo[1,2-a]pyrazin-8-yl]amino]-2-ethylbenzoyl]amino]pentyl]methylammoniumformiat;
2-[[3-[[2-Chlor-4-[[3-[3-(trifluormethyl)-1H-pyrazol-4-yl]imidazo[1,2-a]pyrazin-8-yl]amino]benzoyl]amino]cyclobutyl]amino]ethyltrimethylammoniumformiat;
Azetidin-3-ylmethyl-(carboxymethyl)-[5-[[4-[[3-[1-(cyanomethyl)-3-(trifluormethyl)-pyrazol-4-yl]imidazo[1,2-a]pyrazin-8-yl]amino]-2-ethylbenzoyl]amino]pentyl]-methylammoniumformiat;
Carboxymethyl-[5-[[4-[[3-[1-(cyanomethyl)-3-(trifluormethyl)pyrazol-4-yl]-imidazo[1,2-a]pyrazin-8-yl]amino]-2-ethylbenzoyl]amino]pentyl]dimethyl-ammoniumformiat;
Trimethyl-[2-[4-[2-methyl-4-[[3-[1-methyl-3-(trifluormethyl)pyrazol-4-yl]imidazo[1,2-a]pyrazin-8-yl]amino]benzoyl]piperazin-1-yl]-2-oxoethyl]ammoniumformiat;
4-[2-[2-[[2-Chlor-4-[[3-[4-(cyanomethoxy)-2,3-difluorphenyl]imidazo[1,2-a]pyrazin-8-yl]amino]benzoyl]amino]ethoxy]ethyl]-4-methylpiperazin-4-ium-2-carbonsäureformiat;
Bis(carboxymethyl)-[5-[[4-[[3-(2,3-difluor-4-methoxyphenyl)imidazo[1,2-a]pyrazin-8-yl]amino]-2-ethylbenzoyl]amino]pentyl]methylammonium-2,2,2-trifluoracetat;
4-[[3-[4-(4-Aminobut-2-inoxy)phenyl]imidazo[1,2-a]pyrazin-8-yl]amino]-N-[2-(1,1-dimethylpiperidin-1-ium-4-yl)ethyl]-N,2-dimethylbenzamidformiat;
Carboxymethyl-[5-[[4-[[3-[1-(2,2-difluorethyl)-3-(trifluormethyl)pyrazol-4-yl]-imidazo[1,2-a]pyrazin-8-yl]amino]-2-ethylbenzoyl]amino]pentyl]dimethyl-ammoniumformiat;
1-(Azetidin-3-ylmethyl)-N-[(2S)-2-[[4-[[3-[4-(difluormethoxy)-2,3-difluorphenyl]-imidazo[1,2-a]pyrazin-8-yl]amino]-2-ethylbenzoyl]amino]propyl]-1-methylpiperidin-1-ium-4-carboxamidformiat;
2-[4-[[[4-[[3-(2,3-Difluor-4-methoxyphenyl)imidazo[1,2-a]pyrazin-8-yl]amino]-2-ethylbenzoyl]amino]methyl]-1-methylpiperidin-1-ium-1-yl]essigsäure-2,2,2-trifluoracetat;
[2-Chlor-4-[[3-[4-(difluormethoxy)-2,3-difluorphenyl]imidazo[1,2-a]pyrazin-8-yl]amino]phenyl]-[4-[1-methyl-1-(pyrrolidin-3-ylmethyl)piperidin-1-ium-4-carbonyl]piperazin-1-yl]methanonformiat;
N-[3-[[2-Chlor-4-[[3-[4-(difluormethoxy)-2,3-difluorphenyl]imidazo[1,2-a]pyrazin-8-yl]amino]benzoyl]amino]propyl]-1-methyl-1-(pyrrolidin-3-ylmethyl)piperidin-1-ium-4-carboxamidformiat;
N-[3-[[4-[[3-(2,3-Difluor-4-pyrimidin-2-yloxyphenyl)imidazo[1,2-a]pyrazin-8-yl]-amino]-2-ethylbenzoyl]amino]propyl]-1-methyl-1-(pyrrolidin-3-ylmethyl)piperidin-1-ium-4-carboxamidformiat;
4-[[3-(2,3-Difluor-4-pyrimidin-2-yloxyphenyl)imidazo[1,2-a]pyrazin-8-yl]amino]-2-ethyl-N-[[1-methyl-1-(pyrrolidin-3-ylmethyl)piperidin-1-ium-4-yl]-methyl]benzamidformiat;
4-[[3-[2,3-Difluor-4-(2-methyltriazol-4-yl)oxyphenyl]imidazo[1,2-a]pyrazin-8-yl]amino]-2-ethyl-N-[[1-methyl-1-(pyrrolidin-3-ylmethyl)piperidin-1-ium-4-yl]methyl]benzamidformiat;
[4-[[3-[4-(Difluormethoxy)-2,3-difluorphenyl]imidazo[1,2-a]pyrazin-8-yl]amino]-2-ethylphenyl]-[4-methyl-4-(pyrrolidin-3-ylmethyl)piperazin-4-ium-1-yl]methanonformiat;
Methyl-(2S)-2-[[1-(azetidin-3-ylmethyl)-1-methylpiperidin-1-ium-4-carbonyl]amino]-3-[[4-[[3-(2,3-difluor-4-methoxyphenyl)imidazo[1,2-a]pyrazin-8-yl]amino]-2-ethylbenzoyl]amino]propanoatformiat-2,2,2-trifluoracetat (Mischsalz);
Methyl-(2S)-2-[[1-(azetidin-3-ylmethyl)-1-methylpiperidin-1-ium-4-carbonyl]amino]-4-[[4-[[3-(2,3-difluor-4-methoxyphenyl)imidazo[1,2-a]pyrazin-8-yl]amino]-2-ethylbenzoyl]amino]butanoatformiat-2,2,2-trifluoracetat (Mischsalz);
Methyl-(2S)-3-[[1-(azetidin-3-ylmethyl)-1-methylpiperidin-1-ium-4-carbonyl]amino]-2-[[4-[[3-(2,3-difluor-4-methoxyphenyl)imidazo[1,2-a]pyrazin-8-yl]amino]-2-ethylbenzoyl]amino]propanoatformiat-2,2,2-trifluoracetat (Mischsalz);
Methyl-(2S)-2-[[1-(azetidin-3-ylmethyl)-1-methylpiperidin-1-ium-4-carbonyl]amino]-4-[[4-[[3-(2,3-difluor-4-methoxyphenyl)imidazo[1,2-a]pyrazin-8-yl]amino]-2-ethylbenzoyl]amino]butanoatformiat-2,2,2-trifluoracetat (Mischsalz);
Methyl-1-[1-(azetidin-3-ylmethyl)-1-methylpiperidin-1-ium-4-carbonyl]-4-[[[4-[[3-(2,3-difluor-4-methoxyphenyl)imidazo[1,2-a]pyrazin-8-yl]amino]-2-ethylbenzoyl]amino]methyl]piperidin-4-carboxylatformiat-2,2,2-trifluoracetat (Mischsalz);
Methyl-2-[1-[1-(azetidin-3-ylmethyl)-1-methylpiperidin-1-ium-4-carbonyl]-4-piperidyl]-2-[[4-[[3-(2,3-difluor-4-methoxyphenyl)imidazo[1,2-a]pyrazin-8-yl]amino]-2-ethylbenzoyl]amino]acetatformiat-2,2,2-trifluoracetat (Mischsalz);
1-(Azetidin-3-ylmethyl)-N-[3-[[4-[[3-(2,3-difluor-4-methoxyphenyl)imidazo[1,2-a]-pyrazin-8-yl]amino]-2-ethylbenzoyl]amino]cyclobutyl]-1-methylpiperidin-1-ium-4-carboxamidformiat - 2,2,2-trifluoracetat (Mischsalz);
1-(Azetidin-3-ylmethyl)-N-[3-[[[4-[[3-(2,3-difluor-4-methoxyphenyl)imidazo[1,2-a]pyrazin-8-yl]amino]-2-ethylbenzoyl]amino]methyl]cyclobutyl]-1-methylpiperidin-1-ium-4-carboxamidformiat-2,2,2-trifluoracetat (Mischsalz);
2-[1-(Azetidin-3-ylmethyl)-4-[[[4-[[3-(2,3-difluor-4-methoxyphenyl)imidazo[1,2-a]pyrazin-8-yl]amino]-2-ethylbenzoyl]amino]methyl]piperidin-1-ium-1-yl]essigsäure-bis(2,2,2-trifluoracetat);
1-(Azetidin-3-ylmethyl)-N-[3-[[4-[[3-(2,3-difluor-4-methoxyphenyl)imidazo[1,2-a]pyrazin-8-yl]amino]-2-ethylbenzoyl]amino]propyl]-1-methylpiperidin-1-ium-4-carboxamid-bis(trifluoracetat);
N-[3-[[2-[(3S)-1-(Azetidin-3-ylmethyl)-1-methylpyrrolidin-1-ium-3-yl]acetyl]amino]propyl]-4-[[3-(2,3-difluor-4-methoxyphenyl)imidazo[1,2-a]pyrazin-8-yl]amino]-2-ethylbenzamid-bis(2,2,2-trifluoracetat);
N-[3-[[2-[1-(Azetidin-3-ylmethyl)-1-methylpiperidin-1-ium-4-yl]acetyl]amino]propyl]-4-[[3-(2,3-difluor-4-methoxyphenyl)imidazo[1,2-a]pyrazin-8-yl]amino]-2-ethylbenzamid-bis(2,2,2-trifluoracetat);
4-[[3-(2,3-Difluor-4-methoxyphenyl)imidazo[1,2-a]pyrazin-8-yl]amino]-2-ethyl-N-[[1-methyl-1-[2-oxo-2-(pyrrolidin-3-ylmethylamino)ethyl]piperidin-1-ium-4-yl]methyl]benzamid-bis(2,2,2-trifluoracetat);
N-[[1-[1-(Azetidin-3-ylmethyl)-1-methylpiperidin-1-ium-4-carbonyl]-4-piperidyl]-methyl]-4-[[3-(2,3-difluor-4-methoxyphenyl)imidazo[1,2-a]pyrazin-8-yl]amino]-2-ethylbenzamid-bis(2,2,2-trifluoracetat);
N-[[1-(Azetidin-3-ylmethyl)-1-methylpiperidin-1-ium-4-yl]methyl]-4-[[3-(2,3-difluor-4-methoxyphenyl)imidazo[1,2-a]pyrazin-8-yl]amino]-2-ethylbenzamid-2,2,2-trifluoracetat-bis(2,2,2-trifluoracetat);
4-[[3-(2,3-Difluor-4-methoxyphenyl)imidazo[1,2-a]pyrazin-8-yl]amino]-N-[[1-[(1,1-dimethylpyrrolidin-1-ium-3-yl)methyl]-1-methylpiperidin-1-ium-4-yl]methyl]-2-ethylbenzamiddiformiat;
N-[1-(Azetidin-3-ylmethyl)-1-methylpiperidin-1-ium-4-yl]-4-[[3-(2,3-difluor-4-methoxyphenyl)imidazo[1,2-a]pyrazin-8-yl]amino]-2-ethylbenzamid-bis(2,2,2-trifluoracetat);
4-[[3-(2,3-Difluor-4-methoxyphenyl)imidazo[1,2-a]pyrazin-8-yl]amino]-2-ethyl-N-[rac-(1S,5R)-3-(azetidin-3-ylmethyl)-3-methyl-3-azoniabicyclo[3.1.0]hexan-6-yl]benzamid-bis(2,2,2-trifluoracetat);
N-[[1-(Azetidin-3-ylmethyl)-1-methylpyrrolidin-1-ium-3-yl]methyl]-2-ethyl-4-[[3-[3-(trifluormethyl)-1H-pyrazol-4-yl]imidazo[1,2-a]pyrazin-8-yl]amino]benzamid-bis(2,2,2-trifluoracetat);
Bis(azetidin-3-ylmethyl)-[3-[[4-[[3-(2,3-difluor-4-methoxyphenyl)imidazo[1,2-a]-pyrazin-8-yl]amino]-2-ethylbenzoyl]amino]propyl]methylammonium-bis(2,2,2-trifluoracetat);
4-[[3-(2,3-Difluor-4-methoxyphenyl)imidazo[1,2-a]pyrazin-8-yl]amino]-2-ethyl-N-[[1-methyl-1-(3-pyridylmethyl)piperidin-1-ium-4-yl]methyl]benzamid;2,2,2-trifluoracetat;
N-[[1-(Azetidin-3-ylmethyl)-1-(2-hydroxyethyl)piperidin-1-ium-4-yl]methyl]-4-[[3-(2,3-difluor-4-methoxyphenyl)imidazo[1,2-a]pyrazin-8-yl]amino]-2-ethylbenzamid-2,2,2-trifluoracetat;
4-[[3-(2,3-Difluor-4-methoxyphenyl)imidazo[1,2-a]pyrazin-8-yl]amino]-2-ethyl-N-[[1-methyl-1-(pyrrolidin-2-ylmethyl)piperidin-1-ium-4-yl]methyl]benzamid-2,2,2-trifluoracetat;
N-[[1-(Azetidin-3-ylmethyl)-1-ethylpiperidin-1-ium-4-yl]methyl]-4-[[3-(2,3-difluor-4-methoxyphenyl)imidazo[1,2-a]pyrazin-8-yl]amino]-2-ethylbenzamidformiat;
1-[2-[[4-[[3-(2,3-Difluor-4-methoxyphenyl)imidazo[1,2-a]pyrazin-8-yl]amino]-2-ethylbenzoyl]amino]ethyl]-1-methylpyrrolidin-1-ium-3-carboxamid-2,2,2-trifluoracetat;
4-[[3-(2,3-Difluor-4-phenoxyphenyl)imidazo[1,2-a]pyrazin-8-yl]amino]-2-ethyl-N-[[1-methyl-1-(pyrrolidin-3-ylmethyl)piperidin-1-ium-4-yl]methyl]benzamidformiat; und
1-(2-Methoxy-2-oxoethyl)-4-(2-(4-((3-(4-methoxyphenyl)imidazo[1,2-a]pyrazin-8-yl)amino)-2-methylbenzamido)ethyl)-1-methylpiperidin-1-iumformiat.

12. Verbindung der Formel (IIa) oder (IIIa) nach einem der Ansprüche 1 bis 11 oder ein pharmazeutisch unbedenkliches Salz davon zur Verwendung als therapeutisch wirksame Substanz.

13. Pharmazeutische Zusammensetzung, umfassend eine Verbindung der Formel (IIa) oder (IIIa) nach einem der Ansprüche 1 bis 11 oder ein pharmazeutisch unbedenkliches Salz davon und einen therapeutisch inerten Träger.

14. Verbindung der Formel (IIa) oder (IIIa) nach einem der Ansprüche 1 bis 11 oder ein pharmazeutisch unbedenkliches Salz davon zur Verwendung als Antibiotikum.

15. Verbindung der Formel (IIa) oder (IIIa) nach einem der Ansprüche 1 bis 11 oder ein pharmazeutisch unbedenkliches Salz davon zur Verwendung bei der Behandlung oder Prävention von Infektionen und daraus resultierenden Krankheiten, die durch *Acinetobacter baumannii* verursacht werden.

## Revendications

1. Composé de formule (IIa) ou (IIIa) ou un sel pharmaceutiquement acceptable de celui-ci 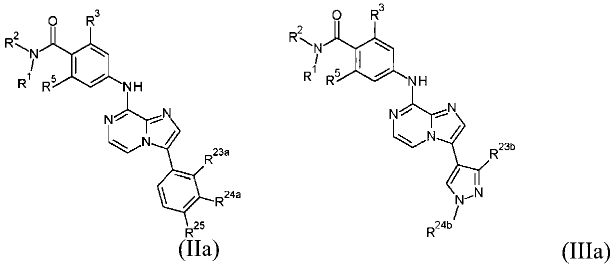 dans lequel :
R¹ est choisi parmi un R⁶-alkyle en C₁-C₆-, un R⁶-alkyle en C₁-C₆-CH(alcoxy en C₁-C₆-carbonyl)-, un R⁷-alkyle en C₁-C₆-X-alkyle en C₁-C₆-, un groupe 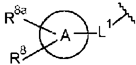 et un groupe 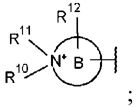 et
R² est choisi parmi un hydrogène et un alkyle en C₁-C₆ ;
ou R¹ et R², pris conjointement avec l'atome d'azote auquel ils sont liés, forment un hétérocycle à 3 à 14 chaînons qui est substitué par R⁹ et R^{9a} ;
R³ est choisi parmi un hydrogène, un halogène et un alkyle en C₁-C₆ ; ou
R³ et R², pris conjointement avec les atomes auxquels ils sont liés, forment un hétérocycle à 3 à 14 chaînons ;
R⁵ est choisi parmi un hydrogène, un alkyle en C₁-C₆ et un halogène ;
R⁶ représente un groupe R¹⁶-L²- ;
R⁷ représente un groupe R¹⁶-L³- ;
R⁸ représente un groupe R¹⁶-L⁴- ;
R^{8a}, R^{9a}, R^{11a}, R¹², R¹⁸, R¹⁹, R²⁰, R²¹ et R²² sont indépendamment choisis parmi un hydrogène, un halogène, un hydroxy, un cyano, un amino, un carbamoyle, un carboxy, un alkyle en C₁-C₆, un alcoxy en C₁-C₆, un halogénoalkyle en C₁-C₆, un cyanoalkyle en C₁-C₆, un carboxyalkyle en C₁-C₆, un hydroxyalkyle en C₁-C₆, un halogénoalcoxy en C₁-C₆, un cyanoalcoxy en C₁-C₆, un alcynyloxy en C₂-C₆, un aminoalcynyloxy en C₂-C₆, un aryloxy, un (hétéroaryle à 5 à 14 chaînons)oxy, un alkyle en C₁-C₆-(hétéroaryle à 5 à 14 chaînons)oxy et un alcoxy en C₁-C₆-carbonyle ;
R⁹ représente un groupe R¹⁶-L⁵- ou un groupe 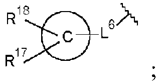
R¹⁰ est choisi parmi un alkyle en C₁-C₆, un alcoxy en C₁-C₆-carbonylalkyle en C₁-C₆, un alkyle en C₁-C₆-NH-alkyle en C₁-C₆, un aminoalkyle en C₁-C₆, un carbamoyle, un carbamoylalkyle en C₁-C₆, un carboxyalkyle en C₁-C₆, un cyanoalkyle en C₁-C₆, un halogénoalkyle en C₁-C₆, un hydroxyalkyle en C₁-C₆ et un groupe 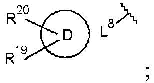
R¹¹ est choisi parmi un alkyle en C₁-C₆, un carbamoyle, un carboxy, un carboxyalkyle en C₁-C₆ et un hydroxyalkyle en C₁-C₆ ;
R¹³ et R¹⁴ sont indépendamment choisis parmi un alkyle en C₁-C₆, un aminoalkyle en C₁-C₆, un carbamoylalkyle en C₁-C₆, un carboxyalkyle en C₁-C₆, un cyanoalkyle en C₁-C₆, un hydroxyalkyle en C₁-C₆, un halogénoalkyle en C₁-C₆, un alcoxy en C₁-C₆-carbonylalkyle en C₁-C₆ et un groupe 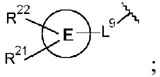
ou R¹³ et R¹⁴, pris conjointement avec l'atome d'azote auquel ils sont liés, forment un hétérocycle à 3 à 14 chaînons qui est éventuellement substitué par 1 à 3 substituants choisis parmi un halogène, un amino, un hydroxy, un cyano, un alkyle en C₁-C₆, un halogénoalkyle en C₁-C₆, un alcoxy en C₁-C₆ et un halogénoalcoxy en C₁-C₆ ;
R¹⁵ représente un alkyle en C₁-C₆ ;
R¹⁶ est choisi parmi un groupe 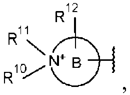 un groupe 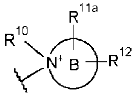 et un groupe 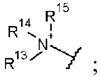
R¹⁷ représente un groupe R¹⁶-L⁷- ;
R^{23a} représente un hydrogène ou un halogène ;
R^{23b} représente un halogénoalkyle en C₁-C₆ ;
R^{24a} représente un hydrogène ou un halogène ;
R^{24b} est choisi parmi un hydrogène, un alkyle en C₁-C₆, un cyanoalkyle en C₁-C₆ et un halogénoalkyle en C₁-C₆ ;
R²⁵ est choisi parmi un halogène, un alcoxy en C₁-C₆, un cyanoalcoxy en C₁-C₆, un halogénoalcoxy en C₁-C₆, un alcynyloxy en C₂-C₆, un aminoalcynyloxy en C₂-C₆, un aryloxy, un (hétéroaryle à 5 à 14 chaînons)oxy et un alkyle en C₁-C₆-(hétéroaryle à 5 à 14 chaînons)oxy ;
L¹ est choisi parmi une liaison covalente, un carbonyle et -alkyle en C₁-C₆- ;
L² et L³ sont indépendamment choisis parmi une liaison covalente, un carbonyle, -alkyle en C₁-C₆-C(O)-NH-, -C(O)-NH-, -NH-C(O)-, -C(O)-NH-CH(alcoxy en C₁-C₆-carbonyle)-, -alkyle en C₁-C₆-CH(OH)-alkyle en C₁-C₆-C(O)- et -alkyle en C₁-C₆-NH- ;
L⁴, L⁵, L⁶, L⁷ et L¹ sont indépendamment choisis parmi une liaison covalente, un carbonyle, -alkyle en C₁-C₆-, -alkyle en C₁-C₆-C(O)-, -alkyle en C₁-C₆-NH-C(O)-, -alkyle en C₁-C₆-C(O)-NH-, -alkyle en C₁-C₆-NH-C(O)-alkyle en C₁-C₆-, -alkyle en C₁-C₆-C(O)-NH-alkyle en C₁-C₆-, -C(O)-NH-, -NH-C(O)-, -C(O)-NH-CH(alcoxy en C₁-C₆-carbonyle)-, -alkyle en C₁-C₆-CH(OH)-alkyle en C₁-C₆-C(O)- et -alkyle en C₁-C₆-NH- ;
L⁹ représente -alkyle en C₁-C₆- ou -C(O)-alkyle en C₁-C₆- ;
A, C, D et E sont chacun indépendamment choisis parmi un aryle en C₆-C₁₀, un cycloalkyle en C₃-C₁₀, un hétérocyclyle à 3 à 14 chaînons et un hétéroaryle à 5 à 14 chaînons ;
B représente un hétérocycle à 3 à 14 chaînons ; et
X est choisi parmi O, S, SO, SO₂, NH et N(alkyle en C₁-C₆).

2. Composé de formule (IIa) ou (IIIa) selon la revendication 1, ou un sel pharmaceutiquement acceptable de celui-ci, dans lequel :
R¹ est choisi parmi un R⁶-alkyle en C₁-C₆-, un R⁶-alkyle en C₁-C₆-CH(alcoxy en C₁-C₆-carbonyl)-, un R⁷-alkyle en C₁-C₆-O-alkyle en C₁-C₆-, un groupe 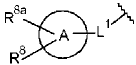 et un groupe 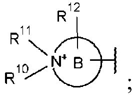
R² est choisi parmi un hydrogène et un alkyle en C₁-C₆ ;
R⁶ représente un groupe R¹⁶-L²- ;
R⁷ représente un groupe R¹⁶-L³- ;
R⁸ représente un groupe R¹⁶-L⁴- ;
R^{8a} représente un hydrogène ou un alcoxy en C₁-C₆-carbonyle ;
R¹⁰ est choisi parmi un alkyle en C₁-C₆, un alcoxy en C₁-C₆-carbonylalkyle en C₁-C₆, un alkyle en C₁-C₆-NH-alkyle en C₁-C₆, un aminoalkyle en C₁-C₆, un carbamoylalkyle en C₁-C₆, un carboxyalkyle en C₁-C₆, un cyanoalkyle en C₁-C₆ et un groupe 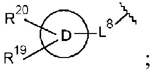
R¹¹ est choisi parmi un alkyle en C₁-C₆, un carbamoyle, un carboxyalkyle en C₁-C₆ et un hydroxyalkyle en C₁-C₆ ;
R^{11a} représente un hydrogène ou un carboxy ;
R¹² représente un hydrogène ou un hydroxy ;
R¹³ est choisi parmi un alkyle en C₁-C₆, un aminoalkyle en C₁-C₆, un carbamoylalkyle en C₁-C₆, un carboxyalkyle en C₁-C₆, un cyanoalkyle en C₁-C₆, un hydroxyalkyle en C₁-C₆, un alcoxy en C₁-C₆-carbonylalkyle en C₁-C₆ et un groupe 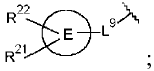 et
R¹⁴ est choisi parmi un alkyle en C₁-C₆, un carboxyalkyle en C₁-C₆, un groupe 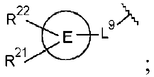
ou R¹³ et R¹⁴, pris conjointement avec l'atome d'azote auquel ils sont liés, forment un hétérocycle à 3 à 14 chaînons ;
R¹⁵ représente un alkyle en C₁-C₆ ;
R¹⁶ est choisi parmi un groupe 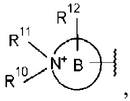 un groupe 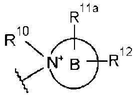 et un groupe 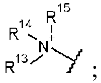
R¹⁷ représente un groupe R¹⁶-L⁷- ;
R¹⁸ représente un hydrogène ;
R¹⁹ est choisi parmi un hydrogène, un amino et un alkyle en C₁-C₆ ;
R²⁰ représente un hydrogène ou un alkyle en C₁-C₆ ;
R²¹ et R²² représentent tous deux un hydrogène ;
L¹ représente une liaison covalente ou -alkyle en C₁-C₆- ;
L² est choisi parmi une liaison covalente, un carbonyle, -alkyle en C₁-C₆-C(O)-NH-, -C(O)-NH-, -C(O)-NH-CH(alcoxy en C₁-C₆-carbonyle)-, -alkyle en C₁-C₆-CH(OH)-alkyle en C₁-C₆-C(O)- et -alkyle en C₁-C₆-NH- ;
L³ représente une liaison covalente ;
L⁴ est choisi parmi un carbonyle, -alkyle en C₁-C₆-C(O)-, -alkyle en C₁-C₆-C(O)-NH-, -C(O)-NH-, -alkyle en C₁-C₆-CH(OH)-alkyle en C₁-C₆-C(O)- et -alkyle en C₁-C₆-NH- ;
L⁷ représente -alkyle en C₁-C₆- ;
L⁸ est choisi parmi une liaison covalente, -alkyle en C₁-C₆- et -alkyle en C₁-C₆-NH-C(O)-alkyle en C₁-C₆- ;
L⁹ représente -alkyle en C₁-C₆- ou -C(O)-alkyle en C₁-C₆- ;
A représente un cycloalkyle en C₃-C₁₀ ou un hétérocycle à 3 à 14 chaînons ;
B représente un hétérocycle à 3 à 14 chaînons ;
D est choisi parmi un cycloalkyle en C₃-C₁₀, un hétérocyclyle à 3 à 14 chaînons et un hétéroaryle à 5 à 14 chaînons ; et
E représente un hétérocyclyle à 3 à 14 chaînons.

3. Composé de formule (IIa) ou (IIIa) selon la revendication 1, ou un sel pharmaceutiquement acceptable de celui-ci, dans lequel :
R¹ est choisi parmi un R⁶-alkyle en C₁-C₆-, un R⁷-alkyle en C₁-C₆-O-alkyle en C₁-C₆- et un groupe 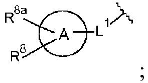
R² est choisi parmi un hydrogène et un alkyle en C₁-C₆ ;
R⁶ représente un groupe R¹⁶-L²- ;
R⁷ représente un groupe R¹⁶-L³- ;
R⁸ représente un groupe R¹⁶-L⁴- ;
R^{8a} représente un hydrogène ;
R¹⁰ est choisi parmi un carbamoylalkyle en C₁-C₆, un cyanoalkyle en C₁-C₆, un alkyle en C₁-C₆, un carboxyalkyle en C₁-C₆ et un groupe 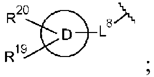
R¹¹ représente un alkyle en C₁-C₆ ou un carboxyalkyle en C₁-C₆ ;
R^{11a} et R¹² représentent tous deux un hydrogène ;
R¹³ est choisi parmi un alkyle en C₁-C₆, un aminoalkyle en C₁-C₆, un carbamoylalkyle en C₁-C₆ et un groupe 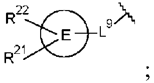
R¹⁴ représente un alkyle en C₁-C₆ ou un carboxyalkyle en C₁-C₆ ;
R¹⁵ représente un alkyle en C₁-C₆ ;
R¹⁶ est choisi parmi un groupe 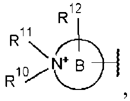 un groupe 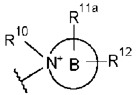 et un groupe 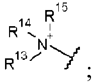
R¹⁹ et R²⁰ représentent tous deux un hydrogène ;
R²¹ et R²² représentent tous deux un hydrogène ;
L¹, L² et L³ représentent une liaison covalente ;
L⁴ représente -alkyle en C₁-C₆-C(O)- ou -alkyle en C₁-C₆-C(O)-NH- ;
L⁸ et L⁹ représentent -alkyle en C₁-C₆- ;
A représente un cycloalkyle en C₃-C₁₀ ou un hétérocycle à 3 à 14 chaînons ;
B représente un hétérocycle à 3 à 14 chaînons ;
D représente un hétérocyclyle à 3 à 14 chaînons ; et
E représente un hétérocyclyle à 3 à 14 chaînons.

4. Composé de formule (IIa) ou (IIIa) selon la revendication 1, ou un sel pharmaceutiquement acceptable de celui-ci, dans lequel :
R¹ est choisi parmi R⁶-(CH₂)n-, R⁷-(CH₂)₂-O-(CH₂)₂- et un groupe 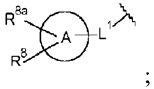
R² est choisi parmi un hydrogène et un méthyle ;
R⁶ représente un groupe R¹⁶-L²- ;
R⁷ représente un groupe R¹⁶-L³- ;
R⁸ représente un groupe R¹⁶-L⁴- ;
R^{8a} représente un hydrogène ;
R¹⁰ est choisi parmi un carbamoyl-CH₂-, un méthyle, un carboxy-CH₂-, un cyano-CH₂- et un groupe 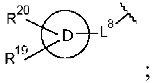
R¹¹ représente un méthyle ou un carboxy-CH₂- ;
R^{11a} et R¹² représentent tous deux un hydrogène ;
R¹³ est choisi parmi un méthyle, un aminopropyle, un carbamoyl-CH₂- et un groupe 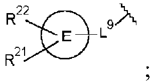
R¹⁴ représente un méthyle ou un carboxyméthyle ;
R¹⁵ représente un méthyle ;
R¹⁶ est choisi parmi un groupe 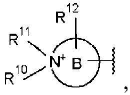 un groupe 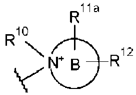 et un groupe 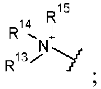
R¹⁹ et R²⁰ représentent tous deux un hydrogène ;
R²¹ et R²² représentent tous deux un hydrogène ;
L¹, L² et L³ représentent une liaison covalente ;
L⁴ représente -CH₂-C(O)- ou -CH₂-C(O)-NH- ;
L⁸ et L⁹ représentent tous deux -CH₂- ;
A représente un cyclohexyle ou un pyrrolidinyle ;
B représente un pyrrolidinyle, un pipéridyle, un 3-azabicyclo[3.1.0]hexan-6-yle ;
D représente une pyrrolidine ou une azétidine ;
E représente une azétidine ; et
n représente 1, 2 ou 5.

5. Composé de formule (IIa) ou (IIIa) selon la revendication 1, ou un sel pharmaceutiquement acceptable de celui-ci, dans lequel :
R¹ et R², pris conjointement avec l'atome d'azote auquel ils sont liés, forment un hétérocycle à 3 à 14 chaînons qui est substitué par R⁹ et R^{9a} ;
R⁹ représente un groupe R¹⁶-L⁵- ou un groupe 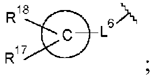
R^{9a} est choisi parmi un hydrogène, un hydroxy et un alcoxy en C₁-C₆-carbonyle ;
R¹⁰ est choisi parmi un alkyle en C₁-C₆, un alkyle en C₁-C₆-NH-alkyle en C₁-C₆, un aminoalkyle en C₁-C₆, un carbamoylalkyle en C₁-C₆ et un groupe 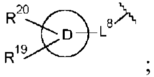
R¹¹ représente un alkyle en C₁-C₆ ;
R¹² représente un hydrogène ou un hydroxy ;
R¹³ représente un alkyle en C₁-C₆ ou un groupe 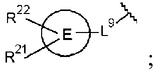 et
R¹⁴ et R¹⁵ représentent tous deux un alkyle en C₁-C₆ ;
R¹⁶ représente un groupe 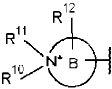 ou un groupe 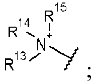
R¹⁷ représente un groupe R¹⁶-L⁷- ;
R¹⁸ représente un hydrogène ;
R¹⁹ représente un hydrogène ou un amino ;
R²⁰, R²¹, R²² représentent un hydrogène ;
B représente un hétérocycle à 3 à 14 chaînons ;
C représente un hétérocycle à 3 à 14 chaînons ;
D représente un cycloalkyle en C₃-C₁₀ ou un hétérocyclyle à 3 à 14 chaînons ;
E représente un hétérocycle à 3 à 14 chaînons ;
L⁵ est choisi parmi une liaison covalente, un carbonyle, -alkyle en C₁-C₆-, -alkyle en C₁-C₆-C(O)- et -alkyle en C₁-C₆ NH-C(O)- ;
L⁶ représente un carbonyle ;
L⁷ représente -alkyle en C₁-C₆- ; et
L⁸ et L⁹ représentent tous deux -alkyle en C₁-C₆-.

6. Composé de formule (IIa) ou (IIIa) selon la revendication 1, ou un sel pharmaceutiquement acceptable de celui-ci, dans lequel :
R¹ et R², pris conjointement avec l'atome d'azote auquel ils sont liés, forment un hétérocycle à 3 à 14 chaînons qui est substitué par R⁹ et R^{9a} ;
R⁹ représente un groupe R¹⁶-L⁵- ;
R^{9a} représente un hydrogène ;
R¹⁰ représente un carbamoylalkyle en C₁-C₆ ou un groupe 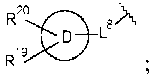
R¹¹ représente un alkyle en C₁-C₆ ;
R¹³, R¹⁴ et R¹⁵ représentent un alkyle en C₁-C₆ ;
R¹⁶ représente un groupe 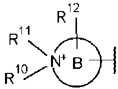 ou un groupe 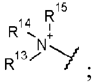
R¹², R¹⁹ et R²⁰ représentent un hydrogène ;
B représente un hétérocycle à 3 à 14 chaînons ;
D représente un hétérocycle à 3 à 14 chaînons ;
L⁵ est choisi parmi un carbonyle, -alkyle en C₁-C₆- et -alkyle en C₁-C₄-C(O)- ; et
L⁸ représente -alkyle en C₁-C₆-.

7. Composé de formule (IIa) ou (IIIa) selon la revendication 1, ou un sel pharmaceutiquement acceptable de celui-ci, dans lequel :
R¹ et R², pris conjointement avec l'atome d'azote auquel ils sont liés, forment un cycle pipérazinyle qui est substitué par R⁹ et R^{9a} ;
R⁹ représente un groupe R¹⁶-L⁵- ;
R^{9a} représente un hydrogène ;
R¹⁰ représente un carbamoylméthyle ou un groupe 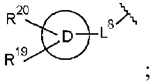
R¹¹ représente un méthyle ;
R¹³, R¹⁴ et R¹⁵ représentent un méthyle ;
R¹⁶ représente un groupe 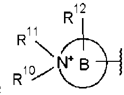 ou un groupe 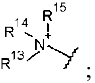
R¹², R¹⁹ et R²⁰ représentent un hydrogène ;
B représente un pipéridyle ;
D représente un pyrrolidinyle ;
L⁵ est choisi parmi un carbonyle, -(CH₂)₂- et -CH₂-C(O)- ; et
L⁸ représente -CH₂-.

8. Composé de formule (IIa) ou (IIIa) selon l'une quelconque des revendications 1 à 7, ou un sel pharmaceutiquement acceptable de celui-ci, dans lequel R₃ est choisi parmi un fluor, un chlore, un méthyle et un éthyle ;
ou R³ et R², pris conjointement avec les atomes auxquels ils sont liés, forment un cycle 6-oxo-2,3-dihydropyridinyle.

9. Composé de formule (IIa) ou (IIIa) selon l'une quelconque des revendications 1 à 8, ou un sel pharmaceutiquement acceptable de celui-ci, dans lequel R⁵ représente un hydrogène ou un alkyle en C₁-C₆.

10. Composé de formule (IIa) ou (IIIa) selon l'une quelconque des revendications 1 à 8, ou un sel pharmaceutiquement acceptable de celui-ci, dans lequel R⁵ représente un hydrogène ou un méthyle.

11. Composé de formule (IIa) ou (IIIa) selon la revendication 1, ou un sel pharmaceutiquement acceptable de celui-ci, dans lequel le composé de formule (IIa) ou (IIIa) est choisi parmi :
l'iodure de 2-(2-(4-((3-(4-(difluorométhoxy)phényl)imidazo[1,2-a]pyrazin-8-yl)amino)-2-méthylbenzamido)éthoxy)-N,N,N-triméthyléthanaminium ;
le formiate de 4-(2-(4-((3-(4-(difluorométhoxy)phényl)imidazo[1,2-a]pyrazin-8-yl)amino)-N,2-diméthylbenzamido)éthyl)-1,1-diméthylpipéridin-1-ium ;
le formiate de 1-éthyl-4-(2-(4-((3-(4-méthoxyphényl)imidazo[1,2-a]pyrazin-8-yl)amino)-2-méthylbenzamido)éthyl)-1-méthylpipéridin-1-ium ;
le 2,2,2-trifluoroacétate de 2-chloro-4-[[3-(2,3-difluoro-4-méthoxy-phényl)imidazo[1,2-a]pyrazin-8-yl]amino]-N-[2-(4,4-diméthylpipérazin-4-ium-1-yl)éthyl]-N-méthyl-benzamide ;
le 2,2,2-trifluoroacétate de 4-[[3-(2,3-difluoro-4-méthoxy-phényl)imidazo[1,2-a]pyrazin-8-yl]amino]-N-[2-(1,1-diméthylpipéridin-1-ium-4-yl)éthyl]-N,2-diméthyl-benzamide ;
le formiate de 1-(carboxyméthyl)-4-(2-(4-((3-(4-méthoxyphényl)imidazo[1,2-a]pyrazin-8-yl)amino)-2-méthylbenzamido)éthyl)-1-méthylpipéridin-1-ium ;
le formiate de 4-(2-(4-((3-(4-(difluorométhoxy)phényl)imidazo[1,2-a]pyrazin-8-yl)amino)-N,2-diméthylbenzamido)éthyl)-1,1-diéthylpipéridin-1-ium;
le formiate de 2-(2-(4-((3-(2,3-difluoro-4-méthoxyphényl)imidazo[1,2-a]pyrazin-8-yl)amino)-2-éthylbenzamido)éthoxy)-N,N,N-triméthyléthanaminium ;
le formiate de 6-(4-((3-(4-(difluorométhoxy)phényl)imidazo[1,2-a]pyrazin-8-yl)amino)-2-méthylbenzamido)-N,N,N-triméthylhexan-1-aminium ;
l'iodure de 2-[4-[2-chloro-4-[[3-[4-(cyanométhoxy)-2,3-difluorophényl]imidazo[1,2-a]pyrazin-8-yl]amino]benzoyl]pipérazin-1-yl]éthyl-triméthylazanium ;
le formiate de 2-(1-(4-((3-(3-fluoro-4-méthoxyphényl)imidazo[1,2-a]pyrazin-8-yl)amino)-2-méthylbenzoyl)pipéridine-4-carboxamido)-N,N,N-triméthyléthanaminium ;
le formiate de 3-(4-((3-(4-chloro-2,3-difluorophényl)imidazo[1,2-a]pyrazin-8-yl)amino)-2-éthylbenzamido)-N,N,N-triméthylpropan-1-aminium ;
le formiate de 2-(1-(4-((3-(4-(difluorométhoxy)phényl)imidazo[1,2-a]pyrazin-8-yl)amino)-2-méthylbenzoyl)pipéridin-4-yl)-N,N,N-triméthyléthanaminium ;
le formiate de [4-[[3-[4-(difluorométhoxy)phényl]imidazo[1,2-a]pyrazin-8-yl]amino]-2-méthyl-phényl]-[4-(4,4-diméthylpipérazin-4-ium-1-carbonyl)pipérazin-1-yl]méthanone ;
le formiate de [2-[4-[2-chloro-4-[[3-(3-fluoro-4-méthoxyphényl)imidazo[1,2-a]pyrazin-8-yl]amino]benzoyl]pipérazin-1-yl]-2-oxoéthyl]-triméthylazanium ;
l'iodure de 2-(4-(4-((3-(3-fluoro-4-méthoxyphényl)imidazo[1,2-a]pyrazin-8-yl)amino)-2-méthylbenzoyl)pipérazin-1-yl)-N,N,N-triméthyl-2-oxoéthan-1-aminium ;
le formiate de [4-[4-[4-[[3-[4-(difluorométhoxy)phényl]imidazo[1,2-a]pyrazin-8-yl]amino]-2-méthyl-benzoyl]pipérazine-1-carbonyl]morpholin-2-yl]méthyl-triméthyl-ammonium ;
l'iodure de 2-chloro-4-[[3-[4-(cyanométhoxy)-2,3-difluoro-phényl]imidazo[1,2-a]pyrazin-8-yl]amino]-N-[2-(4,4-diméthylpipérazin-4-ium-1-yl)éthyl]-N-méthyl-benzamide ;
le chlorure de 2-(2-(4-((3-(3-fluoro-4-méthoxyphényl)imidazo[1,2-a]pyrazin-8-yl)amino)-N,2-diméthylbenzamido)éthoxy)-N,N,N-triméthyléthan-1-aminium ;
le formiate de 2-[[4-[4-[[3-[4-(difluorométhoxy)phényl]imidazo[1,2-a]pyrazin-8-yl]amino]-2-méthyl-benzoyl]pipérazine-1-carbonyl]amino]éthyl-triméthyl-ammonium ;
le formiate de 2-[[(3S,4R)-1-[4-[[3-(3-fluoro-4-méthoxy-phényl)imidazo[1,2-a]pyrazin-8-yl]amino]-2-méthyl-benzoyl]-3-hydroxy-pipéridine-4-carbonyl]amino]éthyl-triméthyl-ammonium ;
le 2,2,2-trifluoroacétate de 2-chloro-4-[[3-[4-(cyanométhoxy)-2,3-difluoro-phényl]imidazo[1,2-a]pyrazin-8-yl]amino]-N-[2-(1,1-diméthylpipéridin-1-ium-4-yl)éthyl]-N-méthyl-benzamide ;
le formiate de 4-(4-(2-(4-((3-(4-(difluorométhoxy)phényl)imidazo[1,2-a]pyrazin-8-yl)amino)-N,2-diméthylbenzamido)éthyl)pipéridin-1-yl)-2-hydroxy-N,N,N-triméthyl-4-oxobutan-1-aminium ;
le formiate de 2-(2-(4-((3-(2,3-difluoro-4-méthoxyphényl)imidazo[1,2-a]pyrazin-8-yl)amino)-2-éthylbenzamido)éthoxy)-N,N,N-triéthyléthan-1-aminium ;
l'iodure de 2-[2-[[4-[[3-[4-(cyanométhoxy)-2,3-difluoro-phényl]imidazo[1,2-a]pyrazin-8-yl]amino]-2-éthyl-benzoyl]amino]éthoxy]éthyl-triméthyl-ammonium ;
le chlorure de [2-[4-[4-[[3-[4-(difluorométhoxy)phényl]imidazo[1,2-a]pyrazin-8-yl]amino]-2-méthyl-benzoyl]pipérazin-1-yl]-2-oxo-éthyl]-triméthyl-ammonium ;
l'iodure de 2-[2-[[4-[[3-[2-chloro-4-(cyanométhoxy)-3-fluoro-phényl]imidazo[1,2-a]pyrazin-8-yl]amino]-2-éthyl-benzoyl]amino]éthoxy]éthyl-triméthyl-ammonium ;
le formiate de 2-[1-[4-[[3-[4-(cyanométhoxy)-2,3-difluoro-phényl]imidazo[1,2-a]pyrazin-8-yl]amino]-2-méthyl-benzoyl]-4-pipéridyl]éthyl-triméthyl-ammonium ;
le 2,2,2-trifluoroacétate de 2-[4-[2-bromo-4-[[3-(3-fluoro-4-méthoxy-phényl)imidazo[1,2-a]pyrazin-8-yl]amino]benzoyl]pipérazin-1-yl]éthyl-triméthyl-ammonium ;
le 2,2,2-trifluoroacétate de 2-[2-[[2-bromo-4-[[3-(2,3-difluoro-4-méthoxy-phényl)imidazo[1,2-a]pyrazin-8-yl]amino]benzoyl]amino]éthoxy]éthyl-triméthyl-ammonium ;
le formiate de 2-[2-[6-[[3-(2,3-difluoro-4-méthoxy-phényl)imidazo[1,2-a]pyrazin-8-yl]amino]-1-oxo-3,4-dihydroisoquinoléin-2-yl]éthoxy]éthyl-triméthyl-ammonium ;
l'iodure de 2-[2-[[2-chloro-4-[[3-[4-(cyanométhoxy)-2,3-difluoro-phényl]imidazo[1,2-a]pyrazin-8-yl]amino]benzoyl]amino]éthoxy]éthyl-tripropyl-ammonium ;
le formiate de 2-chloro-4-[[3-[4-(cyanométhoxy)-2,3-difluoro-phényl]imidazo[1,2-a]pyrazin-8-yl]amino]-N-[2-[2-(1-méthylpyrrolidin-1-ium-1-yl)éthoxy]éthyl]benzamide ;
le formiate de 3-[[2-chloro-4-[[3-(2,3-difluoro-4-méthoxy-phényl)imidazo[1,2-a]pyrazin-8-yl]amino]benzoyl]amino]propyl-triméthyl-ammonium ;
le formiate de 3-[[2-chloro-4-[[3-(2,3-difluoro-4-méthoxy-phényl)imidazo[1,2-a]pyrazin-8-yl]amino]benzoyl]amino]propyl-triéthyl-ammonium ;
le formiate de tributyl-[3-[[2-chloro-4-[[3-(2,3-difluoro-4-méthoxy-phényl)imidazo[1,2-a]pyrazin-8-yl]amino]benzoyl]amino]propyl]ammonium ;
le formiate de carboxyméthyl-[2-[2-[[4-[[3-(2,3-difluoro-4-méthoxy-phényl)imidazo[1,2-a]pyrazin-8-yl]amino]-2-éthyl-benzoyl]amino]éthoxy]éthyl]-diméthyl-ammonium ;
le formiate de 2-[2-[[4-[[3-(2,3-difluoro-4-méthoxy-phényl)imidazo[1,2-a]pyrazin-8-yl]amino]-2-éthyl-benzoyl]amino]éthoxy]éthyl-(2-hydroxyéthyl)-diméthyl-ammonium ;
le 2,2,2-trifluoroacétate de 2-[2-[[4-[[3-(2,3-difluoro-4-prop-2-ynoxy-phényl)imidazo[1,2-a]pyrazin-8-yl]amino]-2-éthyl-benzoyl]amino]éthoxy]éthyl-triméthyl-ammonium ;
le formiate de (2-amino-2-oxo-éthyl)-[2-[2-[[4-[[3-(2,3-difluoro-4-méthoxy-phényl)imidazo[1,2-a]pyrazin-8-yl]amino]-2-éthyl-benzoyl]amino]éthoxy]éthyl]-diméthyl-ammonium ;
le formiate de 3-carboxypropyl-[2-[2-[[4-[[3-(2,3-difluoro-4-méthoxy-phényl)imidazo[1,2-a]pyrazin-8-yl]amino]-2-éthyl-benzoyl]amino]éthoxy]éthyl]-diméthyl-ammonium ;
le formiate de 2-(4-(2-chloro-4-((3-(4-(cyanométhoxy)-2,3-difluorophényl)imidazo[1,2-a]pyrazin-8-yl)amino)benzoyl)pipérazin-1-yl)-N,N,N-triméthyl-2-oxoéthan-1-aminium ;
l'iodure de 2-(4-(2-chloro-4-((3-(2,3-difluoro-4-méthoxyphényl)imidazo[1,2-a]pyrazin-8-yl)amino)benzoyl)pipérazin-1-yl)-N,N,N-triméthyl-2-oxoéthan-1-aminium ;
le formiate de (2S,4R)-2-(4-(4-((3-(2,3-difluoro-4-méthoxyphényl)imidazo[1,2-a]pyrazin-8-yl)amino)-2-méthylbenzoyl)pipérazine-1-carbonyl)-4-hydroxy-1,1-diméthylpyrrolidin-1-ium ;
l'iodure de [2-[4-[4-[[3-[4-(cyanométhoxy)-2,3-difluorophényl]imidazo[1,2-a]pyrazin-8-yl]amino]-2-méthylbenzoyl]pipérazin-1-yl]-2-oxoéthyl]-triméthylazanium ;
le chlorhydrate d'acide 4-[4-[2-[[4-[[3-[4-(difluorométhoxy)phényl]imidazo[1,2-a]pyrazin-8-yl]amino]-2-méthylbenzoyl]-méthylamino]éthyl]-1-méthylpipéridin-1-ium-1-yl]butanoïque ;
le formiate de [2-[2-[[2-chloro-4-[[3-[3-(trifluorométhyl)-1H-pyrazol-4-yl]imidazo[1,2-a]pyrazin-8-yl]amino]benzoyl]amino]éthylamino]-2-oxo-éthyl]-triméthyl-ammonium ;
le formiate de [2-[[3-[[2-chloro-4-[[3-(2,3-difluoro-4-méthoxy-phényl)imidazo[1,2-a]pyrazin-8-yl]amino]benzoyl]amino]cyclobutyl]amino]-2-oxo-éthyl]-triméthyl-ammonium ;
le chlorure de 2-(4-(4-((3-(2,3-difluoro-4-méthoxyphényl)imidazo[1,2-a]pyrazin-8-yl)amino)-2-méthylbenzamido)pipéridin-1-yl)-N,N,N-triméthyl-2-oxoéthan-1-aminium ;
le formiate de [2-[3-[[2-chloro-4-[[3-[3-(trifluorométhyl)-1H-pyrazol-4-yl]imidazo[1,2-a]pyrazin-8-yl]amino]benzoyl]amino]propylamino]-2-oxo-éthyl]-triméthyl-ammonium ;
le formiate de [2-[[3-[[2-chloro-4-[[3-[3-(trifluorométhyl)-1H-pyrazol-4-yl]imidazo[1,2-a]pyrazin-8-yl]amino]benzoyl]amino]cyclobutyl]amino]-2-oxo-éthyl]-triméthyl-ammonium ;
le formiate de [2-[4-[2-chloro-4-[[3-[3-(trifluorométhyl)-1H-pyrazol-4-yl]imidazo[1,2-a]pyrazin-8-yl]amino]benzoyl]pipérazin-1-yl]-2-oxo-éthyl]-triméthyl-ammonium ;
le chlorure de 2-(((1s,4s)-4-(4-((3-(2,3-difluoro-4-méthoxyphényl)imidazo[1,2-a]pyrazin-8-yl)amino)-2-méthylbenzamido)cyclohexyl)amino)-N,N,N-triméthyl-2-oxoéthan-1-aminium ;
le formiate de [2-[4-[4-[[3-[1-(cyanométhyl)-3-(trifluorométhyl)pyrazol-4-yl]imidazo[1,2-a]pyrazin-8-yl]amino]-2-méthyl-benzoyl]pipérazin-1-yl]-2-oxo-éthyl]-triméthyl-ammonium ;
le formiate de [2-[4-[2-chloro-4-[[3-[1-(cyanométhyl)-3-(trifluorométhyl)pyrazol-4-yl]imidazo[1,2-a]pyrazin-8-yl]amino]benzoyl]pipérazin-1-yl]-2-oxo-éthyl]-triméthyl-ammonium ;
l'hydrogénocarbonate de 2-(2-(4-((3-(2,3-difluoro-4-méthoxyphényl)imidazo[1,2-a]pyrazin-8-yl)amino)-2-fluoro-6-méthylbenzamido)éthoxy)-N,N,N-triméthyléthan-1-aminium ;
le formiate de 2-[[3-[[2-chloro-4-[[3-(2,3-difluoro-4-méthoxy-phényl)imidazo[1,2-a]pyrazin-8-yl]amino]benzoyl]amino]cyclobutyl]amino]éthyl-triméthyl-ammonium ;
le chlorure de (S)-2-(3-(4-((3-(2,3-difluoro-4-méthoxyphényl)imidazo[1,2-a]pyrazin-8-yl)amino)-2-méthylbenzamido)pyrrolidin-1-yl)-N,N,N-triméthyl-2-oxoéthan-1-aminium ;
le chlorure de 2-(3-(4-((3-(2,3-difluoro-4-méthoxyphényl)imidazo[1,2-a]pyrazin-8-yl)amino)-2-méthylbenzamido)pipéridin-1-yl)-N,N,N-triméthyl-2-oxoéthan-1-aminium ;
le chlorure de 2-(((1r,4r)-4-(4-((3-(2,3-difluoro-4-méthoxyphényl)imidazo[1,2-a]pyrazin-8-yl)amino)-2-méthylbenzamido)cyclohexyl)amino)-N,N,N-triméthyl-2-oxoéthan-1-aminium ;
le chlorure de (R)-2-(3-(4-((3-(2,3-difluoro-4-méthoxyphényl)imidazo[1,2-a]pyrazin-8-yl)amino)-2-méthylbenzamido)pyrrolidin-1-yl)-N,N,N-triméthyl-2-oxoéthan-1-aminium ;
le formiate de 2-amino-N-(2-(3-(4-((3-(2,3-difluoro-4-méthoxyphényl)imidazo[1,2-a]pyrazin-8-yl)amino)-2-éthylbenzamido)azétidin-1-yl)-2-oxoéthyl)-N,N-diméthyl-2-oxoéthan-1-aminium ;
le chlorure de 2-(((1s,3s)-3-(4-((3-(2,3-difluoro-4-méthoxyphényl)imidazo[1,2-a]pyrazin-8-yl)amino)-2-méthylbenzamido)cyclobutyl)amino)-N,N,N-triméthyl-2-oxoéthan-1-aminium ;
le formiate de 3-(2-chloro-4-((3-(2,3-difluoro-4-méthoxyphényl)imidazo[1,2-a]pyrazin-8-yl)amino)benzamido)-N-(2-hydroxyéthyl)-N,N-diméthylpropan-1-aminium ;
le formiate de N-(cyanométhyl)-2-(2-(4-((3-(2,3-difluoro-4-méthoxyphényl)imidazo[1,2-a]pyrazin-8-yl)amino)-2-éthylbenzamido)éthoxy)-N,N-diméthyléthan-1-aminium ;
le formiate de N-(2-(2-(4-((3-(2,3-difluoro-4-méthoxyphényl)imidazo[1,2-a]pyrazin-8-yl)amino)-2-éthylbenzamido)éthoxy)éthyl)-2-éthoxy-N,N-diméthyl-2-oxoéthan-1-aminium ;
le monoiodure de monoformiate de mono(N-(2-amino-2-oxoéthyl)-5-(4-((3-(2,3-difluoro-4-méthoxyphényl)imidazo[1,2-a]pyrazin-8-yl)amino)-2-éthylbenzamido)-N,N-diméthylpentan-1-aminium) ;
le formiate de trans-[2-[[4-[[4-[[3-(2,3-difluoro-4-méthoxyphényl)imidazo[1,2-a]pyrazin-8-yl]amino]-2-éthylbenzoyl]amino]cyclohexyl]amino]-2-oxoéthyl]-triméthylazanium ;
le formiate de 1-(cyanométhyl)-1-(2-(2-(4-((3-(1-(cyanométhyl)-3-(trifluorométhyl)-1H-pyrazol-4-yl)imidazo[1,2-a]pyrazin-8-yl)amino)-2-éthylbenzamido)éthoxy)éthyl)pyrrolidin-1-ium ;
le formiate de 4-(cyanométhyl)-4-(2-(2-(4-((3-(1-(cyanométhyl)-3-(trifluorométhyl)-1H-pyrazol-4-yl)imidazo[1,2-a]pyrazin-8-yl)amino)-2-éthylbenzamido)éthoxy)éthyl)morpholin-4-ium ;
le formiate de 1-(2-amino-2-oxoéthyl)-4-(4-(4-((3-(4-(difluorométhoxy)-2,3-difluorophényl)imidazo[1,2-a]pyrazin-8-yl)amino)-2-éthylbenzoyl)pipérazine-1-carbonyl)-1-méthylpipéridin-1-ium ;
le bis(2,2,2-trifluoroacétate) de 5-[[4-[[3-[4-(difluorométhoxy)-2,3-difluoro-phényl]imidazo[1,2-a]pyrazin-8-yl]amino]-2-éthyl-benzoyl]amino]pentyl-diméthyl-(2-oxo-2-pipérazin-1-yl-éthyl)ammonium ;
le bis(2,2,2-trifluoroacétate) de N-[[3-(azétidin-3-ylméthyl)-3-méthyl-3-azoniabicyclo[3.1.0]hexan-6-yl]méthyl]-4-[[3-[4-(difluorométhoxy)-2,3-difluoro-phényl]imidazo[1,2-a]pyrazin-8-yl]amino]-2-éthyl-benzamide ;
le 2,2,2-trifluoroacétate de 1-(azétidin-3-ylméthyl)-4-(4-(4-((3-(4-(difluorométhoxy)-2,3-difluorophényl)imidazo[1,2-a]pyrazin-8-yl)amino)-2-éthylbenzoyl)pipérazine-1-carbonyl)-1-méthylpipéridin-1-ium ;
le bis(2,2,2-trifluoroacétate) de N-[3-[[4-[[3-[4-(difluorométhoxy)-2,3-difluoro-phényl]imidazo[1,2-a]pyrazin-8-yl]amino]-2-éthyl-benzoyl]amino]propyl]-1-méthyl-1-(pyrrolidin-3-ylméthyl)pipéridin-1-ium-4-carboxamide ;
le bis(2,2,2-trifluoroacétate) de [4-[1-(2-aminoéthyl)-1-méthyl-pipéridin-1-ium-4-carbonyl]pipérazin-1-yl]-[4-[[3-[4-(difluorométhoxy)-2,3-difluoro-phényl]imidazo[1,2-a]pyrazin-8-yl]amino]-2-éthyl-phényl]méthanone ;
le bis(2,2,2-trifluoroacétate) de [4-[[3-[4-(difluorométhoxy)-2,3-difluoro-phényl]imidazo[1,2-a]pyrazin-8-yl]amino]-2-éthyl-phényl]-[4-[1-méthyl-1-(pyrrolidin-3-ylméthyl)pipéridin-1-ium-4-carbonyl]pipérazin-1-yl]méthanone ;
le bis(2,2,2-trifluoroacétate) de [4-[[3-[4-(difluorométhoxy)-2,3-difluoro-phényl]imidazo[1,2-a]pyrazin-8-yl]amino]-2-éthyl-phényl]-[4-[1-méthyl-1-[2-(méthylamino)éthyl]pipéridin-1-ium-4-carbonyl]pipérazin-1-yl]méthanone ;
le bis(2,2,2-trifluoroacétate) de [4-[1-[(3-amino-1-bicyclo[1.1.1]pentanyl)méthyl]-1-méthyl-pipéridin-1-ium-4-carbonyl]pipérazin-1-yl]-[4-[[3-[4-(difluorométhoxy)-2,3-difluoro-phényl]imidazo[1,2-a]pyrazin-8-yl]amino]-2-éthyl-phényl]méthanone ;
le diformiate de N-[2-[1-(azétidin-3-ylméthyl)-1-méthyl-pipéridin-1-ium-4-yl]éthyl]-4-[[3-[4-(difluorométhoxy)-2,3-difluoro-phényl]imidazo[1,2-a]pyrazin-8-yl]amino]-2-éthyl-benzamide ;
le diformiate de N-[[1-(azétidin-3-ylméthyl)-1-méthyl-pipéridin-1-ium-4-yl]méthyl]-4-[[3-[4-(difluorométhoxy)-2,3-difluoro-phényl]imidazo[1,2-a]pyrazin-8-yl]amino]-2-éthyl-benzamide ;
le bis(2,2,2-trifluoroacétate) de N-[[1-(azétidin-3-ylméthyl)-1-méthyl-pyrrolidin-1-ium-3-yl]méthyl]-4-[[3-[4-(difluorométhoxy)-2,3-difluoro-phényl]imidazo[1,2-a]pyrazin-8-yl]amino]-2-éthyl-benzamide ;
le bis(2,2,2-trifluoroacétate) de bis(azétidin-3-ylméthyl)-[5-[[4-[[3-[4-(difluorométhoxy)-2,3-difluoro-phényl]imidazo[1,2-a]pyrazin-8-yl]amino]-2-éthyl-benzoyl]amino]pentyl]-méthyl-ammonium ;
le diformiate de N-[2-[1-(azétidin-3-ylméthyl)-1-méthyl-azétidin-1-ium-3-yl]éthyl]-4-[[3-[4-(difluorométhoxy)-2,3-difluoro-phényl]imidazo[1,2-a]pyrazin-8-yl]amino]-2-éthyl-benzamide ;
le diformiate de N-[2-[1-(azétidin-3-ylméthyl)-1-méthyl-pipéridin-1-ium-4-yl]éthyl]-4-[[3-[4-(difluorométhoxy)-2,3-difluoro-phényl]imidazo[1,2-a]pyrazin-8-yl]amino]-2-éthyl-benzamide ;
le bis(2,2,2-trifluoroacétate) de N-[3-[4-(azétidin-3-ylméthyl)-4-méthyl-pipérazin-4-ium-1-yl]-3-oxo-propyl]-4-[[3-[4-(difluorométhoxy)-2,3-difluoro-phényl]imidazo[1,2-a]pyrazin-8-yl]amino]-2-éthyl-benzamide ;
le 4-[1-(azétidin-3-ylméthyl)-1-méthyl-pipéridin-1-ium-4-carbonyl]-1-[4-[[3-[4-(difluorométhoxy)-2,3-difluoro-phényl]imidazo[1,2-a]pyrazin-8-yl]amino]-2-éthyl-benzoyl]pipérazine-2-carboxylate bis(2,2,2-trifluoroacétate) de méthyle ;
le 1-[1-(azétidin-3-ylméthyl)-1-méthyl-pipéridin-1-ium-4-carbonyl]-4-[4-[[3-[4-(difluorométhoxy)-2,3-difluoro-phényl]imidazo[1,2-a]pyrazin-8-yl]amino]-2-éthyl-benzoyl]pipérazine-2-carboxylate tris(2,2,2-trifluoroacétate) de méthyle ;
le bis(2,2,2-trifluoroacétate) de [4-[[3-[4-(difluorométhoxy)-2,3-difluoro-phényl]imidazo[1,2-a]pyrazin-8-yl]amino]-2-éthyl-phényl]-[2-[1-méthyl-1-(pyrrolidin-3-ylméthyl)pipéridin-1-ium-4-carbonyl]-2,6-diazaspiro[3.3]heptan-6-yl]méthanone ;
le bis(2,2,2-trifluoroacétate) d'azétidin-3-ylméthyl-[5-[[4-[[3-[4-(difluorométhoxy)-2,3-difluoro-phényl]imidazo[1,2-a]pyrazin-8-yl]amino]-2-éthyl-benzoyl]amino]pentyl]-diméthyl-ammonium ;
le 2,2,2-trifluoroacétate de [4-[[3-[4-(difluorométhoxy)-2,3-difluoro-phényl]imidazo[1,2-a]pyrazin-8-yl]amino]-2-éthyl-phényl]-[4-[4-méthyl-4-(pyrrolidin-3-ylméthyl)pipérazin-4-ium-1-carbonyl]-1-pipéridyl]méthanone ;
le formiate/2,2,2-trifluoroacétate de N-[[1-(azétidin-3-ylméthyl)-1-méthyl-pyrrolidin-1-ium-3-yl]méthyl]-1-[4-[[3-[4-(difluorométhoxy)-2,3-difluoro-phényl]imidazo[1,2-a]pyrazin-8-yl]amino]-2-éthyl-benzoyl]pipéridine-4-carboxamide (sel mixte) ;
le 2,2,2-trifluoroacétate d'acide formique de [4-[2-(azétidin-3-ylméthyl)-2-méthyl-6-aza-2-azoniaspiro[3.3]-6-carbonyl]-1-pipéridyl]-[4-[[3-[4-(difluorométhoxy)-2,3-difluoro-phényl]imidazo[1,2-a]pyrazin-8-yl]amino]-2-éthyl-phényl]méthanone (sel mixte) ;
la [4-[[3-[4-(difluorométhoxy)-2,3-difluoro-phényl]imidazo[1,2-a]pyrazin-8-yl]amino]-2-éthyl-phényl]-[4-[rac-(1S,4S)-5-(azétidin-3-ylméthyl)-5-méthyl-2-aza-5-azoniabicyclo[2.2.1]heptane-2-carbonyl]-1-pipéridyl]méthanone ; acide formique ; 2,2,2-trifluoroacétate (sel mixte) ;
le formiate/2,2,2-trifluoroacétate de [4-[6-(azétidin-3-ylméthyl)-6-méthyl-3-aza-6-azoniabicyclo[3.1.1]heptane-3-carbonyl]-1-pipéridyl]-[4-[[3-[4-(difluorométhoxy)-2,3-difluoro-phényl]imidazo[1,2-a]pyrazin-8-yl]amino]-2-éthyl-phényl]méthanone (sel mixte) ;
le bis(2,2,2-trifluoroacétate) de N-[2-[[4-[[3-[4-(difluorométhoxy)-2,3-difluoro-phényl]imidazo[1,2-a]pyrazin-8-yl]amino]-2-éthyl-benzoyl]amino]éthyl]-1-méthyl-1-(pyrrolidin-3-ylméthyl)pipéridin-1-ium-4-carboxamide ;
le 2,2,2-trifluoroacétate de [4-[[3-[4-(difluorométhoxy)-2,3-difluoro-phényl]imidazo[1,2-a]pyrazin-8-yl]amino]-2-fluoro-phényl]-[4-[1-méthyl-1-(pyrrolidin-3-ylméthyl)pipéridin-1-ium-4-carbonyl]pipérazin-1-yl]méthanone ;
le formiate de 3-aminopropyl-(carboxyméthyl)-[5-[[4-[[3-(2,3-difluoro-4-méthoxy-phényl)imidazo[1,2-a]pyrazin-8-yl]amino]-2-éthyl-benzoyl]amino]pentyl]-méthyl-ammonium ;
le 2,2,2-trifluoroacétate de 5-aminopentyl-(carboxyméthyl)-[5-[[4-[[3-(2,3-difluoro-4-méthoxy-phényl)imidazo[1,2-a]pyrazin-8-yl]amino]-2-éthyl-benzoyl]amino]pentyl]-méthyl-ammonium ;
le formiate de 4-aminobutyl-(carboxyméthyl)-[5-[[4-[[3-(2,3-difluoro-4-méthoxy-phényl)imidazo[1,2-a]pyrazin-8-yl]amino]-2-éthyl-benzoyl]amino]pentyl]-méthyl-ammonium ;
le formiate d'azétidin-3-ylméthyl-(carboxyméthyl)-[5-[[4-[[3-(2,3-difluoro-4-méthoxy-phényl)imidazo[1,2-a]pyrazin-8-yl]amino]-2-éthyl-benzoyl]amino]pentyl]-méthyl-ammonium ;
le 2,2,2-trifluoroacétate d'azétidin-3-ylméthyl-(3-carboxypropyl)-[5-[[4-[[3-(2,3-difluoro-4-méthoxy-phényl)imidazo[1,2-a]pyrazin-8-yl]amino]-2-éthyl-benzoyl]amino]pentyl]-méthyl-ammonium ;
le formiate d'azétidin-3-ylméthyl-(carboxyméthyl)-[5-[[4-[[3-[1-(2,2-difluoroéthyl)-3-(trifluorométhyl)pyrazol-4-yl]imidazo[1,2-a]pyrazin-8-yl]amino]-2-éthyl-benzoyl]amino]pentyl]-méthyl-ammonium ;
le formiate de 2-[[3-[[2-chloro-4-[[3-[3-(trifluorométhyl)-1H-pyrazol-4-yl]imidazo[1,2-a]pyrazin-8-yl]amino]benzoyl]amino]cyclobutyl]amino]éthyl-triméthyl-ammonium ;
le formiate d'azétidin-3-ylméthyl-(carboxyméthyl)-[5-[[4-[[3-[1-(cyanométhyl)-3-(trifluorométhyl)pyrazol-4-yl]imidazo[1,2-a]pyrazin-8-yl]amino]-2-éthyl-benzoyl]amino]pentyl]-méthyl-ammonium ;
le formiate de carboxyméthyl-[5-[[4-[[3-[1-(cyanométhyl)-3-(trifluorométhyl)pyrazol-4-yl]imidazo[1,2-a]pyrazin-8-yl]amino]-2-éthyl-benzoyl]amino]pentyl]-diméthyl-ammonium ;
le formiate de triméthyl-[2-[4-[2-méthyl-4-[[3-[1-méthyl-3-(trifluorométhyl)pyrazol-4-yl]imidazo[1,2-a]pyrazin-8-yl]amino]benzoyl]pipérazin-1-yl]-2-oxo-éthyl]ammonium ;
le formiate d'acide 4-[2-[2-[[2-chloro-4-[[3-[4-(cyanométhoxy)-2,3-difluoro-phényl]imidazo[1,2-a]pyrazin-8-yl]amino]benzoyl]amino]éthoxy]éthyl]-4-méthyl-pipérazin-4-ium-2-carboxylique ;
le 2,2,2-trifluoroacétate de bis(carboxyméthyl)-[5-[[4-[[3-(2,3-difluoro-4-méthoxy-phényl)imidazo[1,2-a]pyrazin-8-yl]amino]-2-éthyl-benzoyl]amino]pentyl]-méthyl-ammonium ;
le formiate de 4-[[3-[4-(4-aminobut-2-ynoxy)phényl]imidazo[1,2-a]pyrazin-8-yl]amino]-N-[2-(1,1-diméthylpipéridin-1-ium-4-yl)éthyl]-N,2-diméthyl-benzamide ;
le formiate de carboxyméthyl-[5-[[4-[[3-[1-(2,2-difluoroéthyl)-3-(trifluorométhyl)pyrazol-4-yl]imidazo[1,2-a]pyrazin-8-yl]amino]-2-éthyl-benzoyl]amino]pentyl]-diméthyl-ammonium ;
le formiate de 1-(azétidin-3-ylméthyl)-N-[(2S)-2-[[4-[[3-[4-(difluorométhoxy)-2,3-difluoro-phényl]imidazo[1,2-a]pyrazin-8-yl]amino]-2-éthyl-benzoyl]amino]propyl]-1-méthyl-pipéridin-1-ium-4-carboxamide ;
le 2,2,2-trifluoroacétate d'acide 2-[4-[[[4-[[3-(2,3-difluoro-4-méthoxy-phényl)imidazo[1,2-a]pyrazin-8-yl]amino]-2-éthyl-benzoyl]amino]méthyl]-1-méthyl-pipéridin-1-ium-1-yl]acétique ;
le formiate de [2-chloro-4-[[3-[4-(difluorométhoxy)-2,3-difluoro-phényl]imidazo[1,2-a]pyrazin-8-yl]amino]phényl]-[4-[1-méthyl-1-(pyrrolidin-3-ylméthyl)pipéridin-1-ium-4-carbonyl]pipérazin-1-yl]méthanone ;
le formiate de N-[3-[[2-chloro-4-[[3-[4-(difluorométhoxy)-2,3-difluoro-phényl]imidazo[1,2-a]pyrazin-8-yl]amino]benzoyl]amino]propyl]-1-méthyl-1-(pyrrolidin-3-ylméthyl)pipéridin-1-ium-4-carboxamide ;
le formiate de N-[3-[[4-[[3-(2,3-difluoro-4-pyrimidin-2-yloxy-phényl)imidazo[1,2-a]pyrazin-8-yl]amino]-2-éthyl-benzoyl]amino]propyl]-1-méthyl-1-(pyrrolidin-3-ylméthyl)pipéridin-1-ium-4-carboxamide ;
le formiate de 4-[[3-(2,3-difluoro-4-pyrimidin-2-yloxy-phényl)imidazo[1,2-a]pyrazin-8-yl]amino]-2-éthyl-N-[[1-méthyl-1-(pyrrolidin-3-ylméthyl)pipéridin-1-ium-4-yl]méthyl]benzamide ;
le formiate de 4-[[3-[2,3-difluoro-4-(2-méthyltriazol-4-yl)oxy-phényl]imidazo[1,2-a]pyrazin-8-yl]amino]-2-éthyl-N-[[1-méthyl-1-(pyrrolidin-3-ylméthyl)pipéridin-1-ium-4-yl]méthyl]benzamide ;
le formiate de [4-[[3-[4-(difluorométhoxy)-2,3-difluoro-phényl]imidazo[1,2-a]pyrazin-8-yl]amino]-2-éthyl-phényl]-[4-méthyl-4-(pyrrolidin-3-ylméthyl)pipérazin-4-ium-1-yl]méthanone ;
le formiate/2,2,2-trifluoroacétate de (2S)-2-[[1-(azétidin-3-ylméthyl)-1-méthyl-pipéridin-1-ium-4-carbonyl]amino]-3-[[4-[[3-(2,3-difluoro-4-méthoxy-phényl)imidazo[1,2-a]pyrazin-8-yl]amino]-2-éthyl-benzoyl]amino]propanoate de méthyle (sel mixte) ;
le formiate/2,2,2-trifluoroacétate de (2S)-2-[[1-(azétidin-3-ylméthyl)-1-méthyl-pipéridin-1-ium-4-carbonyl]amino]-4-[[4-[[3-(2,3-difluoro-4-méthoxy-phényl)imidazo[1,2-a]pyrazin-8-yl]amino]-2-éthyl-benzoyl]amino]butanoate de méthyle (sel mixte) ;
le formiate/2,2,2-trifluoroacétate de (2S)-2-[[1-(azétidin-3-ylméthyl)-1-méthyl-pipéridin-1-ium-4-carbonyl]amino]-3-[[4-[[3-(2,3-difluoro-4-méthoxy-phényl)imidazo[1,2-a]pyrazin-8-yl]amino]-2-éthyl-benzoyl]amino]propanoate de méthyle (sel mixte) ;
le formiate/2,2,2-trifluoroacétate de (2S)-2-[[1-(azétidin-3-ylméthyl)-1-méthyl-pipéridin-1-ium-4-carbonyl]amino]-4-[[4-[[3-(2,3-difluoro-4-méthoxy-phényl)imidazo[1,2-a]pyrazin-8-yl]amino]-2-éthyl-benzoyl]amino]butanoate de méthyle (sel mixte) ;
le formiate/2,2,2-trifluoroacétate de 1-[1-(azétidin-3-ylméthyl)-1-méthyl-pipéridin-1-ium-4-carbonyl]-4-[[[4-[[3-(2,3-difluoro-4-méthoxy-phényl)imidazo[1,2-a]pyrazin-8-yl]amino]-2-éthyl-benzoyl]amino]méthyl]pipéridine-4-carboxylate de méthyle (sel mixte) ;
le formiate/2,2,2-trifluoroacétate de 2-[1-[1-(azétidin-3-ylméthyl)-1-méthyl-pipéridin-1-ium-4-carbonyl]-4-pipéridyl]-2-[[4-[[3-(2,3-difluoro-4-méthoxy-phényl)imidazo[1,2-a]pyrazin-8-yl]amino]-2-éthyl-benzoyl]amino]acétate de méthyle (sel mixte) ;
le formiate/2,2,2-trifluoroacétate de 1-(azétidin-3-ylméthyl)-N-[3-[[4-[[3-(2,3-difluoro-4-méthoxy-phényl)imidazo[1,2-a]pyrazin-8-yl]amino]-2-éthyl-benzoyl]amino]cyclobutyl]-1-méthyl-pipéridin-1-ium-4-carboxamide (sel mixte) ;
le formiate/2,2,2-trifluoroacétate de 1-(azétidin-3-ylméthyl)-N-[3-[[[4-[[3-(2,3-difluoro-4-méthoxy-phényl)imidazo[1,2-a]pyrazin-8-yl]amino]-2-éthyl-benzoyl]amino]méthyl]cyclobutyl]-1-méthyl-pipéridin-1-ium-4-carboxamide (sel mixte) ;
le bis(2,2,2-trifluoroacétate) d'acide 2-[1-(azétidin-3-ylméthyl)-4-[[[4-[[3-(2,3-difluoro-4-méthoxy-phényl)imidazo[1,2-a]pyrazin-8-yl]amino]-2-éthyl-benzoyl]amino]méthyl]pipéridin-1-ium-1-yl]acétique ;
le bis(trifluoroacétate) de 1-(azétidin-3-ylméthyl)-N-[3-[[4-[[3-(2,3-difluoro-4-méthoxy-phényl)imidazo[1,2-a]pyrazin-8-yl]amino]-2-éthyl-benzoyl]amino]propyl]-1-méthyl-pipéridin-1-ium-4-carboxamide ;
le bis(2,2,2-trifluoroacétate) de N-[3-[[2-[(3S)-1-(azétidin-3-ylméthyl)-1-méthyl-pyrrolidin-1-ium-3-yl]acétyl]amino]propyl]-4-[[3-(2,3-difluoro-4-méthoxy-phényl)imidazo[1,2-a]pyrazin-8-yl]amino]-2-éthyl-benzamide ;
le bis(2,2,2-trifluoroacétate) de N-[3-[[2-[1-(azétidin-3-ylméthyl)-1-méthyl-pipéridin-1-ium-4-yl]acétyl]amino]propyl]-4-[[3-(2,3-difluoro-4-méthoxy-phényl)imidazo[1,2-a]pyrazin-8-yl]amino]-2-éthyl-benzamide ;
le bis(2,2,2-trifluoroacétate) de 4-[[3-(2,3-difluoro-4-méthoxy-phényl)imidazo[1,2-a]pyrazin-8-yl]amino]-2-éthyl-N-[[1-méthyl-1-[2-oxo-2-(pyrrolidin-3-ylméthylamino)éthyl]pipéridin-1-ium-4-yl]méthyl]benzamide ;
le bis(2,2,2-trifluoroacétate) de N-[[1-[1-(azétidin-3-ylméthyl)-1-méthyl-pipéridin-1-ium-4-carbonyl]-4-pipéridyl]méthyl]-4-[[3-(2,3-difluoro-4-méthoxy-phényl)imidazo[1,2-a]pyrazin-8-yl]amino]-2-éthyl-benzamide ;
le N-[[1-(azétidin-3-ylméthyl)-1-méthyl-pipéridin-1-ium-4-yl]méthyl]-4-[[3-(2,3-difluoro-4-méthoxy-phényl)imidazo[1,2-a]pyrazin-8-yl]amino]-2-éthyl-benzamide ; 2,2,2-trifluoroacétate bis(2,2,2-trifluoroacétate) ;
le diformiate de 4-[[3-(2,3-difluoro-4-méthoxy-phényl)imidazo[1,2-a]pyrazin-8-yl]amino]-N-[[1-[(1,1-diméthylpyrrolidin-1-ium-3-yl)méthyl]-1-méthyl-pipéridin-1-ium-4-yl]méthyl]-2-éthyl-benzamide ;
le bis(2,2,2-trifluoroacétate) de N-[1-(azétidin-3-ylméthyl)-1-méthyl-pipéridin-1-ium-4-yl]-4-[[3-(2,3-difluoro-4-méthoxy-phényl)imidazo[1,2-a]pyrazin-8-yl]amino]-2-éthyl-benzamide ;
le bis(2,2,2-trifluoroacétate) de 4-[[3-(2,3-difluoro-4-méthoxy-phényl)imidazo[1,2-a]pyrazin-8-yl]amino]-2-éthyl-N-[rac-(1S,5R)-3-(azétidin-3-ylméthyl)-3-méthyl-3-azoniabicyclo[3.1.0]hexan-6-yl]benzamide ;
le bis(2,2,2-trifluoroacétate) de N-[[1-(azétidin-3-ylméthyl)-1-méthyl-pyrrolidin-1-ium-3-yl]méthyl]-2-éthyl-4-[[3-[3-(trifluorométhyl)-1H-pyrazol-4-yl]imidazo[1,2-a]pyrazin-8-yl]amino]benzamide ;
le bis(2,2,2-trifluoroacétate) de bis(azétidin-3-ylméthyl)-[3-[[4-[[3-(2,3-difluoro-4-méthoxy-phényl)imidazo[1,2-a]pyrazin-8-yl]amino]-2-éthyl-benzoyl]amino]propyl]-méthyl-ammonium ;
le 4-[[3-(2,3-difluoro-4-méthoxy-phényl)imidazo[1,2-a]pyrazin-8-yl]amino]-2-éthyl-N-[[1-méthyl-1-(3-pyridylméthyl)pipéridin-1-ium-4-yl]méthyl]benzamide ; 2,2,2-trifluoroacétate ;
le 2,2,2-trifluoroacétate de N-[[1-(azétidin-3-ylméthyl)-1-(2-hydroxyéthyl)pipéridin-1-ium-4-yl]méthyl]-4-[[3-(2,3-difluoro-4-méthoxy-phényl)imidazo[1,2-a]pyrazin-8-yl]amino]-2-éthyl-benzamide ;
le 2,2,2-trifluoroacétate de 4-[[3-(2,3-difluoro-4-méthoxy-phényl)imidazo[1,2-a]pyrazin-8-yl]amino]-2-éthyl-N-[[1-méthyl-1-(pyrrolidin-2-ylméthyl)pipéridin-1-ium-4-yl]méthyl]benzamide ;
le formiate de N-[[1-(azétidin-3-ylméthyl)-1-éthyl-pipéridin-1-ium-4-yl]méthyl]-4-[[3-(2,3-difluoro-4-méthoxy-phényl)imidazo[1,2-a]pyrazin-8-yl]amino]-2-éthyl-benzamide ;
le 2,2,2-trifluoroacétate de 1-[2-[[4-[[3-(2,3-difluoro-4-méthoxy-phényl)imidazo[1,2-a]pyrazin-8-yl]amino]-2-éthyl-benzoyl]amino]éthyl]-1-méthyl-pyrrolidin-1-ium-3-carboxamide ;
le formiate de 4-[[3-(2,3-difluoro-4-phénoxy-phényl)imidazo[1,2-a]pyrazin-8-yl]amino]-2-éthyl-N-[[1-méthyl-1-(pyrrolidin-3-ylméthyl)pipéridin-1-ium-4-yl]méthyl]benzamide ; et
le formiate de 1-(2-méthoxy-2-oxoéthyl)-4-(2-(4-((3-(4-méthoxyphényl)imidazo[1,2-a]pyrazin-8-yl)amino)-2-méthylbenzamido)éthyl)-1-méthylpipéridin-1-ium.

12. Composé de formule (IIa) ou (IIIa) selon l'une quelconque des revendications 1 à 11, ou un sel pharmaceutiquement acceptable de celui-ci, pour une utilisation comme substance thérapeutiquement active.

13. Composition pharmaceutique comprenant un composé de formule (IIa) ou (IIIa) selon l'une quelconque des revendications 1 à 11, ou un sel pharmaceutiquement acceptable de celui-ci, et un véhicule thérapeutiquement inerte.

14. Composé de formule (IIa) ou (IIIa) selon l'une quelconque des revendications 1 à 11, ou un sel pharmaceutiquement acceptable de celui-ci, pour une utilisation comme antibiotique.

15. Composé de formule (IIa) ou (IIIa) selon l'une quelconque des revendications 1 à 11, ou un sel pharmaceutiquement acceptable de celui-ci, pour une utilisation dans le traitement ou la prévention d'infections et de maladies résultantes provoquées par *Acinetobacter baumannii.*
